# EUROPEAN PATENT SPECIFICATION

(11) **EP 1 966 151 B1**
(45) Date of publication and mention of the grant of the patent: **05.10.2011**
(21) Application number: 06845064.2
(22) Date of filing: 11.12.2006
(51) Int. Cl.: C07D 231/56, C07D 403/12, C07D 409/12, C07D 413/12, C07D 417/12, A61K 31/506, A61K 31/427, A61K 31/428, A61K 31/496, A61P 35/00, C07D 417/14, C07D 403/14

(54) **POLYCYCLIC INDAZOLE DERIVATIVES THAT ARE ERK INHIBITORS**
POLYZYKLISCHE INDAZOL-DERIVATE ALS ERK-HEMMER
DERIVES POLYCYCLIQUES D'INDAZOLE INHIBITEURS DES ERK

(30) Priority: 13.12.2005 US 749856 P
(43) Date of publication of application: 10.09.2008
(73) Proprietor: Schering Corporation, Kenilworth, NJ 07033-0530 (US)
(72) Inventor: COOPER, Alan, West Caldwell, NJ 07006 (US); DENG, Yongqi, Newton, MA 02459 (US); SHIPPS, Gerald, W., Jr., Stoneham, MA 02180 (US); SHIH, Neng-yang, Warren, NJ 07059 (US); ZHU, Hugh, Warren , NJ 07059 (US); SUN, Robert, Natick , MA 01760 (US); KELLY, Joseph, Parlin, NJ 08859 (US); DOLL, Ronald, Convent Station, NJ 07960 (US); NAN, Yang, Malden, MA 02148 (US); WANG, Tong, Cambridge, MA 02139 (US); DESAI, Jagdish, Monroe Township, NJ 08831 (US); WANG, James, Westfield, NJ 07090 (US); DONG, Youhao, Edison, NJ 08820 (US); MADISON, Vincent, S., Mountain Lakes, NJ 07046 (US); XIAO, Li, Cranbury, NJ 08512 (US); HRUZA, Alan, Hackettstown, NJ 07840 (US); SIDDIQUI, M., Arshad, Newton, MA 02464 (US); SAMATAR, Ahmed, West Windsor, NJ 08550 (US); PALIWAL, Sunil, Monroe Township, NJ 08831 (US); TSUI, Hon-Chung, East Brunswick, NJ 08816 (US); CELEBI, Azim, A., Burlingame, CA 94010 (US); WU, Yiji, Toronto , Ortario M2N 6Z5 (CA); BOGA, Sobhana, Babu, Scotch Plains, NJ 07076 (US)
(74) Representative: Hussain, Deeba
(86) International application number: PCT/US2006/046959
(87) International publication number: WO 2007/070398

(56) References cited:
- US-A1- 2002 103 229

## Description

### BACKGROUND

The processes involved in tumor growth, progression, and metastasis are mediated by signaling pathways that are activated in cancer cells. The ERK pathway plays a central role in regulating mammalian cell growth by relaying extracellular signals from ligand-bound cell surface tyrosine kinase receptors such as erbB family, PDGF, FGF, and VEGF receptor tyrosine kinase. Activation of the ERK pathway is via a cascade of phosphorylation events that begins with activation of Ras. Activation of Ras leads to the recruitment and activation of Raf, a serine-threonine kinase. Activated Raf then phosphorylates and activates MEK1/2, which then phosphorylates and activates ERK1/2. When activated, ERK1/2 phosphorylates several downstream targets involved in a multitude of cellular events including cytoskeletal changes and transcriptional activation. The ERK/MAPK pathway is one of the most important for cell proliferation, and it is believed that the ERK/MAPK pathway is frequently activated in many tumors. Ras genes, which are upstream of ERK1/2, are mutated in several cancers including colorectal, melanoma, breast and pancreatic tumors. The high Ras activity is accompanied by elevated ERK activity in many human tumors. In addition, mutations of BRAF, a serine-threonine kinase of the Raf family, are associated with increased kinase activity. Mutations in BRAF have been identified in melanomas (60%), thyroid cancers (greater than 40%) and colorectal cancers. These observations indicate that the ERK1/2 signalling pathway is an attractive pathway for anticancer therapies in a broad spectrum of human tumours.

US 2002/103229 discloses indazole derivatives having activity as selective inhibitors of Jun N-terminal kinase (JNK).

Therefore, a welcome contribution to the art would be small-molecules (i.e., compounds) that inhibit ERK activity (i.e., ERK1 and ERK2 activity), which small-molecules would be useful for treating a broad spectrum of cancers, such as, for example, melanoma, pancreatic cancer, thryroid cancer, colorectal cancer, lung cancer, breast cancer, and ovarian cancer. Such a contribution is provided by this invention.

### SUMMARY OF THE INVENTION

This invention provides compounds that inhibit the activity of ERK1 and/or the activity of ERK2.

The compounds of this invention also inhibit the phosphorylation of ERK1 and ERK2.

Thus, this invention provides compounds that are ERK inhibitors (i.e., ERK1 inhibitors and/or ERK2 inhibitors), said compounds being of the formula 1.0: 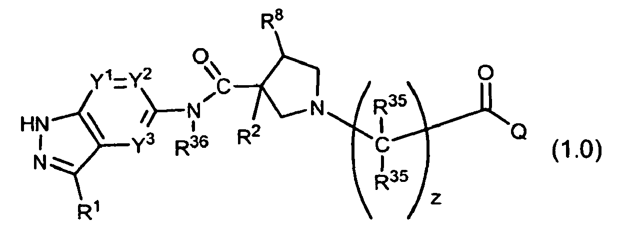 or the pharmaceutically acceptable salts thereof, wherein:
Y¹, Y², and Y³ are each independently selected from the group consisting of: C, N and substituted carbon;
Q is selected from the group consisting of: piperidinyl, piperazinyl, tetrahydropyridinyl (e.g., 1,2,3,6-tetrahydopyridinyl), bridged piperazinyl, bridged piperidinyl, bridged tetrahydropyridinyl, substituted piperidinyl, substituted piperazinyl, substituted tetrahydropyridinyl (e.g., a substituted 1,2,3,6-tetrahydo-pyridinyl), bridged substituted piperazinyl, bridged substituted piperidinyl, and bridged substituted tetrahydropyridinyl;
z is 1 to 3 (and preferably 1); and
R¹, R², R⁸, R³⁵ and R³⁶ are as defined below.

This invention provides compounds of formula 1.0 (for example, as described in any one of Embodiment Nos. 1 to 106) in pure or isolated form.

This invention also provides a pharmaceutical composition comprising an effective amount of at least one (e.g., 1, 2 or 3, 1 or 2, and usually 1) compound of formula 1.0 (for example, as described in any one of Embodiment Nos. 1 to 161) and a pharmaceutically acceptable carrier.

This invention also provides a pharmaceutical composition comprising an effective amount of at least one (e.g., 1, 2 or 3, 1 or 2, and usually 1) compound of formula 1.0 (for example, as described in any one of Embodiment Nos. 1 to 161) and an effective amount of at least one other (e.g., 1, 2 or 3, 1 or 2, and usually 1) pharmaceutically active ingredient (such as, for example, a chemotherapeutic agent), and a pharmaceutically acceptable carrier.

Also disclosed is a method of inhibiting ERK (i.e., inhibiting the activity of ERK) in a patient in need of such treatment comprising administering to said patient an effective amount of at least one (e.g., 1, 2 or 3, 1 or 2, and usually 1) compound of formula 1.0 (for example, as described in any one of Embodiment Nos. 1 to 161).

Also disclosed is a method of inhibiting ERK1 (i.e., inhibiting the activity of ERK1) in a patient in need of such treatment comprising administering to said patient an effective amount of at least one (e.g., 1, 2 or 3, 1 or 2, and usually 1) compound of formula 1.0 (for example, as described in any one of Embodiment Nos. 1 to 161).

Also disclosed is a method of inhibiting ERK2 (i.e., inhibiting the activity of ERK2) in a patient in need of such treatment comprising administering to said patient an effective amount of at least one (e.g., 1, 2 or 3, 1 or 2, and usually 1) compound of formula 1.0 (for example, as described in any one of Embodiment Nos. 1 to 161).

Also disclosed is a method of inhibiting ERK1 and ERK2 (i.e., inhibiting the activity of ERK1 and ERK2) in a patient in need of such treatment comprising administering to said patient an effective amount of at least one (e.g., 1, 2 or 3, 1 or 2, and usually 1) compound of formula 1.0 (for example, as described in any one of Embodiment Nos. 1 to 161).

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with at least one (e.g., 1, 2 or 3, 1 or 2, and usually 1) signal transduction inhibitor.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with at least one (e.g., 1, 2 or 3, 1 or 2, and usually 1) signal transduction inhibitor.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma, in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said cancer is selected from the group consisting of: melanoma, pancreatic cancer, thryroid cancer, colorectal cancer, lung cancer, breast cancer, and ovarian cancer.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with an effective amount of at least one (e.g., 1, 2 or 3, 1 or 2, or 1) chemotherapeutic agent and wherein said cancer is selected from the group consisting of: melanoma, pancreatic cancer, thryroid cancer, colorectal cancer, lung cancer, breast cancer, and ovarian cancer.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said cancer is selected from the group consisting of: melanoma, pancreatic cancer, thryroid cancer, colorectal cancer, lung cancer, breast cancer, and ovarian cancer.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with an effective amount of at least one (e.g., 1, 2 or 3, 1 or 2, or 1) chemotherapeutic agent and wherein said cancer is selected from the group consisting of: melanoma, pancreatic cancer, thryroid cancer, colorectal cancer, lung cancer, breast cancer, and ovarian cancer.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating melanoma in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating melanoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating melanoma in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating melanoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating pancreatic cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating pancreatic cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating pancreatic cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating pancreatic cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating thyroid cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating thyroid cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating thyroid cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating thyroid cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating colorectal cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating colorectal cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating colorectal cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating colorectal cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating lung cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating ovarian cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating ovarian cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating ovarian cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating ovarian cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer (i.e., post-menopausal and premenopausal breast cancer, e.g., hormone-dependent breast cancer) in a patient in need of such treatment, wherein said medicament is in a form for administration in combination with hormonal therapies (i.e., antihormonal agents).

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer (i.e., post-menopausal and premenopausal breast cancer, e.g., hormone-dependent breast cancer) in a patient in need of such treatment, wherein said medicament is in a form for administration in combination with hormonal therapies (i.e., antihormonal agents).

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer (i.e., post-menopausal and premenopausal breast cancer, e.g., hormone-dependent breast cancer) in a patient in need of such treatment, wherein said medicament is in a form for administration in combination with hormonal therapies (i.e., antihormonal agents) and in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating breast cancer (i.e., post-menopausal and premenopausal breast cancer, e.g., hormone-dependent breast cancer) in a patient in need of such treatment, wherein said medicament is in a form for administration in combination with hormonal therapies (i.e., antihormonal agents) and in combination with an effective amount of at least one (e.g., 1, 2 or 3, 1 or 2, or 1) chemotherapeutic agent.

The uses for the manufacture of a medicament for treating breast cancer described herein include the treatment of hormone-dependent metastatic and advanced breast cancer, adjuvant therapy for hormone-dependent primary and early breast cancer, the treatment of ductal carcinoma in situ, and the treatment of inflammatory breast cancer in situ.

The medicaments for treating hormone-dependent breast cancer can also be used to prevent breast cancer in patients having a high risk of developing breast cancer.

Thus, this invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for preventing breast cancer (i.e., post-menopausal and premenopausal breast cancer, e.g., hormone-dependent breast cancer) in a patient in need of such treatmentwherein said medicament is in a form for administration in combination with hormonal therapies (i.e., antihormonal agents).

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for preventing breast cancer (i.e., post-menopausal and premenopausal breast cancer, e.g., hormone-dependent breast cancer) in a patient in need of such treatmentwherein said medicament is in a form for administration in combination with hormonal therapies (i.e., antihormonal agents).

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for preventing breast cancer (i.e., post-menopausal and premenopausal breast cancer, e.g., hormone-dependent breast cancer) in a patient in need of such treatmentwherein said medicament is in a form for administration in combination with hormonal therapies (i.e., antihormonal agents) and in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for preventing breast cancer (i.e., post-menopausal and premenopausal breast cancer, e.g., hormone-dependent breast cancer) in a patient in need of such treatmentwherein said medicament is in a form for administration in combination with hormonal therapies (i.e., antihormonal agents) and in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) a in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) in a patient in need of such treatment, wherein said medicament is in a form for administration in combination with an effective amount of a chemotherapeutic agent and wherein said chemotherapeutic agent is temozolomide.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating brain cancer (e.g., glioma, such as glioma blastoma multiforme) in a patient in need of such treatment, wherein said medicament is in a form for administration in combination with an effective amount of a chemotherapeutic agent, wherein said chemotherapeutic agent is temozolomide.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating prostate cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating prostate cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating prostate cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating prostate cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myelodysplastic syndrome in a patient in need of such treatment. This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myelodysplastic syndrome in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myelodysplastic syndrome in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myelodysplastic syndrome in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating acute myelogenous leukemia (AML) in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating acute myelogenous leukemia (AML) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating acute myelogenous leukemia (AML)in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating acute myelogenous leukemia (AML)in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelomonocytic leukemia (CMML) in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelomonocytic leukemia (CMML) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelomonocytic leukemia (CMML) in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelomonocytic leukemia (CMML) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelogenous leukemia (chronic myeloid leukemia, CML) in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelogenous leukemia (chronic myeloid leukemia, CML) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelogenous leukemia (chronic myeloid leukemia, CML) in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating chronic myelogenous leukemia (chronic myeloid leukemia, CML) in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating myeloid leukemias in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating bladder cancer in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating bladder cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating bladder cancer in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating bladder cancer in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating non-Hodgkin's lymphoma in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating non-Hodgkin's lymphoma in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating multiple myeloma in a patient in need of such treatment.

This invention also provides a use of an effective amount of at least one (e.g., 1, 2 or 3, 1 or 2, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration in combination with an effective amount of at least one (e.g., 1, 2 or 3, 1 or 2, or 1) chemotherapeutic agent.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating multiple myeloma in a patient in need of such treatment.

This invention also provides a use of an effective amount of a pharmaceutical composition comprising an effective amount of at least one (e.g., 1, 2 or 3, 1 or 2, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration in combination with an effective amount of at least one (e.g., 1, 2 or 3, 1 or 2, or 1) chemotherapeutic agent.

In the uses of this invention the compounds of this invention can be in a form for administration concurrently or sequentially (i.e., consecutively) with the chemotherapeutic agents or the signal transduction inhibitor.

The medicaments for treating cancers described herein can optionally be in a form for administration with an effective amount of radiation (i.e., the medicaments for treating cancers described herein are optionally in a form for administration with radiation therapy).

### DETAILED DESCRIPTION OF THE INVENTION

As described herein, unless otherwise indicated, the use of a drug or compound in a specified period is per treatment cycle. For example, once a day means once per day of each day of the treatment cycle. Twice a day means twice per day each day of the treatment cycle. Once a week means one time per week during the treatment cycle. Once every three weeks means once per three weeks during the treatment cycle.

The following abbreviations have the following meanings unless defined otherwise:
- ACN: Acetonitrile
- AcOH: Acetic acid
- DAST: (diethylamino)sulfur trifluoride
- DCC: Dicyclohexylcarbodiimide
- DCU: Dicyclohexylurea
- DCM: Dichloromethane
- DIAD: Diisopropylazodicarboxylate
- DIEA: Diisopropylethylamine
- DMAP: 4-Dimethylaminopyridine
- DME: Dimethoxyethane
- DMF: Dimethylformamide
- DMFDMA: N,N-Dimethylformamide dimethylacetal
- DMSO: Dimethyl sulfoxide
- DTT: Dithiothreitol
- EDCI: 1-(3-dimethylamino-propyl)-3-ethylcarbodiimide hydrochloride
- EtOAc: Ethyl acetate
- EtOH: Ethanol
- HATU: N,N,N',N'-Tetramethyl-O-(7-Azabenzotriazol-1-yl)Uronium hexafluorophosphate
- Hex: hexanes
- HOBt: 1-Hydroxylbenzotriazole
- HPLC: High pressure liquid chromatography
- LCMS: Liquid chromatography mass spectrometry
- LDA: Lithium diisopropylamide
- mCPBA: meta-Chloroperoxybenzoic acid
- MeOH: Methanol
- MTT: (3-[4,5-dimethyl-thiazol-2-yl]-2,5-diphenyltetrazolium bromide, Thiazolyl blue)
- NMR: Nuclear magnetic resonance
- PFP: Pentafluorophenol
- PMB: p-methoxybenzyl
- Pyr: Pyridine
- RT: Room temperature
- SEMCl: 2-(Trimethylsily)ethoxy methyl chloride
- TEA: Triethylamine
- Tr: Triphenyl methane
- TrCl: Triphenyl methane chloride
- TFA: Trifluoroacetic acid
- THF: Tetrahydrofuran
- TLC: Thin layer chromatography
- TMS: Trimethylsilyl

As used herein, unless otherwise specified, the following terms have the following meanings:
"anti-cancer agent" means a drug (medicament or pharmaceutically active ingredient) for treating cancer;
"antineoplastic agent" means a drug (medicament or pharmaceutically active ingredient) for treating cancer (i.e., a chemotherapeutic agent);
"at least one", as used in reference to the number of compounds of this invention means for example 1-6, generally 1-4, more generally 1, 2 or 3, and usually one or two, and more usually one;
"at least one", as used in reference to the number of chemotherapeutic agents used, means for example 1-6, generally 1-4, more generally 1, 2 or 3, and usually one or two, or one;
"chemotherapeutic agent" means a drug (medicament or pharmaceutically active ingredient) for treating cancer (i.e., and antineeoplastic agent);
"compound" with reference to the antineoplastic agents, includes the agents that are antibodies;
"concurrently" means (1) simultaneously in time (e.g., at the same time); or (2) at different times during the course of a common treatment schedule;
"consecutively" means one following the other;
"different" as used in the phrase "different antineoplastic agents" means that the agents are not the same compound or structure; preferably, "different" as used in the phrase "different antineoplastic agents" means not from the same class of antineoplastic agents; for example, one antineoplastic agent is a taxane, and another antineoplastic agent is a platinum coordinator compound;
"effective amount" or "therapeutically effective amount" is meant to describe an amount of compound or a composition of the present invention, or an amount of radiation, effective in treating or inhibiting the diseases or conditions described herein, and thus producing the desired therapeutic, ameliorative, inhibitory or preventative effect; thus, for example, in the uses for the manufacture of medicaments for treating cancer described herein "effective amount" (or "therapeutically effective amount") means, for example, the amount of the compound (or drug), or radiation, that results in: (a) the reduction, alleviation or disappearance of one or more symptoms caused by the cancer, (b) the reduction of tumor size, (c) the elimination of the tumor, and/or (d) long-term disease stabilization (growth arrest) of the tumor; for example, in the treatment of lung cancer (e.g., non small cell lung cancer) a therapeutically effective amount is that amount that alleviates or eliminates cough, shortness of breath and/or pain; also, for example, an effective amount, or a therapeutically effective amount of the ERK inhibitor (i.e., a compound of this invention) is that amount which results in the reduction in ERK (ERK1 and/or ERK2) activity and phosphorylation; the reduction in ERK activity may be determined by the analysis of pharmacodynamic markers such as phosphorylated RSK1,2 and phosphorylated ERK1,2, using techniques well known in the art;
"Ex" in the tables represents "Example";
"one or more" has the same meaning as "at least one";
"patient" means an animal, such as a mammal (e.g., a human being, and preferably a human being);
"prodrug" means compounds that are rapidly transformed, for example, by hydrolysis in blood, in vivo to the parent compound, i.e., to the compounds of formula 1.0 or to a salt and/or to a solvate thereof; a thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference; the scope of this invention includes Prodrugs of the novel compounds of this invention;
"sequentially" represents (1) administration of one component of the use ((a) compound of the invention, or (b) chemotherapeutic agent, signal transduction inhibitor and/or radiation therapy) followed by administration of the other component or components; after adminsitration of one component, the next component can be administered substantially immediately after the first component, or the next component can be administered after an effective time period after the first component; the effective time period is the amount of time given for realization of maximum benefit from the administration of the first component; and
"solvate" means a physical association of a compound of this invention with one or more solvent molecules; this physical association involves varying degrees of ionic and covalent bonding, including hydrogen bonding; in certain instances the solvate will be capable of isolation, for example when one or more solvent molecules are incorporated in the crystal lattice of the crystalline solid; "solvate" encompasses both solution-phase and isolatable solvates; non-limiting examples of suitable solvates include ethanolates, methanolates, and the like; "hydrate" is a solvate wherein the solvent molecule is H₂O.

As used herein, unless otherwise specified, the following terms have the following meanings, and unless otherwise specified, the definitions of each term (i.e., moiety or substituent) apply when that term is used individually or as a component of another term (e.g., the definition of aryl is the same for aryl and for the aryl portion of arylalkyl, alkylaryl, arylalkynyl, and the like):
"acyl" means an H-C(O)-, alkyl-C(O)-, alkenyl-C(O)-, Alkynyl-C(O)-, cycloalkyl-C(O)-, cycloalkenyl-C(O)-, or cycloalkynyl-C(O)- group in which the various groups are as defined below (and as defined below, the alkyl, alkenyl, alkynyl, cycloalkyl, cycloalkenyl and cycloalkynyl moieties can be substituted); the bond to the parent moiety is through the carbonyl; preferred acyls contain a lower alkyl; Non-limiting examples of suitable acyl groups include formyl, acetyl, propanoyl, 2-methylpropanoyl, butanoyl and cyclohexanoyl;
"alkenyl" means an aliphatic hydrocarbon group (chain) comprising at least one carbon to carbon double bond, wherein the chain can be straight or branched, and wherein said group comprises about 2 to about 15 carbon atoms; Preferred alkenyl groups comprise about 2 to about 12 carbon atoms in the chain; and more preferably about 2 to about 6 carbon atoms in the chain; branched means that one or more lower alkyl groups, such as methyl, ethyl or propyl, or alkenyl groups are attached to a linear alkenyl chain; "lower alkenyl" means an alkenyl group comprising about 2 to about 6 carbon atoms in the chain, and the chain can be straight or branched; the term "substituted alkenyl" means that the alkenyl group is substituted by one or more independently selected substituents, and each substituent is independently selected from the group consisting of: halo, alkyl, aryl, cycloalkyl, cyano, alkoxy and -S(alkyl); non-limiting examples of suitable alkenyl groups include ethenyl, propenyl, n-butenyl, 3-methylbut-2-enyl, n-pentenyl, octenyl and decenyl;
"alkoxy" means an alkyl-O- group (i.e., the bond to the parent moiety is through the ether oxygen) in which the alkyl group is unsubstituted or substituted as described below; non-limiting examples of suitable alkoxy groups include methoxy, ethoxy, n-propoxy, isopropoxy, n-butoxy and heptoxy;
"alkoxycarbonyl" means an alkyl-O-CO- group (i.e., the bond to the parent moiety is through the carbonyl) wherein the alkyl group is unsubstituted or substituted as previously defined; non-limiting examples of suitable alkoxycarbonyl groups include methoxycarbonyl and ethoxycarbonyl;
"alkyl" (including the alkyl portions of other moieties, such as trifluoroalkyl and alkyloxy) means an aliphatic hydrocarbon group (chain) that can be straight or branched wherein said group comprises about 1 to about 20 carbon atoms in the chain; preferred alkyl groups comprise about 1 to about 12 carbon atoms in the chain; more preferred alkyl groups comprise about 1 to about 6 carbon atoms in the chain; branched means that one or more lower alkyl groups, such as methyl, ethyl or propyl, are attached to a linear alkyl chain; "lower alkyl" means a group comprising about 1 to about 6 carbon atoms in the chain, and said chain can be straight or branched; the term "substituted alkyl" means that the alkyl group is substituted by one or more independently selected substituents, and wherein each substituent is independently selected from the group consisting of: halo, aryl, cycloalkyl, cyano, hydroxy, alkoxy, alkylthio, amino, -NH(alkyl), -NH(cycloalkyl), -N(alkyl)₂, carboxy, -C(O)O-alkyl and -S(alkyl); non-limiting examples of suitable alkyl groups include methyl, ethyl, n-propyl, isopropyl, n-butyl, t-butyl, n-pentyl, heptyl, nonyl, decyl, fluoromethyl, trifluoromethyl and cyclopropylmethyl;
"alkylaryl" (or alkaryl) means an alkyl-aryl- group (i.e., the bond to the parent moiety is through the aryl group) wherein the alkyl group is unsubstituted or substituted as defined above, and the aryl group is unsubstituted or substituted as defined below; preferred alkylaryls comprise a lower alkyl group; non-limiting examples of suitable alkylaryl groups include o-tolyl, p-tolyl and xylyl;
"alkylheteroaryl" means an alkyl-heteroaryl- group (i.e., the bond to the parent moiety is through the heteroaryl group) wherein the alkyl is unsubstituted or substituted as defined above and the heteroaryl group is unsubstituted or substituted as defined below;
"alkylsulfinyl" means an alkyl-S(O)- group (i.e., the bond to the parent moiety is through the sulfinyl) wherein the alkyl group is unsubstituted or substituted as previously defined; preferred groups are those in which the alkyl group is lower alkyl;
"alkylsulfonyl" means an alkyl-S(O₂)- group (i.e., the bond to the parent moiety is through the sulfonyl) wherein the alkyl group is unsubstituted or substituted as previously defined; preferred groups are those in which the alkyl group is lower alkyl;
"alkylthio" means an alkyl-S- group (i.e., the bond to the parent moiety is through the sulfur) wherein the alkyl group is unsubstituted or substituted as previously described; non-limiting examples of suitable alkylthio groups include methylthio, ethylthio, i-propylthio and heptylthio;
"alkynyl" means an aliphatic hydrocarbon group (chain) comprising at least one carbon to carbon triple bond, wherein the chain can be straight or branched, and wherein the group comprises about 2 to about 15 carbon atoms in the; preferred alkynyl groups comprise about 2 to about 12 carbon atoms in the chain; and more preferably about 2 to about 4 carbon atoms in the chain; Branched means that one or more lower alkyl groups, such as methyl, ethyl or propyl, are attached to a linear alkynyl chain; "lower alkynyl" means an alkynyl group comprising about 2 to about 6 carbon atoms in the chain, and the chain can be straight or branched; non-limiting examples of suitable alkynyl groups include ethynyl, propynyl, 2-butynyl, 3-methylbutynyl, n-pentynyl, and decynyl; the term "substituted alkynyl" means that the alkynyl group is substituted by one or more independently selected, and each substituent is independently selected from the group consisting of alkyl; aryl and cycloalkyl;
"amino means a -NH₂ group;
"aralkenyl" (or arylalkenyl) means an aryl-alkenyl- group (i.e., the bond to the parent moiety is through the alkenyl group) wherein the aryl group is unsubstituted or substituted as defined below, and the alkenyl group is unsubstituted or substituted as defined above; preferred aralkenyls contain a lower alkenyl group; non-limiting examples of suitable aralkenyl groups include 2-phenethenyl and 2-naphthylethenyl;
"aralkyl" (or arylalkyl) means an aryl-alkyl- group (i.e., the bond to the parent moiety is through the alkyl group) wherein the aryl is unsubstituted or substituted as defined below and the alkyl is unsubstituted or substituted as defined above; preferred aralkyls comprise a lower alkyl group; non-limiting examples of suitable aralkyl groups include benzyl, 2-phenethyl and naphthalenylmethyl;
"aralkyloxy" (or arylalkyloxy) means an aralkyl-O- group (i.e., the bond to the parent moiety is through the ether oxygen) wherein the aralkyl group is unsubstituted or substituted as previously described; non-limiting examples of suitable aralkyloxy groups include benzyloxy and 1- or 2-naphthalenemethoxy;
"aralkoxycarbonyl" means an aralkyl-O-C(O)- group (i.e., the bond to the parent moiety is through the carbonyl) wherein the aralkyl group is unsubstituted or substituted as previously defined; a non-limiting example of a suitable aralkoxycarbonyl group is benzyloxycarbonyl;
"aralkylthio" means an aralkyl-S- group (i.e., the bond to the parent moiety is through the sulfur) wherein the aralkyl group is unsubstituted or substituted as previously described; a non-limiting example of a suitable aralkylthio group is benzylthio;
"aroyl" means an aryl-C(O)- group (i.e., the bond to the parent moiety is through the carbonyl) wherein the aryl group is unsubstituted or substituted as defined below; non-limiting examples of suitable groups include benzoyl and 1- and 2-naphthoyl;
"aryl" (sometimes abbreviated "ar") means an aromatic monocyclic or multicyclic ring system comprising about 6 to about 14 carbon atoms, preferably about 6 to about 10 carbon atoms; the aryl group can be optionally substituted with one or more independently selected "ring system substituents" (defined below). Non-limiting examples of suitable aryl groups include phenyl and naphthyl;
"arylalkynyl" means an aryl-alkynyl- group (i.e., the bond to the parent moiety is through the alkynyl group) wherein the aryl group is unsubstituted or substituted as defined above, and the alkynyl group is unsubstituted or substitutedas defined above;
"arylaminoheteroaryl" means an aryl-amino-heteroaryl group (i.e., the bond to the parent moiety is through the heteroaryl group) wherein the aryl group is unsubstituted or substituted as defined above, the amino group is as defined above (i.e., a -NH- here), and the heteroaryl group is unsubstituted or substituted as defined below;
"arylheteroaryl" means an aryl-heteroarylgroup-(i.e., the bond to the parent moiety is through the heteroaryl group) wherein the aryl group is unsubstituted or substituted as defined above, and the heteroaryl group is unsubstituted or substituted as defined below;
"aryloxy" means an aryl-O- group (i.e., the bond to the parent moiety is through the ether oxygen) wherein the aryl group is unsubstituted or substituted as defined above; non-limiting examples of suitable aryloxy groups include phenoxy and naphthoxy;
"aryloxycarbonyl" means an aryl-O-C(O)- group (i.e., the bond to the parent moiety is through the carbonyl) wherein the aryl group is unsubstituted or substituted as previously defined; non-limiting examples of suitable aryloxycarbonyl groups include phenoxycarbonyl and naphthoxycarbonyl;
"arylsulfinyl" means an aryl-S(O)- group (i.e., the bond to the parent moiety is through the sulfinyl) wherein aryl is unsubstituted or substituted as previously defined;
"arylsulfonyl" means an aryl-S(O₂)- group (i.e., the bond to the parent moiety is through the sulfonyl) wherein aryl is unsubstituted or substituted as previously defined;
"arylthio" means an aryl-S- group (i.e., the bond to the parent moiety is through the sulfur) wherein the aryl group is unsubstituted or substituted as previously described; non-limiting examples of suitable arylthio groups include phenylthio and naphthylthio;
"cycloalkenyl" means a non-aromatic mono or multicyclic ring system comprising about 3 to about 10 carbon atoms, preferably about 5 to about 10 carbon atoms that contains at least one carbon-carbon double bond; preferred cycloalkenyl rings contain about 5 to about 7 ring atoms; the cycloalkenyl can be optionally substituted with one or more independently selected "ring system substituents" (defined below); Non-limiting examples of suitable monocyclic cycloalkenyls include cyclopentenyl, cyclohexenyl, cycloheptenyl, and the like; a non-limiting example of a suitable multicyclic cycloalkenyl is norbornylenyl;
"cycloalkyl" means a non-aromatic mono- or multicyclic ring system comprising about 3 to about 7 carbon atoms, preferably about 3 to about 6 carbon atoms; the cycloalkyl can be optionally substituted with one or more independently selected "ring system substituents" (defined below); non-limiting examples of suitable monocyclic cycloalkyls include cyclopropyl, cyclopentyl, cyclohexyl, cycloheptyl and the like; non-limiting examples of suitable multicyclic cycloalkyls include 1-decalin, norbornyl, adamantyl and the like;
"cycloalkylalkyl" means a cycloalkyl-alkyl-group (i.e., the bond to the parent moiety is through the alkyl group) wherein the cycloalkyl moiety is unsubstituted or substituted as defined above, and the alkyl moiety is unsubstituted or substituted as defined above;
"halo" means fluoro, chloro, bromo, or iodo groups; preferred halos are fluoro, chloro or bromo, and more preferred are fluoro and chloro;
"halogen" means fluorine, chlorine, bromine, or iodine; preferred halogens are fluorine, chlorine and bromine;
"haloalkyl" means an alkyl, as defined above, wherein one or more hydrogen atoms on the alkyl is replaced by a halo group, as defined above;
"heteroaralkenyl" means a heteroaryl-alkenyl- group (i.e., the bond to the parent moiety is through the alkenyl group) wherein the heteroaryl group is unsubstituted or substituted as defined below, and the alkenyl group is unsubstituted or substituted as defined above;
"heteroaralkyl" (or heteroarylalkyl) means a heteroaryl-alkyl- group (i.e., the bond to the parent moiety is through the alkyl group) in which the heteroaryl is unsubstituted or substituted as defined below, and the alkyl group is unsubstituted or substituted as defined above; preferred heteroaralkyls comprise an alkyl group that is a lower alkyl group; non-limiting examples of suitable aralkyl groups include pyridylmethyl, 2-(furan-3-yl)ethyl and quinolin-3-ylmethyl;
"heteroaralkylthio" means a heteroaralkyl-S- group wherein the heteroaralkyl group is unsubstituted or substituted as defined above;
"heteroaryl" means an aromatic monocyclic or multicyclic ring system comprising about 5 to about 14 ring atoms, preferably about 5 to about 10 ring atoms, in which one or more of the ring atoms is an element other than carbon, for example nitrogen, oxygen or sulfur, alone or in combination; preferred heteroaryls comprise about 5 to about 6 ring atoms; the "heteroaryl" can be optionally substituted by one or more independently selected "ring system substituents" (defined below); the prefix aza, oxa or thia before the heteroaryl root name means that at least a nitrogen, oxygen or sulfur atom, respectively, is present as a ring atom; a nitrogen atom of a heteroaryl can be optionally oxidized to the corresponding N-oxide; non-limiting examples of suitable heteroaryls include pyridyl, pyrazinyl, furanyl, thienyl, pyrimidinyl, isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like;
"heteroarylalkynyl" (or heteroaralkynyl) means a heteroaryl-alkynyl- group (i.e., the bond to the parent moiety is through the alkynyl group) wherein the heteroaryl group is unsubstituted or substituted as defined above, and the alkynyl group is unsubstituted or substituted as defined above;
"heteroarylaryl" (or heteroararyl) means a heteroaryl-aryl- group (i.e., the bond to the parent moiety is through the aryl group) wherein the heteroaryl group is unsubstituted or substituted as defined above, and the aryl group is unsubstituted or substituted as defined above;
"heteroarylheteroarylaryl" means a heteroaryl-heteroaryl- group (i.e., the bond to the parent moiety is through the last heteroaryl group) wherein each heteroaryl group is independently unsubstituted or substituted as defined above;
"heteroarylsulfinyl" means a heteroaryl-SO- group wherein the heteroaryl group is unsubstituted or substituted as defined above;
"heteroarylsulfonyl" means a heteroaryl-SO₂- group wherein the heteroaryl group is unsubstituted or substituted as defined above;
"heteroarylthio" means a heteroaryl-S- group wherein the heteroaryl group is unsubstituted or substituted as defined above;
"heterocyclenyl" (or heterocycloalkenyl) means a non-aromatic monocyclic or multicyclic ring system comprising about 3 to about 10 ring atoms, preferably about 5 to about 10 ring atoms, in which one or more of the atoms in the ring system is an element other than carbon (for example one or more heteroatoms independently selected from the group consisting of nitrogen, oxygen and sulfur atom), and which contains at least one carbon-carbon double bond or carbon-nitrogen double bond; there are no adjacent oxygen and/or sulfur atoms present in the ring system; Preferred heterocyclenyl rings contain about 5 to about 6 ring atoms; the prefix aza, oxa or thia before the heterocyclenyl root name means that at least a nitrogen, oxygen or sulfur atom, respectively, is present as a ring atom; the heterocyclenyl can be optionally substituted by one or more independently selected "Ring system substituents" (defined below); the nitrogen or sulfur atom of the heterocyclenyl can be optionally oxidized to the corresponding N-oxide, S-oxide or S,S-dioxide; non-limiting examples of suitable monocyclic azaheterocyclenyl groups include 1,2,3,4-tetrahydropyridine, 1,2-dihydropyridyl, 1,4-dihydropyridyl, 1,2,3,6-tetrahydropyridine, 1,4,5,6-tetrahydropyrimidine, 2-pyrrolinyl, 3-pyrrolinyl, 2-imidazolinyl, 2-pyrazolinyl, and the like; Non-limiting examples of suitable oxaheterocyclenyl groups include 3,4-dihydro-2H-pyran, dihydrofuranyl, fluorodihydrofuranyl, and the like; A non-limiting example of a suitable multicyclic oxaheterocyclenyl group is 7-oxabicyclo[2.2.1]heptenyl; non-limiting examples of suitable monocyclic thiaheterocyclenyl rings include dihydrothiophenyl, dihydrothiopyranyl, and the like;
"heterocycloalkylalkyl" (or heterocyclylalkyl) means a heterocycloalkylalkyl- group (i.e., the bond to the parent moiety is through the alkyl group) wherein the heterocycloalkyl group (i.e., the heterocyclyl group) is unsubstituted or substituted as defined below, and the alkyl group is unsubstituted or substituted as defined above;
"heterocyclyl" (or heterocycloalkyl) means a non-aromatic saturated monocyclic or multicyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination; there are no adjacent oxygen and/or sulfur atoms present in the ring system; preferred heterocyclyls contain about 5 to about 6 ring atoms; the prefix aza, oxa or thia before the heterocyclyl root name means that at least a nitrogen, oxygen or sulfur atom respectively is present as a ring atom; the heterocyclyl can be optionally substituted by one or more independently selected "ring system substituents" (defined below); the nitrogen or sulfur atom of the heterocyclyl can be optionally oxidized to the corresponding N-oxide, S-oxide or S,S-dioxide; non-limiting examples of suitable monocyclic heterocyclyl rings include piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, tetrahydrothiopyranyl, and the like;
"hydroxyalkyl" means a HO-alkyl- group wherein the alkyl group is substituted or unsubstituted as defined above; preferred hydroxyalkyls comprise a lower alkyl; Non-limiting examples of suitable hydroxyalkyl groups include hydroxymethyl and 2-hydroxyethyl; and
"ring system substituent" means a substituent attached to an aromatic or non-aromatic ring system that, for example, replaces an available hydrogen on the ring system; ring system substituents are each independently selected from the group consisting of: alkyl, aryl, heteroaryl, aralkyl, alkylaryl, aralkenyl, heteroaralkyl, alkylheteroaryl, heteroaralkenyl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, R⁶⁰R⁶⁵N-, R⁶⁰R⁶⁵N-alkyl-, R⁶⁰R⁶⁵NC(O)- and R⁶⁰R⁶⁵NSO₂-, wherein R⁶⁰ and R⁶⁵ are each independently selected from the group consisting of: hydrogen, alkyl, aryl, and aralkyl; "Ring system substituent" also means a cyclic ring of 3 to 7 ring atoms, wherein 1-2 ring atoms can be heteroatoms, attached to an aryl, heteroaryl, heterocyclyl or heterocyclenyl ring by simultaneously substituting two ring hydrogen atoms on said aryl, heteroaryl, heterocyclyl or heterocyclenyl ring; Non-limiting examples include: 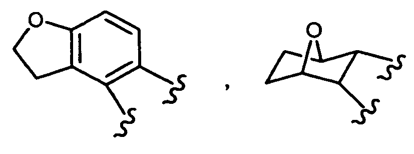 and the like

Lines drawn into a ring mean that the indicated bond may be attached to any of the substitutable ring carbon atoms.

Any carbon or heteroatom with unsatisfied valences in the text, schemes, examples, structural formulae, and any Tables herein is assumed to have the hydrogen atom or atoms to satisfy the valences.

One or more compounds of the invention may also exist as, or optionally converted to, a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTech., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, for example I. R. spectroscopy, show the presence of the solvent (or water) in the crystals as a solvate (or hydrate).

The term "pharmaceutical composition" is also intended to encompass both the bulk composition and individual dosage units comprised of more than one (e.g., two) pharmaceutically active agents such as, for example, a compound of the present invention and an additional agent selected from the lists of the additional agents described herein, along with any pharmaceutically inactive excipients. The bulk composition and each individual dosage unit can contain fixed amounts of the afore-said "more than one pharmaceutically active agents". The bulk composition is material that has not yet been formed into individual dosage units. An illustrative dosage unit is an oral dosage unit such as tablets, capsules, pills and the like. Similarly, the herein-described use of a pharmaceutical composition of the present invention for the manufacture of a medicament for treating a patient is also intended to encompass medicaments that are in a form for administration of the afore-said bulk composition and individual dosage units.

Prodrugs of the compounds of the invention are also contemplated herein. The term "prodrug", as employed herein, denotes a compound that is a drug precursor which, upon administration to a subject, undergoes chemical conversion by metabolic or chemical processes to yield a compound of formula 1.0 or a salt and/or solvate thereof. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press, both of which are incorporated herein by reference thereto.

For example, if a compound of formula 1.0, or a pharmaceutically acceptable salt, hydrate or solvate of the compound, contains a carboxylic acid functional group, a prodrug can comprise an ester formed by the replacement of the hydrogen atom of the acid group with a group such as, for example, (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di (C₁-C₂)alkylcarbamoyl-(C1-C2)alkyl and piperidino-, pyrrolidino- or morpholino(C₂-C₃)alkyl, and the like.

Similarly, if a compound of formula 1.0 contains an alcohol functional group, a prodrug can be formed by the replacement of the hydrogen atom of the alcohol group with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate), and the like.

If a compound of formula 1.0 incorporates an amine functional group, a prodrug can be formed by the replacement of a hydrogen atom in the amine group with a group such as, for example, R⁷⁰-carbonyl, R⁷⁰O-carbonyl, NR⁷⁰R⁷⁵-carbonyl where R⁷⁰ and R⁷⁵ are each independently (C₁-C₁₀)alkyl, (C₃-C₇) cycloalkyl, benzyl, or R⁷⁰-carbonyl is a natural α-aminoacyl or natural α-aminoacyl, -C(OH)C(O)OY⁸⁰ wherein Y⁸⁰ is H, (C₁-C₆)alkyl or benzyl, -C(OY⁸²)Y⁸⁴ wherein Y⁸² is (C₁-C₄) alkyl and Y⁸⁴ is (C₁-C₆)alkyl, carboxy (C₁-C₆)alkyl, amino(C₁-C₄)alkyl or mono-N-or di-N,N-(C₁-C₆)alkylaminoalkyl, -C(Y⁸⁶)Y⁸⁸ wherein Y⁸⁶ is H or methyl and Y⁸⁸ is mono-N- or diN,N-(C₁-C₆)alkylamino morpholino, piperidin-1-yl or pyrrolidin-1-yl, and the like.

This invention also includes the compounds of this invention in isolated and purified form.

Polymorphic forms of the compounds of formula 1.0, and of the salts, solvates and prodrugs of the compounds of formula 1.0, are intended to be included in the present invention.

Certain compounds of the invention may exist in different isomeric (e.g., enantiomers, diastereoisomers, atropisomers) forms. The invention contemplates all such isomers both in pure form and in admixture, including racemic mixtures. Enol forms are also included.

All stereoisomers (for example, geometric isomers, optical isomers and the like) of the present compounds (including those of the salts, solvates and prodrugs of the compounds as well as the salts and solvates of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate" "prodrug" and the like, is intended to equally apply to the salt, solvate and prodrug of enantiomers, stereoisomers, rotamers, tautomers, racemates or prodrugs of the inventive compounds.

Diasteromeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diasteromeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of Formula (I) may be atropisomers (e.g., substituted biaryls) and are considered as part of this invention. Enantiomers can also be separated by use of chiral HPLC column.

The compounds of formula 1.0 form salts that are also within the scope of this invention. Reference to a compound of formula 1.0 herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of formula 1.0 contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable salts) are preferred. Salts of the compounds of the formula 1.0 may be formed, for example, by reacting a compound of formula 1.0 with an amount of acid or base, such as an equivalent amount, in a medium such as one in which the salt precipitates or in an aqueous medium followed by lyophilization. Acids (and bases) which are generally considered suitable for the formation of pharmaceutically useful salts from basic (or acidic) pharmaceutical compounds are discussed, for example, by S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; in The Orange Book (Food & Drug Administration, Washington, D.C. on their website); and P. Heinrich Stahl, Camille G. Wermuth (Eds.), Handbook of Pharmaceutical Salts: Properties, Selection, and Use, (2002) Int'I. Union of Pure and Applied Chemistry, pp. 330-331. These disclosures are incorporated herein by reference thereto.

Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, methyl sulfates, 2-naphthalenesulfonates, nicotinates, nitrates, oxalates, pamoates, pectinates, persulfates, 3-phenylpropionates, phosphates, picrates, pivalates, propionates, salicylates, succinates, sulfates, sulfonates (such as those mentioned herein), tartarates, thiocyanates, toluenesulfonates (also known as tosylates,) undecanoates, and the like.

Exemplary basic salts include ammonium salts, alkali metal salts such as sodium, lithium, and potassium salts, alkaline earth metal salts such as calcium and magnesium salts, aluminum salts, zinc salts, salts with organic bases (for example, organic amines) such as benzathines, diethylamine, dicyclohexylamines, hydrabamines (formed with N,N-bis(dehydroabietyl)ethylenediamine), N-methyl-D-glucamines, N-methyl-D-glucamides, t-butyl amines, piperazine, phenylcyclohexylamine, choline, tromethamine, and salts with amino acids such as arginine, lysine and the like. Basic nitrogen-containing groups may be quarternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

All such acid and base salts are intended to be pharmaceutically acceptable salts within the scope of the invention and all acid and base salts are considered equivalent to the free forms of the corresponding compounds for purposes of the invention.

Compounds of formula 1.0, and salts, solvates and prodrugs thereof, may exist in their tautomeric form (for example, as an amide or imino ether). All such tautomeric forms are contemplated herein as part of the present invention.

In hetero-atom containing ring systems of this invention, there are no hydroxyl groups on carbon atoms adjacent to a N, O or S, and there are no N or S groups on carbon adjacent to another heteroatom. Thus, for example, in the ring: 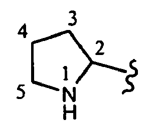 there is no -OH attached directly to carbons marked 2 and 5.

The compounds of formula 1.0 may exist in different tautomeric forms, and all such forms are embraced within the scope of the invention. Also, for example, all keto-enol and imine-enamine forms of the compounds are included in the invention.

Tautomeric forms such as, for example, the moieties: 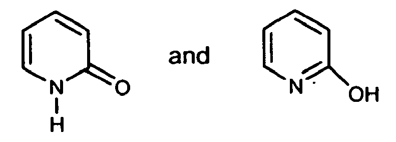 are considered equivalent in certain embodiments of this invention.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound" or "stable structure" is meant a compound that is sufficiently robust to survive isolation to a useful degree of purity from a reaction mixture, and formulation into an efficacious therapeutic agent.

The term "optionally substituted" means optional substitution with the specified groups, radicals or moieties.

The term "purified", "in purified form" or "in isolated and purified form" for a compound refers to the physical state of said compound after being isolated from a synthetic process or natural source or combination thereof. Thus, the term "purified", "in purified form" or "in isolated and purified form" for a compound refers to the physical state of said compound after being obtained from a purification process or processes described herein or well known to the skilled artisan, in sufficient purity to be characterizable by standard analytical techniques described herein or well known to the skilled artisan.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in organic Synthesis (1991), Wiley, New York.

When any variable (e.g., aryl, heterocycle, R³, etc.) occurs more than one time in any moiety or in any compound of formula 1.0, its definition on each occurrence is independent of its definition at every other occurrence.

As used herein, the term "composition" is intended to encompass a product comprising the specified ingredients in the specified amounts, as well as any product which results, directly or indirectly, from combination of the specified ingredients in the specified amounts.

The present invention also embraces isotopically-labelled compounds of the present invention which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

Certain isotopically-labelled compounds of formula 1.0 (e.g., those labeled with ³H and ¹⁴C) are useful in compound and/or substrate tissue distribution assays. Tritiated (i.e., ³H) and carbon-14 (i.e., ¹⁴C) isotopes are particularly preferred for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Isotopically labelled compounds of formula 1.0 can generally be prepared by following procedures analogous to those disclosed in the Schemes and/or in the Examples hereinbelow, by substituting an appropriate isotopically labelled reagent for a non-isotopically labelled reagent.

This invention provides compounds of formula 1.0: 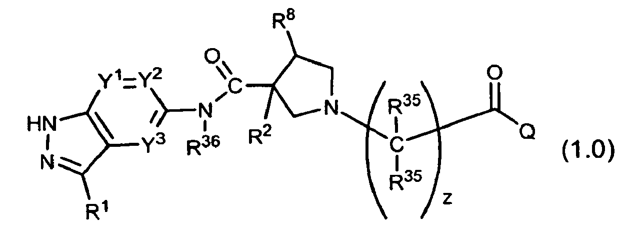 or the pharmaceutically acceptable salts thereof, wherein:
Y¹, Y², and Y³ are each independently selected from the group consisting of: -CH=, -N= and -CR⁹= (preferably Y¹, Y², and Y³ are each -CH=);
z is 1 to 3 (i.e., 1, 2 or 3, and preferably 1);
Q is a substituent selected from the group consisting of: 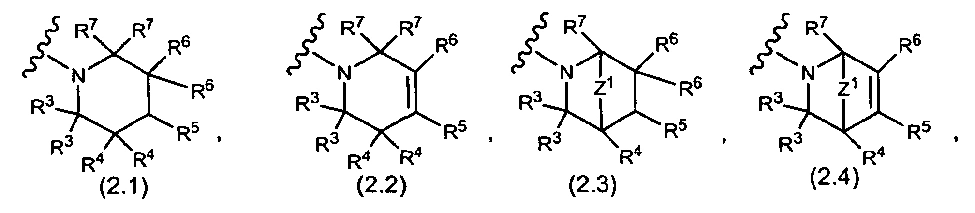 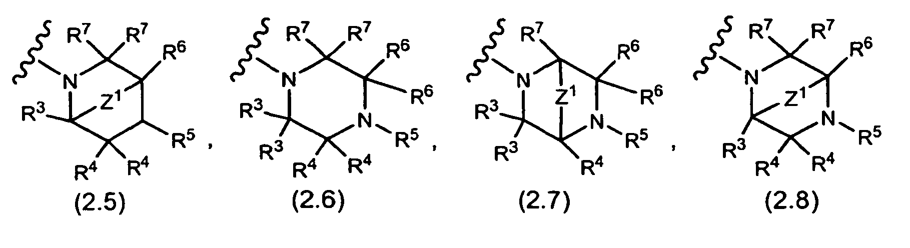 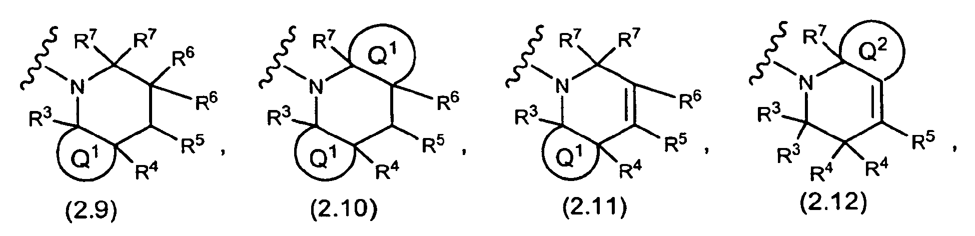 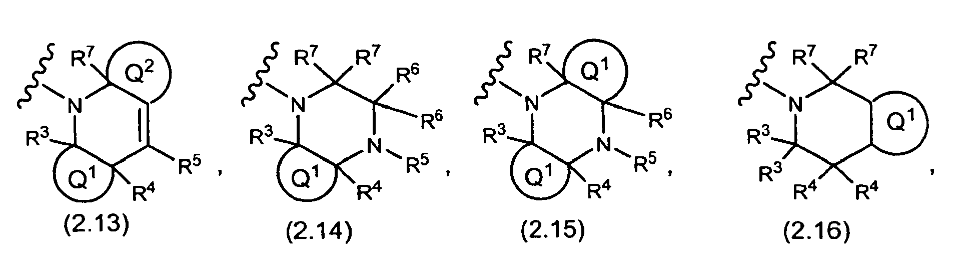 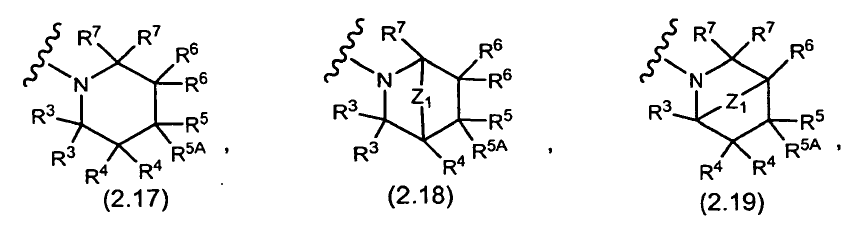 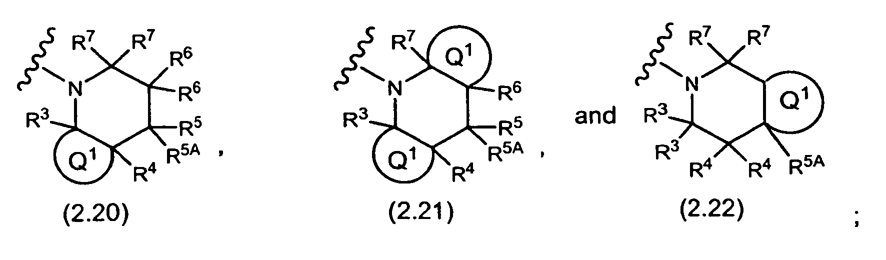
Each Q¹ represents a ring independently selected from the group consisting of: cycloalkyl, substituted cycloalkyl, heterocycloalkyl, substituted heterocycloalkyl, aryl, substituted aryl, heteroaryl, and substituted heteroaryl, wherein said substituted rings are substituted with 1 to 3 substituents independently selected from the group consisting of: the R¹⁰ moieties; provided that when Q¹ is aryl, heteroaryl, substituted aryl or substituted heteroaryl then the carbon atoms at the ring junction (i.e., the two carbon atoms common to the fused rings) are not substituted;
Q² represents a ring selected from the group consisting of: cycloalkyl, substituted cycloalkyl, heterocycloalkyl, and substituted heterocycloalkyl, wherein said substituted rings are substituted with 1 to 3 substituents independently selected from the group consisting of: the R¹⁰ moieties;
Z¹ represents -(C(R²⁴)₂)_{w}- wherein each R²⁴ is independently selected from the group consisting of: H, alkyl (e.g., C₁ to C₆ alkyl, for example methyl) and F, and wherein w is 1, 2 or 3, and generally w is 1 or 2, and usually w is 1, and wherein in one example each R²⁴ is H, and in another example w is 1, and in another example each R²⁴ is H and w is 1, preferably w is 1 and each R²⁴ is H (i.e., preferably Z¹ is -CH₂-);
Z² is selected from the group consisting of: -N(R⁴⁴)-, -O- and -C(R⁴⁶)₂- (e.g., Z² is -NH-, -O- or -CH₂-);
m is 1 to 6;
n is 1 to 6;
p is 0 to 6;
t is 0, 1, or 2;
R¹ is selected from the group consisting of:
   (1) -CN,
   (2) -NO₂,
   (3) -OR¹⁰,
   (4) -SR¹⁰,
   (5) -N(R¹⁰)₂,
   (6) R¹⁰,
   (7) -C(O)R¹⁰ (in one example R¹⁰ is a 4 to 6 membered heterocycloalkyl ring, in another example R¹⁰ is a 4 to 6 membered heterocycloalkyl ring comprising one nitrogen atom, and in another example R¹⁰ is a 4 to 6 membered heterocycloalkyl ring comprising one nitrogen atom wherein said ring is bound to the carbonyl moiety (-C(O)-) through the ring nitrogen),
   (8) -(C(R³⁰)₂)ₙ-NR³²-C(O)-R¹⁰ (e.g., -(CH₂)ₙ-NH-C(O)-R¹⁰, for example wherein n is 1), wherein in one example n is 1, each R³⁰ is H, R³² is H, and R¹⁰ is selected from the group consisting of: cycloalkyl (e.g., cyclopropyl) and alkyl (e.g., methyl and i-propyl), and wherein in another example n is 1, each R³⁰ is H, R³² is H, and R¹⁰ is selected from the group consisting of: methyl, i-propyl and cyclopropyl,
   (9) -(C(R³⁰)₂)ₙ-NR³²-S(O)ₜ-R¹⁰ (e.g., -(CH₂)ₙ-NH-S(O)ₜ-R¹⁰, for example wherein n is 1 and t is 2) wherein in one example n is 1, each R³⁰ is H, R³² is H, t is 2, and R¹⁰ is selected from the group consisting of: cycloalkyl (e.g., cyclopropyl) and alkyl (e.g., methyl and i-propyl), and wherein in another example n is 1, each R³⁰ is H, R³² is H, t is 2, R¹⁰ is selected from the group consisting of: methyl, i-propyl and cyclopropyl, and wherein in another example n is 1, each R³⁰ is H, R³² is H, t is 2, and R¹⁰ is methyl,
   (10) -(C(R³⁰)₂)ₙ-NR³²-C(O)-N(R³²)-R¹⁰ (e.g., -(CH₂)ₙ-NH-C(O)-NH-R¹⁰, for example wherein n is 1) wherein in one example n is 1, each R³⁰ is H, each R³² is H, and R¹⁰ is alkyl (e.g., methyl and i-propyl), and wherein in another example n is 1, each R³⁰ is H, each R³² is H, and R¹⁰ is selected from the group consisting of: methyl and i-propyl,
   (11) 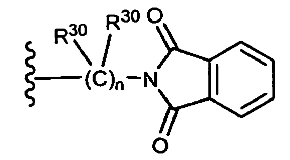 wherein in one example n is 1 and each R³⁰ is H, i.e., a moiety of the formula: 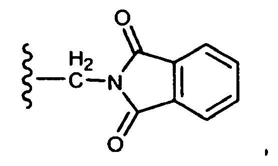
   (12) -CF₃,
   (13) -C(O)OR¹⁰ wherein in one example R¹⁰ is selected from the group consisting of: H, alkyl (e.g., methyl and ispropyl) and cyclopropyl (e.g., cyclopropyl), and wherein in another example R¹⁰ is selected from the group consisting of: H and alkyl, and wherein in another example R¹⁰ is selected from the group consiting of: H and methyl,
   (14) -(C(R³⁰)₂)ₙR¹³ (e.g., -(CH₂)ₙR¹³) wherein in one example n is 1, each R³⁰ is H, and R¹³ is selected from the group consisting of: -OH and -N(R¹⁰)₂, wherein each R¹⁰ is independently selected, and wherein in another example n is 1, each R³⁰ is H, and R¹³ is selected from the group consisting of: -OH and -N(R¹⁰)₂, and each R¹⁰ is H (i.e., R¹³ is -OH or -NH₂),
   (15) alkenyl (e.g., -CH=CHCH₃),
   (16) -NR³²-C(O)-R¹⁴ (e.g., -NH-C(O)-R¹⁴) wherein in one example R³² is H and R¹⁴ is selected from the group consisting of: cycloalkyl (e.g., cyclopropyl), alkyl (e.g., methyl and propyl), aryl (e.g., phenyl), amino (i.e., -NH₂), and heteroaryl (e.g., pyridyl, such as, for example 2- pyridyl, 3-pyridyl, 4-pyridyl, pyrazolyl and imidazolyl), and wherein in another example R³² is H and R¹⁴ is selected from the group consisting of: cyclopropyl, methyl, propyl, phenyl, and amino,
   (17) 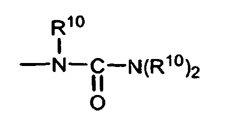 wherein each R¹⁰ is independently selected, for example:
      (a) in one example moiety (20) is: 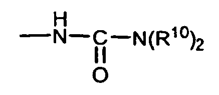 wherein each R¹⁰ is independently selected,
      (b) in another example moiety (20) is: 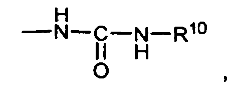 and
      (c) in another example moiety (20) is: 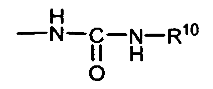 wherein R¹⁰ is selected from the group consisting of: aryl (e.g., phenyl) and alkyl (e.g., ethyl, and preferably R¹⁰ is phenyl or ethyl,
   (18) 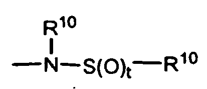 wherein each R¹⁰ is independently selected, and wherein in one example each R¹⁰ is independently selected and t is 2, and wherein in another example moiety (18) is -NH-S(O)ₜ-R¹⁰, and wherein in another example moiety (18) is -NH-S(O)ₜ-R¹⁰ wherein t is 2, and wherein in another example moiety (18) is -NH-S(O)ₜ-R¹⁰. t is 2, and R¹⁰ is alkyl (e.g., methyl),
   (19) 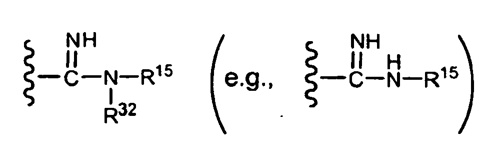 (also written as -C(NH)N(R¹⁵)R³² and -C(NH)NH(R¹⁵), respectively), wherein in one example R¹⁵ is -OH, and in another example R³² is H and R¹⁵ is -OH,
   (20) -C(O)-NR³²-(C(R³⁰)₂)ₚ-OR¹⁰ (e.g., -C(O)-NH-(CH₂)ₚ-OR¹⁰, and, for example, -C(O)-NH-(CH₂)ₚ-OR¹⁰ wherein p is 2) wherein:
      (a) in one example p is 2,
      (b) in another example R³² is H,
      (c) in another example R¹⁰ is selected from the group consisting of: H and alkyl (e.g., methyl),
      (d) in another example R¹⁰ is selected from the group consisting of: H and alkyl (e.g., methyl), and R³² is H,
      (e) in another example R¹⁰ is selected from the group consisting of: H and alkyl (e.g., methyl), R³² is H, an p is 2,
      (f) in another example R³² is H, each R³⁰ is H, and R¹⁰ is alkyl,
      (g) in another example R³² is H, each R³⁰ is H, and R¹⁰ is methyl,
      (h) in another example R³² is H, each R³⁰ is H, p is 2 and R¹⁰ is alkyl, and
      (i) in another example R³² is H, each R³⁰ is H, p is 2 and R¹⁰ is methyl,
   (21) -C(O)N(R¹⁰)₂ wherein each R¹⁰ is independently selected, and preferably each R¹⁰ is independently selected from the group consisting of: (a) H, (b) alkyl (e.g., methyl, butyl, and i-propyl), (c) heteroaryl (e.g., pyridyl), (d) aryl (e.g., phenyl), and (e) cycloalkyl (e.g., cyclopropyl), wherein for example, each R¹⁰ is selected from the group consisting of: H, methyl, butyl, i-propyl, pyridyl, phenyl and cyclopropyl, wherein, for example, said -C(O)N(R¹⁰)₂ moiety is selected from the group consisting of: -C(O)NH₂, -C(O)NH(CH₃), -C(O)NH(CH)(CH₃)₂ (i.e., -C(O)NH(i-propyl)), -C(O)NH(C₄H₉), -C(O)NH(C₆H₅) (i.e., -C(O)NH(phenyl)), -C(O)NH(C₃H₅) (i.e., -C(O)NH(cyclopropyl), and -C(O)NH(C₅H₄N) (i.e., -C(O)NH(pyridyl), such as 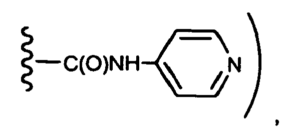
   (22) -C(O)-NR³²-C(R¹⁸)₃ (e.g., -C(O)-NH-C(R¹⁸)₃) wherein each R¹⁸ is independently selected from the group consisting of: R¹⁰ and -C(O)OR¹⁹, and R¹⁹ is selected from the group consisting of:alkyl (e.g., methyl) and substituted arylalkyl (e.g., -CH₂C₆H₄OH (i.e.,hydroxybenzyl) such as, for example, -p-CH₂C₆H₄OH (i.e., p-OHbenzyl), and wherein:
      (a) in one example R¹⁸ and R¹⁹ are as defined above with the proviso that at least one R¹⁸ substitutent is other than H (e.g., in one example one R¹⁸ is H and the remaining two R¹⁸ groups are other than H, and in another example two R¹⁸ substituents are H and the remaining R¹⁸ substituent is other than H),
      (b) in another example R¹⁸ is selected from the group consisting of: H, aryl (e.g., phenyl), substituted aryl (e.g., substituted phenyl, such as, for example halophenyl-, such as, for example, flurophenyl (e.g., o-F-phenyl)), and -C(O)OR¹⁹,
      (c) in another example R¹⁸ is selected from the group consisting of: H, phenyl, flurophenyl (e.g., o-F-phenyl), -C(O)OCH₃, -C(O)OCH₂C₆H₄OH (i.e., -C(O)O(OHbenzyl), such as, -C(O)O(p-OHbenzyl)),
      (d) in another example R¹⁸ is selected from the group consisting of: H, aryl (e.g., phenyl), substituted aryl (e.g., substituted phenyl, such as, for example halophenyl-, such as, for example, flurophenyl (e.g., o-F-phenyl)), and -C(O)OR¹⁹, provided that at least one R¹⁸ substitutent is other than H (e.g., in one example one R¹⁸ is H and the remaining two R¹⁸ groups are other than H, and in another example two R¹⁸ substituents are H and the remaining R¹⁸ substituent is other than H),
      (e) in another example R¹⁸ is selected from the group consisting of: H, phenyl, flurophenyl (e.g., o-F-phenyl), -C(O)OCH₃, -C(O)OCH₂C₆H₄OH (i.e., -C(O)O(OHbenzyl), such as, -C(O)O(p-OHbenzyl)), provided that at least one R¹⁸ substitutent is other than H (e.g., in one example one R¹⁸ is H and the remaining two R¹⁸ groups are other than H, and in another example two R¹⁸ substituents are H and the remaining R¹⁸ substituent is other than H),
      (f) in another example R³² is H, and each R¹⁸ is independently selected from the group consisting of: R¹⁰ and -C(O)OR¹⁹, and R¹⁹ is selected from the group consisting of:alkyl (e.g., methyl) and substituted arylalkyl (e.g., -CH₂C₆H₄OH (i.e.,hydroxybenzyl) such as, for example, -p-CH₂C₆H₄OH (i.e., p-OHbenzyl),
      (g) in another example R³² is H and R¹⁸ and R¹⁹ are as defined in paragraph (a),
      (h) in another example R³² is H and R¹⁸ and R¹⁹ are as defined in paragraph (b),
      (i) in another example R³² is H and R¹⁸ and R¹⁹ are as defined in paragraph (c),
      (j) in another example R³² is H and R¹⁸ and R¹⁹ are as defined in paragraph (d),
      (k) in another example R³² is H and R¹⁸ and R¹⁹ are as defined in paragraph (e), and
      (l) in another example R³² is H and R¹⁸ and R¹⁹ are as defined in paragraph (f),
   (23) -C(O)-NR³²-(C(R³⁰)₂)ₙC(O)-N(R¹⁰)₂ (e.g., -C(O)-NH-(CH₂)ₙ-C(O)-NH₂), and wherein:
      in one example R³² is H,
      in another example each R³⁰ is H,
      in another example n is 1,
      in another example n is 1 and R³² is H,
      in another example each R¹⁰ is H,
      in another example R³² is H and each R³⁰ is H,
      in another example R³² is H, each R³⁰ is H and n is 1,
      in another example R³² is H, each R³⁰ is H, n is 1, and each R¹⁰ is H,
      in another example R³² is H, n is 1, each R³⁰ is independently selected from the group consisting of: H and alkyl, and each R¹⁰ is independently selected from the group consisting of: H and alkyl, and
      in another example R³² is H, n is 1, and each R³⁰ is independently selected from the group consisting of: H, methyl, ethyl and i-propyl (or each R³⁰ is independently selected from the group consisting of H and i-propyl, or one R³⁰ is i-propyl and the other R³⁰ is H), and each R¹⁰ is independently selected from the group consisting of: H methyl, ethyl and i-propyl (or each R¹⁰ is H),
   (24) heterocycloalkenyl, such as, for example: 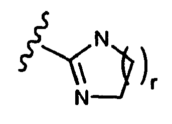 wherein r is 1 to 3, and wherein in one example r is 1, i.e., in one example the heterocycloalkenyl is dihydroimidazolyl, such as, for example: 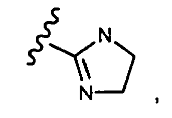
   (25) 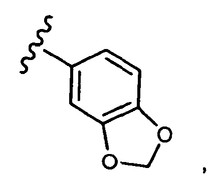 and
   (26) arylalkenyl- (aralkenyl-), for example, aryl(C₂ to C₆)alkenyl-, such as for example, -CH=CH-phenyl;
      R² is selected from the group consisting of:
      (1) H,
      (2) -CN,
      (3) halo (e.g., F),
      (4) alkyl (e.g., C₁ to C₆ alkyl, such as, for example, methyl, ethyl and propyl),
      (5) substituted alkyl (e.g., substituted C₁ to C₆ alkyl, such as, for example, substituted methyl and substituted ethyl) wherein said substituted alkyl is substituted with 1 to 3 substitutents (e.g., 1 substituent) selected from the group consisting of: (a) -OH, (b) -O-alkyl (e.g., -O-(C₁-C₃alkyl), (c) -O-alkyl (e.g., -O-(C₁-C₃alkyl)) substituted with 1 to 3 F atoms (examples of said -O substituted alkyl portion include, but are not limited to, -OCHF2 and -OCF₃), and (d) -N(R⁴⁰)₂ wherein each R⁴⁰ is independently selected from the group consisting of: (i) H, (ii) C₁-C₃ alkyl (e.g., methyl), (iii) -CF₃, and (e) halo (for example F, Cl, and Br, and also for example F, examples of a halo substituted alky group include, but are not limited to, -CHF₂) (examples of said substituted alkyl groups described in (5) include but are not limited to-CH(OH)CH₃),
      (6) alkynyl (e.g., ethynyl),
      (7) alkenyl (e.g., -CH₂-CH=CH₂),
      (8) -(CH₂)ₘR¹¹,
      (9) -N(R²⁶)₂,
      (10) -OR²³ (e.g., -OH, -OCH₃ and -O-phenyl),
      (11) -N(R²⁶)C(O)R⁴² wherein in one example R²⁶ is H or C₁ to C₆ alkyl (e.g., methyl) and R⁴² is alkyl (e.g., methyl), and in another example -N(R²⁶)C(O)R⁴² is -NHC(O)CH₃,
      (12) cycloalkyl (e.g., C₃ to C₆ cycloalkyl, such as, for example, cyclopropyl and cyclohexyl),
      (13) cycloalkylalkyl (e.g., C₃ to C₆ cycloalkyl-(C₁ to C₃)alkyl-, such as, for example, cyclopropyl-CH₂- and cyclohexyl-CH₂-),
      (14) 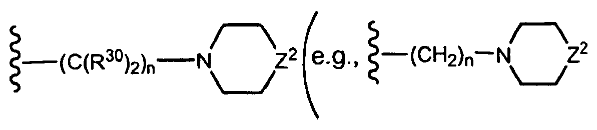 such as 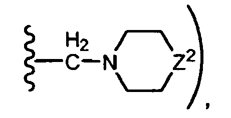
      (15) -O-(substituted alkyl) wherein said substituted alkyl is substituted with 1 to 3 F atoms (examples of said -O-(substituted alkyl) moiety include, but are not limited to, -OCHF₂ and -OCF₃),
      (16) -S(O)ₜ-alkyl, such as, for example, (a) -S-alkyl (i.e., t is 0) such as, for example, -S-CH₃, and (b) -S(O)₂-alkyl (i.e., t is 2) such as, for example, -S(O)₂CH₃,
      (17) -C(O)-alkyl (e.g., -C(O)CH₃),
      (18) 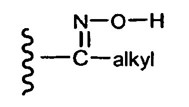 wherein methyl is an example of said alkyl moiety,
      (19) 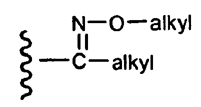 wherein each alkyl is independently selected, examples of this moiety include, but are not limited to: 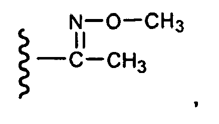
      (20) 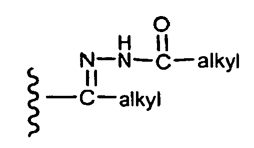 which each alkyl is independently selected, examples of this moiety include, but are not limited to, 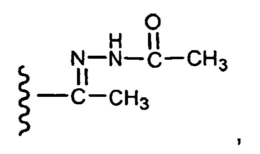
      (21) 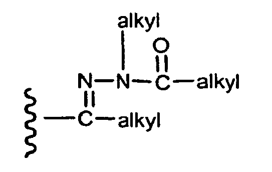 wherein each alkyl is independently selected,
      (22) -N(R⁴⁸)-C(O)-R⁴⁸ wherein each R⁴⁸ is independently selected from the group consisting of: H and alkyl (e.g., C₁ to C₆ alkyl, such as, for example, methyl), and wherein examples of this moiety include, but are not limited to, -NH-C(O)-H, and -N(CH₃)-C(O)H, and
      (23) -C(O)-alkyl, such as, for example, -C(O)-(C₁-C₆ alkyl), such as, for example, -C(O)CH₃; and
         wherein:
         (a) in one example said (14) moiety is 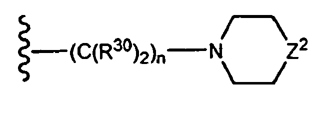 and n is 1,
         (b) in another example said (14) moiety is 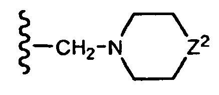 (i.e., n is 1, and each R³⁰ is H),
         (c) in another example Z² is -NH- in (a),
         (d) in another example Z² is -NH- in (b),
         (e) in another example Z² is -O- in (a),
         (f) in another example Z² is -O- in (b),
         (g) in another example Z² is -CH₂- in (a),
         (h) in another example Z² is -CH₂- in (b),
         (i) in another example R² is -(CH₂)ₘR¹¹ and m is 1,
         (j) in another example R² is -N(R²⁶)₂,
         (k) in another example R² is -N(R²⁶)₂, and each R²⁶ is H (i.e., R² is -NH₂),
         (l) in another example R² is -OR²³, and
         (m) in another example R² is -OH (i.e., R²³ is H);
         each R³, R⁴, R⁵, R⁶ and R⁷ is independently selected from the group consisting of:
         (1) H,
         (2) alkenyl (e.g., -CH₂CH=CH₂),
         (3) substituted alkenyl,
         (4) alkyl,
         (5) substituted alkyl,
         (6) cycloalkyl,
         (7) substituted cycloalkyl,
         (8) cycloalkylalkyl-,
         (9) substituted cycloalkylalkyl-,
         (10) heterocycloalkyl,
         (11) substituted heterocycloalkyl,
         (12) heterocycloalkylalkyl-,
         (13) substituted heterocycloalkylalkyl-,
         (14) -C(O)R¹⁰ wherein in one example R¹⁰ is selected from the group consisting of: alkyl (e.g., C₁ to C₆, e.g., methyl),
         (15) arylheteroaryl- (e.g., phenylthiadiazolyl-),
         (16) substituted arylheteroaryl- (e.g., substituted phenylthiadiazolyl-),
         (17) heteroarylaryl-, such as, for example, pyrimidinylphenyl-, pyrazinylphenyl-, pyridinylphenyl- (i.e., pyridylphenyl-), furanylphenyl-, thienylphenyl-, and thiazolylphenyl-,
         (18) substituted heteroarylaryl-, such as, for example, substituted pyrimidinylphenyl-, substituted pyrazinylphenyl-, substituted pyridinylphenyl- (i.e., substituted pyridylphenyl-), substituted furanylphenyl-, substituted thienylphenyl-, substituted thiazolylphenyl-, and substituted pyrimidinylphenyl,
         (19) aryl (e.g., phenyl),
         (20) substituted aryl (e.g., substituted phenyl),
         (21) heteroaryl (e.g., thiazolyl, thienyl, pyridyl, and pyrimidinyl),
         (22) substituted heteroaryl (e.g., substituted thiazolyl, substituted pyridyl and substituted pyrimidinyl), examples of substituted heteroaryl groups include, for example bromothiazolyl-, bromopyrimidinyl-, fluoropyrimidinyl-, and ethenylpyrimidinyl-,
         (23) heteroarylheteroaryl- (e.g., pyrimidinylpyridyl-, and pyrimidinylthiazolyl-),
         (24) substituted heteroarylheteroaryl- (e.g., substituted pyrimidinyl- pyridyl-),
         (25) arylaminoheteroaryl- (e.g., phenyl-NH-oxadiazolyl-),
         (26) substituted arylaminoheteroaryl- (e.g., substituted phenyl-NH-oxadiazolyl-),
         (27) arylalkynyl- (e.g., aryl(C₂ to C₄)alkynyl such as, for example phenylethynyl-),
         (28) substituted arylalkynyl- (e.g., substituted aryl(C₂ to C₄)alkynyl-, such as, for example, substituted phenylethynyl-),
         (29) heteroarylalkynyl- (e.g., heteroaryl(C₂ to C₄)alkynyl-, such as, for example, pyrimidinylethynyl-),
         (30) substituted heteroarylalkynyl- (e.g., substituted heteroaryl(C₂ to C₄)alkynyl-, such as, for example substituted pyrimidinylethynyl-),
         wherein said R³, R⁴, R⁵, R⁶ and R⁷ substituted groups (7), (9), (11), (13), (16), (18), (20), (22), (24), (26), (28) and (30) are substituted with 1 to 3 substituents independently selected from the group consisting of: -NH₂, alkyl (e.g., C₁ to C₆ alkyl, e.g., methyl, ethyl, and i-propyl), alkenyl (e.g., C₂ to C₆ alkenyl, such as, for example -CH=CH₂), halo (e.g., F, Cl and Br, and in another example F), -C(O)-NH-R²⁸ (e.g., -C(O)-NH-CH₃), -C(O)OR²⁸ (e.g., -C(O)OC₂H₅), and -C(O)R²⁸ (e.g., -C(O)CH₃),
         wherein said R³, R⁴, R⁵, R⁶ and R⁷ substituted groups (3) and (5) are substituted with 1 to 3 substituents independently selected from the group consisting of: -NH₂, halo (e.g., F, Cl and Br, and in another example F), -C(O)-NH-R²⁸ (e.g., -C(O)-NH-CH₃), -C(O)OR²⁸ (e.g., -C(O)OC₂H₅), and -C(O)R²⁸ (e.g., -C(O)CH₃), and
         wherein:
         in one example said substituted heteroarylaryl (moiety (18) above) is substituted with 1 to 3 substituents independently selected from the group consisting of: -NH₂, alkyl (e.g., C₁ to C₆ alkyl, e.g., methyl), halo (e.g., F, Cl and Br, such as, for example F),
         in another example said substituted aryl (moiety (20) above) is substituted with 1 to 3 substituents independently selected from the group consisting of halo (e.g., F, Cl and Br), -C(O)-NH-R²⁸ (e.g., -C(O)-NH-CH₃), -C(O)OR²⁸ (e.g., -C(O)O-C₂H₅), and -C(O)R²⁸ (e.g., -C(O)CH₃), and
         in another example said substituted heteroaryl (moiety (22) above) is substituted with 1 to 3 substitutents selected from the group consisting of: halo (e.g., Br, F, and Cl), alkenyl (e.g., C₂ to C₆ alkenyl, such as, for example, -CH=CH₂);
R^{5A} is selected from the group consisting of: halo (for example, F, Cl, and Br, and in another example F), -OH, and -O-alkyl (such as, for example, -O-(C₁ to C₆ alkyl), also, for example, -O-(C₁ to C₃ alkyl), also for example, -O-(C₁ to C₂ alkyl), and in one example -O-CH₃);
R⁸ is selected from the group consisting of: H, -OH, -N(R¹⁰)₂ (e.g., -NH₂), -NR¹⁰C(O)R¹² (e.g., -NHC(O)CH₃), and alkyl (e.g., methyl);
each R⁹ is independently selected from the group consisting of:halogen, -CN, -NO₂ -OR¹⁰, -SR¹⁰, -N(R¹⁰)₂, and R¹⁰;
each R¹⁰ is independently selected from the group consisting of: H, alkyl, aryl, arylalkyl, heteroaryl, heteroarylalkyl, cycloalkyl, cycloalkylalkyl, heterocycloalkyl, heterocycloalkylalkyl, alkylheteroaryl-, alkylaryl-, substituted alkyl, substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl-, substituted alkylaryl-, heterocycloalkenyl 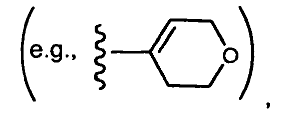 and substituted heterocycloalkenyl, and wherein:
   said R¹⁰ substituted alkyl is substituted with 1 to 3 substituents independently selected from the group consisting of: -NH₂, -NHR²⁰, -NO₂, -CN, -OR²⁶, halo (e.g., F, Cl and Br, and in another example F), -C(O)-NH-R²⁶ (e.g., -C(O)-NH-CH₃, i.e., R²⁶ is alkyl, such as methyl), -C(O)OR²⁶ (e.g., -C(O)OC₂H₅, i.e., R²⁶ is alkyl, such as ethyl), and -C(O)R²⁶ (e.g., -C(O)CH₃, i.e., R²⁶ is alkyl, such as methyl), and
   said R¹⁰ substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl- are substituted with 1 to 3 substituents independently selected from the group consisting of: (1) -NH₂, (2) -NO₂, (3) -CN, (4) -OH, (5) -OR²⁰, (6) -OCF₃, (7) alkyl (e.g., C₁ to C₆ alkyl) substituted with 1 to 3 independently selected halo atoms (e.g., F, Cl and Br), examples of the substituted alkyl include, but are not limited to, -CF₃, -CHF₂ and-CH₂F, (8) -C(O)R³⁸ (e.g., R³⁸ is H or alkyl (e.g., C₁ to C₆ alkyl, such as, for example, methyl or ethyl), for example, R³⁸ is alkyl (e.g., methyl), thus, an example of -C(O)R³⁸ is -C(O)CH₃), (9) alkyl (e.g., C₁ to C₆ alkyl, e.g., methyl, ethyl, and i-propyl), (10) alkenyl (e.g., C₂ to C₆ alkenyl, such as, for example -CH=CH₂), (11) halo (e.g., F, Cl and Br, and in another example F), (12) -C(O)-NH-R²⁶ (e.g., -C(O)-NH-CH₃), (13) -C(O)OR³⁸ (e.g., R³⁸ is H or alkyl (e.g., C₁ to C₆ alkyl, such as, for example, methyl or ethyl), for example, R³⁸ is alkyl (e.g., methyl or ethyl), thus, for example, -C(O)OR³⁸ is -C(O)OC₂H₅), (14) -C(O)-NR³²-(C(R³⁰)₂)ₙ-N(R³⁸)₂ (e.g., -C(O)-NH-(CH₂)ₙ-N(R³⁸)₂) (wherein (a) in one example R³² is H, (b) in another example each R³⁰ is H, (c) in another example n is 2, (d) in another example each R³⁸ is independently selected, (e) in another example each R³⁸ is independently selected from the group consisting of: H and alkyl (e.g., methyl), (f) in another example R³² is H, each R³⁰ is H, and each R³⁸ is independently selected, (g) in another example R³² is H, each R³⁰ is H, and each R³⁸ is independently selected from the group consisting of: H and alkyl (e.g., methyl), (15) -S(O)ₜR³⁸ (wherein in one example t is 2, and in another example R³⁸ is alkyl (e.g., methyl), and in another example t is 2 and R³⁸ is alkyl (e.g., methyl)), (16) -C(O)-NR³²-R³⁸ (e.g., -C(O)-NR³²-R³⁸) (wherein one example R³² is H, in another example R³⁸ is alkyl (e.g., propyl), and in another example R³² is H and R³⁸ is alkyl (e.g., propyl)), (17) -NR³²-C(O)-R³⁸ (e.g., -NH-C(O)-R³⁸) (wherein in one example R³² is H, in another example R³⁸ is alkyl (e.g., methyl), and in another example R³² is H and R³⁸ is alkyl (e.g., methyl)),
      (18) 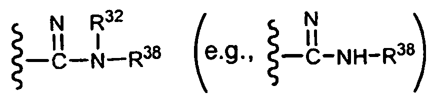 (wherein in one example R³² is H, in another example R³⁸ is H, and in another example R³² is H and R³⁸ is H), (19) -NHR²⁰ (e.g., -NHCH₃, -NHC₂H₅), and (20) cycloalkyl (e.g., C₃ to C₆ cycloalkyl, such as, for example, cyclopropyl);
   R¹¹ is selected from the group consisting of: F, -OH, -CN, -OR¹⁰, -NHNR¹R¹⁰, -SR¹⁰ and heteroaryl (e.g., triazolyl, such as, for example, 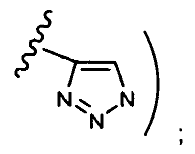
   R¹² is selected from the group consisting of: alkyl, aryl, heteroaryl, cycloalkyl, cycloalkylalkyl, heterocycloalkyl and heterocycloalkylalkyl;
   R¹⁴ is selected from the group consisting of: alkyl, aryl, heteroaryl, cycloalkyl, cycloalkylalkyl-, heterocycloalkyl, alkylheterocycloalkyl, heterocycloalkylalkyl-, alkylheteroaryl- and alkylaryl-;
   R¹⁵ is selected from the group consisting of: H, -OH, alkyl, aryl, heteroaryl, cycloalkyl, cycloalkylalkyl-, heterocycloalkyl and heterocycloalkylalkyl-, alkylheteroaryl- and alkylaryl-;.
   R²⁰ represents alkyl (e.g., C₁ to C₆ alkyl, such as, for example, methyl or ethyl);
   R²³ is selected from the group consisting of: H, alkyl (e.g., C₁ to C₆ alkyl, such as, for example, methyl and i-propyl), aryl (e.g., phenyl), cycloalkyl (e.g., C₃ to C₆ cycloalkyl, such as, for example, cyclopropyl and cyclohexyl), and cycloalkylalkyl-(e.g., C₃ to C₆ cycloalkylalkyl-, such as -(CH₂)ₙ-cycloalkyl, such as -(CH₂)ₙ-(C₃ to C₆)cycloalkyl, wherein each H of each -(CH₂)ₙ- moiety can independently be substituted with an alkyl group (e.g., C₁ to C₆ alkyl, such as, for example, methyl), and wherein in one example n is 1 and the -CH₂- moiety is not substituted, that is, -CH₂-cycloalkyl, such as, -CH₂-cyclopropyl, is an example of said cycloalkylalkyl- moiety);
   each R²⁶ is independently selected from the group consisting of: H and alkyl (e.g., C₁ to C₆ alkyl, such as, for example, methyl and ethyl);
   R²⁸ is alkyl (e.g., C₁ to C₆ alkyl, such as, for example, methyl or ethyl);
   each R³⁰ is independently selected from the group consisting of: H, alkyl (e.g., C₁ to C₆ alkyl, such as, for example methyl, ethyl and i-propyl), and F, and wherein in one example each R³⁰ is H;
   each R³² is independently selected from the group consisting of: H and alkyl (e.g., C₁ to C₆ alkyl, such as, for example methyl, ethyl and propyl), and wherein each R³² is generally H;
   each R³⁵ is independently selected from the group consisting of: H and C₁ to C₆ alkyl (e.g., methyl, ethyl, i-propyl, and propyl), and wherein in one example both R³⁵ substitutents are the same or different alkyl groups (e.g., both R³⁵ groups are the same alkyl group, such as methyl), and in another example one R³⁵ group is H and the other R³⁵ group is alkyl, such as methyl), and in another example each R³⁵ is preferably H;
   R³⁶ is selected from the group consisting of: H, alkyl (e.g., C₁ to C₆ alkyl, such as, for example, methyl, ethyl and propyl), and -O-alkyl (e.g., -O-(C₁ to C₆) alkyl, such as, for example, -O-(C₁ to C₂) alkyl, such as, for example, -OCH₃), and preferably R³⁶ is selected from the group consisting of H and methyl, and more preferably R³⁶ is H;
   each R³⁸ is independently selected from the group consisting of: H, alkyl, aryl, arylalkyl, heteroaryl, heteroarylalkyl, cycloalkyl, cycloalkylalkyl, heterocycloalkyl, heterocycloalkylalkyl, alkylheteroaryl-, alkylaryl-, substituted alkyl, substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl-, and wherein:
      said R³⁸ substituted alkyl is substituted with 1 to 3 substituents independently selected from the group consisting of: -NH₂, -NO₂ -CN, -OR²⁶, halo (e.g., F, Cl and Br, and in another example F), -C(O)-NH-R²¹ (e.g., -C(O)-NH-CH₃), -C(O)OR²⁸ (e.g., -C(O)OC₂H₅), and -C(O)R²⁸ (e.g., -C(O)CH₃), and
      said R³⁸ substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl- are substituted with 1 to 3 substituents independently selected from the group consisting of: (1) -NH₂, (2) -NO₂, (3) -CN, (4) -OH, (5) -OR²¹, (6) -OCF₃, (7) -CF₃, (8) -C(O)R²⁶ (e.g., R²⁶ is H or C₁ to C₆ alkyl, such as, for example, methyl or ethyl, for example, R²⁶ is alkyl (e.g., methyl), thus, an example of -C(O)R¹⁶ is -C(O)CH₃), (9) alkyl (e.g., C₁ to C₆ alkyl, e.g., methyl, ethyl, and i-propyl), (10) alkenyl (e.g., C₂ to C₆ alkenyl, such as, for example -CH=CH₂), (11) halo (e.g., F, C₁ and Br, and in another example F), (12) -C(O)-NH-R²⁶ (e.g., -C(O)-NH-CH₃), (13) -C(O)OR²⁶ (e.g., R²⁶ is H or e.g., C₁ to C₆ alkyl, such as, for example, methyl or ethyl, for example, R²⁶ is alkyl (e.g., methyl or ethyl), thus, for example, -C(O)OR²⁶ is -C(O)OC₂H₅), (14) -C(O)-NR³²-(C(R³⁰)₂)ₙ-N(R²⁶)₂ (e.g., -C(O)-NH-(CH₂)ₙ-N(R²⁶)₂) (wherein (a) in one example R³² is H, (b) in another example each R³⁰ is H, (c) in another example n is 2, (d) in another example each R²⁶ is independently selected, (e) in another example each R²⁶ is independently selected from the group consisting of: H and methyl), (f) in another example R³² is H, each R³⁰ is H, and each R²⁶ is independently selected, (g) in another example R³² is H, each R³⁰ is H, and each R²⁶ is independently selected from the group consisting of: H and methyl), (15) -S(O)ₜR²⁶ (wherein in one example t is 2, and in another example R²⁶ is methyl, and in another example t is 2 and R²⁶ is methyl), (16) -C(O)N(R³²)(R²⁶) (wherein in one example R³² is H, in another example R²⁶ is alkyl (e.g., propyl), and in another example R³² is H and R²⁶ is alkyl (e.g., propyl)), (17) -NR³²C(O)R²⁶ (e.g., -NHC(O)R²⁶) (wherein in one example R³² is H, in another example R²⁶ is alkyl (e.g., methyl), and in another example R³² is H and R²⁶ is alkyl (e.g., methyl)), (18) 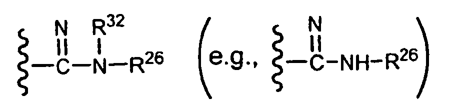 (wherein in one example R³² is H, in another example R²⁶ is H, and in another example R³² is H and R²⁶ is H); and (19) -NHR²⁰;
      R⁴² is selected from the group consisting of: alkyl (e.g., C₁ to C₆ alkyl, such as, for example -CH₃), aryl (e.g., phenyl), heteroaryl (e.g., thiazolyl and pyridyl), and cycloalkyl (e.g., C₃ to C₆ cycloalkyl, such as, for example, cyclopropyl);
      R⁴⁴ is selected from the group consisting of: H, alkyl (e.g., C₁ to C₆ alkyl, such as, for example, C₁ to C₃ alkyl, such as, for example, methyl, ethyl and i-propyl), cycloalkyl (e.g., C₃ to C₆ cycloalkyl, such as, for example, cyclopropyl and cyclohexyl), and cycloalkylalkyl (e.g., (C₃ to C₆)cycloalky(C₁ to C₆)alkyl, such as, for example, (C₃ to C₆)cycloalky(C₁ to C₃)alkyl, such as, for example, (C₃ to C₆)cycloalky-methyl-, such as, for example, cyclopropyl-methyl- and cyclohexyl-methyl-), and in one example, R⁴⁴ is H; and
      Each R⁴⁶ is independently selected from the group consisting of: H, alkyl (e.g., C₁ to C₆ alkyl, such as, for example, C₁ to C₃ alkyl, such as, for example, methyl, ethyl and i-propyl), cycloalkyl (e.g., C₃ to C₆ cycloalkyl, such as, for example, cyclopropyl and cyclohexyl), and cycloalkylalkyl (e.g., (C₃ to C₆)cycloalky(C₁ to C₆)alkyl, such as, for example, (C₃ to C₆)cycloalky(C₁ to C₃)alkyl, such as, for example, (C₃ to C₆)cycloalky-methyl-, such as, for example, cyclopropyl-methyl- and cyclohexyl-methyl-), and in one example, each R⁴⁶ is H.

When R¹ is a cycloalkyl group (i.e., R¹ is R¹⁰ wherein R¹⁰ is cycloalkyl), examples of said cycloalkyl group include, but are limited to, cyclopropyl and cyclobutyl.

When R¹ is a heterocycloalkyl group (i.e., R¹ is R¹⁰ wherein R¹⁰ is heterocycloalkyl), examples of said heterocycloalkyl group include, but are limited to, morpholinyl, pyrrolidinyl, piperidinyl and piperazinyl.

When R¹ is a heteroaryl group (i.e., R¹ is R¹⁰ and R¹⁰ is heteroaryl), examples of said heteroaryl group include, but are not limited to,
(a) unsubstituted heteroaryl,
(b) heteroaryl substituted with 1 to 3 substituents independently selected from the group consisting of: -C(O)R³⁸ (e.g., R³⁸ is alkyl such as methyl) ,-NHR²⁰ (e.g., -NHCH₃), -OR²⁰ (e.g., -OCH₃), cycloalkyl (e.g., cyclopropyl) and halo (e.g., Cl),
(c) heteroaryl selected from the group consisting of: pyrrolyl, pyrazolyl, imidazolyl, furanyl, thienyl, thiazolyl, pyridyl, pyridyl N-O, and pyrimidinyl,
(d) heteroaryl selected from the group consisting of: pyrrolyl, pyrazolyl, imidazolyl, furanyl, thienyl, thiazolyl, pyridyl, pyridyl N-O, and pyrimidinyl, wherein said heteroaryl is substituted with 1 to 3 substituents independently selected from the group consisting of: -C(O)R³⁸ (e.g., R³⁸ is alkyl such as methyl) -NHR²⁰ (e.g., -NHCH₃), -OR²⁰ (e.g., -OCH₃), cycloalkyl (e.g., cyclopropyl) and halo (e.g., Cl), and
(e) heteroaryl selected from the group consisting of: thienyl substituted with -C(O)R³⁸ (such as, for example, thienyl substituted with -C(O)CH₃), thiazolyl substituted with -NHR²⁰ such as, for example (thazolyl substituted with-NHCH₃), pyridyl substituted with halo (such as, for example, pyridyl substituted with -Cl), pyridyl substituted with -OR²⁰ (such as, for example, pyridyl substituted with methyl), and pyrimidinyl substituted with -OR²⁰ (such as, for example, pyrimidinyl substituted with -OCH₃).

When R¹ is a heteroarylalkyl group (i.e., R¹ is R¹⁰ and R¹⁰ is heteroarylalkyl), examples of said heteroarylalkyl group include, but are not limited to,
(a) unsubstituted heteroarylalkyl-
(b) heteroarylalkyl- substituted with 1 to 3 substituents independently selected from the group consisting of: -C(O)R³⁸ (e.g., R³⁸ is alkyl such as methyl), -NHR²⁰ (e.g., -NHCH₃), -OR²⁰ (e.g., -OCH₃), and halo (e.g., Cl),
(c) heteroarylalkyl- selected from the group consisting of: pyrrolylalkyl- (e.g., pyrrolylCH₂-), pyrazolylalkyl- (e.g., pyrazolylCH₂-), imidazolylalkyl- (e.g., imdazolyl-CH₂-), furanylalkyl- (e.g., furanylCH₂-), thienylalkyl- (e.g., thienylCH₂-), thiazolylalkyl-(e.g., thiazolylCH₂-), pyridylalkyl- (e.g., pyridylCH₂-), pyridyl N-O alkyl- (e.g., pyridyl(NO)CH₂-), and pyrimidinylalkyl- (e.g., pyrimidinylCH₂-),
(d) heteroarylalkyl- selected from the group consisting of: pyrrolylalkyl- (e.g., pyrrolylCH₂-), pyrazolylalkyl- (e.g., pyrazolylCH₂-), imidazolylalkyl- (e.g., imdazolylCH₂-), furanylalkyl- (e.g., furanylCH₂-), thienylalkyl- (e.g., thienylCH₂-), thiazolylalkyl- (e.g., thiazolylCH₂-), pyridylalkyl- (e.g., pyridylCH₂-), pyridyl N-O alkyl- (e.g., pyridyl(NO)CH₂-), and pyrimidinylalkyl- (e.g., pyrimidinylCH₂-), wherein said heteroaryl is substituted with 1 to 3 substituents independently selected from the group consisting of: -C(O)R³⁸ (e.g., R³⁸ is alkyl such as methyl) ,-NHR²⁰ (e.g., -NHCH₃), -OR²⁰ (e.g., -OCH₃), and halo (e.g., Cl), and
(e) heteroarylalkyl- selected from the group consisting of: thienylalkyl-substituted with a -C(O)R²⁰ group (such as, for example, thienylCH₂- substituted with -C(O)CH₃), thiazolylalkyl- substituted with-NHR²⁰ such as, for example (thazolylCH₂-substituted with-NHCH₃), pyridylalkyl- substituted with halo (such as, for example, pyridylCH₂- substituted with -Cl), pyridylalkyl- substituted with -OR²⁰ (such as, for example, pyridylCH₂- substituted with methyl), and pyrimidinylalky- substituted with-OR²⁰ (such as, for example, pyrimidinylCH₂- substituted with -OCH₃).

When R¹ is an aryl group (i.e., R¹ is R¹⁰ and R¹⁰ is aryl), examples of said aryl group include, but are not limited to, phenyl and naphthyl, and preferably phenyl.

When R¹ is an arylalkyl group (i.e., R¹ is R¹⁰ and R¹⁰ is arylalkyl), examples of said arylalkyl group include, but are not limited to, -(C(R³⁰)₂)ₙphenyl (e.g., -(CH₂)ₙphenyl), wherein in one example said arylalkyl- is -(C(R³⁰)₂)ₙphenyl wherein n is 1, and in another example said arylalkyl- is -(CH₂)ₙphenyl wherein n is 1 (i.e., said arylalkyl- is benzyl).

When R¹ is a substituted arylalkyl group (i.e., R¹ is R¹⁰ and R¹⁰ is a substituted arylalkyl), examples of said substituted arylalkyl group include, but are not limited to, -(C(R³⁰)₂)ₙsubstituted phenyl (e.g., -(CH₂)ₙsubstituted phenyl), wherein in one example said substituted arylalkyl- is -(C(R³⁰)₂)ₙ substituted phenyl wherein n is 1, and in another example said substituted arylalkyl- is -(CH₂)ₙsubstituted phenyl wherein n is 1 (i.e., said substituted arylalkyl- is substituted benzyl), wherein the aryl moiety of said substituted arylalkyl is substituted with 1 to 3 substituents independently selected from the group consisiting of: halo (e.g., F, Cl and Br), -CF₃, and -OR²⁰ (e.g., -OCH₃).

Those skilled in the art will appreciate that when Q¹ is aryl, substituted aryl, heteroaryl or substituted heteroaryl the two carbon atoms common to the two fused rings are not substituted. Thus, there is no R³ and no R⁴ groups in 2.9 when Q¹ is aryl, substituted aryl, heteroaryl or substituted heteroaryl. There is no R³ and no R⁴ groups in 2.10 when Q¹ fused to the R³ and R⁴ positions is aryl, substituted aryl, heteroaryl or substituted heteroaryl. There is no R⁶ and no R⁷ groups in 2.10 when Q¹ fused to the R⁶ and R⁷ positions is aryl, substituted aryl, heteroaryl or substituted heteroaryl. There is no R³ and no R⁴ groups in 2.11 when Q¹ fused to the R³ and R⁴ positions is aryl, substituted aryl, heteroaryl or substituted heteroaryl. There is no R³ and no R⁴ groups in 2.13 when Q¹ fused to the R³ and R⁴ positions is aryl, substituted aryl, heteroaryl or substituted heteroaryl. There is no R³ and no R⁴ groups in 2.14 when Q¹ fused to the R³ and R⁴ positions is aryl, substituted aryl, heteroaryl or substituted heteroaryl. There is no R³ and no R⁴ groups in 2.15 when Q¹ fused to the R³ and R⁴ positions is aryl, substituted aryl, heteroaryl or substituted heteroaryl. There is no R⁶ and no R⁷ groups in 2.15 when Q¹ fused to the R³ and R⁴ positions is aryl, substituted aryl, heteroaryl or substituted heteroaryl.

In one embodiment of the compounds of formula 1.0, z is 1. Thus, in this embodiment the compounds of formula 1.0 have the formula 1.0A1:

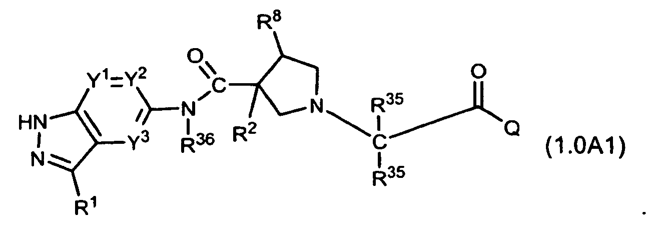

In another embodiment of the compounds of formula 1.0, z is 1 and R³⁶ is H. Thus, in this embodiment the compounds of formula 1.0 have the formula 1.0A:

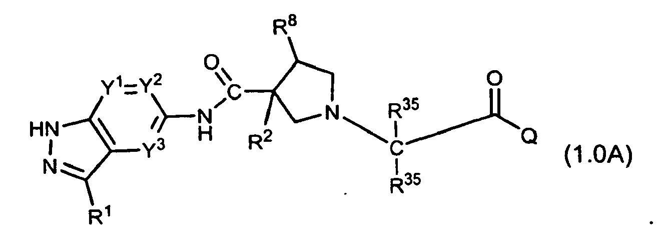

In another embodiment of the compounds of formula 1.0, Z is 1 and and R³⁶ is -OCH₃.

In another embodiment of the compounds of formula 1.0, z is 1, and each R³⁵ is independently selected from the group consisting of: H, methyl, ethyl, i-propyl and propyl (e.g., one R³⁵ is H and the other is methyl, or both R³⁵ substituents are methyl, or preferably both R³⁵ substitutents are H).

In another embodiment of the compounds of formula 1.0, z is 1, each R³⁵ is independently selected from the group consisting of: H, methyl, ethyl, i-propyl and propyl (e.g., one R³⁵ is H and the other is methyl, or both R³⁵ substituents are methyl, or preferably both R³⁵ substitutents are H), and R³⁶ is selected from the group consisting of: H, methyl, ethyl and propyl.

In another embodiment of the compounds of formula 1.0, z is 1, each R³⁵ is independently selected from the group consisting of: H, methyl, ethyl, i-propyl and propyl (e.g., one R³⁵ is H and the other is methyl, or both R³⁵ substituents are methyl, or preferably both R³⁵ substitutents are H), and R³⁶ is selected from the group consisting of: H and methyl.

In another embodiment of the compounds of formula 1.0, z is 1, each R³⁵ is independently selected from the group consisting of: H, methyl, ethyl, i-propyl and propyl (e.g., one R³⁵ is H and the other is methyl, or both R³⁵ substituents are methyl, or preferably both R³⁵ substitutents are H), and R³⁶ is: H.

In another embodiment of the compounds of formula 1.0, each R³⁵ is H. Thus, in this embodiment the compounds of formula 1.0 have the formula 1.0B1:

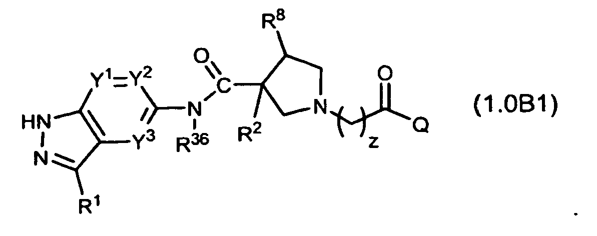

In another embodiment of the compounds of formula 1.0, each R³⁵ is H and R³⁶ is H. Thus, in this embodiment the compounds of formula 1.0 have the formula 1.0B:

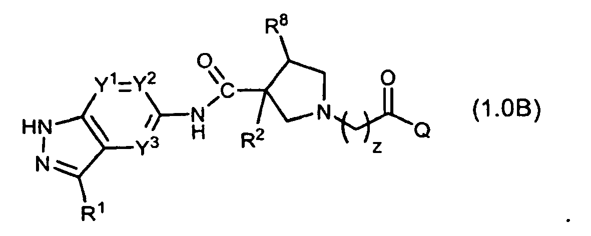

In another embodiment of the compounds of formula 1.0, z is preferably 1 and each R³⁵ is preferably H. Thus, in this embodiment the compounds of formula 1.0 have the formula 1.0C1:

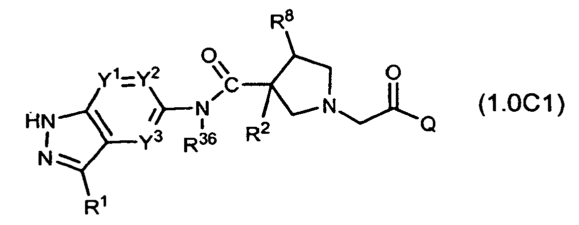

In another embodiment of the compounds of formula 1.0, z is preferably 1, each R³⁵ is preferably H, and R³⁶ is H. Thus, preferably the compounds of formula 1.0 have the formula 1.0C:

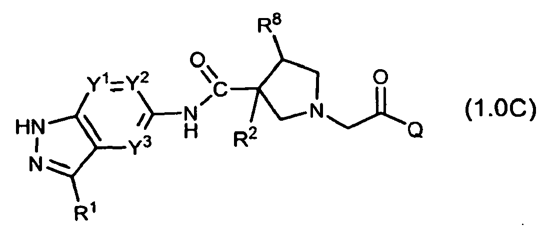

Another embodiment of this invention is directed to compounds of formula 1.0 having the formula 1.1 A:

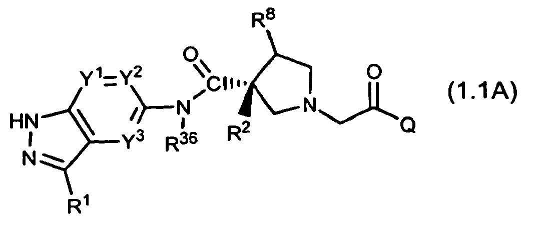

Another embodiment of this invention is directed to compounds of formula 1.0 having the formula 1.1: 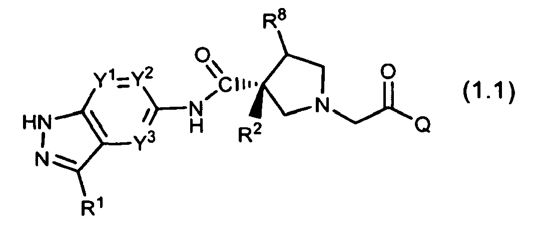 wherein all substituents are as defined for formula 1.0.

Another embodiment of this invention is directed to compounds of formulas 1.0 and 1.1 A wherein Y¹, Y², and Y³ are -CH=. Thus, one embodiment of this invention is directed to compounds of formula 1.2A:

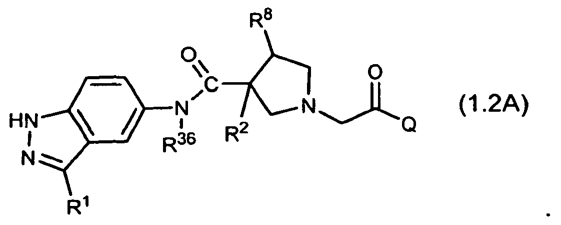

Another embodiment of this invention is directed to compounds of formulas 1.0 and 1.1 wherein Y¹, Y², and Y³ are -CH=. Thus, one embodiment of this invention is directed to compounds of formula 1.2: 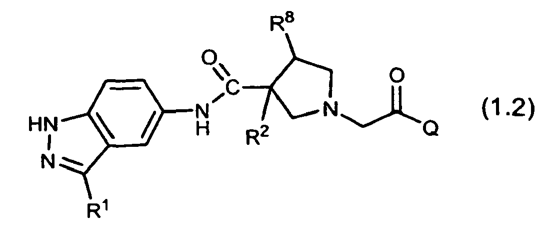 wherein all substitutents are as defined for formula 1.0.

Another embodiment of this invention is directed to compounds of formula 1.0 having the formula 1.3A:

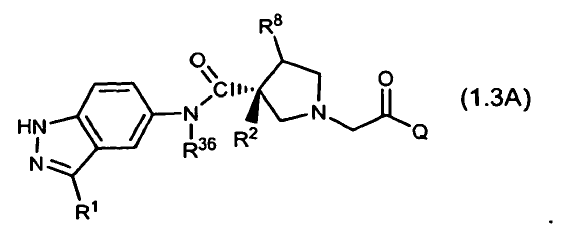

Another embodiment of this invention is directed to compounds of formula 1.0 having the formula 1.3: 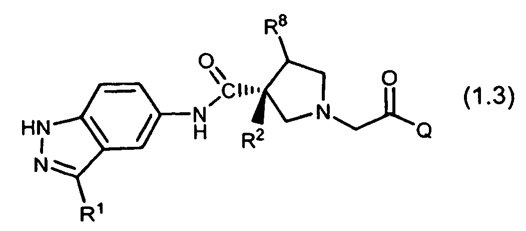 wherein all substituents are as defined for formula 1.0.

Examples of Q include, but are not limited to: moieties 2.1, 2.2, 2.3., 2.4, 2.5, 2.6, 2.7, 2.8, 2.9, 2.10, 2.11, 2.14, or 2.15 wherein each R³, R⁴, R⁶, and R⁷ is independently selected from the group consisting of: H and alkyl (e.g., C₁ to C₆ alkyl, such as, for example methyl).

Examples of Q also include, but are not limited to: moieties 2.1, 2.2, 2.3., 2.4, 2.5, 2.6, 2.7, 2.8, 2.9, 2.10, 2.11, 2.14, or 2.15 wherein each R³, R⁴, R⁶, and R⁷ is H.

Examples of Q also include, but are not limited to: moieties 2.17, 2.18, 2.19, 2.20 and 2.21 wherein each R³, R⁴, R⁶, and R⁷ is independently selected from the group consisting of: H and alkyl (e.g., C₁ to C₆ alkyl, such as, for example methyl).

Examples of Q also include, but are not limited to: moieties 2.17, 2.18, 2.19, 2.20 and 2.21 wherein each R³, R⁴, R⁶, and R⁷ is H.

Examples of Q include, but are not limited to: moieties 2.12, 2.13, or 2.16 wherein each R³, R⁴, and R⁷ is independently selected from the group consisting of: H and alkyl (e.g., C₁ to C₆ alkyl, such as, for example methyl).

Examples of Q also include, but are not limited to: moieties 2.12, 2.13, or 2.16 wherein each R³, R⁴, and R⁷ is H.

Examples of Q include, but are not limited to: moiety 2.22 wherein each R³, R⁴, and R⁷ is independently selected from the group consisting of: H and alkyl (e.g., C₁ to C₆ alkyl, such as, for example methyl).

Examples of Q also include, but are not limited to: moiety 2.22 wherein each R³, R⁴, and R⁷ is H.

Thus, in one example of Q, Q is moiety 2.1 wherein each R³, R⁴, R⁶, and R⁷ is independently selected from the group consisting of: H and alkyl (e.g., C₁ to C₆ alkyl, such as, for example methyl).

In another example of Q, Q is moiety 2.1 wherein each R³, R⁴, R⁶, and R⁷ is independently selected from the group consisting of: H and methyl.

In another example of Q, Q is moiety 2.1 wherein each R³, R⁴, R⁶, and R⁷ is H.

In another example of Q, Q is moiety 2.2 wherein each R³, R⁴, R⁶, and R⁷ is independently selected from the group consisting of: H and alkyl (e.g., C₁ to C₆ alkyl, such as, for example methyl).

In another example of Q, Q is moiety 2.2 wherein each R³, R⁴, R⁶, and R⁷ is independently selected from the group consisting of: H and methyl.

In another example of Q, Q is moiety 2.2 wherein each R³, R⁴, R⁶, and R⁷ is H.

In another example of Q, Q is moiety 2.3 wherein each R³, R⁴, R⁶, and R⁷ is independently selected from the group consisting of: H and alkyl (e.g., C₁ to C₆ alkyl, such as, for example methyl).

In another example of Q, Q is moiety 2.3 wherein each R³, R⁴, R⁶, and R⁷ is independently selected from the group consisting of: H and methyl.

In another example of Q, Q is moiety 2.3 wherein each R³, R⁴, R⁶, and R⁷ is H.

In another example of Q, Q is moiety 2.4 wherein each R³, R⁴, R⁶, and R⁷ is independently selected from the group consisting of: H and alkyl (e.g., C₁ to C₆ alkyl, such as, for example methyl).

In another example of Q, Q is moiety 2.4 wherein each R³, R⁴, R⁶, and R⁷ is independently selected from the group consisting of: H and methyl.

In another example of Q, Q is moiety 2.4 wherein each R³, R⁴, R⁶, and R⁷ is H.

In another example of Q, Q is moiety 2.5 wherein each R³, R⁴, R⁶, and R⁷ is independently selected from the group consisting of: H and alkyl (e.g., C₁ to C₆ alkyl, such as, for example methyl).

In another example of Q, Q is moiety 2.5 wherein each R³, R⁴, R⁶, and R⁷ is independently selected from the group consisting of: H and methyl.

In another example of Q, Q is moiety 2.5 wherein each R³, R⁴, R⁶, and R⁷ is H. In another example of Q, Q is moiety 2.6 wherein each R³, R⁴, R⁶, and R⁷ is independently selected from the group consisting of: H and alkyl (e.g., C₁ to C₆ alkyl, such as, for example methyl).

In another example of Q, Q is moiety 2.6 wherein each R³, R⁴, R⁶, and R⁷ is independently selected from the group consisting of: H and methyl.

In another example of Q, Q is moiety 2.7 wherein each R³, R⁴, R⁶, and R⁷ is independently selected from the group consisting of: H and alkyl (e.g., C₁ to C₆ alkyl, such as, for example methyl).

In another example of Q, Q is moiety 2.7 wherein each R³, R⁴, R⁶, and R⁷ is independently selected from the group consisting of: H and methyl.

In another example of Q, Q is moiety 2.7 wherein each R³, R⁴, R⁶, and R⁷ is H.

In another example of Q, Q is moiety 2.8 wherein each R³, R⁴, R⁶, and R⁷ is independently selected from the group consisting of: H and alkyl (e.g., C₁ to C₆ alkyl, such as, for example methyl).

In another example of Q, Q is moiety 2.8 wherein each R³, R⁴, R⁶, and R⁷ is independently selected from the group consisting of: H and methyl.

In another example of Q, Q is moiety 2.8 wherein each R³, R⁴, R⁶, and R⁷ is H.

In another example of Q, Q is moiety 2.9 or 2.10 wherein each R³, R⁴, R⁶, and R⁷ is independently selected from the group consisting of: H and alkyl (e.g., C₁ to C₆ alkyl, such as, for example methyl).

In another example of Q, Q is moiety 2.9 or 2.10 wherein each R³, R⁴, R⁶, and R⁷ is independently selected from the group consisting of: H and methyl.

In another example of Q, Q is moiety 2.9 or 2.10 wherein each R³, R⁴, R⁶, and R⁷ is H.

In another example of Q, Q is moiety 2.11 wherein each R³, R⁴, R⁶, and R⁷ is independently selected from the group consisting of: H and alkyl (e.g., C₁ to C₆ alkyl, such as, for example methyl).

In another example of Q, Q is moiety 2.11 wherein each R³, R⁴, R⁶, and R⁷ is independently selected from the group consisting of: H and methyl.

In another example of Q, Q is moiety 2.11 wherein each R³, R⁴, R⁶, and R⁷ is H.

In another example of Q, Q is moiety 2.12 or 2.13 wherein each R³, R⁴, and R⁷ is independently selected from the group consisting of: H and alkyl (e.g., C₁ to C₆ alkyl, such as, for example methyl).

In another example of Q, Q is moiety 2.12 or 2.13 wherein each R³, R⁴, and R⁷ is independently selected from the group consisting of: H and methyl.

In another example of Q, Q is moiety 2.12 or 2.13 wherein each R³, R⁴, and R⁷ is H.

In another example of Q, Q is moiety 2.14 or 2.15 wherein each R³, R⁴, R⁶, and R⁷ is independently selected from the group consisting of: H and alkyl (e.g., C₁ to C₆ alkyl, such as, for example methyl).

In another example of Q, Q is moiety 2.14 or 2.15 wherein each R³, R⁴, R⁶, and R⁷ is independently selected from the group consisting of: H and methyl.

In another example of Q, Q is moiety 2.14 or 2.15 wherein each R³, R⁴, R⁶, and R⁷ is H.

In another example of Q, Q is moiety 2.16 wherein each R³, R⁴, and R⁷ is H.

In another example of Q, Q is moiety 2.17 wherein each R³, R⁴, R⁶, and R⁷ is independently selected from the group consisting of: H and alkyl (e.g., C₁ to C₆ alkyl, such as, for example methyl).

In another example of Q, Q is moiety 2.17 wherein each R³, R⁴, R⁶, and R⁷ is independently selected from the group consisting of: H and methyl.

In another example of Q, Q is moiety 2.17 wherein each R³, R⁴, R⁶, and R⁷ is H.

In another example of Q, Q is moiety 2.18 wherein each R³, R⁴, R⁶, and R⁷ is independently selected from the group consisting of: H and alkyl (e.g., C₁ to C₆ alkyl, such as, for example methyl).

In another example of Q, Q is moiety 2.18 wherein each R³, R⁴, R⁶, and R⁷ is independently selected from the group consisting of: H and methyl.

In another example of Q, Q is moiety 2.18 wherein each R³, R⁴, R⁶, and R⁷ is H.

In another example of Q, Q is moiety 2.19 wherein each R³, R⁴, R⁶, and R⁷ is independently selected from the group consisting of: H and alkyl (e.g., C₁ to C₆ alkyl, such as, for example methyl).

In another example of Q, Q is moiety 2.19 wherein each R³, R⁴, R⁶, and R⁷ is independently selected from the group consisting of: H and methyl.

In another example of Q, Q is moiety 2.19 wherein each R³, R⁴, R⁶, and R⁷ is H.

In another example of Q, Q is moiety 2.20 wherein each R³, R⁴, R⁶, and R⁷ is independently selected from the group consisting of: H and alkyl (e.g., C₁ to C₆ alkyl, such as, for example methyl).

In another example of Q, Q is moiety 2.20 wherein each R³, R⁴, R⁶, and R⁷ is independently selected from the group consisting of: H and methyl.

In another example of Q, Q is moiety 2.20 wherein each R³, R⁴, R⁶, and R⁷ is H.

In another example of Q, Q is moiety 2.21 wherein each R³, R⁴, R⁶, and R⁷ is independently selected from the group consisting of: H and alkyl (e.g., C₁ to C₆ alkyl, such as, for example methyl).

In another example of Q, Q is moiety 2.21 wherein each R³, R⁴, R⁶, and R⁷ is independently selected from the group consisting of: H and methyl.

In another example of Q, Q is moiety 2.21 wherein each R³, R⁴, R⁶, and R⁷ is H.

In another example of Q, Q is moiety 2.22 wherein each R³, R⁴, and R⁷ is independently selected from the group consisting of: H and alkyl (e.g., C₁ to C₆ alkyl, such as, for example methyl).

In another example of Q, Q is moiety 2.22 wherein each R³, R⁴, and R⁷ is independently selected from the group consisting of: H and methyl.

In another example of Q, Q is moiety 2.22 wherein each R³, R⁴, and R⁷ is H.

Another example of the Q substituent 2.3 is: 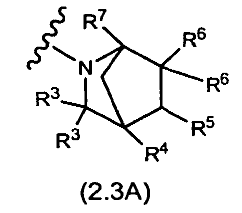 (i.e., each R²⁴ is H and w is 1).

Another example of the Q substituent 2.3 is: 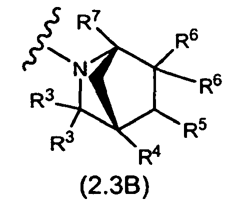 (i.e., each R²⁴ is H and w is 1).

Another example of the Q substitutent 2.3 is: 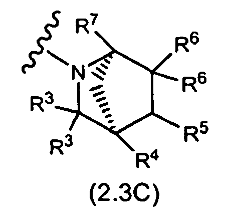 (i.e., each R²⁴ is H and w is 1).

An example of the Q substituent 2.4 is: 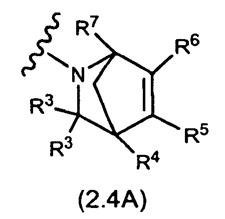 (i.e., each R²⁴ is H and w is 1).

Another example of the Q substituent 2.4 is: 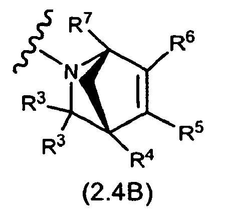 (i.e., each R²⁴ is H and w is 1).

Another example of the Q substituent 2.4 is: 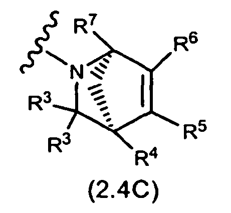 (i.e., each R²⁴ is H and w is 1).

An example of the Q substituent 2.5 is: 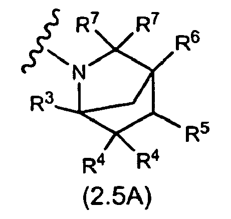 (i.e., each R²⁴ is H and w is 1).

Another example of the Q substituent 2.5 is: 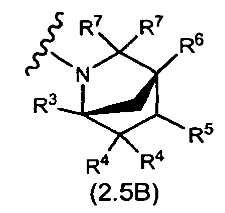 (i.e., each R²⁴ is H and w is 1).

Another example of the Q substituent 2.5 is: 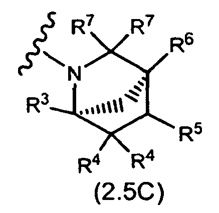 (i.e., each R²⁴ is H and w is 1).

An example of the Q substituent 2.6 is:

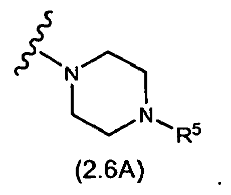

An example of the Q substituent 2.7 is: 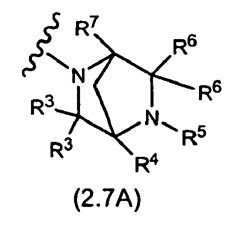 (i.e., each R²⁴ is H and w is 1).

An example of the Q substituent 2.7 is: 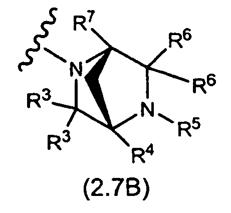 (i.e., each R²⁴ is H and w is 1).

An example of the Q substituent 2.7 is: 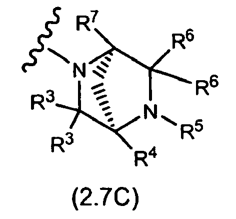 (i.e., each R²⁴ is H and w is 1).

An example of the Q substituent 2.8 is: 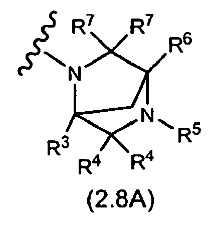 (i.e., each R²⁴ is H and w is 1).

Another example of the Q substituent 2.8 is: 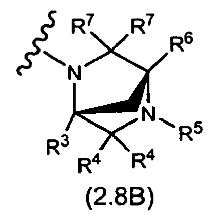 (i.e., each R²⁴ is H and w is 1).

Another example of the Q substituent 2.8 is: 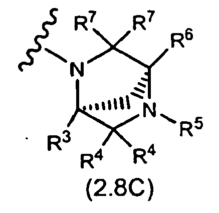 (i.e., each R²⁴ is H and w is 1).

Another example of the Q substituent 2.3 is:

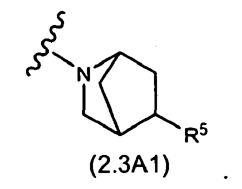

Another example of the Q substituent 2.3 is:

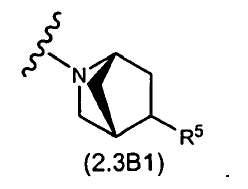

Another example of the Q substituent 2.3 is:

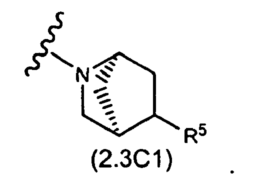

Another example of the Q substituent 2.4 is:

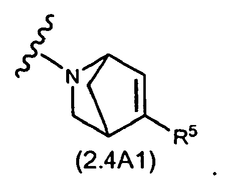

Another example of the Q substituent 2.4 is:

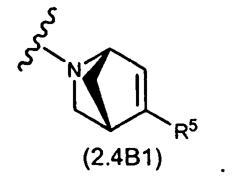

Another example of the Q substituent 2.4 is:

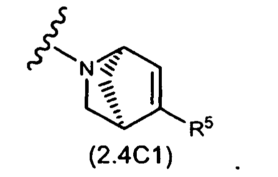

Another example of the Q substituent 2.5 is:

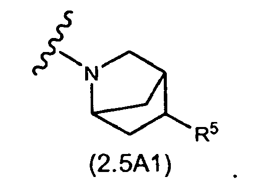

Another example of the Q substituent 2.5 is:

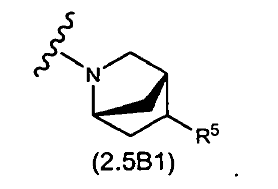

Another example of the Q substituent 2.5 is:

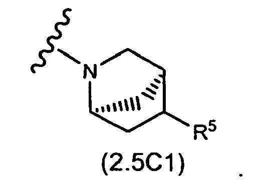

Another example of the Q substituent 2.7 is:

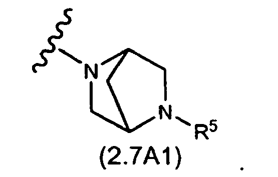

Another example of the Q substituent 2.7 is:

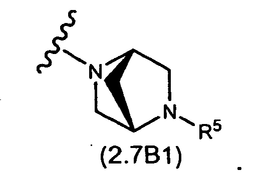

Another example of the Q substituent 2.7 is:

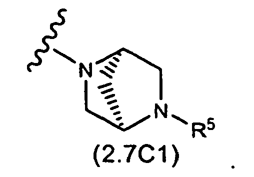

Another example of the Q substituent 2.8 is:

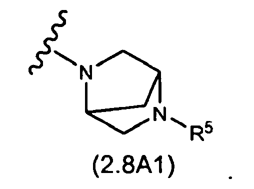

Another example of the Q substituent 2.8 is:

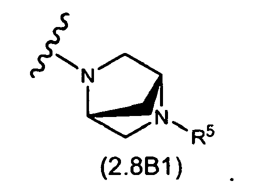

Another example of the Q substituent 2.8 is:

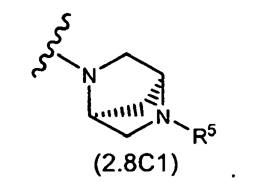

Another example of the Q substitutent is the piperazine ring: 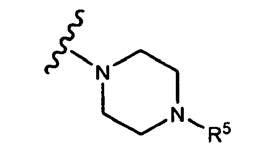 substituted with one or two substituents independently selected from the group consisting of R³ groups, provided that said one or two substitutents are not H. In one embodiment said substituents are selected from the group consisting of alkyl groups (e.g., C₁ to C₆ alkyl, e.g., methyl). In another embodiment there is one substituent on said piperazine ring. In another embodiment there is one substituent on said piperazine ring and said substituent is methyl.

Another example of the Q substituent is the piperazine ring:

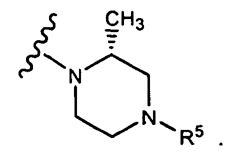

Another example of the Q substitutent is the piperidine ring: 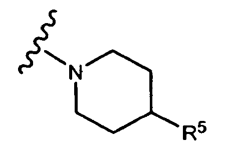 substituted with one or two substituents independently selected from the group consisting of R³ groups, provided that said one or two substitutents are not H. In one embodiment said substituents are selected from the group consisting of alkyl groups (e.g., C₁ to C₆ alkyl, e.g., methyl). In another embodiment there is one substituent on said piperidine ring. In another embodiment there is one substituent on said piperidine ring and said substituent is methyl.

In one example of the Q substituent 2.16 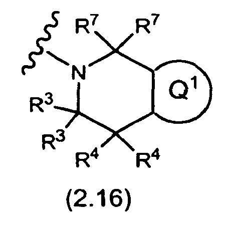 Q¹ is heteroaryl.

In another example of the Q substituent 2.16 Q¹ is aryl.

Thus, one example of the Q substituent 2.16 is 2.16A: 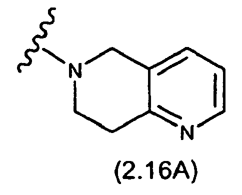 (i.e., Q¹ is pyridyl, and each R³, R⁴ and R⁷ is H).

In another example, the Q substituent 2.16 is 2.16A1:

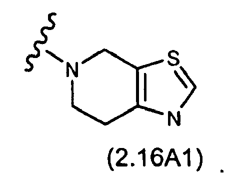

Another example of the Q substitutent 2.16 is 2.16B: 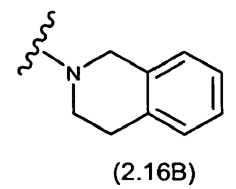 (i.e., Q¹ is phenyl, and each R³, R⁴ and R⁷ is H).

When the Q substitutent comprises two Q¹ rings, each Q¹ ring is independently selected. Generally, the Q¹ cycloalkyl rings and the Q¹ substituted cycloalkyl rings comprise 5 to 7 ring carbons. In general, the heterocycloalkyl Q¹ rings and the substituted heterocycloalky Q¹ rings comprise 5 to 7 ring carbons and comprise 1 to 3 (generally 1 or 2, or generally 1) ring heteroatoms selected from the group consisting of: O, N and S. In general, the heteroaryl Q¹ rings and the substituted heteroaryl Q¹ rings comprise 5 to 7 ring carbons and comprise 1 to 3 (generally 1 or 2, or generally 1) ring heteroatoms selected from the group consisting of: O, N and S. Examples of the Q¹ rings include, but are not limited to: piperidinyl, piperazinyl, pyranyl, pyrrolidinyl, morpholinyl, thiomorpholinyl, pyridyl, pyrimidinyl, pyrrolyl, pyrazolyl, furanyl, thienyl, thiazolyl, imidazolyl, cyclopentyl, cyclohexyl and cycloheptyl. Examples of the Q¹ rings also include, but are not limited to: substituted piperidinyl, substituted piperazinyl, substituted pyranyl, substituted pyrrolidinyl, substituted morpholinyl, substituted thiomorpholinyl, substituted pyridyl, substituted pyrimidinyl, substituted pyrrolyl, substituted pyrazolyl, substituted furanyl, substituted thienyl, substituted thiazolyl, substituted imidazolyl, substituted cyclopentyl, substituted cyclohexyl and substituted cycloheptyl wherein said substituted Q¹ rings are substituted with 1 to 3 substitutents selected from the R¹⁰ moieties.

Generally, the Q² cycloalkyl rings and the Q² substituted cycloalkyl rings comprise 5 to 7 ring carbons. In general, the heterocycloalkyl Q² rings and the substituted heterocycloalky Q¹ rings comprise 5 to 7 ring carbons and comprise 1 to 3 (generally 1 or 2, or generally 1) ring heteroatoms selected from the group consisting of: O, N and S.

Examples of the Q² rings include, but are not limited to: piperidinyl, piperazinyl, pyranyl, pyrrolidinyl, cyclopentyl, cyclohexyl and cycloheptyl. Examples of the Q² rings also include, but are not limited to: substituted piperidinyl, substituted piperazinyl, substituted pyranyl, substituted pyrrolidinyl, substituted morpholinyl, substituted thiomorpholinyl, substituted cyclopentyl, substituted cyclohexyl and substituted cycloheptyl wherein said substituted Q¹ rings are substituted with 1 to 3 substitutents selected from the R¹⁰ moieties.

In one example the Q substituent 2.17 is: 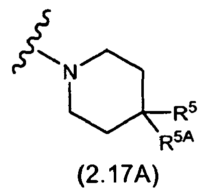 wherein R^{5A} is halo.

Another example of the Q substituent 2.17 is:

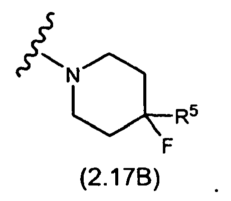

Another example of the Q substituent 2.17 is:

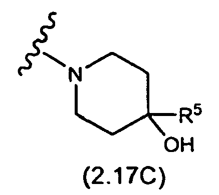

Another example of the Q substituent 2.17 is: 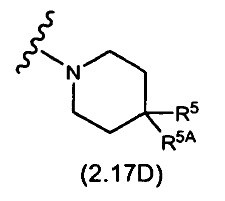 wherein R^{5A} is alkoxy, i.e., -O-(C₁ to C₆)alkyl, such as, for example, -O-(C₁ to C₃)alkyl, or -O-(C₁ to C₂)alkyl.

Another example of the Q substituent 2.17 is:

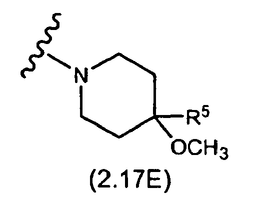

Examples of R¹ for the compounds of this invention (e.g., compounds of formulas 1.0, 1.0A1, 1.0B1, 1.0C1, 1.1, 1.1A, 1.2, 1.2A, 1.3 and 1.3A) include, but are not limited to:

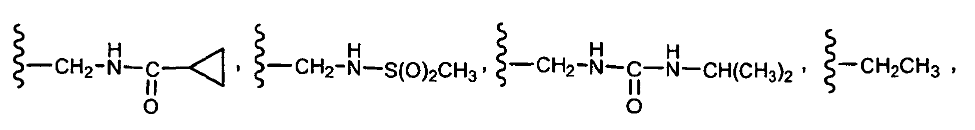

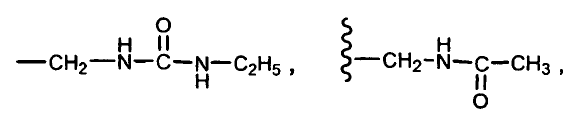

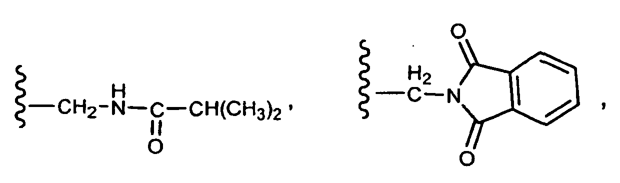

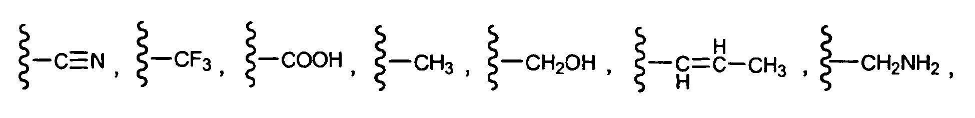

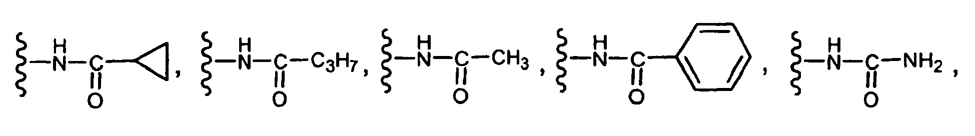

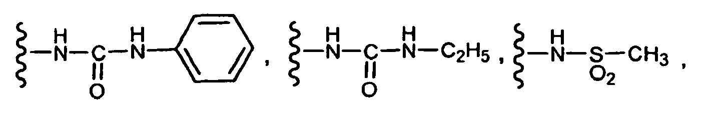

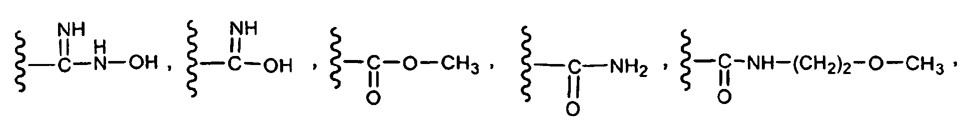

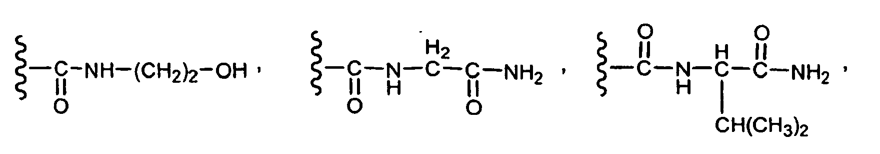

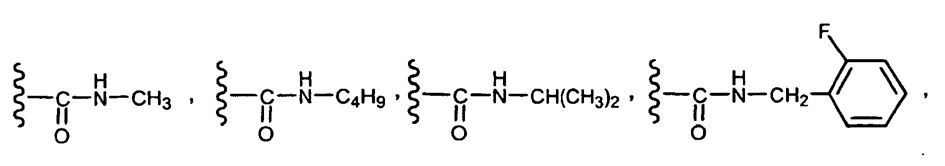

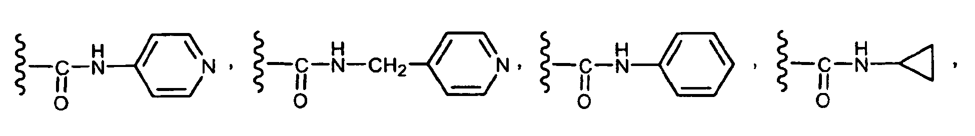

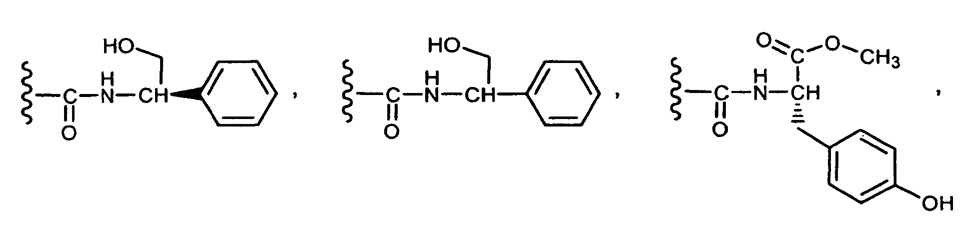

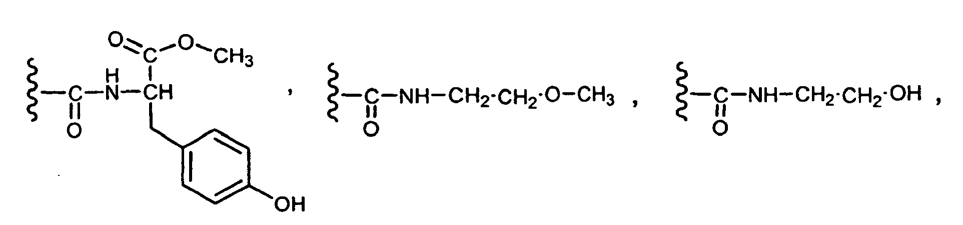

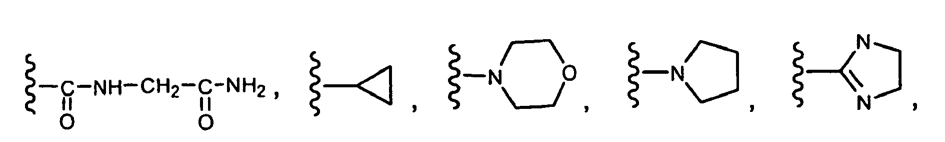

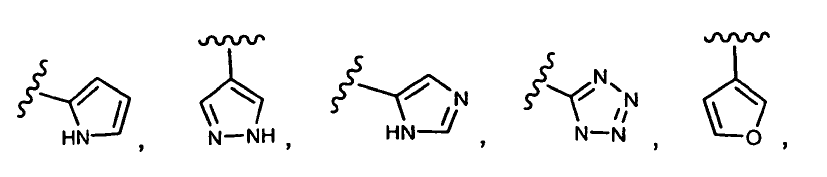

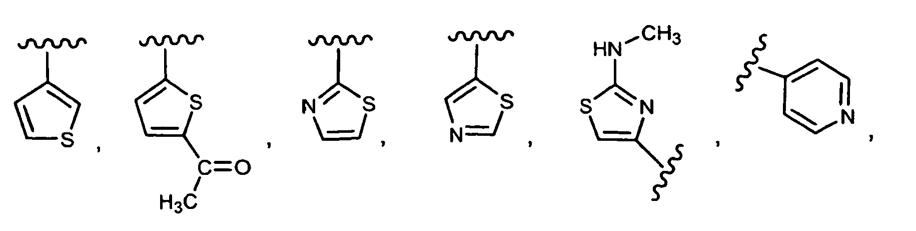

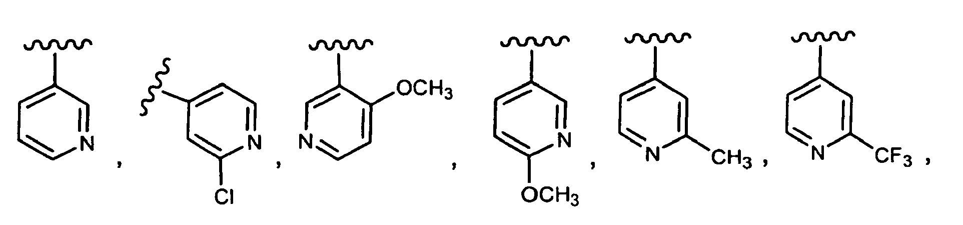

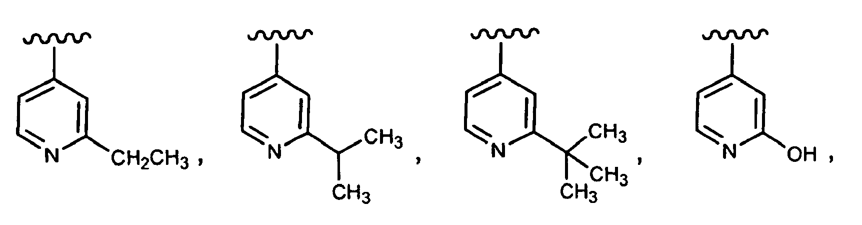

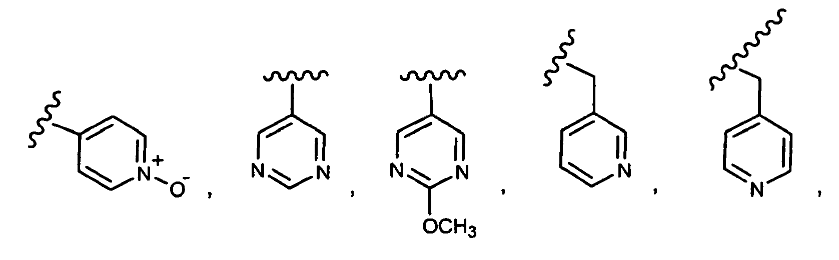

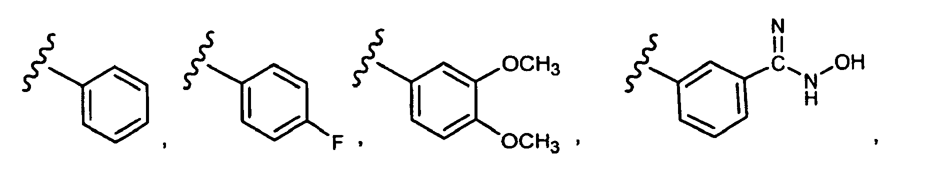

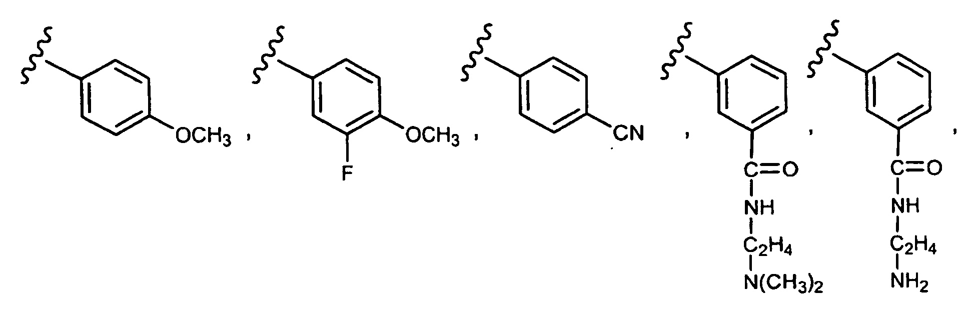

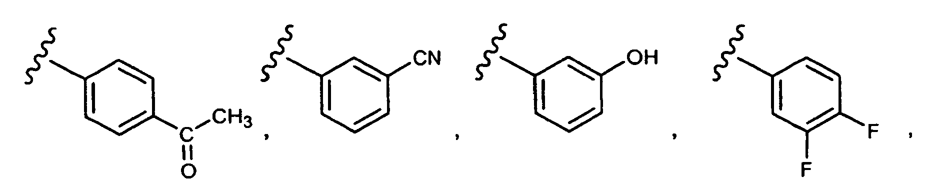

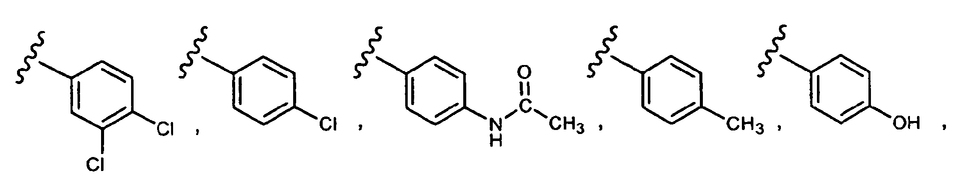

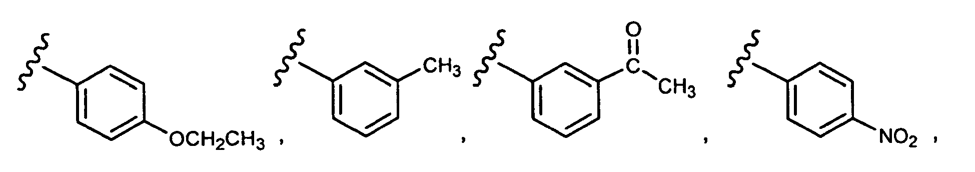

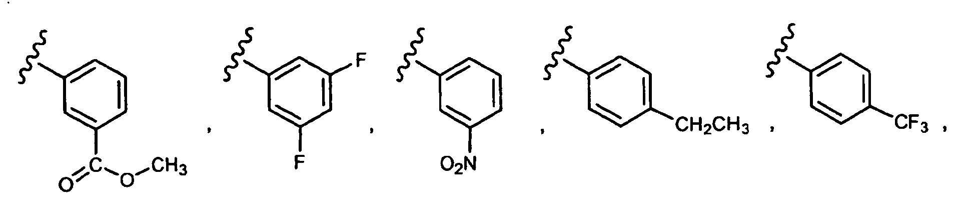

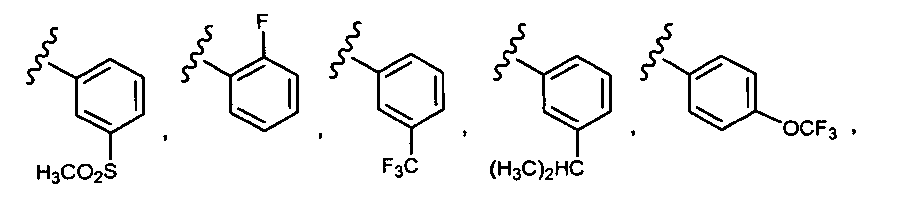

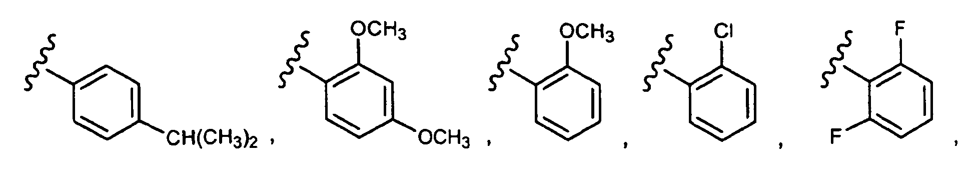

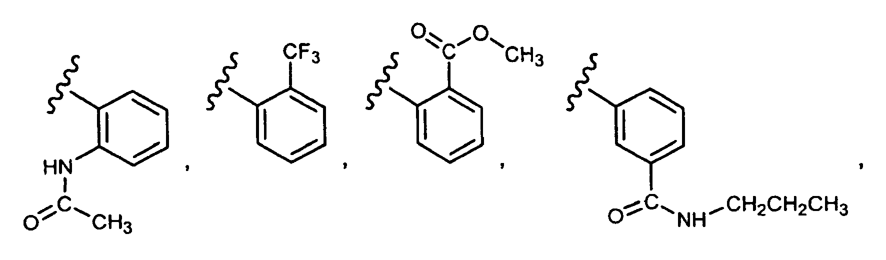

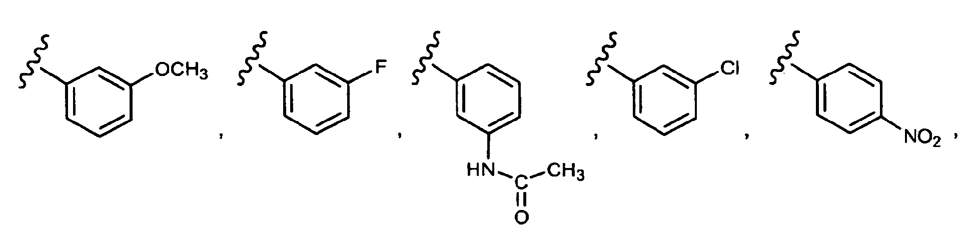

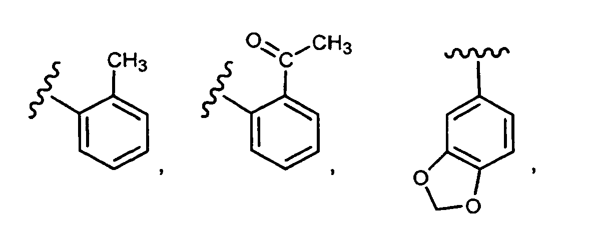

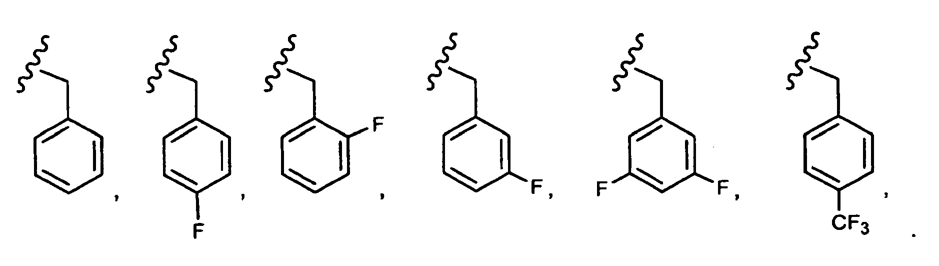

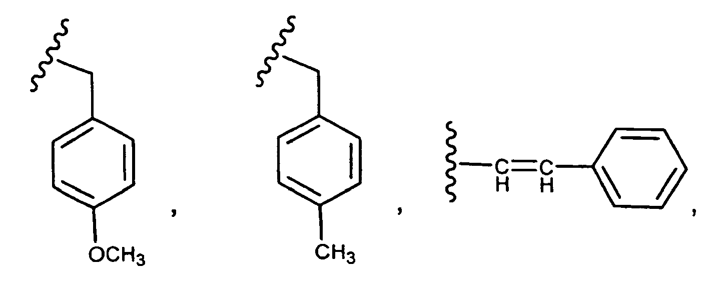

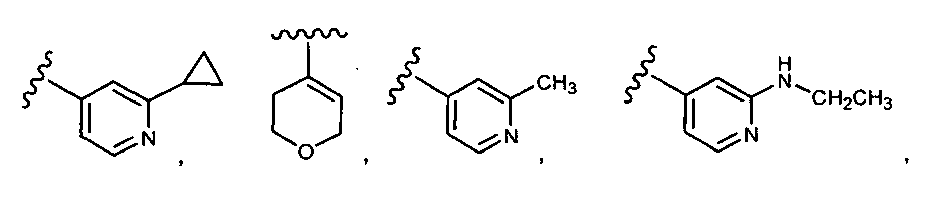

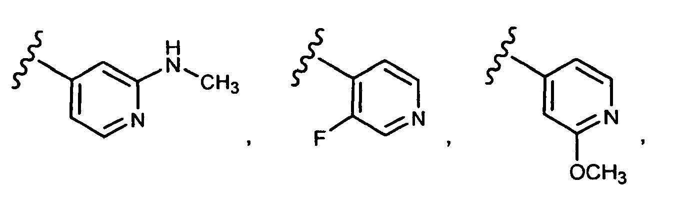

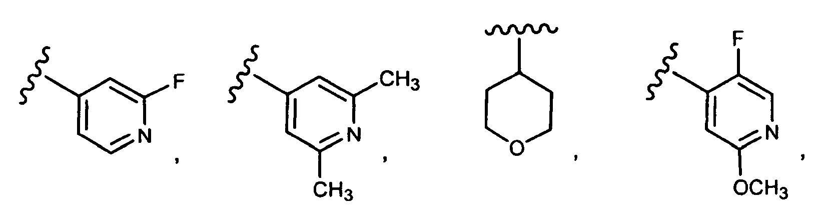

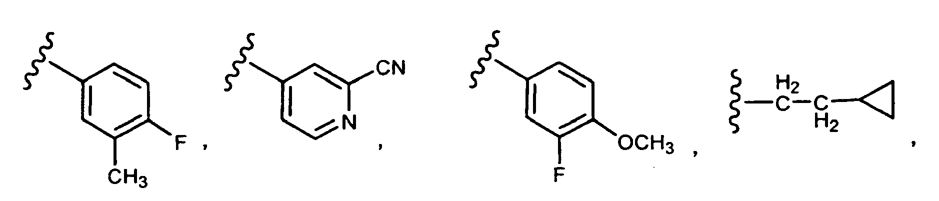

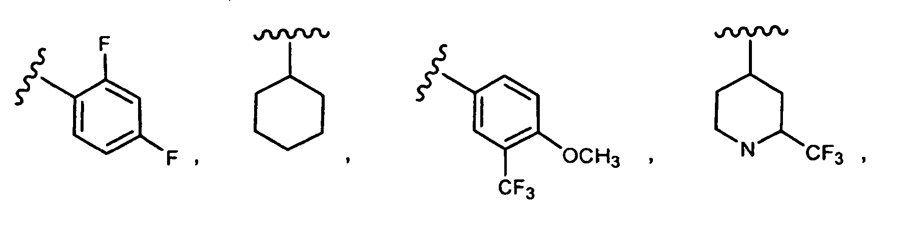

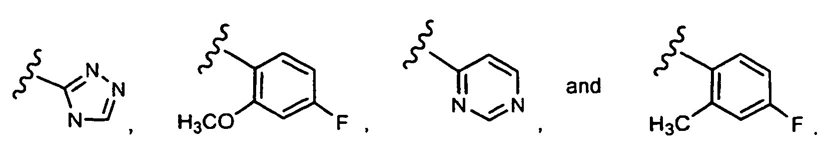

R¹, in one embodiment of this invention, is aryl (e.g., phenyl).

R¹, in one embodiment of this invention is substituted aryl, such as,

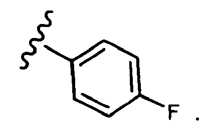

R¹, in another embodiment of this invention, is heteroaryl (e.g., in one embodiment R¹ is pyridyl N-oxide, and in another embodiment R¹ is pyridyl, such as

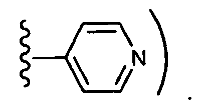

R¹, in one embodiment of this invention, is substituted heteroaryl (e.g., substituted pyridyl).

R¹, in one embodiment of this invention, is substituted heteroaryl (e.g., substituted pyridyl), such as, for example:

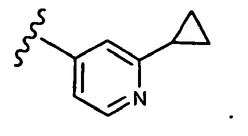

Examples of R⁵ for the compounds of this invention (e.g., compounds of formulas 1.0, 1.0A1, 1.0B1, 1.0C1, 1.1, 1.1A, 1.2, 1.2A, 1.3 and 1.3A) include but are not limited to:

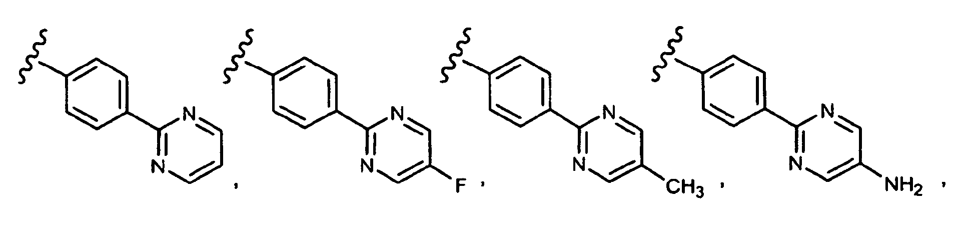

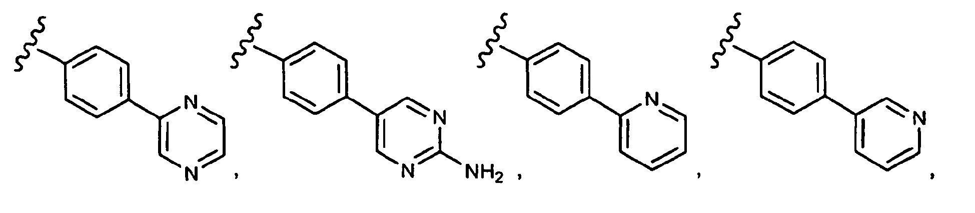

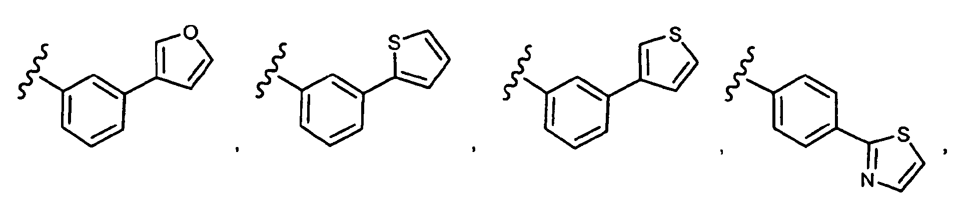

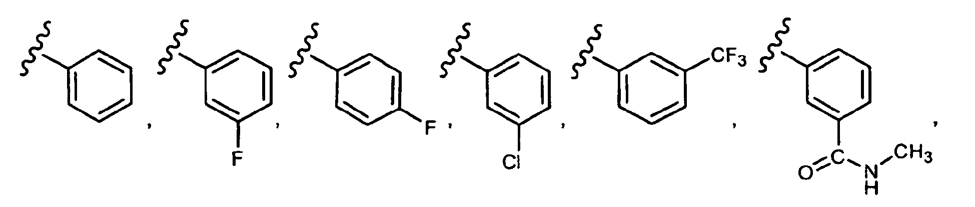

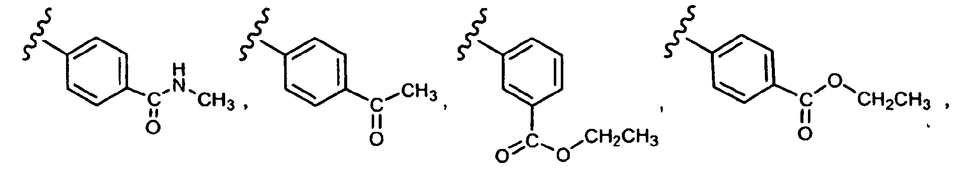

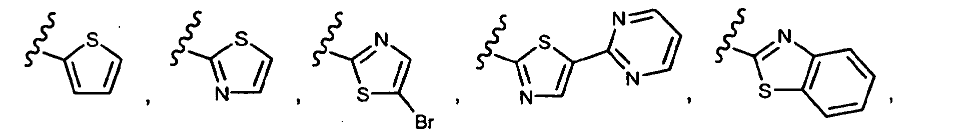

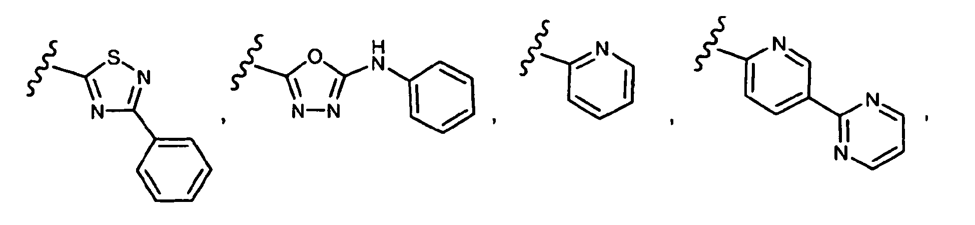

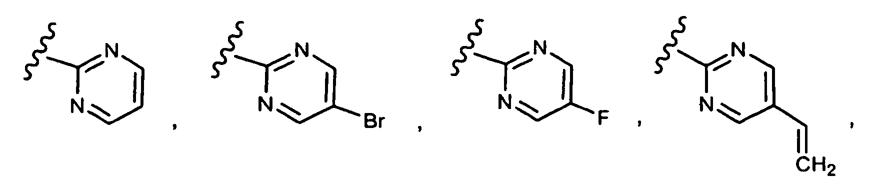

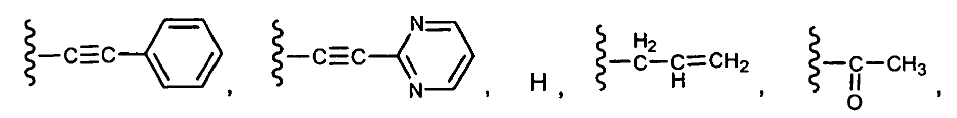

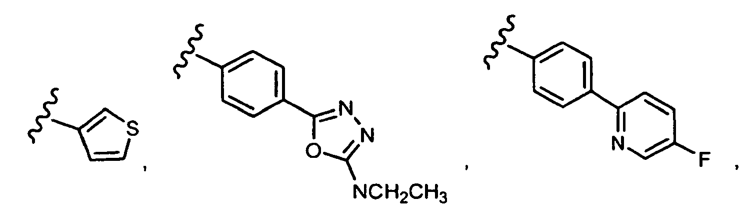

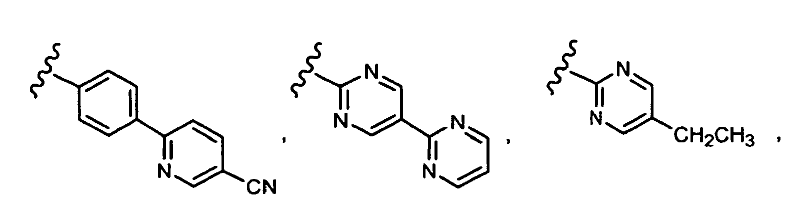

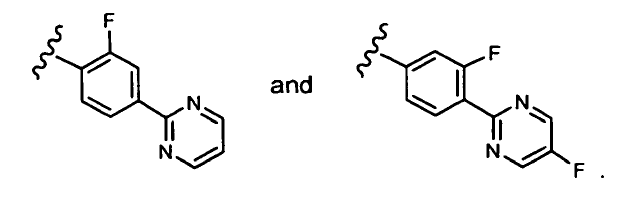

In another embodiment of this invention, R⁵ is selected from the group consisting of : 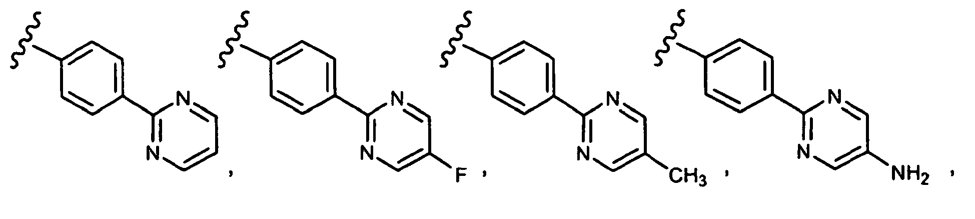 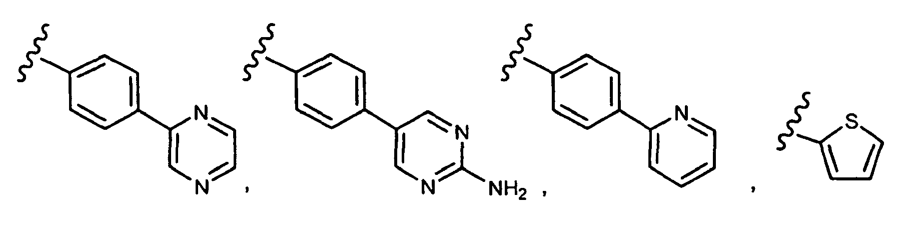 and

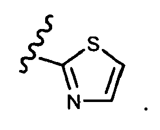

In another embodiment of this invention, R⁵ is selected from the group consisting of: 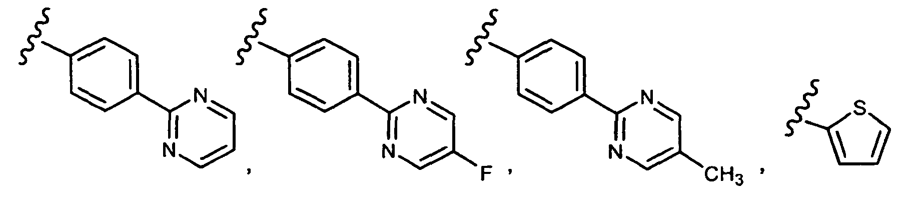 and

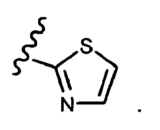

In another embodiment of this invention, R⁵ is:

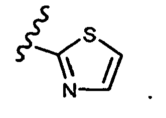

In another embodiment of this invention, R⁵ is:

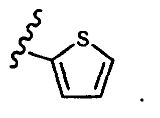

In another embodiment of this invention, R⁵ is:

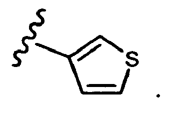

R², in one embodiment of this invention, is -(CH₂)ₘR¹¹, wherein R¹¹ is -OR¹⁰.

R², in another embodiment of this invention, is -(CH₂)ₘR¹¹, wherein R¹¹ is -OR¹⁰, and R¹⁰ is H or alkyl.

R², in another embodiment of this invention, is -(CH₂)ₘR¹¹, wherein R¹¹ is -OR¹⁰, and R¹⁰ alkyl (e.g., methyl).

R², in another embodiment of this invention, is -(CH₂)ₘR¹¹, wherein m is 1 and R¹¹ is -OR¹⁰.

R², in another embodiment of this invention, is -(CH₂)ₘR¹¹, wherein m is 1, R¹¹ is -OR¹⁰, and R¹⁰ is H or alkyl.

R², in another embodiment of this invention, is -(CH₂)ₘR¹¹, wherein m is 1, R¹¹ is -OR¹⁰, and R¹⁰ alkyl.

R², in another embodiment of this invention, is -(CH₂)ₘR¹¹, wherein m is 1, R¹¹ is -OR¹⁰, and R¹⁰ methyl (i.e., R² is -CH₂OCH₃).

R², in another embodiment of this invention, is -OR²³ wherein R²³ is alkyl, and said alkyl is methyl (i.e., R² is -OCH₃).

R², in another embodiment of this invention, is alkynyl. An example of an alkynyl group is ethynyl: 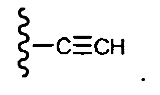 Another example of an alkynyl group is propynyl:

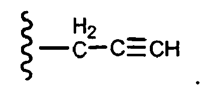

R², in another embodiment of this invention, is alkenyl. An example of an alkenyl group is -CH₂-CH=CH₂.

Preferably R² is selected from the group consisting of: ethynyl, -OCH₃, and -CH₂OCH₃.

Additional examples of the R² -(CH₂)ₘR¹¹ group include, but are not limited to -CH₂OH, -CH₂CN, -CH₂OC₂H₅, -(CH₂)₃OCH₃, -CH₂F and -CH₂-triazolyl, such as,

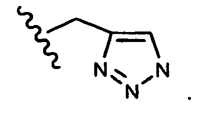

Additional examples of R² include, but are not limited to, H, -CH₂-morpholinyl, -SCH₃, -OC₂H₅, -OCH(CH₃)₂, -CH₂N(CH₃)₂, -CN, -CH(OH)CH₃, -C(O)CH₃, -CH₂C≡CCH₃, -CH(CH₃)₂, -C(CH₃)=CH₂, -C(CH₃)=NOCH₃, -C(CH₃)=NOH, -C(CH₃)=NNHC(O)CH₃, -NH₂, -NHC(O)H, -NHCH₃, -CH₂-O-CH₂-cyclopropyl, -CH₂-O-CHF₂, -OCHF₂, -CHF₂, -CH₂C(CH₃)=CH₃, -CH₂CH₂CH₃, -N(CH₃)₂, -CH₂CH₃, -CF₃, -CH=CH₂, and -C(OH)(CH₃)₂.

R³, in one embodiment of this invention, is independently selected from the group consisting of: H and alkyl.

R³, in another embodiment of this invention, is independently selected from the group consisting of: H and methyl.

R³, in another embodiment of this invention, is H.

R⁴, in one embodiment of this invention, R⁴ H.

R⁴, in another embodiment of this invention, is selected from the group consisting of: H and alkyl.

R⁴, in another embodiment of this invention, is selected from the group consisting of: H and methyl.

R⁶, in one embodiment of this invention, is R⁶ H.

R⁷, in one embodiment of this invention, is independently selected from the group consisting of: H and alkyl.

R⁷, in another embodiment of this invention, is independently selected from the group consisting of: H and methyl.

R⁷, in one embodiment of this invention, is H.

R⁸, in one embodiment of this invention, is H.

Y¹, in one embodiment of this invention, is carbon.

Y², in one embodiment of this invention, is carbon.

Y³ in one embodiment of this invention, is carbon.

Y¹, Y² and Y³, in one embodiment of this invention, are carbon.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16, and each R³, R⁴, and R⁷ is independently selected from the group consisting of: H and methyl.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16A, and each R³, R⁴, and R⁷ is independently selected from the group consisting of: H and methyl.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16B, and each R³, R⁴, and R⁷ is independently selected from the group consisting of: H and methyl.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16, and each R³, R⁴, and R⁷ is H.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent G1 is 2.16A, and each R³, R⁴, and R⁷ is H.

One embodiment of this invention is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16B, and each R³, R⁴, and R⁷ is H.

The compounds of this invention inhibit the activity of ERK1 and ERK2 Thus, also disclosed is a method of inhibiting ERK in mammals, especially humans, by the administration of an effective amount (e.g., a therapeutically effective amount) of one or more (e.g., one) compounds of this invention. The administration of the compounds of this invention to patients, to inhibit ERK1 and/or ERK2, is useful in the treatment of cancer.

In any of the uses for the manufacture of a medicament for treating cancer described herein, unless stated otherwise, the medicaments can optionally be in a form for administration with an effective amount of one or more (e.g., 1, 2 or 3, or 1 or 2, or 1) chemotherapeutic agents. The chemotherapeutic agents can be administered currently or sequentially with the compounds of this invention.

The uses for the manufacture of medicaments for treating cancer described herein include uses wherein a combination of drugs (i.e., compounds, or pharmaceutically active ingredients, or pharmaceutical compositions) are used (i.e., the uses for the manufacture of medicaments for treating cancer of this invention include combination therapies). Those skilled in the art will appreciate that the drugs are generally administered individually as a pharmaceutical composition. The use of a pharmaceutical composition comprising more than one drug is within the scope of this invention.

In any of the uses for the manufacture of medicaments for treating cancer described herein, unless stated otherwise, the medicaments can optionally be in a form for administration with an effective amount of radiation therapy. For radiation therapy, γ-radiation is preferred.

Examples of cancers which may be treated by the uses of this invention include, but are not limited to: (A) lung cancer (e.g., lung adenocarcinoma and non small cell lung cancer), (B) pancreatic cancers (e.g., pancreatic carcinoma such as, for example, exocrine pancreatic carcinoma), (C) colon cancers (e.g., colorectal carcinomas, such as, for example, colon adenocarcinoma and colon adenoma), (D) myeloid leukemias (for example, acute myelogenous leukemia (AML), CML, and CMML), (E) thyroid cancer, (F) myelodysplastic syndrome (MDS), (G) bladder carcinoma, (H) epidermal carcinoma, (I) melanoma, (J) breast cancer, (K) prostate cancer, (L) head and neck cancers (e.g., squamous cell cancer of the head and neck), (M) ovarian cancer, (N) brain cancers (e.g., gliomas, such as glioma blastoma multiforme), (O) cancers of mesenchymal origin (e.g., fibrosarcomas and rhabdomyosarcomas), (P) sarcomas, (Q) tetracarcinomas, (R) nuroblastomas, (S) kidney carcinomas, (T) hepatomas, (U) non-Hodgkin's lymphoma, (V) multiple myeloma, and (W) anaplastic thyroid carcinoma.

Chemotherapeutic agents (antineoplastic agent) include but are not limited to: microtubule affecting agents, alkylating agents, antimetabolites, natural products and their derivatives, hormones and steroids (including synthetic analogs), and synthetics.

Examples of alkylating agents (including nitrogen mustards, ethylenimine derivatives, alkyl sulfonates, nitrosoureas and triazenes) include: Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide.

Examples of antimetabolites (including folic acid antagonists, pyrimidine analogs, purine analogs and adenosine deaminase inhibitors) include: Methotrexate, 5-Fluorouracil, Floxuridine, Cytarabine, 6-Mercaptopurine, 6-Thioguanine, Fludarabine phosphate, Pentostatine, and Gemcitabine.

Examples of natural products and their derivatives (including vinca alkaloids, antitumor antibiotics, enzymes, lymphokines and epipodophyllotoxins) include: Vinblastine, Vincristine, Vindesine, Bleomycin, Dactinomycin, Daunorubicin, Doxorubicin, Epirubicin, Idarubicin, Paclitaxel (paclitaxel is a microtubule affecting agent and is commercially available as Taxol^{®}), Paclitaxel derivatives (e.g. taxotere), Mithramycin, Deoxyco-formycin, Mitomycin-C, L-Asparaginase, Interferons (especially IFN-a), Etoposide, and Teniposide.

Examples of hormones and steroids (including synthetic analogs) include: 17α-Ethinylestradiol, Diethylstilbestrol, Testosterone, Prednisone, Fluoxymesterone,

Dromostanolone propionate, Testolactone, Megestrolacetate, Tamoxifen, Methylprednisolone, Methyl-testosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, and Zoladex.

Examples of synthetics (including inorganic complexes such as platinum coordination complexes): Cisplatin, Carboplatin, Hydroxyurea, Amsacrine, Procarbazine, Mitotane, Mitoxantrone, Levamisole, and Hexamethylmelamine.

Examples of other chemotherapeutics include: Navelbene, CPT-11, Anastrazole, Letrazole, Capecitabinbe, Reloxafine, and Droloxafine.

A microtubule affecting agent (e.g., paclitaxel, a paclitaxel derivative or a paclitaxel-like compound), as used herein, is a compound that interferes with cellular mitosis, i.e., having an anti-mitotic effect, by affecting microtubule formation and/or action. Such agents can be, for instance, microtubule stabilizing agents or agents which disrupt microtubule formation.

Microtubule affecting agents, useful in the uses of this invention, are well known to those skilled in the art and include, but are not limited to: Allocolchicine (NSC 406042), Halichondrin B (NSC 609395), Colchicine (NSC 757), Colchicine derivatives (e.g., NSC 33410), Dolastatin 10 (NSC 376128), Maytansine (NSC 153858), Rhizoxin (NSC 332598), Paclitaxel (Taxol^{®}, NSC 125973), Paclitaxel derivatives (e.g., Taxotere, NSC 608832), Thiocolchicine (NSC 361792), Trityl Cysteine (NSC 83265), Vinblastine Sulfate (NSC 49842), Vincristine Sulfate (NSC 67574), Epothilone A, Epothilone, Discodermolide (see Service, (1996) Science, 274:2009), Estramustine, Nocodazole, MAP4, and the like. Examples of such agents are described in, for example, Bulinski (1997) J. Cell Sci. 110:3055-3064, Panda (1997) Proc. Natl. Acad. Sci. USA 94:10560-10564, Muhlradt (1997) Cancer Res. 57:3344-3346, Nicolaou (1997) Nature 387:268-272, Vasquez (1997) Mol. Biol. Cell. 8:973-985, and Panda (1996) J. Biol. Chem. 271:29807-29812.

Chemotherapeutic agents with paclitaxel-like activity include, but are not limited to, paclitaxel and paclitaxel derivatives (paclitaxel-like compounds) and analogues. Paclitaxel and its derivatives (e.g. Taxol and Taxotere) are available commercially. In addition, methods of making paclitaxel and paclitaxel derivatives and analogues are well known to those of skill in the art (see, e.g., U.S. Patent Nos: 5,569,729; 5,565,478; 5,530,020; 5,527,924; 5,508,447; 5,489,589; 5,488,116; 5,484,809; 5,478,854; 5,478,736; 5,475,120; 5,468,769; 5,461,169; 5,440,057; 5,422,364; 5,411,984; 5,405,972; and 5,296,506).

More specifically, the term "paclitaxel" as used herein refers to the drug commercially available as Taxol^{®} (NSC number: 125973). Taxol^{®} inhibits eukaryotic cell replication by enhancing polymerization of tubulin moieties into stabilized microtubule bundles that are unable to reorganize into the proper structures for mitosis. Of the many available chemotherapeutic drugs, paclitaxel has generated interest because of its efficacy in clinical trials against drug-refractory tumors, including ovarian and mammary gland tumors (Hawkins (1992) Oncology, 6: 17-23, Horwitz (1992) Trends Pharmacol. Sci. 13: 134-146, Rowinsky (1990) J. Natl. Canc. Inst. 82: 1247-1259).

Additional microtubule affecting agents can be assessed using one of many such assays known in the art, e.g., a semiautomated assay which measures the tubulin-polymerizing activity of paclitaxel analogs in combination with a cellular assay to measure the potential of these compounds to block cells in mitosis (see Lopes (1997) Cancer Chemother. Pharmacol. 41:37-47).

Generally, activity of a test compound is determined by contacting a cell with that compound and determining whether or not the cell cycle is disrupted, in particular, through the inhibition of a mitotic event. Such inhibition may be mediated by disruption of the mitotic apparatus, e.g., disruption of normal spindle formation. Cells in which mitosis is interrupted may be characterized by altered morphology (e.g., microtubule compaction, increased chromosome number, etc.).

Compounds with possible tubulin polymerization activity can be screened in vitro. For example, the compounds are screened against cultured WR21 cells (derived from line 69-2 wap-ras mice) for inhibition of proliferation and/or for altered cellular morphology, in particular for microtubule compaction. In vivo screening of positive-testing compounds can then be performed using nude mice bearing the WR21 tumor cells. Detailed protocols for this screening method are described by Porter (1995) Lab. Anim. Sci., 45(2):145-150.

Other methods of screening compounds for desired activity are well known to those of skill in the art. Typically such assays involve assays for inhibition of microtubule assembly and/or disassembly. Assays for microtubule assembly are described, for example, by Gaskin et al. (1974) J. Molec. Biol., 89: 737-758. U.S. Patent No. 5,569,720 also provides in vitro and in vivo assays for compounds with paclitaxel-like activity.

Thus, in the uses of this invention wherein at least one chemotherapeutic agent is used, examples of said chemotherapeutic agents include those selected from the group consisting of: microtubule affecting agents, alkylating agents, antimetabolites, natural products and their derivatives, hormones and steroids (including synthetic analogs), and synthetics.

In the uses of this invention wherein at least one chemotherapeutic agent is used, examples of said chemotherapeutic agents also include: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

In the uses of this invention wherein at least one chemotherapeutic agent is used, examples of such chemotherapeutic agents include:
(1) taxanes such as paclitaxel (TAXOL^{®}) and/or docetaxel (Taxotere^{®});
(2) platinum coordinator compounds, such as, for example, carboplatin, cisplatin and oxaliplatin (e.g. Eloxatin);
(3) EGF inhibitors that are antibodies, such as: HER2 antibodies (such as, for example trastuzumab (Herceptin^{®}), Genentech, Inc.), Cetuximab (Erbitux, IMC-C225, ImClone Systems), EMD 72000 (Merck KGaA), anti-EFGR monoclonal antibody ABX (Abgenix), TheraCIM-h-R3 (Center of Molecular Immunology), monoclonal antibody 425 (Merck KGaA), monoclonal antibody ICR-62 (ICR, Sutton, England); Herzyme (Elan Pharmaceutical Technologies and Ribozyme Pharmaceuticals), PKI 166 (Novartis), EKB 569 (Wyeth-Ayerst), GW 572016 (GlaxoSmithKline), CI 1033 (Pfizer Global Research and Development), trastuzmab-maytansinoid conjugate (Genentech, Inc.), mitumomab (Imclone Systems and Merck KGaA) and Melvax II (Imclone Systems and Merck KgaA);
(4) EGF inhibitors that are small molecules, such as, Tarceva (TM) (OSI-774, OSI Pharmaceuticals, Inc.), and Iressa (ZD 1839, Astra Zeneca);
(5) VEGF inhibitors that are antibodies such as: bevacizumab (Genentech, Inc.), and IMC-1C11 (ImClone Systems), DC 101 (a KDR VEGF Receptor 2 from ImClone Systems);
(6) VEGF kinase inhibitors that are small molecules such as SU 5416 (from Sugen, Inc), SU 6688 (from Sugen, Inc.), Bay 43-9006 (a dual VEGF and bRAF inhibitor from Bayer Pharmaceuticals and Onyx Pharmaceuticals);
(7) estrogen receptor antagonists or selective estrogen receptor modulators (SERMs), such as tamoxifen, idoxifene, raloxifene, trans-2,3-dihydroraloxifene, levormeloxifene, droloxifene, MDL 103,323, and acolbifene (Schering Corp.);
(8) anti-tumor nucleoside derivatives such as 5-fluorouracil, gemcitabine, capecitabine, cytarabine (Ara-C), fludarabine (F-Ara-A), decitabine, and chlorodeoxyadenosine (Cda, 2-Cda);
(9) epothilones such as BMS-247550 (Bristol-Myers Squibb), and EPO906 (Novartis Pharmaceuticals);
(10) topoisomerase inhibitors such as topotecan (Glaxo SmithKline), and Camptosar (Pharmacia);
(11) vinca alkaloids, such as, navelbine (Anvar and Fabre, France), vincristine and vinblastine;
(12) antibodies that are inhibitors of αVβ3 integrins, such as, LM-609 (see, Clinical Cancer Research, Vol. 6, page 3056-3061, August 2000, the disclosure of which is incorporated herein by reference thereto);
(13) folate antagonists, such as Methotrexate (MTX), and Premetrexed (Alimta);
(14) ribonucleotide reductase inhibitors, such as Hydroxyurea (HU);
(15) anthracyclines, such as Daunorubicin, Doxorubicin (Adriamycin), and Idarubicin;
(16) biologics, such as interferon (e.g., Intron-A and Roferon), pegylated interferon (e.g., Peg-Intron and Pegasys), and Rituximab (Rituxan, antibody used for the treatment of non-Hodgkin's lymphoma);
(17) thalidomide (or related imid);
(18) Bcr/abl kinase inhibitors, such as, for example Gleevec (STI-571), AMN-17, ONO12380, SU11248 (Sunitinib) and BMS-354825
(19) MEK1 and/or MEK2 inhibitors, such as PD0325901 and Arry-142886 (AZD6244);
(20) IGF-1 and IGF-2 inhibitors that are small molecules, such as, for example, NVP-AEW541;
(21) small molecule inhibitors of RAF and BRAF kinases, such as, for example, BAY 43-9006 (Sorafenib);
(22) small molecule inhibitors of cell cycle dependent kinases such as CDK1, CDK2, CDK4 and CDK6, such as, for example, CYC202, BMS387032, and Flavopiridol;
(23) alkylating agents, such as, for example, Temodar® brand of temozolomide;
(24) farnesyl protein transferase inhibitors, such as, for example:
   (a) Sarasar® brand of lonifarnib (i.e., 4-[2-[4-(3,10-dibromo-8-chloro-6,11-dihydro-5H-benzo[5,6]cyclohepta[1,2-*b*]byridin-11-yl)-1-piperidinyl)-2-oxoethyl]-1-piperidinecarboxamide, see for example, U.S. 5,874,442 issued February 23, 1999, and U.S. 6,632,455 issued October 14, 2003 the disclosures of each being incorporated herein by reference thereto),
   (b) Zarnestra® brand of tipifarnib (i.e., (R)-6-amino[(4-chlorophenyl)(1-methyl-1H-imidazol-5-yl)methyl]-4-(3-chlorophenyl)-1-methyl-2(1H)-quinolinone, see for example, WO 97/16443 published May 9, 1997 and U.S. 5,968,952 issued October 19, 1999, the disclosures of each being incorporated herein by reference thereto), and
   (c) Bristol-Myers Squibb 214662: 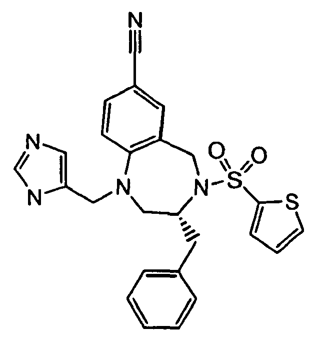
(see WO97/30992 published August 28, 1997, U.S. 6,011,029 issued January 4, 2000, and U.S. 6,455,523, the disclosures of each being incorporated herein by reference thereto).

The Bcr/abl kinase inhibitors, EGF receptor inhibitors, and HER-2 antibodies (EGF receptor inhibitors that are antibodies) described above are also known as signal transduction inhibitors. Therefore, chemotherapeutic agents, as used herein, include signal transduction inhibitors.

Typical signal transduction inhibitors, that are chemotherapeutic agents, include but are not limited to: (i) Bcr/abl kinase inhibitors such as, for example, STI 571 (Gleevec), (ii) Epidermal growth factor (EGF) receptor inhibitor such as, for example, Kinase inhibitors (Iressa, OSI-774) and antibodies (Imclone: C225 [Goldstein et al. (1995), Clin Cancer Res. 1:1311-1318], and Abgenix: ABX-EGF) and (iii) HER-2/neu receptor inhibitors such as, for example, Herceptin® (trastuzumab).

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA), the Physician's Desk Reference, 56th Edition, 2002 (published by Medical Economics company, Inc. Montvale, NJ 07645-1742), and the Physician's Desk Reference, 57th Edition, 2003 (published by Thompson PDR, Montvale, NJ 07645-1742); the disclosures of which is incorporated herein by reference thereto.

For example, the compound of formula 1.0 (e.g., a pharmaceutical composition comprising the compound of formula 1.0); can be administered orally (e.g., as a capsule), and the chemotherapeutic agents can be administered intravenously, usually as an IV solution. The use of a pharmaceutical composition comprising more than one drug is within the scope of this invention.

The compound of formula 1.0 and the chemotherapeutic agents are administered in therapeutically effective dosages to obtain clinically acceptable results, e.g., reduction or elimination of symptoms or of the tumor. Thus, the compound of formula 1.0 and chemotherapeutic agents can be administered concurrently or consecutively in a treatment protocol. The administration of the chemotherapeutic agents can be made according to treatment protocols already known in the art.

In general when more than one chemotherapeutic agent is used in the uses of this invention, the chemotherapeutic agents are for administration on the same day either concurrently or consecutively in their standard dosage form. For example, the chemotherapeutic agents are usually administered intravenously, preferably by an IV drip using IV solutions well known in the art (e.g., isotonic saline (0.9% NaCl) or dextrose solution (e.g., 5% dextrose)).

When two or more chemotherapeutic agents are used, the chemotherapeutic agents are generally administered on the same day; however, those skilled in the art will appreciate that the chemotherapeutic agents can be administered on different days and in different weeks. The skilled clinician can administer the chemotherapeutic agents according to their recommended dosage schedule from the manufacturer of the agent and can adjust the schedule according to the needs of the patient, e.g., based on the patient's response to the treatment. For example, when gemcitabine is used in combination with a platinum coordinator compound, such as, for example, cisplatin, to treat lung cancer, both the gemcitabine and the cisplatin are given on the same day on day one of the treatment cycle, and then gemcitabine is given alone on day 8 and given alone again on day 15.

The compounds of this invention and chemotherapeutic agents can be administered in a treatment protocol that usually lasts one to seven weeks, and is repeated typically from 6 to 12 times. Generally the treatment protocol can last one to four weeks. Treatment protocols of one to three weeks can also be used. A treatment protocol of one to two weeks can also be used. During this treatment protocol or cycle the compounds of this invention can be administered daily while the chemotherapeutic agents can be administered one or more times a week. Generally, a compound of this invention can be administered daily (i.e., once per day), and in one embodiment twice per day, and the chemotherapeutic agent is administered once a week or once every three weeks. For example, the taxanes (e.g., Paclitaxel (e.g., Taxol^{®}) or Docetaxel (e.g.,Taxotere^{®})) can be administered once a week or once every three weeks.

However, those skilled in the art will appreciate that treatment protocols can be varied according to the needs of the patient. Thus, the combination of compounds (drugs) used in the uses of this invention can be administered in variations of the protocols described above. For example, the compounds of this invention can be administered discontinuously rather than continuously during the treatment cycle. Thus, for example, during the treatment cycle the compounds of this invention can be administered daily for a week and then discontinued for a week, with this administration repeating during the treatment cycle. Or the compounds of this invention can be administered daily for two weeks and discontinued for a week, with this administration repeating during the treatment cycle. Thus, the compounds of this invention can be administered daily for one or more weeks during the cycle and discontinued for one or more weeks during the cycle, with this pattern of administration repeating during the treatment cycle. This discontinuous treatment can also be based upon numbers of days rather than a full week. For example, daily dosing for 1 to 6 days, no dosing for 1 to 6 days with this pattern repeating during the treatment protocol. The number of days (or weeks) wherein the compounds of this invention are not dosed do not have to equal the number of days (or weeks) wherein the compounds of this invention are dosed. Usually, if a discontinuous dosing protocol is used, the number of days or weeks that the compounds of this invention are dosed is at least equal or greater than the number of days or weeks that the compounds of this invention are not dosed.

The chemotherapeutic agent could be given by bolus or continuous infusion. The chemotherapeutic agent could be given daily to once every week, or once every two weeks, or once every three weeks, or once every four weeks during the treatment cycle. If administered daily during a treatment cycle, this daily dosing can be discontinuous over the number of weeks of the treatment cycle. For example, dosed for a week (or a number of days), no dosing for a week (or a number of days, with the pattern repeating during the treatment cycle.

The compounds of this invention can be administered orally, preferably as a solid dosage form, and in one embodiment as a capsule, and while the total therapeutically effective daily dose can be administered in one to four, or one to two divided doses per day, generally, the therapeutically effective dose is given once or twice a day, and in one embodiment twice a day. The compounds of this invention can be administered in an amount of about 50 to about 400 mg once per day, and can be administered in an amount of about 50 to about 300 mg once per day. The compounds of this invention are generally administered in an amount of about 50 to about 350 mg twice a day, usually 50 mg to about 200 mg twice a day, and in one embodiment about 75 mg to about 125 mg administered twice a day, and in another embodiment about 100 mg administered twice a day.

If the patient is responding, or is stable, after completion of the therapy cycle, the therapy cycle can be repeated according to the judgment of the skilled clinician. Upon completion of the therapy cycles, the patient can be continued on the compounds of this invention at the same dose that was administered in the treatment protocol, or, if the dose was less than 200mg twice a day, the dose can be raised to 200 mg twice a day. This maintenance dose can be continued until the patient progresses or can no longer tolerate the dose (in which case the dose can be reduced and the patient can be continued on the reduced dose).

The chemotherapeutic agents, used with the compounds of this invention, are administered in their normally prescribed dosages during the treatment cycle (i.e., the chemotherapeutic agents are administered according to the standard of practice for the administration of these drugs). For example: (a) about 30 to about 300 mg/m² for the taxanes; (b) about 30 to about 100 mg/m² for Cisplatin; (c) AUC of about 2 to about 8 for Carboplatin; (d) about 2 to about 4 mg/m² for EGF inhibitors that are antibodies; (e) about 50 to about 500 mg/m² for EGF inhibitors that are small molecules; (f) about 1 to about 10 mg/m² for VEGF kinase inhibitors that are antibodies; (g) about 50 to about 2400 mg/m² for VEGF inhibitors that are small molecules; (h) about 1 to about 20 mg for SERMs; (i) about 500 to about 1250 mg/m² for the anti-tumor nucleosides 5-Fluorouracil, Gemcitabine and Capecitabine; (j) for the anti-tumor nucleoside Cytarabine (Ara-C) 100-200mg/m²/day for 7 to 10 days every 3 to 4 weeks, and high doses for refractory leukemia and lymphoma, i.e., 1 to 3 gm/m² for one hour every 12 hours for 4-8 doses every 3 to four weeks; (k) for the anti-tumor nucleoside Fludarabine (F-ara-A) 10-25mg/m²/day every 3 to 4 weeks; (I) for the anti-tumor nucleoside Decitabine 30 to 75 mg/m² for three days every 6 weeks for a maximum of 8 cycles; (m) for the anti-tumor nucleoside Chlorodeoxyadenosine (CdA, 2-CdA) 0.05-0.1 mg/kg/day as continuous infusion for up to 7 days every 3 to 4 weeks; (n) about 1 to about 100 mg/m² for epothilones; (o) about 1 to about 350 mg/m² for topoisomerase inhibitors; (p) about 1 to about 50 mg/m² for vinca alkaloids; (q) for the folate antagonist Methotrexate (MTX) 20-60 mg/m² by oral, IV or IM every 3 to 4 weeks, the intermediate dose regimen is 80-250 mg/m² IV over 60 minutes every 3 to 4 weeks, and the high dose regimen is 250-1000mg/m² IV given with leucovorin every 3 to 4 weeks; (r) for the folate antagonist Premetrexed (Alimta) 300-600 mg/m² (10 minutes IV infusion day 1) every 3 weeks; (s) for the ribonucleotide reductase inhibitor Hydroxyurea (HU) 20-50 mg/kg/day (as needed to bring blood cell counts down); (t) the platinum coordinator compound Oxaliplatin (Eloxatin) 50-100 mg/m² every 3 to 4 weeks (preferably used for solid tumors such as non-small cell lung cancer, colorectal cancer and ovarian cancer); (u) for the anthracycline daunorubicin 10-50 mg/m²/day IV for 3-5 days every 3 to 4 weeks; (v) for the anthracycline Doxorubicin (Adriamycin) 50-100 mg/m² IV continuous infusion over 1-4 days every 3 to 4 weeks, or 10-40 mg/m² IV weekly; (w) for the anthracycline Idarubicin 10-30 mg/m² daily for 1-3 days as a slow IV infusion over 10-20 minutes every 3 to 4 weeks; (x) for the biologic interferon (Intron-A, Roferon) 5 to 20 million IU three times per week; (y) for the biologic pegylated interferon (Peg-intron, Pegasys) 3 to 4 micrograms/kg/day chronic sub cutaneous (until relapse or loss of activity); (z) for the biologic Rituximab (Rituxan) (antibody used for non-Hodgkin's lymphoma) 200-400mg/m² IV weekly over 4-8 weeks for 6 months; (aa) for the alkylating agent temozolomide 75 mg/m² to 250mg/m², for example, 150 mg/m², or for example, 200 mg/m², such as 200mg/m² for 5 days; and (bb) for the MEK1 and/or MEK2 inhibitor PD0325901, 15 mg to 30 mg, for example, 15 mg daily for 21 days every 4 weeks.

Gleevec can be used orally in an amount of about 200 to about 800 mg/day. Thalidomide (and related imids) can be used orally in amounts of about 200 to about 800 mg/day, and can be contiuously dosed or used until releapse or toxicity. See for example Mitsiades et al., "Apoptotic signaling induced by immunomodulatory thalidomide analoqs in human multiple myeloma cells;therapeutic implications", Blood, 99(12):4525-30, June 15, 2002, the disclosure of which is incorporated herein by reference thereto.

The FPT inhibitor Sarasar® (brand of lonifarnib) can be administered orally (e.g., capsule) in amounts of about 50 to about 200 mg given twice a day, or in amounts of about 75 to about 125 mg given twice a day, or in amounts of about 100 to about 200 mg given twice a day, or in an amount of about 100 mg given twice a day.

Paclitaxel (e.g., Taxol%, for example, can be administered once per week in an amount of about 50 to about 100 mg/m² and in another example about 60 to about 80 mg/m². In another example Paclitaxel (e.g., Taxol^{®}) can be administered once every three weeks in an amount of about 150 to about 250 mg/m² and in another example about 175 to about 225 mg/m².

In another example, Docetaxel (e.g., Taxotere^{®}) can be administered once per week in an amount of about 10 to about 45 mg/m². In another example Docetaxel (e.g., Taxotere^{®}) can be administered once every three weeks in an amount of about 50 to about 100 mg/m².

In another example Cisplatin can be administered once per week in an amount of about 20 to about 40 mg/m². In another example Cisplatin can be administered once every three weeks in an amount of about 60 to about 100 mg/m².

In another example Carboplatin can be administered once per week in an amount to provide an AUC of about 2 to about 3. In another example Carboplatin can be administered once every three weeks in an amount to provide an AUC of about 5 to about 8.

Other embodiments of this invention are described below. The embodiments have been numbered for the purpose of making it easier to refer to the embodiments. The term "in any one of Embodiment Nos." or the term "of any of Embodiment Nos.", as used below, means that the particular embodiment using that term is intended to cover any one of the embodiments referred to as if any one of the referred to embodiments had been individually described. "Nos." is an abbreviation for Numbers.

Embodiment No. 1 is directed to a compound of formula 1.0, preferably a compound of formula 1.0C, wherein Q is selected from the group consisting of substituents 2.1, 2.2, 2.3, 2.3A, 2.3B, 2.3C, 2.4A, 2.4B, 2.4C, 2.5A, 2.5B, 2.5C, 2.6A, 2.7A, 2.7B, 2.7C, 2.8A, 2.8B, 2.8C, 2.9 to 2.14, 2.15, 2.16 (e.g., 2.16A or 2.16B), 2.17, 2.17A, 2.17B, 2.17C, 2.17D, 2.17E, 2.18, 2.19, 2.20, 2.21 and 2.22.

Embodiment No. 2 is directed to a compound of formula 1.1 wherein Q is selected from the group consisting of substituents 2.1, 2.2, 2.3, 2.3A, 2.3B, 2.3C, 2.4A, 2.4B, 2.4C, 2.5A, 2.5B, 2.5C, 2.6A, 2.7A, 2.7B, 2.7C, 2.8A, 2.8B, 2.8C, 2.9 to 2.14, 2.15, 2.16 (e.g., 2.16A or 2.16B), 2.17, 2.17A, 2.17B, 2.17C, 2.17D, 2.17E, 2.18, 2.19, 2.20, 2.21 and 2.22.

Embodiment No. 3 is directed to a compound of formula 1.2 wherein Q is selected from the group consisting of substituents 2.1, 2.2, 2.3, 2.3A, 2.3B, 2.3C, 2.4A, 2.4B, 2.4C, 2.5A, 2.5B, 2.5C, 2.6A, 2.7A, 2.7B, 2.7C, 2.8A, 2.8B, 2.8C, 2.9 to 2.14, 2.15, 2.16 (e.g., 2.16A or 2.16B), 2.17, 2.17A, 2.17B, 2.17C, 2.17D, 2.17E, 2.18, 2.19, 2.20, 2.21 and 2.22.

Embodiment No. 4 is directed to a compound of formula 1.3 wherein Q is selected from the group consisting of substituents 2.1, 2.2, 2.3, 2.3A, 2.3B, 2.3C, 2.4A, 2.4B, 2.4C, 2.5A, 2.5B, 2.5C, 2.6A, 2.7A, 2.7B, 2.7C, 2.8A, 2.8B, 2.8C, 2.9 to 2.14, 2.15, 2.16 (e.g., 2.16A or 2.16B), 2.17, 2.17A, 2.17B, 2.17C, 2.17D, 2.17E, 2.18, 2.19, 2.20, 2.21 and 2.22.

Embodiment No. 5 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.1.

Embodiment No. 6 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.2.

Embodiment No. 7 is directed to a compound of formula 1.0 (e.g., 1.1, 1.1A, 1.2, 1.2A, 1.3 or 1.3A) wherein substituent Q is 2.3 (e.g., 2.3A, 2.3B or 2.3C).

Embodiment No. 8 is directed to a compound of formula 1.0, preferably a compound of formula 1.0C, (e.g., 1.1, 1.1A, 1.2, 1.2A. 1.3 or 1.3A) wherein substituent Q is 2.4 (e.g., 2.4A, 2.4B or 2.4C).

Embodiment No. 9 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.5 (e.g., 2.5A, 2.5B or 2.5C).

Embodiment No. 10 is directed to any of compounds of formulas to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.6 (e.g., 2.6A).

Embodiment No. 11 is directed to a compound of formula 1.0, preferably a compound of formula 1.0C1 and more preferably a compound of formula 1.0C, (e.g., 1.1, 1.1 A, 1.2, 1.2A, 1.3 or 1.3A) wherein substituent Q is 2.7.

Embodiment No. 12 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.8.

Embodiment No. 13 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.9.

Embodiment No. 14 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.10.

Embodiment No. 15 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.11.

Embodiment No. 16 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.12.

Embodiment No. 17 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.13.

Embodiment No. 18 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.14.

Embodiment No. 19 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.15.

Embodiment No. 20 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.16.

Embodiment No. 21 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.17 (e.g., 2.17A, 2.17B, 2.17C, 2.17D, or 2.17E).

Embodiment No. 22 is directed to a compound of formula 1.0, preferably a compound of formula 1.0C1 and more preferably a compound of formula 1.0C, (e.g., 1.1, 1.1 A, 1.2, 1.2A, 1.3 or 1.3A) wherein substituent Q is 2.18.

Embodiment No. 23 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.19.

Embodiment No. 24 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.20.

Embodiment No. 25 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.21.

Embodiment No. 26 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is 2.22.

Embodiment No. 27 is directed to a compound of formula 1.3 wherein substituent Q is 2.1.

Embodiment No. 28 is directed to a compound of formula 1.3 wherein substituent Q is 2.2.

Embodiment No. 29 is directed to a compound of formula 1.3 wherein substituent Q is 2.3.

Embodiment No. 30 is directed to a compound of formula 1.3 wherein substituent Q is 2.6.

Embodiment No. 31 is directed to a compound of formula 1.3 wherein substituent Q is 2.6A.

Embodiment No. 32 is directed to a compound of formula 1.3 wherein substituent Q is 2.7A.

Embodiment No. 33 is directed to a compound of formula 1.3 wherein substituent Q is 2.7B.

Embodiment No. 34 is directed to a compound of formula 1.3 wherein substituent Q is 2.7C.

Embodiment No. 35 is directed to a compound of formula 1.3 wherein substituent Q is 2.17.

Embodiment No. 36 is directed to a compound of formula 1.3 wherein substituent Q is 2.17A.

Embodiment No. 37 is directed to a compound of formula 1.3 wherein substituent Q is 2.17B.

Embodiment No. 38 is directed to a compound of formula 1.3 wherein substituent Q is 2.17C.

Embodiment No. 39 is directed to a compound of formula 1.3 wherein substituent Q is 2.17D.

Embodiment No. 40 is directed to a compound of formula 1.3 wherein substituent Q is 2.17E.

Embodiment No. 41 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein each R³, R⁴, R⁶, and R⁷ is independently selected from the group consisting of: H and alkyl.

Embodiment No. 42 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein each R³, R⁴, R⁶, and R⁷ is independently selected from the group consisting of: H and methyl.

Embodiment No. 43 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein each R³, R⁴, R⁶, and R⁷ is H.

Embodiment No. 44 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.1, 2.2, 2.3A, 2.3B, and 2.3C.

Embodiment No. 45 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.1, 2.2, 2.3A, 2.3B, and 2.3C, and each R³, R⁴, R⁶, and R⁷ is independently selected from the group consisting of: H and alkyl.

Embodiment No. 46 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2_{.}1, 2.2, 2.3A, 2.3B, and 2.3C, and each R³, R⁴, R⁶, and R⁷ is independently selected from the group consisting of: H and methyl.

Embodiment No. 47 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.1, 2.2, 2.3A, 2.3B, and 2.3C, and each R³, R⁴, R⁶, and R⁷ is H.

Embodiment No. 48 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moiety 2.17.

Embodiment No. 49 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moiety 2.17, and each R³, R⁴, R⁶, and R⁷ is independently selected from the group consisting of: H and alkyl.

Embodiment No. 50 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moiety 2.17, and each R³, R⁴, R⁶, and R⁷ is independently selected from the group consisting of: H and methyl.

Embodiment No. 51 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moiety 2.17, and each R³, R⁴, R⁶, and R⁷ is H.

Embodiment No. 52 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.1, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 53 is directed to a compound of formula 1.0, preferably a compound of formula 1.0C1 and more preferably a compound of formula 1.0C, (e.g., 1.1, 1.1 A, 1.2, 1.2A, 1.3 or 1.3A) wherein substituent Q is selected from the group consisting of: moieties 2.2, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 54 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.3A, 2.3B, 2.3C, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 55 is directed to a compound of formula 1.2 or 1.3 wherein each R³, R⁴, R⁶, and R⁷ is independently selected from the group consisting of: H and alkyl.

Embodiment No. 56 is directed to a compound of formula 1.2 or 1.3 wherein each R³, R⁴, R⁶, and R⁷ is independently selected from the group consisting of: H and methyl.

Embodiment No. 57 is directed to a compound of formula 1.2 or 1.3 wherein each R³, R⁴, R⁶, and R⁷ is H.

Embodiment No. 58 is directed to a compound of formula 1.2 or 1.3 wherein substituent Q is selected from the group consisting of: moieties 2.1, 2.2, 2.3A, 2.3B and 2.3C.

Embodiment No. 59 is directed to a compound of formula 1.2 or 1.3 wherein substituent Q is selected from the group consisting of: moieties 2.1, 2.2, 2.3A, 2.3B and 2.3C, and each R³, R⁴, R⁶, and R⁷ is independently selected from the group consisting of: H and alkyl.

Embodiment No. 60 is directed to a compound of formula 1.2 or 1.3 wherein substituent Q is selected from the group consisting of: moieties 2.1, 2.2, 2.3A, 2.3B and 2.3C, and each R³, R⁴, R⁶, and R⁷ is independently selected from the group consisting of: H and methyl.

Embodiment No. 61 is directed to a compound of formula 1.2 or 1.3 wherein substituent Q is selected from the group consisting of: moieties 2.1, 2.2, 2.3A, 2.3B and 2.3C, and each R³, R⁴, R⁶, and R⁷ is H.

Embodiment No. 62 is directed to a compound of formula 1.2 or 1.3 wherein substituent Q is moiety 2.17.

Embodiment No. 63 is directed to a compound of formula 1.2 or 1.3 wherein substituent Q is moiety 2.17, and each R³, R⁴, R⁶, and R⁷ is independently selected from the group consisting of: H and alkyl.

Embodiment No. 64 is directed to a compound of formula 1.2 or 1.3 wherein substituent Q is moiety 2.17, and each R³, R⁴, R⁶, and R⁷ is independently selected from the group consisting of: H and methyl.

Embodiment No. 65 is directed to a compound of formula 1.2 or 1.3 wherein substituent Q is moiety 2.17, and each R³, R⁴, R⁶, and R⁷ is H.

Embodiment No. 66 is directed to a compound of formula 1.2 or 1.3 wherein substituent Q is selected from the group consisting of: moiety 2.1, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 67 is directed to a compound of formula 1.2 or 1.3 wherein substituent Q is selected from the group consisting of: moiety 2.2, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 68 is directed to a compound of formula 1.2 or 1.3 wherein substituent Q is selected from the group consisting of: moieties 2.3A, 2.3B and 2.3C, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 69 is directed to a compound of formula 1.3 wherein each R³, R⁴, R⁶, and R⁷ is independently selected from the group consisting of: H and alkyl.

Embodiment No. 70 is directed to a compound of formula 1.3 wherein each R³, R⁴, R⁶, and R⁷ is independently selected from the group consisting of: H and methyl.

Embodiment No. 71 is directed to a compound of formula 1.3 wherein each R³, R⁴, R⁶, and R⁷ is H.

Embodiment No. 72 is directed to a compound of formula 1.3 wherein substituent Q is selected from the group consisting of: moieties 2.1, 2.2, 2.3A, 2.3B and 2.3C.

Embodiment No. 73 is directed to a compound of formula 1.3 wherein substituent Q is selected from the group consisting of: moieties 2.1, 2.2, 2.3A, 2.3B and 2.3C, and each R³, R⁴, R⁶, and R⁷ is independently selected from the group consisting of: H and alkyl.

Embodiment No. 74 is directed to a compound of formula 1.3 wherein substituent Q is selected from the group consisting of: moieties 2.1, 2.2, 2.3A, 2.3B and 2.3C, and each R³, R⁴, R⁶, and R⁷ is independently selected from the group consisting of: H and methyl.

Embodiment No. 75 is directed to a compound of formula 1.3 wherein substituent Q is selected from the group consisting of: moieties 2.1, 2.2, 2.3A, 2.3B and 2.3C, and each R³, R⁴, R⁶, and R⁷ is H.

Embodiment No. 76 is directed to a compound of formula 1.3 wherein substituent Q is moiety 2.17.

Embodiment No. 77 is directed to a compound of formula 1.3 wherein substituent Q is moiety 2.17, and each R³, R⁴, R⁶, and R⁷ is independently selected from the group consisting of: H and alkyl.

Embodiment No. 78 is directed to a compound of formula 1.3 wherein substituent Q is moiety 2.17, and each R³, R⁴, R⁶, and R⁷ is independently selected from the group consisting of: H and methyl.

Embodiment No. 79 is directed to a compound of formula 1.3 wherein substituent Q is moiety 2.17, and each R³, R⁴, R⁶, and R⁷ is H.

Embodiment No. 80 is directed to a compound of formula 1.3 wherein substituent Q is selected from the group consisting of: moiety 2.1, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 81 is directed to a compound of formula 1.3 wherein substituent Q is selected from the group consisting of: moiety 2.2, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 82 is directed to a compound of formula 1.3 wherein substituent Q is selected from the group consisting of: moieties 2.3A1, 2.3B and 2:3C, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 83 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.6, 2.7A, 2.7B and 2.7C.

Embodiment No. 84 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.6, 2.7A, 2.7B and 2.7C, and each R³, R⁴, R⁶, and R⁷ is independently selected from the group consisting of: H and alkyl.

Embodiment No. 85 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.6, 2.7A, 2.7B and 2.7C, and each R³, R⁴, R⁶, and R⁷ is independently selected from the group consisting of: H and methyl.

Embodiment No. 86 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.6, 2.7A, 2.7B and 2.7C, and each R³, R⁴, R⁶, and R⁷ is H.

Embodiment No. 87 is directed to a compound of formula 1.0, preferably a compound of formula 1.0C1 and more preferably a compound of formula 1.0C, (e.g., 1.1, 1.1 A, 1.2, 1.2A, 1.3 or 1.3A) wherein substituent Q is selected from the group consisting of: moiety 2.6, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 88 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moiety 2.7A, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 89 is directed to a compound of formula 1.0, preferably a compound of formula 1.0C1 and more preferably a compound of formula 1.0C, (e.g., 1.1, 1.1A, 1.2, 1.2A, 1.3 or 1.3A) wherein substituent Q is selected from the group consisting of: moieties 2.7B and 2.7C, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 90 is directed to a compound of formula 1.2 or 1.3 wherein substituent Q is selected from the group consisting of: moieties 2.6, 2.7A, 2.7B and 2.7C.

Embodiment No. 91 is directed to a compound of formula 1.2 or 1.3 wherein substituent Q is selected from the group consisting of: moieties 2.6, 2.7A, 2.7B and 2.7C, and each R³, R⁴, R⁶, and R⁷ is independently selected from the group consisting of: H and alkyl.

Embodiment No. 92 is directed to a compound of formula 1.2 or 1.3 wherein substituent Q is selected from the group consisting of: moieties 2.6, 2.7A, 2.7B and 2.7C, and each R³, R⁴, R⁶, and R⁷ is independently selected from the group consisting of: H and methyl.

Embodiment No. 93 is directed to a compound of formula 1.2 or 1.3 wherein substituent Q is selected from the group consisting of: moieties 2.6, 2.7A, 2.7B and 2.7C, and each R³, R⁴, R⁶ , and R⁷ is independently selected from the group consisting of: H.

Embodiment No. 94 is directed to a compound of formula 1.2 or 1.3 wherein substituent Q is selected from the group consisting of: moiety 2.6, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 95 is directed to a compound of formula 1.2 or 1.3 wherein substituent Q is selected from the group consisting of: moiety 2.7A, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 96 is directed to a compound of formula 1.2 or 1.3 wherein substituent Q is selected from the group consisting of: moieties 2.7A and 2.7B, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 97 is directed to a compound of formula 1.3 wherein substituent Q is selected from the group consisting of: moieties 2.6, 2.7A, 2.7B and 2.7C.

Embodiment No. 98 is directed to a compound of formula 1.3 wherein substituent Q is selected from the group consisting of: moieties 2.6, 2.7A, 2.7B and 2.7C, and each R³, R⁴, R⁶, and R⁷ is independently selected from the group consisting of: H and alkyl.

Embodiment No. 99 is directed to a compound of formula 1.3 wherein substituent Q is selected from the group consisting of: moieties 2.6, 2.7A, 2.7B and 2.7C, and each R³, R⁴, R⁶, and R⁷ is independently selected from the group consisting of: H and methyl.

Embodiment No. 100 is directed to a compound of formula 1.3 wherein substituent Q is selected from the group consisting of: moieties 2.6, 2.7A, 2.7B and 2.7C, and each R³, R⁴, R⁶, and R⁷ is H.

Embodiment No. 101 is directed to a compound of formula 1.3 wherein substituent Q is selected from the group consisting of: moiety 2.6, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 102 is directed to a compound of formula 1.3 wherein substituent Q is selected from the group consisting of: moiety 2.7A, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 103 is directed to a compound of formula 1.3 wherein substituent Q is selected from the group consisting of: moieties 2.7B and 2.7C, and: (1) each R³, R⁴, R⁶, and R⁷ is independently selected from the group consisting of: H and alkyl, or (2) each R³, R⁴, R⁶, and R⁷ is independently selected from the group consisting of: H and methyl, or (3) each R³, R⁴, R⁶, and R⁷ is H.

Embodiment No. 104 is directed to a compound of any one of Embodiment Nos. 1 to 103, wherein R¹ is selected from the group consisting of:

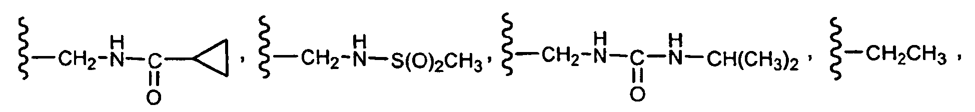

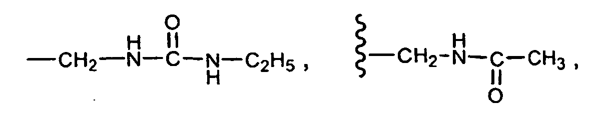

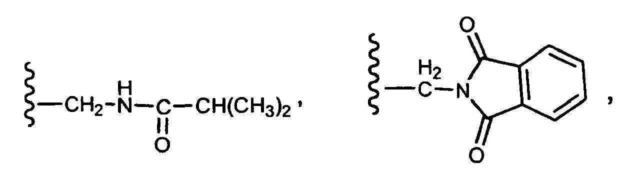

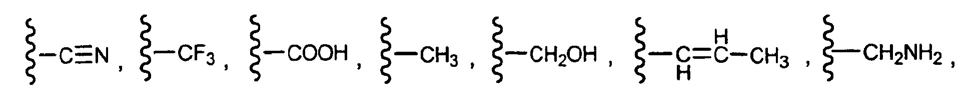

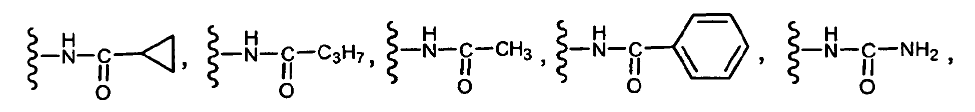

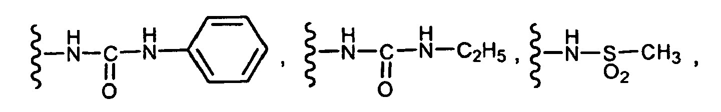

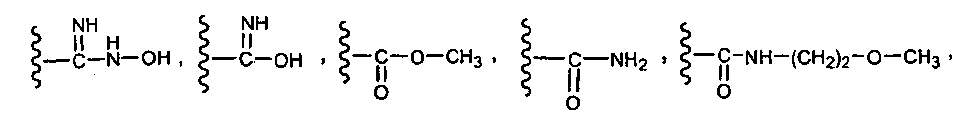

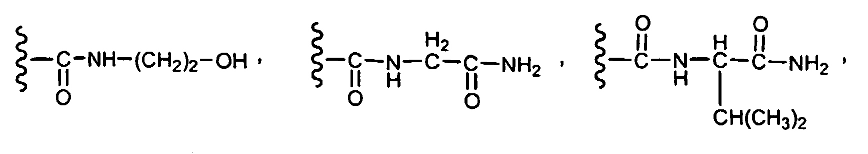

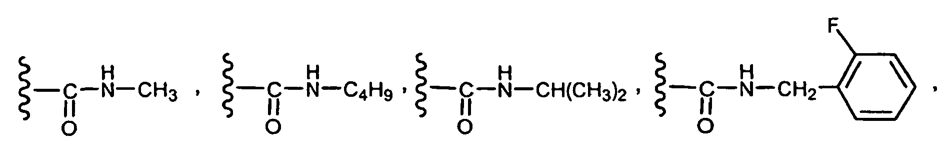

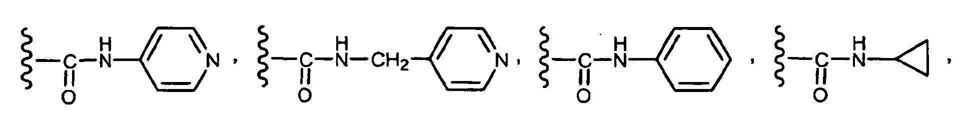

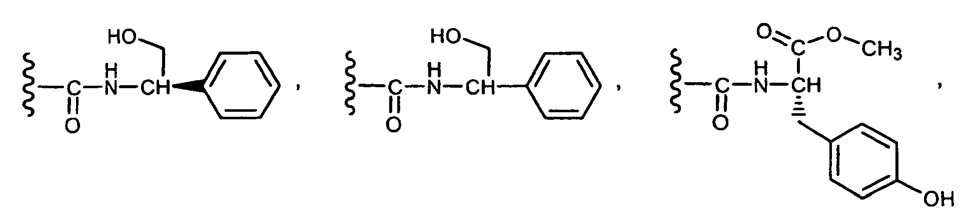

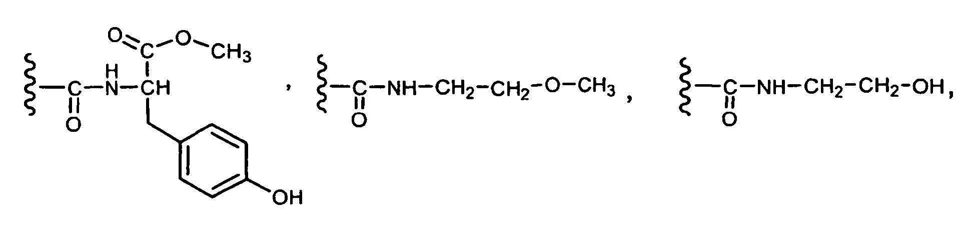

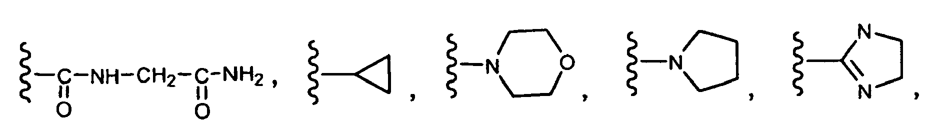

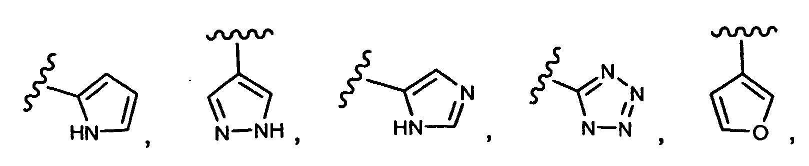

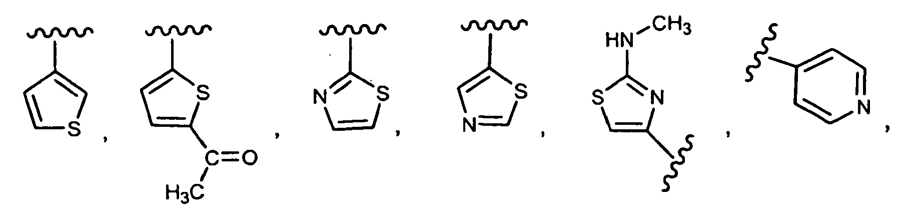

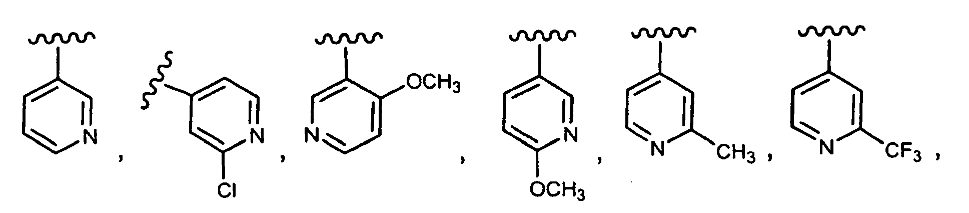

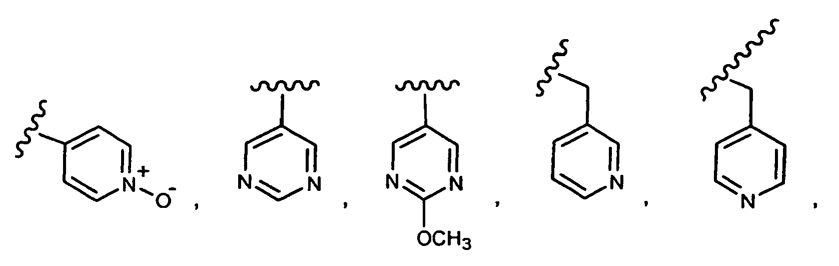

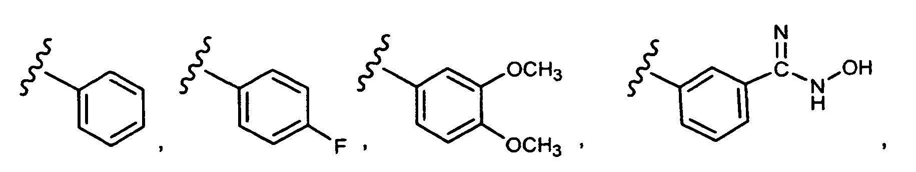

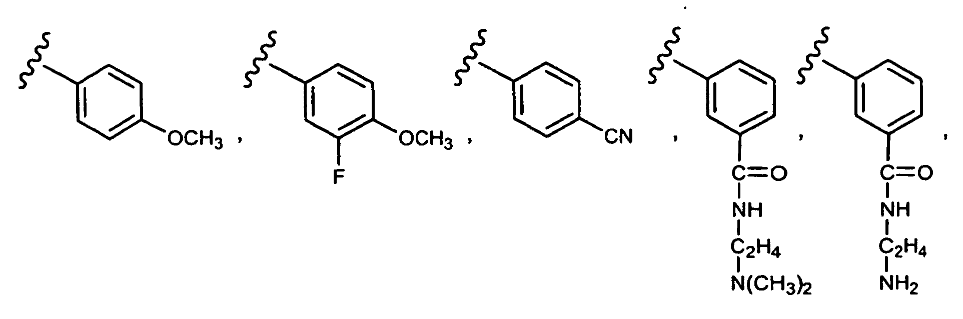

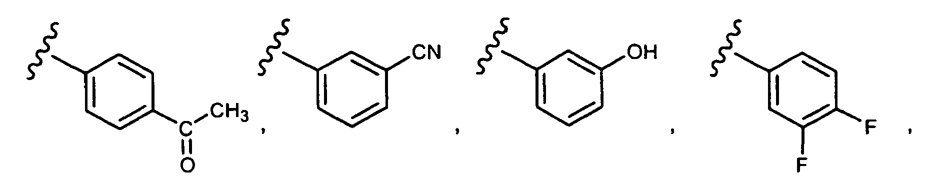

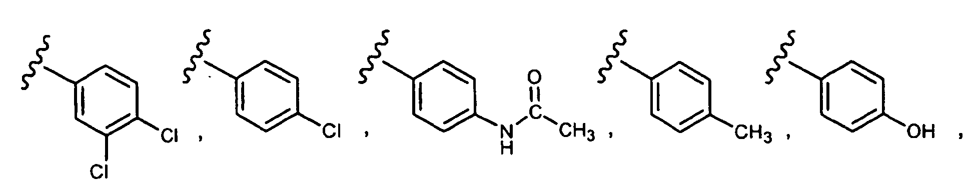

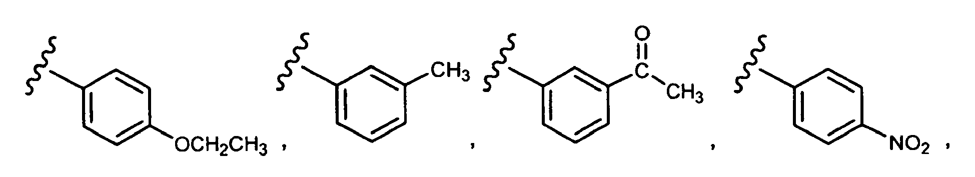

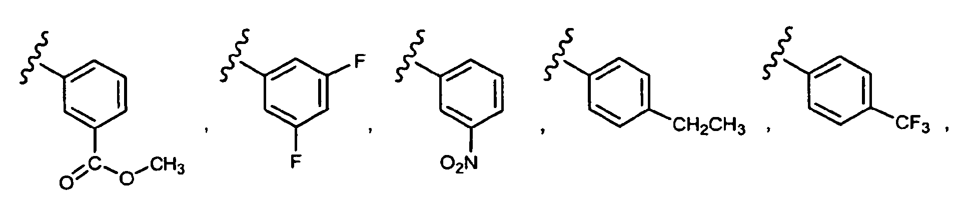

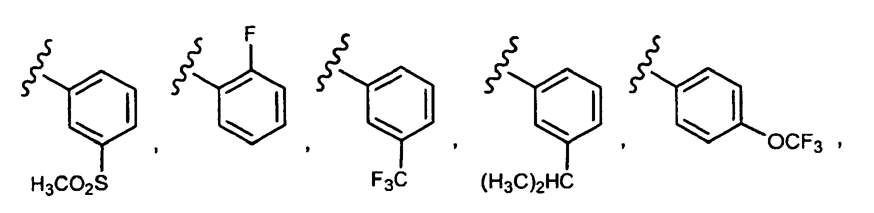

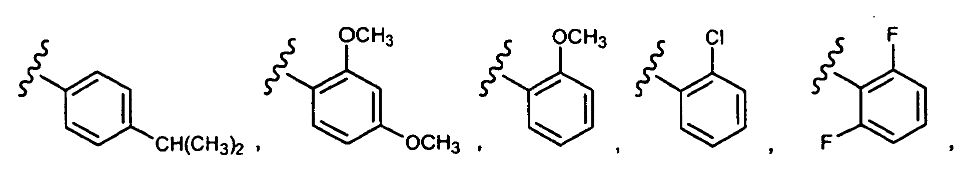

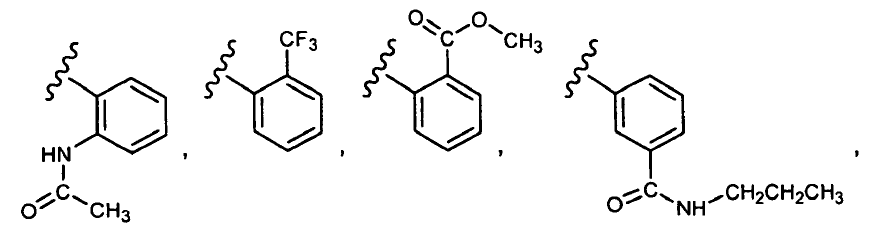

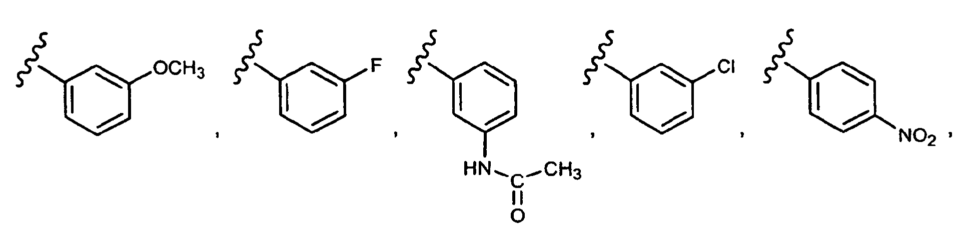

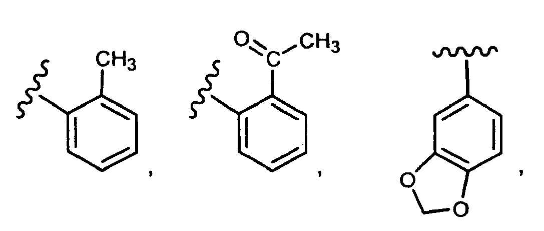

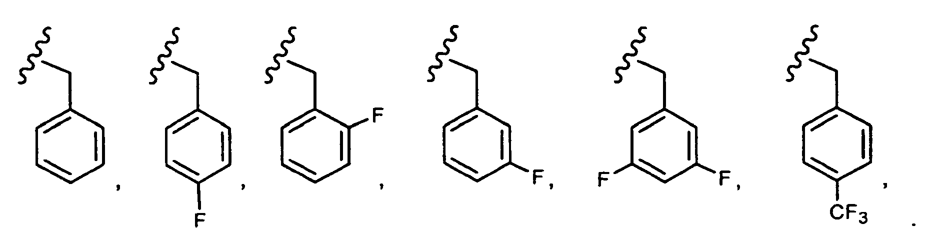

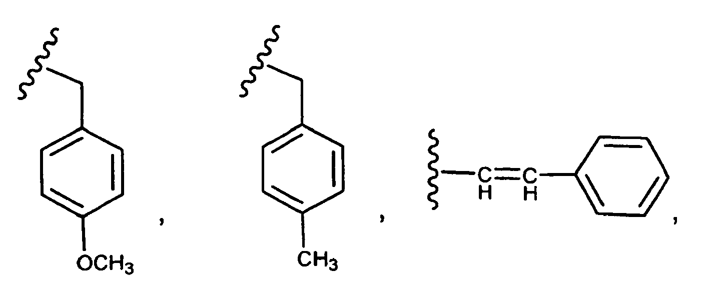

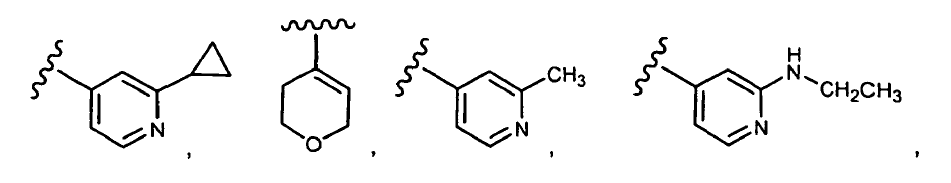

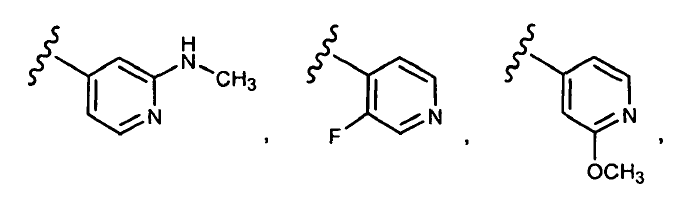

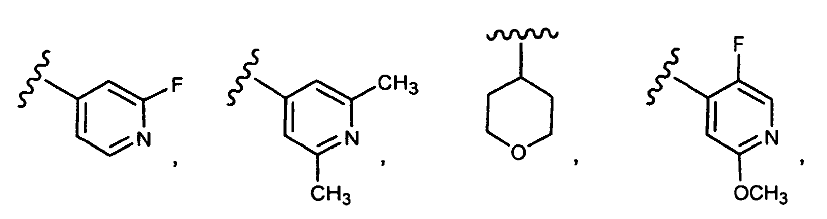

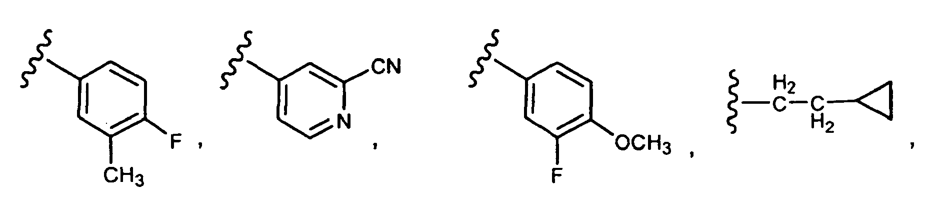

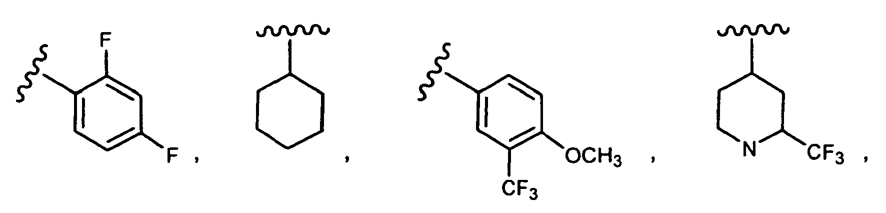

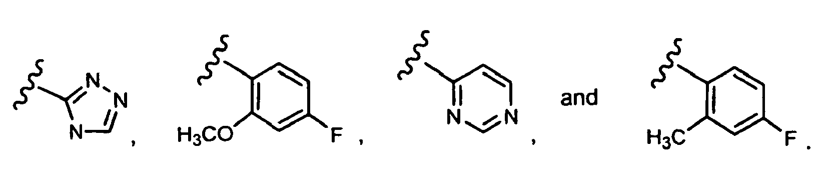

Embodiment No. 105 is directed to a compound of any one of Embodiment Nos. 1 to 103, wherein R¹ is aryl (e.g., phenyl).

Embodiment No. 106 is directed to a compound of any one of Embodiment Nos. 1 to 103, wherein R¹ is substituted aryl (e.g., substituted phenyl).

Embodiment No. 107 is directed to a compound of any one of Embodiment Nos. 1 to 103, wherein R¹ is heteroaryl (e.g., pyridyl, such as

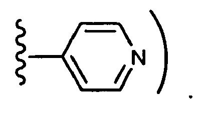

Embodiment No. 108 is directed to a compound of any one of Embodiment Nos. 1 to 103, wherein R¹ is substituted heteroaryl (e.g., substituted pyridyl).

Embodiment No. 109 is directed to a compound of any one of Embodiment Nos. 1 to 103, wherein R¹ is pyridyl substituted with cycloalkyl (e.g., cyclopropyl).

Embodiment No. 110 is directed to a compound of any one of Embodiment Nos. 1 to 103, wherein R¹ is pyridyl substituted with cyclopropyl.

Embodiment No. 111 is directed to a compound of any one of Embodiment Nos. 1 to 103, wherein R¹ is:

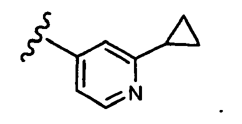

Embodiment No. 112 is directed to a compound of any one of Embodiment Nos. 1 to 103, wherein R¹ is phenyl substituted with halo.

Embodiment No. 113 is directed to a compound of any one of Embodiment Nos. 1 to 103, wherein R¹ is phenyl substituted with F.

Embodiment No. 114 is directed to a compound of any one of Embodiment Nos. 1 to 103, wherein R¹ is p-F-phenyl.

Embodiment No. 115 is directed to a compound of any one of Embodiment Nos. 1 to 103, wherein R¹ is pyridyl substituted with -CF₃.

Embodiment No. 116 is directed to a compound of any one of Embodiment Nos. 1 to 103, wherein R¹ is:

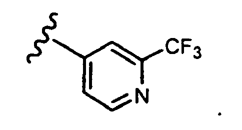

Embodiment No. 117 is directed to a compound of any one of Embodiment Nos. 1 to 103, wherein R¹ is pyridyl substituted with alkyl.

Embodiment No. 118 is directed to a compound of any one of Embodiment Nos. 1 to 103, wherein R¹ is pyridyl substituted with methyl.

Embodiment No. 119 is directed to a compound of any one of Embodiment Nos. 1 to 103, wherein R¹ is:

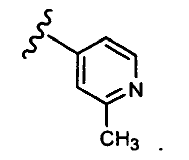

Embodiment No. 120 is directed to a compound of any one of Embodiment Nos. 1 to 103 wherein R⁵ is selected from the group consisting of:

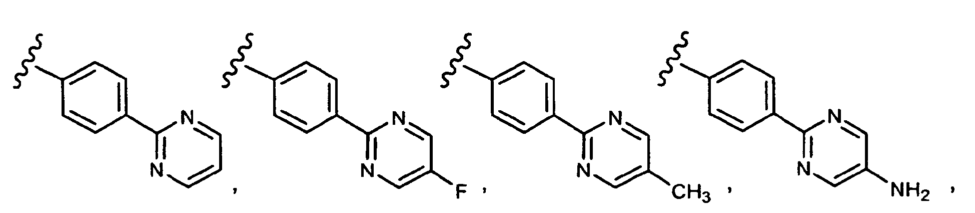

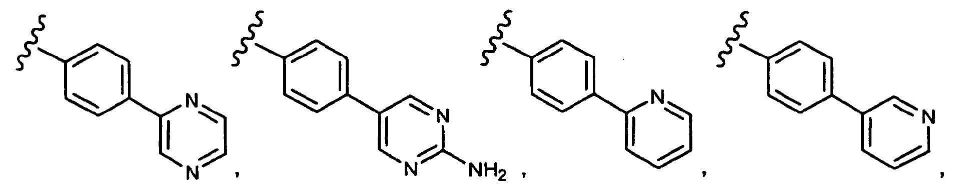

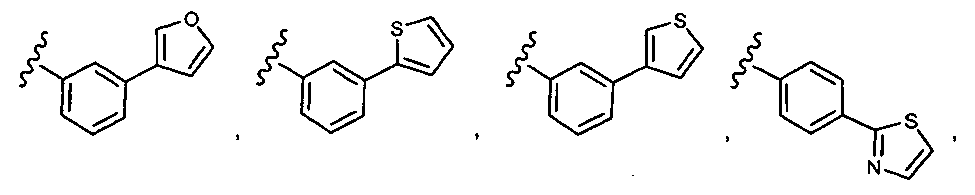

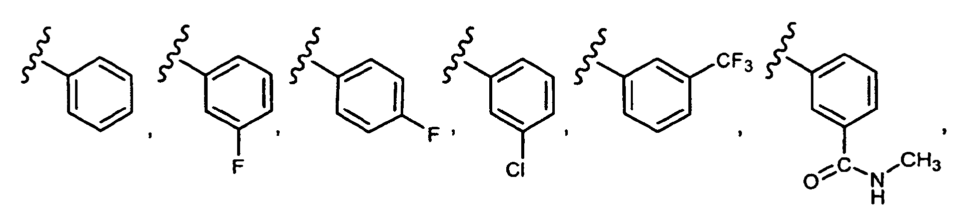

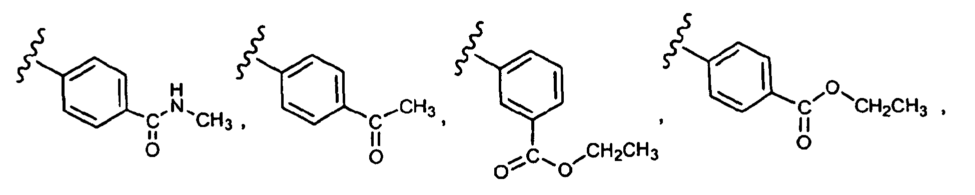

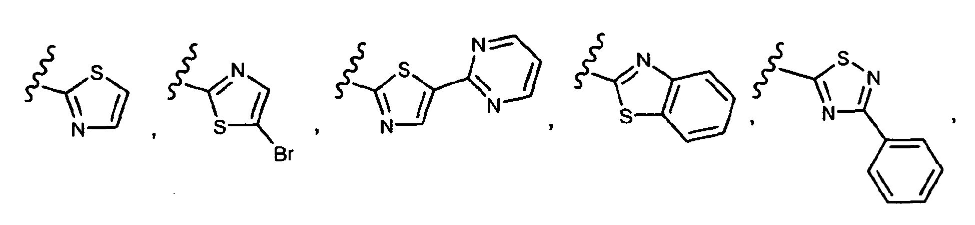

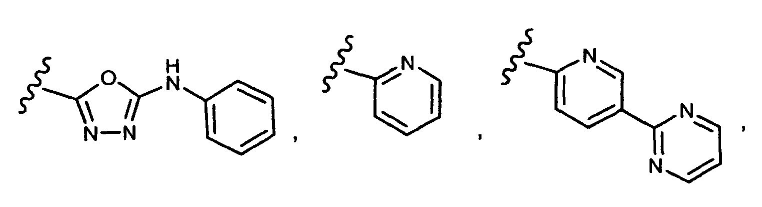

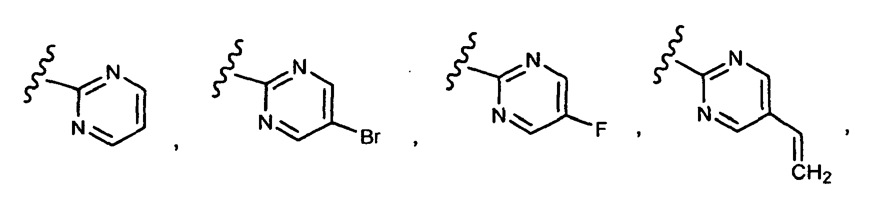

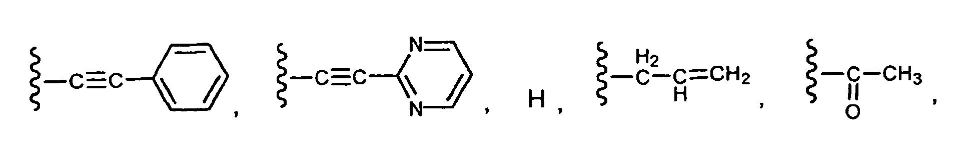

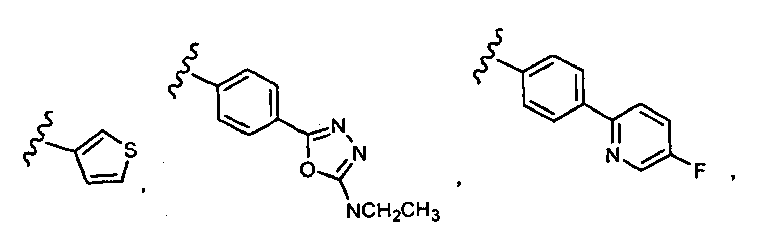

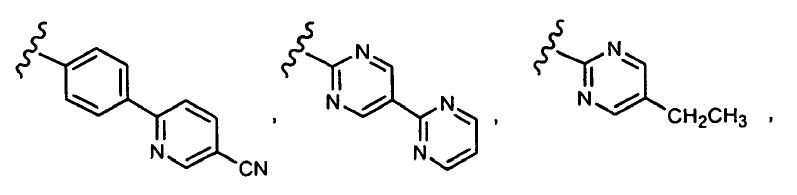

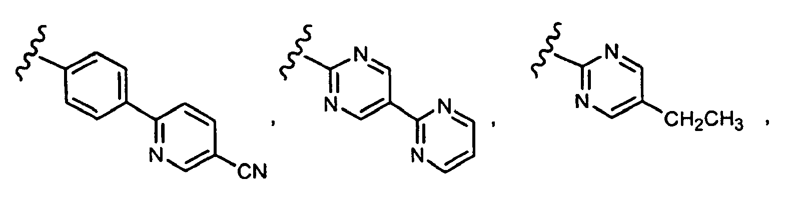

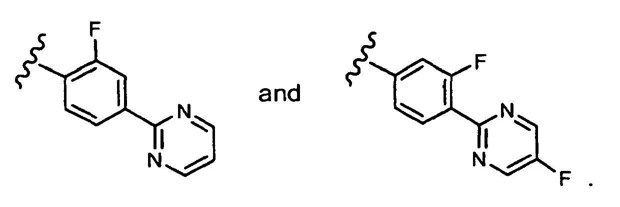

Embodiment No. 121 is directed to a compound of any one of Embodiment Nos. 1 to 103 wherein R⁵ is selected from the group consisting of:

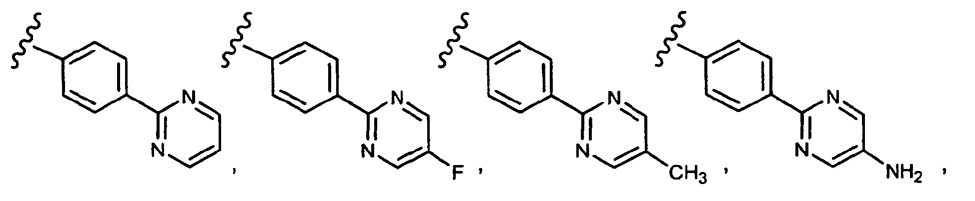

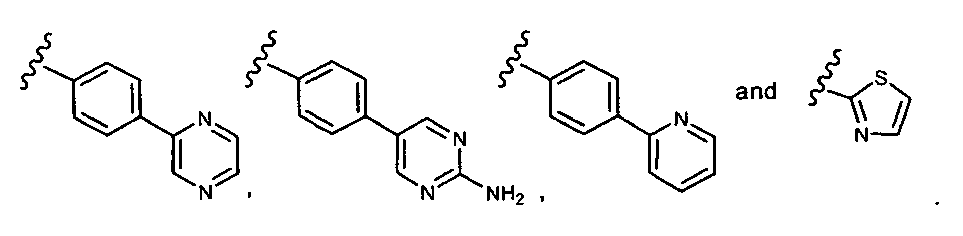

Embodiment No. 122 is directed to a compound of any one of Embodiment Nos. 1 to 103 wherein R⁵ is selected from the group consisting of:

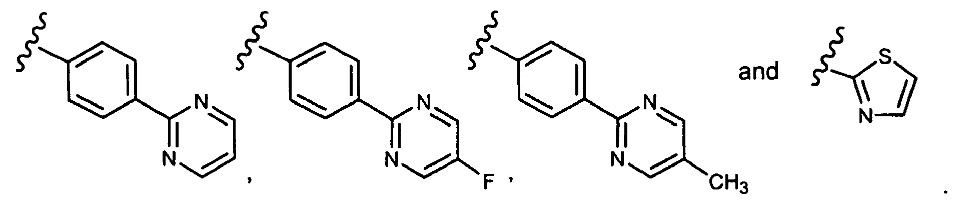

Embodiment No. 123 is directed to a compound of any one of Embodiment Nos. 1 to 103 wherein R⁵ is

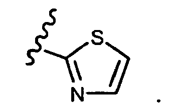

Embodiment No. 124 is directed to a compound of any one of Embodiment Nos. 1 to 103 wherein R⁵ is

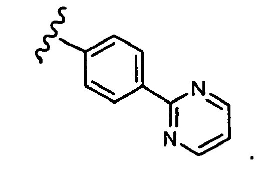

Embodiment No. 125 is directed to a compound of any one of Embodiment Nos. 1 to 103 wherein R⁵ is

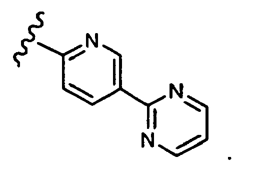

Embodiment No. 126 is directed to a compound of any one of Embodiment Nos. 1 to 103 wherein R⁵ is

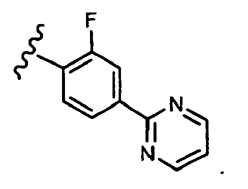

Embodiment No. 127 is directed to a compound of any one of Embodiment Nos. 1 to 103 wherein R⁵ is

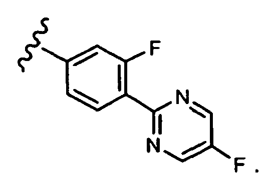

Embodiment No. 128 is directed to a compound of any one of Embodiment Nos. 1 to 103 wherein R¹ is selected from the group consisting of the R¹ groups of any one of Embodiment Nos. 105, 105, 106, 107, 108, 109, 110, 111, 112, 113, 114, 115, 116, 117 or 118, and wherein R⁵ is selected from the group consisting of the R⁵ groups in any one of Embodiment Nos. 119, 120, 121, 122, 123, 124, 125 or 126.

Embodiment No. 129 is directed to a compound of any one of Embodiment Nos. 1 to 128 wherein R² is selected from the group consisting of H, -CH₂OH and -CH₂F.

Embodiment No. 130 is directed to a compound of any one of Embodiment Nos. 1 to 128 wherein R² is H.

Embodiment No. 131 is directed to a compound of any one of Embodiment Nos. 1 to 128 wherein R² is -OR²³ wherein R²³ is alkyl.

Embodiment No. 132 is directed to a compound of any one of Embodiment Nos. 1 to 128 wherein R² is -OCH₃.

Embodiment No. 133 is directed to a compound of any one of Embodiment Nos. 1 to 128 wherein R² is -CN.

Embodiment No. 134 is directed to a compound of any one of Embodiment Nos. 1 to 128 wherein R² is -OCHF₂.

Embodiment No. 135 is directed to a compound of any one of Embodiment Nos.1 to 30, 32 to 35, 41, 42, 44 to 46, 48 to 50, 52 to 56, 58 to 60, 62 to 64, 66 to 70, 72 to 74, 76 to 78, 80 to 85, 87 to 92, 94 to 99 and 101 to 134 wherein R³ is -CH₃, provided that, as those skilled in the art will appreciate, Embodiment No. 135 does not apply to any previous embodiment wherein R³ has already been limited to H.

Embodiment No. 136 is directed to a compound of any one of Embodiment Nos.1 to 30, 32 to 35, 41, 42, 44 to 46, 48 to 50, 52 to 56, 58 to 60, 62 to 64, 66 to 70, 72 to 74, 76 to 78, 80 to 85, 87 to 92, 94 to 99 and 101 to 134 wherein R⁴ is -CH₃, provided that, as those skilled in the art will appreciate, Embodiment No. 135 does not apply to any previous embodiment wherein R⁴ has already been limited to H.

Embodiment No. 137 is directed to a compound of any one of Embodiment Nos.1 to 15, 18 to 19, 21 to 30, 32 to 35, 41 to 42, 44 to 46, 48 to 50, 52 to 56, 58 to 60, 62, 64, 66 to 70, 72 to 74, 76 to 78, 80 to 85, 87 to 92, 94 to 99 and 101 to 134 wherein R⁶ is-CH₃, provided that, as those skilled in the art will appreciate, Embodiment No. 135 does not apply to any previous embodiment wherein R⁶ has already been limited to H, or wherein R⁶ is absent from the formula (e.g., when Q is 2.12, 2.13 or 2.16).

Embodiment No. 138 is directed to a compound of any one of Embodiment Nos.1 to 30, 32 to 35, 41, 42, 44 to 46, 48 to 50, 52 to 56, 58 to 60, 62 to 64, 66 to 70, 72 to 74, 76 to 78, 80 to 85, 87 to 92, 94 to 99 and 101 to 134 wherein R⁷ is -CH₃, provided that, as those skilled in the art will appreciate, Embodiment No. 135 does not apply to any previous embodiment wherein R⁷ has already been limited to H.

Embodiment No. 139 is directed to a compound selected from the group consisting of the final compounds of Examples 1 to 610, and 611.

Embodiment No. 140 is directed to a compound selected from the group consisting of the final compounds of Examples 6, 336, 412, 413, 462, 469, 480, 487, 489, and 571.

Embodiment No. 141 is directed to a compound selected from the group consisting of the final compounds of Examples 1, 2, 5, 6, 7, 9, 11, 12, 13, 18, 19, 22, 23, 24, 25, 26, 27, 28, 61, 88, 89, 183, 184, 186, 188, 189, 190, 191, 192, 193, 196, 197, 198, 199, 202, 203, 204, 205, 250, 251, 253, 254, 255, 256, 257, 258 and 259.

Embodiment No. 142 is directed to the final compound of Example 6.

Embodiment No. 143 is directed to the final compound of Example 183.

Embodiment No. 144 is directed to the final compound of Example 184.

Embodiment No. 145 is directed to the final compound of Example 186.

Embodiment No. 146 is directed to the final compound of Example 188.

Embodiment No. 147 is directed to the final compound of Example 189.

Embodiment No. 148 is directed to the final compound of Example 190.

Embodiment No. 149 is directed to the final compound of Example 191.

Embodiment No. 150 is directed to the final compound of Example 192.

Embodiment No. 151 is directed to the final compound of Example 193.

Embodiment No. 152 is directed to the final compound of Example 336.

Embodiment No. 153 is directed to the final compound of Example 412.

Embodiment No. 154 is directed to the final compound of Example 413.

Embodiment No. 155 is directed to the final compound of Example 462.

Embodiment No. 156 is directed to the final compound of Example 469.

Embodiment No. 157 is directed to the final compound of Example 480.

Embodiment No. 158 is directed to the final compound of Example 487.

Embodiment No. 159 is directed to the final compound of Example 489.

Embodiment No. 160 is directed to the final compound of Example 571.

Embodiment No. 161 is directed to a compound of any one of Embodiment Nos. 1 to 160 in pure and isolated form.

Embodiment No. 162 is directed to a pharmaceutical composition comprising an effective amount of at least one compound (e.g., 1, 2 or 3, or 1 or 2, or 1, and usually 1) of formula 1.0, preferably a compound of formula 1.0C, and a pharmaceutically acceptable carrier.

Embodiment No. 163 is directed to a pharmaceutical composition comprising an effective amount of a compound of formula 1.0, preferably a compound of formula 1.0C, and a pharmaceutically acceptable carrier.

Embodiment No. 164 is directed to a pharmaceutical composition comprising an effective amount of at least one compound (e.g., 1, 2 or 3, or 1 or 2, or 1, and usually 1) of formula 1.1, and a pharmaceutically acceptable carrier.

Embodiment No. 165 is directed to a pharmaceutical composition comprising an effective amount of a compound of formula 1.1 and a pharmaceutically acceptable carrier.

Embodiment No. 166 is directed to a pharmaceutical composition comprising an effective amount of at least one compound (e.g., 1, 2 or 3, or 1 or 2, or 1, and usually 1) of formula 1.2, and a pharmaceutically acceptable carrier.

Embodiment No. 167 is directed to a pharmaceutical composition comprising an effective amount of a compound of formula 1.2 and a pharmaceutically acceptable carrier.

Embodiment No. 168 is directed to a pharmaceutical composition comprising an effective amount of at least one compound (e.g., 1, 2 or 3, or 1 or 2, or 1, and usually 1) of formula 1.3, and a pharmaceutically acceptable carrier.

Embodiment No. 169 is directed to a pharmaceutical composition comprising an effective amount of a compound of formula 1.3 and a pharmaceutically acceptable carrier.

Embodiment No. 170 is directed to a pharmaceutical composition comprising an effective amount of at least one compound (e.g., 1, 2 or 3, or 1 or 2, or 1, and usually 1) of any one of Embodiment Nos. 1 to 160 and a pharmaceutically acceptable carrier.

Embodiment No. 171 is directed to a pharmaceutical composition comprising an effective amount of a compound of any one of Embodiment Nos. 1 to 160 and a pharmaceutically acceptable carrier.

Embodiment No. 172 is directed to a pharmaceutical composition comprising an effective amount of at least one compound (e.g., 1, 2 or 3, or 1 or 2, or 1, and usually 1) of Embodiment No. 161 and a pharmaceutically acceptable carrier.

Embodiment No. 173 is directed to a pharmaceutical composition comprising an effective amount of one compound of Embodiment No. 161 and a pharmaceutically acceptable carrier.

Embodiment No. 174 is directed to a pharmaceutical composition of any one of Embodiment Nos. 162 to 173 further comprising an effective amount of at least one (e.g., 1, 2 or 3, or 1 or 2, or 1, and usually 1) other active pharmaceutically active ingredient.

Embodiment No. 175 is directed to a pharmaceutical composition of any one of Embodiment Nos. 162 to 173 further comprising an effective amount of another (i.e., one other) pharmaceutically active ingredient.

Embodiment No. 176 is directed to a pharmaceutical composition of any one of Embodiment Nos. 162 to 173 further comprising an effective amount of at least one (e.g., 1, 2 or 3, or 1 or 2, or 1, and usually 1) chemotherapeutic agent.

Embodiment No. 177 is directed to a pharmaceutical composition of any one of Embodiment Nos. 162 to 173 further comprising an effective amount of a chemotherapeutic agent.

Embodiment No. 178 is directed to a use of an effective amount of at least one (1, 2 or 3, or 1 or 2, or 1, and usually 1) compound of formula 1.0 (preferably formula 1.0C) for the manufacture of a medicament for treating cancer in a patient in need of such treatment.

Embodiment No. 179 is directed to a use of an effective amount of one compound of formula 1.0 (preferably formula 1.0C) for the manufacture of a medicament for treating cancer in a patient in need of such treatment.

Embodiment No. 180 is directed to a use of an effective amount of at least one (1, 2 or 3, or 1 or 2, or 1, and usually 1) compound of formula 1.1 for the manufacture of a medicament for treating cancer in a patient in need of such treatment.

Embodiment No. 181 is directed to a use of an effective amount of one compound of formula 1.1 for the manufacture of a medicament for treating cancer in a patient in need of such treatment.

Embodiment No. 182 is directed to a use of an effective amount of at least one (1, 2 or 3, or 1 or 2, or 1, and usually 1) compound of formula 1.2 for the manufacture of a medicament for treating cancer in a patient in need of such treatment.

Embodiment No. 183 is directed to a use of an effective amount of one compound of formula 1.2 for the manufacture of a medicament for treating cancer in a patient in need of such treatment.

Embodiment No. 184 is directed to a use of an effective amount of at least one (1, 2 or 3, or 1 or 2, or 1, and usually 1) compound of formula 1.3 for the manufacture of a medicament for treating cancer in a patient in need of such treatment.

Embodiment No. 185 is directed to a use of an effective amount of one compound of formula 1.3 for the manufacture of a medicament for treating cancer in a patient in need of such treatment.

Embodiment No. 186 is directed to a use of an effective amount of at least one (1, 2 or 3, or 1 or 2, or 1, and usually 1) compound of any one of Embodiment Nos. 1 to 161 for the manufacture of a medicament for treating cancer in a patient in need of such treatment.

Embodiment No. 187 is directed to a use of an effective amount of a compound of any one of Embodiment Nos. 1 to 161 for the manufacture of a medicament for treating cancer in a patient in need of such treatment.

Embodiment No. 188 is directed to a use for the manufacture of a medicament for treating cancer in any one of Embodiment Nos. 178 to 187 wherein the medicament is in a form for administration with an effective amount of at least one (1, 2 or 3, or 1 or 2, or 1, and usually 1) chemotherapeutic agent.

Embodiment No. 189 is directed to a use for the manufacture of a medicament for treating cancer in any one of Embodiment Nos. 178 to 187 wherein the medicament is in a form for administration with an effective amount of a chemotherapeutic agent.

Embodiment No. 190 is directed to a use of an effective amount of a pharmaceutical composition of any one of Embodiment Nos. 162 to 177 for the manufacture of a medicament for treating cancer in a patient in need of such treatment.

Embodiment No. 191 is directed to a use of an effective amount of a pharmaceutical composition of any one of Embodiment Nos. 162 to 173 for the manufacture of a medicament for treating cancer in a patient in need of such treatment.

Embodiment No. 192 is directed to a use of an effective amount of a pharmaceutical composition of any one of Embodiment Nos. 162 to 173 for the manufacture of a medicament for treating cancer in a patient in need of such treatment, wherein said medicament is in a form for administration in combination with an effective amount of at least one (1, 2 or 3, or 1 or 2, or 1, and usually 1) chemotherapeutic agent

Embodiment No. 193 is directed to a use of an effective amount of a pharmaceutical composition of any one of Embodiment Nos. 162 to 173 for the manufacture of a medicament for treating cancer in a patient in need of such treatment, wherein said medicament is in a form for administration in combination with an effective amount of one chemotherapeutic agent.

Embodiment No. 194 is directed to a use for the manufacture of a medicament for treating cancer of any one of Embodiment Nos. 188, 189, 192 and 193 wherein the chemotherapeutic agent is selected from the group consisting of: paclitaxet, docetaxel, carboplatin, cisplatin, gemcitabine, tamoxifen, Herceptin, Cetuximab, Tarceva, Iressa, bevacizumab, navelbine, IMC-1C11, SU5416 and SU6688.

Embodiment No. 195 is directed to a use for the manufacture of a medicament for treating cancer of any one of Embodiment Nos. 188, 189, 192 and 193 wherein the chemotherapeutic agent is selected from the group consisting of: paclitaxel, docetaxel, carboplatin, cisplatin, navelbine, gemcitabine, and Herceptin.

Embodiment No. 196 is directed to a use for the manufacture of a medicament for treating cancer of any one of Embodiment Nos. 188, 189, 192 and 193 wherein the chemotherapeutic agent is selected from the group consisting of: Cyclophasphamide, 5-Fluorouracil, Temozolomide, Vincristine, Cisplatin, Carboplatin, and Gemcitabine.

Embodiment No. 197 is directed to a use for the manufacture of a medicament for treating cancer of any one of Embodiment Nos. 188, 189, 192 and 193 wherein the chemotherapeutic agent is selected from the group consisting of: Gemcitabine, Cisplatin and Carboplatin.

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with therapeutically effective amounts of at least one (e.g., 1, 2 or 3, or 1 or 2, or 2, or 1) chemotherapeutic agent selected from the group consisting of: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with therapeutically effective amounts of at least two (e.g., 2 or 3, or 2, and usually 2) different antineoplastic agents selected from the group consisting of: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating cancer in a patient in need of such treatment, wherein said medicament is in a form for administration in combination with a therapeutically effective amount of an antineoplastic agent selected from the group consisting of: (1) EGF inhibitors that are antibodies, (2) EGF inhibitors that are small molecules, (3) VEGF inhibitors that are antibodies, and (4) VEGF inhibitors that are small molecules. Radiation therapy can also be used in conjunction with this above combination therapy, i.e., the above medicament using a combination of compounds of the invention and antineoplastic agent can also be in a form for administration with a therapeutically effect amount of radiation.

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating leukemias (e.g., acute myeloid leukemia (AML), and chronic myeloid leukemia (CML)) in a patient in need of such treatment, wherein said medicament is in a form for administration in combination with therapeutically effective amounts of: (1) Gleevec and interferon to treat CML; (2) Gleevec and pegylated interferon to treat CML; (3) Gleevec to treat CML; (4) an anti-tumor nucleoside derivative (e.g., Ara-C) to treat AML; or (5) an anti-tumor nucleoside derivative (e.g., Ara-C) in combination with an anthracycline to treat AML.

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration in combination with therapeutically effective amounts of: (1) a biologic (e.g., Rituxan); (2) a biologic (e.g., Rituxan) and an anti-tumor nucleoside derivative (e.g., Fludarabine); or (3) Genasense (antisense to BCL-2).

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration in combination with therapeutically effective amounts of: (1) a proteosome inhibitor (e.g., PS-341 from Millenium); or (2) Thalidomide (or related imid).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) at least one (e.g., 1, 2 or 3, or 1 or 2, or 2, or 1) antineoplastic agent selected from the group consisting of: (1) taxanes, (2) platinum coordinator compounds, (3) EGF inhibitors that are antibodies, (4) EGF inhibitors that are small molecules, (5) VEGF inhibitors that are antibodies, (6) VEGF kinase inhibitors that are small molecules, (7) estrogen receptor antagonists or selective estrogen receptor modulators, (8) anti-tumor nucleoside derivatives, (9) epothilones, (10) topoisomerase inhibitors, (11) vinca alkaloids, and (12) antibodies that are inhibitors of αVβ3 integrins.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) at least one (e.g., 1, 2 or 3, or 1 or 2, or 2, or 1) antineoplastic agent selected from the group consisting of: (1) taxanes, (2) platinum coordinator compounds, (3) EGF inhibitors that are antibodies, (4) EGF inhibitors that are small molecules, (5) VEGF inhibitors that are antibodies, (6) VEGF kinase inhibitors that are small molecules, (7) estrogen receptor antagonists or selective estrogen receptor modulators, (8) anti-tumor nucleoside derivatives, (9) epothilones, (10) topoisomerase inhibitors, (11) vinca alkaloids, and (12) antibodies that are inhibitors of αVβ3 integrins.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) at least one (e.g., 1, 2 or 3, or 1 or 2, or 2, or 1) antineoplastic agent selected from the group consisting of: (1) taxanes, (2) platinum coordinator compounds, (3) anti-tumor nucleoside derivatives, (4) topoisomerase inhibitors, and (5) vinca alkaloids.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) carboplatin, and (c) paclitaxel.

This invention also provides a use of therapeutically effective amounts of: (a) at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) cisplatin, and (c) gemcitabine.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161). (b) carboplatin, and (c) gemcitabine.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non small cell lung cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) Carboplatin, and (c) Docetaxel.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating cancer in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) an antineoplastic agent selected from the group consisting of: (1) EGF inhibitors that are antibodies, (2) EGF inhibitors that are small molecules, (3) VEGF inhibitors that are antibodies, (4) VEGF kinase inhibitors that are small molecules.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating squamous cell cancer of the head and neck, in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) at least one (e.g., 1, 2 or 3, or 1 or 2, or 2, or 1) antineoplastic agent selected from the group consisting of: (1) taxanes, and (2) platinum coordinator compounds.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating squamous cell cancer of the head and neck, in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) at least one (e.g., 1, 2 or 3, or 1 or 2, or 2, or 1) antineoplastic agent selected from the group consisting of: (1) taxanes, (2) platinum coordinator compounds, and (3) anti-tumor nucleoside derivatives (e.g., 5-Fluorouracil).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating CML in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) Gleevec, and (c) interferon (e.g., Intron-A).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating CML in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) Gleevec; and (c) pegylated interferon (e.g., Peg-Intron, and Pegasys).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating CML in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161) and (b) Gleevec.

This invention also provides a use of a therapeutically effective amount of at least one (e.g., 1, 2 or 3, or 1 or 2, or 1, and usually 1) compound of formula 1.0 (for example, as described in any one of Embodiment Nos. 1 to 161) for the manufacture of a medicament for treating CMML in a patient in need of such treatment.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating AML in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) an anti-tumor nucleoside derivative (e.g., Cytarabine (i.e., Ara-C)).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating AML in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) an anti-tumor nucleoside derivative (e.g., Cytarabine (i.e., Ara-C)), and (c) an anthracycline.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) Rituximab (Rituxan).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), (b) Rituximab (Rituxan), and (c) an anti-tumor nucleoside derivative (e.g., Fludarabine (i.e., F-ara-A).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating non-Hodgkin's lymphoma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) Genasense (antisense to BCL-2).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) a proteosome inhibitor (e.g., PS-341 (Millenium)).

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) Thalidomide or related imid.

This invention also provides a use of at least one (e.g., 1, 2 or 3, or 1 or 2, or 1, and usually 1) compound of formula 1.0 (for example, as described in any one of Embodiment Nos. 1 to 161) for the manufacture of a medicament for treating multiple myeloma in a patient in need of such treatment, wherein said medicament is in a form for administration of therapeutically effective amounts of: (a) at least one (e.g., 1, 2 or 3, or 1 or 2, or 1, and usually 1) compound of formula 1.0 (for example, as described in any one of Embodiment Nos. 1 to 161), and (b) Thalidomide.

This invention is also directed to the uses for the manufacture of a medicament for treating cancer described herein, particularly those described above, wherein in addition to the administration of the compound of formula 1.0 (for example, as described in any one of Embodiment Nos. 1 to 161) and antineoplastic agents, radiation therapy is also administered prior to, during, or after the treatment cycle.

This invention also provides a use of an effective amount of at least one (e.g.; 1, 2 or 3, or 1 or 2, or 1, and usually 1) compound of formula 1.0 (for example, as described in any one of Embodiment Nos. 1 to 161) for the manfacture of a medicament for treating cancer (e.g., lung cancer, prostate cancer and myeloid leukemias) in a patient in need of such treatment, wherein said medicament is in a form for administration in combination with at least one (e.g., 1, 2 or 3, or 1 or 2, or 2, or 1) antineoplastic agent, microtubule affecting agent and/or radiation therapy.

This invention also provides a use of an effective amount of at least one (e.g., 1, 2 or 3, or 1 or 2, or 1, and usually 1) compound of formula 1.0 (for example, as described in any one of Embodiment Nos. 1 to 161) for the manfacture of a medicament for treating cancer in a patient in need of such treatment, wherein said medicament is in a form for administration in combination with an effective amount of at least one (e.g., 1, 2 or 3, or 1 or 2, or 1, and usually 1) signal transduction inhibitor.

Thus, in one example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Paditaxel (e.g., Taxol^{®} is administered once per week in an amount of about 50 to about 100 mg/m², and in another example about 60 to about 80 mg/m², and (3) Carboplatin is administered once per week in an amount to provide an AUC of about 2 to about 3.

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and yet in another example about 100 mg administered twice a day, (2) Paclitaxel (e.g., Taxol^{®} is administered once per week in an amount of about 50 to about 100 mg/m², and in another example about 60 to about 80 mg/m², and (3) Cisplatin is administered once per week in an amount of about 20 to about 40 mg/m².

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Docetaxel (e.g., Taxotere^{®}) is administered once per week in an amount of about 10 to about 45 mg/m², and (3) Carboplatin is administered once per week in an amount to provide an AUC of about 2 to about 3.

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Docetaxel (e.g., Taxotere^{®}) is administered once per week in an amount of about 10 to about 45 mg/m², and (3) Cisplatin is administered once per week in an amount of about 20 to about 40 mg/m².

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Paclitaxel (e.g., Taxol^{®} is administered once every three weeks in an amount of about 150 to about 250 mg/m², and in another example about 175 to about 225 mg/m², and in yet another example 175 mg/m², and (3) Carboplatin is administered once every three weeks in an amount to provide an AUC of about 5 to about 8, and in another example 6.

In another example of treating non small cell lung cancer: (1) the compound of formula 1.0 (for example, as described in any one of Embodiment Nos. 1 to 161) is administered in an amount of 100 mg administered twice a day, (2) Paclitaxel (e.g., Taxol^{®} is administered once every three weeks in an amount of 175 mg/m², and (3) Carboplatin is administered once every three weeks in an amount to provide an AUC of 6.

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Paclitaxel (e.g., Taxol^{®} is administered once every three weeks in an amount of about 150 to about 250 mg/m², and in another example about 175 to about 225 mg/m², and (3) Cisplatin is administered once every three weeks in an amount of about 60 to about 100 mg/m²_{.}

In another example (e.g., treating non small cell lung cancer): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Docetaxel (e.g., Taxotere^{®} is administered once every three weeks in an amount of about 50 to about 100 mg/m², and (3) Carboplatin is administered once every three weeks in an amount to provide an AUC of about 5 to about 8.

In another example (e.g., treating non small cell lung cancer): (1) the compound of formula 1.0 (for example, as described in any one of Embodiment Nos. 1 to 161) is administered in an amount of about 50 mg to about 200 mg twice a day, in another example about 75 mg to about 125 mg administered twice a day, and in yet another example about 100 mg administered twice a day, (2) Docetaxel (e.g., Taxotere^{®} is administered once every three weeks in an amount of about 50 to about 100 mg/m², and (3) Cisplatin is administered once every three weeks in an amount of about 60 to about 100 mg/m².

In another example for treating non small cell lung cancer using the compounds of formula 1.0, Docetaxel and Carboplatin: (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, (2) Docetaxel (e.g., Taxotere^{®} is administered once every three weeks in an amount of about 75 mg/m², and (3) Carboplatin is administered once every three weeks in an amount to provide an AUC of about 6.

In another example of the treatments of non-small cell lung cancer described above the Docetaxel (e.g., Taxotere^{®}) and Cisplatin, the Docetaxel (e.g., Taxotere^{®}) and Carboplatin, the Paclitaxel (e.g., Taxol^{®} and Carboplatin, or the Paclitaxel (e.g., Taxol^{®}) and Cisplatin are administered on the same day.

In another example (e.g., CML): (1) the compound of formula 1.0 (for example, as described in any one of Embodiment Nos. 1 to 161) is administered in an amount of about 100 mg to about 200 mg administered twice a day, (2) Gleevec is administered in an amount of about 400 to about 800 mg/day orally, and (3) interferon (Intron-A) is administered in an amount of about 5 to about 20 million IU three times per week.

In another example (e.g., CML): (1) the compound of formula 1.0 (for example, as described in any one of Embodiment Nos. 1 to 161) is administered in an amount of about 100 mg to about 200 mg administered twice a day, (2) Gleevec is administered in an amount of about 400 to about 800 mg/day orally, and (3) pegylated interferon (Peg-Intron or Pegasys) is administered in an amount of about 3 to about 6 micrograms/kg/day.

In another example (e.g., non-Hodgkin's lymphoma): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, and (2) Genasense (antisense to BCL-2) is administered as a continuous IV infusion at a dose of about 2 to about 5 mg/kg/day (e.g., 3 mg/kg/day) for 5 to 7 days every 3 to 4 weeks.

In another example (e.g., multiple myeloma): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, and (2) the proteosome inhibitor (e.g., PS-341 - Millenium) is administered in an amount of about 1.5mg/m² twice weekly for two consecutive weeks with a one week rest period.

In another example (e.g., multiple myeloma): (1) the compound of formula 1.0 (for example, as described in any one of Embodiment Nos. 1 to 161) is administered in an amount of about 50 mg to about 200 mg twice a day, and in another example about 75 mg to about 125 mg administered twice a day, and in yet another example about 100 mg administered twice a day, and (2) the Thalidomide (or related imid) is administered orally in an amount of about 200 to about 800 mg/day, with dosing being continuous until relapse or toxicity.

In one embodiment of the uses for the manufacture of a medicament for treating cancer of this invention, the chemotherapeutic agents are selected from the group consisting of: paclitaxel, docetaxel, carboplatin, cisplatin, gemcitabine; tamoxifen, Herceptin, Cetuximab, Tarceva, Iressa, bevacizumab, navelbine, IMC-1 C11, SU5416 and SU6688.

In another embodiment of the uses for the manufacture of a medicament for treating cancer of this invention, the chemotherapeutic agents are selected from the group consisting of: paclitaxel, docetaxel, carboplatin, cisplatin, navelbine, gemcitabine, and Herceptin.

Thus, one embodiment of this invention is directed to a use of a therapeutically effective amount of the compound of formula 1.0 (for example, as described in any one of Embodiment Nos. 1 to 161) for the manufacture of a medicament for treating cancer, wherein said medicament is in a form for administration in combination with therapeutically effective amounts of a taxane and a platinum coordination compound.

Another embodiment of this invention is directed to a use of a therapeutically effective amount of the compound of formula 1.0 (for example, as described in any one of Embodiment Nos. 1 to 161) for the manufacture of a medicament for treating cancer, wherein said medicament is in a form for administration in combination with therapeutically effective amounts of a taxane and a platinum coordination compound, wherein said compound of formula 1.0 is for administration every day, said taxane is for administration once per week per cycle, and said platinum coordinator compound is for administration once per week per cycle. In another embodiment the treatment is for one to four weeks per cycle.

Another embodiment of this invention is directed to a use of therapeutically effective amounts of the compound of formula 1.0 (for example, as described in any one of Embodiment Nos. 1 to 161) for the manufacture of a medicament for treating cancer, wherein said medicament is in a form for administration in combination with therapeutically effective amounts of a taxane and a platinum coordination compound, wherein said compound of formula 1.0 is for administration every day, said taxane is for administration once every three weeks per cycle, and said platinum coordinator compound is for administration once every three weeks per cycle. In another embodiment the treatment is for one to three weeks per cycle.

Another embodiment of this invention is directed to a use of a therapeutically effective amount of the compound of formula 1.0 (for example, as described in any one of Embodiment Nos. 1 to 161) for the manufacture of a medicament for treating cancer, wherein said medicament is in a form for administration in combination with therapeutically effective amounts of paclitaxel and carboplatin. In another embodiment, said compound of formulas 1.0 is administered every day, said paclitaxel is administered once per week per cycle, and said carboplatin is administered once per week per cycle. In another embodiment the treatment is for one to four weeks per cycle.

Another embodiment of this invention is directed to a use of a therapeutically effective amount of the compound of formula 1.0 (for example, as described in any one of Embodiment Nos. 1 to 161) for the manufacture of a medicament for treating cancer, wherein said medicament is in a form for administration in combination with therapeutically effective amounts of paclitaxel and carboplatin. In another embodiment, said compound of formula 1.0 is administered every day, said paclitaxel is administered once every three weeks per cycle, and said carboplatin is administered once every three weeks per cycle. In another embodiment the treatment is for one to three weeks per cycle.

Another embodiment of this invention is directed to a use of a therapeutically effective amount of the compound of formula 1.0 (for example, as described in any one of Embodiment Nos. 1 to 161) for the manufacture of a medicament for treating non small cell lung cancer in a patient in need of such treatment, wherein said medicament is in a form for administration of a therapeutically effective amount of the compound of formula 1.0 (for example, as described in any one of Embodiment Nos. 1 to 161) daily, administration of a therapeutically effective amount of carboplatin once a week per cycle, and administration of a therapeutically effective amount of paclitaxel once a week per cycle, wherein the medicament is for administration for one to four weeks per cycle. In another embodiment said compound of formula 1.0 is administered twice per day. In another embodiment said carboplatin and said paclitaxel are administered on the same day, and in another embodiment said carboplatin and said paclitaxel are administered consecutively, and in another embodiment said carboplatin is administered after said paclitaxel.

Another embodiment of this invention is directed to a use of a therapeutically effective amount of a compound of formula 1.0 (for example, as described in any one of Embodiment Nos. 1 to 161) for the manufacture of a medicament for treating non small cell lung cancer in a patient in need of such treatment, wherein said medicament is in a form for administration of a therapeutically effective amount of a compound of formula 1.0 (for example, as described in any one of Embodiment Nos. 1 to 161) daily, administration of a therapeutically effective amount of carboplatin once every three weeks per cycle, and administration of a therapeutically effective amount of paclitaxel once every three weeks per cycle, wherein the medicament is for administration for one to three weeks. In another embodiment compound of formula 1.0 is administered twice per day. In another embodiment said carboplatin and said paclitaxel are administered on the same day, and in another embodiment said carboplatin and said paclitaxel are administered consecutively, and in another embodiment said carboplatin is administered after said paclitaxel.

Another embodiment of this invention is directed to a use of a compound of formula 1.0 (for example, as described in any one of Embodiment Nos. 1 to 161) for the manufacture of a medicament for treating non small cell lung cancer in a patient in need of such treatment, wherein said medicament is in a form for administration of about 50 to about 200 mg of a compound of formula 1.0 (for example, as described in any one of Embodiment Nos. 1 to 161) twice a day, administration of carboplatin once per week per cycle in an amount to provide an AUC of about 2 to about 8 (and in another embodiment about 2 to about 3), and administration of about 60 to about 300 mg/m² (and in another embodiment about 50 to 100mg/m², and in yet another embodiment about 60 to about 80 mg/m²) of paclitaxel once per week per cycle, wherein the medicament is for administration for one to four weeks per cycle. In another embodiment said compound of formula 1.0 is administered in amount of about 75 to about 125 mg twice a day, and in another embodiment about 100 mg twice a day. In another embodiment said carboplatin and said paclitaxel are administered on the same day, and in another embodiment said carboplatin and said paclitaxel are administered consecutively, and in another embodiment said carboplatin is administered after said paclitaxel.

In another embodiment, this invention is directed to a use of a compound of formula 1.0 (for example, as described in any one of Embodiment Nos. 1 to 161) for the manufacture of a medicament for treating non small cell lung cancer in a patient in need of such treatment, wherein said medicament is in a form for administration of about 50 to about 200 mg of a compound of formula 1.0 (for example, as described in any one of Embodiment Nos. 1 to 161) twice a day, administration of carboplatin once every three weeks per cycle in an amount to provide an AUC of about 2 to about 8 (in another embodiment about 5 to about 8, and in another embodiment 6), and administration of about 150 to about 250 mg/m² (and in another embodiment about 175 to about 225 mg/m², and in another embodiment 175 mg/m²) of paclitaxel once every three weeks per cycle, wherein the medicament is for administration for one to three weeks. In another embodiment said compound of formula 1.0 is administered in an amount of about 75 to about 125 mg twice a day, and in another embodiment about 100 mg twice a day. In another embodiment said carboplatin and said paclitaxel are administered on the same day, and in another embodiment said carboplatin and said paclitaxel are administered consecutively, and in another embodiment said carboplatin is administered after said paclitaxel.

Other embodiments of this invention are directed to uses for the manufacture of a medicament for treating cancer as described in the above embodiments (i.e., the embodiments directed to treating cancer and to treating non small cell lung cancer with a taxane and platinum coordinator compound) except that in place of paclitaxel and carboplatin the taxanes and platinum coordinator compounds used together in the uses are: (1) docetaxel (Taxotere®) and cisplatin; (2) paclitaxel and cisplatin; and (3) docetaxel and carboplatin. In another embodiment of the uses of this invention cisplatin is used in amounts of about 30 to about 100 mg/m². In the another embodiment of the uses of this invention docetaxel is used in amounts of about 30 to about 100 mg/m².

In another embodiment this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of a taxane and an EGF inhibitor that is an antibody. In another embodiment the taxane used is paclitaxel, and the EGF inhibitor is a HER2 antibody (in one embodiment Herceptin) or Cetuximab, and in another embodiment Herceptin is used. The length of treatment, and the amounts and administration of said compound of formula 1.0 and the taxane are as described in the embodiments above. The EGF inhibitor that is an antibody is administered once a week per cycle, and in another embodiment is administered on the same day as the taxane, and in another embodiment is administered consecutively with the taxane, For example, Herceptin is administered in a loading dose of about 3 to about 5 mg/m² (in another embodiment about 4 mg/m²), and then is administered in a maintenance dose of about 2 mg/m² once per week per cycle for the remainder of the treatment cycle (usually the cycle is 1 to 4 weeks). In one embodiment the cancer treated is breast cancer.

In another embodiment this invention is directed to a use of a compound of formula 1.0 (for example, as described in any one of Embodiment Nos. 1 to 161) for the manufacture of a medicament for treating cancer, wherein said medicament is in a form for administration of therapeutically effective amounts of: (1) a compound of formula 1.0 (for example, as described in any one of Embodiment Nos. 1 to 161), (2) a taxane, and (3) an antineoplastic agent selected from the group consisting of: (a) an EGF inhibitor that is a small molecule, (b) a VEGF inhibitor that is an antibody, and (c) a VEGF kinase inhibitor that is a small molecule. In another embodiment, the taxane paclitaxel or docetaxel is used. In another embodiment the antineoplastic agent is selected from the group consisting of: tarceva, Iressa, bevacizumab, SU5416, SU6688 and BAY 43-9006. The length of treatment, and the amounts and administration of said compound of formula 1.0 and the taxane are as described in the embodiments above. The VEGF kinase inhibitor that is an antibody is usually given once per week per cycle. The EGF and VEGF inhibitors that are small molecules are usually given daily per cycle. In another embodiment, the VEGF inhibitor that is an antibody is given on the same day as the taxane, and in another embodiment is administered concurrently with the taxane. In another embodiment, when the EGF inhibitor that is a small molecule or the VEGF inhibitor that is a small molecule is administered on the same day as the taxane, the administration is concurrently with the taxane. The EGF or VEGF kinase inhibitor is generally administered in an amount of about 10 to about 500 mg/m².

In another embodiment this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of an anti-tumor nucleoside derivative and a platinum coordination compound.

Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of an anti-tumor nucleoside derivative and a platinum coordination compound, wherein said compound of formula 1.0 is for administration every day, said anti-tumor nucleoside derivative is for administration once per week per cycle, and said platinum coordinator compound is for administration once per week per cycle. Although the treatment can be for one to four weeks per cycle, in one embodiment the treatment is for one to seven weeks per cycle.
Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of an anti-tumor nucleoside derivative and a platinum coordination compound, wherein said compound of formula 1.0 is for administration every day, said an anti-tumor nucleoside derivative is for administration once per week per cycle, and said platinum coordinator compound is for administration once every three weeks per cycle. Although the treatment can be for one to four weeks per cycle, in one embodiment the treatment is for one to seven weeks per cycle.
Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of gemcitabine and cisplatin. In another embodiment, said compound of formula 1.0 is administered every day, said gemcitabine is administered once per week per cycle, and said cisplatin is administered once per week per cycle. In one embodiment the treatment is for one to seven weeks per cycle.

Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of gemcitabine and cisplatin. In another embodiment, said compound of formula 1.0 is administered every day, said gemcitabine is administered once per week per cycle, and said cisplatin is administered once every three weeks per cycle. In another embodiment the treatment is for one to seven weeks.

Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of gemcitabine and carboplatin. In another embodiment said compound of formula 1.0 is administered every day, said gemcitabine is administered once per week per cycle, and said carboplatin is administered once per week per cycle. In another embodiment the treatment is for one to seven weeks per cycle.

Another embodiment of this invention is directed to a use of a therapeutically effective amount of a compound of formula 1.0 (for example, as described in any one of Embodiment Nos. 1 to 161) for the manufacture of a medicament for treating cancer, wherein said medicament is in a form for administration in combination with therapeutically effective amounts of gemcitabine and carboplatin. In another embodiment said compound of formula 1.0 is administered every day, said gemcitabine is administered once per week per cycle, and said carboplatin is administered once every three weeks per cycle. In another embodiment the treatment is for one to seven weeks per cycle.

In the above embodiments using gemcitabine, the compound of formula 1.0 (for example; as described in any one of Embodiment Nos. 1 to 161) and the platinum coordinator compound are administered as described above for the embodiments using taxanes. Gemcitabine is administered in an amount of about 500 to about 1250 mg/m². In one embodiment the gemcitabine is administered on the same day as the platinum coordinator compound, and in another embodiment consecutively with the platinum coordinator compound, and in another embodiment the gemcitabine is administered after the platinum coordinator compound.

Another embodiment of this invention is directed to a use of a compound of formula 1.0 (for example, as described in any one of Embodiment Nos. 1 to 161) for the manufacture of a medicament for treating cancer, wherein said medicament is in a form for administration in combination with an antineoplastic agent selected from: (1) EGF inhibitors that are antibodies, (2) EGF inhibitors that are small molecules, (3) VEGF inhibitors that are antibodies, and (4) VEGF kinase inhibitors that are small molecules all as described above. The treatment is for one to seven weeks per cycle, and generally for one to four weeks per cycle. The compound of formula 1.0 is administered in the same manner as described above for the other embodiments of this invention. The small molecule antineoplastic agents are usually administered daily, and the antibody antineoplastic agents are usually administered once per week per cycle. In one embodiment the antineoplastic agents are selected from the group consisting of: Herceptin, Cetuximab, Tarceva, Iressa, bevacizumab, IMC-1C11, SU5416, SU6688 and BAY 43-9006.

In the embodiments of this invention wherein a platinum coordinator compound is used as well as at least one other antineoplastic agent, and these drugs are administered consecutively, the platinum coordinator compound is generally administered after the other antineoplastic agents have been administered.

Other embodiments of this invention include the administration of a therapeutically effective amount of radiation to the patient in addition to the administration of a compound of formula 1.0 (for example, as described in any one of Embodiment Nos. 1 to 161) and antineoplastic agents in the embodiments described above. Radiation is administered according to techniques and protocols well know to those skilled in the art.

Another embodiment of this invention is directed to a pharmaceutical composition comprising at least two different chemotherapeutic agents and a pharmaceutically acceptable carrier for intravenous administration. Preferably the pharmaceutically acceptable carrier is an isotonic saline solution (0.9% NaCl) or a dextrose solution (e.g., 5% dextrose).

Another embodiment of this invention is directed to a pharmaceutical composition comprising a compound of formula 1.0 (for example, as described in any one of Embodiment Nos. 1 to 161) and at least two different antineoplastic agents and a pharmaceutically acceptable carrier for intravenous administration. Preferably the pharmaceutically acceptable carrier is an isotonic saline solution (0.9% NaCl) or a dextrose solution (e.g., 5% dextrose).

Another embodiment of this invention is directed to a pharmaceutical composition comprising a compound of formula 1.0 (for example, as described in any one of Embodiment Nos. 1 to 161) and at least one antineoplastic agent and a pharmaceutically acceptable carrier for intravenous administration. Preferably the pharmaceutically acceptable carrier is an isotonic saline solution (0.9% NaCl) or a dextrose solution (e.g., 5% dextrose).

Other embodiments of this invention are directed to the use of a combination of at least one (e.g., one) compound of formula 1.0 (for example, as described in any one of Embodiment Nos. 1 to 161) and drugs for the treatment of breast cancer, i.e., this invention is directed to a combination therapy for the treatment of breast cancer. Those skilled in the art will appreciate that the compounds of formula 1.0 and drugs are generally administered as individual pharmaceutical compositions. The use of a pharmaceutical composition comprising more than one drug is within the scope of this invention.

Thus, another embodiment of this invention is directed to a use of a therapeutically effective amount of at least one (e.g., one) compound of formula 1.0 (for example, as described in any one of Embodiment Nos. 1 to 161) for the manufacture of a medicament for treating (or preventing) breast cancer (i.e., postmenopausal and premenopausal breast cancer, e.g., hormone-dependent breast cancer) in a patient in need of such treatment, wherein said medicament is in a form for administration in combination with a therapeutically effective amount of at least one antihormonal agent selected from the group consisting of: (a) aromatase inhibitors, (b) antiestrogens, and (c) LHRH analogues; and said treatment optionally including the administration of at least one chemotherapeutic agent.

The compound of formula 1.0 is preferably administered orally, and in one embodiment is administered in capsule form.

Examples of aromatase inhibitors include but are not limited to: Anastrozole (e.g., Arimidex), Letrozole (e.g., Femara), Exemestane (Aromasin), Fadrozole and Formestane (e.g., Lentaron).

Examples of antiestrogens include but are not limited to: Tamoxifen (e.g., Nolvadex), Fulvestrant (e.g., Faslodex), Raloxifene (e.g., Evista), and Acolbifene.

Examples of LHRH analogues include but are not limited to: Goserelin (e.g., Zoladex) and Leuprolide (e.g., Leuprolide Acetate, such as Lupron or Lupron Depot).

Examples of chemotherapeutic agents include but are not limited to: Trastuzumab (e.g., Herceptin), Gefitinib (e.g., Iressa), Erlotinib (e.g., Erlotinib HCl, such as Tarceva), Bevacizumab (e.g., Avastin), Cetuximab (e.g., Erbitux), and Bortezomib (e.g., Velcade).

Preferably, when more than one antihormonal agent is used, each agent is selected from a different category of agent. For example, one agent is an aromatase inhibitor (e.g., Anastrozole, Letrozole, or Exemestane) and one agent is an antiestrogen (e.g., Tamoxifen or Fulvestrant).

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one antihormonal agent selected from the group consisting of: (a) aromatase inhibitors, (b) antiestrogen, and (c) LHRH analogues; and an effective amount of at least one chemotherapeutic agent.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one antihormonal agent selected from the group consisting of: (a) aromatase inhibitors, (b) antiestrogens, and (c) LHRH analogues.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one antihormonal agent selected from the group consisting of: (a) aromatase inhibitors, and (b) antiestrogens.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one antihormonal agent selected from the group consisting of: (a) aromatase inhibitors and (b) antiestrogens; and at least one chemotherapeutic agent.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one aromatase inhibitor.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of at least one aromatase inhibitor and at least one chemotherapeutic agent.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene, and (c) LHRH analogues that are selected from the group consisting of: Goserelin and Leuprolide; and (3) an effective amount of at least one chemotherapeutic agent selected from the group consisting of: Trastuzumab, Gefitinib, Erlotinib, Bevacizumab, Cetuximab, and Bortezomib.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene, and (c) LHRH analogues that are selected from the group consisting of: Goserelin and Leuprolide.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); and (2) at least one antihormonal agent selected from the group consisting of: (a) aromatase inhibitors that are selected from the group consisting of Anastrozole, Letrozole, Exemestane, Fadrozole and Formestane, and (b) antiestrogens that are selected from the group consisting of: Tamoxifen, Fulvestrant, Raloxifene, and Acolbifene.

Another embodiment of this invention is directed to a use of at least one (e.g., one) compound of formulas 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene; and (3) an effective amount of at least one chemotherapeutic agents are selected from the group consisting of: Trastuzumab, Gefitinib, Erlotinib, Bevacizumab, Cetuximab, and Bortezomib.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); and (2) at least one aromatase inhibitor selected from the group consisting of Anastrozole, Letrozole, Exemestane, Fadrozole and Formestane.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); (2) at least one aromatase inhibitor that is selected from the group consisting of Anastrozole, Letrozole, Exemestane, Fadrozole and Formestane; and (3) an effective amount of at least one chemotherapeutic agent selected from the group consisting of: Trastuzumab, Gefitinib, Erlotinib, Bevacizumab, Cetuximab, and Bortezomib.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); (2) at least one aromatase inhibitor; and (3) at least one LHRH analogue.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment wherein said medicament is in a form for administration of a therapeutically effective amount of:(1) at least one (e.g., one) compound of formula 1.0 (for example, as described in any one of Embodiment Nos. 1 to 161); (2) at least one antiestrogen ; and (3) at least one LHRH analogue.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); (2) at least one aromatase inhibitor that is selected from the group consisting of Anastrozole, Letrozole, Exemestane, Fadrozole and Formestane; and (3) at least one LHRH analogue that is selected from the group consisting of: Goserelin and Leuprolide.

Another embodiment of this invention is directed to a use of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration of a therapeutically effective amount of: (1) at least one (e.g., one) compound of formula 1.0 (for example, as described in any one of Embodiment Nos. 1 to 161); (2) at least one antiestrogen that is selected from the group consisting of: Tamoxifen, Fulvestrant, Raloxifene, and Acolbifene; and (3) at least one LHRH analogue that is selected from the group consisting of: Goserelin and Leuprolide.

Another embodiment of this invention is directed to a use of administering a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrazole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Raloxifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole and an antiestrogen selected from the group consisting of: Tamoxifen, Fulvestrant, Raloxifene, and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole and an antiestrogen selected from the group consisting of: Tamoxifen, Fulvestrant, Raloxifene, and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane and an antiestrogen selected from the group consisting of: Tamoxifen, Fulvestrant, Raloxifene, and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole and an antiestrogen selected from the group consisting of: Tamoxifen, Fulvestrant, Raloxifene, and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane and an antiestrogen selected from the group consisting of: Tamoxifen, Fulvestrant, Raloxifene, and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Tamoxifen and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fulvestrant and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Raloxifene and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Acolbifene and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolein and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole, Tamoxifen, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole, Tamoxifen, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane, Tamoxifen, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole, Tamoxifen, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane, Tamoxifen, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Anastrozole, Fulvestrant, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Letrozole, Fulvestrant, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Exemestane, Fulvestrant, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Fadrozole, Fulvestrant, and a chemotherapeutic agent selected from the group consisting of: Trastuzumab, Gefitinib, Edotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Formestane, Fulvestrant, and a chemotherapeutic agent selected from the group consisting of: Trastuzumab, Gefitinib, Erlotinib, Bevacizumab, Cetuximab, and Bortezomib.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Raloxifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Raloxifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Acolbifene.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Anastrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Letrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Exemestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Fadrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Goserelin and Formestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Anastrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Letrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Exemestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Fadrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for treating or preventing breast cancer in a patient in need of such treatment, wherein said medicament is in a form for administration in combination with a therapeutically effective amount of Leuprolide and Formestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Anastrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Letrozole.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Exemestane.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Anastrozole and Fulvestrant.

Another embodiment of this invention is directed to a use of a therapeutically effective amount of at least one compound of formula I (e.g., one) for the manufacture of a medicament for the treatment or prevention of breast cancer in a patient in need of such treatment, wherein said medicament is in a form for administration in combination with a therapeutically effective amount of Letrozole and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Exemestane and Fulvestrant.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Anastrozole and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Letrozole and Tamoxifen.

Another embodiment of this invention is directed to a use of a therapeutically effective amount of at least one (e.g., one) compound of formula 1.0 (for example, as described in any one of Embodiment Nos. 1 to 161) for the manufacture of a medicament for the treatment or prevention of breast cancer in a patient in need of such treatment, wherein said medicament is in a form for administration in combination with a therapeutically effective amount of Exemestane and Tamoxifen.

Other embodiments of this invention are directed to any of the above described embodiments for the treatment of Breast Cancer wherein the chemotherapeutic agent is Trastuzumab.

Other embodiments of this invention are directed to any of the above described embodiments for the treatment or prevention of Breast Cancer wherein the use is for the manufacture of a medicament for the treatment of breast cancer.

The compound of formula 1.0 (for example, as described in any one of Embodiment Nos. 1 to 161), antihormonal agents and chemotherapeutic agents can be administered concurrently or sequentially.

The antihormonal agents and optional chemotherapeutic agents are administered according to their protocols, dosage amounts, and dosage forms that are well know to those skilled in the art (e.g., the Physician's Desk Reference or published literature). For example, for Tamoxifen, Fulvestrant, Raloxifene, Anastrozole, Letrozole, Exemestane, Leuprolide and Goserelin, see the Physician's Desk Reference, 57^{th} Edition, 2003, published by Thomas PDR at Montvale, N.J. 07645-1742, the disclosure of which is incorporated herein by reference thereto.

In general, in the embodiments directed to the uses for the manufacture of a medicament for treating Breast Cancer: (1) the compound of formula 1.0 (for example, as described in any one of Embodiment Nos. 1 to 161) can be administered daily (e.g., once per day, and in one embodiment twice a day), (2) the aromatase inhibitors can be administered in accordance with the known protocol for the aromatase inhibitor used (e.g., once per day), (3) the antiestrogens can be administered in accordance with the known protocol for the antiestrogen used (e.g., from once a day to once a month), (4) the LHRH analogue can be administered in accordance with the known protocol for the LHRH analogue used (e.g., once a month to once every three months), and (5) the chemotherapeutic agent can be administered in accordance with the known protocol for the chemotherapeutic agent used (e.g., from once a day to once a week).

Radiation therapy, if administered in the above treatments for breast cancer, is generally administered according to known protocols before administration of the compound of formula 1.0 (for example, as described in any one of Embodiment Nos. 1 to 161), antihormonal agents and optional chemotherapeutic agents.

Treatment according to the uses for the manufacture of a medicament for treating breast cancer is continuous (i.e., a continuous dosing schedule is followed). The treatment is continued until there is a complete response, or until the skilled clinician determines that the patient is not benefiting from the treatment (for example, when there is disease progression).

The continuous treatment protocol for breast cancer can be changed to a discontinuous treatment schedule if, in the judgment of the skilled clinician, the patient would benefit from a discontinuous treatment schedule with one or more of the administered drugs. For example, the compound of formula 1.0 (for example, as described in any one of Embodiment Nos. 1 to 161) can be given using a discontinous treatment schedule while the remaining drugs used in the treatment are given as described herein. An example of a discontinuous treatment protocol for the compound of formula 1.0 (for example, as described in any one of Embodiment Nos. 1 to 161) is a repeating cycle of three weeks with the compound of formula 1.0 followed by one week without the compound of formula 1.0.

After a complete response is achieved with the breast cancer treatment, maintenance therapy with the compound of formula 1.0 (for example, as described in any one of Embodiment Nos. 1 to 161) can be continued using the dosing described in the uses of this invention. Maintenance therapy can also include administration of the antihormonal agents using the dosing described in the uses of this invention. Maintenance therapy can just be with the antihormonal agents. For example, after a complete response is achieved, an aromatase inhibitor (e.g., Anastrozole, Letrozole or Exemestane) can be continued for up to five years. Or, for example, an antiestrogen, e.g., Tamoxifen, may be used for up to five years after a complete response is achieved. Or, for example, an antiestrogen (e.g., Tamoxifen) can be used for up to five years after a complete response is achieved followed by use of an aromatase inhibitor (e.g., Anastrozole, Letrozole or Exemestane) for up to five years.

In the embodiments directed to the treatment of breast cancer described above, the compound of formula 1.0 (for example, as described in any one of Embodiment Nos. 1 to 161) is administered continuously in a total daily dose of about 100 mg to about 600 mg. Usually this amount is administered in divided doses, and in one embodiment this amount is administered twice a day. In one embodiment the compound of formula 1.0 (for example, as described in any one of Embodiment Nos. 1 to 161) is dosed twice a day in an amount of about 50 mg to about 300 mg per dose. In another embodiment the compound of formula 1.0 (for example, as described in any one of Embodiment Nos. 1 to 161) is dosed twice a day in an amount of about 100 mg to about 200 mg per dose. Examples include the compound of formula 1.0 (for example, as described in any one of Embodiment Nos. 1 to 161) being dosed twice a day at 100 mg per dose. Examples also include the compound of formula 1.0 (for example, as described in any one of Embodiment Nos. 1 to 161) being dosed twice a day at 200 mg per dose.

Anastrozole is administered p.o. and is dosed once a day in amounts of about 0.5 to about 10 mg per dose, and in one embodiment in an amount of about 1.0 mg per dose.

Letrozole is administered p.o. and is dosed once a day in amounts of about 1.0 to about 10 mg per dose, and in one embodiment in an amount of about 2.5 mg per dose.

Exemestane is administered p.o. and is dosed once a day in amounts of about 10 to about 50 mg per dose, and in one embodiment in an amount of about 25 mg per dose.

Fadrozole is administered p.o. and is dosed twice a day in amounts of about 0.5 to about 10 mg per dose, and in one embodiment in an amount of about 2.0 mg per dose.

Formestane is administered i.m. and is dosed once every two weeks in amounts of about 100 to about 500 mg per dose, and in one embodiment in an amount of about 250 mg per dose.

Tamoxifen is administered p.o. and is dosed once a day in amounts of about 10 to about 100 mg per dose, and in one embodiment in an amount of about 20 mg per dose.

Fulvestrant is administered i.m. and is dosed once a month in amounts of about 100 to about 1000 mg per dose, and in one embodiment in an amount of about 250 mg per dose.

Raloxifene is administered p.o. and is dosed once a day in amounts of about 10 to about 120 mg per dose, and in one embodiment in an amount of about 60 mg per dose.

Acolbifene is administered p.o. and is dosed once a day in amounts of about 5 to about 20 mg per dose, and in one embodiment in an amount of about 20 mg per dose.

Goserelin is administered s.c. and is dosed once a month, or once every three months, in amounts of about 2 to about 20 mg per dose, and in one embodiment in an amount of about 3.6 mg per dose when administered once a month, and in another embodiment in an amount of about 10.8 mg per dose when administered once every three months.

Leuprolide is administered s.c. and is dosed once a month, or once every three months, in amounts of about 2 to about 20 mg per dose, and in one embodiment in an amount of about 3.75 mg per dose when administered once a month, and in another embodiment in an amount of about 11.25 mg per dose when administered once every three months.

Trastuzumab is administered by i.v. and is dosed once a week in amounts of about 2 to about 20 mpk per dose, and in one embodiment in an amount of about 2 mpk per dose. Trastuzumab is generally initially administered in a loading dose that is generally twice the dose of the weekly dose. Thus, for example, a 4 mpk loading dose is administered and then dosing is 2 mpk per dose per week.

Gefitinib is administered p.o. and is dosed once a day in amounts of about 100 to about 1000 mg per dose, and in one embodiment in an amount of about 250 mg per dose.

Erlotinib is administered p.o. and is dosed once a day in amounts of about 100 to about 500 mg per dose, and in one embodiment in an amount of about 150 mg per dose.

Bevacizumab is administered i.v. and is dosed once every two weeks in amounts of about 2.5 to about 15 mg per kilogram of body weight per dose, and in one embodiment in an amount of about 10 mg per kilogram per dose.

Cetuximab is administered i.v. and is dosed once a week in amounts of about 200 to about 500 mg per meter squared dose, and in one embodiment in an amount of about 250 mg per meter squared per dose.

Bortezomib is administered i.v. and is dosed twice a week for 2 weeks followed by a 10 day rest period (21 day treatment cycle) for a maximum of 8 treatment cycles in amounts of about 1.0 to about 2.5 mg per meter squared per dose, and in one embodiment in an amount of about 1.3 mg per meter squared per dose.

Thus in one embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 50 mg to about 300 mg per dose wherein each dose is for administration twice a day, and (2) Anastrozole p.o. in an amount of about 0.5 to about 10 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 100 to 200 mg per dose, wherein each dose is for administration twice a day, and (2) Anastrozole in an amount of about 1.0 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 50 mg to about 300 mg per dose wherein each dose is for administration twice a day, and (2) Letrozole p.o. in an amount of about 1.0 to about 10 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 100 to 200 mg per dose, wherein each dose is for administration twice a day, and (2) Letrozole p.o. in an amount of about 2.5 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 50 mg to about 300 mg per dose wherein each dose is for administration twice a day, and (2) Exemestane p.o. in an amount of about 10 to about 50 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 100 to 200 mg per dose, wherein each dose is for administration twice a day, and (2) Exemestane in an amount of about 25 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 50 mg to about 300 mg per dose wherein each dose is for administration twice a day, and (2) Fulvestrant i.m. in an amount of about 100 to about 1000 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) orally in an amount of about 100 to 200 mg per dose, wherein each dose is for administration twice a day, and (2) Fulvestrant i.m. in an amount of about 250 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, and (2) Tamoxifen p.o. in an amount of about 10 to about 100 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, and (2) Tamoxifen p.o. in an amount of about 20 mg per dose wherein each dose is for administration once a day.

In other embodiments of the invention the compound of formula 1.0 (for example, as described in any one of Embodiment Nos. 1 to 161) is used for the manufacture of a medicament for the treatment (or prevention) of breast cancer in need of such treatment, wherein said medicament is in a form for administration of the compound of formula 1.0 (for example, as described in any one of Embodiment Nos. 1 to 161), one of the aromatase inhibitors (e.g., Anastrozole, Letrozole, or Exemestane, and in one embodiment Anastrozole), and one of the antiestrogens (e.g., Fulvestrant or Tamoxifen), wherein the compound of formula 1.0, aromatase inhibitor and antiestrogen are for administration in the dosages described above.

Thus, for example in another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Anastrozole p.o. in an amount of about 0.5 to about 10 mg per dose wherein each dose is for administration once a day, and (3) Fulvestrant i.m. in an amount of about 100 to about 1000 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o in an amount of about 100 to 200 mg per dose, wherein each dose is for administration twice a day, (2) Anastrozole p.o. in an amount of about 1.0 mg per dose wherein each dose is for administration once a day, and (3) Fulvestrant i.m. in an amount of about 250 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Letrozole p.o in an amount of about 1.0 to about 10 mg per dose wherein each dose is for administration once a day, and (3) Fulvestrant in an amount of about 100 to about 1000 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, (2) Letrozole p.o. in an amount of about 2.5 mg per dose wherein each dose is for administration once a day, and (3) Fulvestrant i.m. in an amount of about 250 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Exemestane p.o. in an amount of about 10 to about 50 mg per dose wherein each dose is for administration once a day, and (3) Fulvestrant i.m. in an amount of about 100 to about 1000 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, (2) Exemestane p.o. in an amount of about 25 mg per dose wherein each dose is for administration once a day, and (3) Fulvestrant i.m. in an amount of about 250 mg per dose wherein each dose is for administration once a month.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Anastrozole p.o. in an amount of about 0.5 to about 10 mg per dose wherein each dose is for administration once a day, and (3) Tamoxifen p.o.in an amount of about 10 to about 100 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, (2) Anastrozole p.o. in an amount of about 1.0 mg per dose wherein each dose is for administration once a day, and (3) Tamoxifen p.o. in an amount of about 20 mg per dose wherein each dose is for administration once a day.

In another embodiment of this invention the compounds of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Letrozole p.o. in an amount of about 1.0 to about 10 mg per dose wherein each dose is for administration once a day, and (3) Tamoxifen p.o. in an amount of about 10 to about 100 mg per dose wherein each dose is for administration once a day.
In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, (2) Letrozole p.o. in an amount of about 2.5 mg per dose wherein each dose is for administration once a day, and (3) Tamoxifen p.o. in an amount of about 20 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 50 mg to about 300 mg per dose wherein each dose is for administration twice a day, (2) Exemestane p.o. in an amount of about 10 to about 50 mg per dose wherein each dose is for administration once a day, and (3) Tamoxifen p.o. in an amount of about 10 to about 100 mg per dose wherein each dose is for administration once a day.

In another embodiment of this invention the compound of formula 1.0 (for example, as described in any one of Embodiment Nos. 1 to 161) is used for the manufacture of a medicament for the treatment (or prevention) of breast cancer in a patient in need of such treatment, wherein said medicament is in a form for administration of: (1) the compound of formula 1.0 (for example, as described in any one of Embodiment Nos. 1 to 161) p.o. in an amount of about 100 to 200 mg per dose, wherein each dose is for administration twice a day, (2) Exemestane p.o. in an amount of about 25 mg per dose wherein each dose is for administration once a day, and (3) Tamoxifen p.o. in an amount of about 20 mg per dose wherein each dose is for administration once a day.

Those skilled in the art will appreciate that when other combinations of antihormonal agents are used, the individual antihormonal agent is used in the amounts specified above for that individual antihormonal agent.

Other embodiments of the treatment of Breast Cancer are directed to the uses for the manufacture of a medicament for treating Breast Cancer described above wherein the compound of formula 1.0 (for example, as described in any one of Embodiment Nos. 1 to 161) is for administration twice a day in an amount of about 100 mg per dose.

Other embodiments of the treatment of Breast Cancer are directed to the uses for the manufacture of a medicament for treating Breast Cancer described above wherein the compound of formula 1.0 (for example, as described in any one of Embodiment Nos. 1 to 161) is for administration twice a day in an amount of about 200 mg per dose.

Other embodiments of the treatment of Breast Cancer are directed to the uses for the manufacture of a medicament for treating Breast Cancer described above wherein a chemotherapeutic agent is for administration in addition to the compound of formula 1.0 (for example, as described in any one of Embodiment Nos. 1 to 161) and antihormonal agent (or antihormonal agents). In these embodiments the dosage ranges of the compound of formula 1.0 and antihormonal agents are as those described above in the combination therapies, or those described above for the individual compound of formula I and antihormonal agents, and the dosages of the chemotherapeutic agents are those described above for the individual chemotherapeutic agent. The dosages for the chemotherapeutic agents are well known in the art.

Other embodiments of this invention are directed to pharmaceutical compositions comprising the compound of formula 1.0 (for example, as described in any one of Embodiment Nos. 1 to 161) and at least one antihormonal agent and a pharmaceutically acceptable carrier.

Other embodiments of this invention are directed to pharmaceutical compositions comprising the compound of formula 1.0 (for example, as described in any one of Embodiment Nos. 1 to 161), at least one antihormonal agent, at least one chemotherapeutic agent, and a pharmaceutically acceptable carrier.

Other embodiments of this invention are directed to pharmaceutical compositions comprising the compound of formula 1.0 (for example, as described in any one of Embodiment Nos. 1 to 161), at least one chemotherapeutic agent, and a pharmaceutically acceptable carrier.

Those skilled in the art will appreciate that the compounds (drugs) used in the uses of this invention are available to the skilled clinician in pharmaceutical compositions (dosage forms) from the manufacturer and are used in those compositions. So, the recitation of the compound or class of compounds in the above described uses can be replaced with a recitation of a pharmaceutical composition comprising the particular compound or class of compounds. For example, the embodiment directed to a use of a therapeutically effective amount of the compound of formula 1.0 for the manufacture of a medicament for treating cancer, wherein said medicament is in a form for administration in combination with therapeutically effective amounts of a taxane and a platinum coordination compound, includes within its scope a use of a therapeutically effective amount of a pharmaceutical composition comprising the compound of formula 1.0 for the manufacture of a medicament for treating cancer, wherein said medicament is in a form for administration in combination with therapeutically effective amounts of a pharmaceutical composition comprising a taxane, and a pharmaceutical composition comprising a platinum coordination compound.

Those skilled in the art will recognize that the actual dosages and protocols for administration employed in the uses of this invention may be varied according to the judgment of the skilled clinician. The actual dosage employed may be varied depending upon the requirements of the patient and the severity of the condition being treated. Determination of the proper dosage for a particular situation is within the skill of the art. A determination to vary the dosages and protocols for administration may be made after the skilled clinician takes into account such factors as the patient's age, condition and size, as well as the severity of the cancer being treated and the response of the patient to the treatment.

The amount and frequency of administration of the compound of formula 1.0 and the chemotherapeutic agents will be regulated according to the judgment of the attending clinician (physician) considering such factors as age, condition and size of the patient as well as severity of the cancer being treated.

The chemotherapeutic agent can be administered according to therapeutic protocols well known in the art. It will be apparent to those skilled in the art that the administration of the chemotherapeutic agent can be varied depending on the cancer being treated and the known effects of the chemotherapeutic agent on that disease. Also, in accordance with the knowledge of the skilled clinician, the therapeutic protocols (e.g., dosage amounts and times of administration) can be varied in view of the observed effects of the administered therapeutic agents on the patient, and in view of the observed responses of the cancer to the administered therapeutic agents.

The initial administration can be made according to established protocols known in the art, and then, based upon the observed effects, the dosage, modes of administration and times of administration can be modified by the skilled clinician.

The particular choice of chemotherapeutic agent will depend upon the diagnosis of the attending physicians and their judgement of the condition of the patient and the appropriate treatment protocol.

The determination of the order of administration, and the number of repetitions of administration of the chemotherapeutic agent during a treatment protocol, is well within the knowledge of the skilled physician after evaluation of the cancer being treated and the condition of the patient.

Thus, in accordance with experience and knowledge, the practicing physician can modify each protocol for the administration of an chemotherapeutic agent according to the individual patient's needs, as the treatment proceeds. All such modifications are within the scope of the present invention.

The particular choice of antihormonal agents, optional chemotherapeutic agents and optional radiation will depend upon the diagnosis of the attending physicians and their judgment of the condition of the patient and the appropriate treatment protocol.

The determination of the order of administration, and the number of repetitions of administration of the antihormonal agents, optional chemotherapeutic agents and optional radiation during a treatment protocol, is well within the knowledge of the skilled physician after evaluation of the breast cancer being treated and the condition of the patient.

Thus, in accordance with experience and knowledge, the practicing physician can modify each protocol for the administration of antihormonal agents, optional chemotherapeutic agents and optional radiation according to the individual patient's needs, as the treatment proceeds. All such modifications are within the scope of the present invention.

The attending clinician, in judging whether treatment is effective at the dosage administered, will consider the general well-being of the patient as well as more definite signs such as relief of cancer-related symptoms (e.g., pain, cough (for lung cancer), and shortness of breath (for lung cancer)), inhibition of tumor growth, actual shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

The compounds of the invention can be made according to the processes described below.

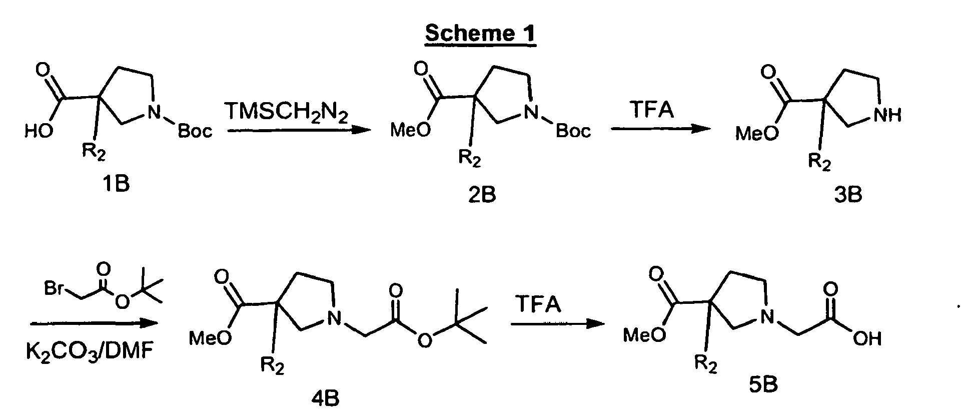

The appropriately substituted pyrrolidine is prepared as follows. The methyl ester of pyrrolidine 1 B is prepared by reaction with TMSdiazomethane. After removing the BOC protecting group, compound 3B is reacted with bromo-tert.butylacetate to obtain 4B. The tert.butyl ester is then removed to obtain intermediate 5B.

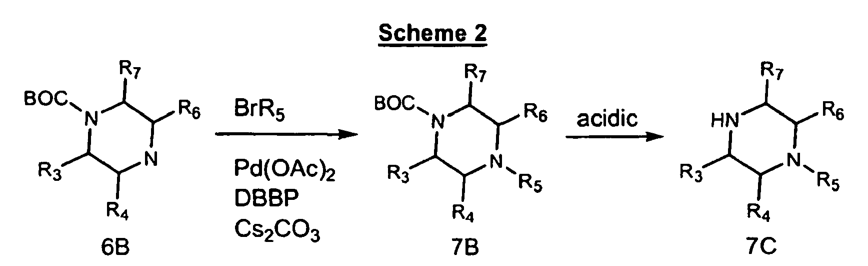

The R⁵ substituted piperazine is prepared by Buchwald type coupling of the piperazine 6B with an aryl bromide in the presence of palladium to obtain the piperazine 7B. The BOC group is removed using acidic conditions (e.g., TFA) to give piperazine 7C.

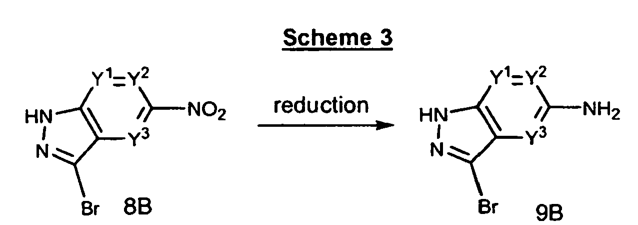

The indazole bromide 9B is prepared by reduction of the indazole nitro compound 8B using reducing conditions such as palladium on carbon in the presence of hydrogen atmosphere.

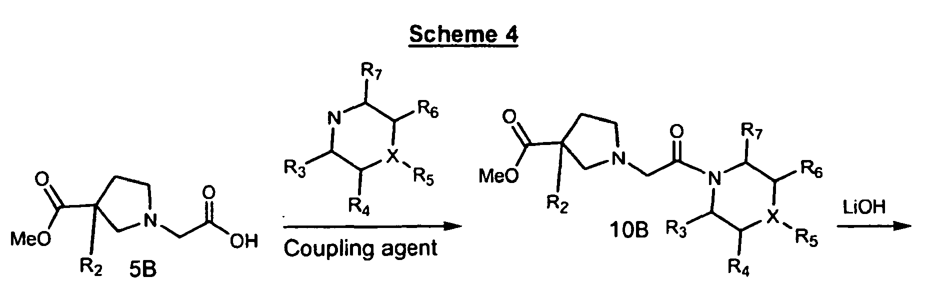

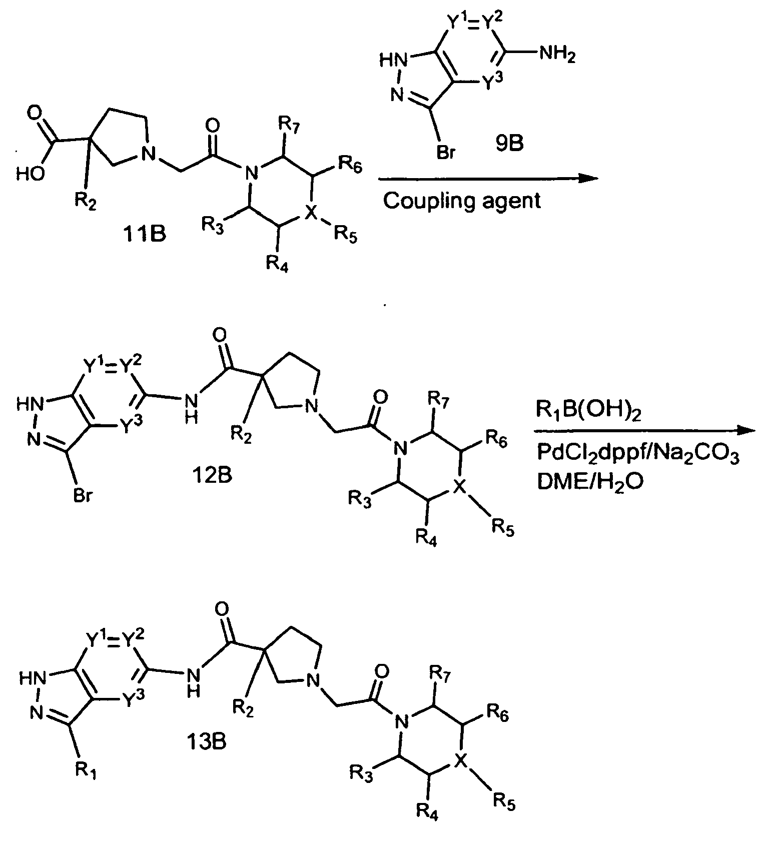

The final molecule is assembled by coupling the pyrrolidine 5B with the piperazine (X is N, see compound 7C from Scheme 2), or with the piperidine (X is C, prepared in Schemes 11 or 12, or prepared according to the Examples described herein, or prepared according to methods well known to those skilled in the art), using standard coupling conditions such as HATU in DMF to obtain 10B. After hydrolysis of the methyl ester to obtain 11B, the indazole intermediate 9B is then coupled using standard coupling conditions such as HATU in DMF to obtain 12B. The indazole is then derivatized at the 3 position by suzuki coupling with an a boronic acid to obtain the final product 13B.

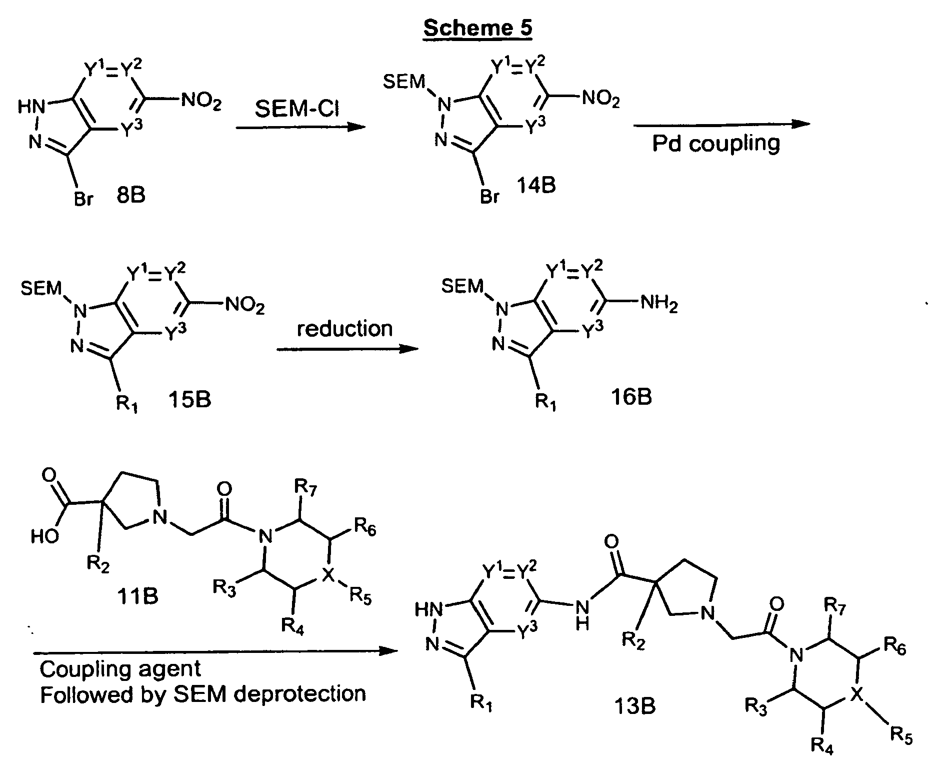

Alternatively, the final product 13B can be prepared by protecting the indazole 8B as the SEM (or trityl) derivative followed by Suzuki coupling to give the appropriately substituted indazole intermediate 15B. After reduction using reducing conditions such as palladium on carbon in the presence of hydrogen atmosphere to obtain 16B, the amine 16B can be coupled to 11 B using standard coupling conditions such as HATU in DMF to obtain 13B.

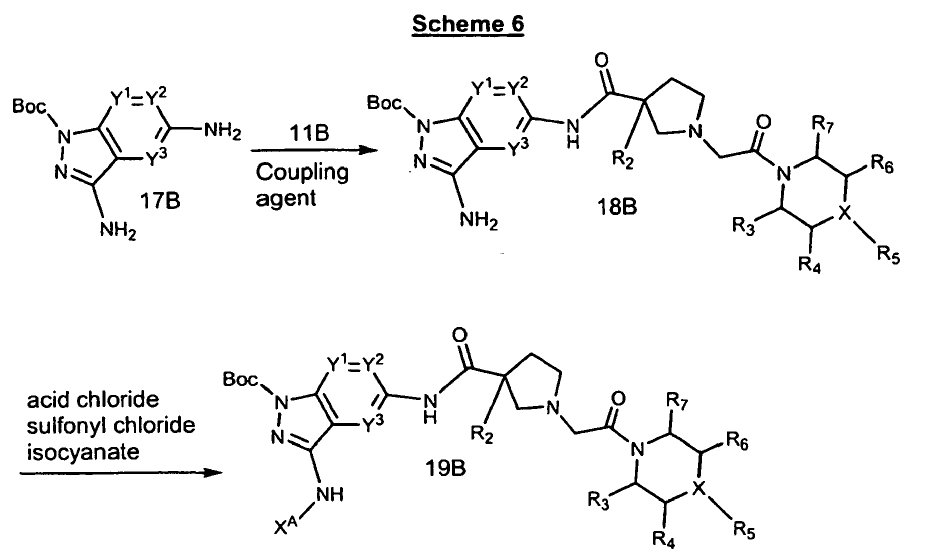

3-Amino substituted indazole derivatives can be prepared according to general Scheme 6 by coupling the Boc protected 3,5-diamino indazole 17B with 11 B under standard coupling conditions such as HATU in DMF to obtain 18B. The 3-amino group can then be reacted with acid chlorides, sulfonyl chlorides and isocyanates to prepare the corresponding amides, sulfonamides and ureas 19B respectively.

Those skilled in the art will appreciate that the moiety -NHX^{A} represents the amino substituted R¹ groups, such as, -N(R¹⁰)₂, -NR³²-C(O)-R¹⁴, -N(R¹⁰)C(O)N(R¹⁰)₂, and -N(R¹⁰)S(O)ₜR¹⁰.

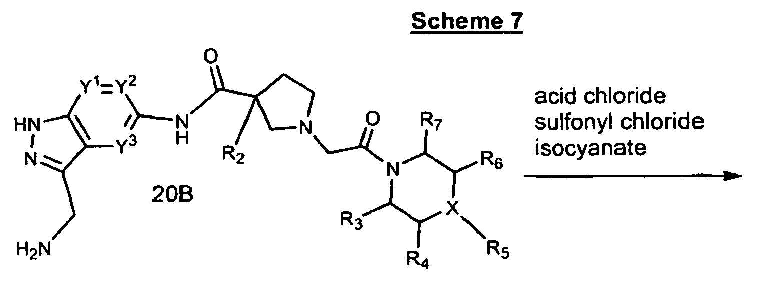

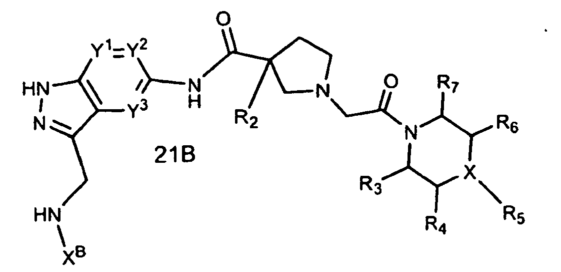

The amino methyl indazole derivative 20B can be prepared according to Example 76 step 1 through 4 by substituting the appropriately substituted rings. The 3-aminomethyl group can then be reacted with acid chlorides, sulfonyl chlorides and isocyanates to prepare the corresponding amides, sulfonamides and ureas 21 B respectively.

Those skilled in the art will appreciate that the moiety -CH₂NHX^{B} represents the amino substituted methyl R¹ groups, such as, -(C(R³⁰)₂)ₙ-NR³²-C(O)-R¹⁰ (wherein n is 1 and each R³⁰ is H), -(C(R³⁰)₂)ₙ-NR³²-S(O)ₜ-R¹⁰ (wherein n is 1 and each R³⁰ is H), -(C(R³⁰)₂)ₙ-NR³²-C(O)-N(R³²)-R¹⁰ (wherein n is 1 and each R³⁰ is H), -(C(R³⁰)₂)ₙR¹³ (wherein n is 1, each R³⁰ is H and R¹³ is -N(R¹⁰)₂),

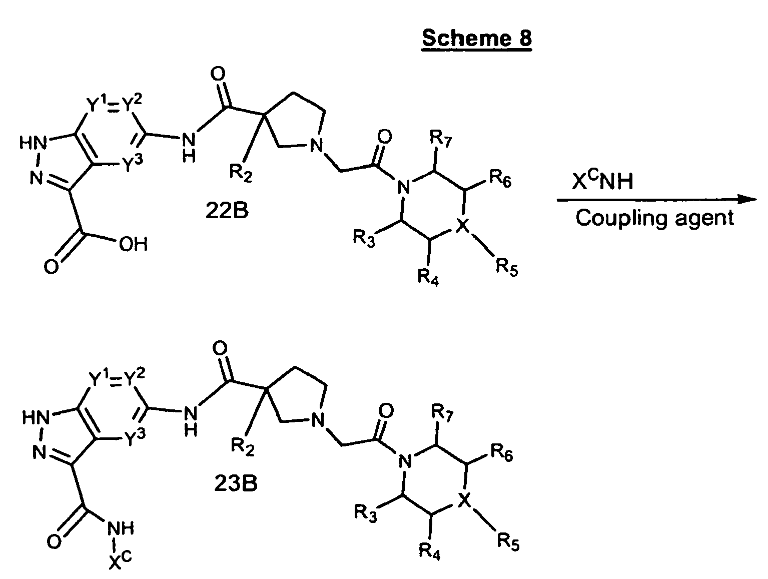

The amide compound 23B can be prepared by following Example 87 substituting the appropriate compound 11 B (General Scheme 4). The resulting acid 22B is then coupled with the appropriate amine under standard coupling conditions such as in the presence of EDC/HOBt to obtain 23B.

Those skilled in the art will appreciate that the moiety -C(O)NHX^{c} represents the amide R¹ groups, such as, -C(O)N(R¹⁰)₂ and -C(O)-NR³²-C(R¹⁸)₃

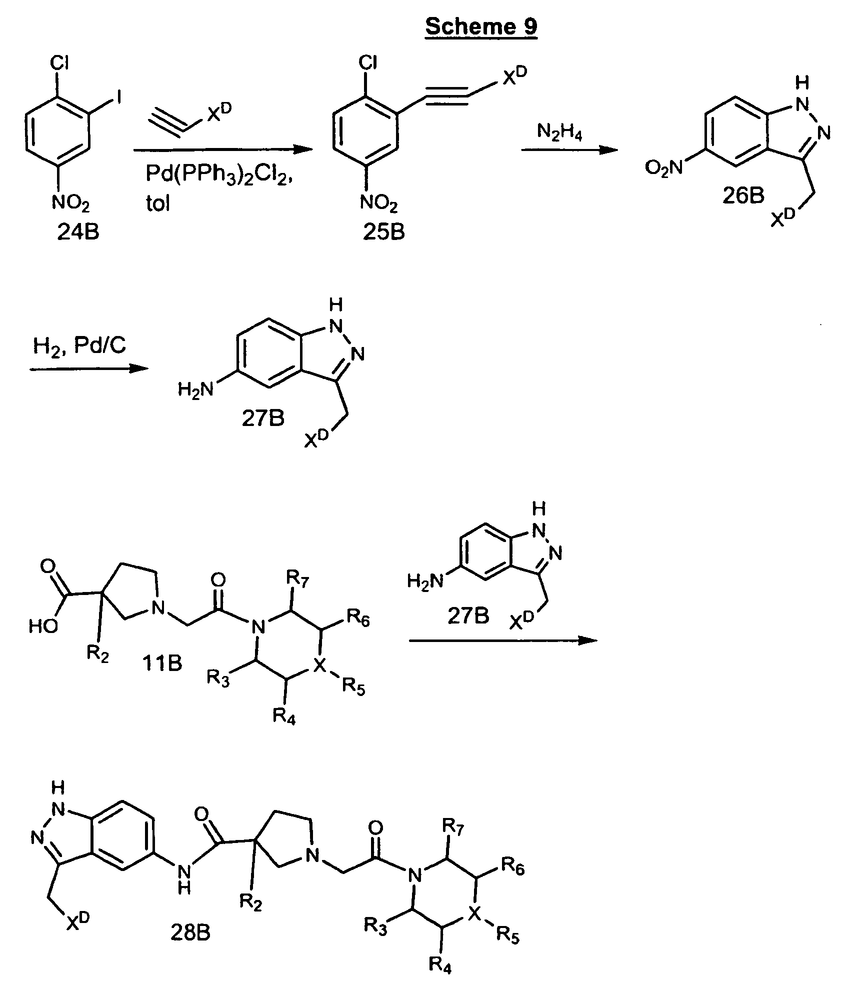

Indazoles of type 27B can be prepared by reacting 24B with the appropriately substituted acetylene in the presence of Pd(PPh₃)₂Cl₂ in toluene to obtain 25B. After heating with hydrazine to obtain 26B, 27B is obtained after hydrogenation. Coupling of 27B with 11 B under standard coupling conditions such as HATU or EDC/HOBt gives 28B.

The moiety -CH₂X^{D} represents R¹ groups that are an R¹⁰ substituent, such as, alkyl, heteroarylalkyl, and arylalkyl.

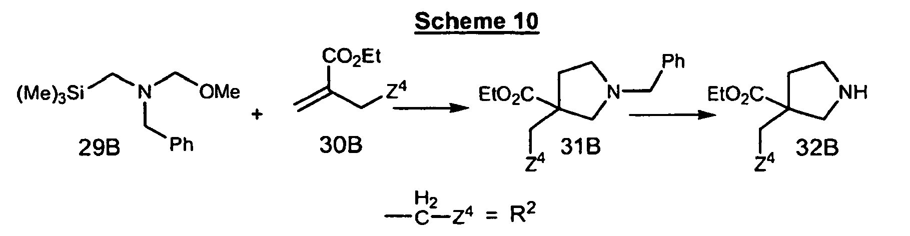

The appropriately substituted pyrrolidine 32B can be obtained by reacting 29B with the appropriately substituted compound 30B in the presence of trifluoroacetic acid to obtain 31 B. Compound 31 B can then be deprotection under hydrogenation conditions (Pd/C, H₂ to obtain 32B).

### Alternative Process For Scheme 10

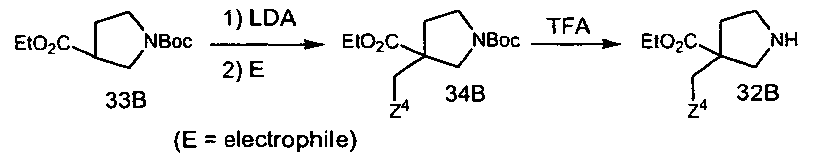

Alternatively, 32B can be obtain by reaction 33B with LDA followed by the addition of a suitable electrophile such as allylbromide, as in example 127, to obtain 34B. Treatment of 34B with trifluoroacetic acid yields 32B. 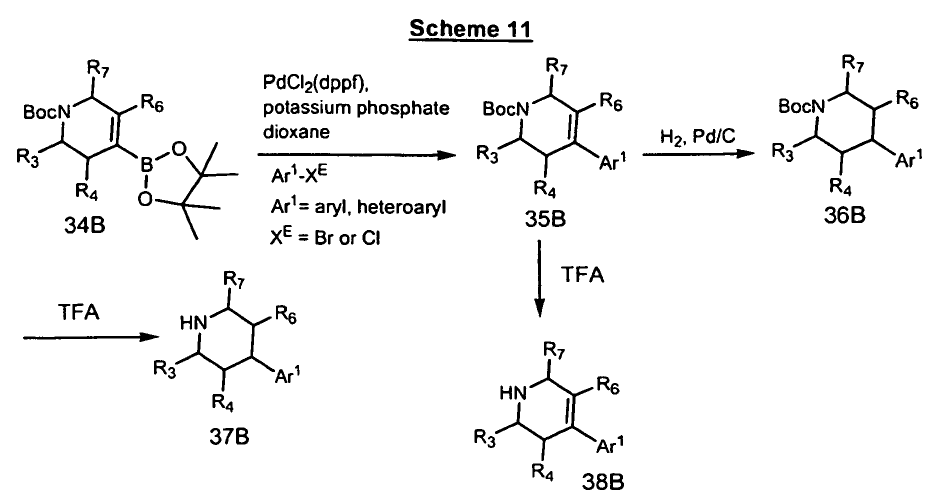 Aryl or heteroaryl substituted piperidines can be prepared by Suzuki coupling of an aryl or heteroaryl halide with the pinicolboronate 34B to obtain 35B. The ring double bond can then be hydrogenated to obtain 36B followed by removal of the Boc protecting group under trifluoroacetic acid conditions. Alternatively the double bond can be retained and the Boc group removed to give 38B.

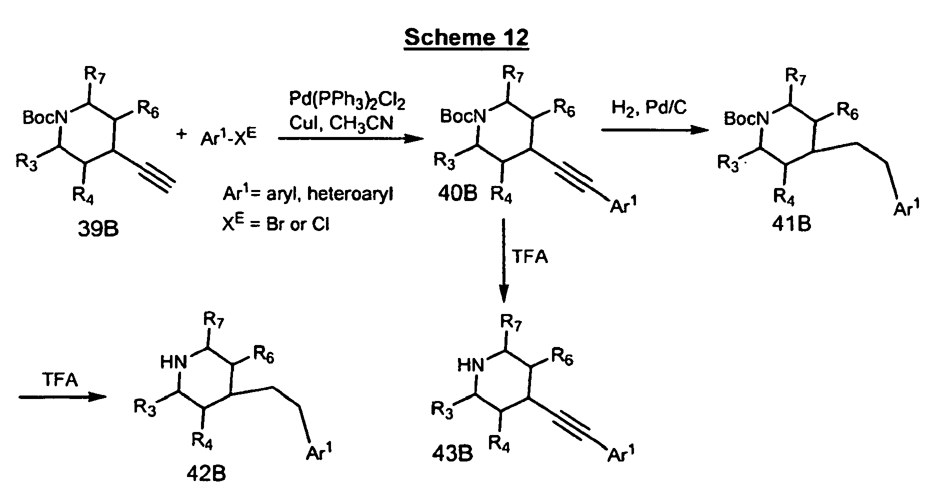

Similarly aryl or heteroaryl substituted piperizines with a 2 carbon spacer can be prepared as shown in Scheme 12 by coupling an aryl or heteroaryl halide with an acetylene derivative 39B that can be prepared according to procedures known in the art to obtain 40B. 40B can then be reduced to 41 B followed by by removal of the Boc protecting group under trifluoroacetic acid conditions. Alternatively the Boc protecting group from 40B can be removed under trifluoroacetic acid conditions to give 43B.

Compounds of this invention are exemplified in the following examples, which should not be construed as limiting the scope of the disclosure. Alternative mechanistic pathways and analogous structures within the scope of the invention may be apparent to those skilled in the art.

The LCMS conditions are: (1) column: C-18 reverse phase, 5um, 4.6 x 50 mm, (2) MS:PE Sciex API-150EX, and (3) HPLC: Shimadzu LC-10 ADvp, 1ml/min, linerar gradient 10% acetonitirle in water to 95% acetonitrile in water, both contain 0.05% TFA

### EXAMPLE 1

### Step (1)

### Preparation of Pyrrolidine-3-carboxylic acid methyl ester

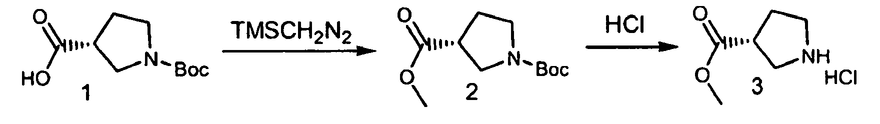

R-pyrrolidine-1,3-dicarboxylic acid 1-tert-butyl ester (2.15 gm, 10 mmol) was dissolved in 12 ml of toluene and 3.5 ml of methanol. Trimethylsilyldiazomethane 2N solution in hexanes( 6.56 ml, 13.12 mmol) was added dropwise and the reaction mixture stirred for 2 hours. The mixture was evaporated to obtain 2.1 gm of an oil. The oil was dissolved in dichloromethane (15 ml) and 5 ml of 4N hydrochloric acid in dioxane added. The reaction mixture was stirred for 1 hr and evaporated to give an oil that crystallizes to give 1.68 gm of title product.

### Step 2

### Preparation of 1-tert-Butoxycarbonylmethyl-pyrrolidine-3-carboxylic acid methyl ester

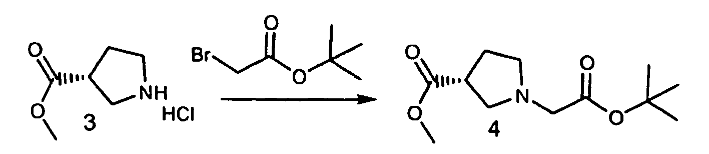

R-pyrrolidine-3-carboxylic acid methyl ester (1.5 gm, 9.1 mmol) was dissolved in N,N-dimethylformamide (45 ml). Diisopropylethylamine (5.7 ml, 31 ml) was added followed by cesium carbonate (4.35 gm, 13.3 mmol). Tert. butylbromoacetate (1.5 ml, 10 mmol) was added dropwise and the reaction mixture stirred for 1 hr. Brine was added to the reaction mixture which was then extracted with ethylacetate three times. The ethylacetate extracts were dried over magnesium sulfate, filtered and evaporated to obtain crude title product. The crude product was chromatographed to obtain 2.15 gm, 97% of title product.

### Step 3

### Preparation of 1-Carboxymethyl-pyrrolidine-3-carboxylic acid methyl ester

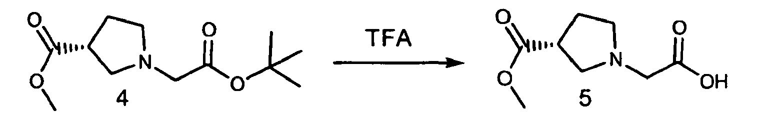

R-1-tert-Butoxycarbonylmethyl-pyrrolidine-3-carboxylic acid methyl ester ( 2.15 gm, 8.8 mmol) was dissolved in 20 ml of 50% trifluoroacetic acid/dichloromethane and stirred for 2 hrs. The reaction mixture was evaporated to an oil and exchanged with hydrochloric acid by dissolving in 20 ml of dichloromethane and adding 10 ml of 1 N HCl in ether to obtain 3.35 gm of a gummy solid.

### Step 4

### Preparation of 4-(4-Bromo-phenyl)-piperazine-1-carboxylic acid tert-butyl ester

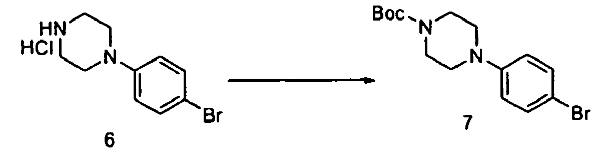

1-(4-Bromo-phenyl)-piperazine hydrochloride( 9 gm, 38 mmol) was dissolved in 250 ml of dichloromethane and 9 ml of triethylamine added. Di-tert.butyldicarbonate (8.34 gm, 39 mmol) was added and the reaction mixture stirred for 1 hr. The reaction mixture was washed with a solution of saturated sodium bicarbonate (100 ml), the organic layer separated, dried over magnesium sulfate and evaporated to obtain 10.19 gm of crystalline product.

### Step 5

### Preparation of 4-(4-boronic acid-phenyl)-piperazine-1-carboxylic acid tert-butyl ester

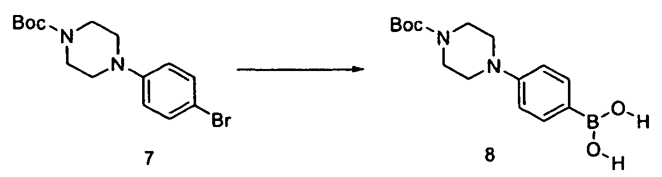

4-(4-Bromo-phenyl)-piperazine-1-carboxylic acid tert-butyl ester (10.19 gm, 30 mmol) was dissolved in 26 ml of tetrahydrofuran. The mixture was cooled to -78 C under a dry nitrogen atmosphere. A 2.5 N solution nButyl lithium in hexanes (26 ml, 65 mmol) was added dropwise and stirred for 30 min. Triisopropylborate ( 14.68 ml, 63.6 mmol) was added over 10 min. and the reaction mixture let warm to ambient temperature gradually. The reaction mixture was stirred for 18 hrs. A saturated solution of Ammonium chloride (75 ml) was added and the reaction mixture stirred for 5 min. 85% o-Phosphoric acid (7.27 gm) was added and the reaction mixture stirred for 1 hr. The reaction mixture was extracted with ethylacetate three times, dried over magnesium sulfate, filtered and evaporated. The crude product was chromatographed on a silica column to obtain 5.74 gm of title product.

### Step 6

### Preparation of 4-(4-Pyrimidin-2-yl-phenyl)-piperazine-1-carboxylic acid tert-butyl ester

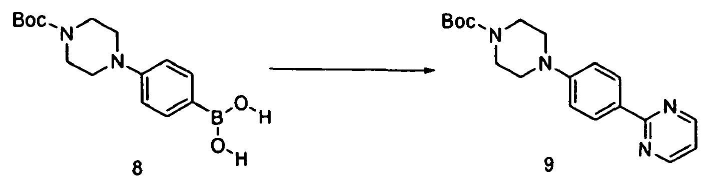

4-(4-boronic acid-phenyl)-piperazine-1-carboxylic acid tert-butyl ester (5.93 gm, 19.3 mmol) was dissolved in 50 ml of a 50% mixture of N,N-dimethylformamide/water. K2CO3 ( 16 gm) was added and the mixture de-gased and purged with nitrogen. Pd (dppf)2Cl2 ( 1.57 gm) and 2-chloropyrimidine (2.72 gm) was added and the reaction mixture stirred at 80 C. After 8 hours the product was extracted into ethylacetate, dried over magnesium sulfate, filtered and evaporated. The crude product was chromatographed on silica gel to obtain 5.03 gm (76.6%) of title product.

### Step 7

### Preparation of 2-(4-Piperazin-1-yl-phenyl)-pyrimidine

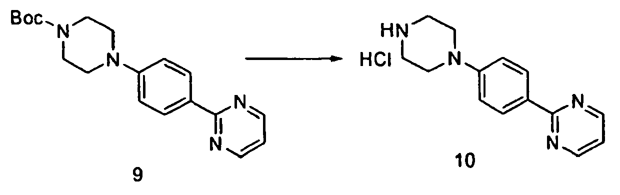

4-(4-Pyrimidin-2-yl-phenyl)-piperazine-1-carboxylic acid tert-butyl ester 5.03 gm was dissolved in 25 ml dichloromethane and 10 ml of 4N HCl dioxane added. After stirring for 2 hrs, the mixture was then evaporated to obtain the title product.

### Step 8

### Preparation of 1-{2-Oxo-2-[4-(4-pyrimidin-2-yl-phenyl)-piperazin-1-yl]-ethyl}-pyrrolidine-3-carboxylic acid methyl ester

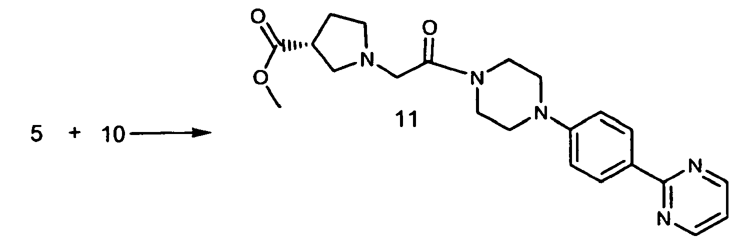

2-(4-Piperazin-1-yl-phenyl)-pyrimidine (compound 10 from Step 7, 14.7 mmol) and 1-Carboxymethyl-pyrrolidine-3-carboxylic acid methyl ester (compound 5 from Step 3, 17.6 mmol) were dissolved in 72 ml of DMF. Triethylamine (8 ml, 57 mmol), 1-hydroxybenztriazole ( 2.29 gm ) and 1-(3-dimethylaminopropyl)-3-ethylcarbodiimide hydrochloride (EDCI) (3.43 gm, 18 mmol) were added and the reaction mixture stirred for 24 hrs. After washing with brine, extracting with dichloromethane, and drying over magnesium sulfate, the mixture was evaporated and chromatographed on silica gel to obtain 5.0 gm of title product.

### Step 9

### Preparation of 1-{2-Oxo-2-[4-(4-pyrimidin-2-yl-phenyl)-piperazin-1-yl]-ethyl}-pyrrolidine-3-carboxylic acid

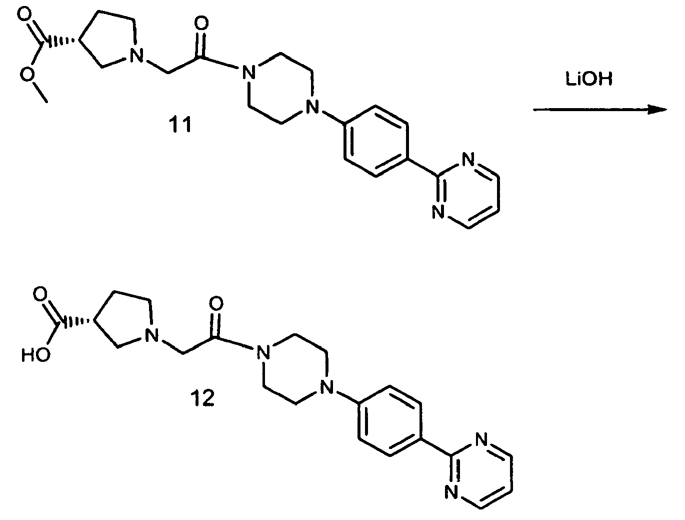

1-{2-Oxo-2-[4-(4-pyrimidin-2-yl-phenyl)-piperazin-1-yl]-ethyl}-pyrrolidine-3-carboxylic acid methyl ester (compound 11 from Step 8, 3.3 gm, 8.06 mmol) was dissolved in methanol and 10 ml of 1 N lithium hydroxide added. The reaction mixture was stirred for 18 hrs. 10 ml of 1 N HCl was added to the reaction mixture and evaporated to a white solid (3.94 gm)

### Step 10

### Preparation of 1-{2-Oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide

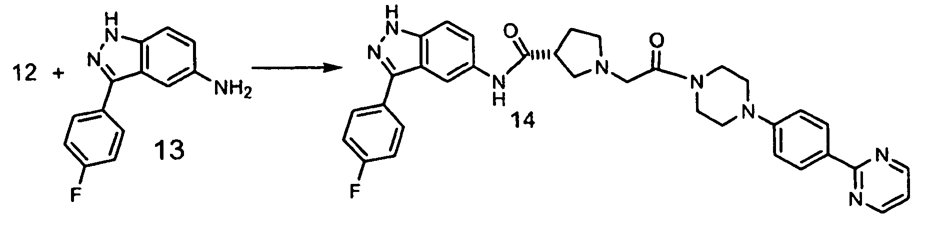

3-(4-Fluoro-phenyl)-1H-indazol-5-ylamine ( 0.11gm, 0.5 mmol) and 1-{2-Oxo-2-[4-(4-pyrimidin-2-yl-phenyl)-piperazin-1-yl]-ethyl}-pyrrolidine-3-carboxylic acid (compound 12 from Step 9, 0.125 gm, 0.25 mmol) were dissolved in 4 ml of DMF. O-(7-azabenzotriazol-1-yl-)-N,N,N,N-tetramethyluronium hexafluorophosphate (HATU) (0.24 gm, 0.63 mmol) and triethylamine ( 0.1 ml, 0.76 mmol) were added to the reaction mixture and the mixture stirred for 18 hrs. The reaction mixture was added to bring and extracted with dichloromethane. After drying over magnesium sulfate and filtered, the mixture was evaporated and chromatographed to obtain 36.7 mg of title product.

### EXAMPLE 2

### Step 1

### Preparation of 3-Bromo-1H-indazol-5-ylamine

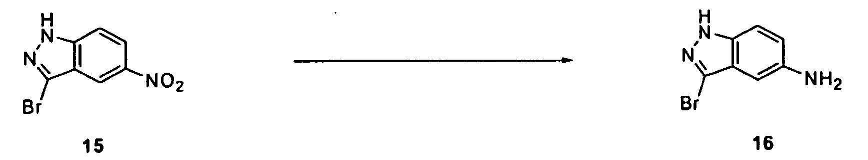

Bromide **15** (4.2 g, 17.4 mmol, prepared according to procedure of Barbet, Eur. J. Med. Chem. Chim. Ther.; Fr; 21; 4; 1986, 359) and stannous chloride hydrate (17.0 g, 75.3 mmol) were dissolved in EtOH (35 mL). The crude was stirred at 70 °C for 2.5 hrs. The crude was cooled to rt and poured into ice water (50 ml). The PH was made basic via addition of 15% wt NaOH (100 mL). The aq layer was extracted with EtOAc. The EtOAc layer was dried over MgSO₄, filtered, and evaporated to give 1.82 g of the crude product.

### Step 2

### Preparation of 1-{2-Oxo-2-[4-(4-pyrimidin-2-yl-phenyl)-piperazin-1-yl]-ethyl}1-pyrrolidine-3-carboxylic acid (3-bromo-1H-indazol-5-yl)-amide

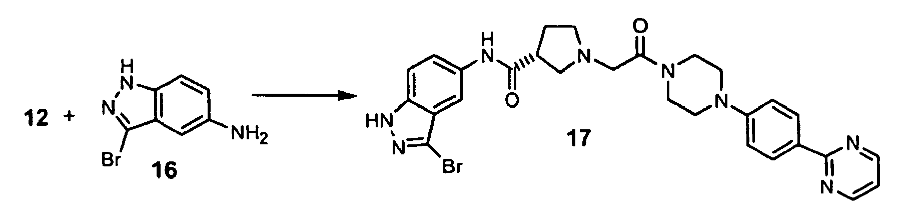

Crude 3-bromo-1H-indazol-5-ylamine **16** (403 mg, 1.9 mmol) and carboxylic acid **12** from Example 1 Step 9 (357 mg, 0.90 mmol) were dissolved in CH₂Cl₂ (3 mL) and DMF (3 ML). To the crude was added triethylamine (1.01 mL), and HATU (722 mg, 1.9 mmol). The crude was stirred at rt for 5 hrs under a stream of nitrogen. To the crude was added additional portions of 3-bromo-1H-indazol-5-ylamine **16** (300 mg, 1.42 mmol), HATU (500 mg, 1.32 mmol), and diisopropylethylamine (0.3 mL). To the crude was added CH₂Cl₂ (5 mL) and DMF (3 ML). The crude was stirred overnight under a stream of nitrogen. The crude was quenched with sat. NaHCO₃ at rt. The crude was diluted in EtOAc. The organic layer was washed with water, brine, dried over Na₂SO₄, filtered, and evaporated. The crude was chromatographed to give 367 mg of the product.

### Step 3

### Preparation of 1-{2-Oxo-2-[4-(4-pyrimidin-2-yl-phenyl)-piperazin-1-yl]-ethyl}-pyrrolidine-3-carboxylic acid[3-(3-cyano-phenyl)-1H-indazol-5-yl]-amide

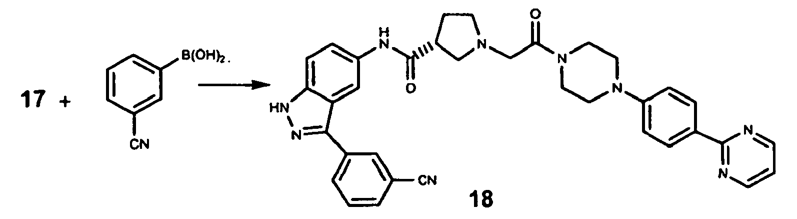

To a solution of bromide **17** from Step 2 (107 mg, 0.18 mmol) in DME (2 mL) and distilled water (0.5 mL) in a 5 mL conical microwave reaction vessel was added boronic acid (107 mg, 0.73 mmol), Na₂CO₃ (100 mg, 0.94 mmol), and PdCl₂dppf (59 mg, 0.072 mmol). The crude was sealed with reaction vessel cap. The crude was heated at 120 °C in an EMRY optimizer microwave for 600 secs. The crude was cooled and quenched with water at rt. The aq layer was extracted with CH₂Cl₂. The organic layer was dried over Na₂SO₄, filtered, and evaporated. The crude was purified via prep plate to give 55 mg of the product. LC Mass Spec M + 1 @retention time: 612.1@3.17

### EXAMPLES 3 to 60

Following a procedure similar to that of Example 2, compound **17** was reacted with R¹B(OH)₂ to prepare, via the microwave Suzuki reaction, the compound **19**: 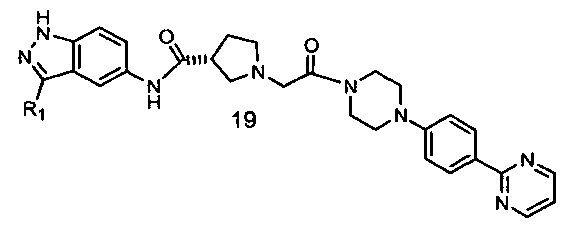 wherein R¹ is defined in Table 1. In Table 1 "Ex" represents "Example".

**Table 1**

| **Ex** | **R¹** | **LCMass Spec M+1 @ ret. time** |
|---|---|---|
| 3 | 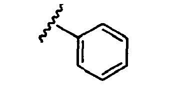 | 587 @ 3.61 mi |
| 4 | 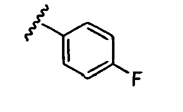 | ------ |
| 5 | 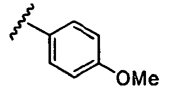 | 617.1@2.86 min |
| 6 | 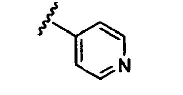 | ------ |
| 7 | 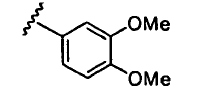 | 647.2 @ 2.83 |
| 8 | 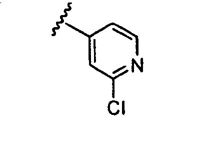 | ------ |
| 9 | 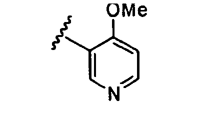 | ------ |
| 10 | 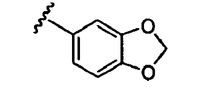 | ------ |
| 11 | 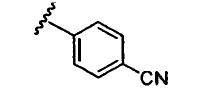 | 612.1 @ 3.09 |
| 12 | 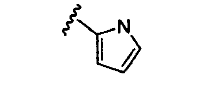 | 576.3 @2.5 |
| 13 | 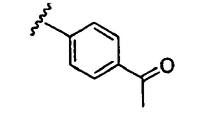 | 629.3 @ 2.57 |
| 14 | 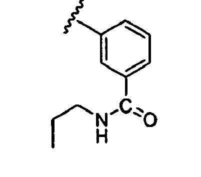 | ------ |
| 15 | 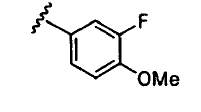 | ------ |
| 16 | 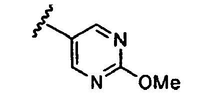 | ------ |
| 17 | 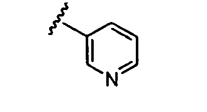 | ------ |
| 18 | 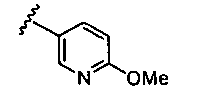 | ------ |
| 19 | 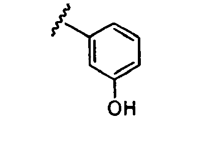 | 603.1 @ 2.87 |
| 20 | 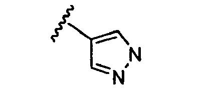 | ------ |
| 21 | 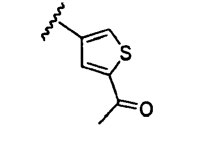 | 635.2 @ 3.13 |
| 22 | 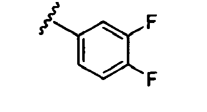 | ------ |
| 23 | 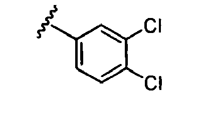 | ------ |
| 24 | 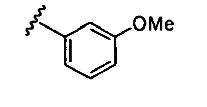 | 617.1 @ 3.24 |
| 25 | 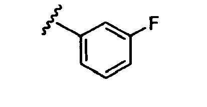 | 605.1 @ 3.24 |
| 26 | 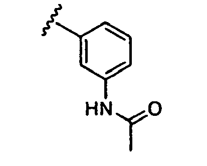 | 644.2 @ 2.87 |
| 27 | 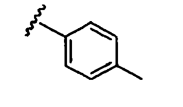 | 601.1 @ 3.29 |
| 28 | 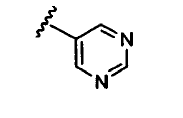 | ------ |
| 29 | 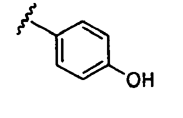 | 601.1 @ 2.81 |
| 30 | 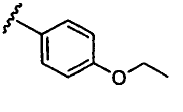 | 631.2 @ 3.13 |
| 31 | 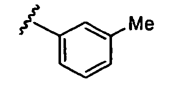 | 601.1 @ 3.27 |
| 32 | 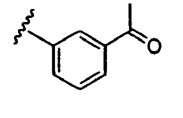 | 629.1 @ 3.02 |
| 33 | 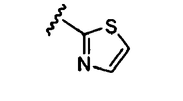 | 594 @ 3.35 |
| 34 | 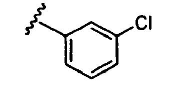 | 621.1 @ 3.40 |
| 35 | 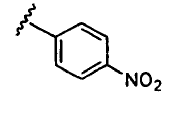 | 632.1 @ 3.31 |
| 36 | 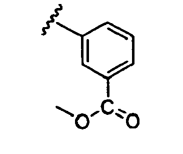 | 645.2 @ 3.22 |
| 37 | 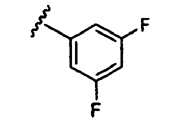 | ------ |
| 38 | 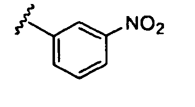 | 632.1 @ 3.29 |
| 39 | 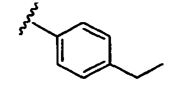 | 615.1 @ 3.24 |
| 40 | 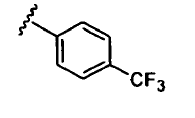 | 655.1 @ 3.48 |
| 41 | 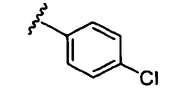 | 621.1 @3.38 |
| 42 | 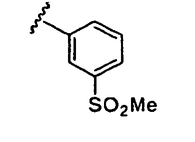 | 652 @2.97 |
| 43 | 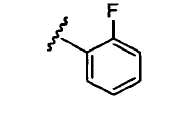 | 605.1 @2.94 |
| 44 | 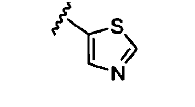 | 594 @ 3.13 |
| 45 | 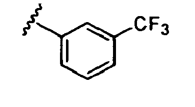 | 655.1 @3.48 |
| 46 | 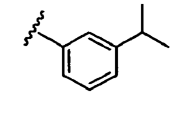 | 629.1 @3.51 |
| 47 | 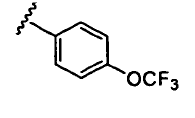 | ------ |
| 48 | 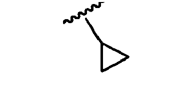 | ------ |
| 49 | 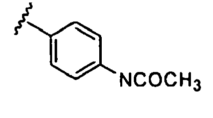 | 644.1 @ 3.54 |
| 50 | 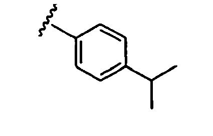 | 629.1 @3.56 |
| 51 | 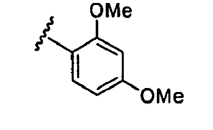 | 647.2 @2.9 |
| 52 | 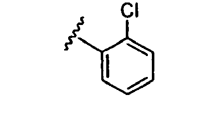 | 621.1 @2.73 |
| 53 | 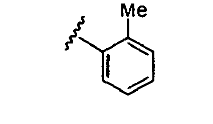 | 601.1 @3.13 |
| 54 | 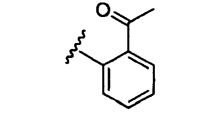 | 629.1 @2.98 |
| 55 | 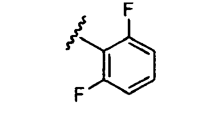 | 623.1 @3.08 |
| 56 | 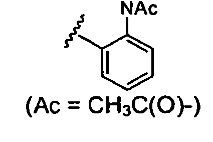 | 644.2 @2.85 |
| 57 | 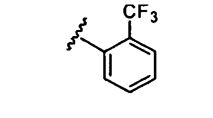 | 655.1 @3.38 |
| 58 | 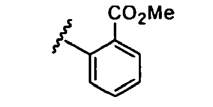 | 645.2 @3.05 |
| 59 | 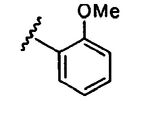 | 617.1 @2.83 |
| 60 | 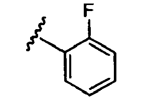 | 605.1 @2.94 |

### EXAMPLE 61

### Step 1

### Preparation of 3-Bromo-5-nitro-1-trityl-1H-indazole

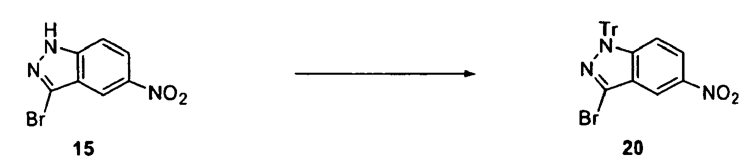

To a solution of **15** (3.8 g, 15.7 mmol) in CH₃CN (100 mL) was added potassium carbonate (10.42 g, 75.4 mmol) followed by the addition of TrCl (15.9 g, 56.4 mmol) at rt for 48 h. The solvent was evaporated and the crude was dissolved in CH₂Cl₂. The crude was quenched with water. The aq. layer was extracted with CH₂Cl₂. The combined organic layer was washed with H₂O, brine, dried over MgSO₄, filtered, and evaporated. The solid crude was placed onto a filter funnel and washed with 20% EtOAc / hexane (1 L) and 5% EtOAc / hexane (500 mL). The crude solid was collected from the filter and dried overnight to afford 7 g of the product.

### Step 2

### Preparation of 5-Nitro-1-trityl-3-(1-trityl-1H-imidazol-4-yl)-1H-indazole

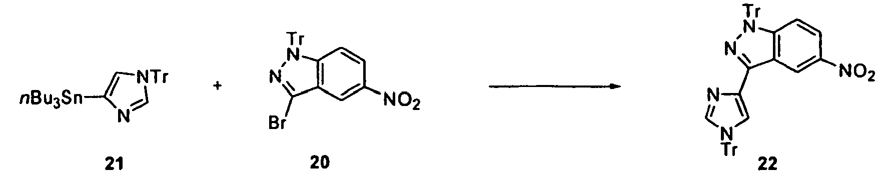

A solution of **21** (2.73 g, 6.19 mmol, prepared according to Jetter, M. C.; Reitz, A. B. Synthesis, 1998, 829-831), bromide **36** (0.73 g, 2.07 mmol) and Pd(PPh)₄ (474 mg, 0.41 mmol) in toluene (10 mL) was heated at 100 °C under an atmosphere of nitrogen for 18 hrs. The crude was cooled, evaporated, and chromatographed to provide 730 mg of the product.

### Step 3

### Preparation of 1-Trityl-3-(1-trityl-1H-imidazol-4-yl)-1H-indazol-5-ylamine

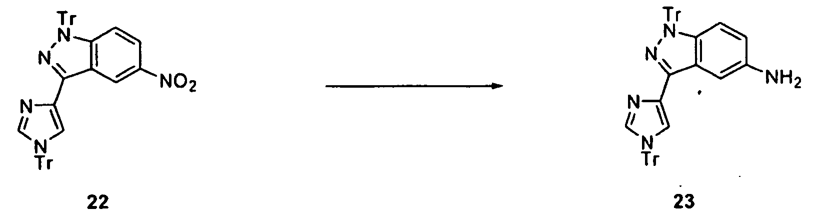

To a solution of **22** (730 mg, 1.02 mmol g, 17.4 mmol) in MeOH (150 mL) was added 10% wt Pd/C (700 mg). The crude was capped with a 3-way stopcock with connections to both vaccum and also a balloon of hydrogen gas. The crude was degassed using house vacuum and represurrized with hydrogen gas. The process was repeated 5X. The crude was stirred under an atmosphere of hydrogen at rt for 3 hrs. The crude was filtered through a microfiber filter. The filtrate was evaporated and used in the following reaction without further purification.

### Step 4

### Preparation of 3-(1H-Imidazol-4-yl)-1H-indazol-5-ylamine

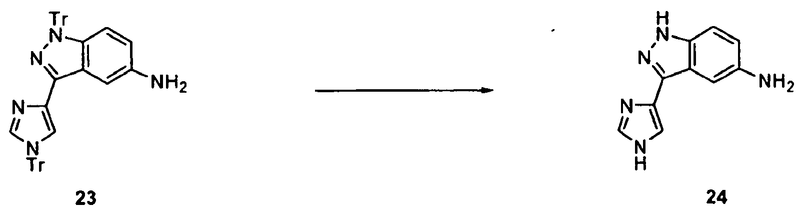

To a solution of ylamine **23** (crude 420 mg, 1.02 mmol) in CH₂Cl₂ (8 mL) and H₂O (1 mL) was added TFA (1 mL) at rt. The crude was stirred at rt for 60 hrs. The crude was quenched with sat. NaHCO₃ at rt. The crude was evaporated and filtered through a plug of Na₂SO₄. The crude was evaporated and purified via preparation plates to give 120 mg of the product.

### Step 5

### Preparation of 1-{2-Oxo-2-[4-(4-pyrimidin-2-yl-phenyl)-piperazin-1-yl]-ethyl}-pyrrolidine-3-carboxylic acid [3-(1H-imidazol-4-yl)-1H-indazol-5-yl]-amide

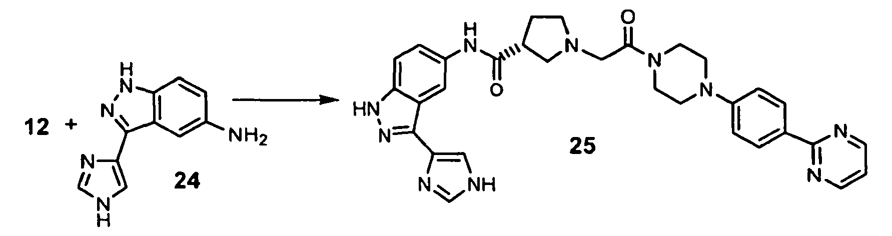

Amine **24** (82 mg, 0.41 mmol) and carboxylic acid **12** (81 mg, 0.21 mmol) were dissolved in CH₂Cl₂ (2 mL) and DMF (2 ML). To the crude was added TEA (1.16 mL), and HATU (156 mg, 0.41 mmol). The crude was stirred at rt for 22 hrs under a stream of nitrogen. The crude was quenched with sat. NaHCO₃ at rt and filtered over a plug of silica gel. The crude was evaporated and purified via preparation plates to give 17 mg of the product.

### EXAMPLE 62

### Step 1

### Preparation of 3-Bromo-5-nitro-1-(2-trimethylsilanyl-ethoxymethyl)-1H-indazole

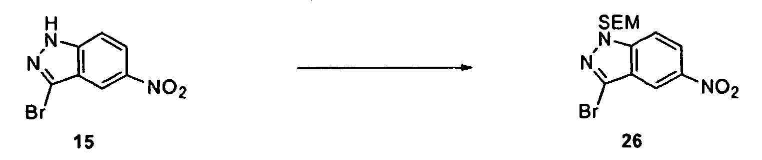

To a solution of **15** (7.9 g, 32.6 mmol) in DMF (163 mL) was added 60% wt NaH (2.0 g, 49 mmol) portionwise over 5 mins at rt. The crude was stirred at rt for 30 mins before the addition of SEMCI (7.5 mL, 42.4 mmol) at rt. The crude was stirred at rt for 58 hrs. The crude was quenched with H₂O and diluted in EtOAc. The aq. layer was extracted with EtOAc 3X. The combined organic layer was washed with H₂O, brine, dried over MgSO₄, filtered, and evaporated. The crude was chromatographed to give 7.4 g of the product.

### Step 2

### Preparation of 5-Amino-1-(2-trimethylsilanyl-ethoxymethyl)-1H-indazole-3-carbonitrile

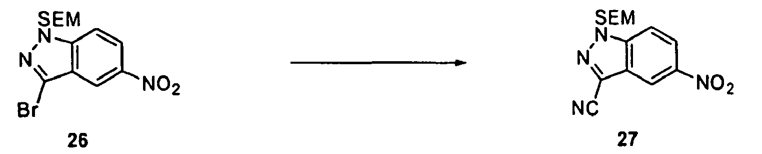

To a solution of bromide **26** (550 mg, 1.34 mmol) in DMF (5 mL) in a 10 mL microwave reaction vessel was added ZnCN₂ (160 mg, 1.34 mmol), and PdCl₂dppf (320 mg, 0.39 mmol). The crude was sealed with reaction vessel cap. The crude was heated at 180 °C in an EMRY optimizer microwave for 1800 secs. The crude was cooled and quenched with water at rt. The crude was diluted in EtOAc and washed with H₂O 3X. The organic layer was dried over Na₂SO₄, filtered, and evaporated. The crude was chromatographed to give 150 mg of the product.

### Step 3

### Preparation of 5-Amino-1-(2-trimethytsilanyl-ethoxymethyl)-1H-indazole-3-carbonitrile

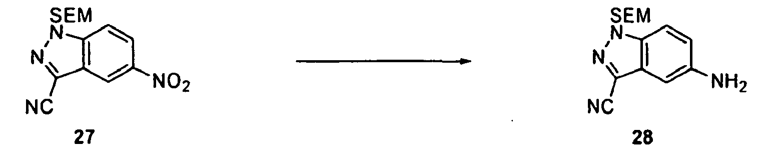

To a solution of nitrile **27** (150 mg, 0.47 mmol) in EtOAc (5 mL) was added 10% wt Pd/C (30 mg). The crude was capped with a 3-way stopcock with connections to both vaccum and also a balloon of hydrogen gas. The crude was degassed using house vacuum and represurrized with hydrogen gas. The process was repeated 5X. The crude was stirred at rt for 4 hrs. The crude was filtered through a microfiber filter. The filtrate was evaporated and purified via preparation plate to give 70 mg of the product.

### Step 4

### Preparation of 1-{2-Oxo-2-[4-(4-pyrimidin-2-yl-phenyl)-piperazin-1-yl]-ethyl}-pyrrolidine-3-carboxylic acid [3-cyano-1-(2-trimethylsilanyl-ethoxymethyl)-1H-indazol-5-yl]-amide

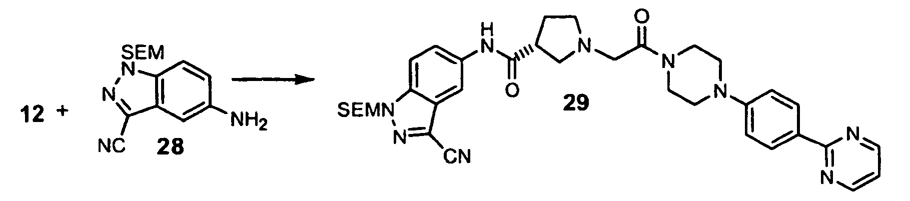

Amine **28** (242 mg, 0.84 mmol) and carboxylic acid **12** (162 mg, 0.42 mmol) were dissolved in CH₂Cl₂ (2 mL) and DMF (2 ML). To the crude was added DIPEA (0.3 mL, 1.68 mmol), and HATU (326 mg, 0.84 mmol). The crude was stirred at rt for 18 hrs under a stream of nitrogen. The crude was quenched with sat. NaHCO₃ at rt. The crude was diluted in EtOAc. The organic layer was washed with water, brine, dried over Na₂SO₄, filtered, and evaporated. The crude was chromatographed to give 212 mg of the product.

### Step 5

### Preparation of 1-{2-Oxo-2-[4-(4-pyrimidin-2-yl-phenyl)-piperazin-1-yl]-ethyl}-pyrrolidine-3-carboxylic acid (3-cyano-1H-indazol-5-yl)-amide

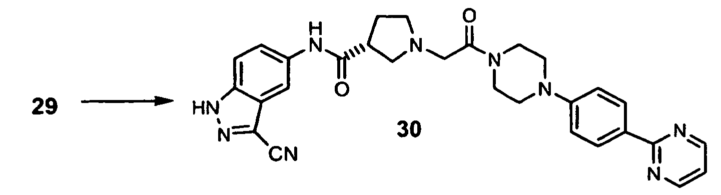

To a solution of **29** (20 mg, 0.03 mmol) in CH₂Cl₂ (2 mL) was added TFA (2 mL) at rt. The crude was stirred at rt for 18 hrs. The crude was evaporated and quenched with sat. NaHCO₃ at rt. The crude was evaporated and filtered through a plug of silica gel and evaporated. The crude was purified via preparation plate to give 13 mg of the product.

### EXAMPLE 63

### Step 1

### Preparation of 1-{2-Oxo-2-[4-(4-pyrimidin-2-yl-phenyl)-piperazin-1-yl]-ethyl}-pyrrolidine-3-carboxylic acid [3-(1H-tetrazol-5-yl)-1-(2-trimethylsilanyl-ethoxymethyl)-1H-indazol-5-yl]-amide

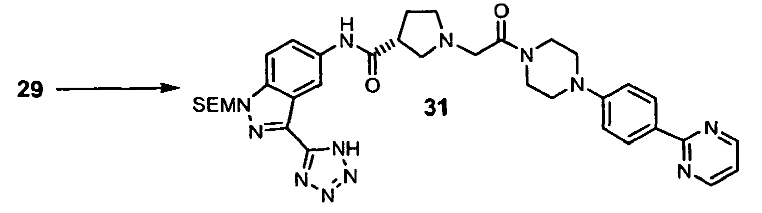

To a solution of **29** from Example 62 Step 4 (40 mg, 0.061 mmol) in DMF was added NaN₃ (12 mg, 0.18 mmol) and NH₄Cl (13 mg, 0.24 mmol). The crude was stirred at 100 °C for 18 hrs. The crude was cool, evaporated, and filtered through a plug of cotton. The crude was evaporated and purified via preparation plate to give 31 mg of a mixture of two isomers.

### Step 2

### Preparation of 1-{2-Oxo-2-[4-(4-pyrimidin-2-yl-phenyl)-piperazin-1-yl]-ethyl}-pyrrolidine-3-carboxylic acid [3-(1H-tetrazol-5-yl)-1H-indazol-5-yl]-amide

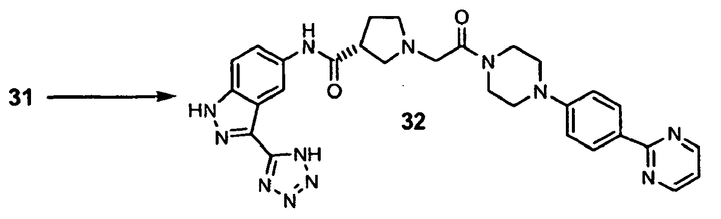

To a solution of **31** from Step 1 (31 mg, 0.054 mmol, 2 isomers) in CH₂Cl₂ (2 mL) was added TFA (2 mL) at rt. The crude was stirred at rt for 5 hrs. The crude was evaporated and quenched with sat. NaHCO₃ at rt. The crude was evaporated and filtered through a plug of silica gel and evaporated. The crude was purified via preparation plate to give 13 mg of the product.

### EXAMPLE 64

### Step 1

### Preparation of 5-[(1-{2-Oxo-2-[4-(4-pyrimidin-2-yl-phenyl)-piperazin-1-yl]-ethyl}-pyrrolidine-3-carbonyl)-amino]-1-(2-trimethylsilanyl-ethoxymethyl)-1H-indazole-3-carboximidic acid

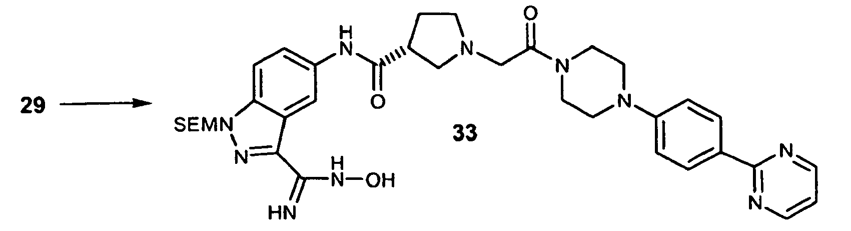

To a solution of nitrile **29** from Example 63 Step 4 (40 mg, 0.06 mmol) in EtOH (0.4 mL) was added DIPEA (0.1 mL, 0.6 mmol) and NH₂OH.HCl (21 mg, 0.3 mmol) at rt. The crude was stirred at 45 °C for 18 hrs. The crude was cooled, evaporated, and filtered through a plug of cotton. The crude was evaporated and purified via preparation plate to give 18 mg of the product.

### Step 2

### Preparation of 5-[(1-{2-Oxo-2-[4-(4-pyrimidin-2-yl-phenyl)-piperazin-1-yl]-ethyl}-pyrrolidine-3-carbonyl)-amino]-1H-indazole-3-carboximidic acid

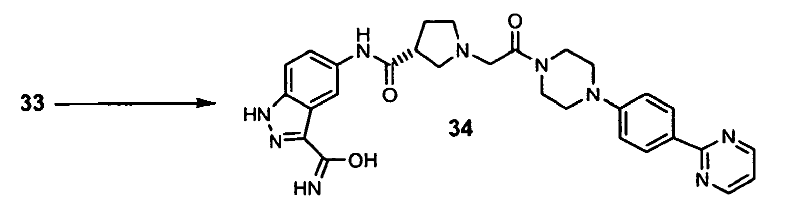

To a solution of **33** from Step 1 (18 mg, 0.03 mmol) in CH₂Cl₂ (2 mL) was added TFA (2 mL) at rt. The crude was stirred at rt for 5 hrs. The crude was evaporated and quenched with sat. NaHCO₃ at rt. The crude was evaporated and filtered through a plug of silica gel and evaporated. The crude was purified via preparation plate to give 18 mg of the product.

### EXAMPLE 65

### Step 1

### Preparation of 3-Amino-5-[(1-{2-oxo-2-[4-(4-pyrimidin-2-yl-phenyl)-piperazin-1-yl]-ethyl}-pyrrolidine-3-carbonyl)-amino]-indazole-1-carboxylic acid tert-butyl ester

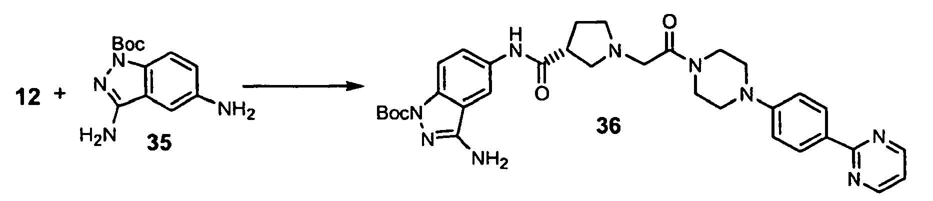

To a solution of amine **35** (283 mg, 1.14 mmol, prepared according to WO 03/064397) in CH₂Cl₂ (5 mL) and DMF (3 ML) was added carboxylic acid **12** from Example 1 Step 9 (375 mg, 0.95 mmol), diisopropylethylamine (0.4 mL, 2.28 mmol), and HATU (433 mg, 1.14 mmol). The crude was stirred overnight at rt under a stream of nitrogen. To the crude was added additional portions of amine **35** (283 mg, 1.14 mmol), HATU (433 mg, 1.14 mmol), and diisopropylethylamine (0.4 mL, 2.28 mmol). To the crude was added CH₂Cl₂ (5 mL) and DMF (3 ML). The crude was stirred at rt for a total of 85 hrs under a stream of nitrogen. The crude was quenched with sat. NaHCO₃ at rt. The crude was diluted in EtOAc. The organic layer was washed with water, brine, dried over Na₂SO₄, filtered, and evaporated. The crude was chromatographed to give 410 mg of the product.

### Step 2

### Preparation of 3-Acetylamino-5-[(1-{2-oxo-2-[4-(4-pyrimidin-2-yl-phenyl)-piperazin-1-yl]-ethyl}-pyrrolidine-3-carbonyl)-amino]-indazole-1-carboxylic acid tert-butyl ester

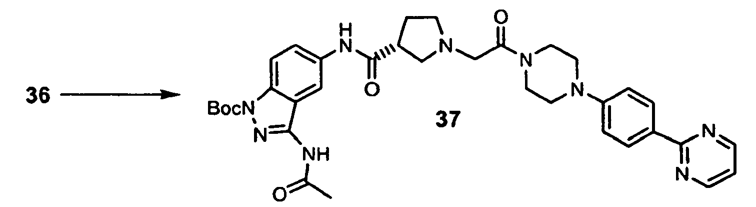

To a solution of **36** from Step 1 in CH₂Cl₂ (0.3 mL) and pyridine (0.3 mL) was added acetyl chloride (50 ul, 0.7 mmol) at rt. The crude was stirred at rt for 30 mins. The crude was quenched with sat. NaHCO₃ at rt. The crude was partitioned between EtOAc and H₂O. The organic layer was washed with H₂O, brine, dried over MgSO₄, filtered, and evaporated. The crude was chromatographed to give 20 mg of the product.

### Step 3

### Preparation of 1-{2-Oxo-2-[4-(4-pyrimidin-2-yl-phenyl)-piperazin-1-yl]-ethyl}-pyrrolidine-3-carboxylic acid (3-acetylamino-1H-indazol-5-yl)-amide

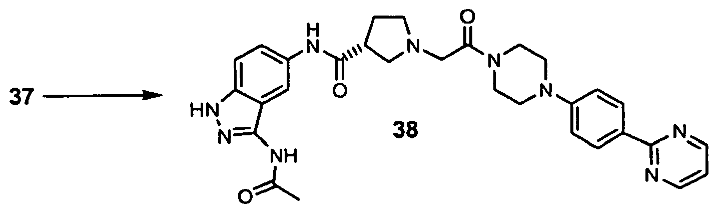

To a solution of **37** from Step 2 (20 mg, 0.03 mmol)37 in CH₂Cl₂ (2 mL) was added TFA (2 mL) at rt. The crude was stirred at rt for 17 hrs. The crude was quenched with sat. NaHCO₃ at rt. The crude was partitioned between EtOAc and H₂O. The organic layer was washed with H₂O, brine, dried over MgSO₄, filtered, and evaporated. The crude was chromatographed to give 6.1 mg of the product.

### EXAMPLES 66 - 73

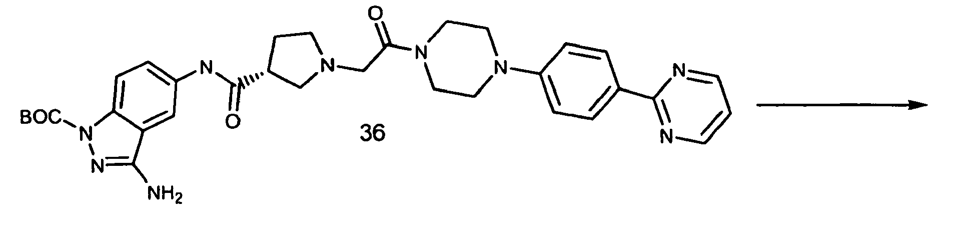

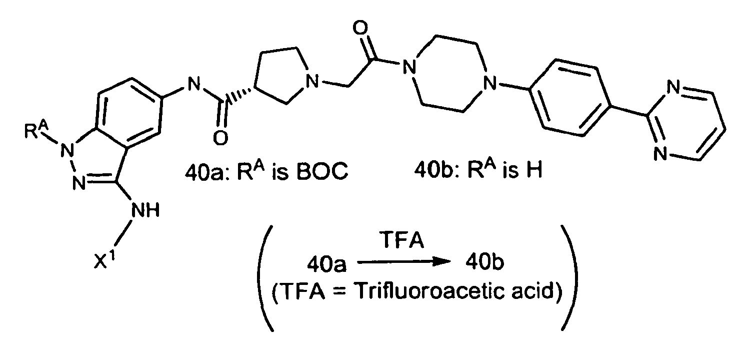

Compound 36 was used to prepare compounds of formula 40b wherein X¹ is defined in Table 2. To obtain the desired X¹ substitutent, Compound 36 is reacted with X¹' s corresponding commercially available acid chloride, isocyanate or sulfonyl chloride. The BOC group is then removed with trifluoroacetic acid. In Table 2 "Ex" represents "Example".

**TABLE 2**

| **Ex** | **X¹** | **LCMass Spec M+1 @ ret. time** |
|---|---|---|
| 66 | H | 526.1 @2.13 |
| 67 | 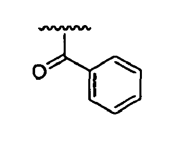 | 630.1 @2.71 |
| 68 | 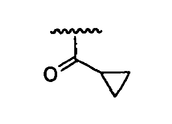 | 594.3 @2.12 |
| 69 | 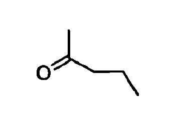 | 596.3 @2.15 |
| 70 | 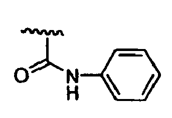 | 645.4 @2.60 |
| 71 | 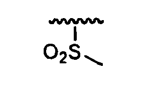 | 604.3 @ 2.10 |
| 72 | 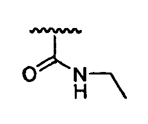 | 597.3 @2.20 |
| 73 | 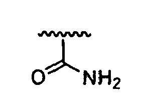 | 669.3 @ 1.98 |

### EXAMPLE 74

### Step 1

### Preparation of 3-Morpholin-4-yl-5-nitro-1H-indazole

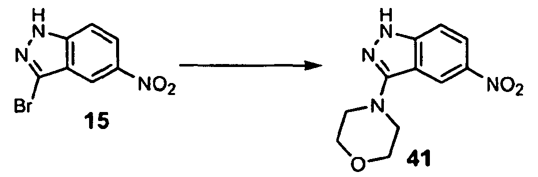

3-Bromo-5-nitro-1H-indazole ( 0.5 gm, 2 mmol) was dissolved in 3.5 ml of morpholine and heated in a sealed tube for 40 hrs. The mixture was then cooled and added to ethylacetate, washed with brine and dried over magnesium sulfate. After chromatography on silica gel using 20-50% ethylacetate/hexanes as eluent the title compound was obtained (210 mg, 0.85 mmol).

### Step 2

### Preparation of 3-Morpholin-4-yl-1H-indazol-5-ylamine

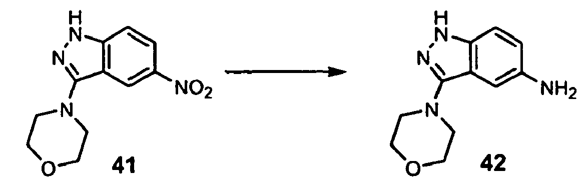

3-Morpholin-4-yl-5-nitro-1H-indazole (210 mg) was dissolved in 10 ml of methanol and the mixture hydrogenated at 1 atm of hydrogen using 10% Pd/C (50 mg) as catalyst. After 24 hrs, the mixture was filtered and evaporated for use in the next step.

### Step 3

### Preparation of 1-{2-Oxo-2-[4-(4-pyrimidin-2-yl-phenyl)-piperazin-1-yl]-ethyl}-pyrrolidine-3-carboxylic acid (3-morpholin-4-yl-1H-indazol-5-yl)-amide

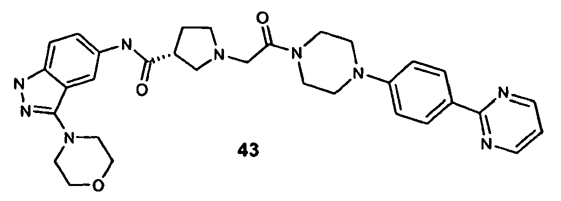

The title compound was prepared following the procedure in Example 1 Step 10 except amine 42 from Step 2 was used instead of amine 13.

### EXAMPLE 75

### Preparation of 1-{2-Oxo-2-[4-(4-pyrimidin-2-yl-phenyl)-piperazin-1-yl]-ethyl}-pyrrolidine-3-carboxylic acid (3-pyrrolidin-1-yl-1H-indazol-5-yl)-amide

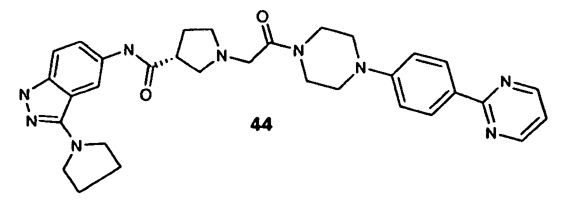

The title compound was prepared following the procedures in Example 74, Steps 1 and 2, and Example 1, Step 10, by using pyrrolidine instead of morpholine in Example 74, Step 1. The amine prepared following the procedure of Example 76, Steps 1 and 2, is used instead of amine 13 in the procedure of Example 1, Step 10 .

### EXAMPLE 76

### Step 1

### Preparation of 3-Bromomethyl-5-nitro-indazole-1-carboxylic acid tert-butyl ester

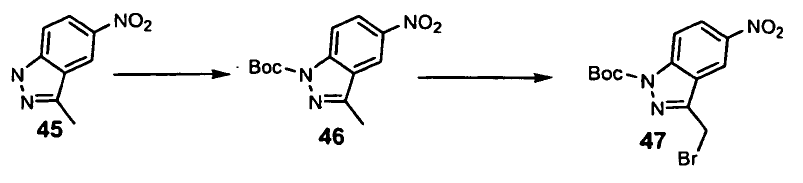

3-Methyl-5-nitro-1H-indazole ( 1.89 gm, 10.2 mmol) was dissolved in 20 ml of acetonitrile. A catalytic amount of DMAP followed by triethylamine ( 2.13 ml, 1.5 eq) and di-tert.butyldicarbonate (2.88 gm, 1.3 eq) was added and stirred for 3 hrs. The reaction mixture was evaporated to dryness and dissolved in ethyl acetate and washed with 0.1 N HCl. The ethyl acetate layer was washed twice with water, dried over magnesium sulfate and chromatographed on silica gel to give a 2.05 gm of Boc compound. The entire amount of Boc compound was dissolved in 20 ml of CCl4 and benzoylperoxide ( 179 mg, 0.74 mmol) and N-bromosuccinimide ( 1.45 gm, 8.14 mmol) were added. The reaction mixture was refluxed for 2 hrs. After 2 hrs 179 mg of benzoylperoxide was added and the reaction mixture refluxed for 18 hrs. The reaction mixture was cooled and washed with water and dried over magnesium sulfate. After chromatography on silica gel, 1.32 gm of title product was obtained.

### Step 2

### Preparation of 2-(5-Nitro-1H-indazol-3-ylmethyl)-isoindole-1,3-dione

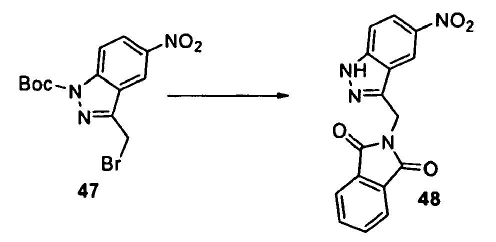

3-Bromomethyl-5-nitro-indazole-1-carboxylic acid tert-butyl ester (0.63g, 1.77 mmol) and potassium phthalimide (0.396g, 1.2 eq) were stirred in DMF (1 mL) at 80 °C for two hours. TLC (20% EtOAc/hexane) showed starting material consumed and new products formed. After DMF was evaporated, a residue, 1.33g, was obtained. The resulting residue was stirred in DCM and filtered. The filtration cake then washed with DCM followed by hexane. The evaporation of the filtrate gave 0.79g of crude. Flash chromatograph (20 - 30 % EtOAc/hexane) gave a mixture, two spots shown on TLC. During the sample preparation, solid precipitated and the solid was collected, 95 mg. MS showed de-Boc product, (M+H)⁺ at 323. An adequate amount of this mixture was treated with TFA in DCM for two hours. TLC showed one spot and MS gave (M+H)⁺ at 323. The remaining mixture was stirred in a mixture of TFA (6 mL) and DCM (10 mL) for two hours. Evaporation of TFA and DCM gave the desired product, 0.71g, (quant. yield).

### Step 3

### Preparation of 2-(5-Amino-1H-indazol-3-ylmethyl)-isoindole-1,3-dione

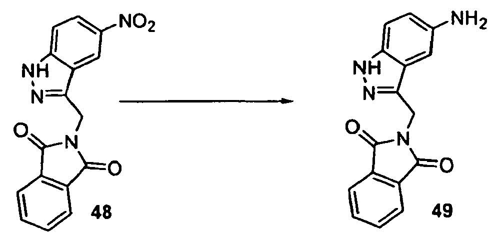

2-(5-Nitro-1H-indazol-3-ylmethyl)-isoindole-1,3-dione (0.71 gm) was dissolved in methanol and hydrogenated at 1 atm. using 10% Pd/C (catalytic) for 18 hrs. The catalyst was filtered and the mixture evaporated to obtain 614 mg of title product.

### Step 4

### Preparation of 1-{2-Oxo-2-[4-(4-pyrimidin-2-yl-phenyl)-piperazin-1-yl]-ethyl}-pyrrolidine-3-carboxylic acid [3-(1,3-dioxo-1,3-dihydro-isoindol-2-ylmethyl)-1H-indazol-5-yl]-amide

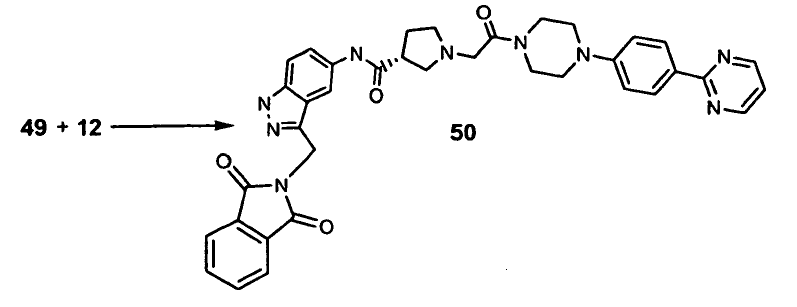

2-(5-Amino-1H-indazol-3-ylmethylyisoindole-1,3-dione (compound 49 from Step 3, 614 gm, 2 mmol) and compound 12 (from Example 1 Step 9) were dissolved in 10 ml of DMF. O-(7-azabenzotriazol-1-yl-)-N,N,N,N-tetramethyluronium hexafluorophosphate (HATU) (660 mg, 1.7 mmol) and diisopropylethylamine ( 0.9 ml, 5 eq.). After stirring 4 hours, the reaction mixture was added to brine and extracted with ethylacetate, dried over magnesium sulfate and evaporated. The crude was chromatographed on silica gel to obtain 440mg of title product.

### EXAMPLE 77

### Preparation of 1-{2-Oxo-2-[4-(4-pyrimidin-2-yl-phenyl)-piperazin-1-yl]-ethyl}-pyrrolidine-3-carboxylic acid (3-aminomethyl-1H-indazol-5-yl)-amide

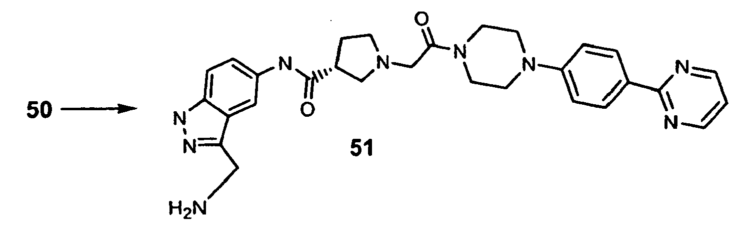

1-{2-Oxo-2-[4-(4-pyrimidin-2-yl-phenyl)-piperazin-1-yl]-ethyl}-pyrrolidine-3-carboxylic acid [3-(1,3-dioxo-1,3-dihydro-isoindol-2-ylmethyl)-1H-indazol-5-yl]-amide (compound 50 from Example 78, 424 mg, 0.633 mmol) was dissolved in ethanol and 0.31 ml of hydrazine hydrate. The mixture was heated at 80 C for 20 hrs and then evaporated to dryness. The mixture was chromatographed on silica gel to obtain 100 mg of title product.

### EXAMPLES 78 TO 84

Following a procedure similar to that of Examples 66 to 73 compound 51 (from Example 77) was used to prepare compounds of formula 52 wherein X² is defined in Table 3. To obtain the desired final compound in Examples 78, 80 and 81, Compound 51 is reacted with X²'s corresponding commercially available acid chloride. To obtain the desired final compound in Example 79, 83 and 84 Compound 51 is reacted with X²'s corresponding commercially available isocyanate. To obtain the desired final compound in Example 82, Compound 51 is reacted with X²'s corresponding to commercially available sulfonyl chloride. In Table 3 "Ex" represents "Example".

**Table 3**

| | |
|---|---|
| 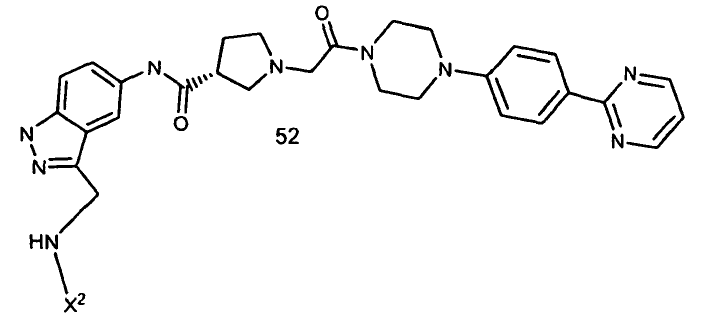 | |

| **Ex** | **X²** |
|---|---|
| 78 | -C(O)CH₃ |
| 79 | -C(O)NH₂ |
| 80 | 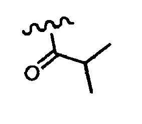 |
| 81 | 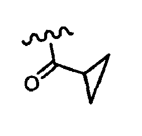 |
| 82 | 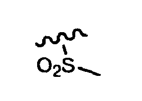 |
| 83 | 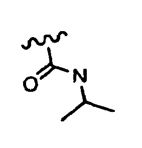 |
| 84 | 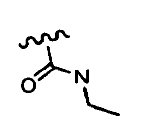 |

### EXAMPLE 85 (PREPARATIVE EXAMPLE)

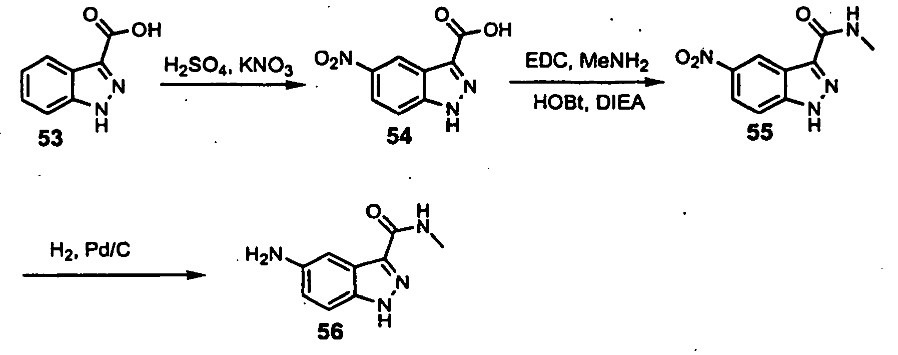

### Step 1

### Preparation of 5-Nitro-1H-indazole-3-carboxylic acid (54)

To a suspension of indazole-3-carboxylic acid (compound 53, 3.0 g, 18 mmol) in 18 mL of concentrated sulfuric acid at 0 C was added potassium nitrate (2.0 g, 18 mmol). The reaction was stirred overnight at room temperature, poured into 150 mL of ice and extracted three times with ethyl acetate. The combined organic layer was washed with brine, dried and concentrated to give compound 54 (2.9 g) as the major isomer.

### Step 2

### Preparation of 5-Nitro-1H-indazole-3-carboxylic acid methylamide (55)

To a solution of compound 54 (100 mg, 0.483 mmol), methylamine hydrochloride (52.2 mg, 0.773 mmol), HOBt (130 mg, 0.966 mmol) and DIEA (0.34 mL, 1.95 mmol) in N-methylpyrrolidinone was added 1-(3-dimethylaminopropyl)-3-ethylcarbodiimide hydrochloride (148 mg, 0.773 mmol). The reaction was stirred at room temperature for 2 hrs and diluted with 10 mL of ethyl acetate. The mixture was washed with water and a yellow solid precipitated. The precipitate was collected by filtration to give compound 55 (67 mg).

### Step 3

### Preparation of 5-Amino-1H-indazole-3-carboxylic acid methylamide (56)

To a suspension of compound 55 (65 mg) in 5 mL of methanol was added catalytic amount of 5% palladium on carbon. The mixture was stirred under a hydrogen atmosphere at room temperature for 4 hrs and filtered through celite. The filtrate was concentrated to afford compound 56 (59 mg).

### EXAMPLE 86

### Step 1

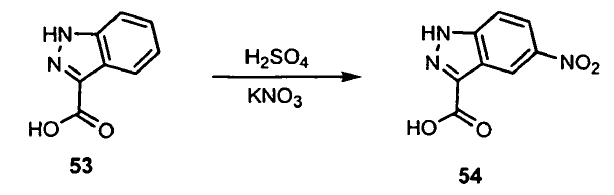

To a suspension of indazole-3-carboxylic acid (3.0 g, 18 mmol) in 18 mL of concentrated sulfuric acid at 0 C was added potassium nitrate (2.0 g, 18 mmol). The reaction was stirred overnight at room temperature, poured into 150 mL of ice and extracted three times with ethyl acetate (90 mL total). The combined organic layer was washed with brine, dried and concentrated to give (54) (2.9 g) as the major product.

### Step 2

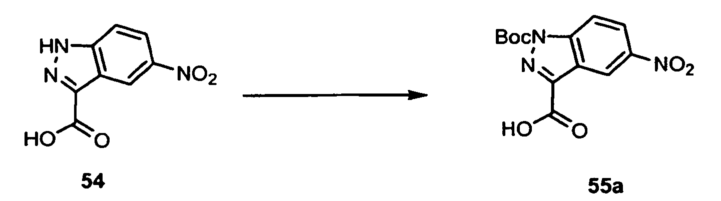

To a solution of compound 54 (230 mg, 1.11 mmol) in 5mL of THF was added sodium hydroxide solution (1 M, 3.3 mL, 3.33 mmol), and then t-butyl dicarbonate (364 mg, 1.67 mmol). The reaction was stirred at room temperature overnight and treated with 3.4 mL of 1 N HCl. The mixture was extracted with ethyl acetate three times. The combined organic layer was dried over sodium sulfate and concentrated to provide compound 55a (307 mg).

### Step 3

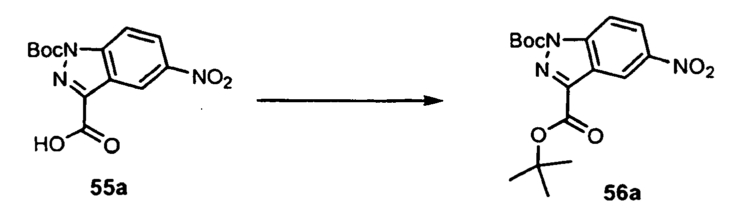

To a solution of compound 55a (307 mg, 1.00 mmol) was added *N,N'-*diisopropyl-*t*-butyl isourea (880 mg, 4.00 mmol). The reaction was heated to reflux overnight and filtered. The filtrate was concentrated and purified by column chromatography (3:1 hexane/ethyl acetate) to provide compound 56a (179 mg).

### Step 4

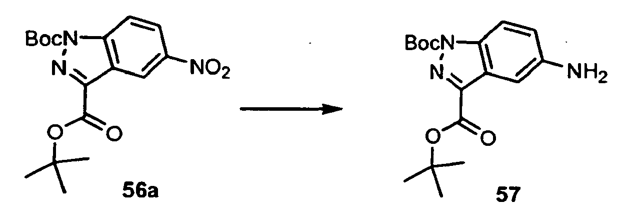

To a mixture of compound 56a (150 mg, 0.413 mmol) in 5 mL of methanol was added catalytic amount of 5 % palladium on carbon. The mixture was stirred under a hydrogen atmosphere at room temperature overnight and filtered through celite. The filtrate was concentrated to provide compound 57 (98 mg). The material was used as such without further purification.

### Step 5

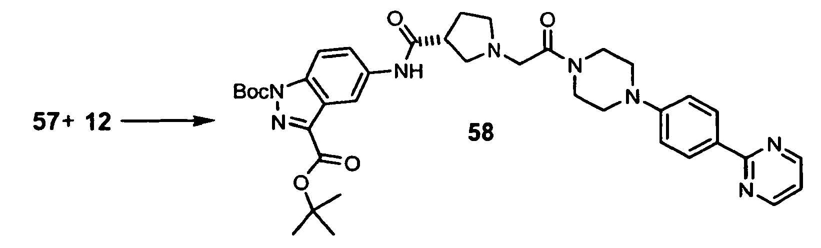

To a solution of compound 57, from Step 4, (98 mg, 0.29 mmol), 12 (TFA salt with LiCl, 211 mg, 0.29 mmol) 1-hydroxybenztriazole (40 mg, 0.29 mmol) and diisopropylamine (0.15 mL, 0.90 mmol) in DMF was added 1-(3-dimethylaminopropyl)-3-ethylcarbodiimide hydrochloride (EDCI) (73 mg, 0.38 mmol). The reaction was stirred overnight at room temperature for 2 hrs, concentrated and purified by reverse phase chromatography to give 58 as a TFA salt (180 mg).

### Step 6

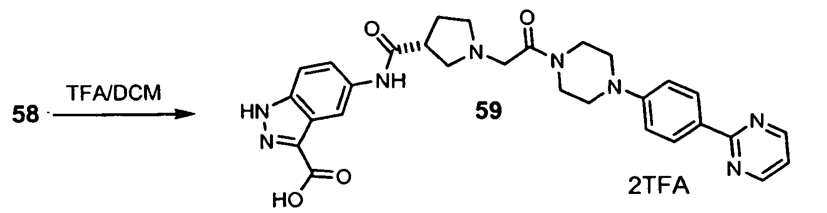

Compound 58, from Step 5, (180 mg, 0.192 mmol) was treated with TFA/DCM (3 mL/3mL) at room temperature. The reaction was stirred for 1 h and concentrated to provide 59 (115 mg).

### Step 7

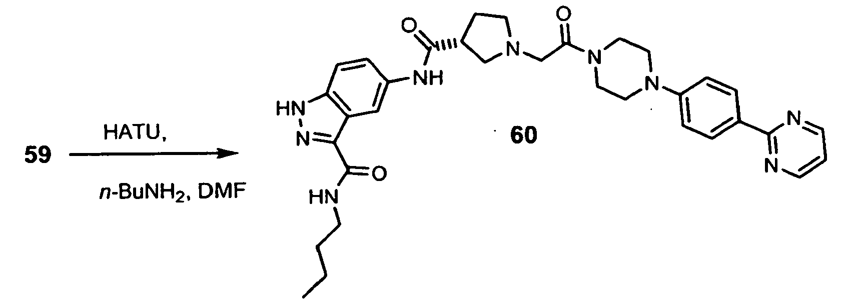

To a solution of compound 59 (10 mg, 0.013 mmol) and n-butylamine (1.9 mg, 0.026mmol) in 1.5 mL of DMF was added HATU (14 mg). The reaction was stirred at room temperature for 2 hrs and concentrated. The residue was purified by reverse-phase chromatography to give 60 (5 mg).

### EXAMPLE 87

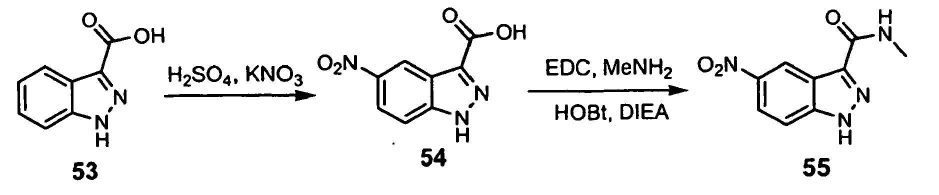

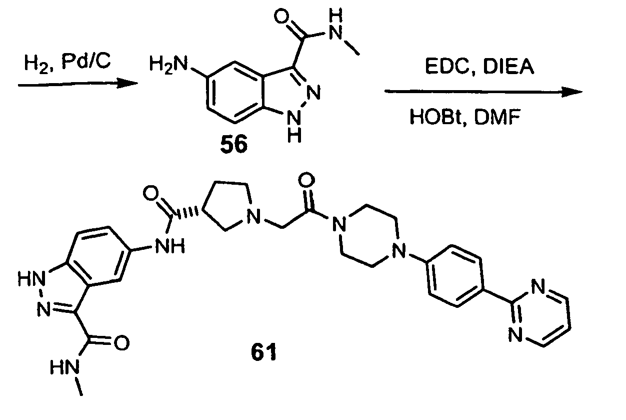

### Step 1

To a suspension of indazole-3-carboxylic acid (compound 53, 3.0 g, 18 mmol) in 18 mL of concentrated sulfuric acid at 0 C was added potassium nitrate (2.0 g, 18 mmol). The reaction was stirred overnight at room temperature, poured into 150 mL of ice and extracted three times with ethyl acetate. The combined organic layer was washed with brine, dried and concentrated to give compound 54 (2.9 g) as the major isomer.

### Step 2

To a solution of compound 54 (100 mg, 0.483 mmol), methylamine hydrochloride (52.2 mg, 0.773 mmol), HOBt (130 mg, 0.966 mmol) and DIEA (0.34 mL, 1.95 mmol) in N-methylpyrrolidinone was added 1-(3-dimethylaminopropyl)-3-ethylcarbodiimide hydrochloride (148 mg, 0.773 mmol). The reaction was stirred at room temperature for 2 hrs and diluted with 10 mL of ethyl acetate. The mixture was washed with water and a yellow solid precipitated. The precipitate was collected by filtration to give compound 55 (67 mg).

### Step 3

To a suspension of compound 55 (65 mg) in 5 mL of methanol was added catalytic amount of 5% palladium on carbon. The mixture was stirred under a hydrogen atmosphere at room temperature for 4 hrs and filtered through celite. The filtrate was concentrated to afford compound 56 (59 mg).

### Step 4

Compound 56 was reacted with compound 12 (Example 1 Step 9) following the procedure of Example 1, Step 10, to prepare compound 61 Mass Spec.: 610, LCMS Retention Time 3.58 minutes.

### EXAMPLES 88 TO 97

### Compounds of formula 62:

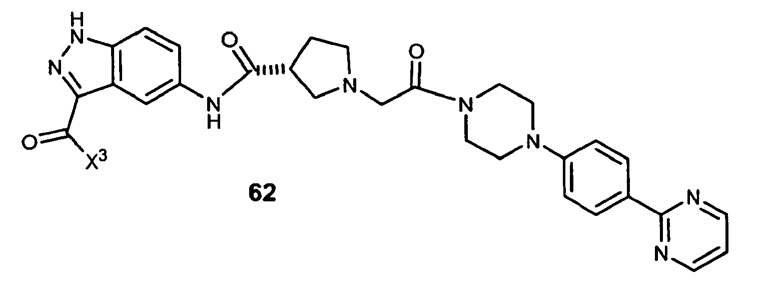

wherein X³ is defined in Table 4 were prepared from compound 59 (from Example 86 Step 6) following a procedure similar to that described in Example 86 Step 7 and using the corresponding amine of X³. In Table 4 "Ex" represents "Example".

**Table 4**

| **Ex** | **X³** | **Retention time (min)** | **Mass Spec** |
|---|---|---|---|
| 88 | 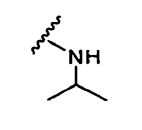 | 3.33 | 596 |
| 89 | 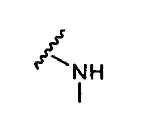 | 2.84 | 568 |
| 90 | 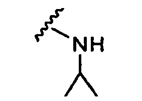 | 3.11 | 594 |
| 91 | 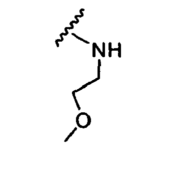 | 2.42 | 612 |
| 92 | 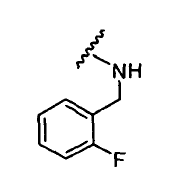 | 3.71 | 662 |
| 93 | 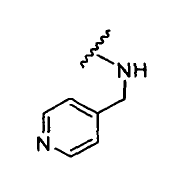 | 2.44 | 645 |
| 94 | 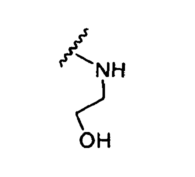 | 2.63 | 598 |
| 95 | 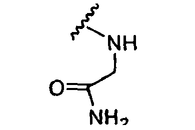 | 2.54 | 611 |
| 96 | 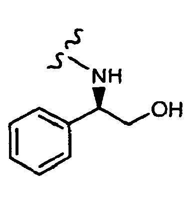 | 3.28 | 674 |
| 97 | 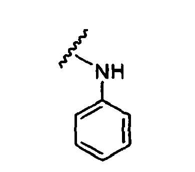 | 3.72 | 630 |

### EXAMPLE 98

### Step 1

### Preparation of 1-tert-Butoxycarbonylmethyl-pyrrolidine-3-carboxylic acid,Lithium salt.

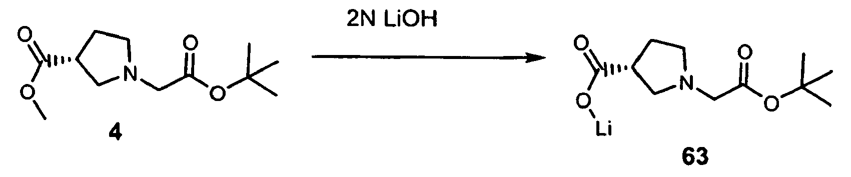

1-tert-Butoxycarbonylmethyl-pyrrolidine-3-carboxylic acid methyl ester 4 (see Example 1 Step 2) (0.753g, 3.098mmol) was dissolved in MeOH/THF (10ml;1/1) and 2N Lithium hydroxide ( 1.5ml; 3mmol) was added. The resultant solution was stirred for 2 hours, and solvent was evaporated yielding title compound **63** as a white solid (0.71g, 100%). Mass Spec ES (230,MH).

### Step 2

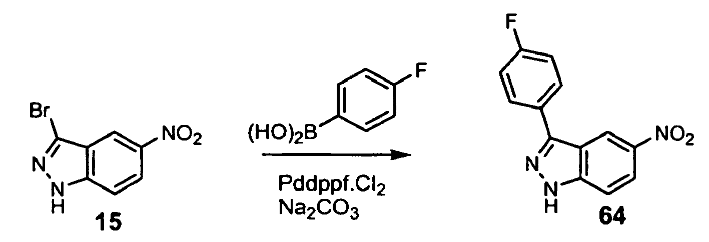

3-Bromo-5-nitro-1H-indazole (Compound 15 (see Example 2 Step 2), 0.5g, 2.06mmol), 4-Flourophenylboronic acid (720mg, 5.14mmol), Pd(dppf)Cl₂ (252mg, 0.31 mmol), and Na₂CO₃ (657mg, 6.20mmol) were added to a 25ml microwave vessel. DME (16ml) and H₂O (4ml) were added subsequently. The mixture was heated under microwave at 150°C for 20 min. The reaction mixture was then filtered through a pad of celite. The filtrate was concentrated, and purified by flash column (25% EtOAc/Hex) to yield Compound 64 (0.3g, 1.17mmol).

### Step 3

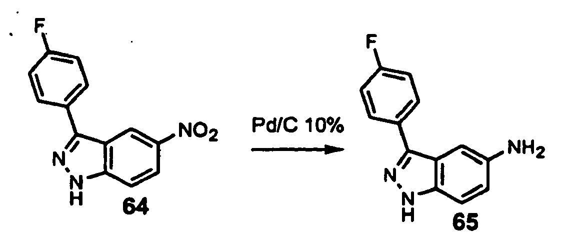

Compound 64 (0.57g, 2.22mmol) was dissolved in EtOAc (20ml) / MeOH (20ml). Pd/C (10 wt. %, cat.) was added. The mixture was subjected to 50PSI H₂ on a par apparatus at room temperature over night. The reaction was then filtered. The filtrate was concentrated to yield Compound 65 (492mg, 2.16mmol).

### Step 4

### Preparation of {3-[3-(4-Fluoro-phenyl)-1H-indazot-5-ylcarbamoyl]-pyrrolidin-1-yl}-acetic acid tert-butyl ester

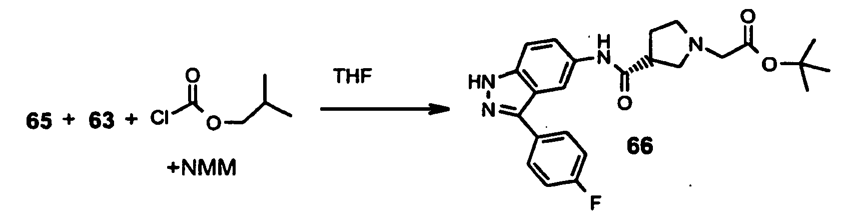

1-tert-Butoxycarbonylmethyl-pyrrolidine-3-carboxylic acid,Lithium salt 63 (from Step 1) (665mg,2.83mmol) and N-Methyl morpholine (0.84ml,7.65mmol) were dissolved in THF (50ml) then cooled solution to 0 C. Isobutyl chloroformate (0.37ml, 2.83mmol) in THF (20ml) was added dropwise, and stirred at 0°C for 1 hour. 3-(4-Fluoro-phenyl)-1H-indazol-5-ylamine 65 (from Step 3) (576mg,2.54mmol) in THF (20ml) was added dropwise then stirred at room temperature for 1 hour. The reaction mixture was diluted with EtOAC (150ml), Water (60ml)and 5% NaOH (10ml). Organic layer was separated, dried over MgSO₄, and solvent evaporated yielding a residue which was dissolved and stirred in Methanol(30ml) and 1N NaOH (10ml) for 10 minutes. Reaction was concentrated,residue extracted with EtOAc (200ml) washed with H₂O (30ml),dried over Na₂SO₄, filtered and concentrated yielding title compound 66 as a white solid (700mg, 63%).

### Step 5

### Preparation of {3-[3-(4-Fluoro-phenyl)-1H-indazol-5-ylcarbamoyl]-pyrrolidin-1-yl}-acetic acid trifluoroacetate.

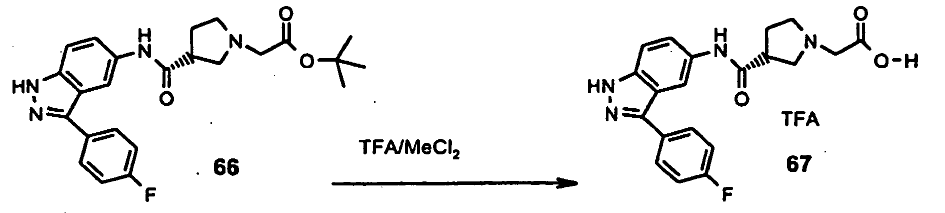

Compound 66 (700mg,1.60mmol) was stirred in trifluoroacetic acid (20%,10ml) for 2 hours,then the solvent was evaporated yielding the title compound as trifluoroacetate salt 67 as a white solid. (790mg,100%) ESMS (MH, 383).

### Step 6

### Preparation of 2-(6-Bromo-pyridin-3-yl)-pyrimidine

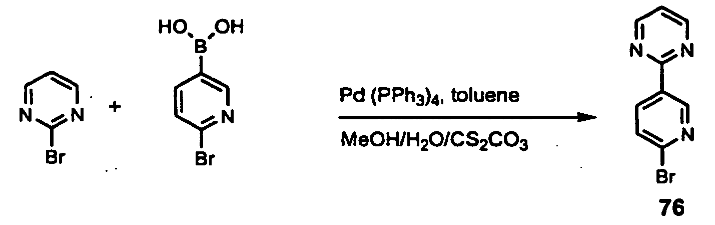

A mixture of 2-bromopyrimidine (0.43g,2.70mmol), 2-bromopyridine-5-boronic acid (0.55g,2.72mmol), tetrakis(triphenylphosphine)palladium(O) (300mg, 0.259mmol), cesium carbonate (1.15g, 3.03mmol) was stirred in MeOH/toluene/water (15ml, 1/1/1) at reflux temperature overnight. The reaction was cooled to room temperature and diluted with EtOAc (200ml) and water (50ml). The organic layer was separated, dried over MgSO₄, filtered and solvent evaporated yielding a residue which was purified on silica gel eluting with 25% v/vEtOAc/hexanes yielding product 76 as white solid. (0.55g, 85%) ESMS (MH, 236).

### Step 7

### Preparation of 2-(6-Piperazin-1-yl-pyridin-3-yl)-pyrimidine

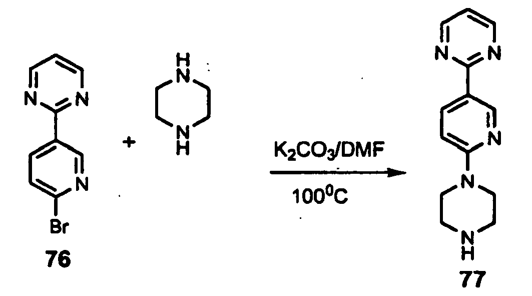

A mixture of 2-(6-Bromo-pyridin-3-yl)-pyrimidine 76 (100mg, 0.425mmol), potassium carbonate (100mg,0.724mmol), and piperazine (100mg, 1.16mmol) in DMF (5ml) were stirred at 100°C for 1 hour. The reaction was cooled,solvent evaporated under reduced pressure, and the residue dissolved in MeCl₂ (150ml), washed with H₂O (50ml),dried over MgSO₄, filtered and evaporated solvent yielding title product 77 as a white solid (100mg,98%). ESMS (MH, 242).

### Step 8

### Preparation of 1-[2-Oxo-2-[4-(5-pyrimidin-2-yl-pyridin-2-yl)-piperazln-1-yl]-ethyl}-pyrrolidine-3-carboxylic acid 13-(4-fluoro-phenyl)-1H-indazol-5-yl]-amide

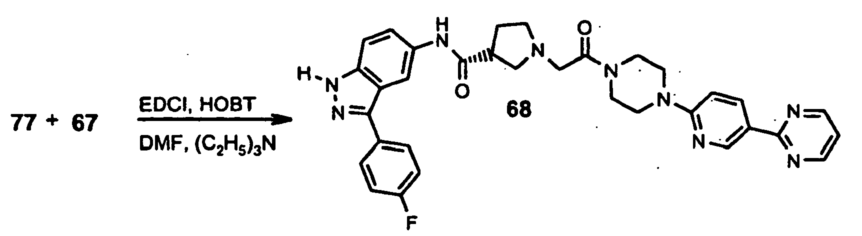

Added triethylamine (0.1 ml, 0.7mmol) to solution of {3-[3-(4-Fluoro-phenyl)-1H-indazol-5-ylcarbamoyl]-pyrrolidin-1-yl}-acetic acid trifluoroacetate 67 (Step 5) (50mg, 0.1008mmol), 2-(6-Piperazin-1-yl-pyridin-3-yl)-pyrimidine 77 (Step 7) (60mg, 0.248mmol), 1-(3-dimethylaminopropyl)-3-ethylcarbodiimide hydrochloride (EDCI) (50mg,0.261 mmol), 1-hydroxybenztriazole monohydrate (30mg,0.222mmol) in DMF (2ml), then stirred overnight at room temperature. The solvent was evaporated and residue purified on silica gel eluting with 7% v/v MeOH/MeCl₂/NH₄OH yielding product 68 as white solid (33mg, 54%). LCMS (MH, 606) Retention time = 2.46 minutes.

### EXAMPLES 99 TO 101.

Following a procedure similar to that of Example 98, and using the appropriate reagents, the compounds in Table 5 were obtained. In Table 5 "Ex" represents "Example".

**Table 5**

| **Ex** | **Compound** | **Mass Spec LCMS MH** | **Retention time Minutes** |
|---|---|---|---|
| 99 | 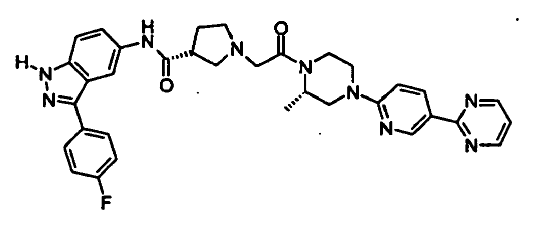 | 620 | 2.45 |
| 100 | 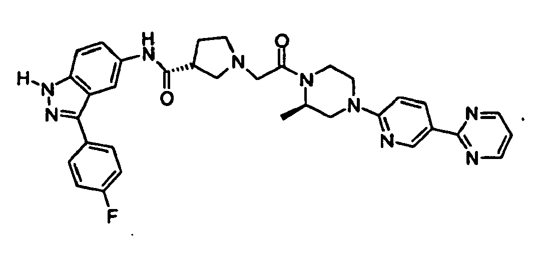 | 620 | 2.43 |
| 101 | 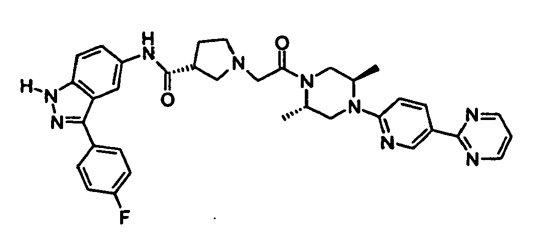 | 647 | 3.23 |

### EXAMPLE 102

### Step 1

### Preparation of 1-Chloro-2-iodo-4-nitro-benzene

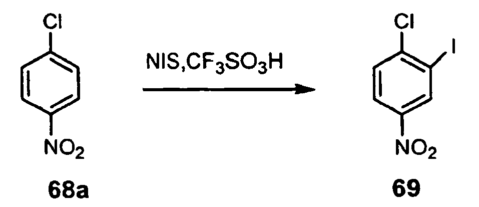

N-Iodosuccinimide (1.71g, 7.60mmol) was added to a solution of 1-chloro-4-nitrobenzene (1.17g, 7.42mmol) in trifluoromethanesulfonic acid (10ml) at 0°C, then stirred 1 hour at room temperature. The reaction was quenched with ice-water and extracted with MeCl₂ (3x50ml). Organics were combined ,washed with 10% sodium bisulfite (20ml) then dried over Na₂SO₄, filtered and evaporated solvent yielding the title compound as a white solid (1.3g, 62%).

### Step 2

### Preparation of 1-Chloro-2-(2-fluoro-phenylethynyl)-4-nitro-benzene

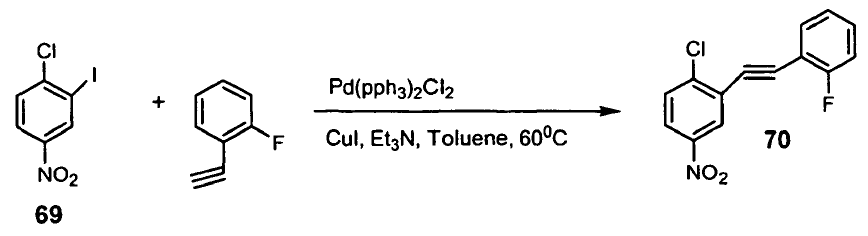

Triethylamine (0.2ml,1.43mmol) was added to a suspension of 1-Chloro-2-iodo-4-nitro-benzene 69 (Step 1) (100mg, 0.354mmol), 1-ethynyl-2-fluorobenzene (100mg, 0.832mmol), copper iodide (100mg, 0.525mmol),Pd(PPh₃)₂Cl₂ (100mg, 0.142mmol) in dimethylformamide (2ml) at room temperature, then stirred at 70°C for 2 hours. Reaction was cooled to room temperature and extracted with ether (50ml), washed with water(20ml), dried (MgSO₄), filtered and solvent evaporated yielding a residue which chromatographed on silica gel eluting with 20% v/v MeCl₂/hexanes yielding title product 70 as pale yellow solid (80mg,82%).

### Step 3

### Preparation of 3-(2-Fluoro-benzyl)-5-nitro-1H-indazole

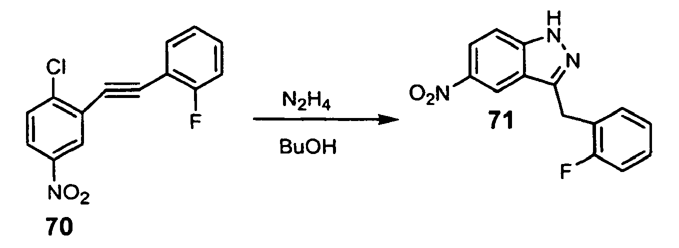

Hydrazine.monohydrate (0.2ml, 4.1 mmol) was added to a solution of 1-chloro-2-(2-fluoro-phenylethynyl)-4-nitro-benzene 70 (Step 2) (80mg, 0.29mmol) in n-butanol (3ml) then refluxed for 2 hours. The mixture was cooled and solvent evaporated under reduced pressure. The residue was purified on silica gel eluting with 5% v/v MeOH/MeCl₂ yielding product as yellow solid (60mg, 76%) ESMS (MH, 272).

### Step 4

### Preparation of 3-(2-Fluoro-benzyl)-1H-indazol-5-ylamine

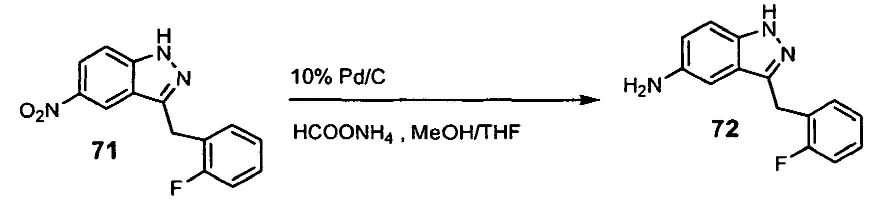

Ammonium formate (50mg, 0.792mmol) and 10% Pd/C (5mg) were added to a solution of 3-(2-fluoro-benzyl)-5-nitro-1H-indazole (50mg,0.184mmol) in MeOH/THF (3ml, 1/1) then refluxed for 3 hours. The reaction was diluted with MeOH (20ml) and filtered through a celite pad. The solvent was evaporated, residue dissolved in MeCl₂, dried over Na₂SO₄, filtered and solvent evaporated yielding title compound as white solid (40mg, 88%) ESMS (MH, 242).

### Step 5

### Preparation of 1-{2-Oxo-2-[4-(4-pyrimidin-2-yl-phenyl)-piperazin-1-yl]-ethyl}-pyrrolidine-3-carboxylic acid [3-(2-fluoro-benzyl)-1H-indazol-5-yl]-amide

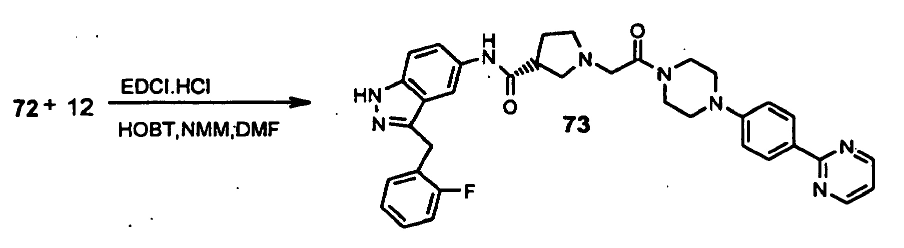

Added N-methylmorpholine (0.2ml) to solution of 3-(2-Fluoro-benzyl)-1H-indazol-5-ylamine 72 (Step 4) (5mg, 0.0207mmol), 1-{2-Oxo-2-[4-(4-pyrimidin-2-yl-phenyl)-piperazin-1-yl]-ethyl}-pyrrolidine-3-carboxylic acid 12 (see Example 1 Step 9) (8mg, 0.0202mmol), 1-(3-dimethylaminopropyl)-3-ethylcarbodiimide hydrochloride (EDCI) (5mg, 0.026mmol), 1-hydroxybenztriazole monohydrate (3mg, 0.022mmol) in DMF (2ml), then stirred overnight at room temperature. The solvent was evaporated and residue purified on silica gel eluting with 7% v/v MeOH/MeCl₂/NH₄OH yielding product as white solid (5mg,39%) ESMS (MH, 619).

### EXAMPLES 103 TO 112

Following a procedure similar to that of Example 102, and using the appropriate reagents, compounds of formula 74: 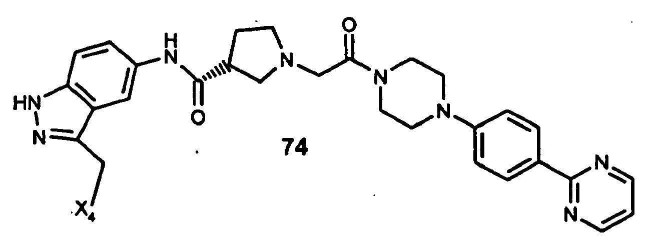 wherein X⁴ is defined in Table 6 were prepared. The arrow indicates the point of attachment of the X⁴ substitutent to the rest of the molecule. No arrow is shown for Example 109 because attachment to any of the carbons of the X⁴ phenyl group results in the same final compound of formula 74. In Table 6 "Ex" represents "Example".

**Table 6**

| **Ex** | **X⁴** | **Mass Spec (ESMS, MH)** | **Retention time minutes** |
|---|---|---|---|
| 103 | 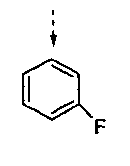 | 619 | 3.10 |
| 104 | 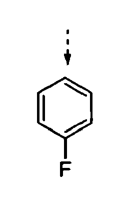 | 619 | 2.79 |
| 105 | 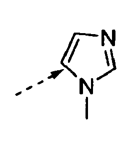 | 606 | 2.31 |
| 106 | 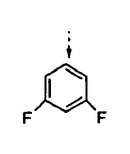 | 637 | 3.43 |
| 107 | 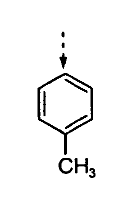 | 615 | 3.13 |
| 108 | 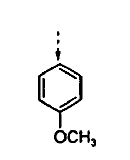 | 631 | 3.17 |
| 109 | 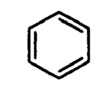 | 601 | 2.70 |
| 110 | 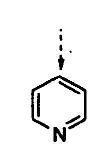 | 602 | 2.36 |
| 111 | 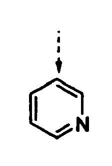 | 602 | 2.41 |
| 112 | 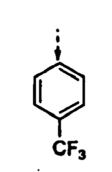 | 669 | 3.24 |

### EXAMPLES 113 to 118

Following a procedure similar to that of Example 102 and using the appropriate reagents, and using 1-{2-[4-(3-Chloro-phenyl)-piperazin-1-yl]-2-oxo-ethyl}-pyrrolidine-3-carboxylic acid 75: 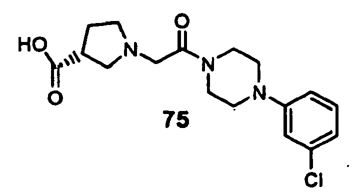 in place of 12, the compounds of formula 76: 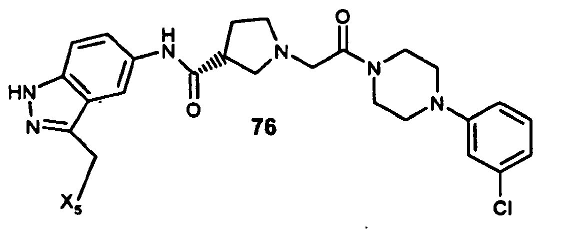 wherein X⁵ is defined in Table 7 were prepared. The arrow indicates the point of attachment of the X⁵ substitutent to the rest of the molecule. In Table 7 "Ex" represents "Example".

**Table 7**

| **Ex** | **X⁵** | **Mass Spec ESMS (MH)** | **Retention time (minutes)** |
|---|---|---|---|
| 113 | 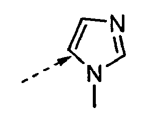 | 561 | 2.63 |
| 114 | 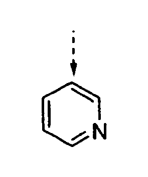 | 558 | 2.67 |
| 115 | 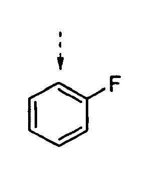 | 575 | 3.40 |
| 116 | 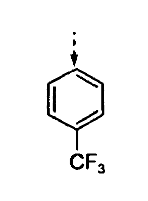 | 625 | 3.59 |
| 117 | 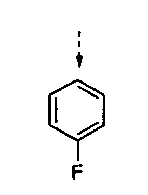 | 575 | 3.30 |
| 118 | 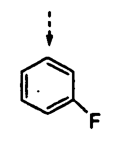 | 575 | 3.52 |

### EXAMPLE 119

### Step 1

### Preparation of 5-Methyl-2-[4-(3-(S)-methyl-piperazin-1-yl)-phenyl]-pyrimidine

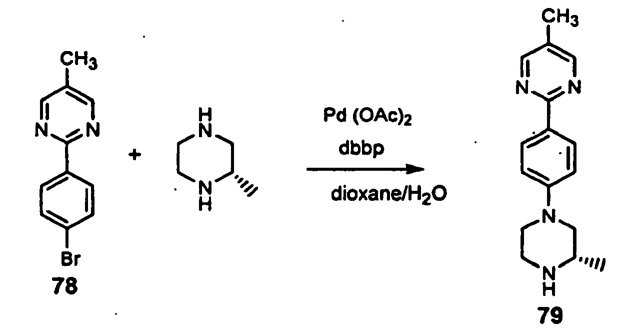

A mixture of 2-(4-bromophenyl)-5-methylpyrimidine 78 (250mg, 1.008mmol), palladium acetate (50mg), cesium carbonate (400mg,1.23mmol), (S)-2-methyl piperazine(200mg, 2mmol) and 2-Di-t-butylphosphino)-biphenyl (50mg, 0.167mmol) was stirred in dioxane:water (10m).v/v 5:1) at reflux temperature for 4 hours. The reaction was cooled,diluted with MeCl₂ (100ml) and H₂O (50ml). The organic layer was separated, dried (MgSO₄), filtered and solvent evaporated. The residue was purified by chromatography eluting with 100% EtOAc then with 10% v/v MeOH/EtOAc/NH₄OH yielding product 79 as a white solid. (220mg.81%) ESMS (MH, 269).

### Step 2

### Preparation of 1-(2-{2-Methyl-4-[4-(5-methyl-pyrimidin-2-yl)-phenyl]-piperazin-1-yl}-2-oxo-ethyl)-pyrrolidine-3-carboxylic acid [3-(4-fluoro-phenyl)-1H-indazol-5-yl]-amide

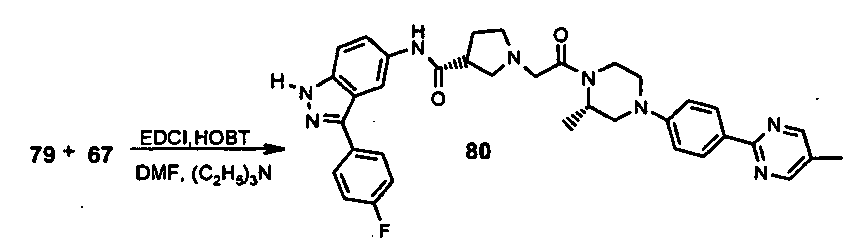

Following a procedure similar to that of Example 98, Step 8, but substituting 79 for 77, the title compound 80 was obtained as a white solid (ESMS, MH 633), Retention Time: 3.07 minutes.

### EXAMPLES 120 TO 123

Following a procedure similar to that of Example 119, and using the appropriate reagent in place of 79 compounds of Examples 120, 121, and 123 in Table 8 were prepared, and the compound of Example 122 could be prepared. In Table 8 "Ex" represents "Example".

**Table 8**

| **Ex** | **Compound** | **Mass spec LCMS MH** | **Retention time (minutes)** |
|---|---|---|---|
| 120 | 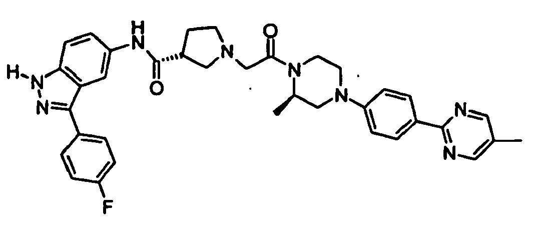 | 633 | 3.45 |
| 121 | 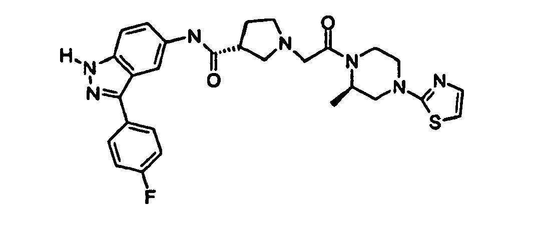 | 548 | 2.36 |
| 122 | 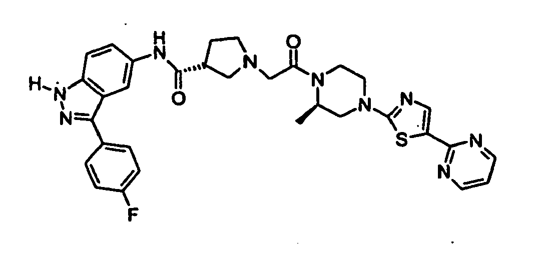 | ------ | ------ |
| 123 | 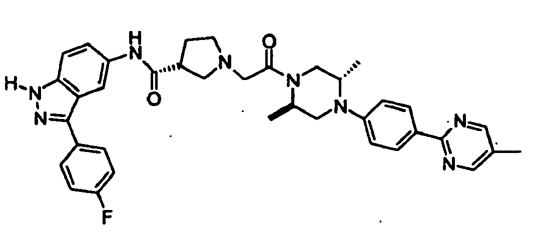 | 647 | 3.23 |

### EXAMPLE 124

### Step 1

### Preparation of 5-(4-Bromo-phenyl)-pyrimidin-2-ylamine

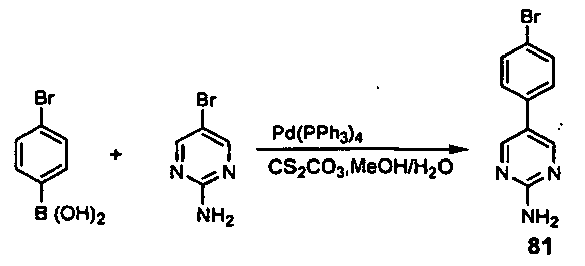

A mixture of 5-bromo-pyrimidin-2-ylamine (0.8g, 4.59mmol), 4-bromophenyl boronic acid(1g, 4.97mmol), tetrakis(triphenylphosphine)palladium(0) (300mg, 0.259mmol), cesium carbonate (1.15g, 3.03mmol) was stirred in MeOH/H₂O (20ml, 1/1) at reflux temperature overnight. The reaction was cooled to room temperature and diluted with EtOAc (200ml) and water (50ml). The organic layer was separated, dried over MgSO₄, filtered and solvent evaporated yielding a residue which was purified on silica gel eluting with 85% v/vEtOAc/hexanes yielding product 81 as white solid. (0.7g, 63%). ESMS (MH, 250).

### Step 2

### Preparation of 5-(4-Piperazin-1-yl-phenyl)-pyrimidin-2-ylamine

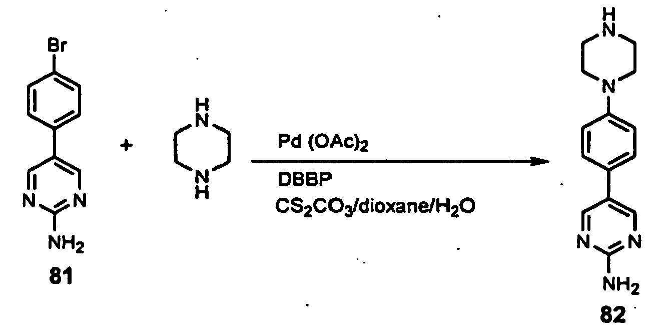

A mixture of 5-(4-bromo-phenyl)-pyrimidin-2-ylamine (100mg, 0.401mmol),palladium acetate (20mg, 0.089mmol), cesium carbonate (200mg, 0.62mmol), piperazine(100mg, 1.16mmol) and 2-di-t-butylphosphino)-biphenyl (50mg, 0.167mmol) was stirred in dioxane:water (10ml, v/v 5:1) at reflux temperature for 4 hours. The reaction was cooled, diluted with MeCl₂ (100ml) and H₂O (50ml). The organic layer was separated, dried (MgSO₄), filtered and solvent evaporated. The residue was purified by chromatography eluting with 100% EtOAc then with 10% v/v MeOH/EtOAc/NH₄OH yielding product 82 as a white solid. (70mg.68%) ESMS (MH, 256).

### Step 3

### Preparation of 1-(2-4-[4-(2-Amino-pyrimidin-5-yl)-phenyl]-piperazin-1-yl}-2-oxo-ethyl)-pyrrolidine-3-carboxylic acid [3-(4-fluoro-phenyl)-1H-indazol-5-yl]-amide

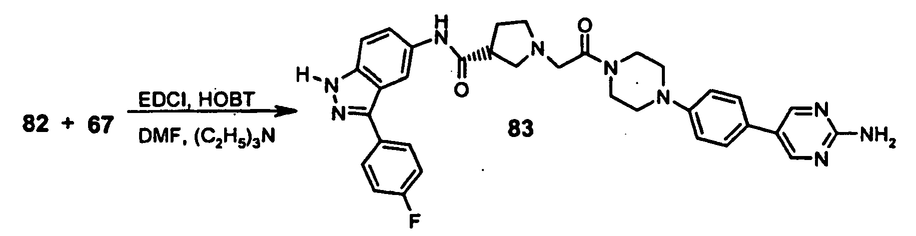

Following a procedure similar to that described in Example 98 Step 8, but substituting 82 for 77, the title product 83 was obtained as a white solid (ESMS, MH 620) C₃₄H₃₅NgFO₂ LCMS (MH 620) Retention time = 2.52 minutes.

### EXAMPLE 125

### Step 1

### Preparation of 2-(tert-butyl-dimethyl-silanyloxymethyl)-acrylic acid ethyl ester

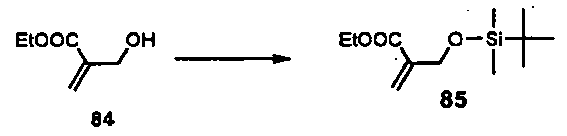

To a stirred solution of 2-hydroxymethyl-acrylic acid ethyl ester (260 mg, 2 mmol) and imidazole (163 mg, 2.4 mmol) in dry DMF (5 ml) was added *tert*butyldimethylsilyl chloride (362 mg, 2.4 mmol). The reaction mixture was stirred overnight and diluted with ether, washed with water three times and dried over MgSO₄. Solvent was removed under reduced pressure to provide a crude product that was purified by column chromatography using a solution of ethyl acetate in hexanes (1:6) to obtain the title product (463 mg, 95%).

### Step 2

### Preparation of 1-benzyl-3-(tert-butyl-dimethyl-silanyloxymethyl)-pyrrolldine-3-carboxylic acid ethyl ester

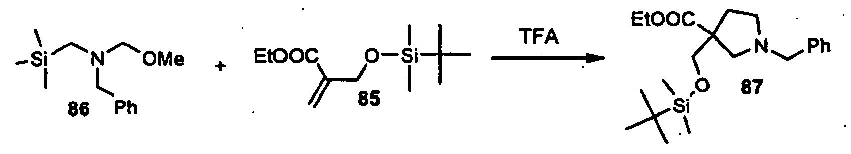

To a cold solution of 2-(*tert-*butyl-dimethyl-silanyloxymethyl)-acrylic acid ethyl ester (463 mg, 1.89 mmol) and *N*-(methoxymethyl)-*N*-(trimethylsilylmethyl)benzylamine (540 µl, 2.11 mmol) in dichloromethane (3 ml) was added at 0 °C trifluoroacetic acid (26 µl, 0.34 mmol). The resulting solution was warmed to room temperature in two hours. The crude product was purified by column chromatography on silica get eluting with a solution of ethyl acetate in hexanes (1:10, 1:5) to give the title compound (490 mg, 69%).

### Step 3

### Preparation of 3-(tert-butyl-dimethyl-silanyloxymethyl)-pyrrolidine-3-carboxylic acid ethyl ester

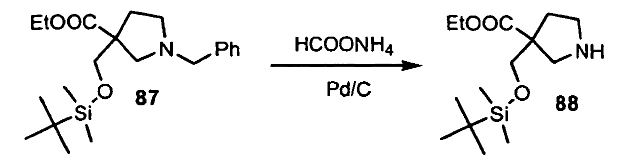

A mixture of 1-benzyl-3-(*tert*-butyl-dimethyl-silanyloxymethyl)-pyrrolidine-3-carboxylic acid ethyl ester (690 mg, 1.83 mmol), ammonium formate (461 mg, 7.31 mmol), 10% Pd/C (100 mg) in methanol (10 ml) and water (1 ml) was refluxed overnight. The mixture was filtered through celite, washed with ethyl acetate. The combined filtrate was concentrated and the residue was taken into ethyl acetate, washed with brine and dried over MgSO₄. Evaporation of solvent provided the title compound as oil (444 mg, 84%).

### Step 4

### Synthesis of 1-tert-butoxycarbonylmethyl-3-(tert-butyl-dimethyl-silanyloxymethyl)-pyrrolidine-3-carboxylic acid ethyl ester

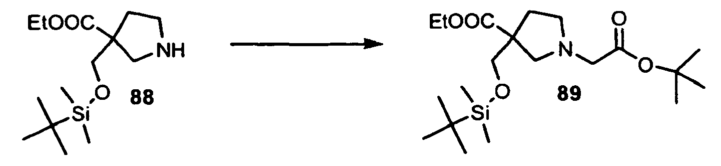

To a stirred mixture of 3-(*tert*-butyl-dimethyl-silanyloxymethylypyrrolidine-3-carboxylic acid ethyl ester (444 mg, 1.54 mmol), triethylamine (214 µl, 1.54 mmol) and cesium carbonate (251 mg, 0.77 mmol) in acetonitrile (5 ml) was added *tert-*butyl bromoacetate (351 µl, 2.38 mmol) slowly. The mixture was stirred for 1 hour, filtered and concentrated. The residue was diluted with ethyl acetate, washed with water and dried over MgSO4. Solvent was removed under reduced pressure to give a crude product that was purified by column chromatography on silica gel. Elution with a solution of ethyl acetate in hexanes (1:4) provided 580 mg (94%) of the title compound.

### Step 5

### Preparation of 1-tert-butoxycarbonylmethyl-3-hydroxymethyl-pyrrolidine-3-carboxylic acid ethyl ester

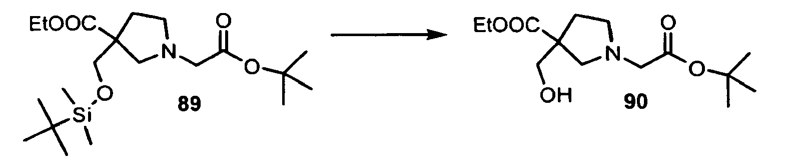

Tetrabutylammonium fluoride (1.8 ml, 1 M in THF) was added to 1-*tert-*butoxycarbonylmethyl-3-(*tert*-butyl-dimethyl-silanyloxymethyl)-pyrrolidine-3-carboxylic acid ethyl ester (726 mg, 1.8 mmol). The reaction solution was stirred for half hour and purified by column chromatography using solution of ethyl acetate in hexanes (1:1), then ethyl acetate to provide the title product (350 mg, 68%).

### Step 6

### Preparation of methyl 2-(methoxymethyl) acrylate

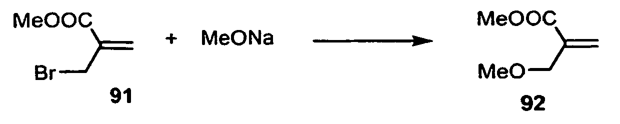

To a stirred mixture of methyl 2-(bromomethyl)acrylate (239 µl, 2 mmol) in petroleum ether (3 ml) was added potassium carbonate (276 mg, 2 mmol), followed by sodium methoxide (119 mg, 2.2 mmol) and methanol (450 µl). The resulting mixture was stirred overnight, filtered, concentrated to a residue that was purified by column chromatography eluting with 10% ether in hexanes to provide the title compound (150 mg, 58%). (Reference: J. Med.Chem.; 42; 15;1999; 2760-2773.)

### Step 7

### Preparation of 1-benzyl-3-methoxymethyl-pyrrolidine-3-carboxylic acid methyl ester

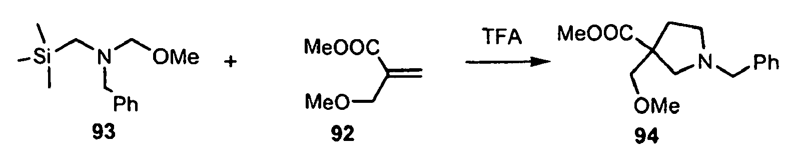

To a stirred solution of methyl 2-(methoxymethyl) acrylate (176 mg, 1.35 mmol) and *N*-(methoxymethyl)-*N*-(trimethylsilylmethyl)benzylamine (416 µl, 1.63 mmol) in dichloromethane (2 ml) was added at 0 °C trifluoroacetic acid (21 µl, 0.27 mmol). The resulting solution was warmed to room temperature and stirred overnight. The crude product was purified by column chromatography on silica, eluted with a solution of ethyl acetate in hexanes (1:3), then 5% methanol in ethyl acetate to give the title compound (293 mg, 82%).

### Step 8

### Preparation of 3-methoxymethyl-pyrrolidine-3-carboxylic acid methyl ester

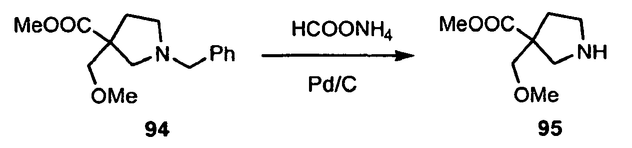

A mixture of 1-benzyl-3-methoxymethyl-pyrrolidine-3-carboxylic acid methyl ester (373 mg, 1.42 mmol), ammonium formate (358 mg, 5.68 mmol), 10% Pd/C (100 mg) and methanol (6 ml) was refluxed overnight. The mixture was filtered through Celite, washed with ethyl acetate. The combined filtrate was concentrated and the residue was taken into ethyl acetate, washed with small amount of water. Aqueous layer was isolated, extracted with dichloromethane three times. The dichloromethane extracts were combined with previous ethyl acetate extracts and dried over MgSO₄. Evaporation of solvents provided the title compound as oil (140 mg, 57%).

### Step 9

### Preparation of 1-tert-butoxycarbonylmethyl-3-methoxymethyl-pyrrolidine-3-carboxylic acid methyl ester

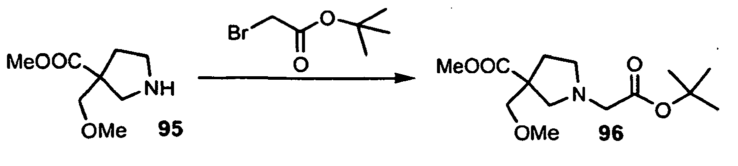

To a stirred mixture of 3-methoxymethyl-pyrrolidine-3-carboxylic acid methyl ester (140 mg, 0.81 mmol), triethylamine (112 µl, 0.82 mmol) and cesium carbonate (263 mg, 0.81 mmol) in acetonitrile (2 ml) was added *tert-*butyl bromoacetate (119 µl, 0.81 mmol) slowly. The mixture was stirred for 15 minutes, filtered and concentrated. The residue was purified by column chromatography on silica gel. Elution with a solution of ethyl acetate and hexanes (1:2) provided 118 mg (51%) of the title compound.

### Step 10

### Preparation of 1-carboxymethyl-3-methoxymethyl-pyrrolidine-3-carboxylic acid methyl ester

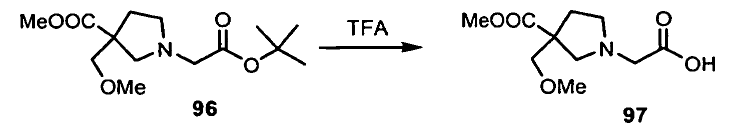

The 1-*tert-*butoxycarbonylmethyl-3-methoxymethyl-pyrrolidine-3-carboxylic acid methyl ester (118 mg) was treated with trifluoroacetic acid (2 ml), stirred for 20 minutes and evaporated to a residue that was exchanged with hydrochloric acid (1 ml, 4N) and lyophilized overnight to a gummy title product.

### Step 11

### Preparation of 3-methoxymethyl-1-{2-oxo-2-[4-(4-pyrimidin-2-yl-phenyl)-piperazin-1-yl]-ethyl}-pyrrolidine-3-carboxylic acid methyl ester

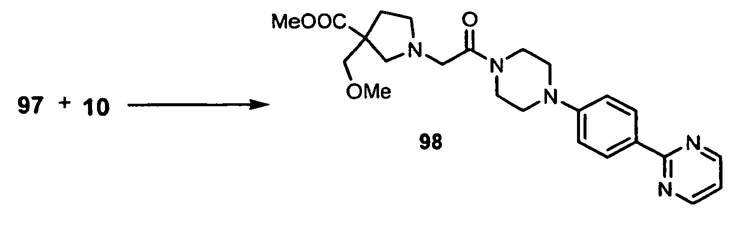

To a solution of 1-carboxymethyl-3-methoxymethyl-pyrrolidine-3-carboxylic acid methyl ester (compound 97) (0.21 mmol), 2-(4-piperazin-1-yl-phenyl)-pyrimidine (compound 10, see Example 1 Step 7) (0.21 mmol), O-(7-azabenzotriazol-1-yl-)-*N,N,N',N'*-tetramethyluronium hexafluorophosphate (HATU) (78 mg, 0.21 mmol) in dry DMF (2 ml) was added *N,N-*diisopropylethylamine (108 µl, 0.62 mmol). The reaction mixture was stirred for 4 hours, and evaporated to a residue that was partitioned in ethyl acetate and saturated sodium carbonate. Organic layer was isolated, washed with water, brine and dried over magnesium sulfate. Evaporation of solvent provided a crude product which was chromatographed with 5% methanol in dichloromethane to furnish the title compound (97 mg).

### Step 12

### Preparation of 3-methoxymethyl-1-{2-oxo-2-[4-(4-pyrimidin-2-yl-phenyl)-piperazin-1-yl]-ethyl}-pyrrolidine-3-carboxylic acid

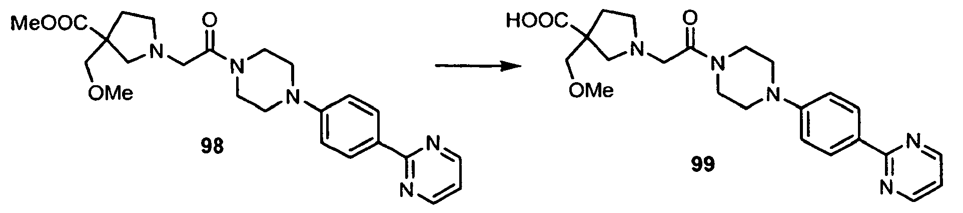

The 1-carboxymethyl-3-methoxymethyl-pyrrolidine-3-carboxylic acid methyl ester (97 mg, 0.21 mmol) was saponified with lithium hydroxide monohydrate (27 mg, 0.64 mmol) in tetrahydronfuran and water (2:1, 3 ml) for 2 hours. The reaction mixture was acidified with 4 N HCl and lyophilized overnight to provide the title compound which was directly used in the next step synthesis.

### Step 13

### Preparation of 3-methoxymethyl-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide

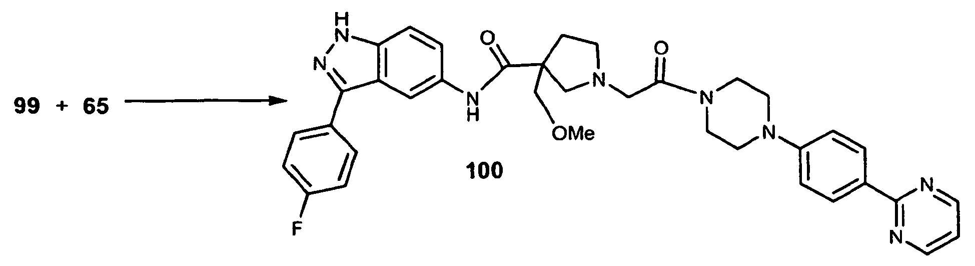

The 3-methoxymethyl-1-{2-oxo-2-[4-(4-pyrimidin-2-yl-phenyl)-piperazin-1-yl]-ethyl}-pyrrolidine-3-carboxylic acid (0.049 mmol), 1-hydroxybenztriazole (7 mg, 0.052 mmol) and 1-(3-dimethylaminopropyl)-3-ethylcarbodiimide hydrochloride (EDCI) (10 mg, 0.052 mmol) were dissolved in dry DMF (1 ml). 3-(4-Fluoro-phenyl)-1*H*-indazol-5-ylamine (12 mg, 0.053 mmol) was added and the reaction mixture was stirred overnight and directly subjected to purification by reversed phase HPLC to obtain 9.15 mg of the title product.
Mass Spec.: 649, Retention Time: 3.93 minutes.

### EXAMPLE 126 (PREPARATIVE EXAMPLE)

### Step 1

### Preparation of 2-fluoromothyl-acrylic acid ethyl ester

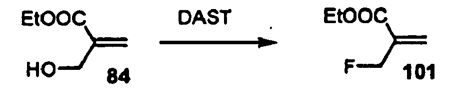

To a solution of (diethylamino)sulfur trifluoride (DAST) (363 µl, 2.76 mmol) in dichloromethane (1 ml) was slowly added at -78 C a solution of 2-hydroxylmethyl acrylic acid ethyl ester (300 mg, 2.31 mmol) in dichloromethane (3 ml). The reaction mixture was allowed to warm to room temperature, re-chilled to -78C and additional DAST (100 µl, 0.76 mmol) was added to ensure complete reaction. The reaction mixture was let to warm to room temperature and quenched with saturated sodium carbonate. Organic layer was isolated, washed with water, brine and dried (MgSO₄). The dichloromethane solution was directly passed through a short silica gel pad, eluted with dichloromethane and the product fractions were collected. The combined fractions (ca. 12 ml) will be directly used in the next step synthesis without further concentration.

### Step 2

### Preparation of 1-benzyl-3-fluoromethyl-pyrrolidine-3-carboxylic acid ethyl ester

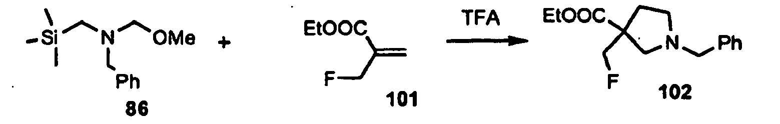

*N*-(methoxymethyl)-*N*-(trimethylsilylmethyl)benzylamine (548 µl, 2.14 mmol) was dissolved in the dichloromethane solution of the 2-fluoromethyl-acrylic acid ethyl ester from the previous reaction and chilled to 0 C. A solution of trifluoroacetic acid (67 µl, 0.87 mmol) in dichloromethane (0.5 ml) was added slowly. The reaction mixture was allowed to warm to room temperature in two hours and directly chromatographed on silica gel. Elution with solutions of ethyl acetate in hexanes (1 :5, 1:4, 1:3) obtained the title product (143 mg) as oil.

### EXAMPLE 127 (PREPARATIVE EXAMPLE)

### Preparation of 3-Allyl-pyrrolidine-1,3-dicartioxylic acid 1-tert-butyl ester 3-methyl ester

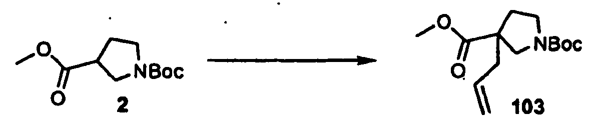

Pyrrolidine-1,3-dicarboxylic acid 1-tert-butyl ester 3-methyl ester 2 (see Example 1, Step 1) (4.58 g, 20 mmol) was dissolved in THF (100 mL) and cooled down to -78°C in a dry ice-acetone bath. LDA (12 mL, 2.0 M, 24 mmol) was then added dropwise. The mixture was stirred at -78°C for 1 hr. Allyl bromide (5.3 mL, 61 mmol) was added in neat. The reaction was allowed to warm to rt naturally and stirred for 24 hrs. It was then quenched with sat. NH4Cl solution, extracted with ethyl acetate 2X150 mL. The organic layer was washed with brine, dried (MgSO4) and concentrated. The crude was purified on silica gel column using 4:1 hexanes/ethyl acetate to get the title compound (3.6g) as a yellow oil. MS (292, MNa).

### EXAMPLE 128 (PREPARATIVE EXAMPLE)

### Step1

### Preparation of (S,S)-5-[4-(4,4,5,5-Tetramethyt-[1,3,2]dioxaborolan-2-yl)-phenyl]-2,5-diaza-bicyclo[2.2.1]heptane-2-carboxylic acid tert-butyl ester

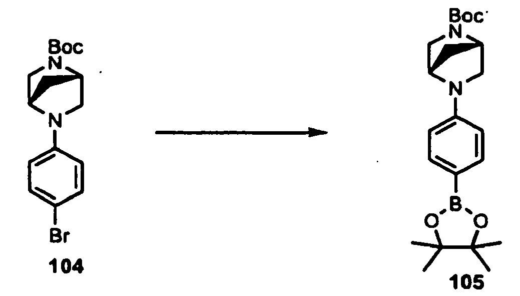

A mixture of (S,S)-5-(4-Bromo-phenyl)-2,5-diaza-bicyclo[2.2.1]heptane-2-carboxylic acid tert-butyl ester (4.0g, 11.3 mmol), Bis(pinacolato)diboron (4.0g, 15.7 mmol), KOAc (3.2g) and Cl₂Pd(dppf)CH₂Cl₂ (800 mg) in 40 mL dioxane was evacuated and recharged with N₂ several times. The reaction mixture was then heated to 85°C overnight. After cooling down to rt, 150 mL ethyl acetate and 30 mL water was added. The mixture was filtered through a pad of Celite and washed with additional ethyl acetate. The separated organic layer was dried (MgSO4) and concentrated. The crude was purified on silica gel column eluting with 30% to 50 % ethyl acetate/hexanes to yield the title compound as a white solid (3.3 g). MS (401, MH)

### Step 2

### Preparation of (S,S).5.[4-(5-Fluoro-pyrimidin-2-yl)-phenyl]-2,5-diaza-bicyclo[2.2.1]-heptane-2-carboxylic acid tert-butyl ester

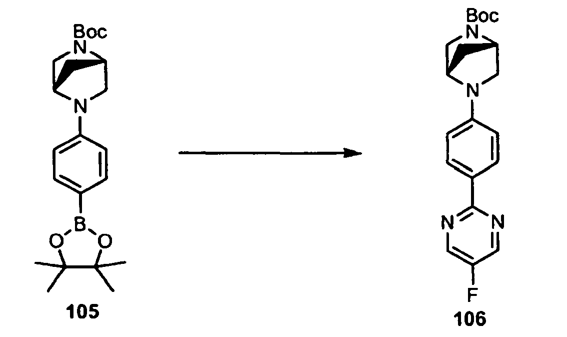

A mixed DMF/H2O (5 mU5 mL) solution of (S,S)-5-[4-(4,4,5,5-Tetramethyl-[1,3,2]dioxaborolan-2-yl)-phenyl]-2,5-diaza-bicyclo[2.2.1] heptane-2-carboxylic acid tert-butyl ester (800 mg, 2mmol), 2-chloro-5-fluoro-pyrimidine (340 mg, 2.6 mmol), K₂CO₃ (552 mg, 4 mmol) and Cl₂Pd(dppf)CH₂Cl₂ (160 mg) was evacuated and recharged with N₂ several times. The reaction was heated at 70 °C over 18 hrs. After cooling down to rt, 40 mL ethyl acetate and 10 mL water was added. The mixture was filtered through a pad of Celite and washed with additional ethyl acetate. The separated organic layer was dried (MgSO₄) and concentrated. The crude was purified on silica gel column eluting with 50 % ethyl acetate/hexanes to yield the title compound (420 mg) as a light yellow solid.

In a similar manner, 106a: 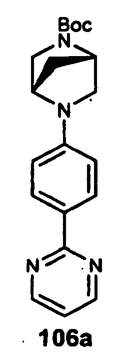 was prepared by substituting 2-chloropyrimidine for 2-chloro-5-fluoro-pyrimidine.

### EXAMPLE 129 (PREPARATIVE EXAMPLE)

### Preparation of 4-[4-(5-Fluoro-pyrimidin-2-yl)-phenyl]-piperazine-1-carboxylic acid tert-butyl ester

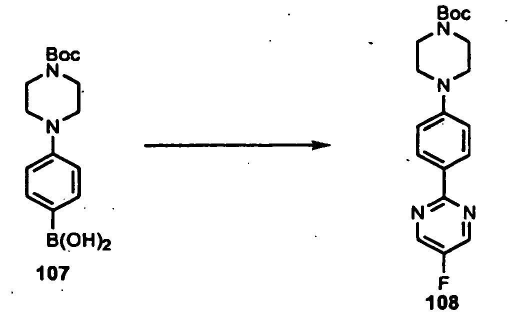

4-[4-(5-Fluoro-pyrimidin-2-yl)-phenyl]-piperazine-1-carboxylic acid tert-butyl ester was prepared similarly as the above substituting (S,S)-5-[4-(4,4.5,5-tetra**methyl[1,3,2]dioxaborolan-2-yl)-phenyl]-2,5-diaza-bicyclo[2.2.1]** heptane-2-carboxylic acid tert-butyl ester with 4-[4-(*tert-*Butoxycarbony)piperazin-1-yl]phenylboronic' acid (C. Chen et. al. J. Org. Chem. 2003, 68, 2633).

### EXAMPLE 130 (PREPARATIVE EXAMPLE)

### Preparation of (S,S)-5-(5-Vinyl-pyrimidin-2-yl)-2,5-diaza-bicyclo[2.2.1]heptane-2-carboxylic acid tert-butyl ester

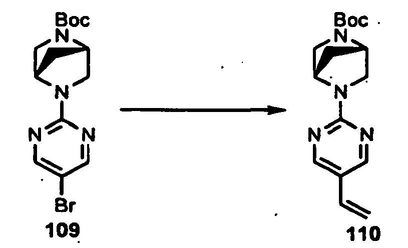

(S,S)-5-(5-Bromo-pyrimidin-2-y)2,5-diaza-bicyclo[2.2.1]heptane-2-carboxylic acid tert-butyl ester (177 mg, 0.5 mmol), tributyl vinyl tin (634 mg, 2 mmol) and Cl₂Pd(dppf)CH₂Cl₂ (60 mg) was mixed in DMF (3 mL). The mixture was heated at 90 °C over 3 days. The cooled down reaction was participate between ethyl acetate (50 mL) and H₂O (10 mL). The organic layer was washed with H₂O (10 mL), brine (10 mL), dried (MgSO₄) and filtered. The conc. filtrate was purified on silica gel column eluting with 33% to 50% ethyl acetate/hexanes to yield the title compound as a whit solid (54 mg). MS (303, MH).

### EXAMPLE 131 (PREPARATIVE EXAMPLE)

### Preparation of 1H-Pyrazolo[3,4-b]pyridin-5-ylamine

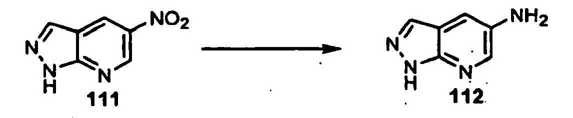

5-Nitro-1H-pyrazolo[3,4-b]pyridine (prepared according to the procedure in Can.. J. Chem. 1988, 66(3), 420) (133 mg, 0.81 mmol) was mixed with SnCl₂ (1.Og) in EtOH/CH₃Ph (4 mU2mL) and heated at 70°C for 3 hrs. The reaction-was cooled to rt and concentrated to dryness. The residue was participated between 1.0 N NaOH (10 mL) and ethyl acetate (50 mL). The organic layer was washed with brine once, dried (MgSO₄ and filtered. The filtrate was concentrated and the resulting crude was purified by prep. TLC using 10:2 CH₂Cl₂/Methanol(2 N NH₃). The title compound (15.0 mg) was isolated as a yellow solid.

### EXAMPLE 132 (PREPARATIVE EXAMPLE)

### Preparation of 4-Hydroxy-4-thiazol-2-yl-piperidine-l-carboxylic acid tert-butyl ester

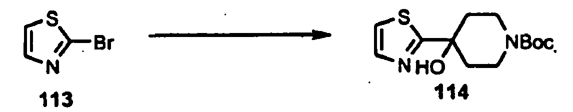

2-Bromo-thiazole (0.27 mL, 2.99 mmol) was dissolved in Et₂O (8 mL) and cooled down to - 78 °C. BuLi (1.3 mL, 2.5 M) was added dropwise. The resulting yellow solution was stirred, at -78 °C for 45 min. 4-Oxo-piperidine-1-carboxylic acid tert-butyl ester (720 mg, 3.61 mmol) in Et₂O (5 mL) was then added dropwise. The reaction temperature rose to rt naturally overnight. H₂O (10 mL) was added to quench the reaction and extracted with ethyl acetate. The combined organic layer was dried (MgSO₄), filtered and concentrated. The residue was purified on silica gel column eluting with 33% to 50% ethyl acetate/hexanes to give 4-Hydroxy-4-thiazol-2-yi-piperidine-1-carboxylic acid tert-butyl ester (800 mg) as a colorless oil.

### EXAMPLE 133 (PREPARATIVE EXAMPLE)

### Step 1

### Preparation of 4-Thiazol-2-yl-piperazine-1-carboxylic acid tert-butyl ester

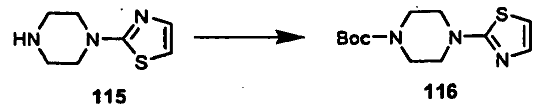

To a solution of 1-thiazol-2-yl-piperazine (2 g, 12 mmol), triethylamine (2.4 g, 24 mmol) and DMAP (150 mg, 1.2 mmol) in acetonitrile (15 ml) was added di-*tert-*butyl dicarbonate. The resulted reaction mixture was stirred at RT for 3 hours. Then water (20 mL) was added and the formed slurry was stirred for 30 min. The formed product was collected by filtration and washed with water. After dry in air, 2.8 g product was obtained (90 % yield)

### Step 2

### Preparation of 4-(5-Bromo-thiazol-2-yi)-piperazine-1-carboxylic acid tert-butyl ester

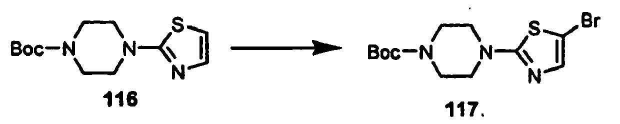

To a mixture containing 4-thiazol-2-yl-piperazine-1-carboxylic acid tert-butyl ester (0.5 g, 1.9 mmol) and cesium carbonate (0.62 mmol) in chloroform (5 mL) at 0 °C, bromine (110 mL) was added through a syringe. After the addition, the reaction mixture was stirred at room temperature for 1 hour. Water was added and the organic layer was collected and dried over sodium sulfate. After removal of solvent, 0.6 g of product was obtained (95% yield).

### EXAMPLE 134 (PREPARATIVE EXAMPLE)

### Preparation of 5-Thiazol-2-yl-2, 5-diaza-bicyclo[2.2.1]heptane-2-carboxylic acid tert-butyl ester

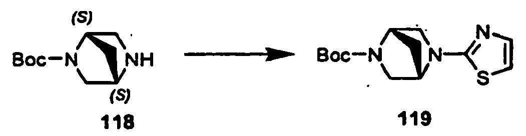

A mixture of 2-bromothiazole (200mg,1.22mmol), palladium acetate (15 mg, 0.06 mmol), sodium *tert-*butoxide (217 mg, 2.26 mmol), (S, S) 2,5-diaza-bicyclo[2.2.1]heptane-2-carboxylic acid tert-butyl ester (280mg, 1.4 mmol) and 2-Di-t-butylphosphino)-biphenyl (37 mg, 0.118mmol) was stirred in dioxane (10ml) at 80°C for overnight. The reaction was cooled, diluted with ethyl acetate (40 ml) and H₂O (50ml).The organic layer was separated, dried (Na₂SO₄), filtered and solvent evaporated. The residue was purified by chromatography eluting with 5% MeOH/DCM yielding product as a white solid. (180 mg, 52% yield)

### EXAMPLE 135 (PREPARATIVE EXAMPLE)

### Step 1

### Preparation of 4-(5-Pyrimidin-2-yl-thlazol-2-yl)-piperazine-1-carboxylic acid tert-butyl ester

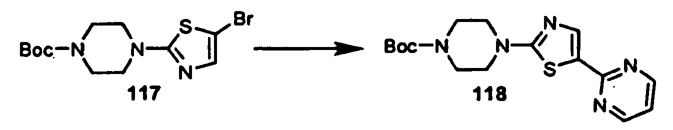

A round bottom flask containing 4-(5-bromo-thiazol-2-yl)-piperazine-1-carboxylic acid tert-butyl ester (100 mg, 0.29 mmol), 2-tributylstannanyl-pyrimidine (130 mg, 0.36 mmol), cesium fluoride (85 mg, 0.56 mmol) and palladium di-tert-butylphosphine was degassed three times with Ar. Dioxane was added and the formed reaction mixture was stirred at 90°C overnight under Ar. Then the reaction mixture was filter through celite and the solvent was remove under vacuum and crude product was used directly in the next step.

### Step 2

### Preparation of 2-(2-Piperazin-1-yl-thiazol-5-yl)-pyrimidine

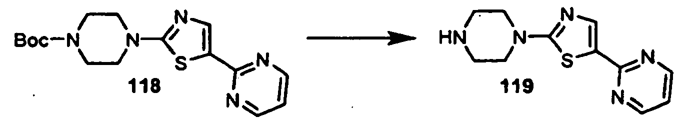

To the crude product obtained in the previous step, was added 90% TFA (1 mL) and the reaction mixture was stirred at ambient temperature for 1 hour. The excess TFA was removed under vacuum and the residue was purified using prep- .. HPLC to give desired product (45 mg, 44 % yield for two steps) as TFA salt.

### Step 3

### Preparation of 5-[(1-{2-Oxo-2-[4-(5-pyrimidin-2-yl-thiazol-2-yl)-piperazin-1-yl]-ethyl}-pyrrolidine-3-carbonyl)-amino]-1H-indazole-3-carboxylic acid methylamide

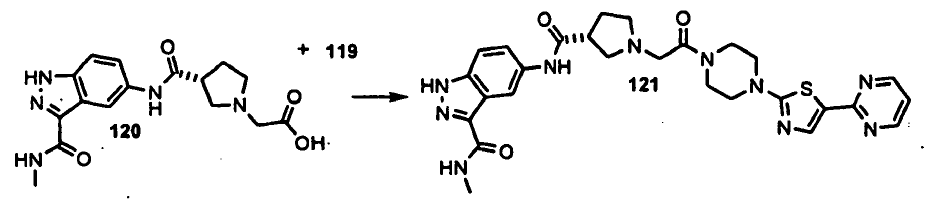

To a solution of [3-(3-methylcarbamoyl-1H-indazol-5-ylcarbamoyl)-pyrrolidin-1-yl]-acetic acid (15 mg, 0.043 mmol, prepared by a procedure similar to that of Example 98 using the indazole of Example 85 instead of indazole 65) and HOBt (7 mg, 0.052 mmol) in DMF (0.5 mL) at 0 °C, EDCI (10 mg, 0.052 mmol) was added and the resulted reaction mixture was stirred at this temperature for 0.5 hour. To this solution was added 2-(2-Piperazin-1-yl-thiazol-5-yl)-pyrimidine (18 mg, 0.052 mmol) made from previous step, followed by DIEA (7 µL). The reaction was stirred at 0 °C for 1 hour and was gradually warm up to room temperature and was continuously stirred for overnight. Ethyl acetate (5 mL) was then added, followed by water (10 mL). The organic layer was collected, dry over sodium sulfate, evaporate of solvent and residue was purified using prep-HPLC. To the solution obtained from HPCL, HCl (1N, 2 mL) was added and the solution was brought to dryness under vacuum. The formed residue was then dissolved in acetonitrile/water (3:1) and was lyophilized to give desired product (10 mg, 38 % yield) as hydrochloride salt.

### EXAMPLE 136 (PREPARATIVE EXAMPLE)

### Step 1

### Preparation of 4-(4-Bromo-phenyl)-piperidine-1-carboxylic acid tert-butyl ester

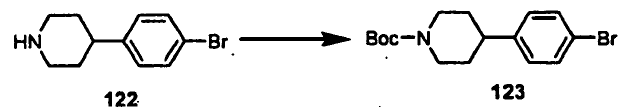

To a solution of 4-(4-bromo-phenyl)-piperidine (2.8 g, 12 mmol), triethylamine (2.4 g, 24 mmol) and DMAP (150 mg, 1.2 mmol) in acetonitrile (15 ml) was added di-*tert-*butyl dicarbonate. The resulted reaction mixture was stirred at RT for 3 hours. Then water (20 mL) was added and the formed slurry was stirred for 30 min. The formed product was collected by filtration and washed with water. After dry in air, 3.8 g product was obtained (95 % yield).

### Step 2

### Preparation of 4-(4-Pyrimidin-2-yl-phenyl)-piperidine-1-carboxylic acid tert-butyl ester

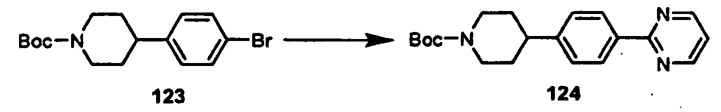

A mixture containing 4-(4-bromo-phenyl)-piperidine-1-carboxylic acid tert-butyl ester (100 mg, 0.29 mmol), 2-tributylstannanyl-pyrimidine (130 mg, 0.36 mmol), cesium fluoride (85 mg, 0.56 mmol) and palladium di-tert-butylphosphine was degassed three times with Ar. Dioxane was added and the formed reaction mixture was stirred at 90°C overnight under Ar. Then the reaction mixture was filter through celite and the solvent was removed under vacuum and crude product was used directly in the next step.

### Step 3

### Preparation of 2-(4-Piperidin-4-yl-phenyl)-pyrimidine

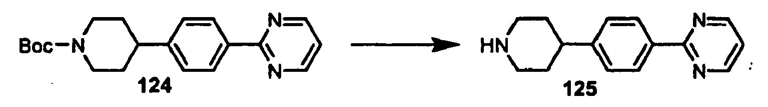

To the crude product obtained in the previous step, was added 90% TFA (1 mL) and the reaction mixture was stirred at ambient temperature for 1 hour. The excess TFA was removed under vacuum and the residue was purified using prep-HPLC to give desired product (38 mg, 37 % yield for two steps) as TFA salt

### EXAMPLE 137 (PREPARATIVE EXAMPLE)

### Step 1

### Preparation of 4-Thiazol-2-yl-3, 6-dihydro-2H-pyridine-1-carboxylic acid tert-butyl ester

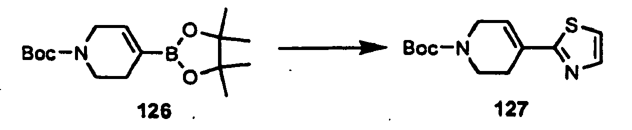

To a mixture of (N-tert-butoxycarbonyl)-1,2,3,6-tetrahydropryidine -2-boronic acid pinacol ester (100 mg, 0.32 mmol), 2-bromothiazole (64 mg, 0.39 mmol), PdCl₂(dppf) (24 mg, 0.03 mmol) and potassium phosphate (213 mg, 1 mmol) was degassed three times with Ar, was added dixoane. The formed reaction mixture was then heated at 80 °C overnight under Ar. After the reaction was complete, the mixture was filter through celite and was chromatographed on a silica column (10% ethyl acetate/DCM) to obtain desired product (30 mg, 35% yield).

### Step 2

### Preparation of 4-Thiazol-2-yl-1, 2, 3, 6-tetrahydro-pyridine

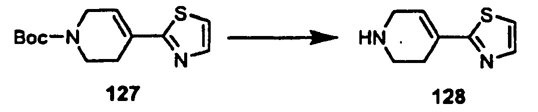

To the product obtained in the previous step, was added 90% TFA (1 mL) and the reaction mixture was stirred at ambient temperature for 1 hour. The excess TFA was removed under vacuum and the residue was purified using prep-HPLC to give desired product (15 mg, 50 % yield) as TFA salt.

### EXAMPLE 138 (PREPARATIVE EXAMPLE)

### Step 1

### Preparation of 4-(methoxy-methyl-carbamoyl)-piperidine-1-carboxylic acid tert-butyl ester

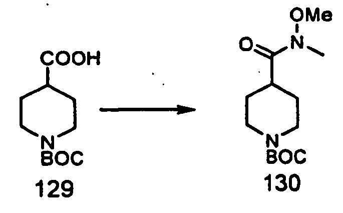

*N*,*O*-dimethylhydroxylamine hydrochloride (851 mg, 8.72 mmols) was suspended in dichloromethane. (6 ml) and cooled to 0 C. *N*,*N*'-diisopropylethylamine (1.66 ml, 9.53 mmols) was added and the mixture was stirred at 0 C until a clear solution was obtained. The resulting solution was kept at 0 C for further use. Boc-isonipecotic acid (2 g, 8.72 mmol), 1-hydroxybenzotriazole (1.2 g, 8.88 mmols) and 1-(3-dimethylaminopropyl)-3-ethylcarbodiimide hydrochloride (EDCl) (1.83 g, 9.58 mmols) were dissolved in DMF (15 ml) and cooled to 0 C. The solution of N,O-dimethylhydroxylamine in dichloromethane was added with stirring, and the resulting reaction mixture was allowed to stir overnight at room temperature. DMF was removed under reduced pressure and residue was partitioned between ethyl acetate and 10% citric acid. Organic layer was isolated, washed with water, saturated NaHCO₃. water and brine and dried over MgSO₄. Solvent was removed under reduced pressure and the residue was purified on silica gel eluting with ethyl acetate in hexanes (2:1) to provide the title compound (1.88 g, 79%). LCMS m/e (295, M + Na).

### Step 2

### Preparation of 4-formyl-piperidine-1-carboxylic acid tert-butyl ester

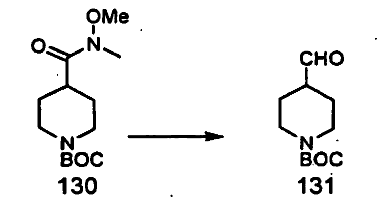

To a mixture of lithium aluminum hydride (1M THF solution, 4.4 ml) in ether (4 ml) was added dropwise at - 60 C 4-(methoxy-methyl-carbamoyl)-piperidine-1-carboxylic acid *tert-*butyl ester (1g, 3.67 mmols) in ether (6 ml). The reaction mixture was allowed to warm to 0-5 C and then re-cooled to -60 C. Celite was added and reaction was quenched with a solution of KHSO₄ (1g) in water (3 ml), filtered through Celite. The filtrate was washed with cold 1 N HCl, saturated NaHCO₃, brine and dried (MgSO₄) and concentrated. The residue was purified by column chromatography on silica gel eluting with ethyl acetate in hexanes (1:1) to provide title compound (656. mg, 84%). (Org. Prep. Proced. Int., 2000, 32, 96.)

### EXAMPLE 139 (PREPARATIVE EXAMPLE)

### Step 1

### Preparation of 4-methyl-benzenesulfonyl azide

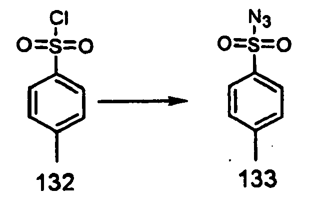

To a solution of tosyl chloride (4 g, 21 mmols) in acetone (60 ml) was added at 0-5 C a solution of sodium azide (1.37 g, 21 mmols) and the resulting solution was stirred at that temperature for 2 hours. Acetone was removed and the aqueous mixture was extracted with ether three times. The combined extracts were dried over MgSO₄. Evaporation of solvents provided tosyl azide (4g, 97%). (Eur. J. Org. Chem. 2003, 821-832.)

### Step 2

### Preparation of (1-diazo-2-oxo-propyl)-phosphonic acid dimethyl ester

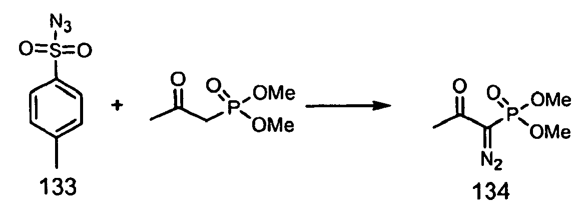

To a suspension of NaH (60% in mineral oil, 0.83 g, 20.8 mmols) in THF (50 ml) was added dropwise at 0 C (2-oxo-propyl)-phosphonic acid dimethyl ester (3.1 g, 18.7 mmols) in THF (50 ml), and the solution was stirred at 0 C for one hour. Tosyl azide (4g, 20 mmols) was added in one portion, stirred at 0 C for 10 minutes, filtered through Celite and concentrated. The residue was purified by column chromatography on silica gel using ethyl acetate to yield the title compound (2.9 g, 81%) as oil. (Eur. J. Org. Chem. 2003, 821-832.)

### Step 3

### Preparation of 4-ethynyl-piperidine-1-carboxylic acid tert-butyl ester

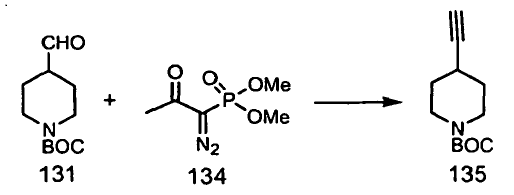

At 0 C, to a stirred mixture of 4-formyl-piperidine-1-carboxylic acid *tert-*butyl ester (358 mg, 1.68 mmols) and potassium carbonate (464 mg, 3.36 mmols) in methanol (16 ml) was added dropwise a solution of (1-diazo-2-oxo-propyl)-phosphonic acid dimethyl ester (323 mg, 1.68 mmols) in methanol (2 ml). The resulting mixture was stirred at room temperature overnight, filtered and concentrated. The residue was chromatographed on silica gel using a solution of ethyl acetate in hexanes (1:5) to provide the title compound (308 mg, 88%) as colorless crystals. LCMS m/e (154, M - *t*-Bu + 2H). (J. Am. Chem. Soc. 2003, 125, 3714.)

### Step 4

### Preparation of 4-phenylethynyl-piperidine-1-carboxylic acid tert-butyl ester

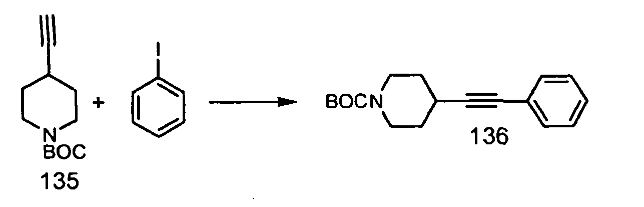

Iodobenzene (135 µl, 1.2 mmols), 4-ethynyl-pipeddine-1-carboxylic acid *tert-*butyl ester (209 mg, 1 mmols) and triethylamine (167 µl, 1.2 mmols) were dissolved in acetonitrile (6 ml). Dichlorobis(triphenylphosphine)palladium(II) (35 mg, 0.05 mmols) and Cul (10 mg, 0.05 mmols) were added, and reaction mixture was stirred at room temperature overnight and continued to stir at 50 C for two more hours before partitioning between ethyl acetate and water. Organic layer was isolated, washed with 1 N HCl, brine and dried (MgSO₄). Solvents were removed and residue was purified by column chromatography on silica gel using solutions of ethyl acetate in hexanes (1:4; 1:2) to yield the title compound (74 mg). LCMS m/e (230, M - *t*-Bu + 2H)

### Step 5

### Preparation of 4-phenylethynyl-piperidine

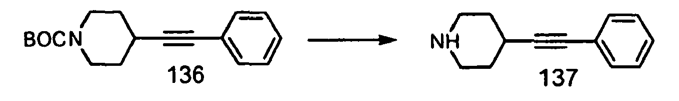

4-Phenylethynyl-piperidine-1-carboxylic acid *tert-*butyl ester was treated with TFA for 10 minutes and concentrated, lyophilized to provide the title product.

### EXAMPLE 140 (PREPARATIVE EXAMPLE)

### Step 1

### Preparation of 4-pyrimidin-2-ylethynyl-piperidine-1-carboxylic acid tert-butyl ester

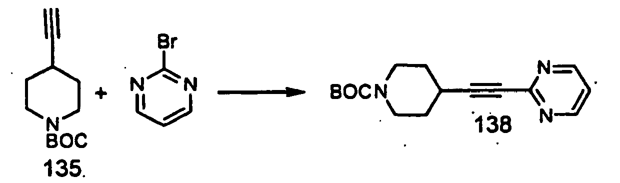

To a suspension of 2-bromopyrimidine (175 mg, 1.1 mmols), dichlorobis(triphenylphosphine)palladium(II) (35 mg, 0.05 mmols) and Cul (10 mg, 0.05 mmols) was added a solution of 4-ethynyl-piperidine-1-carboxylic acid *tert-*butyl ester (209 mg, 1 mmol). The mixture was stirred overnight, filtered through Celite, concentrated. The residue was partitioned between ethyl acetate and water, organic layer was isolated, dried (MgSO₄), and concentrated. The residue was chromatographed on silica gel eluting with ethyl acetate in hexanes (1:1) to give unreacted 2-bromopyrimidine (130 mg), then the title compound (23 mg). LCMS m/e (288, M + H).

### Step 2

### Preparation of 2-piperidin-4-ylethynyl-pyrimidine

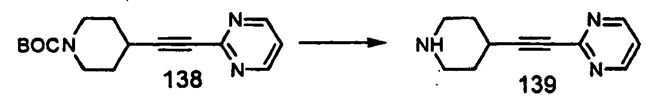

4-Pyrimidin-2-ylethynyl-piperidine-1-carboxylic acid *tert*-butyl ester was treated with TFA for 10 minutes and concentrated, lyophilized to provide the title product.

### EXAMPLE 141

### Preparation of 5-({1-[2-oxo-2-(4-phenylethynyl-piperidin-1-yl)-ethyl]-pyrrolidine-3-carbonyl}-amino)-1H-indazole-3-carboxylic acid methylamide

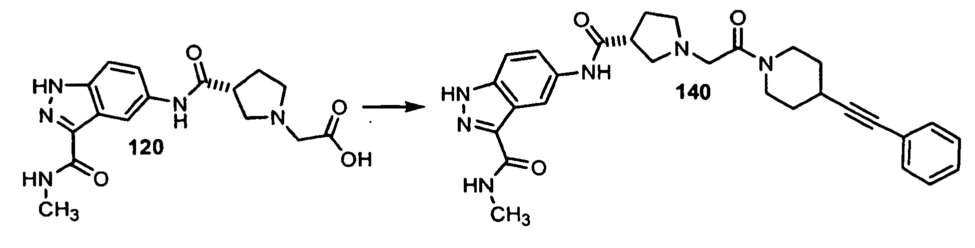

A solution of [3-(3-methylcarbamoyl-1H-indazol-5-ylcarbamoyl)-pyrrolidin-1-yl]-acetic acid (0.13 mmols), 4-phenylethynyl-piperidine (0.13 mmols), 1-hydroxybenztriazole (18 mg, 0.13 mmol) and 1-(3-dimethylaminopropyl)-3-ethylcarbodiimide hydrochloride (EDCI) (27 mg, 0.14 mmol) and DIEA (45 µl, 0.26 mmols) was stirred overnight and directly subjected to purification by reversed phase HPLC to obtain 16 mg of the title product.

### EXAMPLE 142

### Preparation of 5-({1-[2-oxo-2-(4-pyrimidin-2-ylethynyl-piperidin-1-yl)-ethyl]-pyrrolidine-3-carbonyl}-amino)-1H-indazole-3-carboxylic acid methylamide

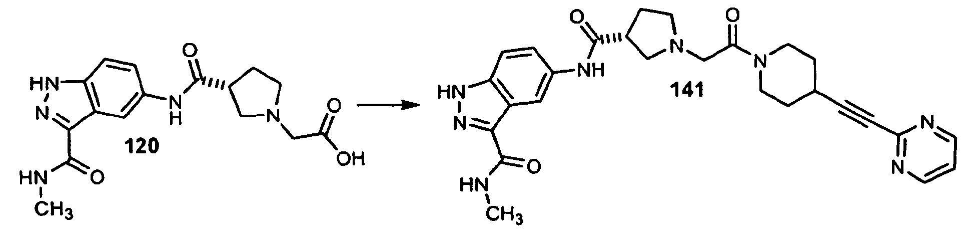

Following a procedure similar to that of Example 141, but using 2-piperidin-4-ylethynyl-pyrimidine instead of 4-phenylethynyl-piperidine the title compound was prepared.

### EXAMPLES 143 TO 151

Following a procedure similar to Example 135, Step 3, but using the appropriately substituted piperazine, and the suitably substituted indazole, and the appropriate amine for MeNH₂ in Example 87, the compounds in Table 9 can be prepared.

**TABLE 9**

| **Example** | **COMPOUND** |
|---|---|
| 143 | 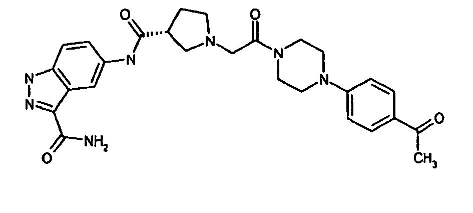 |
| 144 | 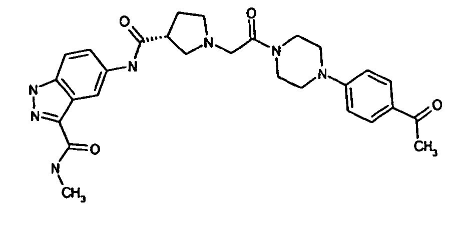 |
| 145 | 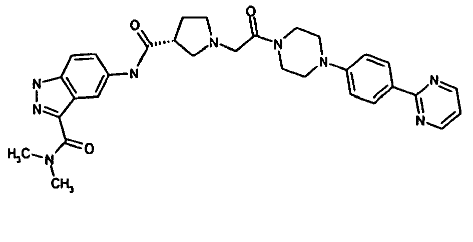 |
| 146 | 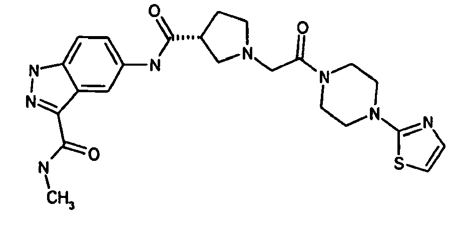 |
| 147 | 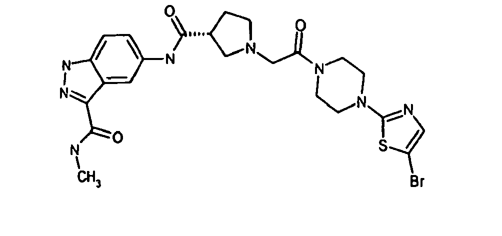 |
| 148 | 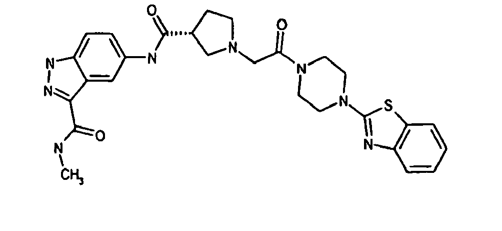 |
| 149 | 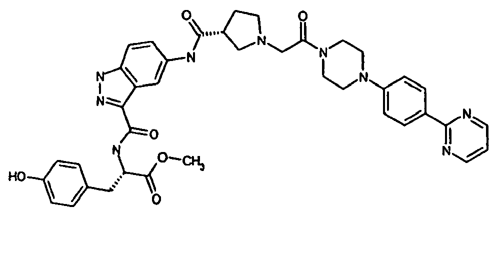 |
| 150 | 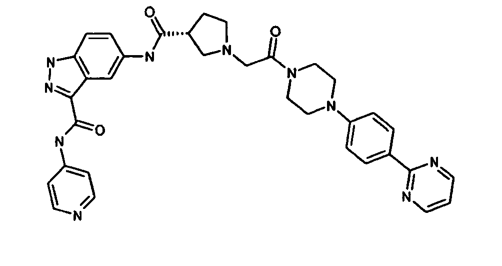 |
| 151 | 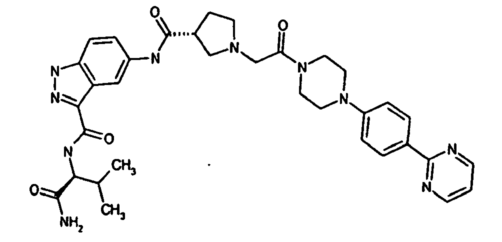 |

### EXAMPLES 152 TO 155

Following a procedure similar to that of Example 85, but using the appropriately substituted piperazine derivative, as prepared according to the procedures in Examples 128-134, the compounds in Table 10 were prepared.

**TABLE 10**

| **Example** | **COMPOUND** |
|---|---|
| 152 | 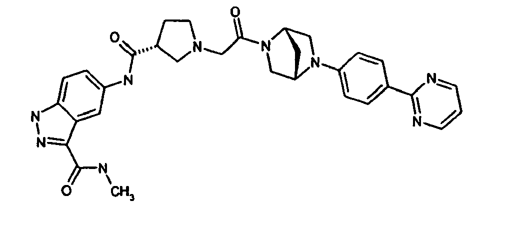 |
| 153 | 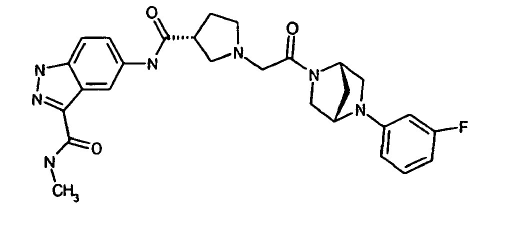 |
| 154 | 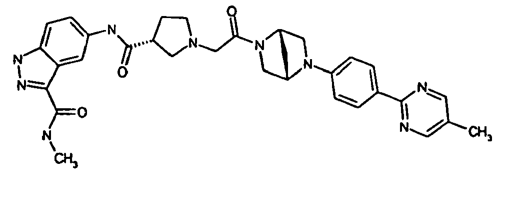 |
| 155 | 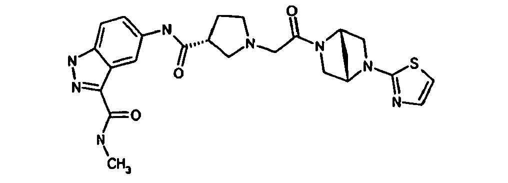 |

### EXAMPLE 156

Following a procedure similar to that of Example 125, Using compound 90 from Example 125 Step 5 in place of compound 96 in Step 10, and using 4-piperazin-1-yl-thiazolyl in place of 2-(4-piperazin-1-yl-phenyl)-pyrimidine in Step 11, the compound in Table 11 was prepared.

**Table 11**

| **Example** | **Compound** | **Mass Spec** | **Retention time (minutes)** |
|---|---|---|---|
| 156 | 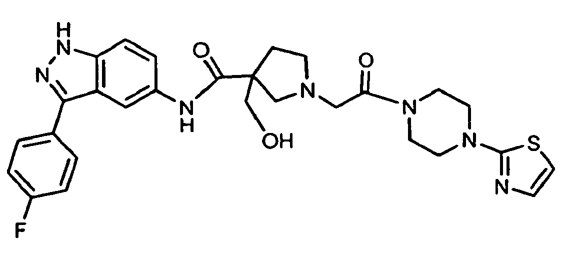 | 564 | 2.68 |

### EXAMPLES 157 and 158

Following a procedure similar to that of Example 125, but using the appropriate pyrrolidene and substituted piperazine, as in Example 128, the compounds in Table 12 can be prepared. In Table 12 "Ex" represents "Example".

**Table 12**

| **Ex** | **Compound** | **Mass Spec** | **Retention time (minutes)** |
|---|---|---|---|
| 157 | 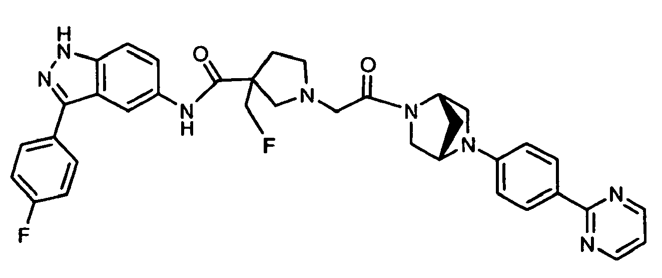 | 649 | 3.74 |
| 158 | 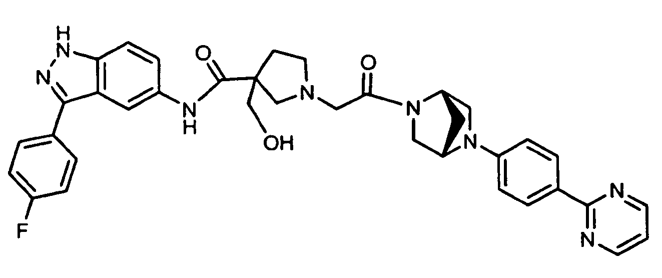 | 647 | 3.56 |

### EXAMPLES 159 TO 164

Following procedures similar to those described herein, for example, Examples 98, 133 and 135, the compounds in Table 13 were prepared. In Table 13 "Ex" represents "Example".

**TABLE 13**

| **EX** | **Structure** | **MS (ESMS, MH)** | **Retention time (min.)** |
|---|---|---|---|
| 159 | 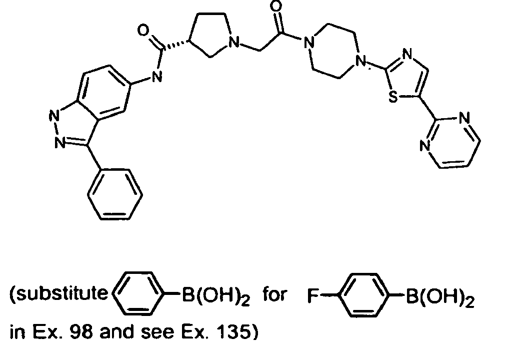 | 594 | 3.25 |
| 160 | 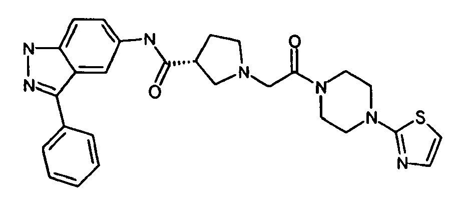 (see Ex. 133) | 516 | 2.65 |
| 161 | 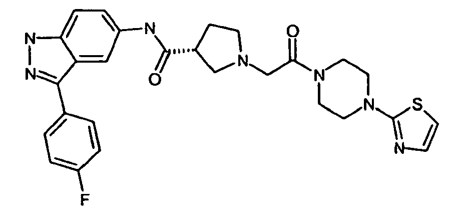 (see Ex. 98 and Ex. 133) | 534 | 2.8 |
| 162 | 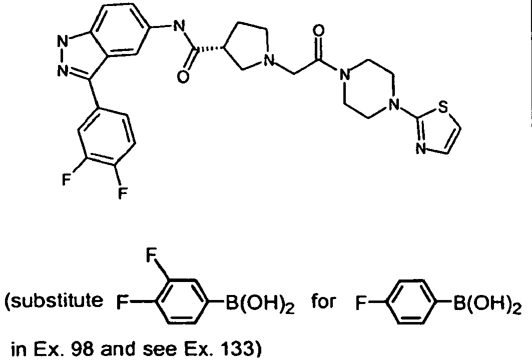 | 552 | 2.95 |
| 163 | 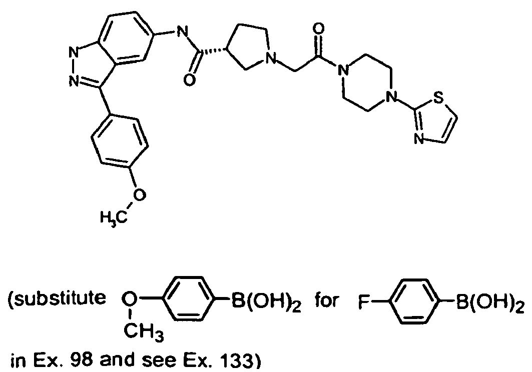 | 546 | 3.02 |
| 164 | 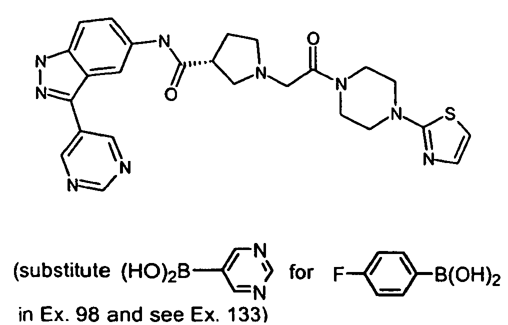 | 518 | 1.93 |

### EXAMPLE 165

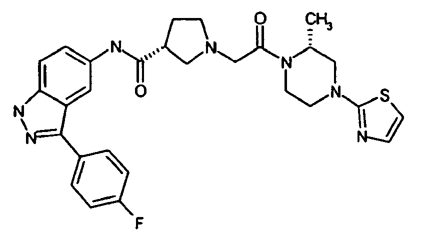

### Step 1

### Preparation of 3-methyl-1-thiazol-2-yl-piperazine

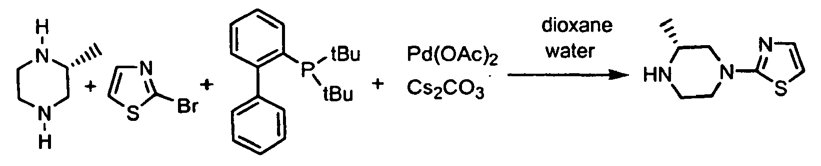

A mixture of 2(R)-methyl piperazine (300 mg, 3 mmol), 2-bromo thiazole (0.27 ml, 3 mmol), (2-biphenylyldi-tert-butylphosphine (134 mg, 0.449 mmol), palladium acetate (101 mg, 0.45 mml), and cesium carbonate (1.46 g, 4.49 mmol) in dioxane 25 ml (v/v 5/1) was kept at reflux temperature for 2 hours, then cooled to room temperature, then filtered through celite, then concentrated and then purified by chromatography eluting with 12%MeOH/MeCl₂/NH₄0H to yield the product as a white solid (145 mg, 26%).

### Step 2

### Preparation of 1-[2-(2-methyl-4-thiazol-2-yl-piperazin-1-yl)-2-oxo-ethyl]-pyrrolidine-3-carboxylic acid [3-(4-fluoro-pbenyl)-1H-indazol-5-yl]-amide

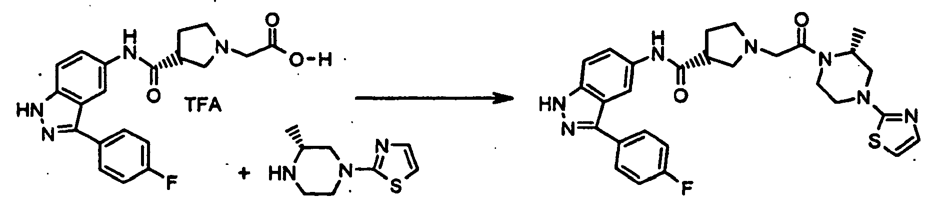

A mixture of 3-methyl-1-thiazol-2-yl-piperazine (11 mg, 0.060 mmol), {3-[3-(4-fluorophenyl)-1H-indazol-5-ylcarbamoyl]-pyrrolidin-1-yl}-acidic acid, trifluoroacetate (30mg, 0.06 mmol), EDCl.HCl (11 mg, 0.06 mmol), 1-hydroxybenzotriazole (9 mg; 0.066) mmol) and NMM (0.04 ml) was stirred in DMF (2ml) overnight at room temperature. The reaction was diluted with EtOAc (50 ml), washed with brine (20 ml), dried over MgSO₄, filtered and the solvent was evaporated. The resulting material was purified on reverse phase HPLC (C₁₈ column), eluting with acetonitrile:water, yielding product which was dissolved in MeCl₂ basified with saturated NaHC0₃; dried over MgSO₄, filtered and solvent evaporated yielding title product (10 mg). LCMS MH 548. Retention time: 2.36 minutes.

### EXAMPLES 166 TO 179

Following procedures similar to those described herein, for example, Examples 85, 98, 137 and 136, the compounds in Table 14 were prepared. In Table 14 "Ex" represents "Example".

**TABLE 14**

| **EX** | **Structure** | **MS (ESMS, MH)** | **Retention time (min.)** |
|---|---|---|---|
| 166 | 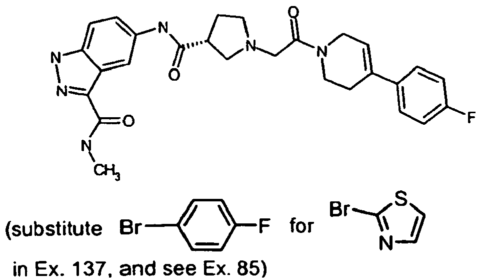 | 505 | 3.42 |
| 167 | 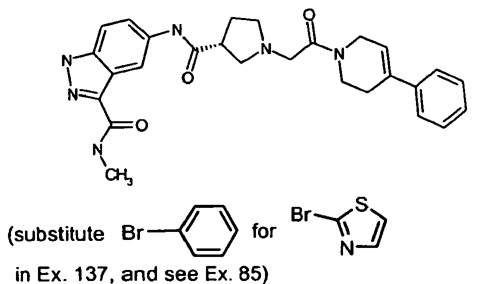 | 487 | 3.34 |
| 168 | 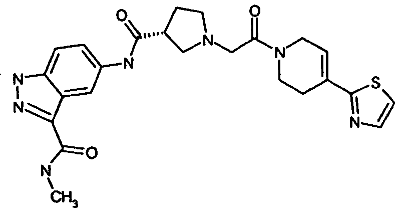 (see Ex. 85 and Ex. 137) | 494 | 2.43 |
| 169 | 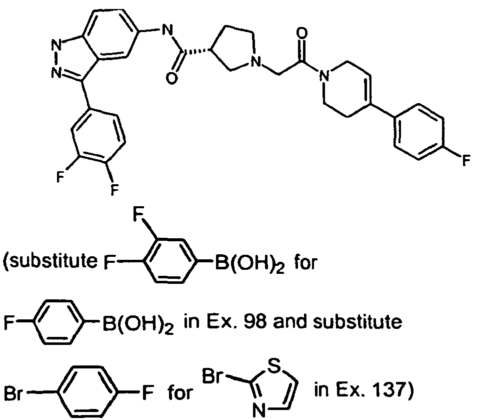 | 560 | 4.27 |
| 170 | 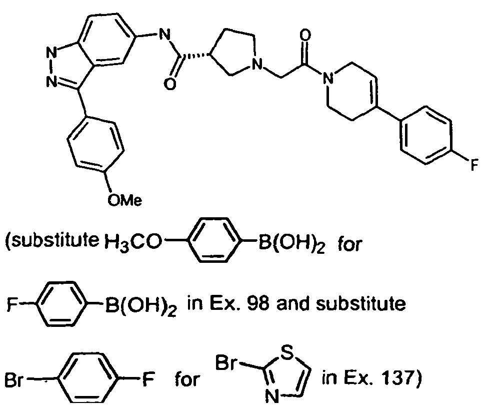 | 554 | 4.01 |
| 171 | 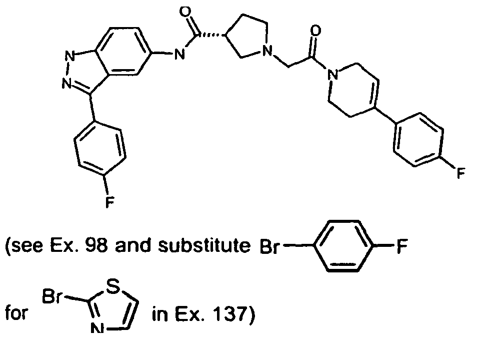 | 542 | 4.11 |
| 172 | 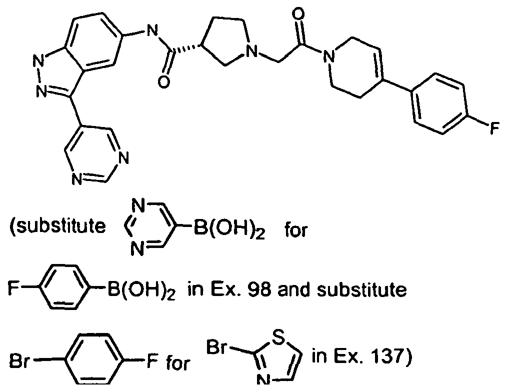 | 526 | 3.35 |
| 173 | 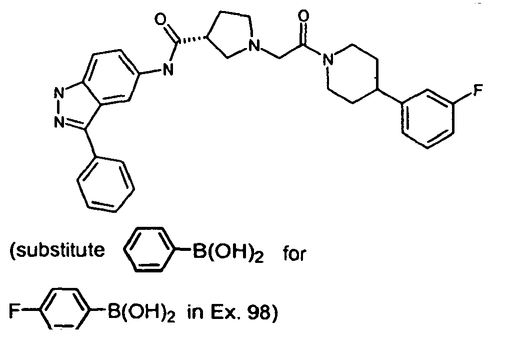 | 526 | 1.51 |
| 174 | 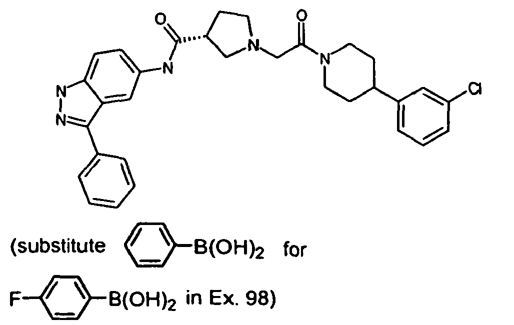 | 542 | 1.59 |
| 175 | 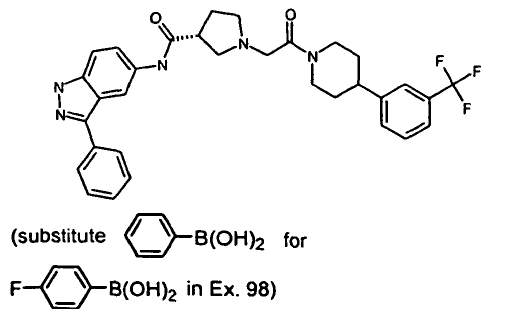 | 576 | 1.64 |
| 176 | 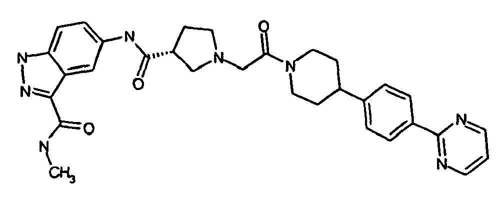 (see Ex. 85 and Ex. 136) | 567 | 3.16 |
| 177 | 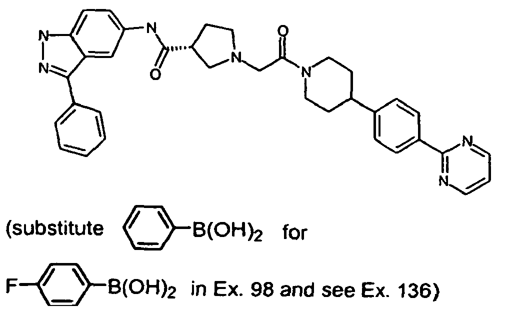 | 586 | 3.85 |
| 178 | 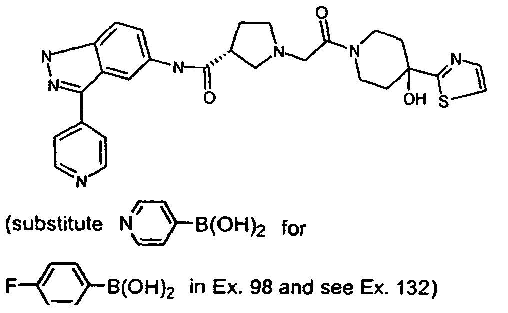 | | |
| 179 | 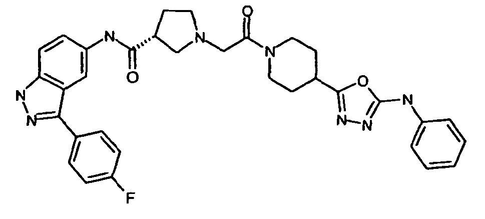 (see Ex.98) | | |

### EXAMPLES 181 and 183 to 259

Following procedures similar to those described herein, for example, Examples 1, 3 to 60, 85, 98, 128, 183, 184, the compounds in Table 15 were prepared. In Table 15 "Ex" represents "Example".

**TABLE 15**

| **EX** | **COMPOUND** |
|---|---|
| 181 | 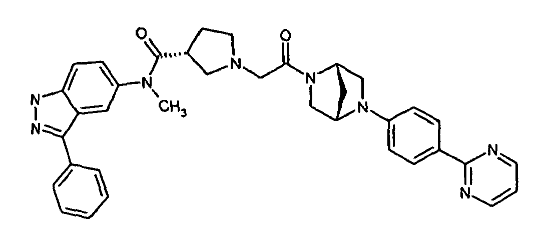 |
| 183 | 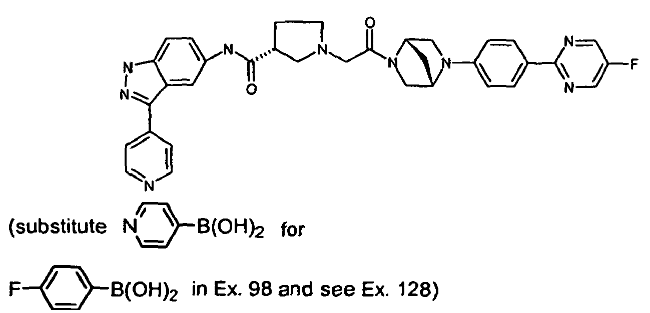 |
| 184 | 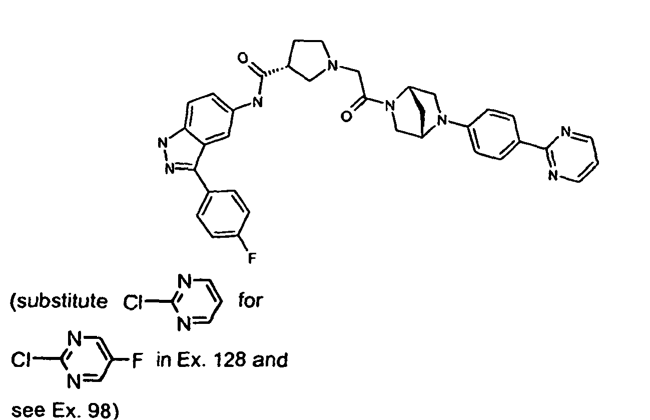 |
| 185 | 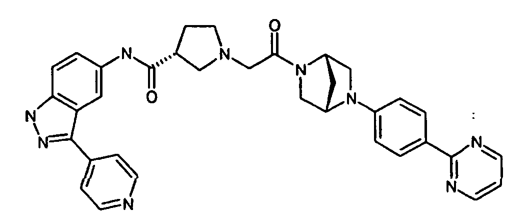 |
| | (see Ex. 183 and Ex. 184) |
| 186 | 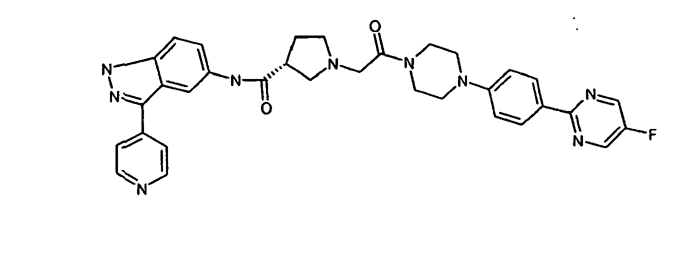 |
| 187 | 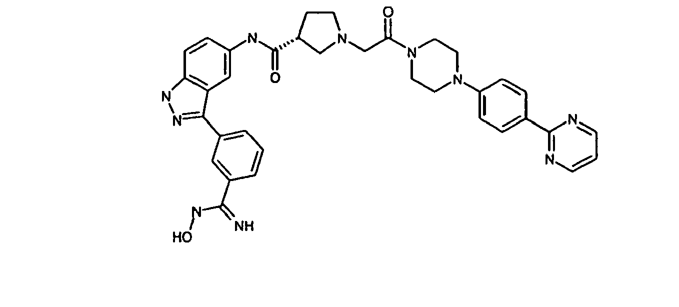 |
| 188 | 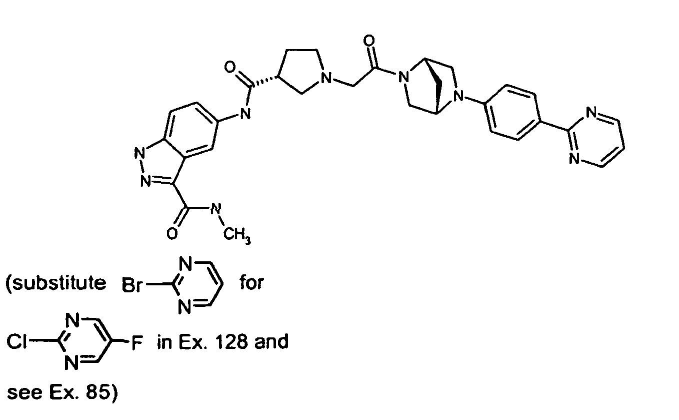 |
| 189 | 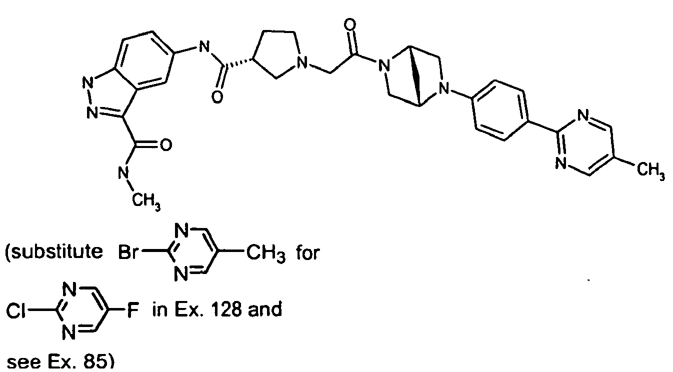 |
| 190 | 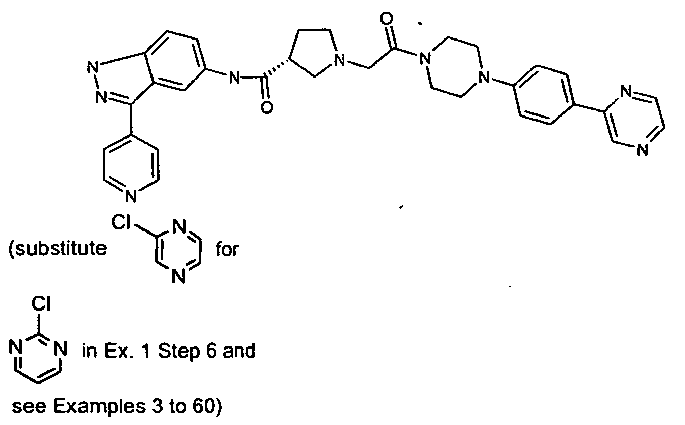 |
| 191 | 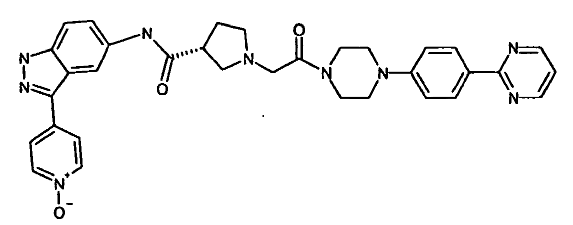 |
| | (see Examples 3 to 60 and Ex. 1) |
| 192 | 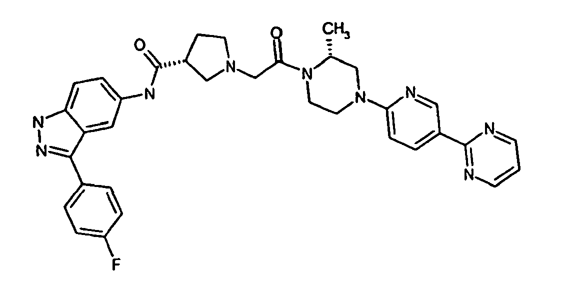 |
| 193 | 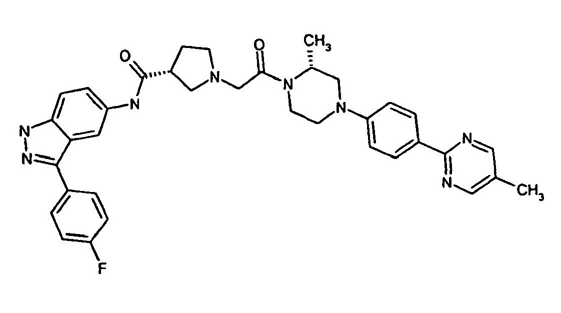 |
| 194 | 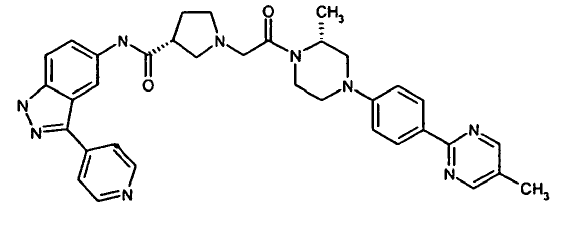 |
| 195 | 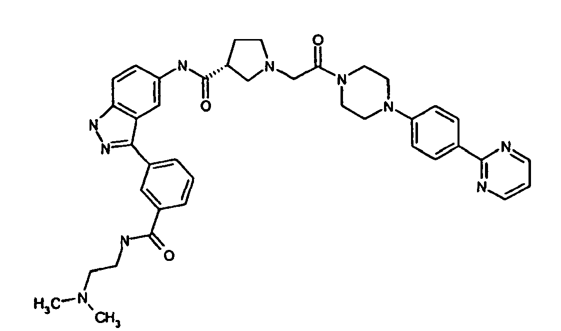 |
| | (see Ex. 1) |
| 196 | 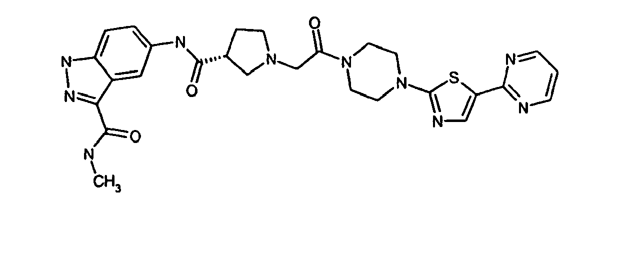 |
| 197 | 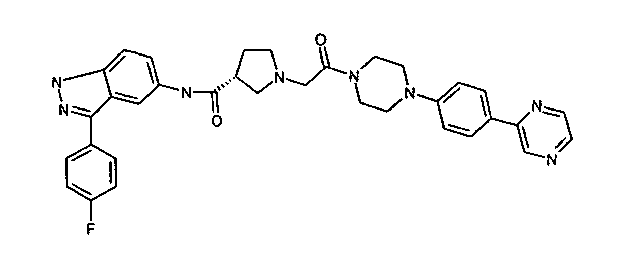 |
| 198 | 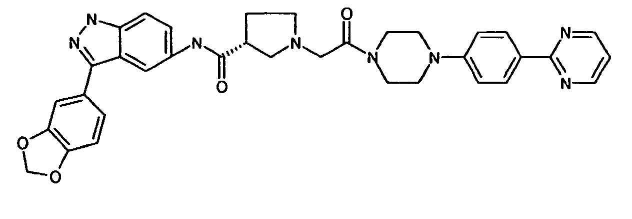 |
| 199 | 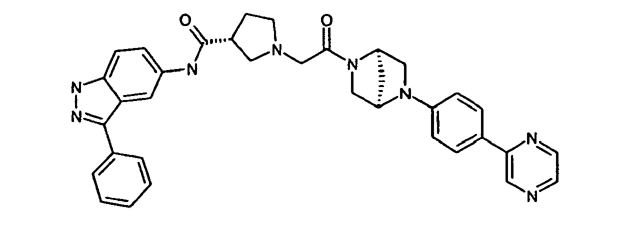 |
| 200 | 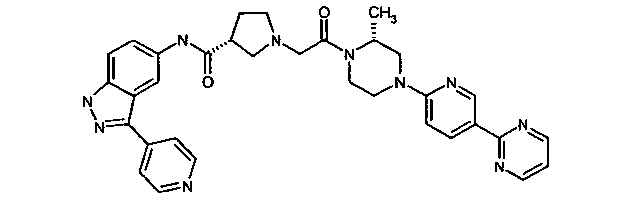 |
| 201 | 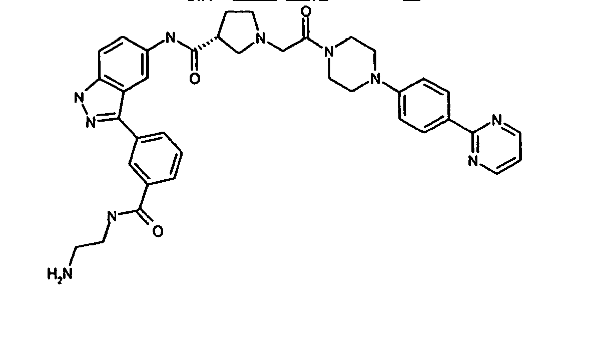 |
| 202 | 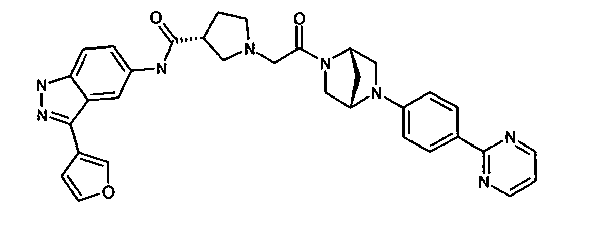 |
| 203 | 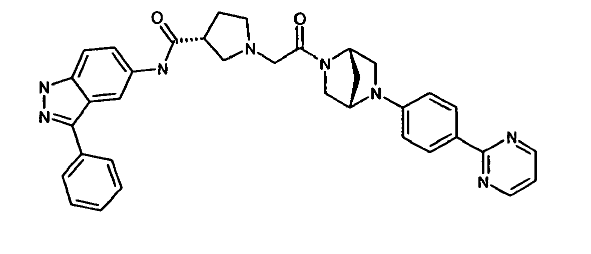 |
| 204 | 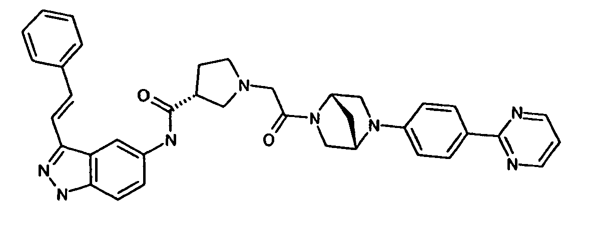 |
| 205 | 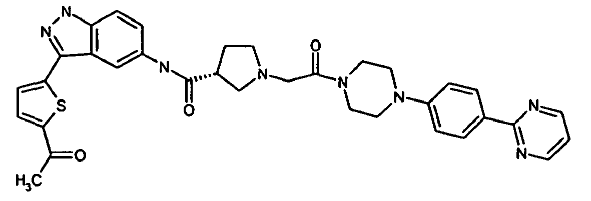 |
| 206 | 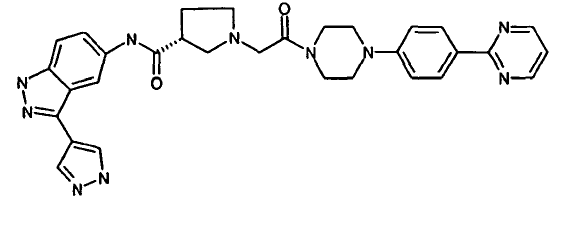 |
| 207 | 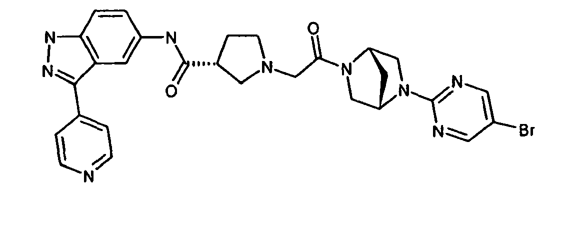 |
| 208 | 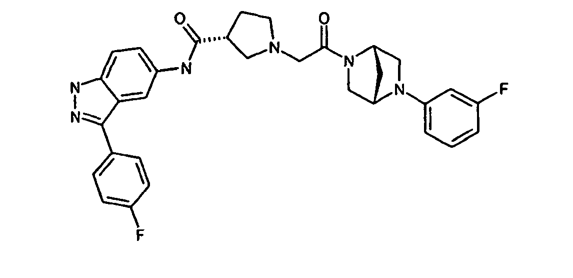 |
| 209 | 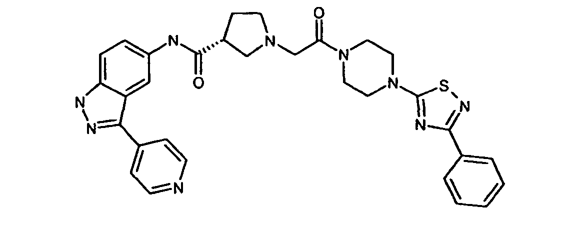 |
| 210 | 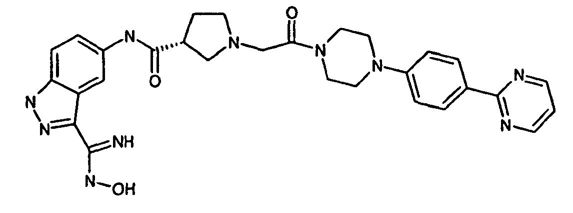 |
| 211 | 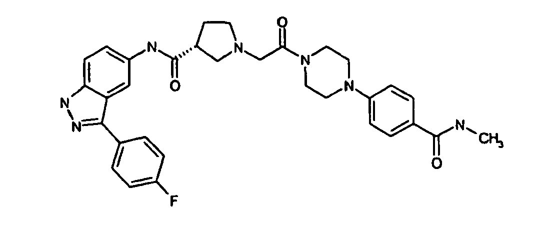 |
| 212 | 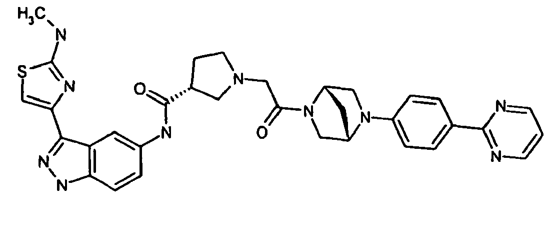 |
| 213 | 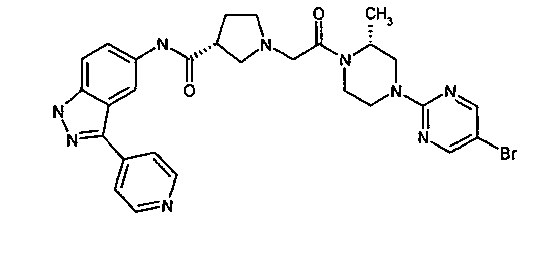 |
| 214 | 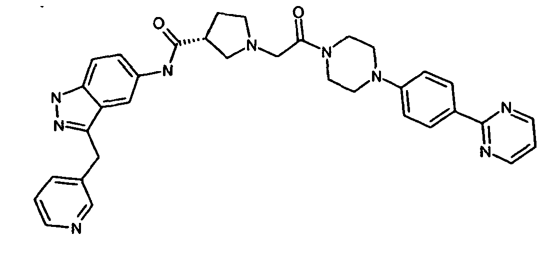 |
| 215 | 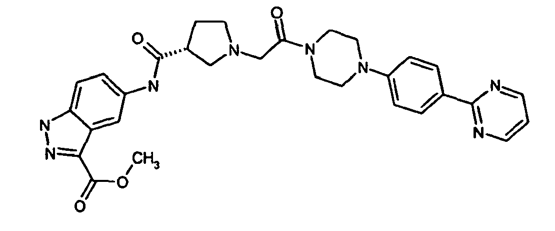 |
| 216 | 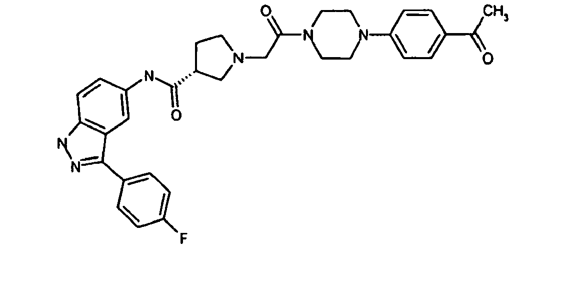 |
| 217 | 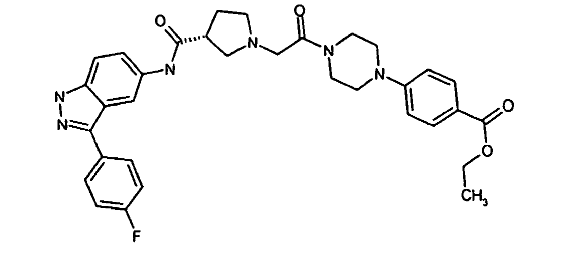 |
| 218 | 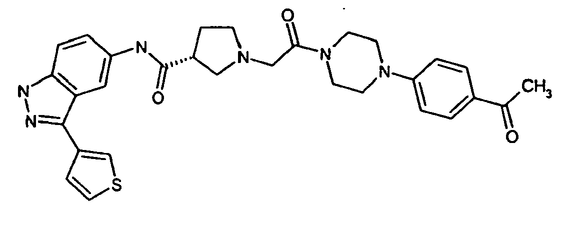 |
| 219 | 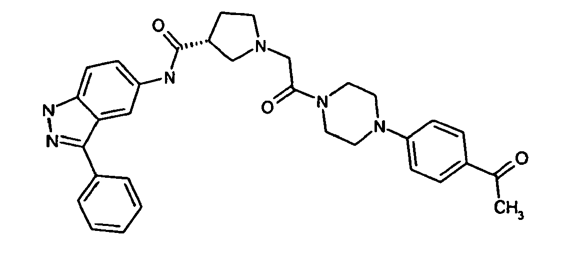 |
| 220 | 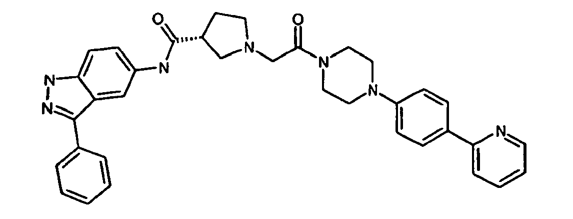 |
| 221 | 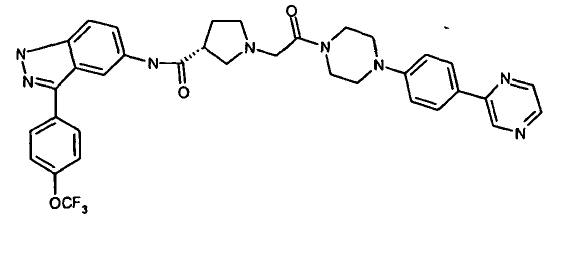 |
| 222 | 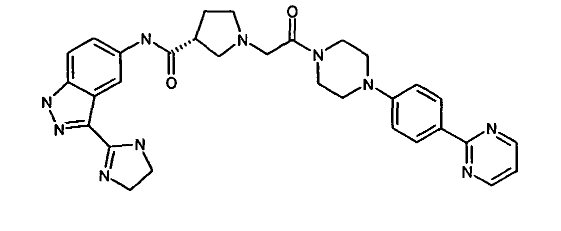 |
| 223 | 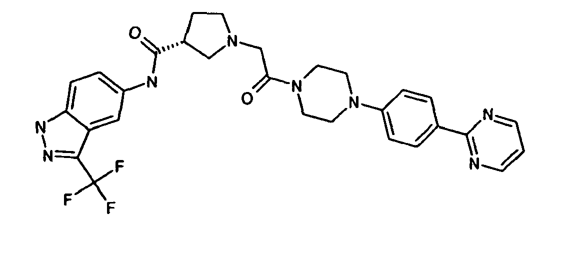 |
| 224 | 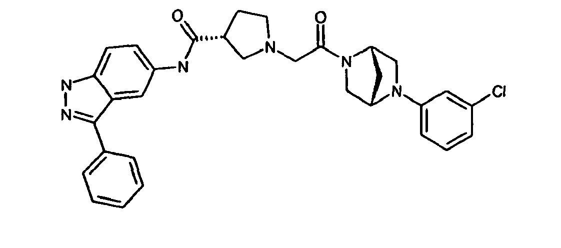 |
| 225 | 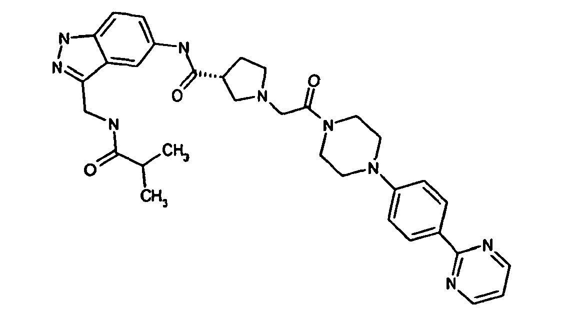 |
| 226 | 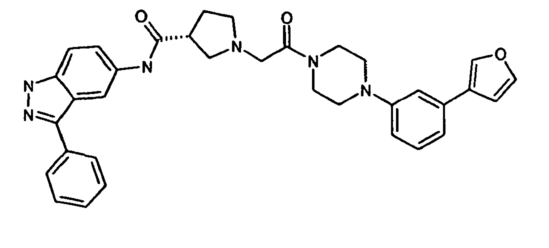 |
| 227 | 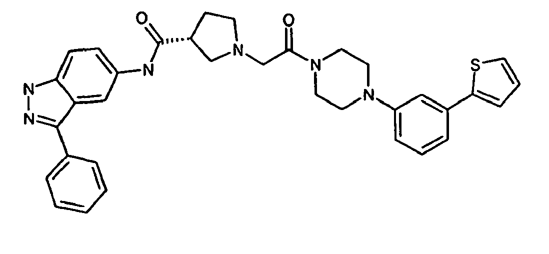 |
| 228 | 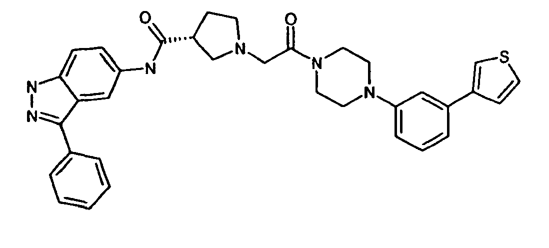 |
| 229 | 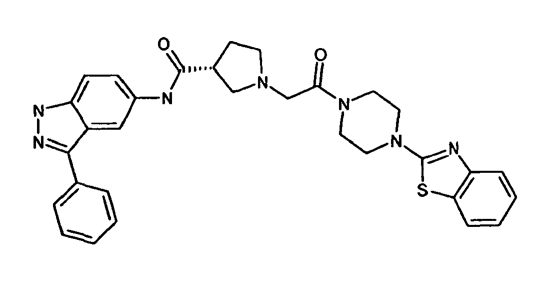 |
| 230 | 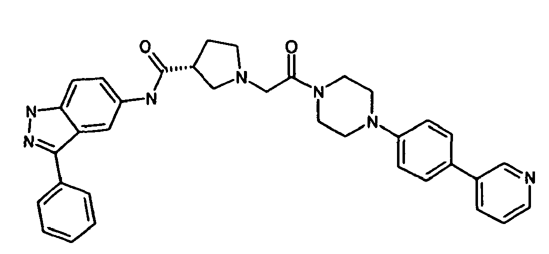 |
| 231 | 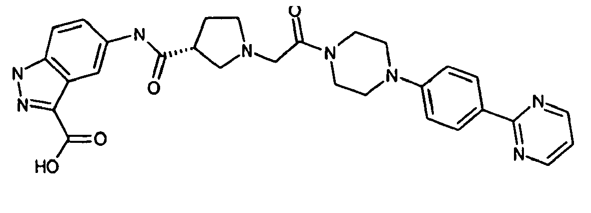 |
| 232 | 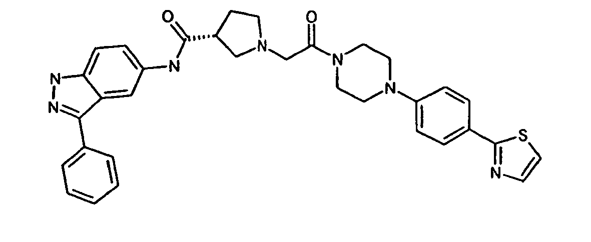 |
| 233 | 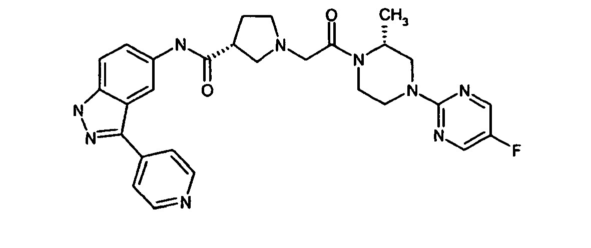 |
| 234 | 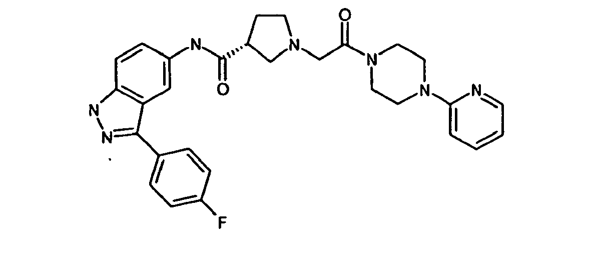 |
| 235 | 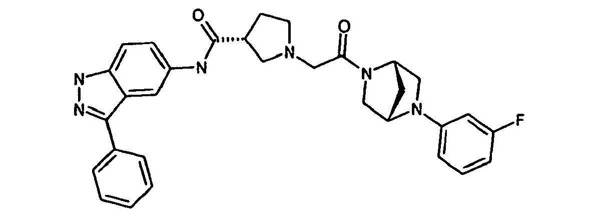 |
| 236 | 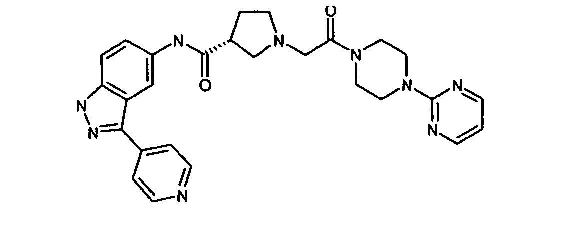 |
| 237 | 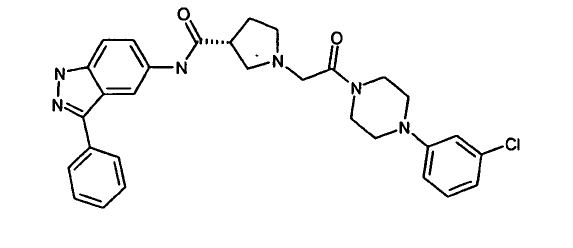 |
| 238 | 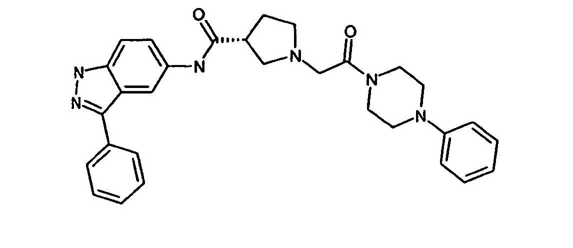 |
| 239 | 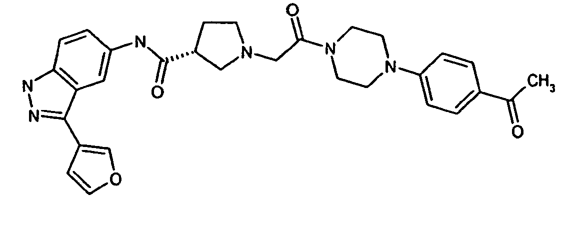 |
| 240 | 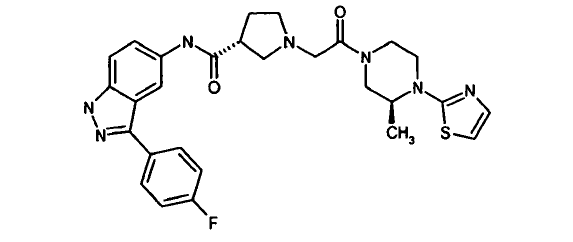 |
| 241 | 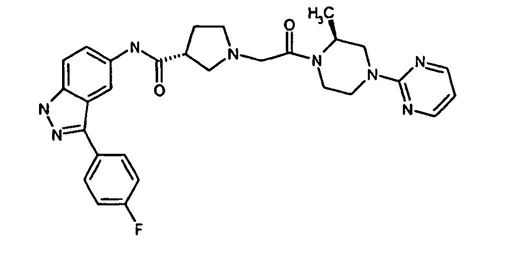 |
| 242 | 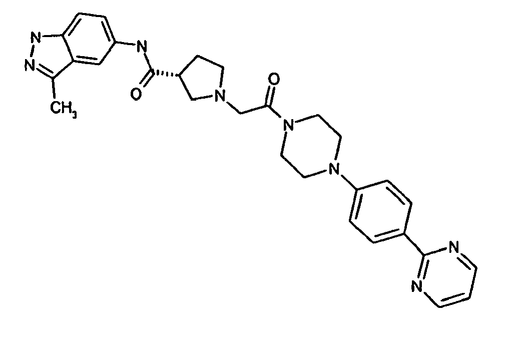 |
| 243 | 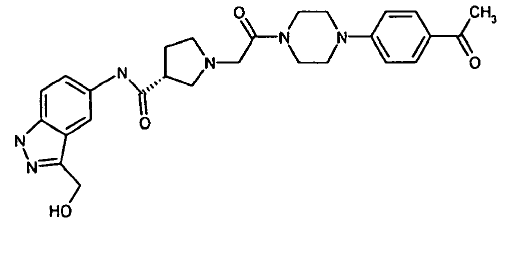 |
| 244 | 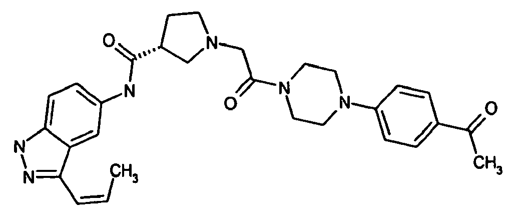 |
| 245 | 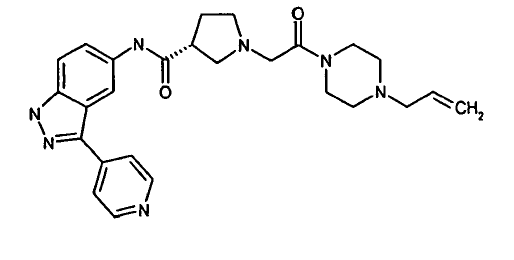 |
| 246 | 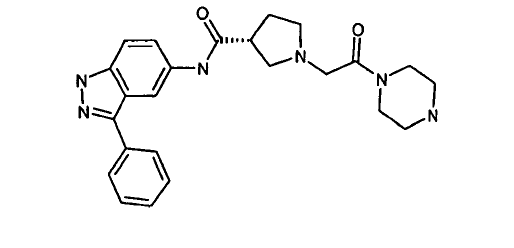 |
| 247 | 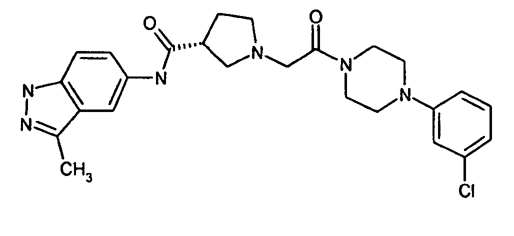 |
| 248 | 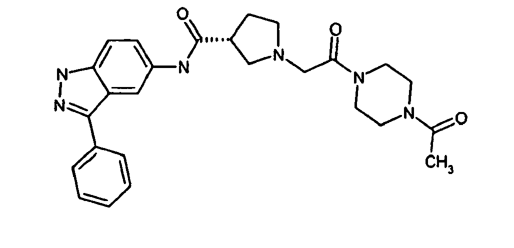 |
| 249 | 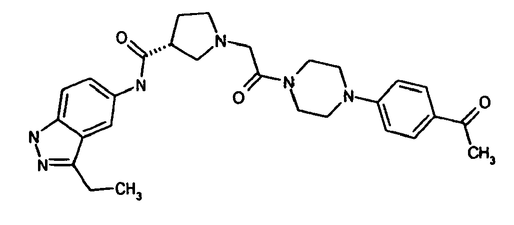 |
| 250 | 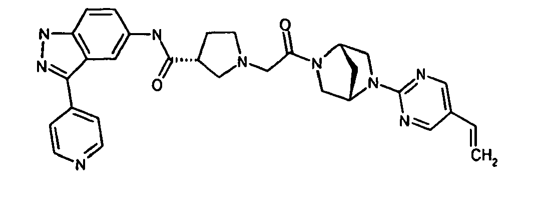 |
| 251 | 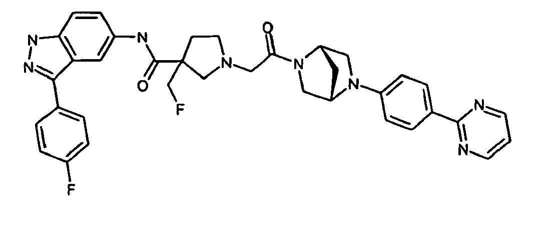 |
| 252 | 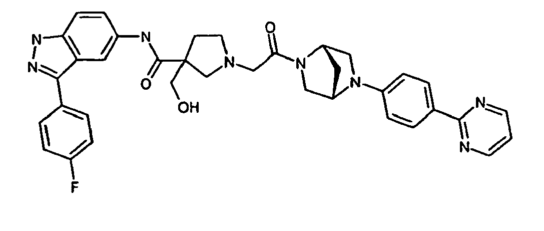 |
| 253 | 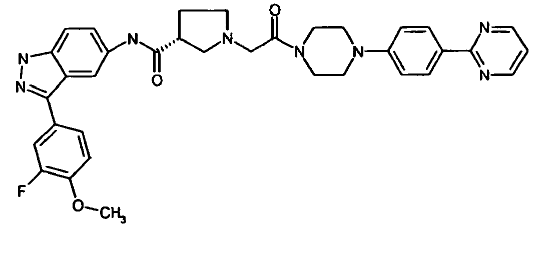 |
| 254 | 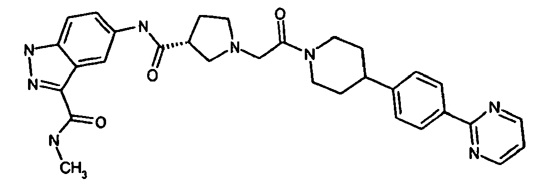 |
| 255 | 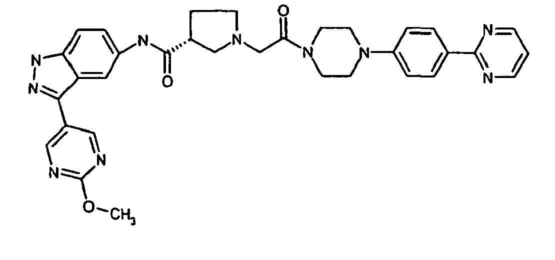 |
| 256 | 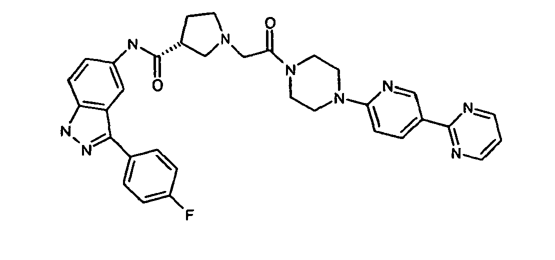 |
| 257 | 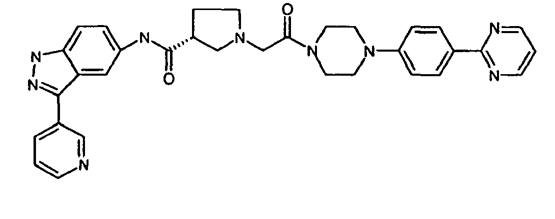 |
| 258 | 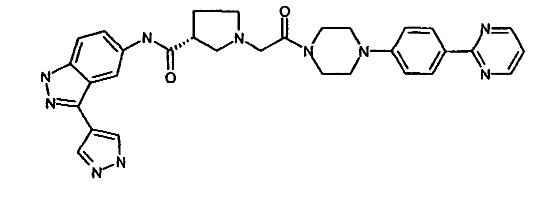 |
| 259 | 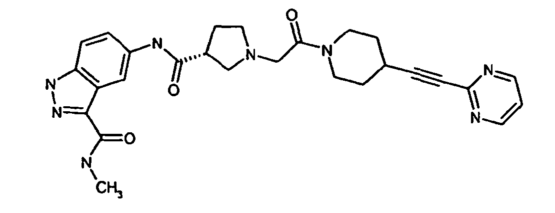 |

### EXAMPLES 260 TO 334

Using the intermediates made from Preparations 1 to 15 described below, and following the procedures described in the reaction Schemes described above, the compounds in Table 16 below were prepared. In Table 16 "Ex" represents "Example".

### Preparation 1

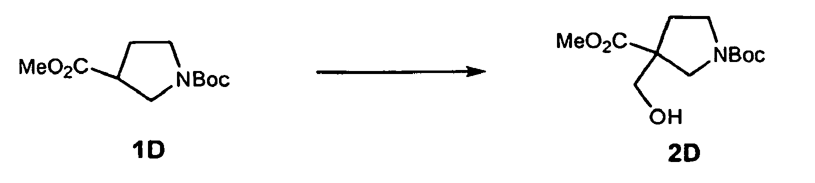

To a solution of LDA (33.5 mmol, 16.7 mL, 2 M in toluene) in 50 mL THF cooled at-78 deg C was added a solution of **1D** (5.9 g, 25.7 mmol) in 50 mL THF drop wise. The crude was stirred at -78 deg C for 45 mins. To the crude was bubbled formaldehyde gas, freshly generated from cracking of para-formaldehde (12 g, 400 mmol). The crude was stirred at -78 deg C for an additional 30 mins. To the crude was added sat NH₄Cl (200 mL). The crude was warmed to rt. The crude was diluted in EtOAc. The organic layer was washed with brine and dried over MgSO₄, filtered, and conc. in vacuum. The crude was purified on Biotage using EtOAc /hexane ( 3 : 7) -> EtOAc / hexane (1 : 1) to give 6.6 g (57%) of the product as a yellow oil.

### Preparation 2

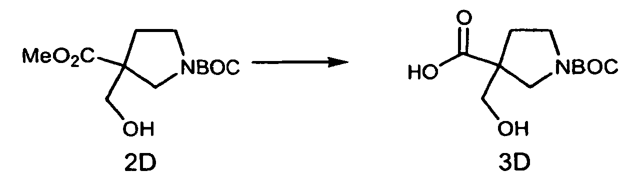

To a solution of ester **2D** (2.5 g, 9.6 mmol) in MeOH (100 mL) was added 1 N NaOH (48 mL, 48.2 mmol) at rt. The crude was stirred at rt overnight. To the crude was added 1 N HCl (47.5 mL, 47.5 mmol). The crude was stirred at rt for 5 mins. PH of crude is made to 5 using PH paper. The crude was conc in vacuum and azeotropped 2X with toluene and dried under high vacuum for use in the HATU coupling with amine **4D** in Preparation 3 below.

### Preparation 3

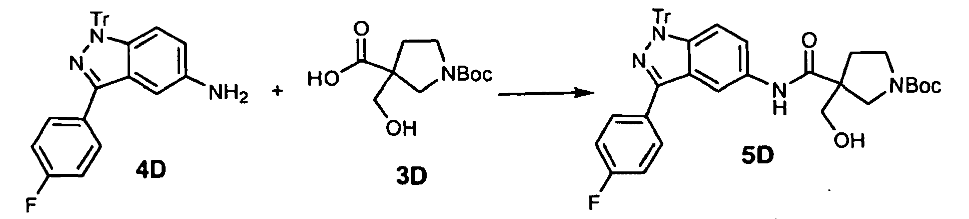

To a suspension of acid **3D** and amine **4D** (5.52 g, 12.5 mmol) in DMF (15 mL) and CH₂Cl₂ (75 mL) was added DIPEA (5 mL, 28.8 mmol) and HATU (5.8 g, 12.5 mmol) at rt. The crude was stirred at rt overnight under a stream of nitrogen. To the crude was added brine and CH₂Cl₂. The aq layer was extracted with EtOAc 2X. The combined organic layer was dried over MgSO₄, filtered, and conc in vacuum. To the crude was added MeOH (200 mL), THF (20 mL) and NaOH (1N, 25 mL, 25 mmol). The crude was stirred at rt for overnight. To the crude was added HCl (1 N, 20 mL). The crude was stirred at rt for 5 mins. The crude was conc in vacuum and dissolved in H₂O and CH₂Cl₂. The aq layer was extracted with EtOAc 2 X. The combined organic layer was dried over MgSO₄, filtered, and conc in vacuum. The crude was purified on Biotage using EtOAc / hexane (2 : 5) -> EtOAc / hexane (3 : 2) to give 2.8 g (42%) of an off white solid 5D. MS (M+H+ = 697.25).

### Preparation 4

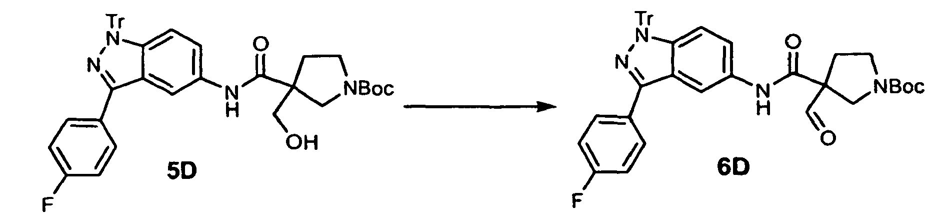

To a solution of alcohol **5D** (3.9 g, 5.6 mmol) in CH₂Cl₂ (65 mL) was added solid NaHCO₃ (1.4 g, 16.8 mmol) and DMP (3.6 g, 8.4 mmol) at rt. The crude was stirred at rt for 1.5 hr. To the crude was added sat. sodium thiosulfate and sat. NaHCO₃ (150 mL : 150 mL) at rt. The crude was vigorously stirred at rt for 30 mins. The aq layer was extracted with CH₂Cl₂ 2X. The combined organic layer was washed with brine, dried over MgSO₄, filtered, and conc in vacuum to give 3.9 g of **6D** (quantitative). MS (M+H+, 695.24)

**Preparation 5**

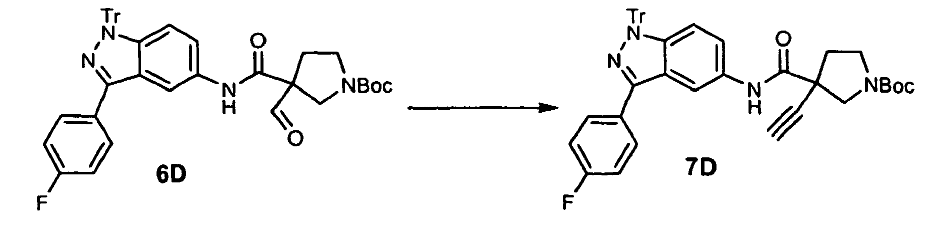

To a solution of crude aldehyde **6D** (571 mg, 0.82 mmol) in MeOH (18 mL) was added K₂CO₃ (1.13 g, 8.2 mmol) and dimethyl-1-diazo-2-oxopropylphosphonate (prepared according to Miller, S.; Liepold, B.; Roth, G. J.; Bestmann, H. J. Synlett, 1996, 521-522) (474 mg, 2.5 mmol) at rt. The crude was stirred at rt for 1.5 hr. To the crude was added sat. NaHCO₃ and EtOAc. The aq layer was extracted with EtOAc. The combined organic layer was washed with brine, dried over MgSO₄, filtered, and conc in vacuum. The crude was purified via prep plate using EtOAc /hexane (3 : 7) to give 408 mg of 7D (72%) as a white solid. LCMS (M+H+, 691.4, rt = 6.15)

### Preparation 6

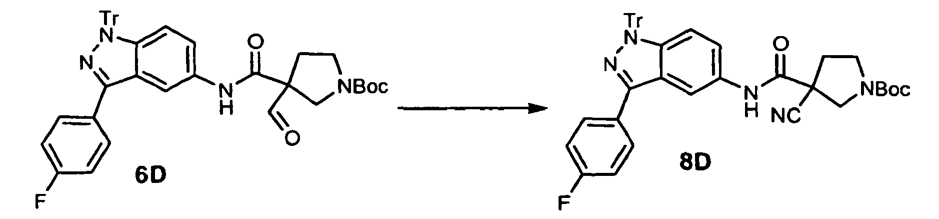

To a solution of crude aldehyde **6D** (315 mg, 0.45 mmol) in THF (2 mL) was added 28% wt NH₄OH (6 mL). The crude was stirred at rt for 2 mins before the addition of iodine (126 mg, 0.50 mmol). The crude was stirred at rt for 45 mins. The crude was quenched with sat. Na₂SO₃ and stirred vigorously for 15 mins. The crude was diluted in CH₂Cl₂. The aq layer was extracted with CH₂Cl₂. The combined organic layer was washed with aq NH₄Cl, brine, dried over MgSO₄, filtered, and conc in vacuum. The crude was purified via prep plate using EtOAc / hexane (2: 5) to give 139 mg of **8D** (45%) as a yellow solid. MS (M+H+, 692.27).

### Preparation 7

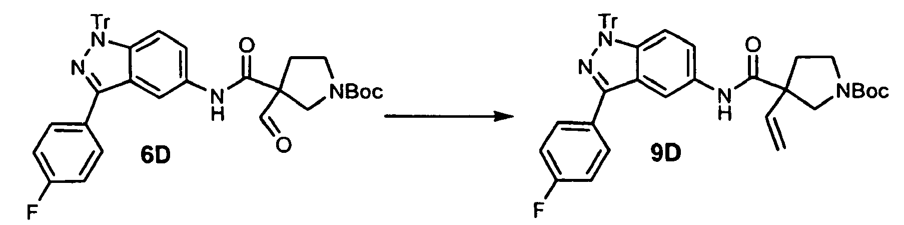

To a solution of crude aldehyde **6D** (600 mg, 0.86 mmol) in THF (5 mL) cooled to -40 deg C was added a solution of Tebbe's reagent (0.5 mL, 0.25 mmol, 0.5 M solution in toluene). The crude was warmed to 0 deg C in 1.5 hrs. The crude was diluted with ether and quenched with 1 N NaOH (1 mL, 1 mmol) at rt. The crude was stirred at rt for 10 mins. The crude was filtered through celite and concentrated in vacuum. The crude was purified via Biotage using EtOAc / hexane to give 165 mg of **9D** (28%) as a yellow solid. MS (M+Na+, 715.24).

### Preparation 8

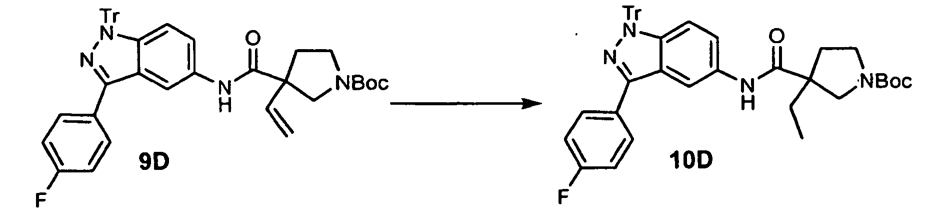

To a solution of olefin **9D** (60 mg, 0.086 mmol) in MeOH (10 mL) was added catalytic amount of 10 % wt palladium on carbon. The mixture was stirred under a hydrogen atmosphere at room temperature overnight and filtered through celite. The filtrate was concentrated to provide **10D** (47 mg). The material was used as such without further purification. MS (M+Na+, 717.28).

### Preparation 9

### Preparation of 3-Prop-2-ynyl-pyrrolidine-1,3-dicarboxylic acid 1-tert-butyl ester 3-methyl ester

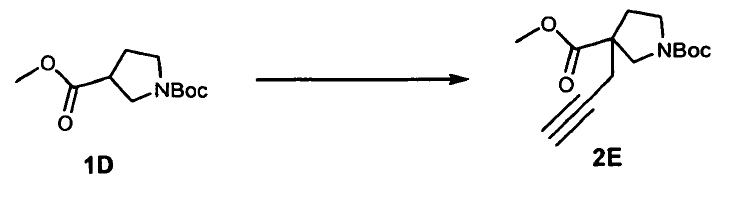

Pyrrolidine-1,3-dicarboxylic acid 1-tert-butyl ester 3-methyl ester 1 (6 mmol) was dissolved in THF (20 mL) and cooled down to -78 °C in a dry ice-acetone bath. LDA (5 mL, 2.0 M, 10 mmol) was then added dropwise. The mixture was stirred at -78°C for 1 hr. Propargyl bromide (1.5 mL) was added in neat. The reaction was allowed to warm to rt naturally and stirred for 24 hrs. It was then quenched with sat. NH4Cl solution, extracted with ethyl acetate 2 X 50 mL. The organic layer was washed with brine, dried (MgSO4) and concentrated. The crude was purified on silica gel column using 4:1 hexanes/ethyl acetate to get the title compound (0.65 g) as an off-white gum. MS (267, M+H)

### Preparation 10

### Preparation of 1-{2-Oxo-2-[4-(4-pyrimidin-2-yl-phenyl)-piperazin-1-yl]-ethyl}-3-(3H-[1,2,3]triazol-4-ylmethyl)-pyrrolidine-3-carboxylic acid [3-(4-fluoro-phenyl)-1H-indazol-5-yl]-amide

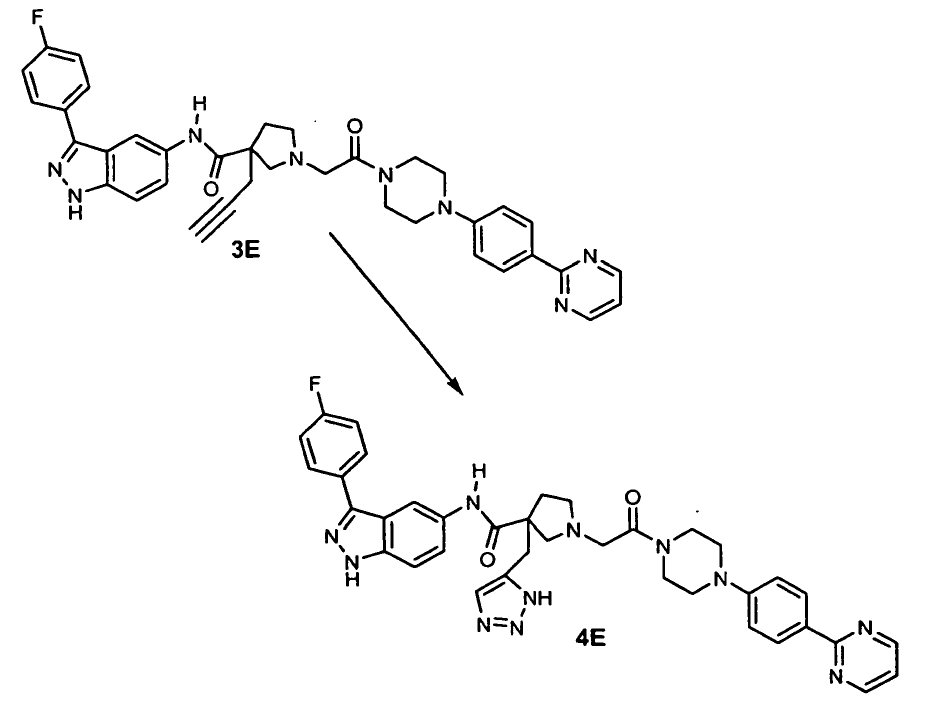

1-{2-Oxo-2-[4-(4-pyrimidin-2-yl-phenyl)-piperazin-1-yl]-ethyl}-3-prop-2-ynyl-pyrrolidine -3-carboxylic acid [3-(4-fluoro-phenyl)-1H-indazol-5-yl]-amide **3E** (0.085 g, 0.13 mmol) and Cul (1.3 mg) was suspended in 9:1 DMF/MeOH (1 mL) under Ar in a sealed vessel. TMSN3 (23 mg, 0.2 mmol) was then added. The mixture was heated at 100 oC overnight. After cooling to rt, the concentrated crude residue was purified by prep-TLC in 10% 2N-NH3/MeOH/CH2Cl2 to yield **4E** as a solid (36 mg). MS (686, M+H)

### Preparation 11

### Preparation of 3-(3-Methoxy-propyl)-pyrrolidine-1,3-dicarboxylic acid 1-tert-butyl ester 3-methyl ester

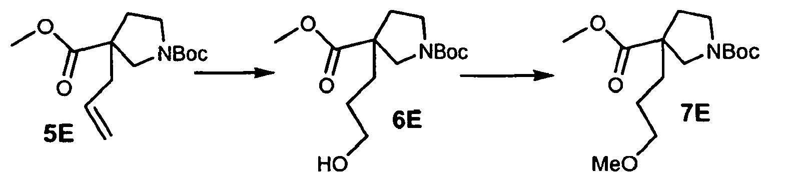

3-Allyl-pyrrolidine-1 ,3-dicarboxylic acid 1-tert-butyl ester 3-methyl ester **5E** (538 mg, 2 mmol) was dissolved in THF (4 mL) and cooled to 0 oC. BH3 (3.8 mL, 1.0 M THF soln) was added in dropwise. After stirring at 0 oC for 15 mins, reaction was raised to rt for another 15 mins. 8 mL 1:1 EtOH/THF, 8 mL PH7 buffer and 8 mL 30 % H2O2 were then added sequentially. The mixture was stirred at rt overnight and then partitioned between ethyl acetate (50 mL) and brine (30 mL). The organic layer was dried and concentrated. The residue was purified on silica eluting with 2:1 to 1:1 hexanes/ethyl acetate to obtain **6E** (326 mg) as a colorless oil. MS (310, M+Na).

3-(3-Hydroxy-propylypyrrolidine-1,3-dicarboxylic acid 1-tert-butyl ester 3-methyl ester **6E** (90 mg) was dissolved in a mixture of THF/DMF (1.5 mL/0.5 mL) at rt. Mel (0.1 mL) was added followed by NaH (40 mg, 40% suspension). After 90 mins, the reaction was quenched with sat. NH4Cl soln and extracted with ethyl acetate. The organic layer was washed with brine, dried and conc. to yield 7E (96 mg) as a colorless oil. MS (324, M+Na).

### Preparation 12

### Preparation of 3-Fluoro-pyrrolidine-1,3-dicarboxylic acid 1-tert-butyl ester 3-methyl ester

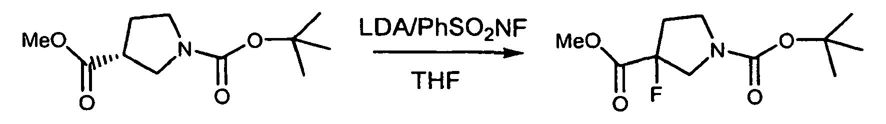

n-Butyl lithium (2.5M/Hexanes, 3.83ml; 9.575 mmol) added dropwise to solution of diisopropylamine (1.36ml; 9.62mmol) at -78°C. Solution was allowed warm to room temperature and stirred for 30 minutes, then cooled to -78°C. A solution of Pyrrolidine-1,3-dicarboxylic acid 1-tert-butyl ester 3-methyl ester (2g, 8.72mmol) in THF (10ml) was added dropwise,warmed to -40°C for 1 hour,then cooled to -70°C.A solution of N-fluorobenzene-sulfonimide (3.02g,9.57mmol) in THF (15ml) was added dropwise,stirred at -78°C for 1hour, then stirred at room temperature overnight. Precipitated solid was filtered,washed with EtOAc (2x150ml). Organic layer was washed with 1 N HCl (30ml), brine (100ml), dried (MgSO₄), filtered and solvent evaporated. The residue was chromatographed on silica gel eluting with 10% v/v EtOAc/hexanes yielding product as colorless oil (1.38g:64% yield). MS (ESMS,MH 249).

### Preparation 13

### Preparation of 3-(1-Acetoxy-ethyl)-1-benzyl-pyrrolidine-3-carboxylic acid methyl ester(3)

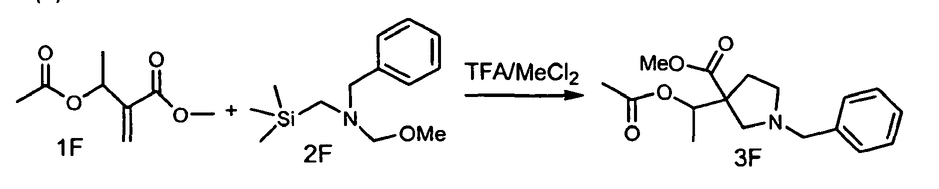

Trifluoroacetic acid (0.5g, 4.38mmol) was added to a solution of methyl-3-acetoxy-2-methylenebutyrate (5g,29.03mmol) (1) and N-Methoxymethyl-N-trimethylsilylmethylbenzylamine (6.89g,29.03mmol) (2) in MeCl₂ (100ml) at 0°C, then stirred overnight at room temperature.The solvent was evaporated,and residue extracted with EtOAc (200ml), NaHCO₃ (30ml and H₂O(100ml). The organic layer was separated,dried (MgSO₄), filtered and solvent evaporated yielding an oil which chromatographed on silica gel eluting with 30% v/v (EtOAc/hexanes) yielding product (3) as colorless oil (7.53g, 85% yield). ESMS MH, 306 (C₁₇H₂₃NO₄)

### Preparation 14

### Preparation of 1-Benzyl-3-(1-hydroxy-ethyl)-pyrrolidine-3-carboxylic acid (4)

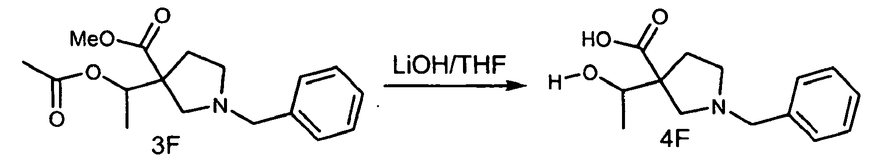

Lithium hydroxide (0.6g,14.29mmol) in H₂O (5ml) was added to a solution of 3-(1-Acetoxy-ethyl)-1-benzyl-pyrrolidine-3-carboxylic acid methyl ester(3F) (2g, 6.55mmol) in THF:MeOH (5:1 v/v) (30ml) at room temperature, then refluxed for 1 hour. The solution was cooled and solvent evaporated yielding the product as the lithium salt of 4F. (2.0g, 100%) ESMS (MH, 250)

### Preparation 15

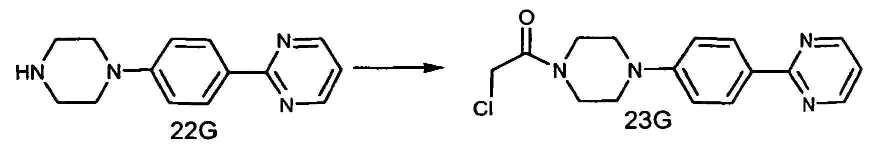

To a solution of **22G** (1.54 g, 6.24 mmol) and TEA (1.16 mL, 8.11 mmol) in CH₂Cl₂ (60 mL) cooled in an icebath was added chloroacetyl chloride (0.6 mL, 7.49 mmol) dropwise. The crude was stirred at 0 deg C for 10 mins. To the crude was added water. The layers were separated, and the aq layer was extracted with CH₂Cl₂. The combined org. layer was washed with brine, dried over MgSO₄, filtered, and conc in vacuum to give 2.06 g of crude 23G. MS (M+H+, 317.4)

**TABLE 16**

| **Ex** | **Compound** |
|---|---|
| 260 | 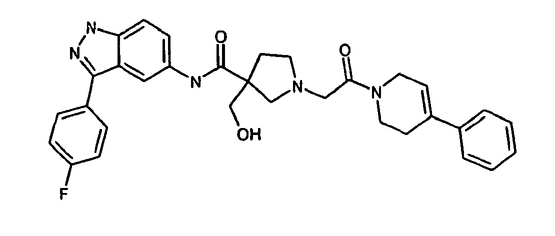 |
| 261 | 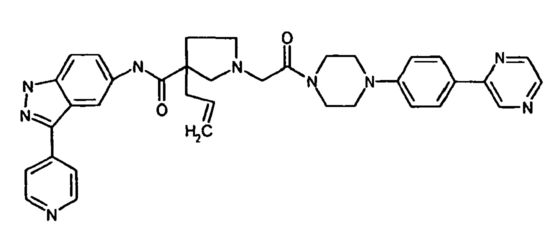 |
| | Mass Spec (ESMS, MH): 628 |
| | Retention Time minutes: 2.27 |
| 262 | 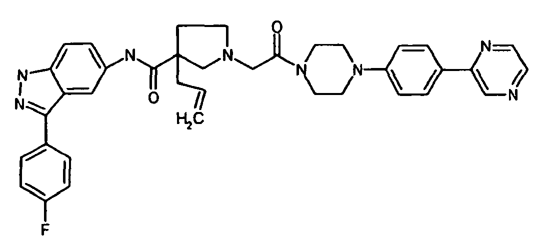 |
| | Mass Spec (ESMS, MH): 645 |
| | Retention Time minutes: 2.96 |
| 263 | 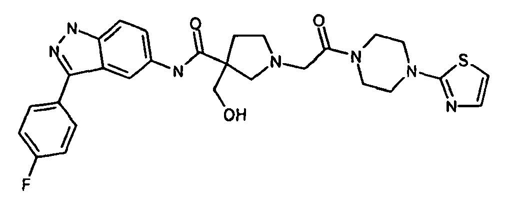 |
| | Mass Spec (ESMS, MH): 564 |
| | Retention Time minutes: 2.68 |
| 264 | 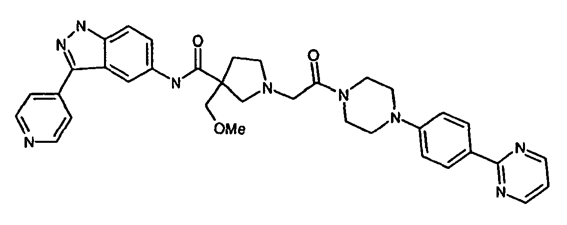 |
| | Mass Spec (ESMS, MH): 632 |
| | Retention Time minutes: 2.67 |
| 265 | 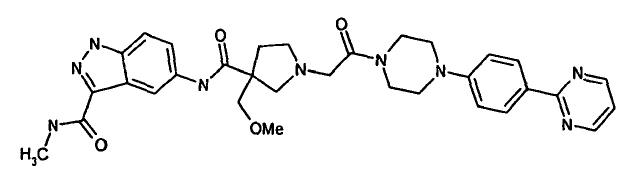 |
| | Mass Spec (ESMS, MH): 612 |
| | Retention Time minutes: 3.01 |
| 266 | 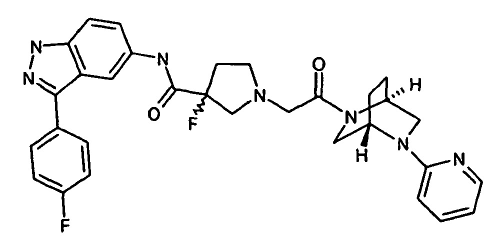 |
| | Mass Spec (ESMS, MH): 572 |
| | Retention Time minutes: 2.5 |
| 267 | 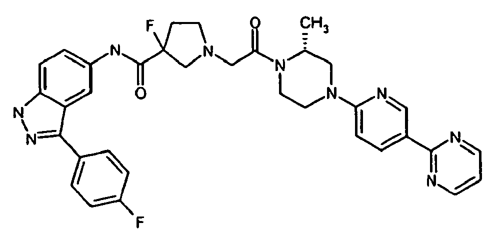 |
| | Mass Spec (ESMS, MH): 638 |
| | Retention Time minutes: 2.68 |
| 268 | 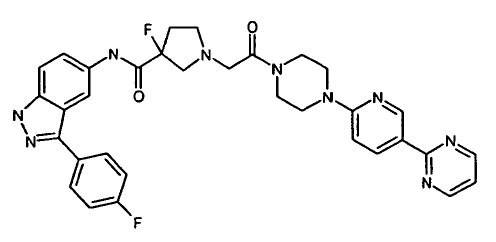 |
| | Mass Spec (ESMS, MH): 624 |
| | Retention Time minutes: 2.61 |
| 269 | 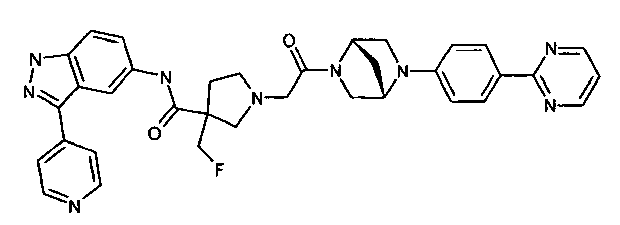 |
| | Mass Spec (ESMS, MH): 632 |
| | Retention Time minutes: 2.51 |
| 270 | 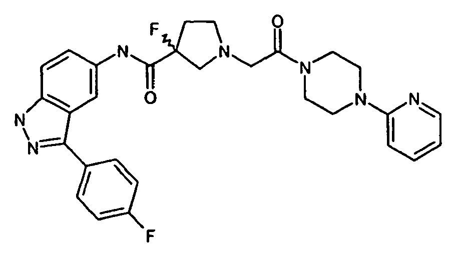 |
| | Mass Spec (ESMS, MH): 546 |
| | Retention Time minutes: 2.70 |
| 271 | 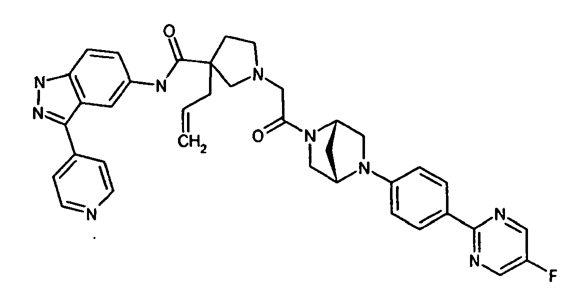 |
| | Mass Spec (ESMS, MH): 658 |
| | Retention Time minutes: 2.88 |
| 272 | 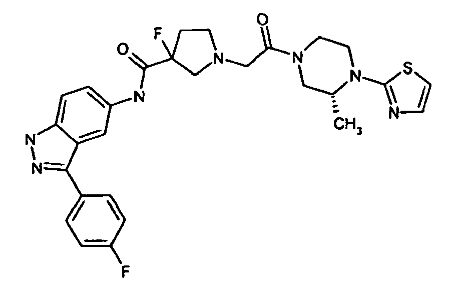 |
| | Mass Spec (ESMS, MH): 566 |
| | Retention Time minutes: 2.66 |
| | 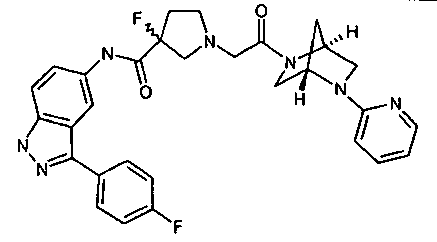 |
| 273 | Mass Spec (ESMS, MH): 558 |
| | Retention Time minutes: 2.49 |
| 274 | 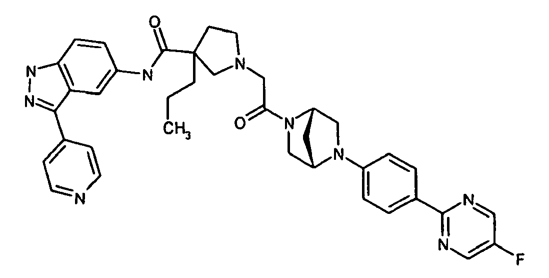 |
| | Mass Spec (ESMS, MH): 660 |
| | Retention Time minutes: 2.54 |
| 275 | 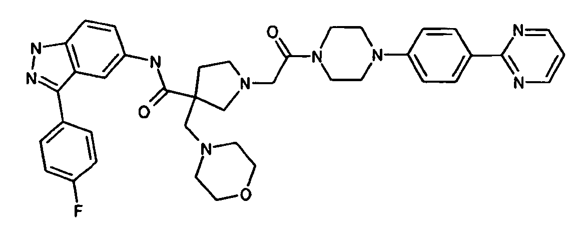 |
| | Mass Spec (ESMS, MH): 704 |
| | Retention Time minutes: 3.72 |
| 276 | 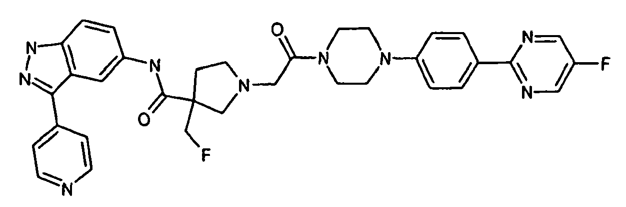 |
| | Mass Spec (ESMS, MH): 638 |
| | Retention Time minutes: 2.82 |
| 277 | 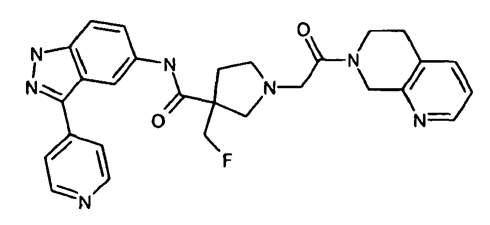 |
| | Mass Spec (ESMS, MH): 514 |
| | Retention Time minutes: 1.64 |
| 278 | 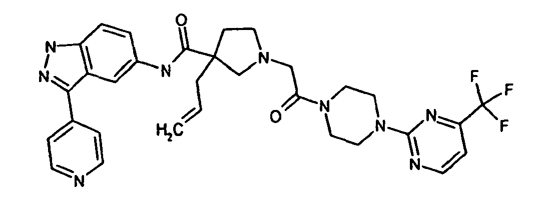 |
| | Mass Spec (ESMS, MH): 620 |
| | Retention Time minutes: 3.25 |
| 279 | 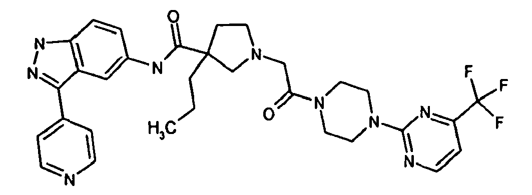 |
| | Mass Spec (ESMS, MH): 622 |
| | Retention Time minutes: 3.27 |
| 280 | 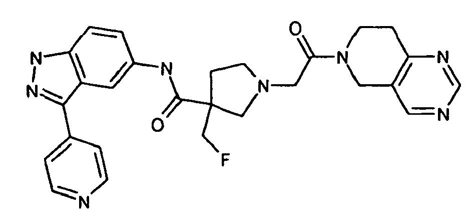 |
| | Mass Spec (ESMS, MH): 515 |
| | Retention Time minutes: 1.75 |
| 281 | 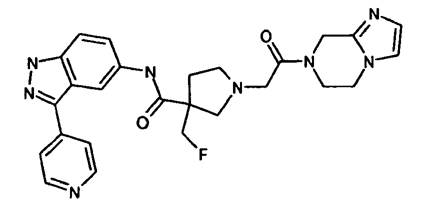 |
| | Mass Spec (ESMS, MH): 503 |
| | Retention Time minutes: 1.44 |
| 282 | 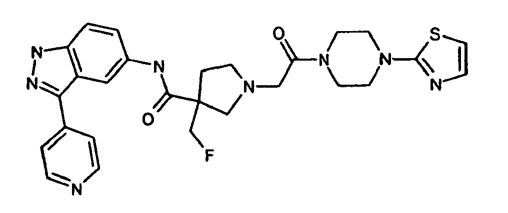 |
| | Mass Spec (ESMS, MH): 549 |
| | Retention Time minutes: 1.73 |
| 283 | 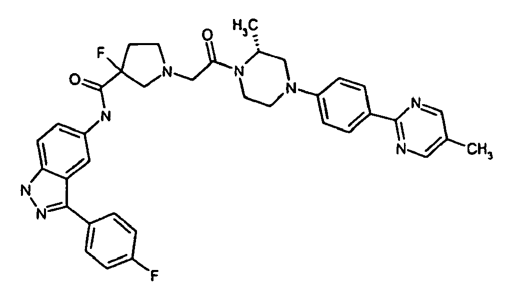 |
| | Mass Spec (ESMS, MH): 651 |
| | Retention Time minutes: 3.09 |
| 284 | 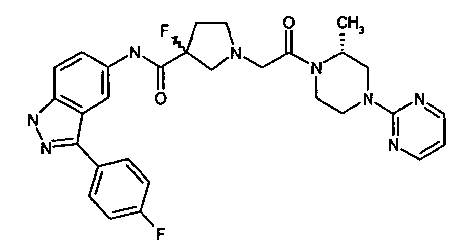 |
| | Mass Spec (ESMS, MH): 561 |
| | Retention Time minutes: 2.62 |
| 285 | 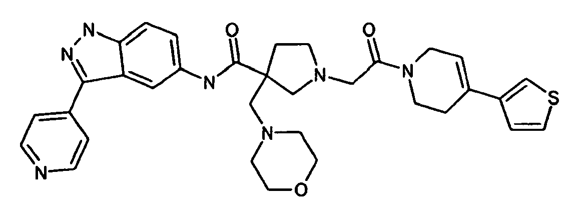 |
| | Mass Spec (ESMS, MH): 612 |
| | Retention Time minutes: 2.84 |
| 286 | 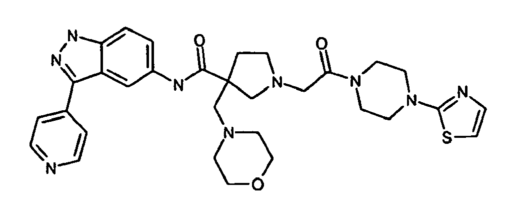 |
| | Mass Spec (ESMS, MH): 616 |
| | Retention Time minutes: 1.89 |
| 287 | 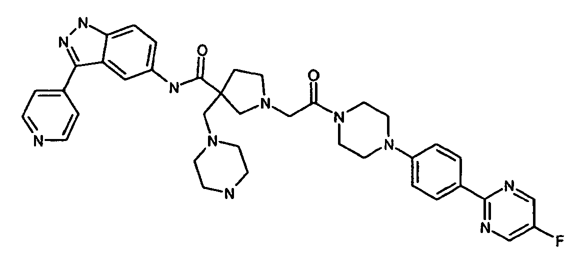 |
| | Mass Spec (ESMS, MH): 704 |
| | Retention Time minutes: 2.59 |
| 288 | 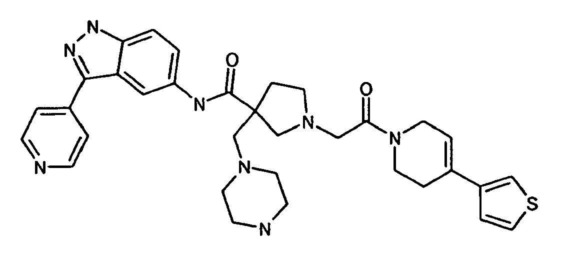 |
| | Mass Spec (ESMS, MH): 611 |
| | Retention Time minutes: 2.30 |
| 289 | 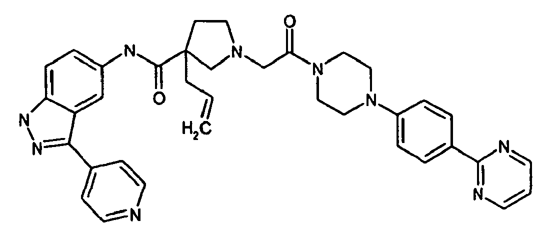 |
| | Mass Spec (ESMS, MH): 628 |
| | Retention Time minutes: 2.25 |
| 290 | 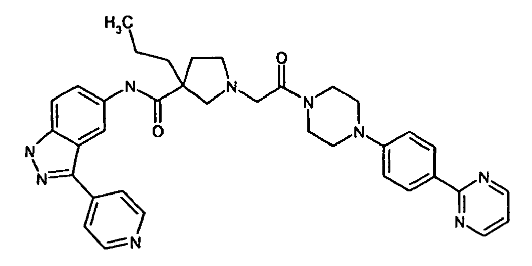 |
| | Mass Spec (ESMS, MH): 630 |
| | Retention Time minutes: 2.54 |
| 291 | 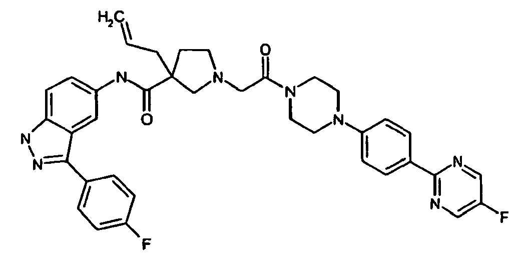 |
| | Mass Spec (ESMS, MH): 663 |
| | Retention Time minutes: 3.22 |
| 292 | 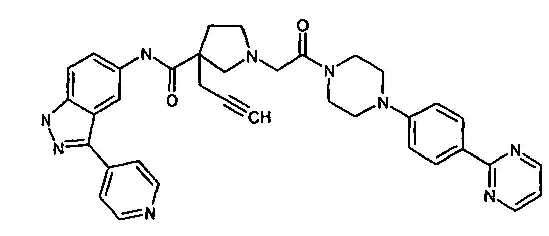 |
| | Mass Spec (ESMS, MH): 626 |
| | Retention Time minutes: 2.31 |
| 293 | 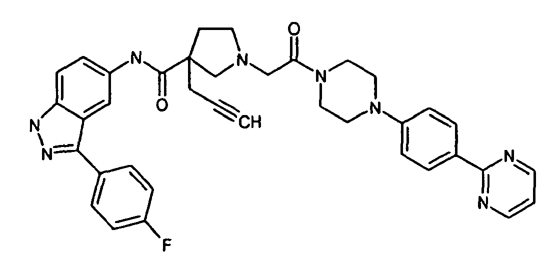 |
| | Mass Spec (ESMS, MH): 643 |
| | Retention Time minutes: 3.22 |
| 294 | 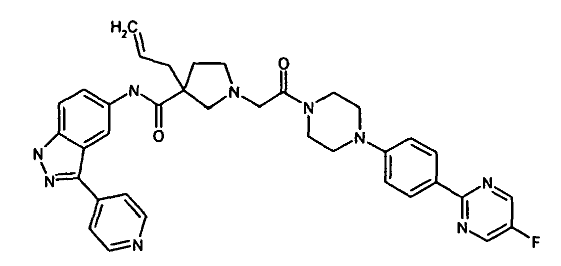 |
| | Mass Spec (ESMS, MH): 646 |
| | Retention Time minutes: 2.26 |
| 295 | 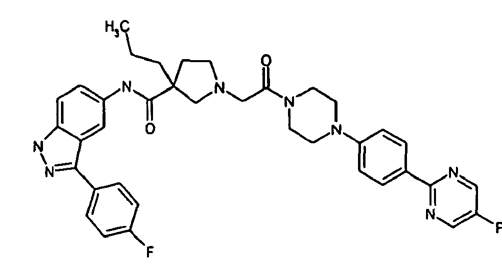 |
| | Mass Spec (ESMS, MH): 665 |
| | Retention Time minutes: 3.24 |
| 296 | 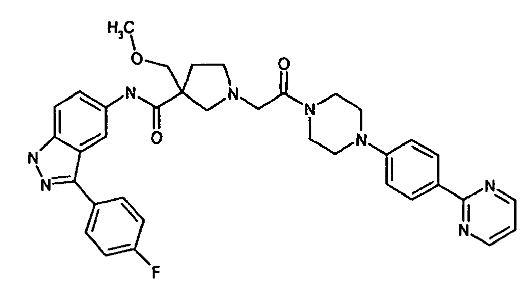 |
| | Mass Spec (ESMS, MH): 649 |
| | Retention Time minutes: 3.15 |
| 297 | 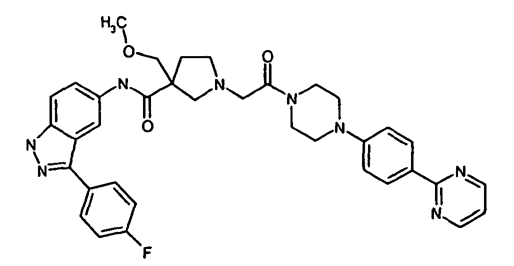 |
| | Mass Spec (ESMS, MH): 649 |
| | Retention Time minutes: 3.16 |
| 298 | 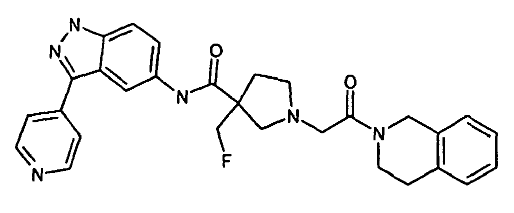 |
| | Mass Spec (ESMS, MH): 513 |
| | Retention Time minutes: 2.66 |
| 299 | 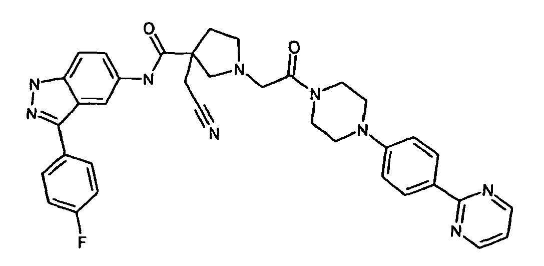 |
| | Mass Spec (ESMS, MH): 644 |
| | Retention Time minutes: 3.14 |
| 300 | 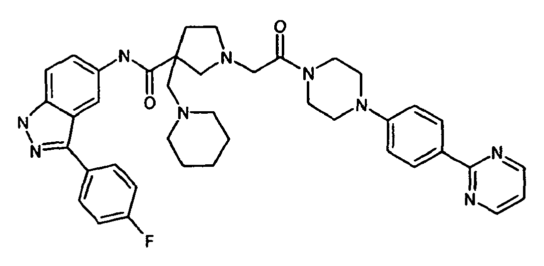 |
| 301 | 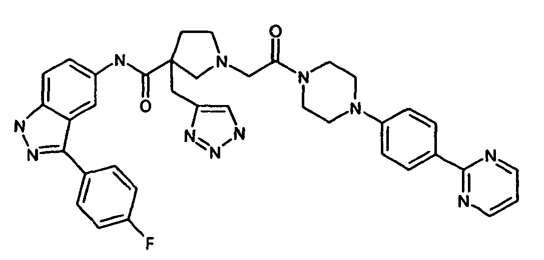 |
| | Mass Spec (ESMS, MH): 686 |
| | Retention Time minutes: 2.79 |
| 302 | 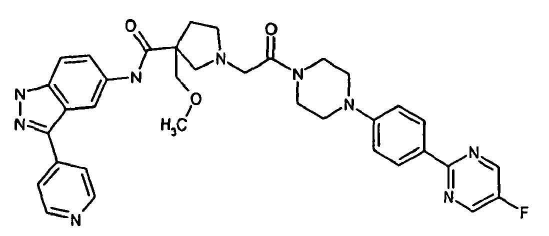 |
| | Mass Spec (ESMS, MH): 650 |
| | Retention Time minutes: 3.07 |
| 303 | 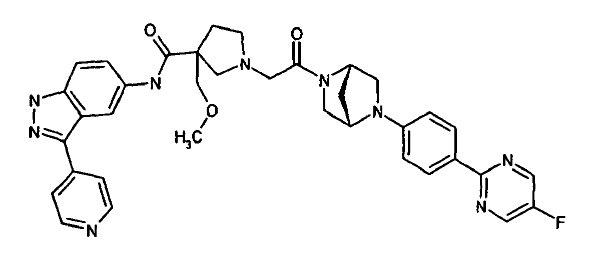 |
| | Mass Spec (ESMS, MH): 662 |
| | Retention Time minutes: 3.05 |
| 304 | 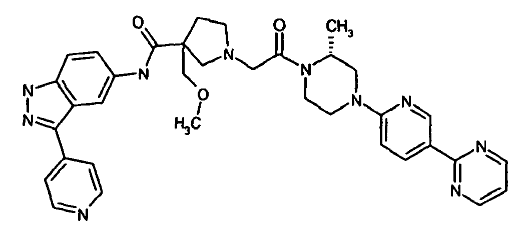 |
| | Mass Spec (ESMS, MH): 647 |
| | Retention Time minutes: 2.23 |
| 305 | 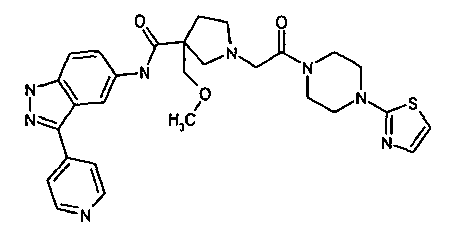 |
| | Mass Spec (ESMS, MH): 561 |
| | Retention Time minutes: 1.86 |
| 306 | 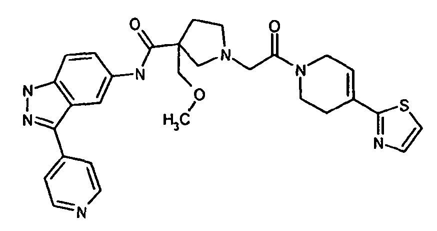 |
| | Mass Spec (ESMS, MH): 5.58 |
| | Retention Time minutes: 2.37 |
| 307 | 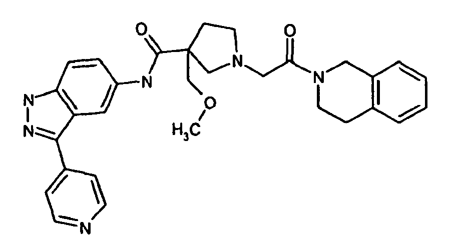 |
| | Mass Spec (ESMS, MH): 525 |
| | Retention Time minutes: 2.68 |
| 308 | 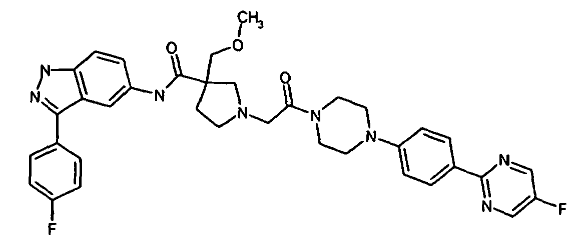 |
| 309 | 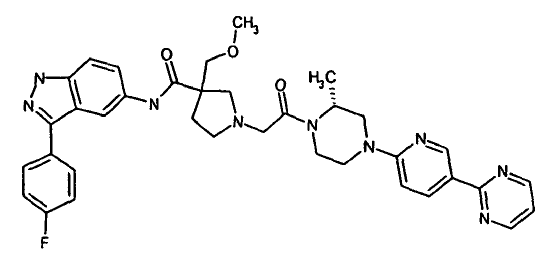 |
| 310 | 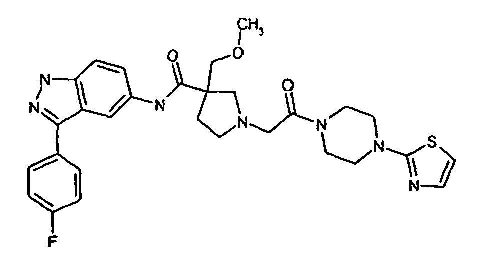 |
| 311 | 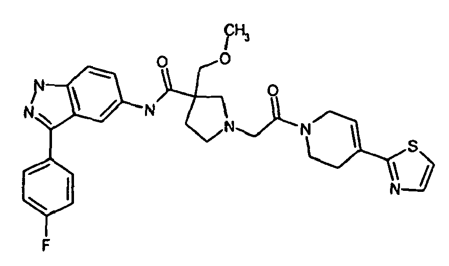 |
| 312 | 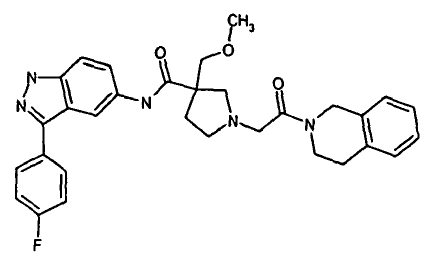 |
| 313 | 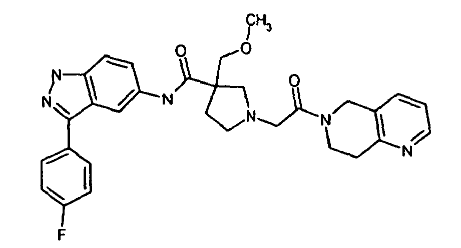 |
| 314 | 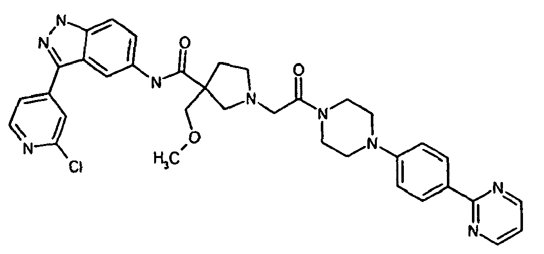 |
| | Mass Spec (ESMS, MH): 666 |
| | Retention Time minutes: 3.70 |
| 315 | 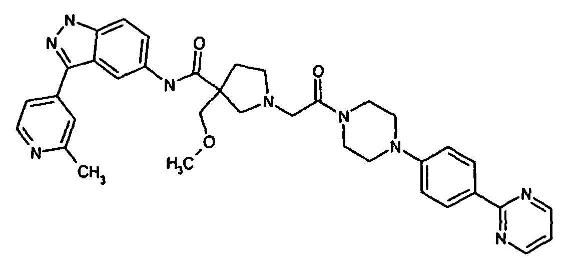 |
| | Mass Spec (ESMS, MH): 646 |
| | Retention Time minutes: 2.70 |
| 316 | 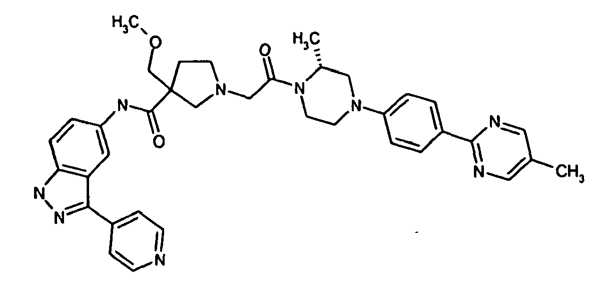 |
| | Mass Spec (ESMS, MH): 660 |
| | Retention Time minutes: 2.48 |
| 317 | 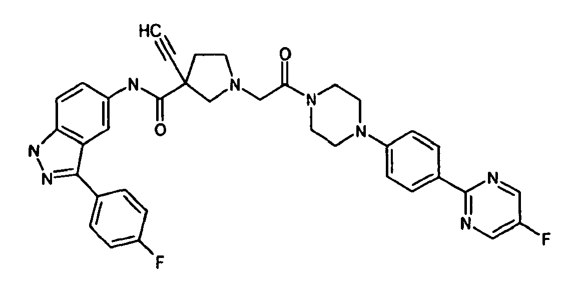 |
| | Mass Spec (ESMS, MH): 647 |
| | Retention Time minutes: 2.98 |
| 318 | 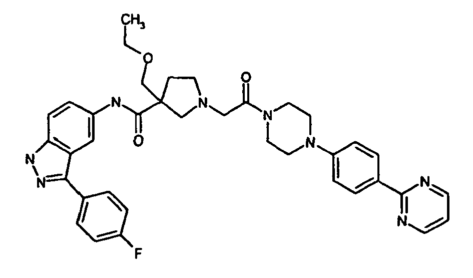 |
| | Mass Spec (ESMS, MH): 663 |
| | Retention Time minutes: 2.79 |
| 319 | 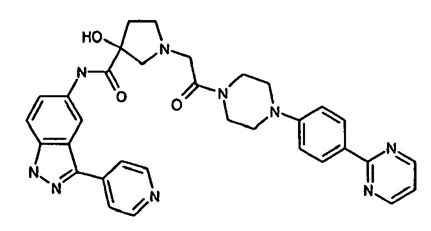 |
| 320 | 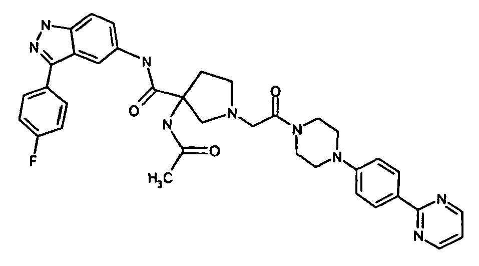 |
| | Mass Spec (ESMS, MH): 662 |
| | Retention Time minutes: 2.73 |
| 321 | 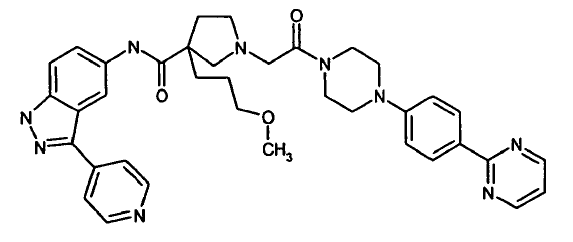 |
| | Mass Spec (ESMS, MH): 660 |
| | Retention Time minutes: 1.93 |
| 322 | 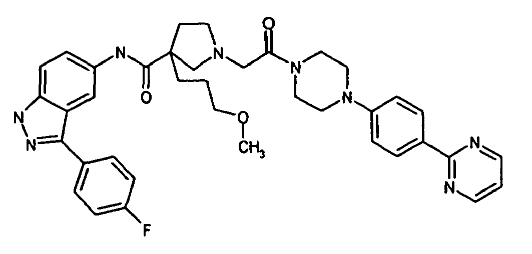 |
| | Mass Spec (ESMS, MH): 677 |
| | Retention Time minutes: 2.70 |
| 323 | 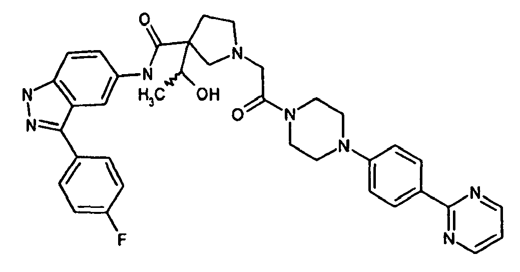 |
| | Mass Spec (ESMS, MH): 649 |
| | Retention Time minutes: 2.72 |
| 324 | 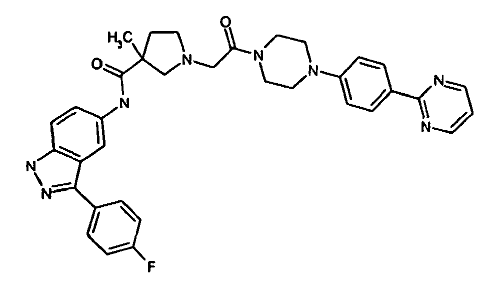 |
| | Mass Spec (ESMS, MH): 619 |
| | Retention Time minutes: 3.61 |
| 325 | 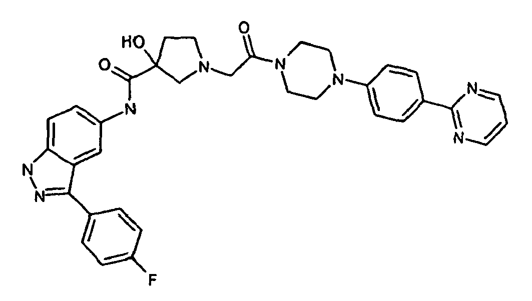 |
| | Mass Spec (ESMS, MH): 621 |
| | Retention Time minutes: 3.69 |
| 326 | 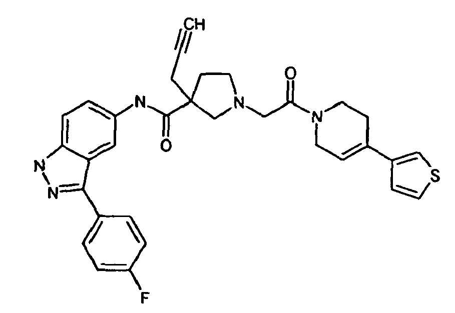 |
| | Mass Spec (ESMS, MH): 568 |
| | Retention Time minutes: 3.52 |
| 327 | 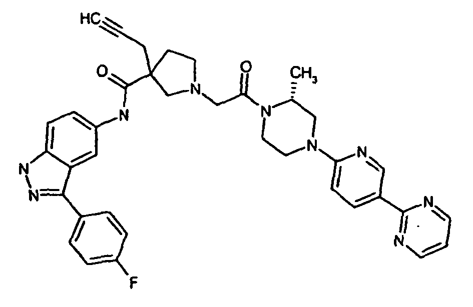 |
| | Mass Spec (ESMS, MH): 658 |
| | Retention Time minutes: 2.68 |
| 328 | 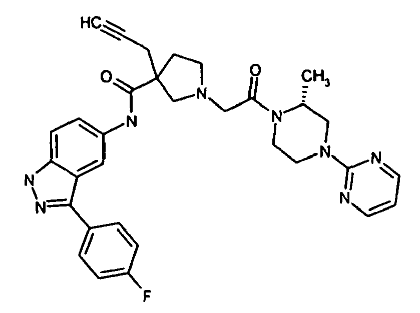 |
| | Mass Spec (ESMS, MH): 581 |
| | Retention Time minutes: 2.95 |
| 329 | 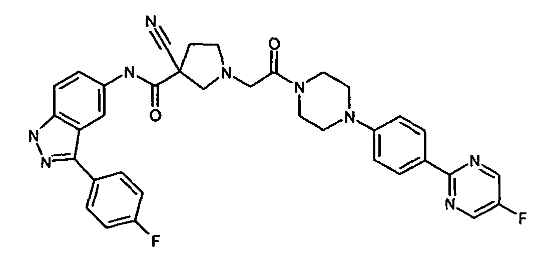 |
| | Mass Spec (ESMS, MH): 648 |
| | Retention Time minutes: 3.18 |
| 330 | 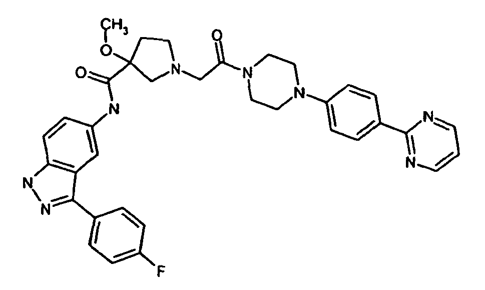 |
| | Mass Spec (ESMS, MH): 635 |
| | Retention Time minutes: 3.82 |
| 331 | 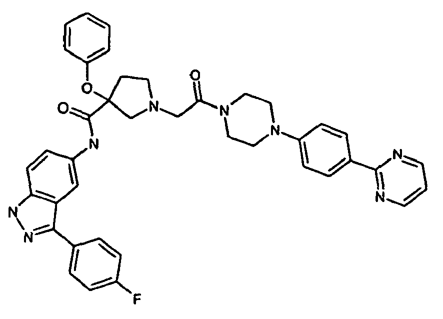 |
| 332 | 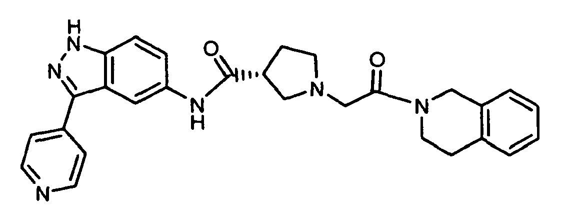 |
| | Mass Spec (ESMS, MH): 481 |
| | Retention Time minutes: 2.55 |
| 333 | 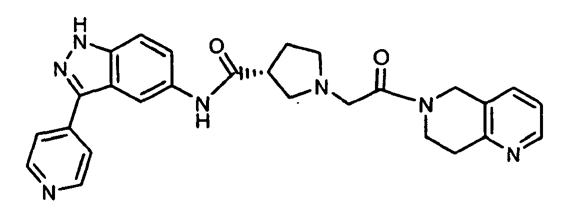 |
| 334 | 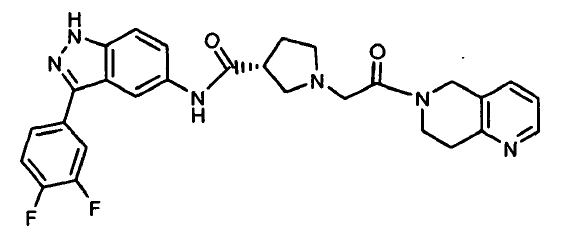 |

### EXAMPLE 335

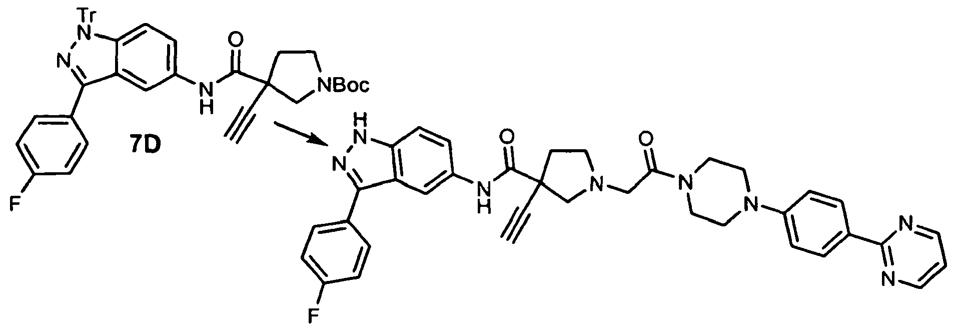

To a solution of **7D** (Preparation 5 in Examples 260-334) (206 mg, 0.30 mmol) in CH₂Cl₂ (3 mL) was added TFA (3 mL). The crude was stirred at rt for 2 hrs. The crude was partially conc in vaccum. The crude was quenched with MeOH (5 mL). The crude was conc in vaccum and azeotroped with toluene 2X and dried under high vac prior to further use. To this crude amine and TEA (0.3 mL, excess) in dioxane (2 mL) was added crude **23G** (Preparation 15 in Examples 260-334) (132 mgs, 0.42 mmol) at rt. The crude was stirred at 80 deg C under nitrogen overnight. The crude was quenched with water and diluted in EtOAc. The layers were separated, and the aq layer was extracted with CH₂Cl₂ 2X. The combined org. layer was washed with brine, dried over Na₂SO₄, filtered, and conc in vaccum. The crude was purified via prep plate using 2M NH₃ in MeOH / CH₂Cl₂ (5 / 95) to give 116 mgs (62%) of an off white solid. LCMS (M+H+, 647.4, retention time = 2.98 min)

### EXAMPLE 336

### Preparation of 3-Methoxymethyl-1-{2-[4-(4-pyrimidin-2-yl-phenyl)-3,6-dihydro-2H-pyridin-1-yl]-acetyl}-pyrrolidine-3-carboxylic acid [3-(2-cyclopropyl-pyridin-4-yl)-1H-indazol-5-yl]-amide

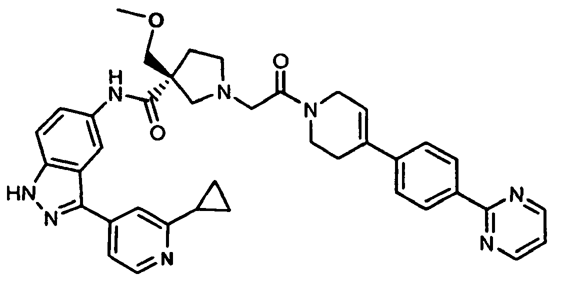

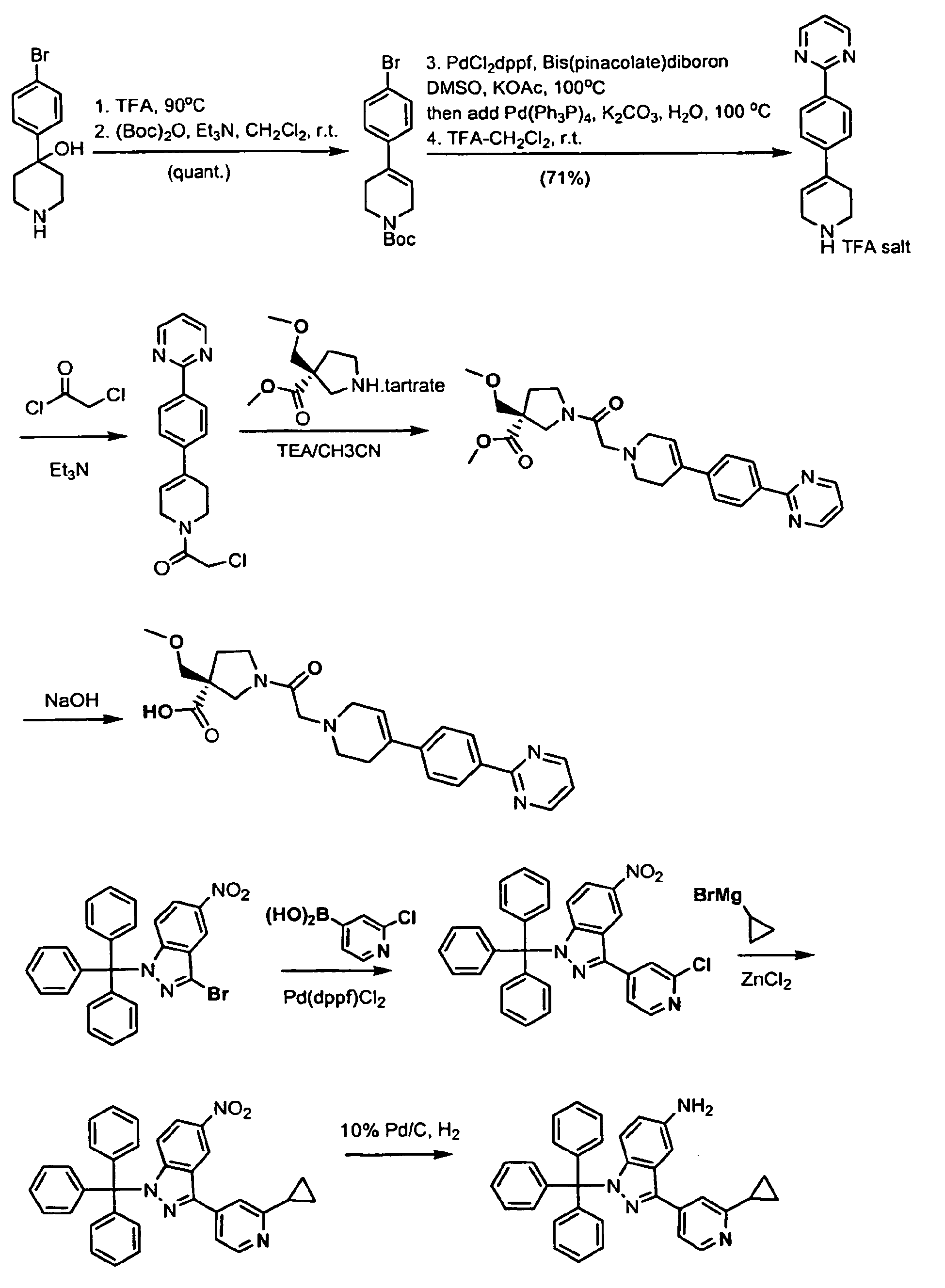

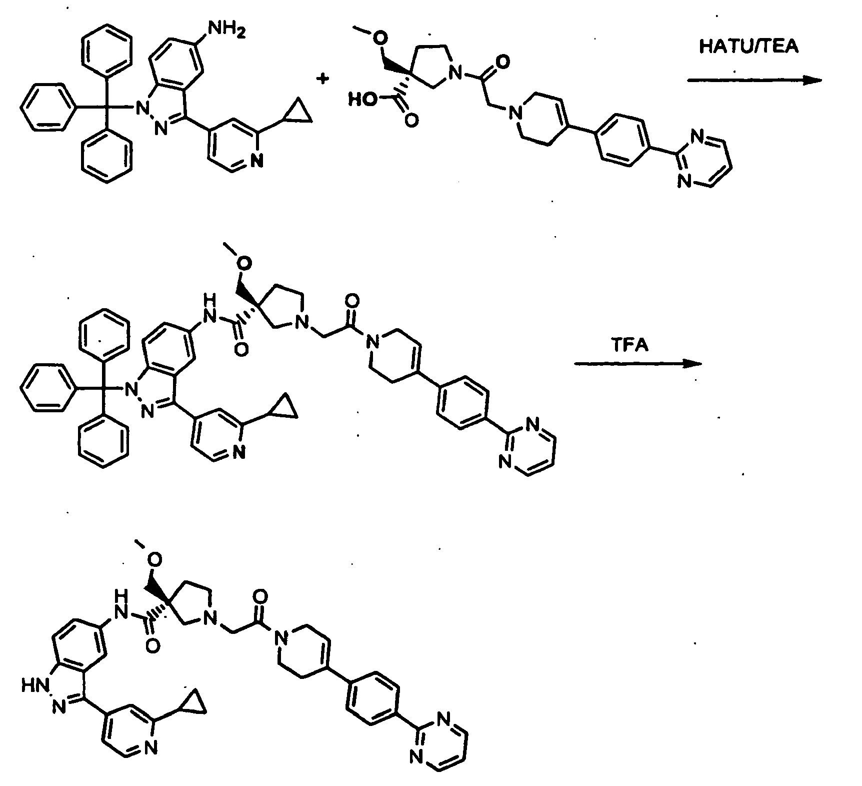

### Procedure for the Preparation of 2-[4-(1,2,3,6-Tetrahydro-pyridin-4-yl)-phenyl]-pyrimidine trifluoroacetic acid salt

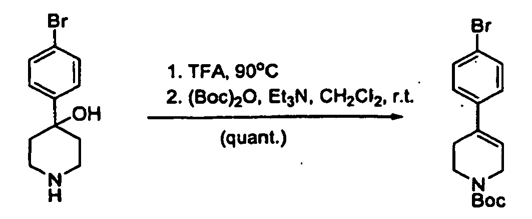

**Step 1**: 4-(4-Bromophenyl)-4-piperidinol (68g, 0.27 mol) was added in small portions to a solution of trifluoroacetic acid (205 ml) at r.t. and the mixture was heated at 90 °C for 2 hr. Solvents were then removed in vacuum to give 4-(4-bromophenyl)-1,2,3,6-tetrahydropyridine as pale yellow oil. The yellow oil was used in the next step without further purification.

**Step 2:** 4-(4-Bromophenyl)-1,2,3,6-tetrahydropyridine (crude from step 1) was stirred in dichloromethane (500 ml) at r.t. Triethylamine (148ml, 1.06 mol) followed by (Boc)₂O (87g, 0.40 mol) were added. The suspension slowly dissolved and the yellow solution was stirred at r.t. for 2 hr. The mixture was washed with water (X2), dried (MgSO₄) and chromatograph through a short pad of silica. The fractions with the product 4-(4-Bromophenyl)-3,6-dihydro-2H-pyridine-1-carboxylic acid tert-butyl ester were combined and solvents were removed in vacuum to give pale yellow oil which solidified on standing at r.t. to become white solid (91g, quant.)

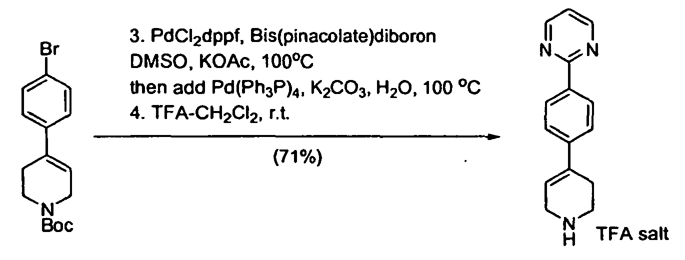

**Step 3:** 4-(4-Bromophenyl)-3,6-dihydro-2H-pyridine-1-carboxylic acid tert-butyl ester (19.5g, 0.058mol), bis(pinacolate)diboron (22.0g, 0.086 mol), PdCl₂(dppf).CH₂Cl₂ (4.74g, 0.0058mol), potassium acetate (1 7.0g, 0.17mol) were weighted into a 1 L 2-necked round bottomed flask equipped with a reflux condenser. Methyl sulfoxide (400ml) was added and the mixture was purged with nitrogen for 20 min before it was heated at 100 °C for 2 hr under nitrogen. The mixture was cooled to r.t. Potassium carbonate (40g, 0.29mol), 2-bromopyrimidine (11.0g, 0.070mol) and water (200ml) were added. The mixture was again purged with nitrogen for 20 min. Palladium tetrakistriphenylphosphine (2.4g, 0.0029mol) was added and the final mixture was stirred at 100 °C for a further 2 hr. After being cooled to r.t., ethyl acetate and water were added. The mixture was filtered through a pad of Celite. Layers were separated and the organic layer was washed with water (X2). The combined aqueous layers were extracted with ethyl acetate (X1). The combined organic layers were stirred with enough charcoal to give a yellow solution. The mixture was filtered through a pad of Celite and the solvents in the filtrate were removed in vacuum to give 4-(4-pyrimidin-2-yl-phenyl)-3,6-dihydro-2H-pyridine-1-carboxylic acid tert-butyl ester as dark brown oil.

**Step 4:** 4-(4-pyrimidin-2-yl-phenyl)-3,6-dihydro-2H-pyridine-1-carboxylic acid tert-butyl ester (crude from step 3) was dissolved in dichloromethane (200ml) and trifluoroacetic acid (22ml, 0.29mol) was added at r.t. The mixture was stirred at r.t. for 5 hr and solvents were removed in vacuum. Diethyl ether was added and off-white solid was formed. The solid was filtered and washed with diethyl ether to give a salt (14.4g, 71%).

### Preparation of 2-Chloro-1-[4-(4-pyrimidin-2-yl-phenyl)-3,6-dihydro-2H-pyridin-1-yl]-ethanone

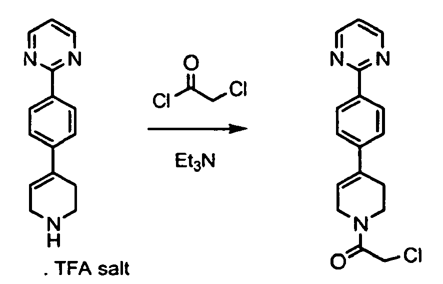

2-[4-(1,2,3,6- Tetrahydro-pyridin-4-yl)-phenyl]-pyrimidine trifluoroacetate (2.3 g, 9.7 mmol) was dissolved in 75 ml of dichloromethane and 4.1 ml of triethylamine added at 0 C. Chloroacetylchloride (0.92 ml, 11.7 mmol) was added and the reaction mixture stirred for 30 min. The reaction mixture was washed with a solution of saturated sodium bicarbonate (80 ml), the organic layer separated, dried over magnesium sulfate and evaporated to obtain 2.41 g of crystalline product.

### Chiral salt resolution of 3-methoxy-pyrrolidine-3-carboxylic acid methyl ester

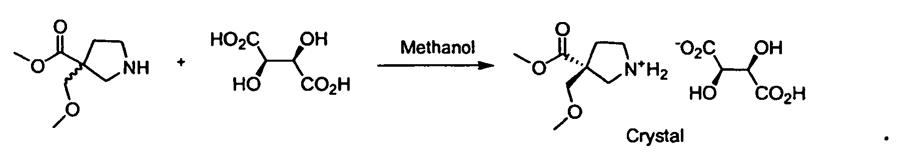

To a solution of 3-Methoxymethyl-pyrrolidine-3-carboxylic acid methyl ester (6.1 g, 35.8 mmol) in methanol (50 mL) was added L-tartaric acid (5.11 g, 34 mmol) and the formed mixture was sonicated to ensure the dissolve of tartaric acid. The solution was left standing at room temperature and crystals started forming after less than 10 minutes. After standing at RT for over night, the formed crystal was filtered, washed with cold methanol and was re-crystallized using 25 mL of methanol to give 2.2 gram material.

### Preparation of 3-Methoxymethyl-1-{2-[4-(4-pyrimidin-2-yl-phenyl)-3,6-dihydro-2H-pyridin-1-yl]-acetyl}-pyrrolidine-3-carboxylic acid methyl ester

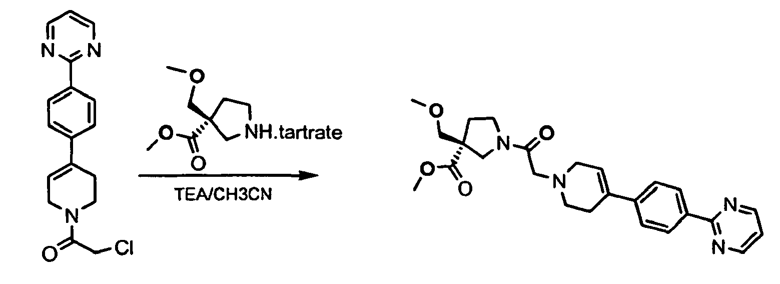

3-Methoxymethyl-pyrrolidine-3-carboxylic acid methyl ester tartaric acid salt ( 1.54 gm, 4.85 mmol) in 25 ml of acetonitrile was added 2-Chloro-1-[4-(4-pyrimidin-2-yl-phenyl)-3,6-dihydro-2H-pyridin-1-yl]-ethanone (2.2 gm, 5.82 mmol), triethylamine (3.4 ml, 24.3 mmol) and the reaction mixture stirred at 80 C for 18 hrs. The reaction mixture was evaporated, and extracted into dichloromethane from water. The dichloromethane extracts were dried over Na₂SO₄, filtered and evaporated. The mixture was chromatographed on silica gel using 2-3 % MeOH/dichlormethane to obtain 1.5 gm of title compound.

### Preparation of 3-(2-Chloro-pyridin-4-yl)-5-nitro-1-trityl-1H-indazole

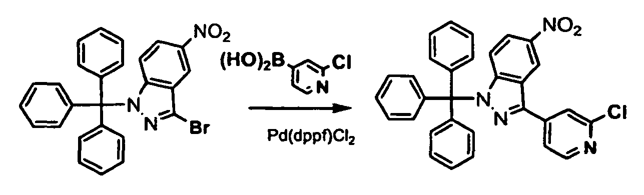

3-Bromo-5-nitro-1-trityl-1H-indazole (24.3 gm, 50 mmol) was stirred and degassed under nitrogen in dioxane/water (150/60 ml). Pyridine-2-chloro-4-boronic acid (8.7 gm, 55 mmol), Pd(dppf)Cl2 (4.1 gm, 5 mmol) and K₃PO₄ (26.5 g, 125 mmol) added. The reaction mixture was stirred at 80 C for 18 hrs. The reaction mixture was evaporated to dryness" dissolved in dichloromethane, filtered through a silica gel plug and washed with 20% ethylacetate/hexanes to obtain 22 gm of title product.

### Preparation of 3-(2-Cyclopropyl-pyridin-4-yl)-5-nitro-1-trityl-1H-indazole

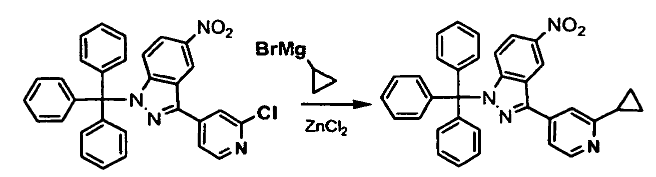

To a tube under nitrogen was added 36.2 ml ZnCl2 (0.5 M in THF,18.02 mmol) followed by cyclopropylmagnesium bromide (34 ml of 0.5 M in THF soln., 16 mmol). The resultant mixture was stirred for 20 min at r.t. NMP (23 ml) was added, stirred for 5 min followed by 3-(2-Chloro-pyridin-4-yl)-5-nitro-1-trityl-1H-indazole (5.5 gm, 10.6 mmol) and Pd[P(tBu)₃]₂ (0.108 gm, 0.22 mmol). The tube was sealed under nitrogen and stirred at 100 C for 18 hrs. The reaction mixture was then cooled and evaporated to dryness. Silica gel chromatography using 5-20% ethylacetate/hexanes gave 2.1 g of title product.

### Preparation of 3-(2-Cyclopropyl-pyridin-d-yl)-1-trityl-1H-indazol-5-ylamine

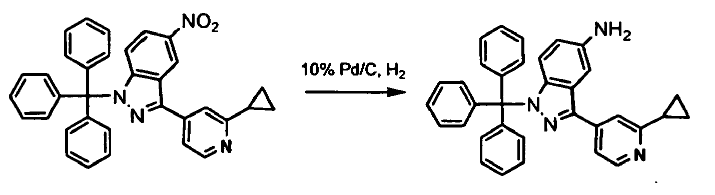

3-(2-Cyclopropyl-pyridin-4-yl)-5-nitro-1-trityl-1H-indazole (2 gm) was dissolved in 20 mol of 50% methanol/toluene and balloon hydrogenated for 18 hrs in the presence of 0.4 gm of 50% by weight 10% Pd/C. The reaction mixture was filtered and evaporated to give the title product.

### Preparation of 3-Methoxymethyl-1-{2-[4-(4-pyrimidin-2-yl-phenyl)-3,6-dihydro-2H-pyridin-1-yl]-acetyl}-pyrrolidine-3-carboxylic acid [3-(2-cyclopropyl-pyridin-4-yl)-1-trityl-1H-indazol-5-yl]-amide

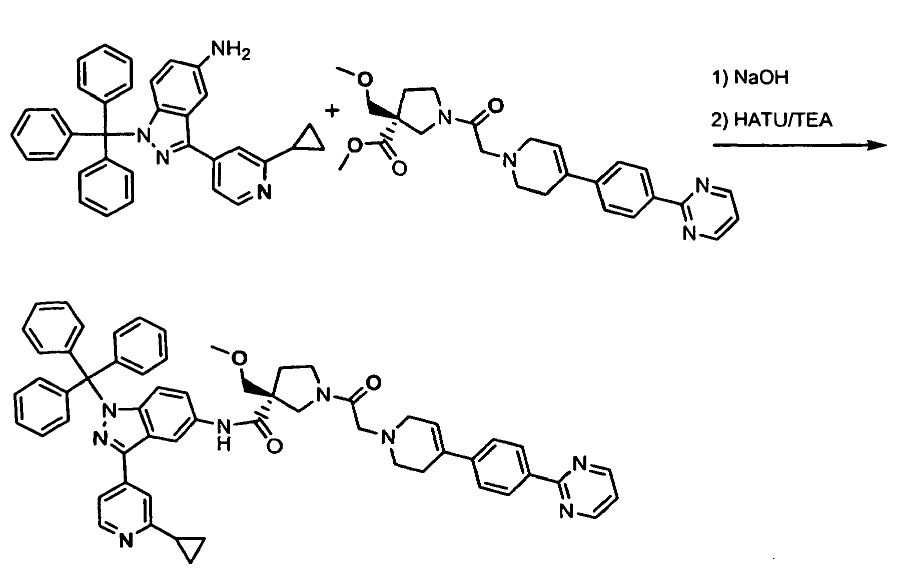

3-Methoxymethyl-1-{2-[4-(4-pyrimidin-2-yl-phenyl)-3,6-dihydro-2H-pyridin-1-yl]-acetyl}-pyrrolidine-3-carboxylic acid methyl ester (500 mg, 1.11 mmol) was added to a solution of 10 ml of 50% methanol/dichloromethane. 5 mL of 1 N NaOH was added and the reaction mixture stirred for 18 hrs. 5.5 ml of 1 N HCl was added and the reaction mixture evaporated to dryness. To the crude reaction mixture was added 3-(2-Cyclopropyl-pyridin-4-yl)-1-trityl-1H-indazol-5-ylamine (601 mg, 1.22 mmol), HATU (464 mg, 6.66 mmol), triethylamine (0.93 ml, 6.66 mmol) and 10 ml of 50% N,N-dimethylformamide/dichloromethane. The reaction mixture was stirred for 18 hrs. followed by washing with brine and extracted with ethylacetate 3X. After evaporation of the organic layers, the crude reaction mixture was chromatographed on silica gel using 1-3% 2N NH₃/methanol/dichlormethane as the eluent to obtain 570 mg of title product.

### Preparation of 3-Methoxymethyl-1-{2-[4-(4-pyrimidin-2-yl-phenyl)-3,6-dihydro-2H-pyridin-1-yl]-acetyl}-pyrrolidine-3-carboxylic acid [3-(2-cyclopropyl-pyridin-4-yl)-1H-indazol-5-yl]-amide

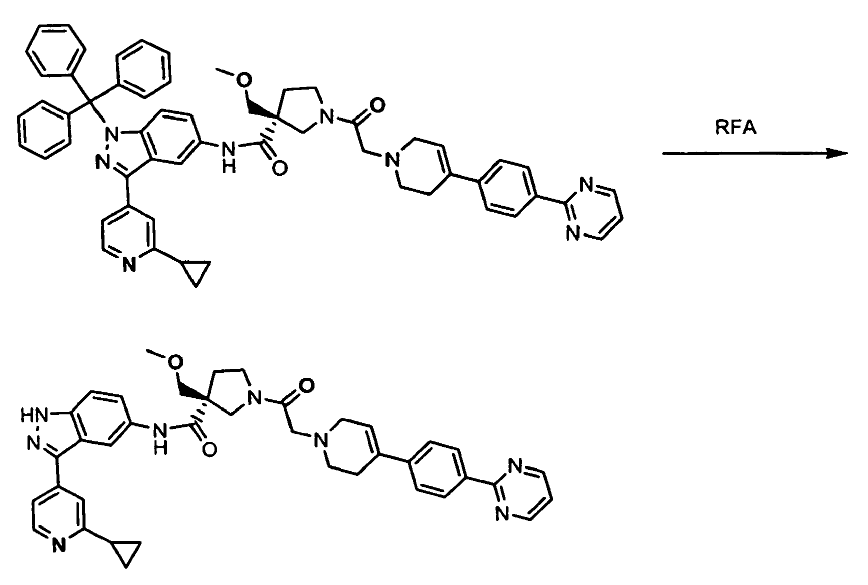

3-Methoxymethyl-1-{2-[4-(4-pyrimidin-2-yl-phenyl)-3,6-dihydro-2H-pyridin-1-yl]-acetyl}-pyrrolidine-3-carboxylic acid [3-(2-cyclopropyl-pyridin-4-yl)-1-trityl-1H-indazol-5-yl]-amide (570 mg, 0.63 mmol) was dissolved in 4 ml of trifluoroacetic acid (TFA), 10 ml dichloromethane and 2 ml of water and the reaction mixture was stirred for 18 hrs. The reaction mixture was evaporated and chromatographed on silica gel using 3-5% 2N NH₃/methanol/dichlormethane to obtain 444 mg of title product. MS (M+1)= 669. Retention time (minutes) 2.67.

### Scheme 13

### Preparation of 3-(3,6-Dihydro-2H-pyran-4-yl)-1-trityl-1H-indazol-5-ylamine and 3-(Tetrahydro-pyran-4-yl)-1-trityl-1H-indazol-5-ylamine

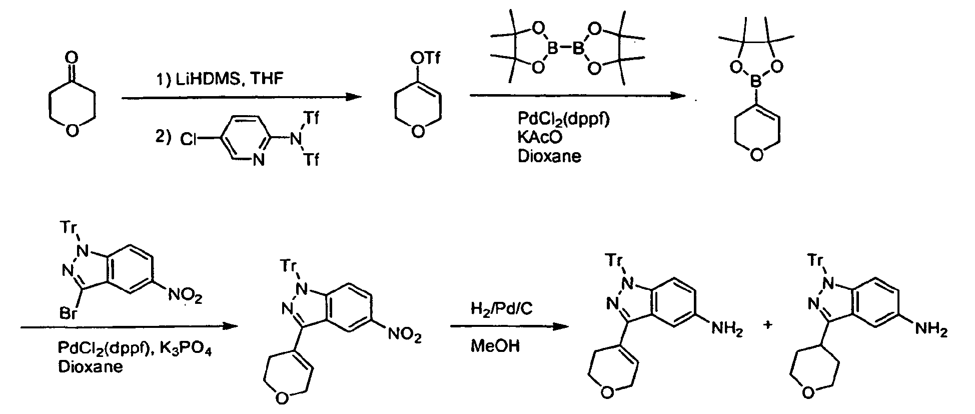

### Step 1: Preparation of Trifluoro-methanesulfonic acid 3,6-dihydro-2H-pyran-4-yl ester

To a solution of 4-tetrahydropyranone (2g, 0.02 mol) in THF (10 mL) at -78 °C, LiHMDS (1 M, 11 ml) was added dropwise through a syringe. The formed solution was then stirred at -78 °C for about 1 hour. The reaction was warmed up to room temperature briefly and was cooled back to -78 °C. Then 2-[N, N-bis(trifluoromethylsulfonyl)amino]-5-chloropyrine (7.85 g, 0.02 mol) was added in four portions. The resulted reaction mixture was gradually warmed up to room temperature and was stirred for overnight. After removal of solvent, the residue was purified using chromatography (eluted with DCM/Hexane = 80/20) to give product as a colorless oil (3g).

### Step 2: Preparation of 4-(4,4,5,5-Tetramethyl-[1,3,2]dioxaborolan-2-yl)-3,6-dihydro-2H-pyran

A 250 mL round bottom flask containing trifluoro-methanesulfonic acid 3, 6-dihydro-2H-pyran-4-yl ester (2.7 g, 11.6 mmol), PdCl₂(dppf) (440 mg, 0.6 mmol) and potassium acetate (3g, 36 mmol) in dioxane (20 mL) was flushed with Ar three times. The reaction mixture was heated at 80 °C for overnight. After the reaction was cooled to room temperature, the reaction mixture was filter through a column packed with celite and the filtrate was concentrated. The residue was purified using column chromatography (10 % hexane in dichloromethane) and product was obtained as white solid (2g, 81% yield).

### Step 3: Preparation of 3-(3,6-Dihydro-2H-pyran-4-yl)-5-nitro-1-trityl-1H-indazole

A mixture containing 4-(4,4,5,5-Tetramethyl-[1,3,2]dioxaborolan-2-yl)-3,6-dihydro-2H-pyran (0.8 g, 3.8 mmol), 3-Bromo-5-nitro-1-trityl-1H-indazole (2g, 4.0 mmol), PdCl₂(dppf) (150 mg, 0.2 mmol) and potassium phosphate (2.6 g, 12 mmol) in dioxane (10 mL) was flushed with Ar and was heated at 80 °C for overnight under Ar. LC-MS indicated the completion of reaction and the mixture was filter through celite. The filtrate was concentrated and was purified on a column (10 hexane in dichloromethane) to give 1.2 g of product (64 % yield).

### Step 4: Preparation of 3-(3,6-Dihydro-2H-pyran-4-yl)-1-trityl-1H-indazol-5-ylamine and 3-(Tetrahydro-pyran-4-yl)-1-trityl-1H-indazol-5-ylamine

To a solution of 3-(3,6-Dihydro-2H-pyran-4-yl)-5-nitro-1-trityl-1H-indazole (100 mg, 0.2 mmol) in methanol was added and Pd/C (10%, 20 mg). The reaction mixture was evacuated under vacuum and hydrogen gas was filled. After repeated three times the reaction was kept under hydrogen atmosphere overnight at room temperature. LCMS showed the starting material was consumed completely and both 3-(3,6-dihydro-2H-pyran-4-yl)-1-trityl-1H-indazol-5-ylamine and 3-(tetrahydro-pyran-4-yl)-1-trityl-1H-indazol-5-ylamine were obtained. The two products were separated using prep-HPLC to give both product in 60 % and 40 % yield respectively.

### Preparation 16

### Step1: Preparation of (2-Nitrophenyl)-(2-trimethylsilanylthiazol-5-yl)methanone

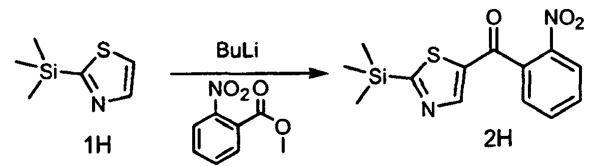

To a solution of 2-trimethylsilylthiazole (2.0 g, 12.7 mmol) in THF at -78 C was added BuLi (5.08 mL, 2.5 M, 12.7 mmol). The reaction was stirred at -78 C for 0.5 hr and methyl 2-nitrobenzoate (2.69 mL, 19.1 mmol) was added. The suspension was stirred at room temperature for 2 hr and quenched with ammonium chloride solution. The mixture was extracted with ethyl acetate and the combined organic layer was dried over sodium sulfate, concentrated and purified by column chromatography to give **2H** (680 mg)

### Step2: Preparation of (2-Aminophenyl)-thiazol-5-yl-methanone

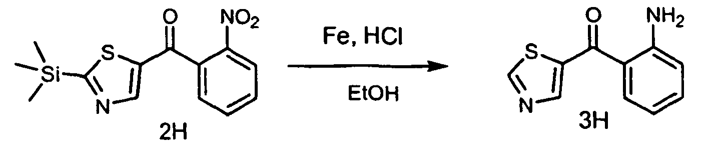

To a solution of **2H** (631 mg, 2.72 mmol) in ethanol/H2O (12 mL/0.5 mL) was added iron (1.5 g, 27.2 mmol), then concentrated HCl (0.5 mL). The reaction was heated to reflux for 2.5 hr and filtered. The filtrated was neutralized with 1.0 N NaOH (5 mL) and extracted with ethyl acetate. The combined organic layer was dried over sodium sulfate, concentrated and purified by column chromatography to give **3H** (280 mg)

### Step 3: Preparation of 3-Thiazol-5-yl-1H-indazole

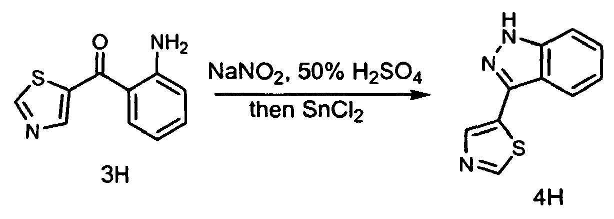

To a solution of **3H** (241 mg, 0.103 mmol) in 3 mL of 50% sulfuric acid at 0 C was added sodium nitrite (97.4 mg, 0.125 mmol). The reaction was stirred at 0 C for 1 hr and room temperature for 5 minutes and chilled to 0 C. To this was added SnCl₂ (673 mg, 0.310 mmol) and stirred at 0 C for 1 hr. The suspension was diluted with water and extracted with ethyl acetate. The combined organic layer was washed with 1 N NaOH solution, dried over sodium sulfate, concentrated and purified by column chromatography to give **4H** (149 mg)

### Step 4: Preparation of 5-Nitro-3-thiazol-5-yl-1H-indazole

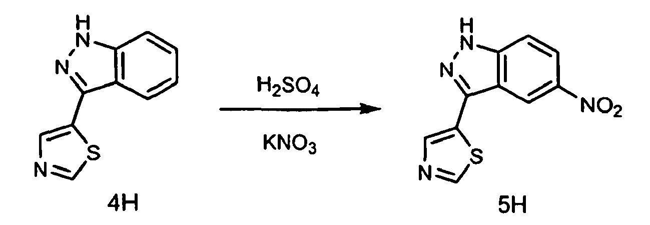

To a solution of **4H** (149 mg, 0.74 mmol) in 2 mL of concentrated sulfuric acid at 0 C was added potassium nitrate (82.3 mg, 0.81 mmol). The reaction was stirred at room temperature for 2.5 hr and poured into ice water. The yellow solid was collected through filtration and dried under vacuum to afford **5H** (98 mg)

### Step 5: Preparation of 3-Thiazol-5-yl-1H-indazol-5-ylamine

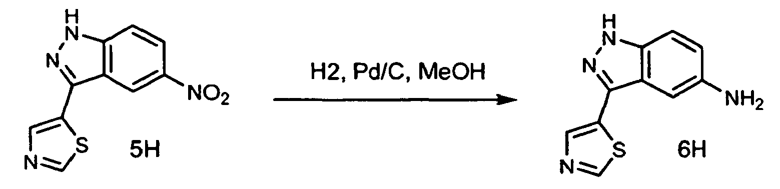

A suspension of **5H** (98 mg, 0.39 mmol) and catalytic amount of Pd on carbon (5%) in methanol was stirred under a hydrogen atmosphere overnight and filtered through celite. The filtrate was concentrated to provide **6H** (62 mg)

### Preparation 17

### Step 1: Preparation of 3-(3-Trifluoromethylphenyl)-5-nitro-1-trityl-1H-indazole

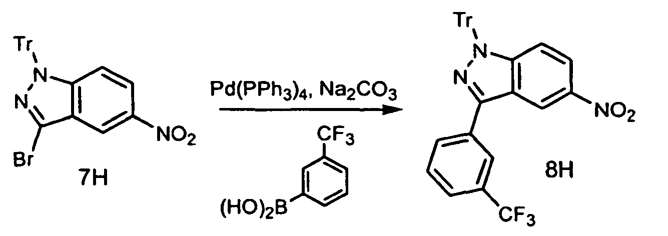

A suspension of **7H** (200 mg, 0.413 mmol), 3-trifluoromethylphenylboronic acid (94 mg, 0.498 mmol), Pd(PPh₃)₄ (48 mg, 0.041 mmol) and 2 M sodium carbonate (0.45 mL) in 3 mL of dioxane/EtOH/H₂O (7/3/2) was heated to 150 C for 10 minutes using microwave. The reaction was partition between ethyl acetate and water. The organic layer was dried over sodium sulfate, concentrated and purified by column chromatography to afford **8H** (189 mg)

### Step 2: Preparation of 3-(3-Trifluoromethyl-phenyl)-1-trityl-1H-indazol-5-ylamine

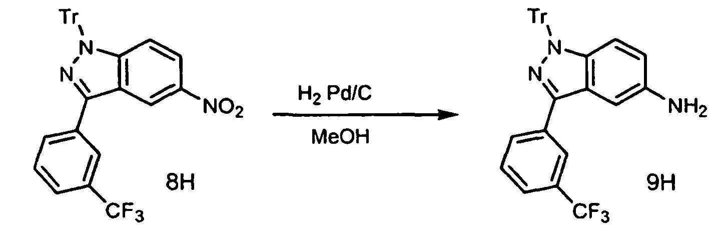

A suspension of **8H** (189 mg, 0.350 mmol) and catalytic amount of Pd on carbon (5%) in methanol was stirred under a hydrogen atmosphere overnight and filtered through celite. The filtrate was concentrated to provide **9H** (139 mg)

### Step 3: Preparation of 3-(3-Trifluoromethyl-phenyl)-1H-indazol-5-ylamine

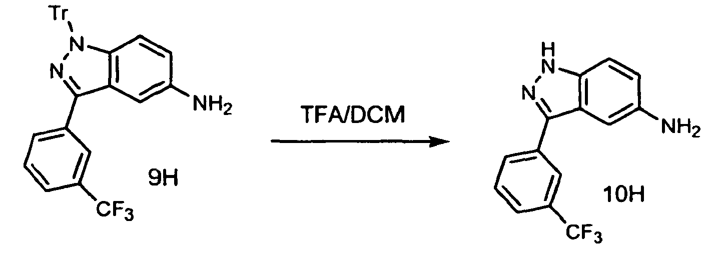

A solution of **9H** (139 mg, 0.268 mmol) in 2 mL of TFA/DCM (1/1) was stirred at room temperature for 1 hr and concentrated. The residue was purified by column chromatography to afford **10H** (61.7 mg)

### Preparation 18

### Preparation of 3-(4-Methoxy-3-trifluoromethyl-phenyl)-1H-indazol-5-ylamine

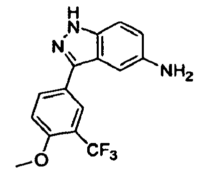

3-(4-Methoxy-3-trifluoromethyl-phenyl)-1H-indazol-5-ylamine was prepared using essentially the same scheme for preparing 10H. The boronic acid for the first step was 4-methoxy-3-trifluoromethylphenylboronic acid.

### Preparation 19

### Preparation of 3-Cyclohexyl-1H-indazol-5-ylamine

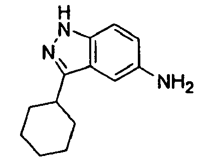

3-Cyclohexyl-1H-indazol-5-ylamine was prepared using essentially the same scheme for preparing **10H**. The boronic acid for the first step was 1-cyclohexene-boronic acid.

### Preparation 20

### Preparation of 3-(2-Fluoro-pyridin-4-yl)-1H-indazol-5-ylamine

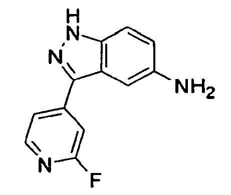

3-(2-Fluoro-pyridin-4-yl)-1H-indazol-5-ylamine was prepared using essentially the same scheme for preparing **10H**. The boronic acid for the first step was 3-fluoro-4-pyridineboronic acid pinacol ester.

### Preparation 21

### Preparation of 4-fluoro-4-thiazol-2-yl-piperidine-1-carboxylic acid tert-butyl ester 7I

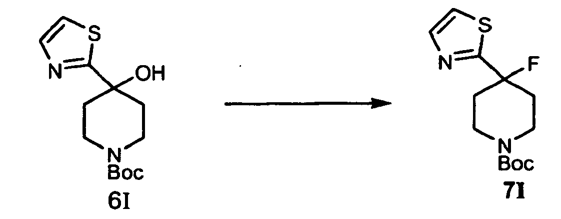

4-Hydroxy-4-thiazol-2-yl-piperidine-1-carboxylic acid tert-butyl ester 61 (500 mg, 1.76 mmol) was dissolved in CH₂Cl₂ (20 mL) and cooled to 0 °C. DAST (0.46 mL, 3.52 mmol) was then added. The mixture was stirred at 0 °C for 1 hr and then quenched with sat. NaHCO₃. The separated organic layer was dried and concentrated *in vacuo.* The crude was purified with silica gel column (eluting with 12.5% ethyl acetate in hexanes) to yield an off-white solid (443 mg) as the title compound.

### Preparation 21A

### Step 1:

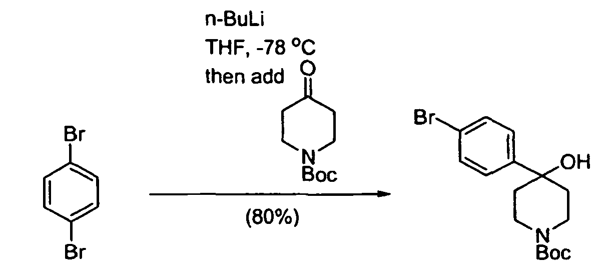

To a solution of 1,4-dibromobenzene (1.0g, 4.24 mmol) in tetrahydrofuran (10ml) at -78 °C under nitrogen, a solution of n-butyl lithium (1.7 ml, 4.24 mmol, 1.6M in hexane) was added slowly. The mixture was allowed to warm from -78 °C to -20 °C in 1 hr. A solution of piperidone (703mg, 3.53 mmol) in tetrahydrofuran (5 ml) was dded at -78 °C and the mixture was stirred at the same temperature for 1 hr. Saturated ammonium chloride solution was added and the mixture was allowed to warm to r.t. Water and ethyl acetate were added and layers were separated. The aqueous layer was extracted with ethyl acetate (X2). The combined organic layers were dried (MgSO₄) and filtered. Solvents were removed in vacuum and column chromatography [ethyl acetate - hexane, 5:1 (v/v)] gave 4-(4-bromophenyl)-4-hydroxypiperidine-1-carboxylic acid tert-butyl ester (1.0g, 80%) as colorless oil.

### Step 2:

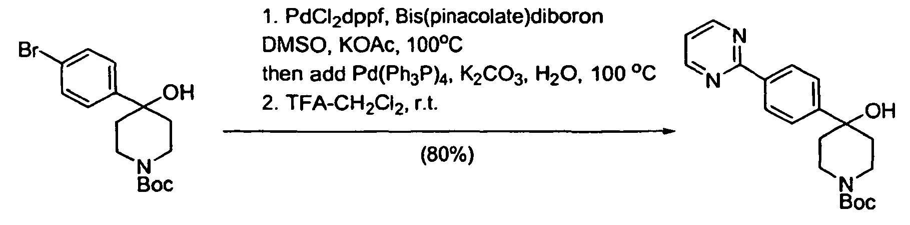

4-(4-bromophenyl)-4-hydroxypiperidine-1-carboxylic acid tert-butyl ester (800mg, 2.25mmol), bis(pinacolate)diboron (856mg, 3.37 mmol), PdCl₂(dppf).CH₂Cl₂ (184mg, 0.23mmol), potassium acetate (660mg, 6.74mmol) were weighted into a sealed-tube. Methyl sulfoxide (20ml) was added and the mixture was purged with nitrogen for 20 min before it was heated at 100 °C for 2 hr under nitrogen. The mixture was cooled to r.t. Potassium carbonate (1.55, 11.2mmol), 2-bromopyrimidine (429mg, 2.70mmol) and water (10ml) were added. The mixture was again purged with nitrogen for 20 min. Palladium tetrakistriphenylphosphine (260mg, 0.23mmol) was added and the final mixture was stirred at 100 °C for a further 2 hr. After being cooled to r.t., ethyl acetate and water were added. The mixture was filtered through a pad of Celite. Layers were separated and the organic layer was washed with water (X2). The combined aqueous layers were extracted with ethyl acetate (X1). The combined organic layers were dried (MgSO₄) and filtered. Solvents were removed in vacuum and column chromatography [ethyl acetate - hexane, 1:1 (v/v)] gave 4-hydroxy-4-(4-pyrimidin-2-ylphenyl)-piperidine-1-carboxylic acid tert-butyl ester (639mg, 80%) as colorless oil.

### Preparation 22

### Preparation of 4-methoxy-4-(4-pyrimidin-2-yl-phenyl)-piperidine-1-carboxylic acid tert-butyl ester 9I

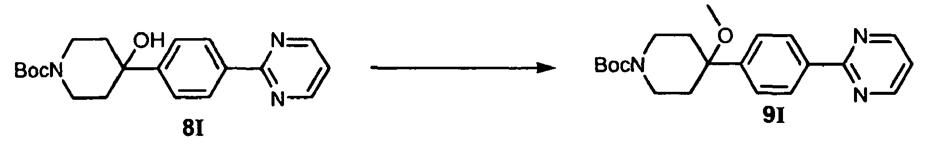

4-Hydroxy-4-(4-pyrimidin-2-yl-phenyl)-piperidine-1-carboxylic acid tert-butyl ester 8I (138 mg, 0.39 mmol) was dissolved in DMF (2 mL) and cooled to 0 °C. Mel (0.1 mL) was added followed by the addition of NaH (26 mg, 60% suspension in mineral oil). After 30 min at 0 °C, the reaction was quenched with sat. NH₄Cl and extracted with ethyl acetate. The combined organic layers was washed with brine, dried and concentrated *in vacuo.* The residue was purified with prep TLC plates (developing with 50% ethyl acetate/hexanes) to yield a colorless film (80 mg) as the title compound.

### Preparation 23

### Preparation of 4-bromo-2,6-dimethyl-pyridine 11

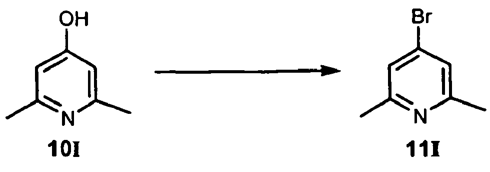

2,6-Dimethyl-pyridin-4-ol 10I (6.16 g, 50 mmol), PBr₅ (11.9 g, 27.65 mmol) and POBr₃ (2.5 mL, 24.6 mmol) was combined and CHCl₃ (2.5 mL) was added. The reaction was heated at 100 °C for 5 hrs and then cooled in an ice bath. Solid KOH was added till PH reached 7-8 followed by extraction with Et₂O (3x75 mL). The combined ether layer was dried and evaporated *in vacuo* to give a thick clear crude oil (10.1 g) as the title compound.

### Preparation 24

### Preparation of 2,6-dimethyl-4-pyridine boronic acid 12

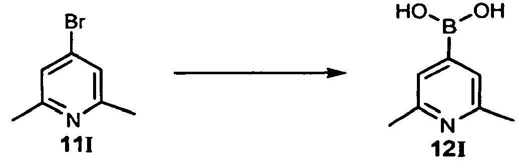

4-Bromo-2,6-dimethyl-pyridine (910 mg, 4.9 mmol) and triisopropyl borate (2.3 mL, 10 mmol) in THF (10 mL) were cooled in a -78 °C bath. BuLi (2.7 M, 7 mL) was added in drop wise. After 3 hrs, the bath was removed. The reaction was acidified with 1 N HCl till pH =1. The separated aqueous layer was neutralized with NaOH and subsequently extracted with ethyl acetate. A crude white solid was obtained (800mg) as the title compound.

### Preparation 25

### Preparation of 2-trifluoromethyl-4-pyridine boronic acid 14

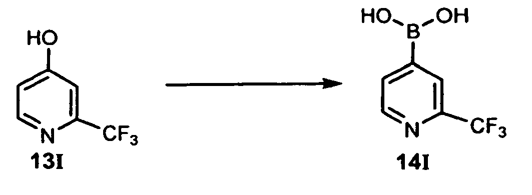

The title compound was prepared from 2-trifluoromethyl-pyridin-4-ol (13I) by a procedure essentially similar to that described in Chem. Het. Cpds, 1997, p. 995, the disclosure of which is incorporated herein by reference thereto.

### EXAMPLE 337

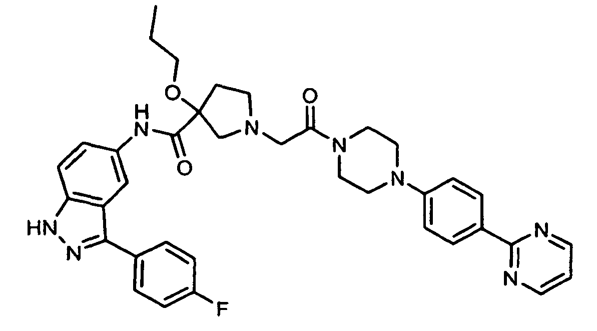

### Step 1: Synthesis of 2-allyloxy-acrylic acid ethyl ester

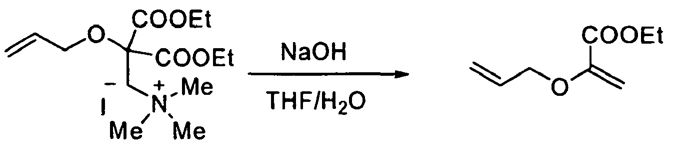

The (2-allyloxy-2,2-bis-ethoxycarbonyl-ethyl)-trimethyl-ammonium iodide was prepared according to a procedure essentially similar to that described in J. Am. Chem. Soc. 1987, 109, 1170-1186, the disclosure of which is incorporated herein by reference thereto. At 0 C, a solution of NaOH (1N, 6 ml) was added into a stirred solution of (2-allyloxy-2,2-bis-ethoxycarbonyl-ethyl)-trimethyl-ammonium iodide (2.1 g) in DMSO and water (9:1, 35 ml) and resulting solution was allowed to stir at room temperature overnight. The reaction mixture was diluted with ether, washed with water three times, brine and dried (MgSO₄). After evaporation of solvent, the title compound (431 mg) was obtained.

### Step 2: Synthesis of 3-allyloxy-1-benzyl-pyrrolidine-3-carboxylic acid ethyl ester

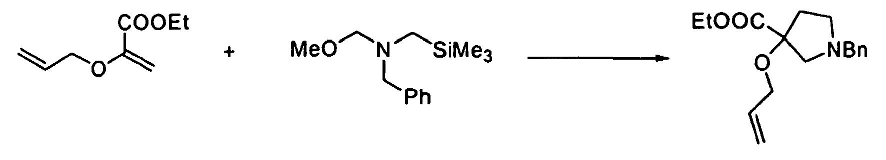

To a stirred solution of the 2-allyloxy-acrylic acid ethyl ester (431 mg) and N-(methoxymethyl)-N-(trimethylsilylmethyl)benzylamine (847 µl) in dichloromethane (10 ml) was added at 0 °C trifluoroacetic acid (21 µl). The resulting solution was warmed to room temperature and stirred overnight. The crude product was purified by column chromatography on silica gel, eluted with a solution of ethyl acetate in hexanes (25-100%) to give the title compound (341 mg).

### Step 3: Synthesis of 3-propoxy-pyrrolidine-3-carboxylic acid ethyl ester

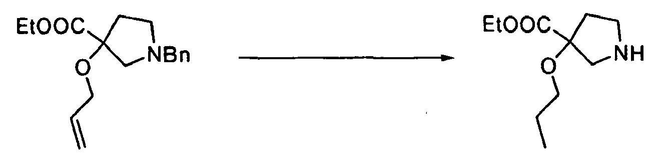

A mixture of 3-allyloxy-1-benzyl-pyrrolidine-3-carboxylic acid ethyl ester (145 mg), ammonium formate (126 mg), 10% Pd/C (20 mg) and methanol (4 ml) was refluxed for 40 minutes. The mixture was filtered through Celite, washed with ethyl acetate. The combined filtrate was concentrated and the residue was taken into ethyl acetate, washed with brine and dried over MgSO₄. Evaporation of solvent provided the title compound as oil (84 mg).

### Step 4: Synthesis of 1-{2-Oxo-2-[4-(4-pyrimidin-2-yl-phenyl)-piperazin-1-yl]-ethyl}-3-propoxy-pyrrolidine-3-carboxylic acid [3-(4-fluoro-phenyl)-1H-indazol-5-yl]-amide

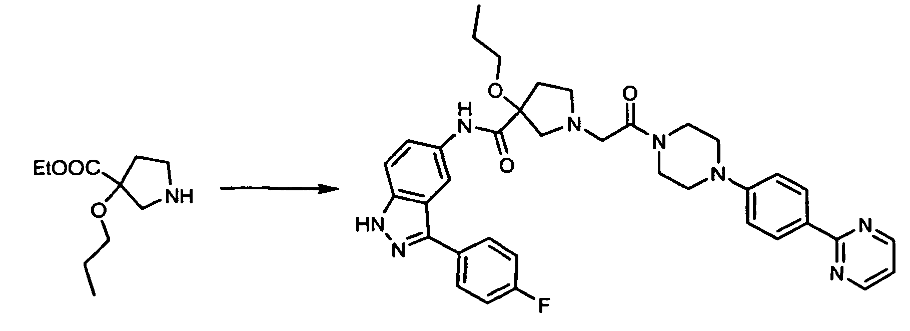

Synthesis of 1-{2-Oxo-2-[4-(4-pyrimidin-2-yl-phenyl)-piperazin-1-yl]-ethyl}-3-propoxy-pyrrolidine-3-carboxylic acid [3-(4-fluoro-phenyl)-1H-indazol-5-yl]-amide from 3-propoxy-pyrrolidine-3-carboxylic acid ethyl ester was essentially similar to the procedures described above for preparing compounds with a pyrrolidine moiety in the core structure, for example, 1, 3, 60, 85, 98, 128, 183 and 184.

### Preparation 26

### Intermediate To The Synthesis of 3-Morpholin-4-ylmethyl-1-[2-oxo-2-(4-thiophen-3-yl-3.6-dihydro-2H-pyridin-1-yl)-ethyl]-pyrrolidine-3-carboxylic acid [3-(2-methyl-ridin-4-yl)-1H-indazol-5-yl]-amide

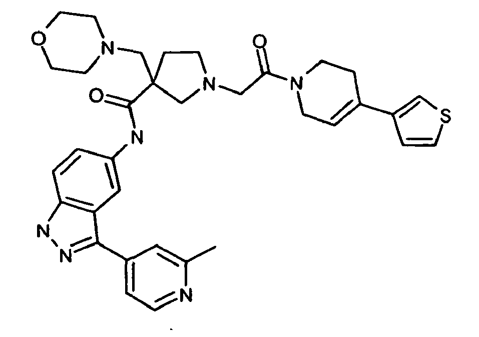

### Step 1: Synthesis of 2-morpholin-4-ylmethyl-acrylic acid methyl ester

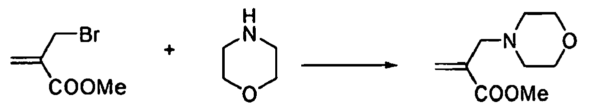

To a mixture of methyl 2-(bromomethyl)acrylate (119 µl, 1 mmol) and K₂CO₃ (138 mg, 1eq) in acetonitrile (2 ml) was added morpholine (96 µl, 1.1mmols). The mixture was stirred overnight, filtered and concentrated. The residue was partitioned between ether and water, and organic layer was isolated, washed with brine and dried (MgSO₄). Solvent was removed and residue was purified by column chromatography. Ethyl acetate eluted out the title compound as clear oil (110 mg, 59%).

### Step 2: Synthesis of 1-benzyl-3-morpholin-4-ylmethyl-pyrrolidine-3-carboxylic acid methyl ester

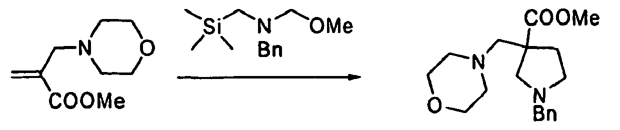

To a stirred cold solution (0 °C) of 2-morpholin-4-ylmethyl-acrylic acid methyl ester (110 mg, 0.594 mmols) and *N*-(methoxylmethyl)-*N*-(trimethylsilylmethyl)-benzylamine (182 µl, 0.71 mmols) in dichloromethane (2 ml) was added slowly trifluoroacetic acid (9 µl, 0.12 mmols). The resulting solution was allowed to stir at rt overnight and directly purified by column chromatography eluting with ethyl acetate to provide title compound as clear oil (86 mg, 45%).

### Preparation 27

### Intermediate To The Synthesis of 1-{2-Oxo-2-[4-(4-pyrimidin-2-yl-phenyl)-piperazin-1-yl]-ethyl}-3-trifluoromethyl-pyrrolidine-3-carboxylic acid [3-(4-fluorophenyl)-1H-indazol-5-yl]-amide

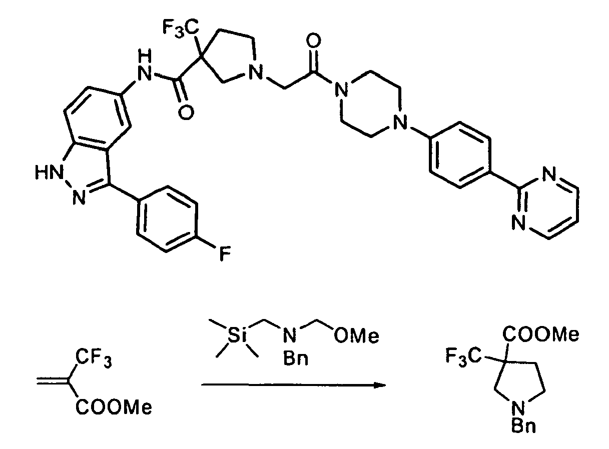

To a stirred cold solution (0 °C) of 2-trifluoromethyl-acrylic acid methyl ester (308 mg, 2 mmol) and *N*-(methoxylmethyl)-*N*-(trimethylsilylmethyl)benzylamine (529 µl, 4.8 mmol) in dichloromethane (3 ml) was added slowly trifluoroacetic acid (31 µl). The resulting solution was allowed to stir at rt overnight and directly purified by column chromatography eluting with ethyl acetate in hexane (1:4) to provide 1-benzyl-3-trifluoromethyl-pyrrolidine-3-carboxylic acid methyl ester as oil (438 mg, 76 %).

### Preparation 28

### Preparation of 3-difluoromethyl-pyrrolidine-1,3-dicarboxylic acid 1-tert-butyl ester 3-ethyl ester (2J)

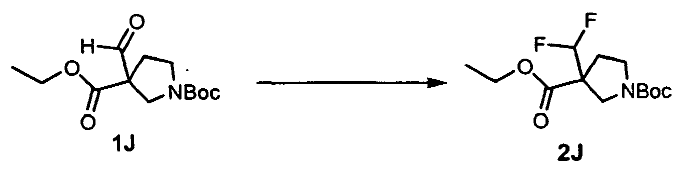

3-Formyl-pyrrolidine-1,3-dicarboxylic acid 1-tert-butyl ester 3-ethyl ester 1J (270 mg, 1mmol) was dissolved in CH₂Cl₂ (5 mL) at r.t. followed by addition of DAST (1.0 mL). After 16 hrs, the mixture was cooled to 0 °C and quenched with sat. NaHCO₃ solution followed by extraction of CH₂Cl₂ (2x5 mL). The combined organic layers was dried and concentrated *in vacuo.* The crude was purified using flash column (eluting with 20% to 33% ethyl acetate in hexanes) to yield **2J** (280 mg) as a yellow oil.

### Preparation 29

### Preparation of 3-But-2-ynyl-pyrrolidine-1,3-dicarboxylic acid 1-tert-butyl ester 3-methyl ester

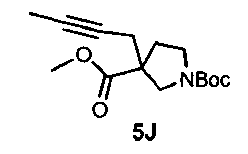

3-But-2-ynyl-pyrrolidine-1,3-dicarboxylic acid 1-tert-butyl ester 3-methyl ester **5J** was prepared essentially similarly as 3-prop-2-ynyl-pyrrolidine-1,3-dicarboxylic acid 1-tert-butyl ester 3-methyl ester by substituting 3-bromo-propyne with 1-bromo-but-2-yne

### Preparation 30

### Preparation of 3-Methylsulfanyl-pyrrolidine-1,3-dicarboxylic acid 1-tert-butyl ester 3-methyl ester

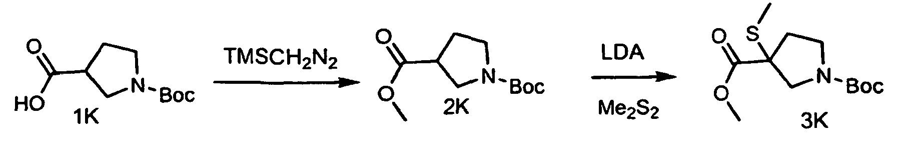

Pyrrolidine-1,3-dicarboxylic acid 1-tert-butyl ester (4.3 gm, 20 mmol) was dissolved in 28 mL of toluene and 3.5 ml of methanol. Trimethylsilyldiazomethane 2N solution in hexanes(13 ml, 26 mmol) was added dropwise at 0 °C and the reaction mixture stirred for 10 min at ambient temperature. The mixture was evaporated to obtain 4.3 gm of oil **2K.**

To the oil 2K (0.5 gm, 2.1 mmol) dissolved in tetrahydrofuran (15 ml) 1.2 ml of lithium diisopropylamide 2N solution in hexanes was added dropwise and the reaction mixture stirred for 1 hr at -78 °C and let warm to ambient temperature gradually. The reaction mixture was stirred for 18 hrs. A saturated solution of Ammonium chloride (25 ml) was added and the reaction mixture stirred for 5 min. The reaction mixture was extracted with ethyl acetate three times (3x25 ml), dried over magnesium sulfate, filtered and evaporated to give 0.386g of title product **3K**.

### Preparation 31

### Preparation of 1-Benzyl-3-ethoxy-pyrrolidine-3-carboxylic acid ethyl ester

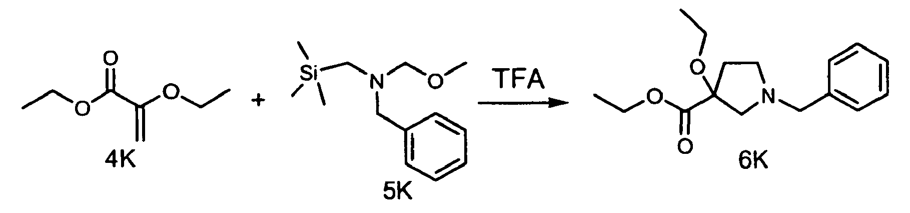

To a stirred solution of 2-ethoxyacrylate (3 g, 20 mmol) (prepared according to a procedure in Helv. Chem. Acta. 1989, 72, 213-223, the disclosure of which is incorporated herein by reference thereto) and *N*-(methoxymethyl)-N-(trimethylsilylmethyl)benzylamine (6.36 ml, 24 mmol) in dichloromethane (30 ml) was added at 0 °C a solution of trifluroacetic acid (0.072 ml, 0.3 mmol) in dichloromethane (2ml). The resulting solution was warmed to room temperature and stirred overnight. The crude product was purified by column chromatography on silica gel eluting with a solution of ethyl acetate in hexane (1:2) to give the title compound **6K** (2.1g, 37%).

### Preparation 32

### Preparation of 1-Benzyl-3-isopropoxy-pyrrolidine-3-carboxylic acid methyl ester

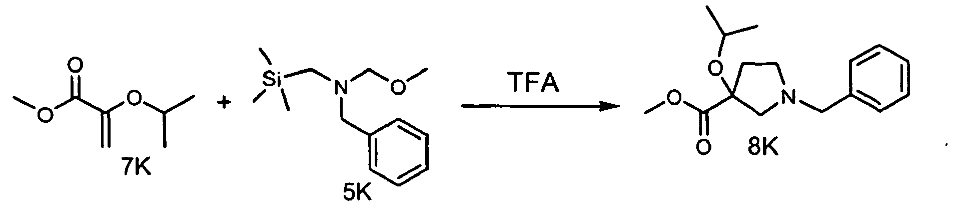

To a stirred solution of 2-iso-propoxymethylacrylate (2 g, 14 mmol) (prepared according to a procedure in Syn. commun. 1977, 7, 43-48, the disclosure of which is incorporated herein by reference thereto) and *N*-(methoxymethyl)-N-(trimethylsilylmethyl)benzylamine (4.26 ml, 17 mmol) in dichloromethane (25 ml) was added at 0 °C a solution of trifluroacetic acid (0.2 ml, 3 mmol) in dichloromethane (2ml). The resulting solution was warmed to room temperature and stirred overnight. The crude product was purified by column chromatography on silica gel eluting with a solution of ethyl acetate in hexane (1:2) to give the title compound **8K** (1.06 g, 28%).

### EXAMPLES 338-347

Following procedures similar to those described herein, for example, Examples 1, 3 to 60, 85, 98, 128, 183, 184, the compounds in Table 17 were prepared. "Ex." represents Example.

**Table 17**

| **Ex.** | **Compound** | **Mass Spec LCMS MH** | **Retention time Minutes** |
|---|---|---|---|
| 338 | 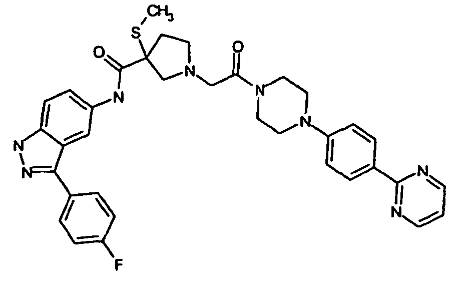 | 651 | 2.89 |
| 339 | 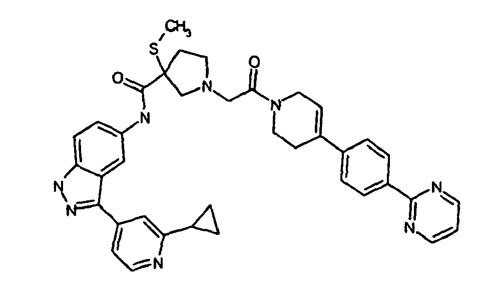 | 671 | 2.30 |
| 340 | 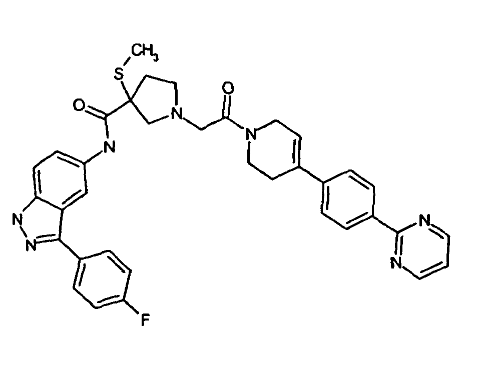 | 648 | 3.10 |
| 341 | 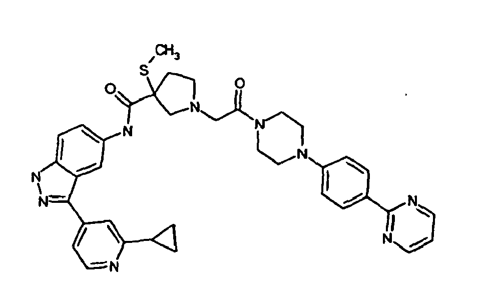 | 674 | 2.17 |
| 342 | 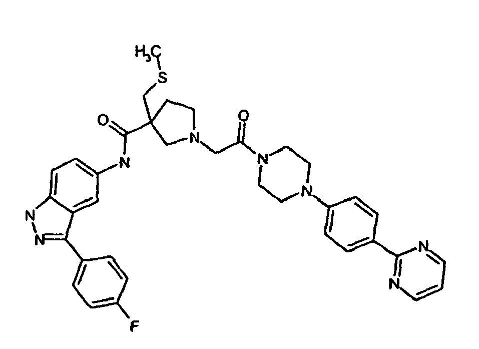 | 665 | 1.42 |
| 343 | 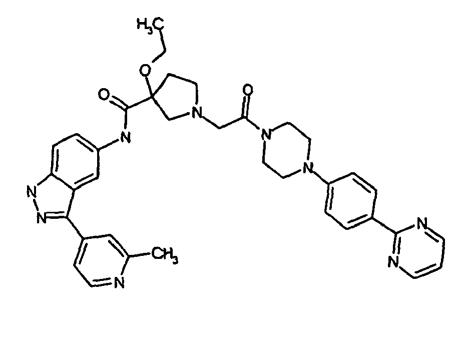 | 646 | 2.19 |
| 344 | 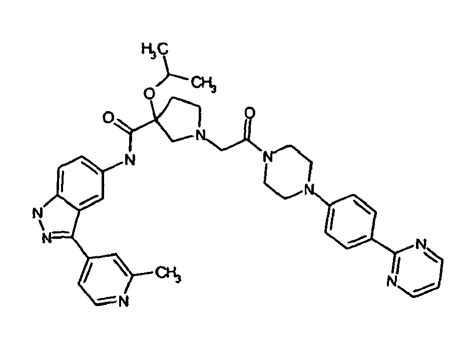 | 676 | 2.31 |
| 345 | 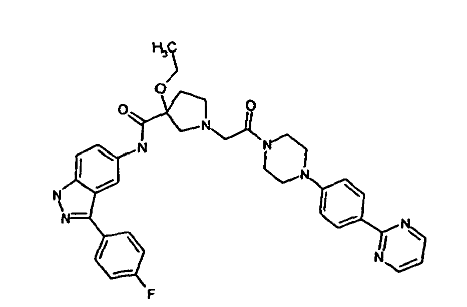 | 649 | 3.01 |
| 346 | 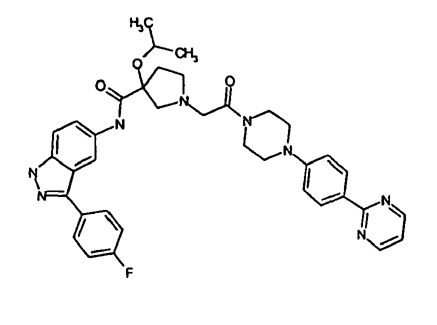 | 663 | 3.21 |
| 347 | 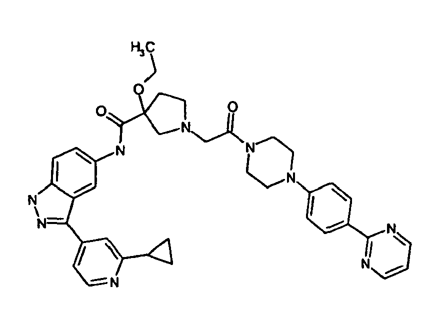 | 672 | 3.32 |

### EXAMPLES 348-349

### Step 1: Preparation of 2-dimethylaminomethyl-acrylic acid methyl ester

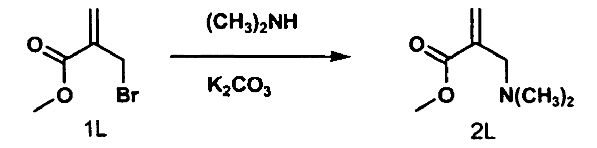

To a stirred solution of 2-bromomethyl-acrylic acid methyl ester (1 g, 5.59 mmol) in dry CH3CN (20 ml) was added 2M solution of dimethylamine in THF (3.07 ml, 6.14mmol) followed by K₂CO₃ (0.93 g, 6.7 mmol). The reaction mixture was stirred overnight, filtered and washed with CH₂Cl₂. The filtrate was concentrated. The crude product was used in next reaction without further purification.

### Step 2: Preparation of 1-benzyl-3-dimethylaminomethyl-pyrrolidine-3-carboxylic acid methyl ester

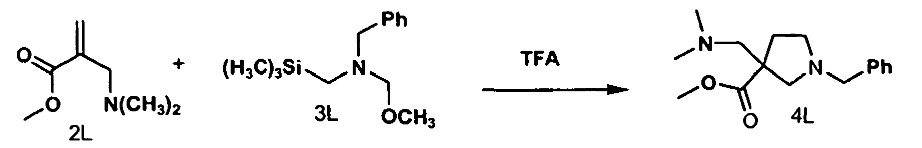

To a cold solution of 2-dimethylaminomethyl-acrylic acid methyl ester (0.266 g, 1.86 mmol) and *N*-(methoxymethyl)-*N*-(trimethylsilylmethyl)benzyl-amine (1.43 ml, 5.59 mmol) in dichloromethane (25 ml) was added at 0 °C trifluoroacetic acid (83 µl, 1.12 mmol). The resulting solution was warmed to room temperature and stirred for 72 hours. Saturated aq. NaHCO₃ (60 ml) was added to the reaction mixture and stirred for 10 minutes. Diluted with dichloromethane (50 ml) and the organic layer was separated. The organic layer was dried over Na₂SO₄, filtered and concentrated. The crude product was purified by column chromatography on silica get eluting with a solution of ethyl acetate in hexanes (1:2) followed by CH₂Cl₂/MeOH/NH₄OH (9/1/0.01) to give the title compound (400 mg, 78%).

### Step 3: Preparation of 3-dimethylaminomethyl-pyrrolidine-3-carboxylic acid methyl ester

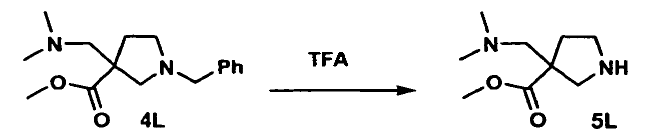

A mixture of 1-benzyl-3-dimethylaminomethyl-pyrrolidine-3-carboxylic acid methyl ester (400 mg), 10% Pd/C (40 mg) and methanol (30 ml) was shaken in a hydrogen parr shaker at 45 psi for overnight. The mixture was filtered through celite, washed with ethyl acetate. The combined filtrate was concentrated to give the title compound as oil (260 mg, 96%).

Using 5L and following procedures essentially similar to those described herein, for example, Examples 1, 3 to 60, 85, 98, 128, 183, 184, the compounds in Table 18 were prepared. "Ex." represents Example.

**Table 18**

| **Ex** | **Compound** | **Mass spec LCMS MH** | **Retention time (minutes)** |
|---|---|---|---|
| 348 | 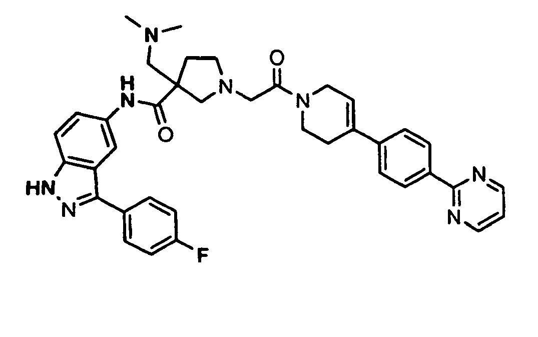 | 659 | 2.55 |
| 349 | 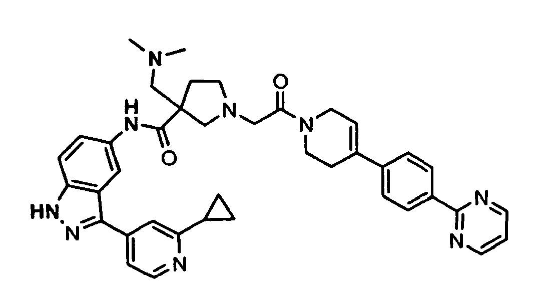 | 682 | 2.07 |

### EXAMPLES 350-352

Following procedures similar to those described herein, for example, Examples 1, 3 to 60, 85, 98, 128, 183, 184, the compounds in Table 19 were prepared. "Ex." represents Example.

**Table 19**

| **Ex** | **Compound** | **Mass spec LCMS MH** | **Retention time (minutes)** |
|---|---|---|---|
| 350 | 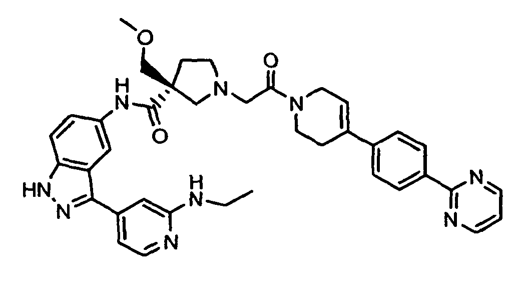 | 672 | 2.38 |
| 351 | 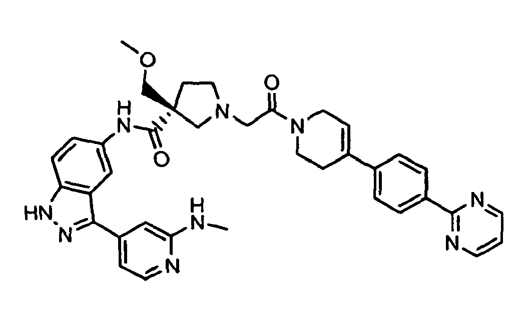 | 658 | 2.43 |
| 352 | 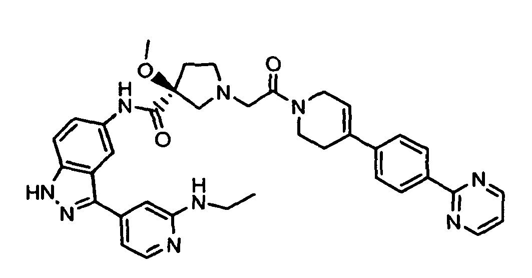 | 658 | 2.28 |

### Preparation 33

### Preparation of 3-{[3-(2-Cyclopropyl-pyridin-4-yl)-1-trityl-1H-indazol-6-yl]-methyl-carbamoyl}-3-methoxy-pyrrolidine-1-carboxylic acid tert-butyl ester (4M)

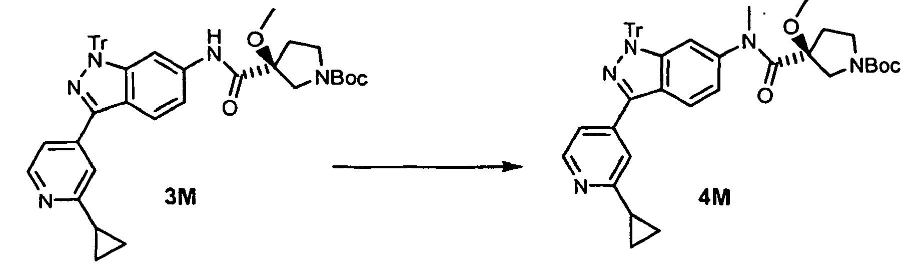

3-[3-(2-Cyclopropyl-pyridin-4-yl)-1-trityl-1H-indazol-6-ylcarbamoyl]-3-methoxy-pyrrolidine-1-carboxylic acid tert-butyl ester **3M** (216 mg, 0.3 mmol) was dissolved in THF (10 mL) at r.t. To this solution was added NaH (40 mg, 60% suspension in mineral oil, 1 mmol). After stirring for 10 min, CH₃I (0.6 mL, 9.6 mmol) was added. The reaction was worked up after 4 hrs by quenching with sat.NH₄Cl solution. After extraction with ethyl acetate, the combined organic layers was dried and concentrated *in vacuo* to obtain **4M**. **4M** is used as the resulting crude.

### Preparation 34

### Preparation of 2-[6-(3-R-Methyl-piperazin-1-yl)-pyridin-3-yl]-pyrimidine

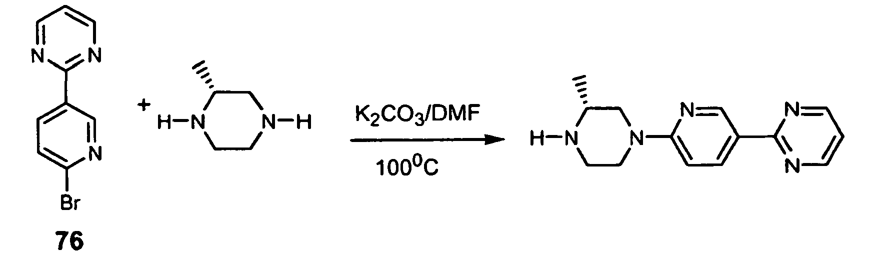

Following essentially the same procedure described in Example 98, Step 7, except substituting an equivalent quantity of 2-R-Methyl piperazine for piperazine the title compound is obtained as a white solid (ESMS MH,256) 95% Yield.

### EXAMPLE 353

### Preparation of 3-Ethynyl-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1H-indazol-5-yl]-amide

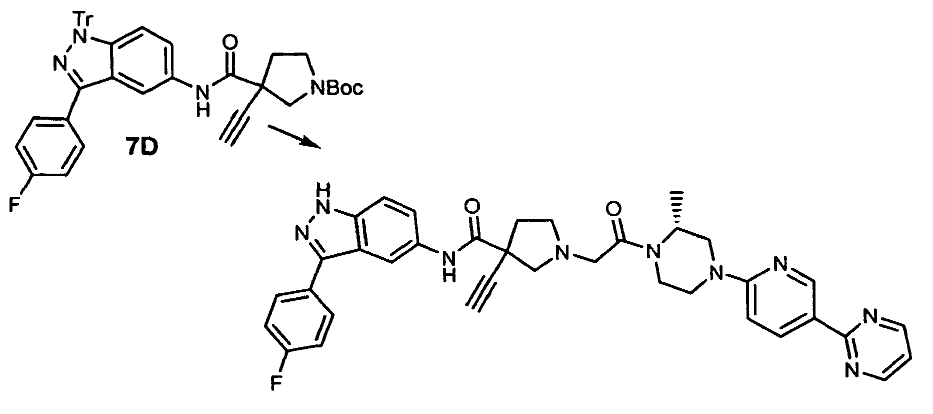

Following essentially the same procedure as Example 335, except substituting an equivalent quantity of 2-[6-(3-R-Methyl-piperazin-1-yl)-pyridin-3-yl]-pyrimidine for **23G**, the title compound was obtained as a white solid (60%) LCMS (MH, 644.4) Retention time = 2.52 minutes.

This racemate was resolved into two single isomers using Chiralpak AD column eluting with 70:30 Hexanes: Isopropanol containing 0.2% Diethylamine.
Isomer A (LCMS) MH 644
Isomer B (LCMS) MH 644

### EXAMPLE 354

### Preparation of 3-(1-Hydroxy-ethyl)-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (1N) (Example 323 in Table 16)

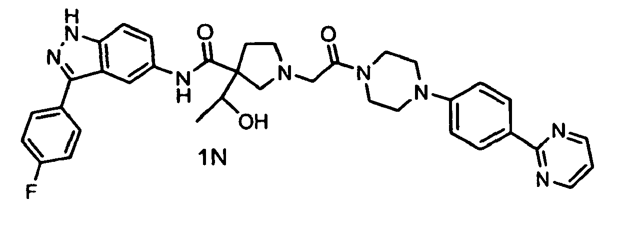

### Step 1: Preparation of 3-(1-Acetoxy-ethyl)-1-benzyl-pyrrolidine-3-carboxylic acid methyl ester (2N)

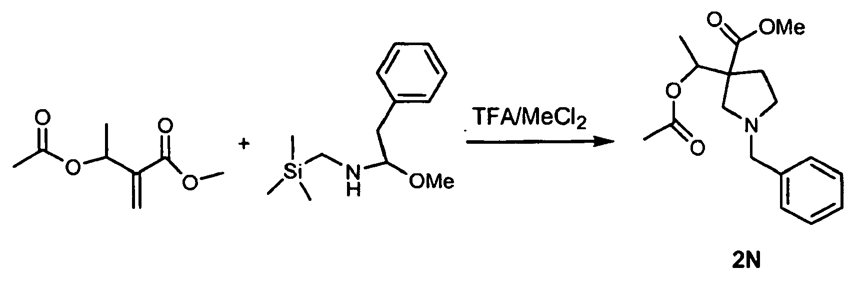

Trifluoroacetic acid (0.5g,4.38mmol) was added to a solution of methyl-3-acetoxy-2-methylenebutyrate (5g, 29.03mmol) and N-methoxymethyl-N-trimethylsilyl benzylamine (6.89g, 29.03mmol) in MeCl₂ (50ml) at 0°C then stirred overnight at room temperature. The solvent was evaporated and the residue extracted with EtOAc (200ml) and H₂O (50ml) and saturated NaHCO₃ solution (50ml). The organic layer was separated, dried over MgSO₄ filtered and evaporated solvent. The residue chromatographed on silica gel eluting with (v:v) EtOAc:hexanes 3:1 yielding compound **2N** as a colorless oil (7g, 81.7%)LCMS (MH, 306) Retention time = 2.08 minutes.

### Step 2: Preparation of 1-Benzyl-3-(1-hydroxy-ethyl)-pyrrolidine-3-carboxylic acid methyl ester (3N)

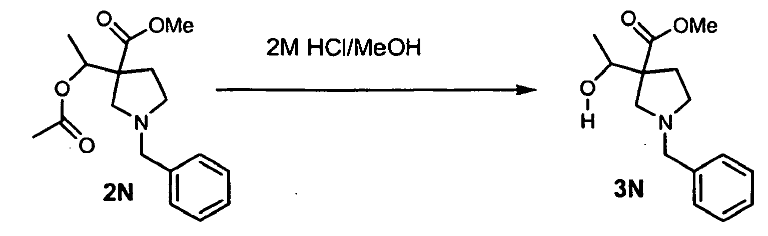

2M HCl (25ml) was added to a solution of 3-(1-Acetoxy-ethyl)-1-benzyl-pyrrolidine-3-carboxylic acid methyl ester (**2N**) (5g, 16.39mmol) in MeOH (50ml) at room temperature, then refluxed for 2 hours. The solvent was evaporated yielding the product (4.3g, 100%) ESMS (MH,264).

### Step 3: Preparation of 1-Benzyl-3-(1-hydroxy-ethyl)-pyrrolidine-3-carboxylic acid,Lithium salt (4N)

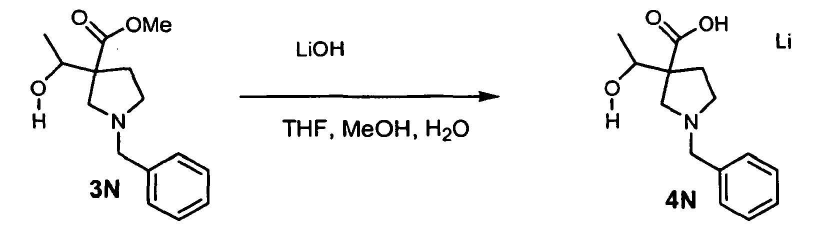

Lithium Hydroxide monohydrate (0.95g,16.37mmol) was added to a solution of 1-Benzyl-3-(1-hydroxy-ethyl)-pyrrolidine-3-carboxylic acid methyl ester (**3N**) (4g, 15.20mmol) in MeOH/THF (1/1, 30ml) at room temperature then refluxed for 2 hours. The reaction was cooled and solvent evaporated yielding product (**4N**) as a solid (4g,100%) ESMS (MH, 250).

### Step 4: Preparation of 1-Benzyl-3-(1-hydroxy-ethyl)-pyrrolidine-3-carboxylic acid [3-(4-fluoro-phenyl)-1-trityl-indazol-5-yl]-amide (5N)

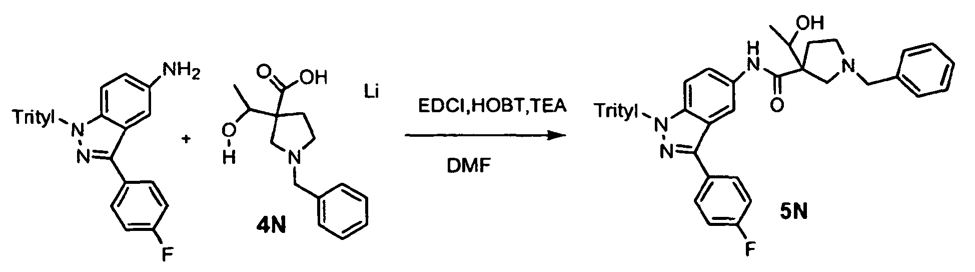

Triethylamine (0.5ml, 6.81 mmol), EDCI (200mg, 1.047mmol) HOBT.H₂O ( 150mg, 1.11mmol) were added to a solution of the indazole (200mg, 0.426mmol) and 1-benzyl-3-(1-hydroxy-ethyl)-pyrrolidine-3-carboxylic acid,Lithium salt (**4N**) (200mg, 0.803mmol) in DMF (3ml) at room temperature, then stirred overnight at ambient temperature. The solvent was evaporated and the residue chromatographed on silica gel eluting with 1:1 v:v EtOAc: Hexanes yielding product **5N** (80mg, 27%) ESMS (MH, 701).

### Step 5: Preparation of 3-(1-Hydroxy-ethyl)-pyrrolidine-3-carboxylic acid [3-(4-fluoro-phenyl)-1Trityl-indazol-5-yl]-amide (6N)

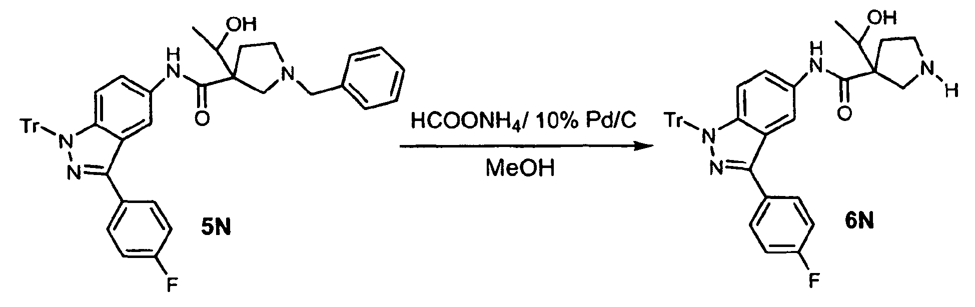

Added ammonium formate (100mg,1.58mmol) to a suspension of 1-Benzyl-3-(1-hydroxy-ethyl)-pyrrolidine-3-carboxylic acid [3-(4-fluoro-phenyl)-1-trityl-indazol-5-yl]-amide (**5N**) (80mg, 0.114mmol) and 10% Pd/C (5mg) in MeOH (5ml) at room temperature then refluxed for 4 hours. The reaction was cooled,diluted with MeOH (20ml) and filtered through a celite pad. The filtrate was concentrated and the residue dissolved in MeCl₂ (40ml), dried over MgSO₄, filtered and solvent evaporated yielding **6N** as a solid (60mg, 87%) ESMS (MH, 611).

### Step 6: Preparation of 3-(1-Hydroxy-ethyl)-1-{2-oxo-2-[4-(4-pyrimidin-2-yl-phenyl)-piperazin-1-yl]-ethyl}-pyrrolidine-3-carboxylic acid [3-(4-fluoro-phenyl)-1Trityl-indazol-5-yl]-amide (7N)

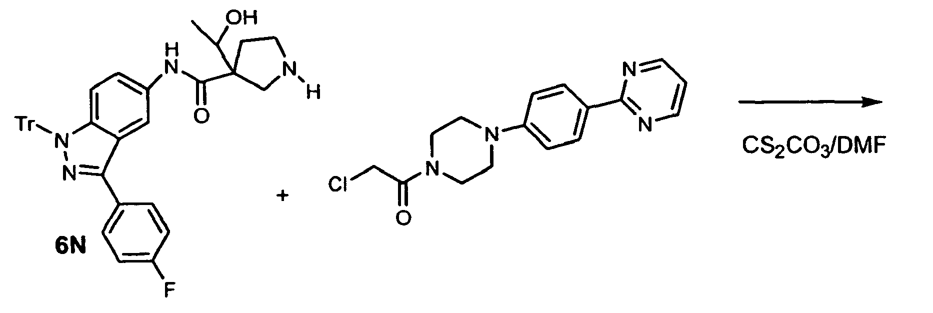

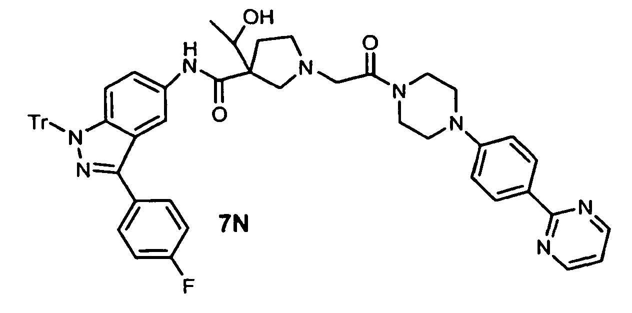

Added Cesium carbonate (60mg, 0.184mmol) to a solution of 3-(1-Hydroxyethyl)-pyrrolidine-3-carboxylic acid [3-(4-fluoro-phenyl)-1Trityl-indazol-5-yl]-amide (**6N**) (60mg, 0.098mmol)and 2-chloro-1-[4-(4-pyrimidin-2-yl-phenyl)-piperazin-1-yl]-ethanone (50mg,0.158mmol) in DMF (2ml) at room temperature, then stirred overnight.

The reaction mixture was diluted with Ether (30ml), insoluble solids filtered, and the solvent was evaporated.The residue was chromatographed on silica gel eluting with 7% MeOH/MeCl₂ yielding **7N** as a white solid (65mg, 75%) LCMS (MH, 891).Retention time = 4.42 minutes.

### Step 7: Preparation of 3-(1-Hydroxy-ethyl)-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (1N)

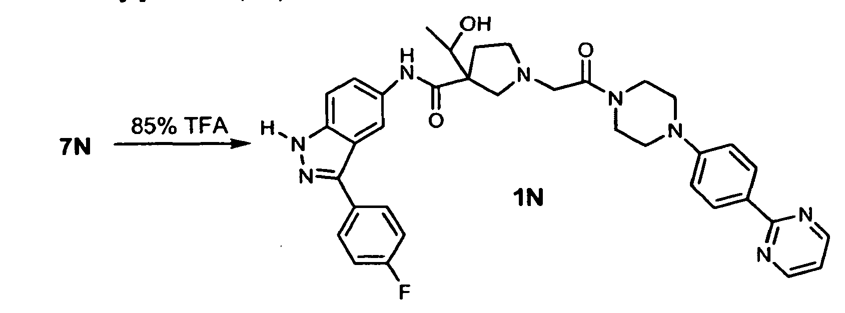

Stirred 3-(1-Hydroxy-ethyl)-1-{2-oxo-2-[4-(4-pyrimidin-2-yl-phenyl)-piperazin-1-yl]-ethyl}-pyrrolidine-3-carboxylic acid [3-(4-fluoro-phenyl)-1Trityl-indazol-5-yl]-amide (**7N**) (65mg, 0.073mmol) in 85% TFA (2ml) at room temperature overnight. Evaporated solvent. Added EtOAc (50ml) H₂O (20ml) 2M NaOH (3ml). Separated organic layer,dried over MgSO₄, filtered and solvent evaporated yielding a residue which purified on silica gel eluting with 5% v/v MeOH:EtOAc yielding two diastereomeric racemates.
Diastereomer A (20mg,42%) LCMS (MH,649) Retention time = 2.70 minutes
Diastereomer B (18mg, 38%)LCMS (MH,649) Retention time = 2.68 minutes.

### EXAMPLE 355

### Step 1: Preparation of 3-(1-Hydroxy-ethyl)-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1Trityl-indazol-5-yl]-amide (9N)

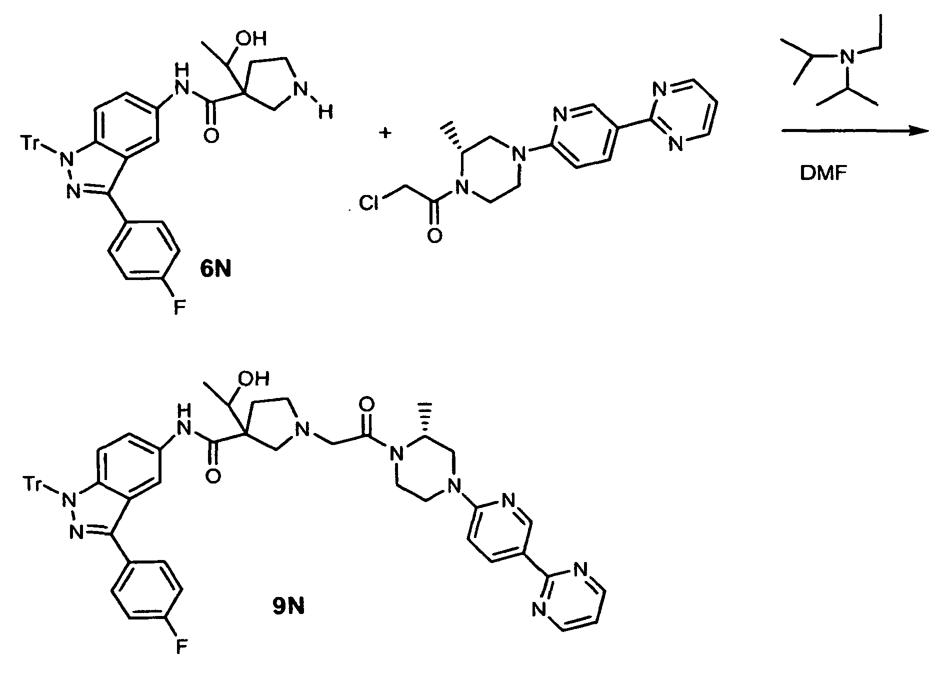

Added N-N- Diisopropylethylamine (0.3ml,1.72mmol) to a mixture of 3-(1-Hydroxy-ethyl)-pyrrolidine-3-carboxylic acid [3-(4-fluoro-phenyl)-1Trityl-indazol-5-yl]-amide (**6N**) (600mg,0.98mmol) and 2-Chloro-1-[2-methyl-4-(5-pyrimidin-2-yl-pyridin-2-yl)-piperazin-1-yl]-ethanone (360mg, 1.15mmol) in DMF (10ml) at room temperature.The solvent was evaporated and the residue extracted with MeCl₂ (100ml) and H₂O (50ml). The organic layer was separated,washed with saturated NaCl solution (20ml), dried over MgSO₄, filtered and solvent evaporated yielding **9N** as a solid (750mg,84%) ESMS (MH,906).

### Step 2: Preparation of 3-Acetyl-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1Trityl-indazol-5-yl]-amide (10N)

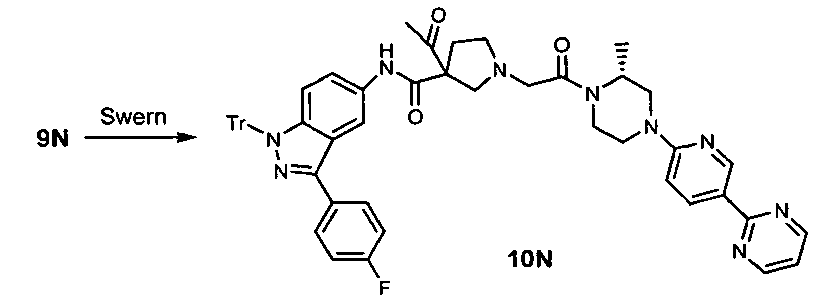

Added DMSO (200mg,2.56mmol) to a solution of oxalyl chloride (180mg,1.40mmol) in MeCl₂ (10ml) at -78°C, then stirred at -78°C for 1 hour. Added 3-(1-Hydroxy-ethyl)-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1Trityl-indazol-5-yl]-amide (**9N**) in MeCl₂ (5ml) at -78°C then stirred at -78°C for 2 hours. Triethylamine (200mg,1.96mmol) was added, the solution allowed warm to room temperature then stirred overnight. Added water (50ml) and MeCl₂ (150ml). The organic layer was separated,dried over MgSO₄, filtered and solvent evaporated yielding **10N** as a solid (600mg,66%) ESMS (MH,904)

### Step 3: Preparation of 3-Acetyl-1-{2-[2-methyl-4-(5-pyrimidin-2-yl-pyridin-2-yl)-piperazin-1-yl]-2-oxo-ethyl-pyrrolidine-3-carboxylic acid [3-(4-fluoro-phenyl)-1H-indazol-5-yl]-amide (11N)

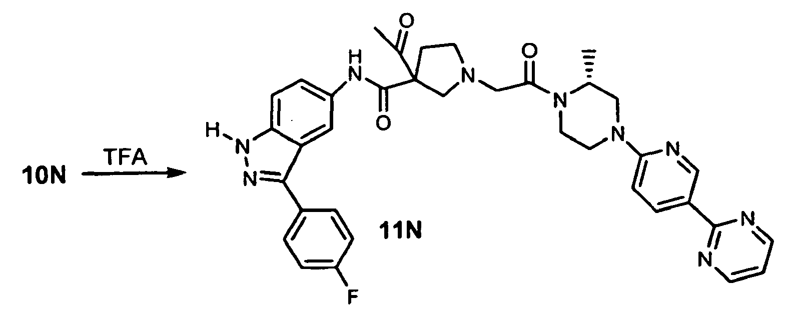

Stirred a solution of 3-Acetyl-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1Trityl-indazol-5-yl]-amide (**10N**) (600mg, 0.664mmol) in trifluoroacetic acid (20ml) at room temperature overnight. The solvent was evaporated, water (100ml) and IN NaOH (10ml) were added. The mixture was extracted with MeCl₂ (2x150ml), dried over MgSO₄, filtered and solvent evaporated yielding a residue which chromatographed on silica gel eluting with 10% v/v MeOH/MeCl₂ containing 2% NH₄OH yielding product as a white solid. (350mg, 79%) LCMS (MH 662.4). Retention time (minutes) 2.21

### EXAMPLE 356

### Preparation of 3-(1-Methoxyimino-ethyl)-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1H-indazol-5-yl]-amide (12N)

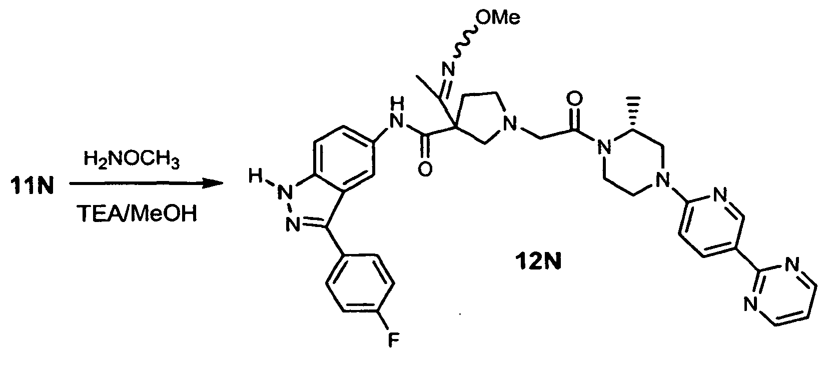

Added methoxylamine hydrochloride (100mg,1.197mmol) and triethylamine(0.2ml, 1.43mmol) to a solution of 3-Acetyl-1-{2-[2-methyl-4-(5-pyrimidin-2-yl-pyridin-2-yl)-piperazin-1-yl]-2-oxo-ethyl}-pyrrolidine-3-carboxylic acid [3-(4-fluorophenyl)-1H-indazol-5-yl]-amide (**11N**) (100mg,0.15mmol) in MeOH (5ml), then stirred for 3 hours at room temperature. The solvent was evaporated and the residue was extracted with MeCl₂, washed with water,dried over Na₂SO₄, filtered and solvent was evaporated yielding a solid which chromatographed on silica gel eluting with 7%v/v MeOH/MeCl₂ containing 2% NH₄OH yielding 2 isomers:
(6mg, 6%) (Z)-Isomer LCMS (MH, 691.4) Retention time = 2.45 minutes.
(60mg, 63%) (E)-Isomer LCMS (MH, 691.4) Retention time = 2.62 minutes.

### EXAMPLE 357

### Preparation of 3-(1-Hydroxyimino-ethyl)-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1H-indazol-5-yl]-amide (13N)

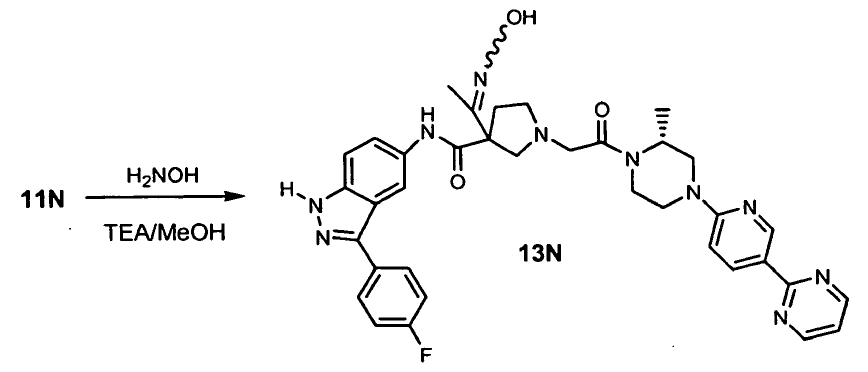

Following essentially the same procedure as described in Example 356, except substituting an equivalent amount of hydroxylamine hydrochloride for methoxylamine hydrochloride, the title compound **13N** was obtained as a single isomer (E) in 70% yield. (E-Isomer) LCMS (MH 677) Retention time = 2.44 minutes.

### EXAMPLE 358

### Preparation of 3-[1-(Acetyl-hydrazono)-ethyl]-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1H-indazol-5-yl]-amide (14N)

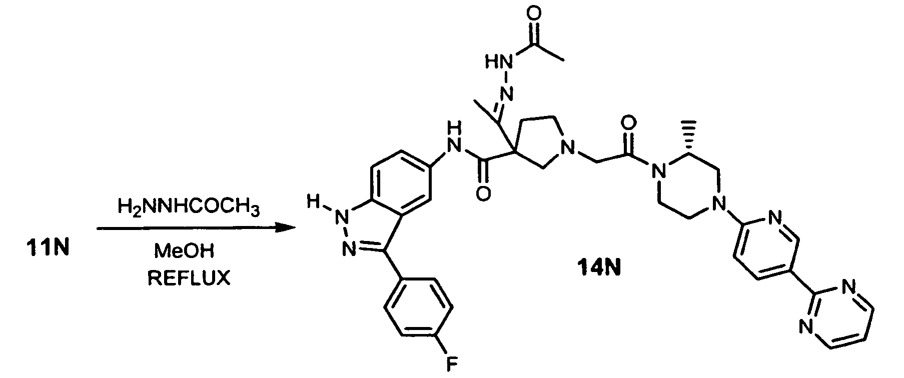

Added acetyl hydrazide (50mg,0.674mmol) to a solution of 3-Acetyl-1-{2-[2-methyl-4-(5-pyrimidin-2-yl-pyridin-2-yl)-piperazin-1-yl]-2-oxo-ethyl}-pyrrolidine-3-carboxylic acid [3-(4-fluoro-phenyl)-1H-indazol-5-yl]-amide (**11N**) (50mg, 0.075mmol) in MeOH (5ml) at room temperature, then refluxed overnight. The reaction was cooled and solvent evaporated.The residue was extracted with MeCl₂ (100ml) and H₂O (40ml). The organic layer separated,dried over MgSO₄, filtered and solvent evaporated.The residue was chromatographed on silica gel eluting with 5% v/v MeOH/MeCl₂ containing 2% NH₄OH yielding title compound **14N** as a white solid (50mg, 92%) LCMS (MH 718.4) Retention time = 2.13 minutes.

### EXAMPLE 359

### Preparation of 3-Amino-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide

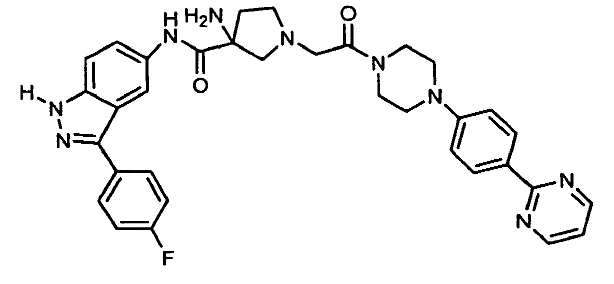

### Step 1: Preparation of 3-Amino-3-[3-(4-fluoro-phenyl)-1trityl-indazol-5-ylcarbamoyl]-pyrrolidine-1-carboxylic acid tert-butyl ester (1)

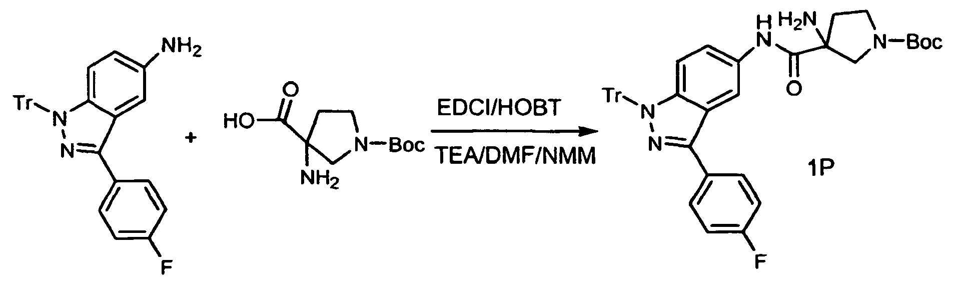

Added 3-Amino-pyrrolidine-1,3-dicarboxylic acid-1-tert-butyl ester (86mg, 0.373mmol) to a solution of 3-(4-Fluoro-phenyl)-1H-indazol-5-ylamine (178mg, 0.379mmol); EDCl.HCl (150mg, 0.785mmol) and HOBT (100mg, 0.740mmol) in DMF (2ml) at room temperature. NMM (0.1 ml) was added and solution stirred overnight.The solvent was evaporated and the residue extracted with MeCl₂ (50ml) washed with H₂O (25ml). The organic layer was dried over MgSO₄ ,filtered and solvent evaporated yielding a residue which chromatographed on silica gel eluting with 3% v/v MeOH/MeCl₂ yielding title compound **1P** (185mg, 71%) ESMS (MH 682).

### Step 2: Preparation of 3-Amino-pyrrolidine-3-carboxylic acid [3-(4-fluoro-phenyl)-1H-indazol-5-yl]-amide,Hydrochloride (2P)

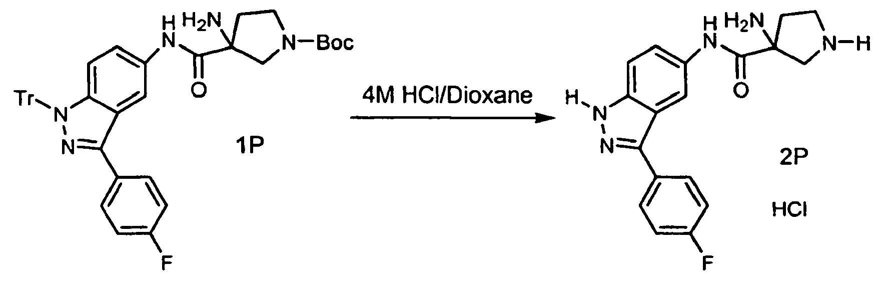

4M HCl/dioxane (2ml) was added to a solution of 3-Amino-3-[3-(4-fluoro-phenyl)-1H-indazol-5-ylcarbamoyl]-pyrrolidine-1-carboxylic acid tert-butyl ester (**1P**) (60mg, 0.088mmol) in MeCl₂ (1ml) at room temperature then stirred for 1 hour at room temperature. The solvent was evaporated. Hexanes (20ml) was added and supernatant decanted.The residue was dried yielding title compound **2P** as a white solid (20mg, 69%). ESMS (MH 340).

### Step 3: Preparation of 3-Amino-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (3P)

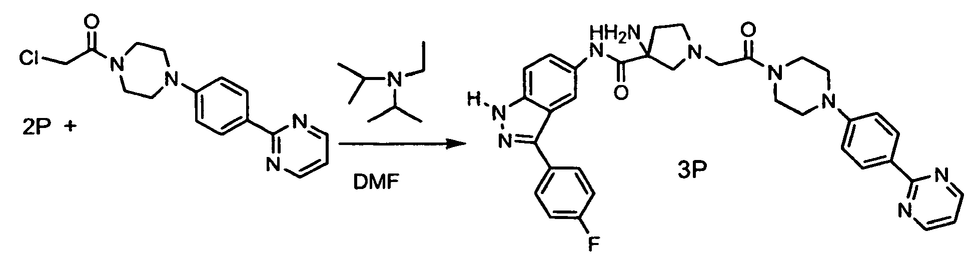

Added N,N-Diisopropylethylamine (50mg, 0.387mmol) to a solution of 3-Amino-pyrrolidine-3-carboxylic acid [3-(4-fluoro-phenyl)-1H-indazol-5-yl]-amide, Hydrochloride (**2P**) (15mg, 0.036mmol) in DMF (1ml) and the solution stirred overnight. The solvent was evaporated and the residue extracted with MeCl₂ (50ml) and H₂O (20ml). The organic layer was separated,dried over MgSO₄, filtered and evaporated solvent yielding a residue which chromatographed on silica gel eluting with v/v 5% MeOH/MeCl₂ containing 2% NH₄OH yielding title compound **3P** as a white solid (10mg,45%) LCMS (MH 620.3) Retention time = 2.78 minutes

### EXAMPLE 360

### Step 1: Preparation of 3-[3-(4-Fluoro-phenyl)-1Trityl-indazol-5-ylcarbamoyl]-3-formylamino-pyrrolidine-1-carboxylic acid tert-butyl ester (4P)

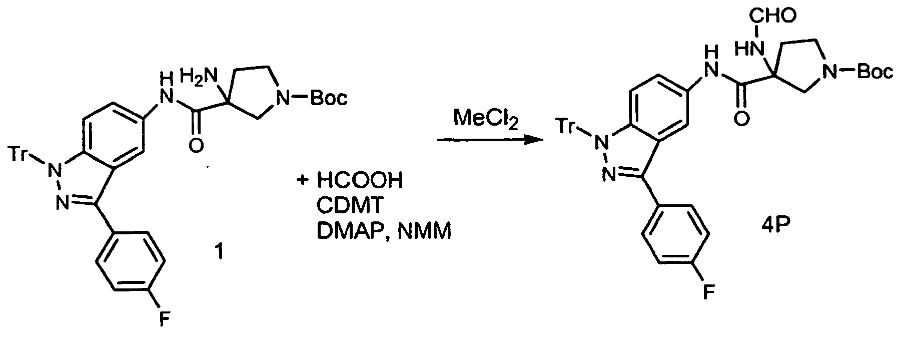

Formic acid (17mg, 0.36mmol) was added to a solution of 3-Amino-3-[3-(4-fluoro-phenyl)-1trityl-indazol-5-ylcarbamoyl]-pyrrolidine-1-carboxylic acid tert-butyl ester (**1P**) 250mg, 0.367mmol); 2-Chloro-4,6-dimethoxy-1,3,5-triazine (77mg, 0.44mmol); DMAP (10mg) and NMM (50mg, 0.49mmol) in MeCl₂ (3ml) at room temperature.The solvent was evaporated and residue chromatographed on silica gel eluting with 3% v/v MeOH/MeCl₂ yielding **4P** as a white solid (210mg,80%) ESMS (MH 710).

### Step 2: Preparation of 3-Formylamino-pyrrolidine-3-carboxylic acid [3-(4-fluorophenyl)-1H-indazol-5-yl]-amide (5P)

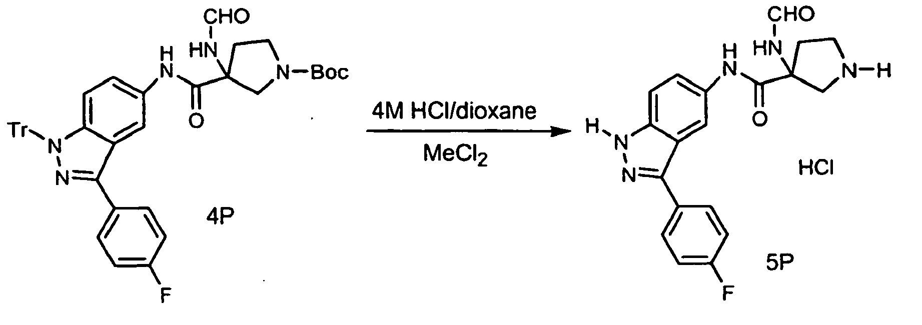

4M HCl (2ml) was added to a solution of 3-[3-(4-Fluoro-phenyl)-1Trityl-indazol-5-ylcarbamoyl]-3-formylamino-pyrrolidine-1-carboxylic acid tert-butyl ester (**4P**) 0.141 (mmol) in MeCl₂ (2ml) at room temperature, then stirred for 1 hour. The solvent was evaporated,hexanes added to residue and supernatant decanted. The residual solid was dried yielding **5P** as a white solid (60mg, 100%) LCMS (MH,368.2) Retention time = 1.91 minutes.

### Step 3: Preparation of 3-Formylamino-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (6P)

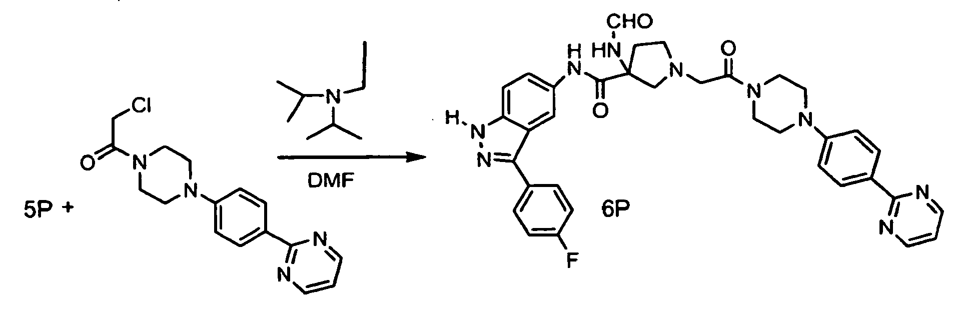

Added N,N-Diisopropylethylamine (50mg,0.387mmol) to a solution of 3-Formylamino-pyrrolidine-3-carboxylic acid [3-(4-fluoro-phenyl)-1H-indazol-5-yl]-amide (**5P**) (60mg, 0.0148mmol) in DMF (2ml) and the solution stirred overnight. The solvent was evaporated and the residue extracted with MeCl₂ (50ml) and H₂O (20ml). The organic layer was separated,dried over MgSO₄, filtered and evaporated solvent yielding a residue which chromatographed on silica gel eluting with v/v 5% MeOH/MeCl₂ containing 2% NH₄OH yielding title compound **6P** as a white solid (60mg, 63%) LCMS (MH 648.4) Retention time = 2.81 minutes.

### EXAMPLE 361

### Preparation of 3-Amino-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (7P)

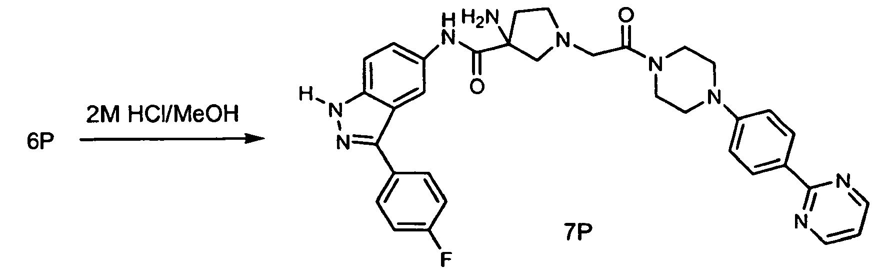

Added 2M HCl (2ml) to a solution of 3-Formylamino-1-{2-oxo-2-[4-(4-pyrimidin-2-yl-phenyl)-piperazin-1-yl]-ethyl}-pyrrolidine-3-carboxylic acid [3-(4-fluoro-phenyl)-1H-indazol-5-yl]-amide (**6P**) (10mg, 0.015mmol) in MeOH (2ml) at room temperature,then stirred for 4days at room temperature. The reaction mixture was diluted with Water (20ml), basified with 1N NaOH (3ml) and extracted with MeCl₂ (3x50ml). The organic layers were combined, dried over MgSO₄, filtered and solvent evaporated yielding **7P** as a white solid (7mg, 73%) LCMS (MH 620.3) Retention time = 2.78 minutes.

### EXAMPLE 362

### Preparation of 3-Formylamino-1-(2-{2-methyl-4-[4-(5-methyl-pyrimidin-2-yl)-phenyl]-piperazin-1-yl}-2-oxo-ethyl)-pyrrolidine-3-carboxylic acid [3-(4-fluorophenyl)-1H-indazol-5-yl]-amide (8P)

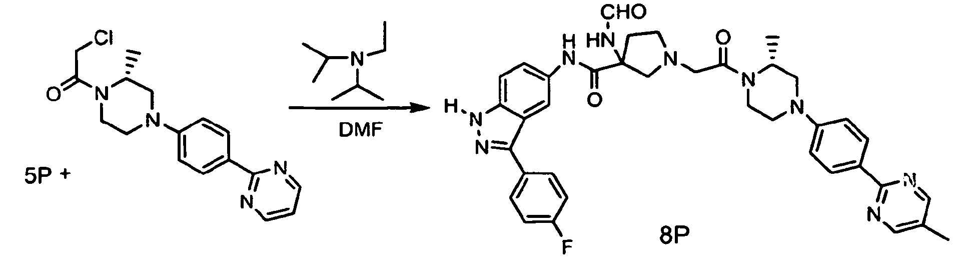

Following essentially the same procedure as described in Step 3 of Example 360 except substituting 2-Chloro-1-[4-(4-pyrimidin-2-yl-phenyl)-piperazin-1-yl]-ethanone with an equivalent quantity of 2-Chloro-1-[2-methyl-4-(4-pyrimidin-2-yl-phenyl)-piperazin-1-yl]-ethanone, the title compound **8P** was obtained. Chromatography on silica gel eluting with 5% v/v MeOH/MeCl₂ containing 2% NH₄OH yields **8P** as a white solid (55mg,55%). LCMS (MH 676.4) Retention time = 2.96 minutes. The product is a mixture of 2 Isomers.

Compound **8P** was separated into single isomers on Chiral HPLC (AD Column) Analytical column (Chiralpak AD 4.6x250). 40% IPA/Hexanes containing 0.2% DEA:
Peak A (isomer A) eluted at 24.501 minutes. LCMS (MH 676.4). Retention time (minutes) 2.96 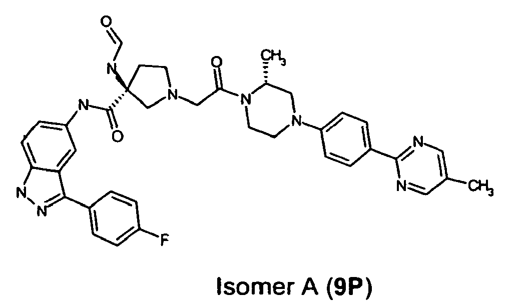
Peak B (isomer B) eluted at 33.036 minutes LCMS (MH 676.4) (**10P**)

### EXAMPLE 363

### Preparation of 3-Amino-1-(2-{2-methyl-4-[4-(5-methyl-pyrimidin-2-yl)-phenyl]-piperazin-1-yl}-2-oxo-ethyl)-pyrrolidine-3-carboxylic acid [3-(4-fluoro-phenyl)-1H-indazol-5-yl]-amide (11P)

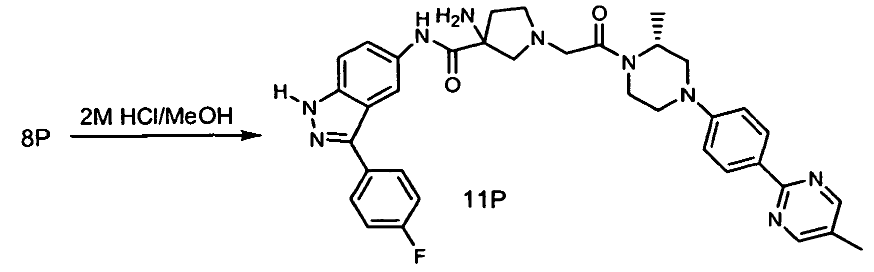

Following essentially the same procedure as Example 361 except substituting compound **6P** with an equivalent quantity of **8P**, the title compound **11P** was obtained as a mixture of 2 isomers. LCMS (MH 648) Retention time = 2.90 minutes.

### EXAMPLE 364

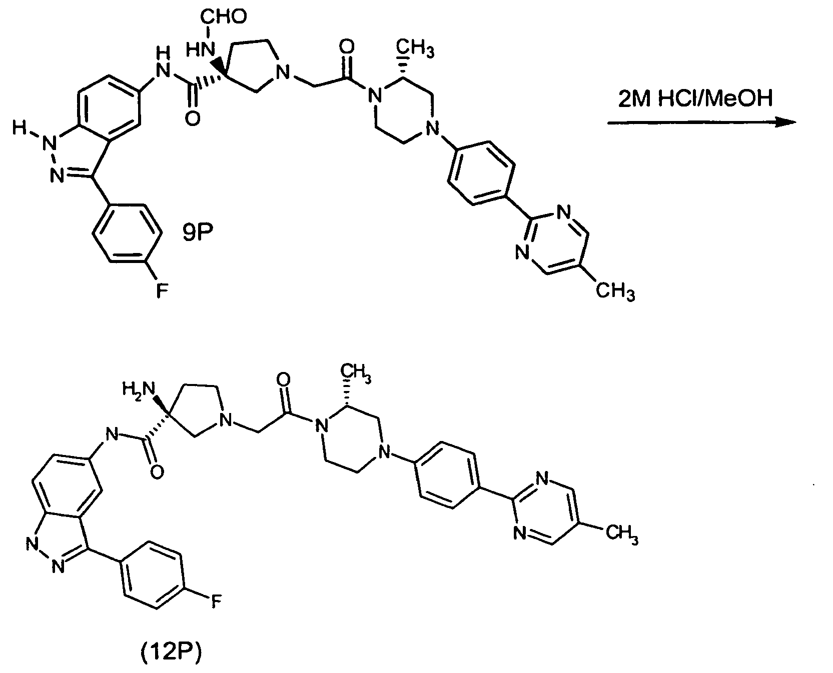

Following essentially the same procedure as Example 361 except substituting compound 6P with an equivalent quantity of compound **9P** (isomer A, Example 372), the title compound **12P** was obtained as a single isomer. LCMS (MH 648.4) Retention time = 2.90 minutes.

### Preparation 35

### Step 1: Preparation of 5-Pyrimidin-2-yl-3',6'-dihydro-2'H-[2,4']bipyridinyl-1'-carboxylic acid tert-butyl ester (2Q)

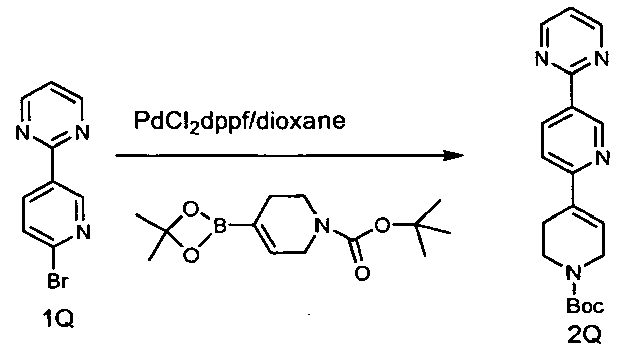

Refluxed mixture of 2-(6-Bromo-pyridin-3-yl)-pyrimidine (**1Q**) (200mg, 0.85mmol), N-tert-butoxycarbonyl-1,2,3,6-tetrahydropyridine-4-boronic acid, pinacol ester (290mg, 0.93mmol); Cesium Carbonate (500mg, 1.538mmol); PdCl₂dppf (30mg) in dioxane/H₂O (10ml v/v 4/1) for 4 hours. Cooled reaction, then evaporated solvent. Extracted with EtOAc (200ml) washed with H₂O (50ml), dried over MgSO₄, filtered and solvent evaporated yielding a solid which chromatographed on silica gel eluting with 30% v/v acetone/hexanes yielding **2Q** as a white solid (110mg, 38%) ESMS (MH,339).

### Step 2: Preparation of 5-Pyrimidin-2-yl-1',2',3',6'-tetrahydro-[2,4']bipyridinyl (3Q)

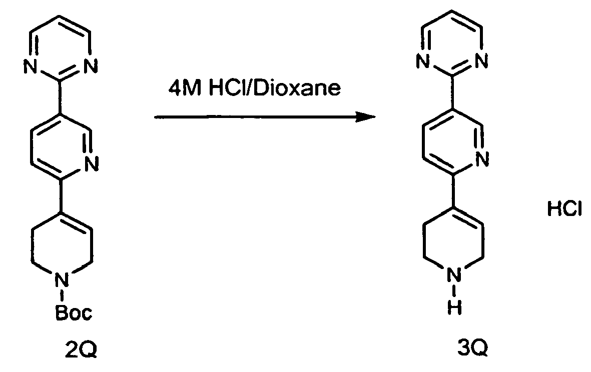

Added 4M HCl/dioxane (5ml) to solution of 5-Pyrimidin-2-yl-3',6'-dihydro-2'H-[2,4']bipyridinyl-1'-carboxylic acid tert-butyl ester (**2Q**) (110mg, 0.325mmol) in MeCl₂ (5ml) at room temperature, then stirred 4 hours. Evaporated solvent. Added MeCl₂ (100ml), H₂O (50ml) and 10% NaOH (3ml). The organic layer was separated, dried over MgSO₄, filtered and solvent evaporated yielding **3Q** as a white solid (90mg, 100%) ESMS (MH, 239) LCMS (MH, 239) Retention time = 1.53 minutes.

### Step 3: Preparation of 2-Chloro-1-(5-pyrimidin-2-yl-3',6'-dihydro-2'H-[2,4']bipyridinyl-1'-yl)-ethanone (4Q)

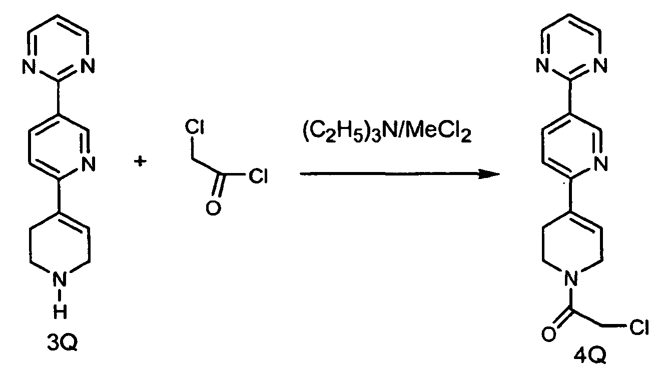

Added chloroacetyl chloride (0.35g, 4.39mmol) in MeCl₂ (15ml) to a solution of 5-Pyrimidin-2-yl-1',2',3',6'-tetrahydro-[2,4']bipyridinyl (**3Q**) (0.4g, 1.68mmol) and triethylamine (0.4g, 2.87mmol) in MeCl₂ (10ml) at 0°C, then stirred 2 hours at 0°C. Added saturated NaHCO₃ solution and stirred an additional hour at 0°C. MeCl₂ (100ml) was added, organic layer separated, dried over Na₂SO₄, filtered and solvent evaporated yielding **4Q** as a pale yellow solid (0.53g, 100%) ESMS (MH 315).

### EXAMPLE 365

### Step 1: Preparation of 3-Amino-pyrrolidine-1,3-dicarboxylic acid 1-tert-butyl ester 3-methyl ester (1T)

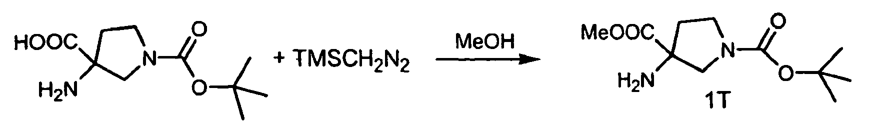

Added (Trimethylsilyl)-diazomethane (2M in hexanes: 15ml, 30mmol) to a solution of 3-Amino pyrrolidine 1,3 dicarboxylic acid,1-tert-butyl ester (900mg, 3.9mmol) in MeOH (5ml) at room temperature. Stirred 10 minutes then evaporated solvent yielding **1T** as an oil (0.9g,98%).

### Step 2: Preparation of 3-Formylamino-pyrrolidine-1,3-dicarboxylic acid 1-tert-butyl ester 3-methyl ester (2T)

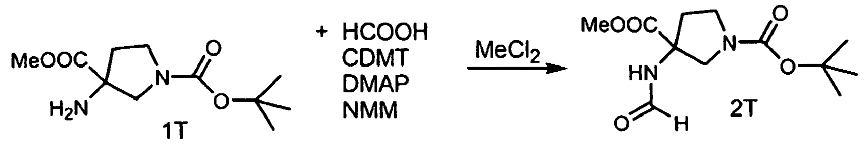

Added formic acid (200mg,4.34mmol) to solution of 3-Amino-pyrrolidine-1,3-dicarboxylic acid 1-tert-butyl ester 3-methyl ester (**1T**) (900mg,3.688mmol); 2-Chloro-4,6 dimethoxy-1,3,5-Triazine (CDMT) (800mg,4.556mmol); DMAP (20mg), and NMM (400mg, 4mmol) at room temperature, then stirred overnight. The solvent was evaporated and the residue extracted with MeCl₂ (200ml), washed with H₂O (50ml), dried over MgSO₄, filtered and solvent evaporated yielding a solid which chromatographed on silica gel eluting with 5% MeOH/MeCl₂ containing 2% NH₄OH yielding 2T as a white solid (725mg, 72%) LCMS (MH 273) Retention time = 2.53 minutes.

### Step 3: Preparation of 3-(Formyl-methyl-amino)-pyrrolidine-1,3-dicarboxylic acid 1-tert-butyl ester 3-methyl ester (3T)

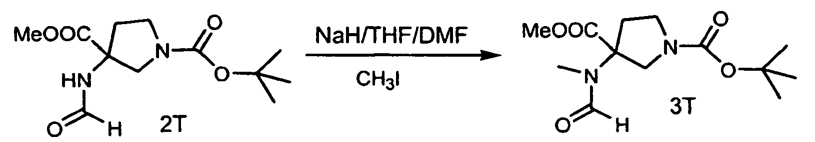

Sodium hydride (60% in oil) (10mg, 0.25mmol) was added to a solution of 3-Formylamino-pyrrolidine-1,3-dicarboxylic acid 1-tert-butyl ester 3-methyl ester (**2T**) (50mg, 0.183mmol) in THF (3ml). DMF (1ml) was added and stirred for 1 hour. Methyl iodide (0.1 ml, 1.60mmol) was added and the reaction was stirred overnight. The solvent was evaporated and the residue extracted with EtOAc (50ml), washed with H₂O (20ml), dried over MgSO₄, filtered and solvent evaporated yielding a residue which chromatographed on silica gel eluting with 3% MeOH/MeCl₂ yielding **3T** (50mg, 94%). LCMS (MH 287) Retention time = 2.77 minutes.

### Step 4: Preparation of 3-(Formyl-methyl-amino)-pyrrolidine-3-carboxylic acid methyl ester, Hydrochloride.(4T)

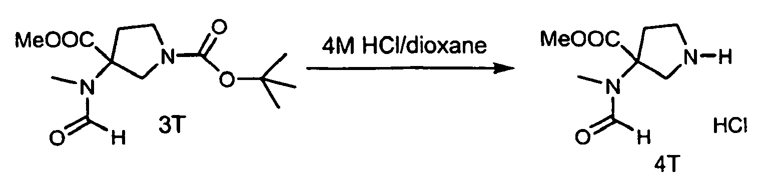

Added 4M HCl/dioxane (2ml) to a solution of 3-(Formyl-methyl-amino)-pyrrolidine-1,3-dicarboxylic acid 1-tert-butyl ester 3-methyl ester (**3T**) (50mg, 0.175mmol) in MeCl₂ (2ml) at room temperature then stirred for 1 hour. The solvent was evaporated yielding **4T** as a white solid (45mg, 100%) ESMS (MH 187).

### Step 5: Preparation of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid methyl ester (5T)

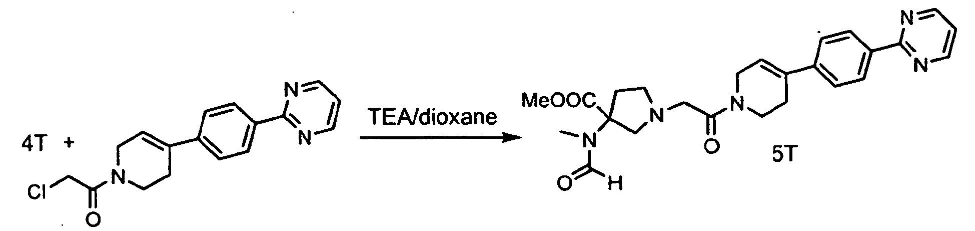

Added triethylamine (0.3ml, 2.15mmol) to a solution of (3-(Formyl-methyl-amino)-pyrrolidine-3-carboxylic acid methyl ester, Hydrochloride.(**4T**) (90mg, 0.405mmol) and 2-Chloro-1-[4-(4-pyrimidin-2-yl-phenyl)-3,6-dihydro-2H-pyridin-1-yl]-ethanone (140mg, 0.447mmol) in dioxane (5ml) at room temperature, then stirred at 90°C for 5 hours. The reaction was cooled and solvent evaporated yielding a residue which chromatographed on silica gel eluting with 7% MeOH/MeCl₂ containing 2% NH₄OH yielding **5T** as a white solid (100mg, 53%) ESMS (MH 464).

### Step 6: Preparation of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid,Lithium salt (6T)

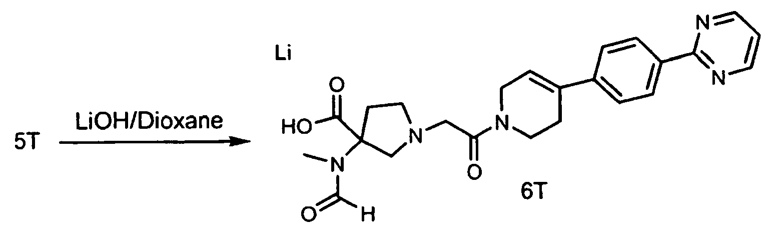

Added Lithium Hydroxide monohydrate (13mg, 0.22mmol) to a solution of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid methyl ester (**5**) (100mg, 0.2159mmol) in dioxane (3ml), then stirred 4 hours at room temperature. The solvent was evaporated yielding **6T** as a white solid (95mg, 98%) LCMS (MH 450.2) Retention time = 2.15 minutes.

### Step 7: Preparation of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1trityl-indazol-5-yl]-amide (7T)

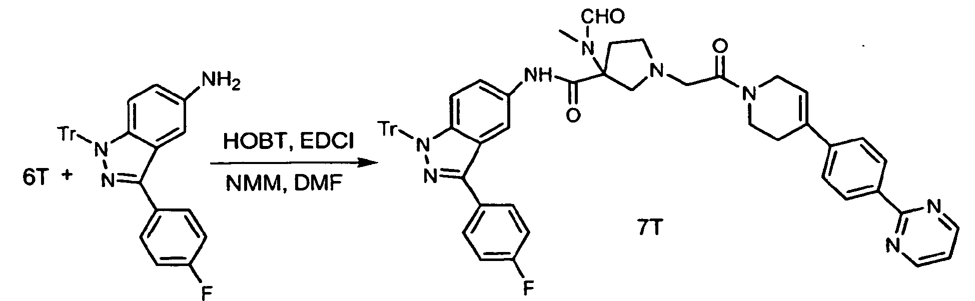

Added EDCl.HCl (75mg, 0.39mmol) and HOBT.H₂O (50mg, 0.37mmol) to a solution of 3-(4-Fluoro-phenyl)-1trityl-indazol-5-ylamine (110mg, 0.234mmol) and 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid,Lithium salt (**6T**) (95mg, 0.211mmol) in DMF (2ml) and NMM (0.1 ml) at room temperature, then stirred overnight. The solvent was evaporated and residue extracted with MeCl₂ (50ml), washed with H₂O (20ml), dried over MgSO₄, filtered and solvent evaporated yielding a residue which chromatographed on silica gel eluting with v/v 9:1 EtOAc: Hexanes yielding **7T** as a white solid (89mg, 44%) ESMS (MH 901).

### Step 8: Preparation of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1H-indazol-5-yl]-amide (8T)

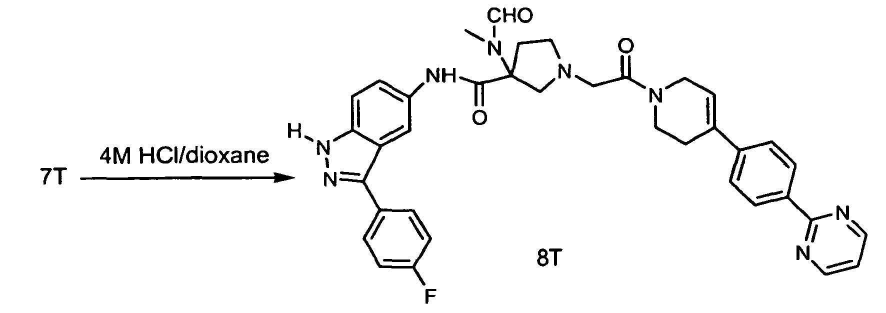

Added 4M HCl/dioxane (2ml) to a solution of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1trityl-indazol-5-yl]-amide (**7T**) (89mg, 0.098mmol) in MeCl₂ (2ml) at room temperature, then stirred for 2 hours. The solvent was evaporated and the residue extracted with MeCl₂ (50ml), H₂O (25ml) and 3M NaOH (2ml). The organic layer was separated, dried over MgSO₄, filtered and solvent evaporated yielding a solid. Hexanes (2x50ml) was added and the supernatant decanted. The residual solid was dried yielding **8T** as a white solid (55mg,84%) LCMS (MH 659) Retention time = 2.98 minutes.

**8T** is a mixture of 2 Enantiomers which separate on a chiralpak AD (4.6x250) column eluting with 60:40 Hex:IPA containing 0.2% DEA.Flow rate = 0.9ml/minute
Peak A elutes at 29.52 minutes.
Peak B elutes at 35.5 minutes.

### EXAMPLE 366

### Preparation of 3-Methylamino-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1H-indazol-5-yl]-amide (9T)

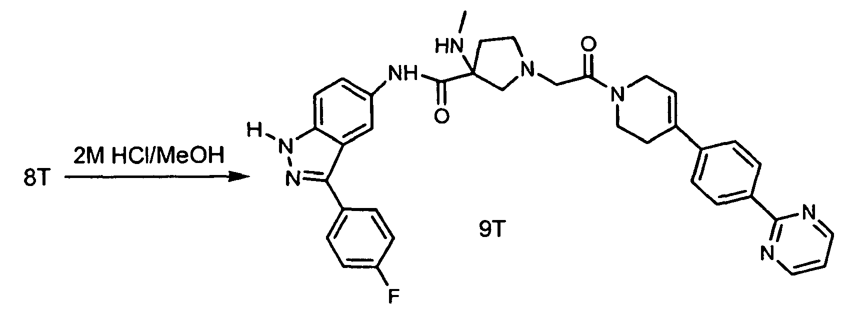

Added 2M HCl (2ml) to a solution of 3-(Formyl-methyl-amino)-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1H-indazol-5-yl]-amide (**8T**) (11mg, 0.017mmol) in MeOH (5ml) at room temperature for 4 days. Evaporated solvent Added H₂O (20ml), basified with IN NaOH (2ml), and extracted with MeCl₂ (50ml). Separated organic layer,dried over MgSO₄, filtered and evaporated solvent,yielding residue which chromatographed on silica gel eluting with 10% MeCl₂/MeOH yielding **9T** (5mg) ESMS (MH 631)

### EXAMPLE 367

### Preparation of 3-Methoxymethyl-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluoro-phenyl)-1H-pyrazolo[4,3-b]pyridin-5-yl]-amide

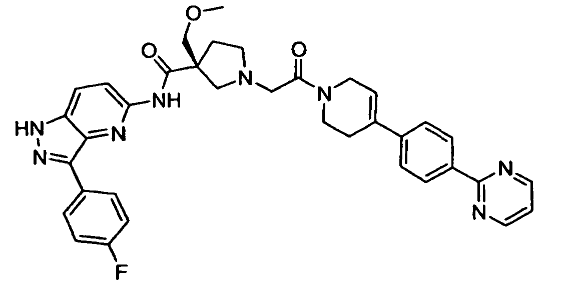

### Step 1: Preparation of 3-bromo-1H-pyrazolo[4,3-b]pyridine

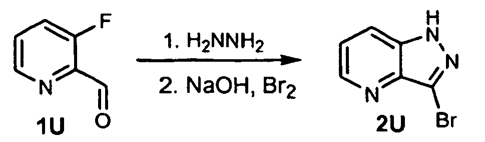

A mixture of 3-fluoro-2-formylpyridine (1.0 g, 8.0 mmol) in 2 mL of anhydrous hydrazine was heated to 110 C and stirred overnight. The reaction was poured to ice water and extracted with ethyl acetate. The combined organic layer was dried and concentrated to give a crude oil (0.5 g), which was subsequently dissolved in 10 mL of NaOH solution (2 N). To this was added bromine (0.6 g, 3.7 mmol) in 5 mL of NaOH solution (2 N) drop-wise. The reaction was stirred at room temperature for 3 hr and quenched by adding NaHSO₃ (0.06 g), then HCl solution (6 mL, 4 N). A solid was precipitated, filtered and air-dried to give **2U** (0.66 g).

### Step 2: Preparation of 3-(4-Fluoro-phenyl)-1H-pyrazolo[4,3-b]pyridine

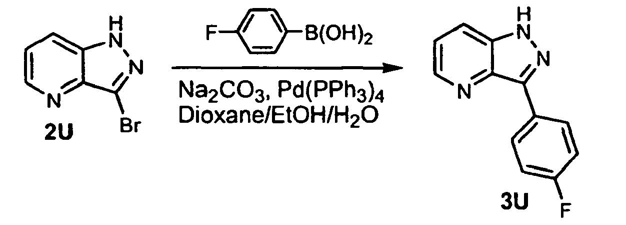

A mixture of **2U** (480 mg, 2.45 mmol), Pd(PPh3)4 (141 mg, 0.122 mmol), 4-fluorophenylboronic acid (412 mg, 2.94 mmol) and sodium carbonate solution (2.4 mL, 2 M, 4.90 mmol) in 5 mL of dioxane/EtOH/H2O (7:3:2) was microwaved at 150 °C for 10 minutes. The mixture was diluted with water and extracted with ethyl acetate. The combined organic layer was dried, concentrated and purified by column chromatography to give **3U** (362 mg).

### Step 3: Preparation of 3-(4-Fluoro-phenyl)-1-trityl-1H-pyrazolo[4,3-b]pyridine 4-oxide

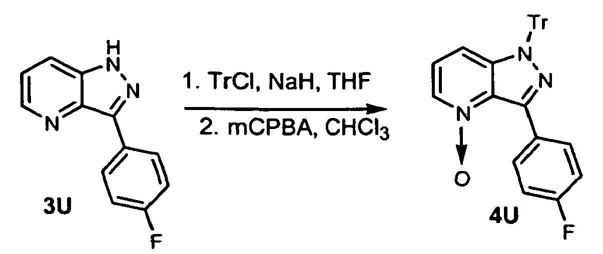

To a solution of **3U** (362 mg, 1.46 mmol) in 8 mL of THF was added NaH (96 mg, 60%, 2.40 mmol) at 0 °C followed by addition of chlorotriphenylmethane (570 mg, 2.04 mmol). The reaction was stirred at room temperature for 1.5 hr and quenched with ammonium chloride solution. The aqueous layer was extracted with ethyl acetate, dried and concentrated to afford crude adduct. To a solution of the trityl adduct (260 mg, 0.57 mmol) in chloroform was added mCPBA (216 mg, 0.86 mmol). The reaction was heated to reflux for 6 hr, diluted with dichloromethane and washed with water. The organic layer was dried, concentrated and purified by column chromatography to give **4U** (169 mg).

### Step 3: Preparation of 3-(4-Fluorophenyl)-1-trityl-1H-pyrazolo[4,3-b]pyridin-5-ylamine

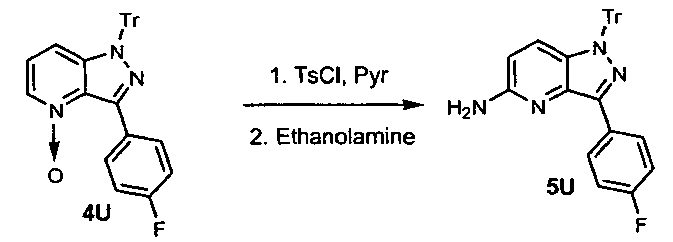

To a solution of **4U** (138 mg, 0.293 mmol) in 2 mL of pyridine was added p-toluenesulfonyl chloride (67 mg, 0.352 mmol). The reaction was stirred at room temperature for 2 hr and the solvent was evaporated. To the crude residue was added 3 mL of ethanol amine. The reaction was stirred at room temperature for 2 hr and poured to ice. The yellow solid was collected by filtration and dried under vacuum to afford **5U** (117 mg).

### Step 4: Preparation of 3-(4-Fluorophenyl)-1H-pyrazolo[4,3-b]pyridin-5-ylamine

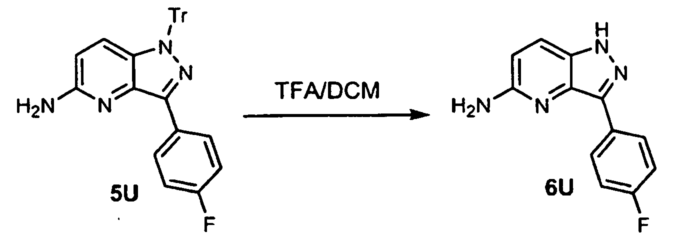

A solution of **5U** (112 mg, 0.238 mmol) in 2 mL of TFA/DCM (1:1) was stirred at room temperature for 1 hr and was concentrated. The residue was purified by column chromatography to give **6U** (48.1 mg).

### Step 5: Preparation of 3-Methoxymethyl-1-{2-oxo-2-[4-(4-pyrimidin-2-yl-phenyl)-3,6-dihydro-2H-pyridin-1-yl]-ethyl}-pyrrolidine-3-carboxylic acid [3-(4-fluorophenyl)-1H-pyrazolo[4,3-b]pyridin-5-yl]-amide

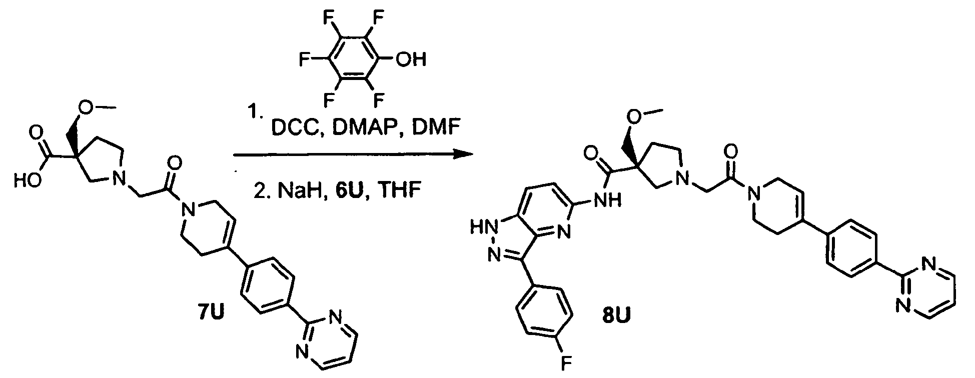

To a solution of **7U** (see, Example 336, 150 mg, 0.283 mmol), pentafluorophenol (61.8 mg, 0.336 mmol) and DMAP (51.3 mg, 0.42 mmol) in DMF was added dicyclohexylcarbodiimide (0.34 mL, 1 M in DCM, 0.336 mmol). The reaction was stirred at room temperature for 4 hr and concentrated. The residue was purified by column chromatography to give the corresponding ester. To a solution of **6U** (62 mg, 0.364 mmol) in THF at 0 °C was added NaH (16.0 mg, 0.437 mmol) and stirred at 0 °C for 0.5 h. To this mixture was added the pentafluorophenyl ester and stirred at room temperature for 1.5 hr. The reaction was quenched by adding ammonium chloride solution. The resulting mixture was concentrated and purified by reverse phase HPLC to afford **8U** (16.9 mg).

### Preparation 36

### Chiral salt resolution of 3-methoxy-pyrrolidine-3-carboxylic acid methyl ester

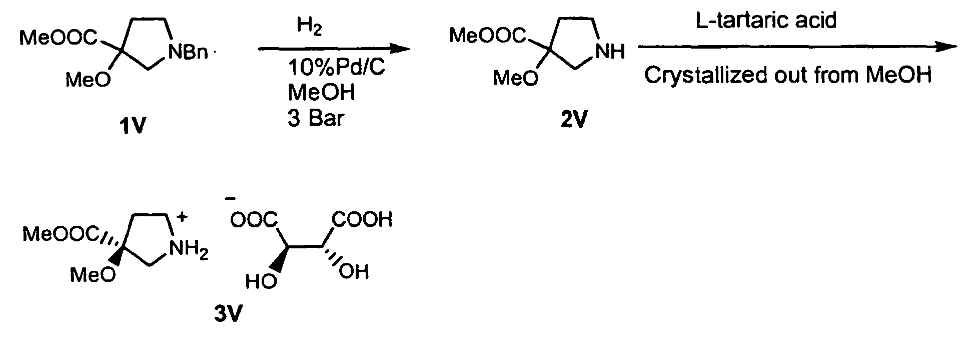

To a solution of compound **1V** (660 mg, 2.65 mmols) in MeOH (30 ml) was charged 10% Pd/C (80 mg). The mixture was hydrogenated at 3 bar overnight using standard Parr apparatus, filtered and washed with MeOH several times.

To the methanol solution (20 ml) containing **2V** (1.32 mmols) was added L-tartaric acid (180 mg, 1.2 mmols), and solid was taken into solution by sonication. Solvent was removed under reduced pressure to a residue to which MeOH (3 ml) was added and the solution was allowed to stand at -20C overnight without disturbing. Crystals formed and MeOH was removed by pipette and crystals were rinsed with MeOH twice. Re-crystallization from MeOH gave 200 mg of complex **3V.**

### EXAMPLES 368-383

Following procedures essentially similar to the examples described above, the compounds in Table 20 are prepared.

**Table 20**

| **Ex** | **Compound** | **M+1** | **Retention time (min)** |
|---|---|---|---|
| 368 | 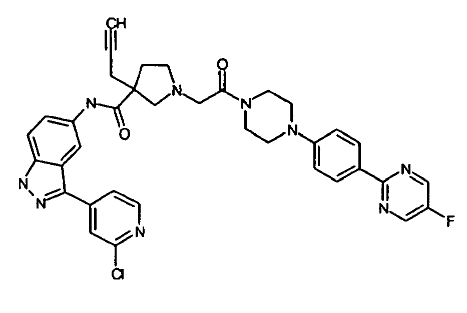 | 678.4 | 3.18 |
| 369 | 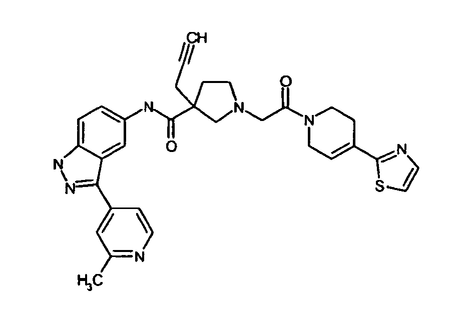 | 566.3 | 1.8 |
| 370 | 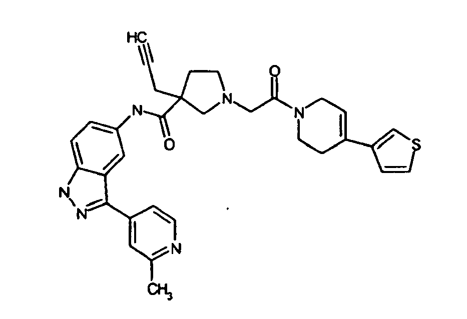 | 565.3 | 2.14 |
| 371 | 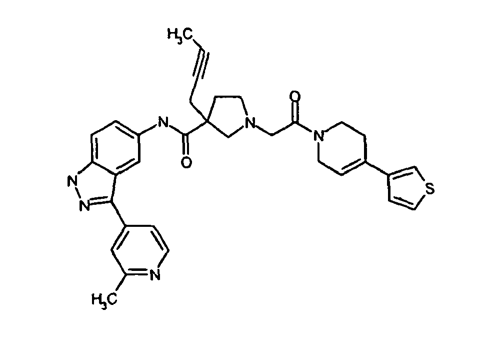 | 579.3 | 2.24 |
| 372 | 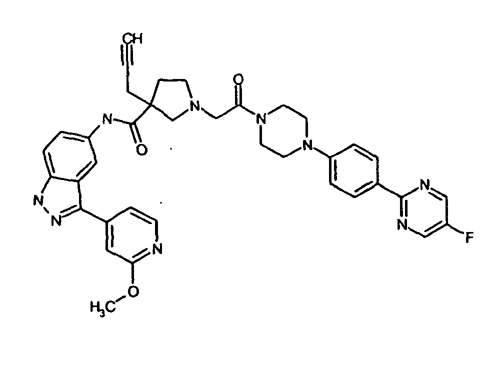 | 674.4 | 2.74 |
| 373 | 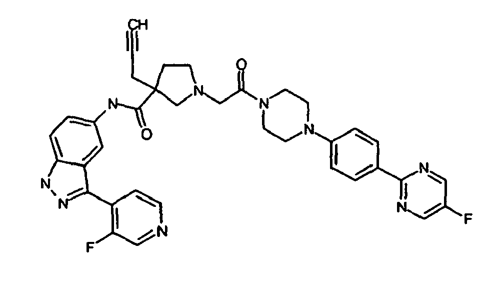 | 662.4 | 2.68 |
| 374 | 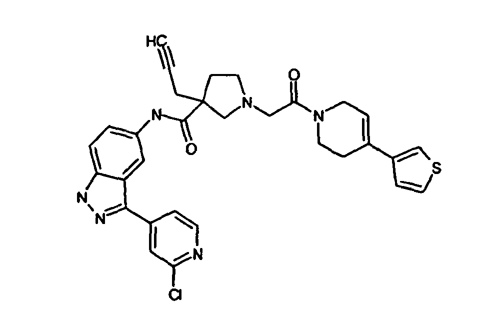 | 585.3 | 2.9 |
| 375 | 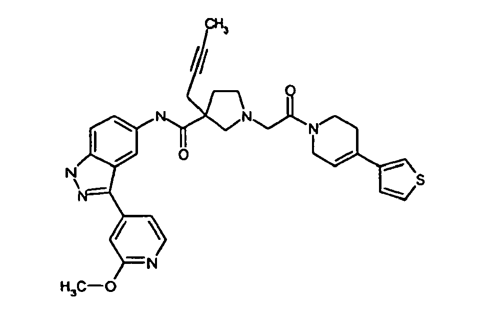 | 595.3 | 2.78 |
| 376 | 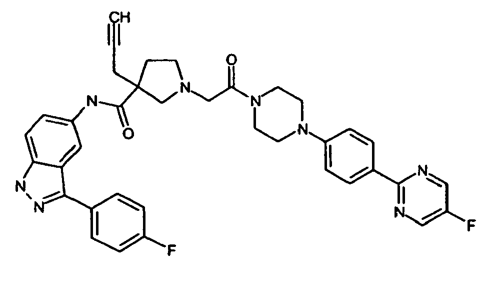 | 661.4 | 3.16 |
| 377 | 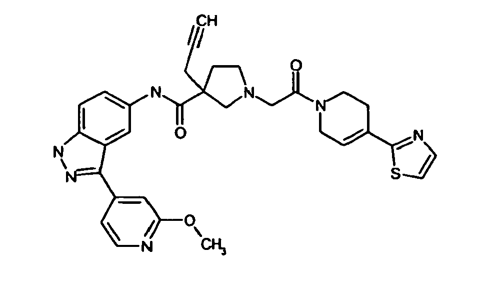 | 582.4 | 2.23 |
| 378 | 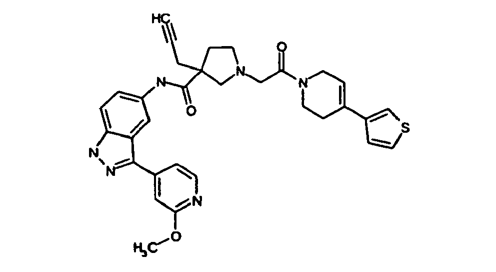 | 581.3 | 2.65 |
| 379 | 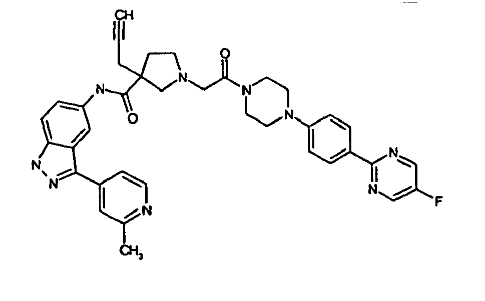 | 658.4 | 2.61 |
| 380 | 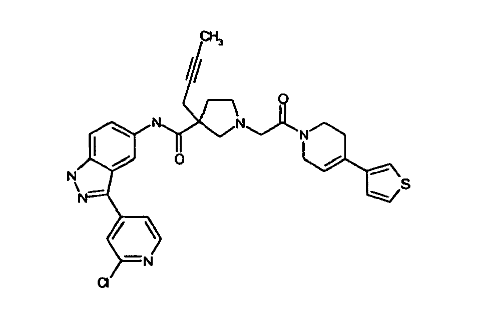 | 599.3 | 3.45 |
| 381 | 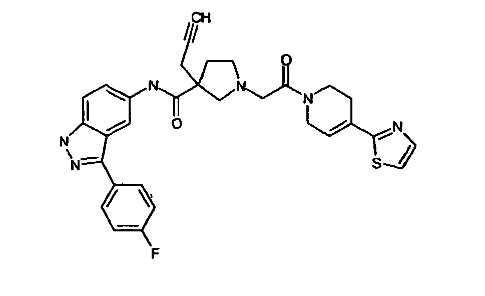 | 569.3 | 2.62 |
| 382 | 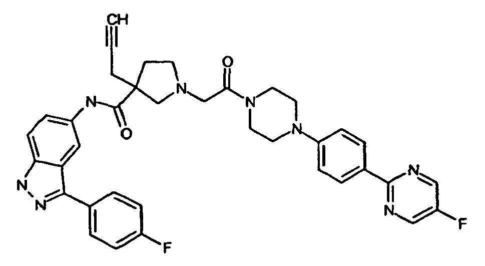 | 661.4 | 3.21 |
| 383 | 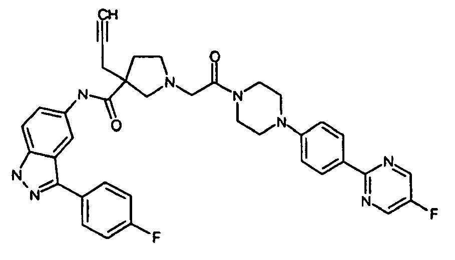 | 661.4 | 3.16 |

### EXAMPLES 384-436

Following procedures essentially similar to the examples described above, the compounds in Table 21 are prepared.

**Table 21**

| **Ex.** | **Compound** | **M+1** | **Retention time (min)** |
|---|---|---|---|
| 384 | 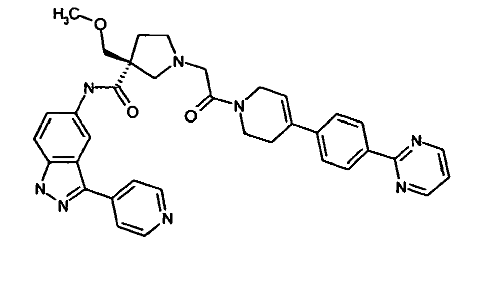 | 629.2 | 2.94 |
| 385 | 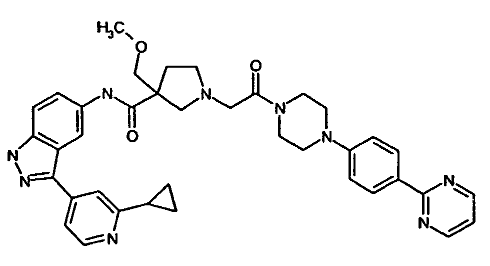 | | |
| 386 | 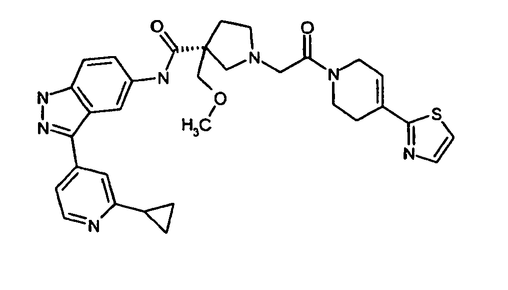 | 598.25 | 2.67 |
| 387 | 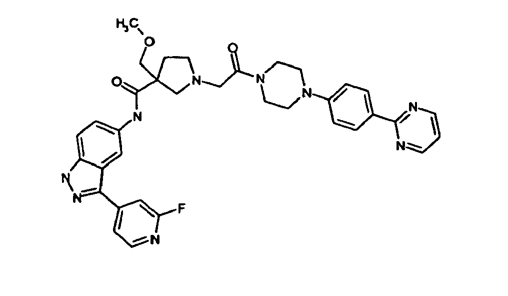 | 650.3 | 3.55 |
| 388 | 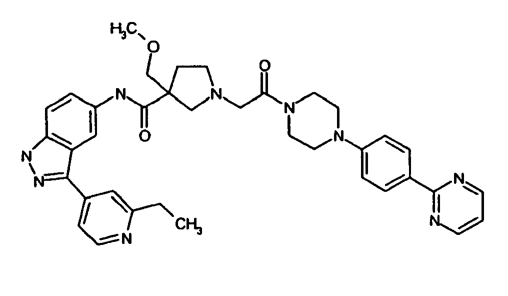 | | |
| 389 | 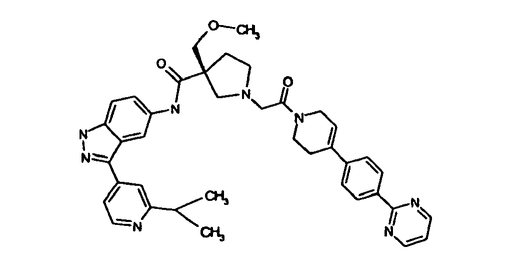 | 669 | 2.34 |
| 390 | 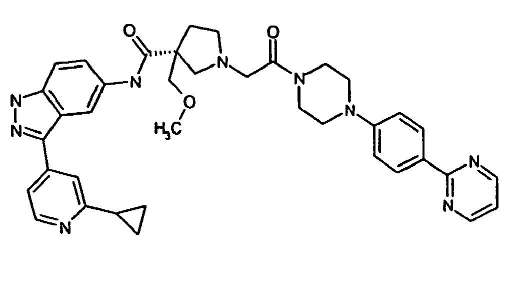 | 672.34 | 2.88 |
| 391 | 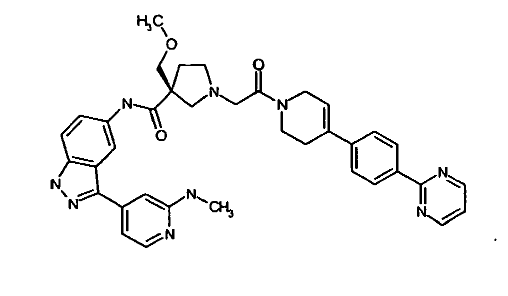 | | |
| 392 | 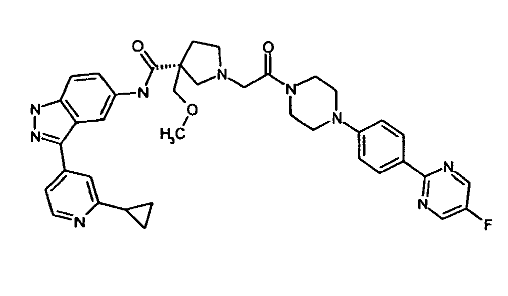 | 690.32 | 3.26 |
| 393 | 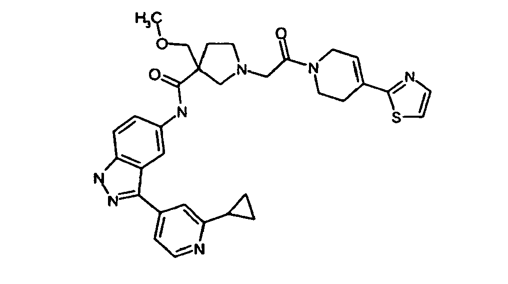 | 598.4 | 2.61 |
| 394 | 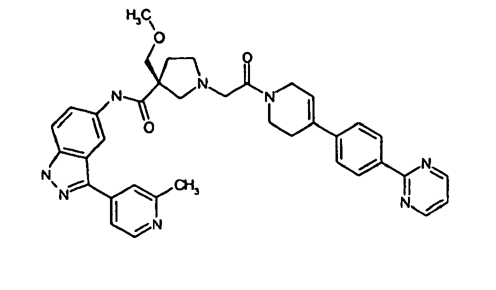 | 643.4 | 2.38 |
| 395 | 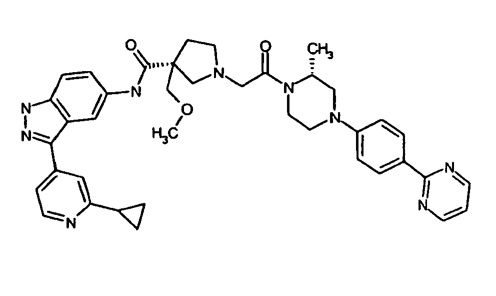 | 686.35 | 3.1 |
| 396 | 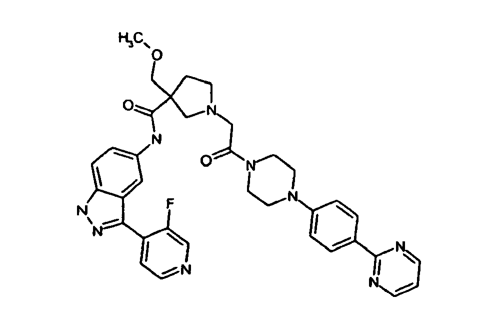 | 650.3 | 3.09 |
| 397 | 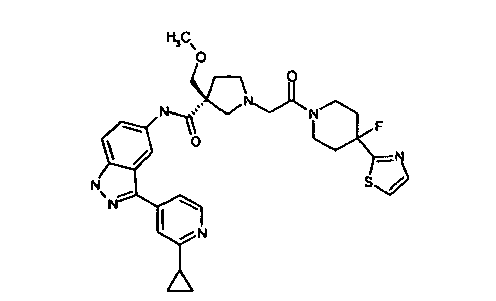 | 618.3 | 2.31 |
| 398 | 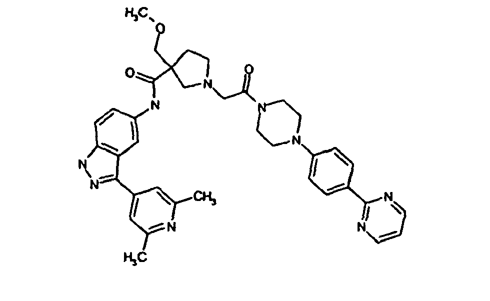 | 660.4 | 2.21 |
| 399 | 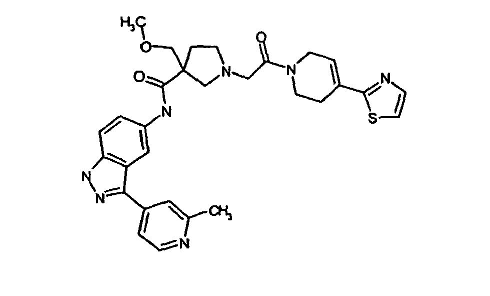 | 572.13 | 2.45 |
| 400 | 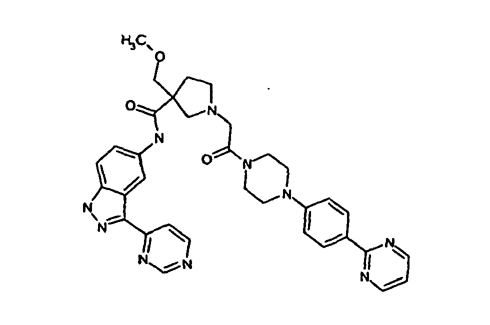 | 633.3 | 3.11 |
| 401 | 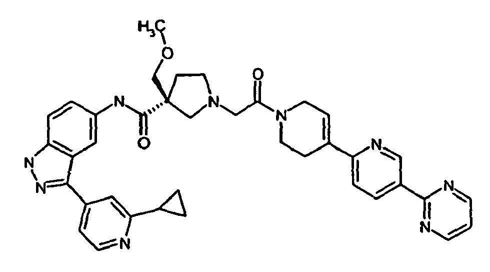 | 670 | 2.11 |
| 402 | 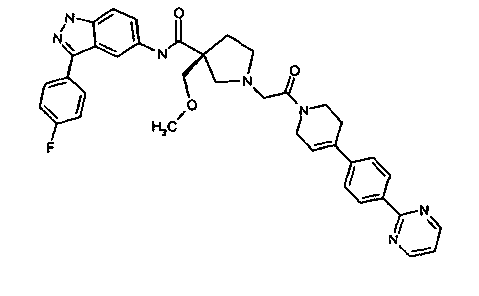 | 640 | 3.1 |
| 403 | 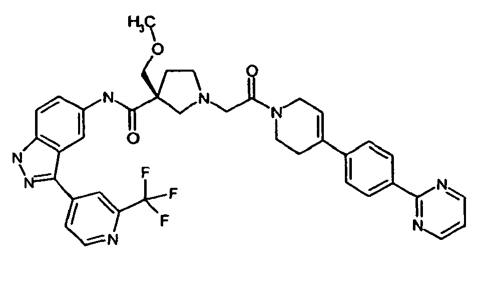 | 697 | 3.15 |
| 404 | 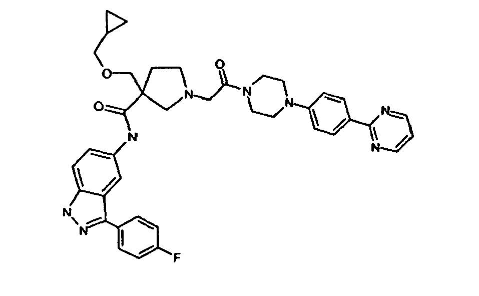 | 689 | 3.05 |
| 405 | 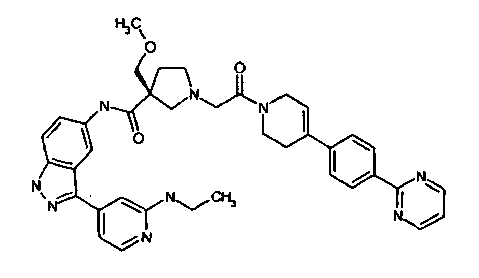 | 672 | 2.38 |
| 406 | 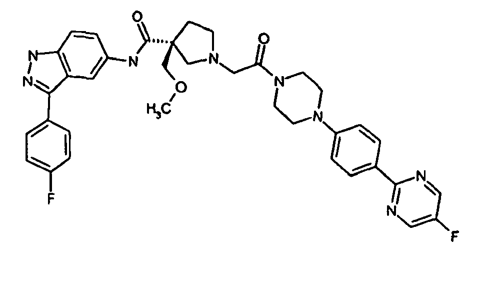 | 667.28 | 3.98 |
| 407 | 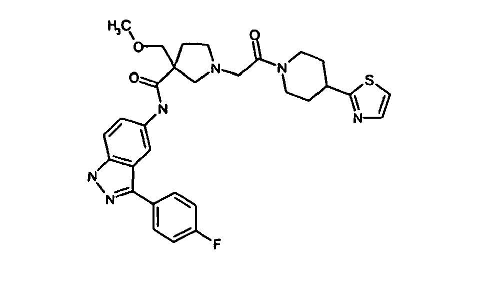 | | |
| 408 | 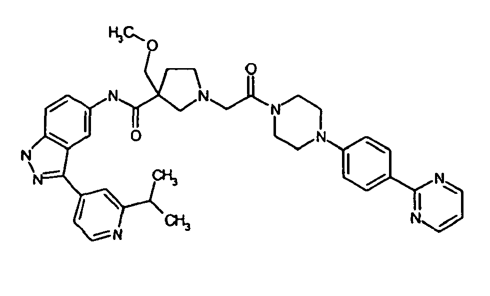 | | |
| 409 | 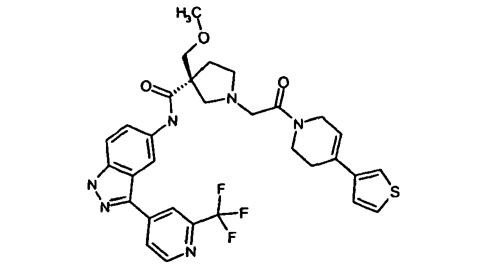 | 625.3 | 3.15 |
| 410 | 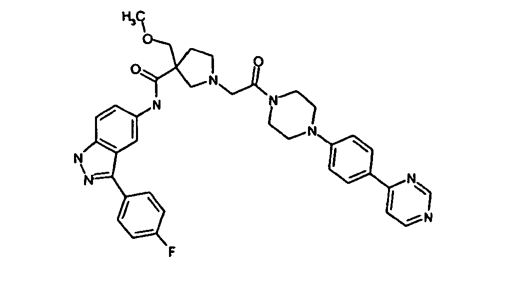 | 686.2 | 3.55 |
| 411 | 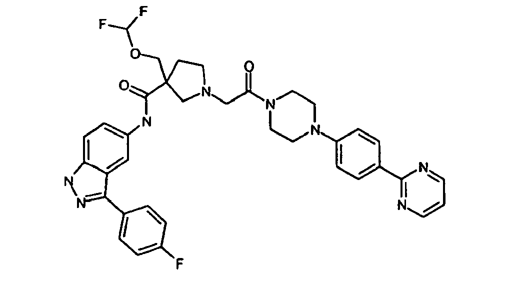 | 685.2 | 4 |
| 412 | 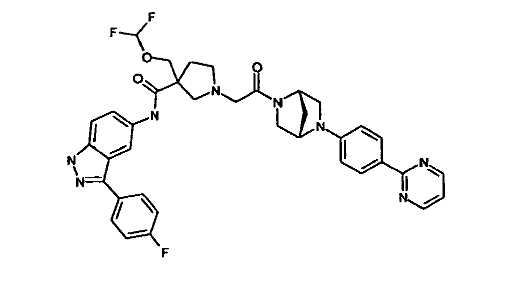 | 697.4 | 3.85 |
| 413 | 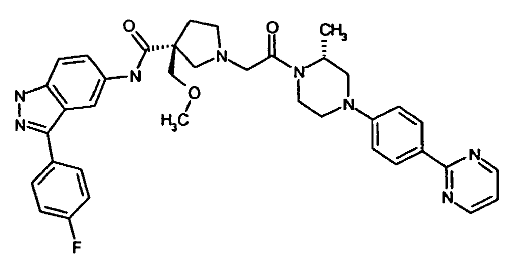 | 663.31 | 3.8 |
| 414 | 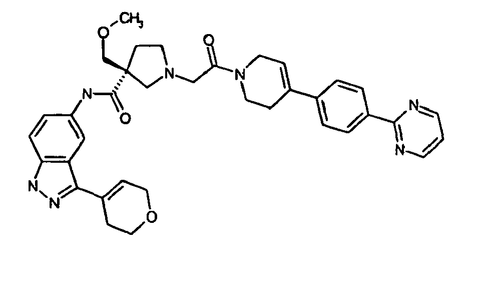 | 634.3 | 3.53 |
| 415 | 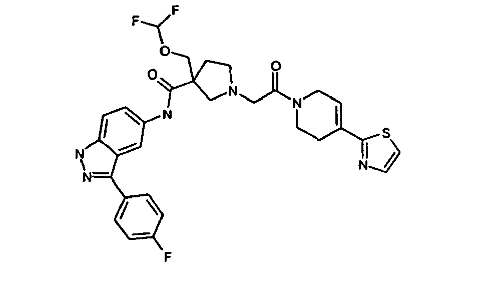 | 611.4 | 3.75 |
| 416 | 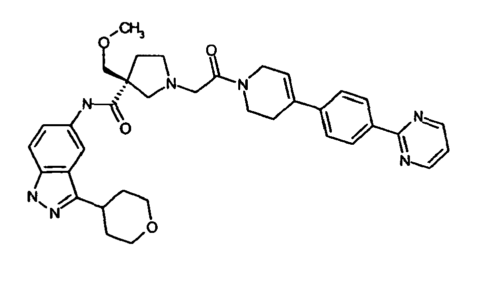 | 636.32 | 3.46 |
| 417 | 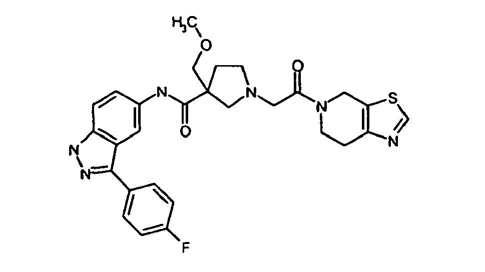 | 549.2 | 3.42 |
| 418 | 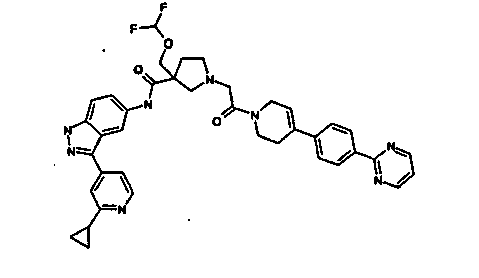 | 705.5 | 3.2 |
| 419 (Reference) | 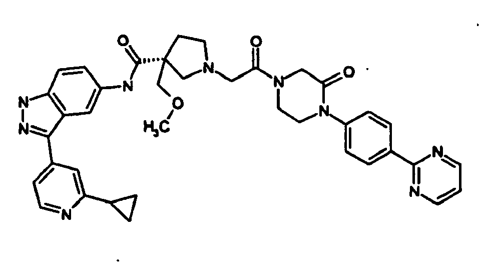 | 686.31 | 2.64 |
| 420 | 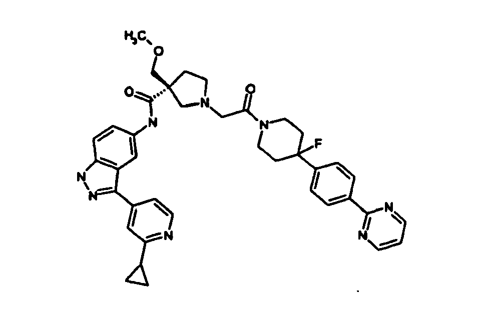 | 689.4 | 2.47 |
| 421 | 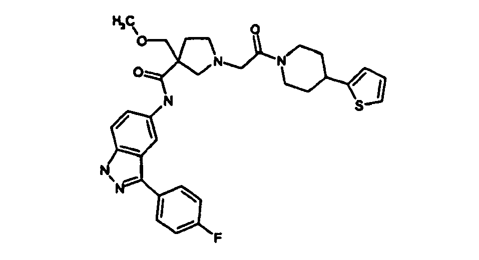 | 576.2 | 4.3 |
| 422 | 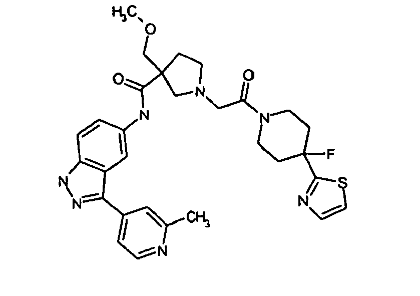 | 592.3 | 1.8 |
| 423 | 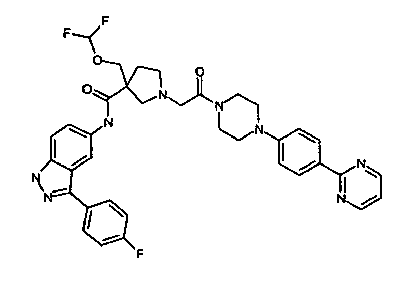 | 685.2 | 4 |
| 424 | 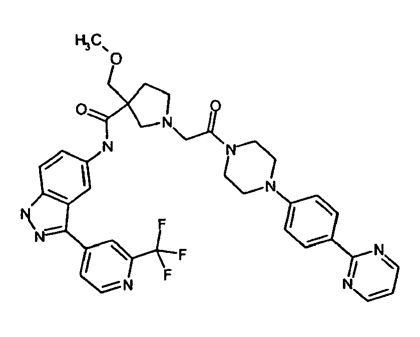 | 700.4 | 2.72 |
| 425 | 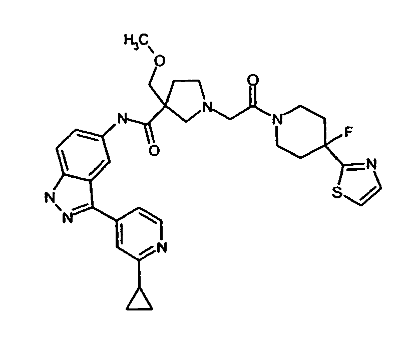 | 618.3 | 2.25 |
| 426 | 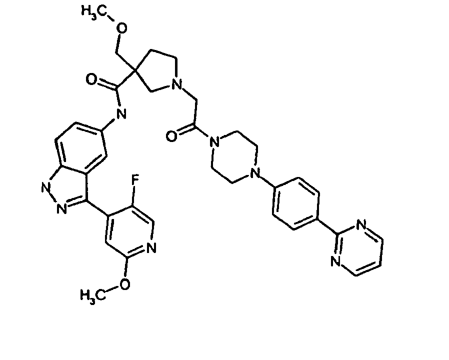 | 680.4 | 3.74 |
| 427 | 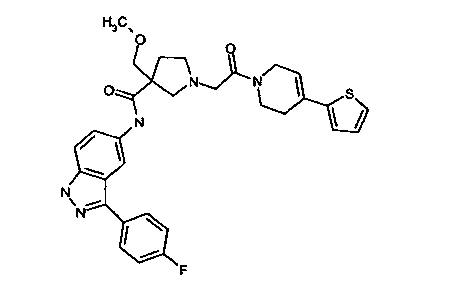 | 574.22 | 4.06 |
| 428 | 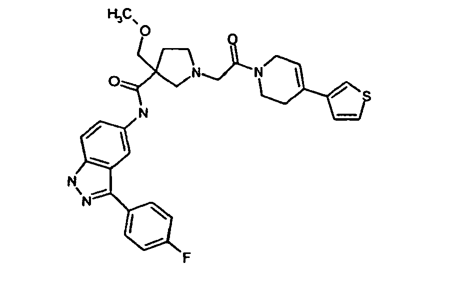 | 574.22 | 4.04 |
| 429 | 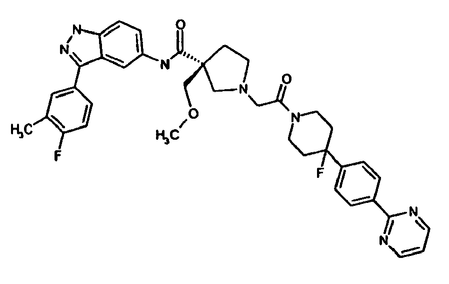 | 680.4 | 3.35 |
| 430 (Reference) | 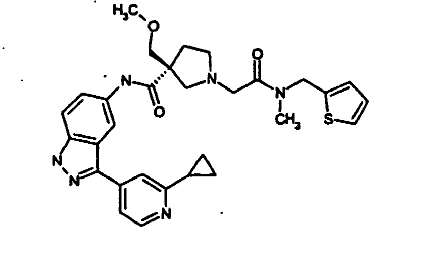 | 559.4 | 2.61 |
| 431 1376872 | 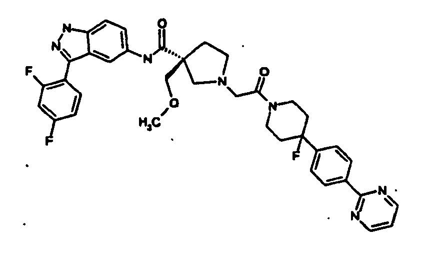 | 684.4 | 2.94 |
| 432 1338111 | 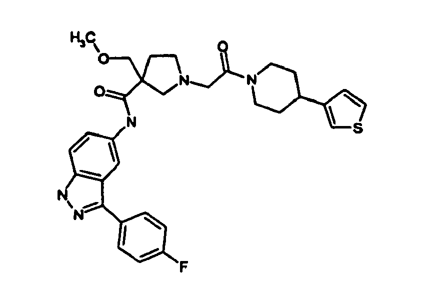 | 575.2 | 4.3 |
| 433 (Reference) | 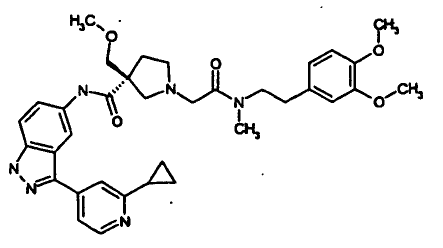 | 627.4 | 2.7 |
| 434 | 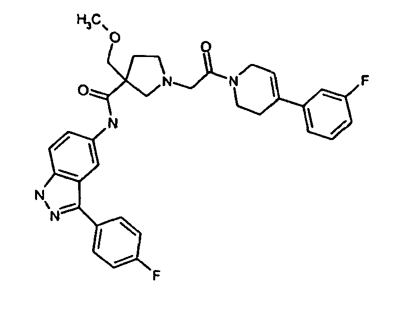 | 586.1 | 4.2 |
| 435 | 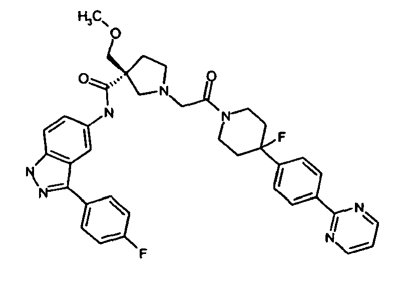 | 666.4 | 3.23 |
| 436 | 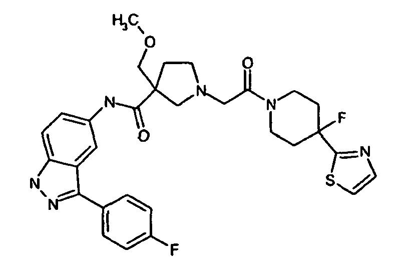 | 595.3 | 2.88 |

### EXAMPLES 437-509

Following procedures essentially similar to the examples described above, the compounds in Table 22 are prepared.

**Table 22**

| **Ex.** | **Compound** | **M+1** | **Retention time (min)** |
|---|---|---|---|
| 437 | 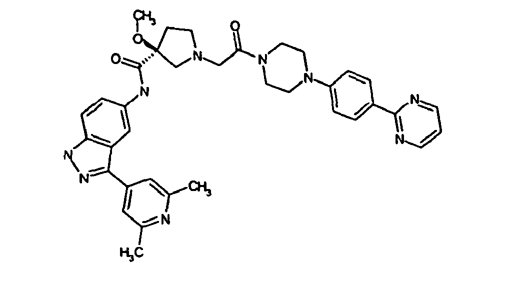 | 646.4 | 1.85 |
| 438 | 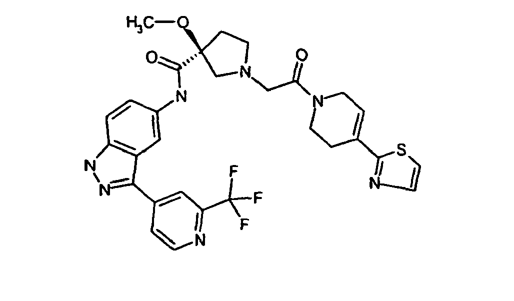 | 612.3 | 2.7 |
| 439 | 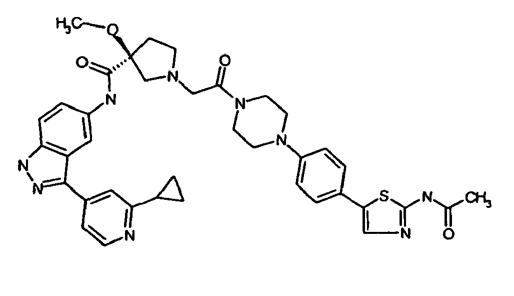 | 720.4 | 2.12 |
| 440 | 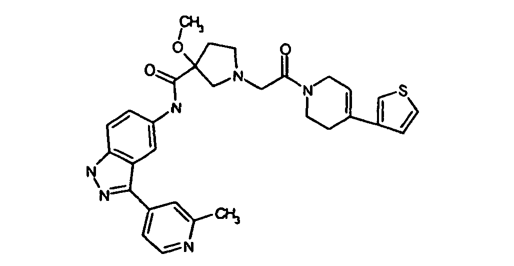 | 557.3 | 2.34 |
| 441 | 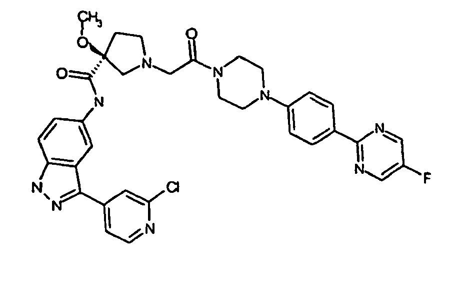 | 670.4 | 4.03 |
| 442 1364199 | 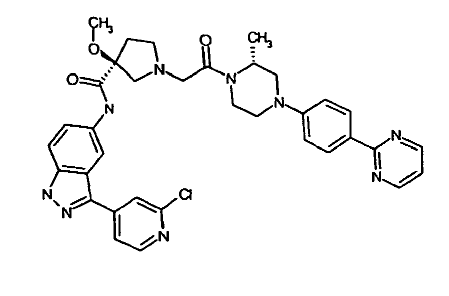 | 666.4 | 3.81 |
| 443 | 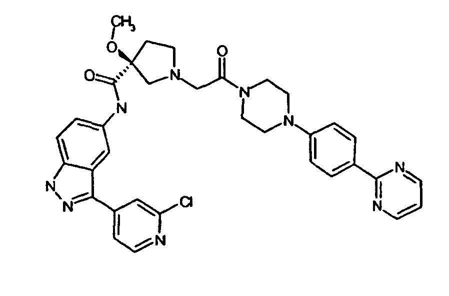 | 652.3 | 3.63 |
| 444 | 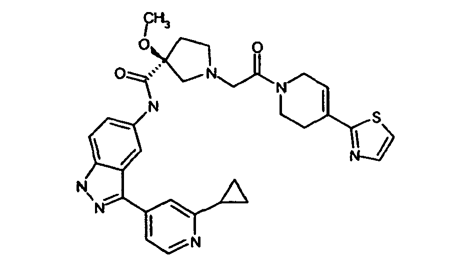 | 584.3 | 2.64 |
| 445 | 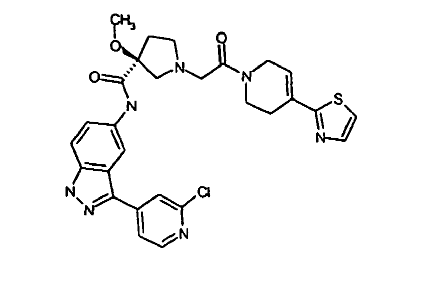 | 578.3 | 3.4 |
| 446 | 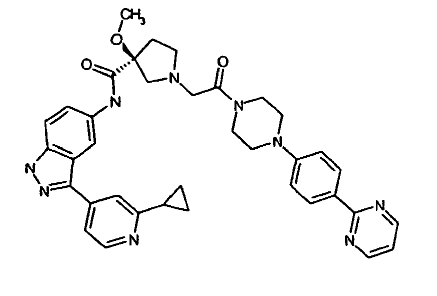 | 658.4 | 2.96 |
| 447 | 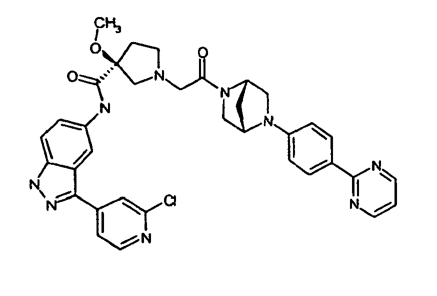 | 664.3 | 3.55 |
| 448 | 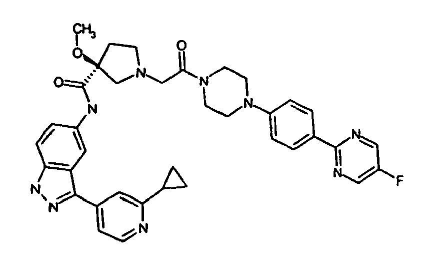 | 676.4 | 3.27 |
| 449 | 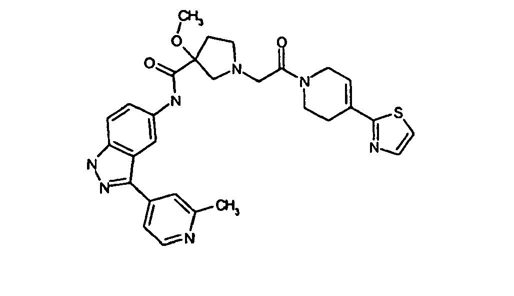 | 558.2 | 2.35 |
| 450 | 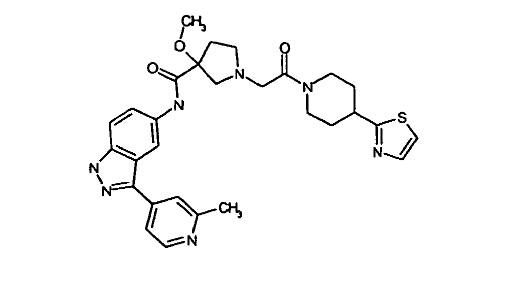 | 560.3 | 2.27 |
| 451 | 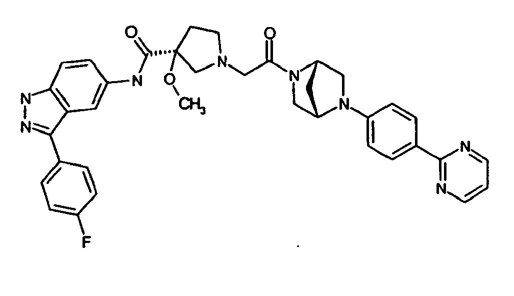 | 647.1 | 3.81 |
| 452 | 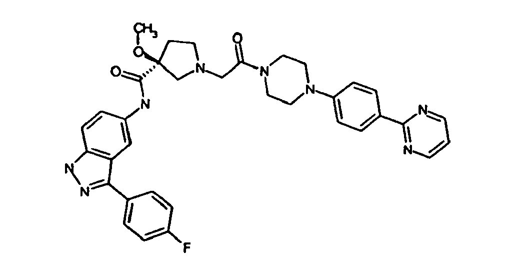 | 672.4 | 3.07 |
| 453 | 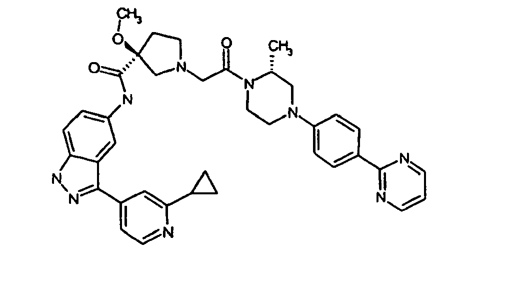 | 658.4 | 2.88 |
| 454 | 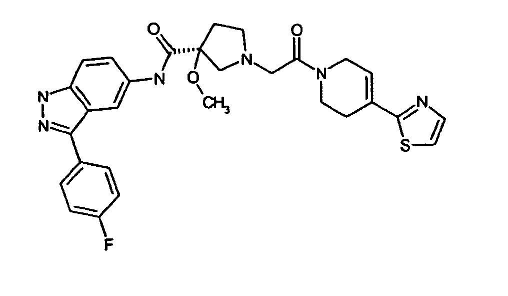 | 561.1 | 3.67 |
| 455 | 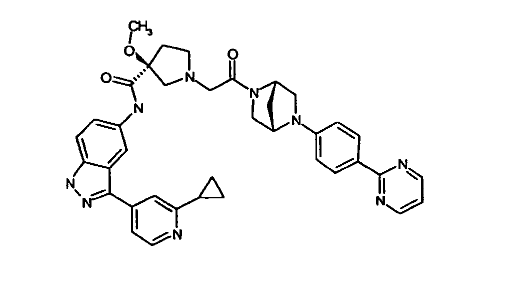 | | |
| 456 | 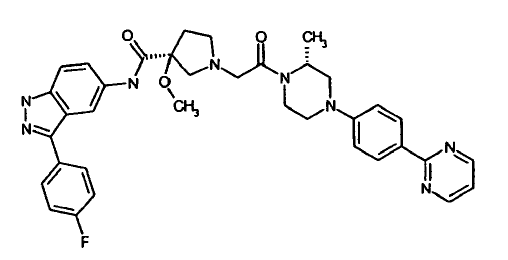 | 649.3 | 4.09 |
| 457 | 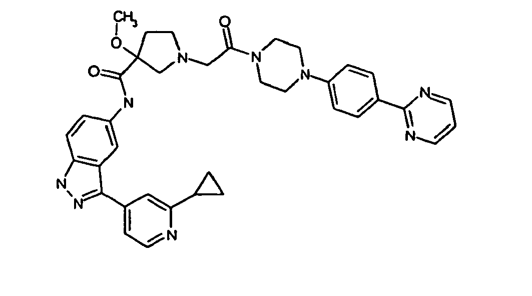 | | |
| 458 | 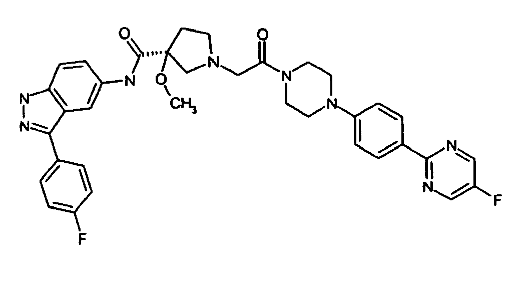 | | |
| 459 | 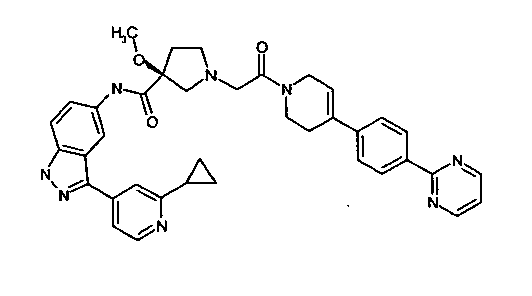 | 655 | 2.11 |
| 460 | 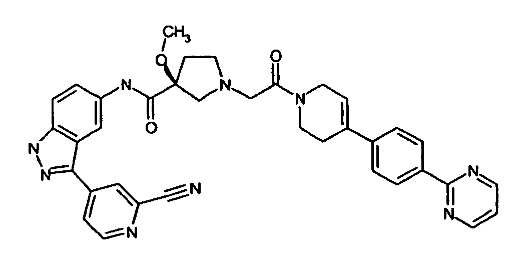 | 640 | 2.75 |
| 461 1375417 | 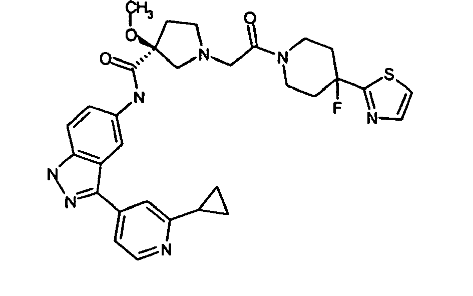 | 604.3 | 2.19 |
| 462 | 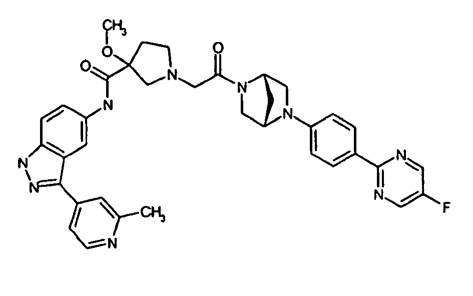 | 662.2 | 3.04 |
| 463 | 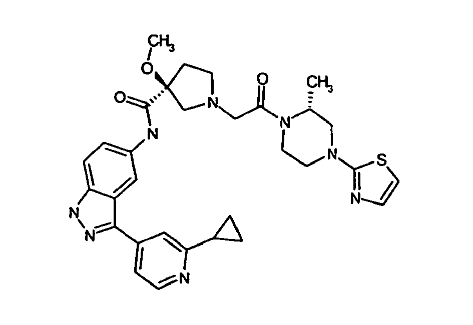 | 647.4 | 3.73 |
| 464 | 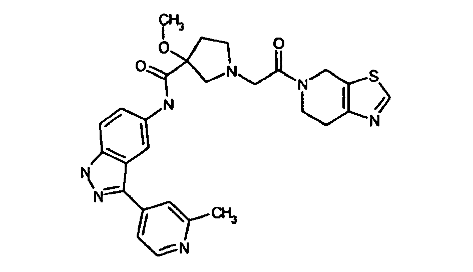 | | |
| 465 | 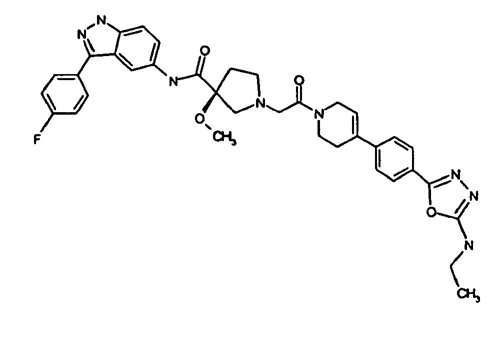 | 665 | 2.6 |
| 466 | 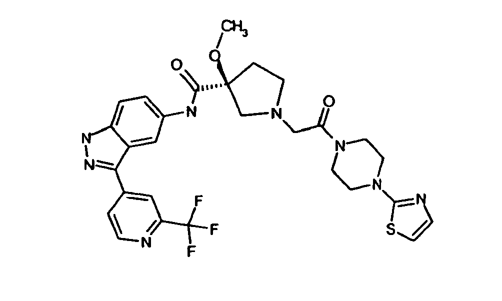 | 615.3 | 2.24 |
| 467 | 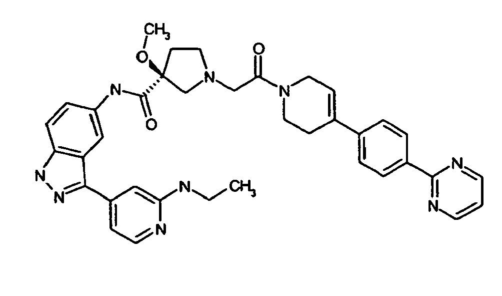 | 658 | 2.28 |
| 468 | 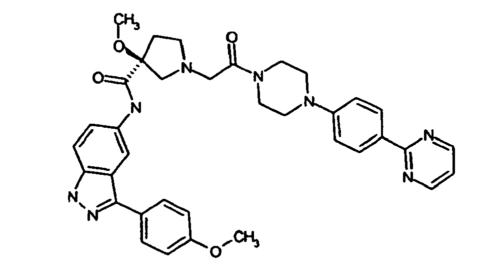 | | |
| 469 | 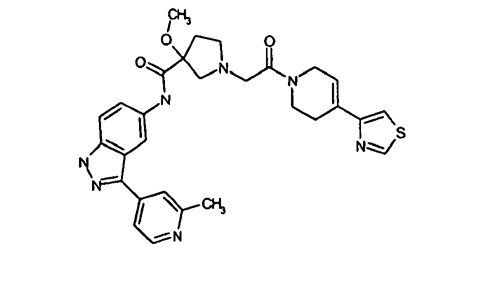 | 558.1 | 2.35 |
| 470 | 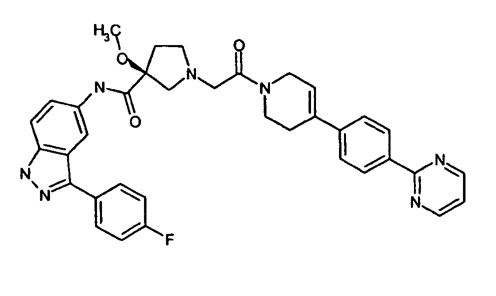 | 632 | 3.09 |
| 471 | 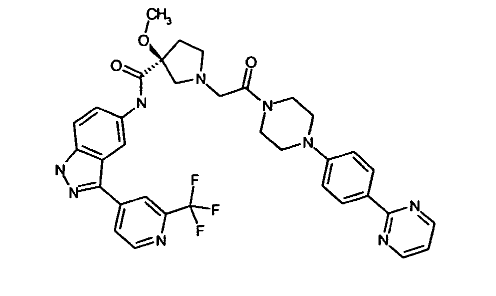 | 686.4 | 2.7 |
| 472 | 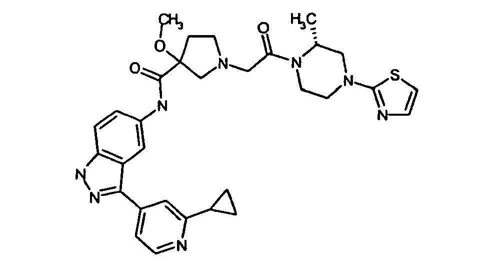 | | |
| 473 | 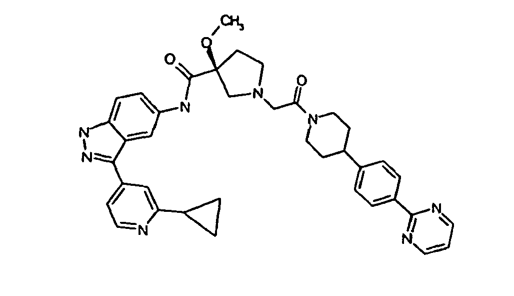 | 657 | 2.35 |
| 474 | 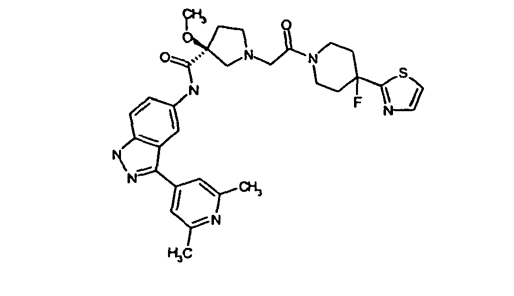 | 592.3 | 1.82 |
| 475 | 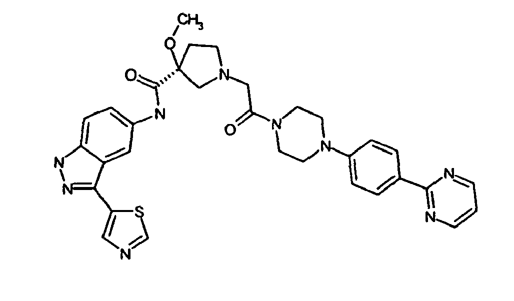 | | |
| 476 | 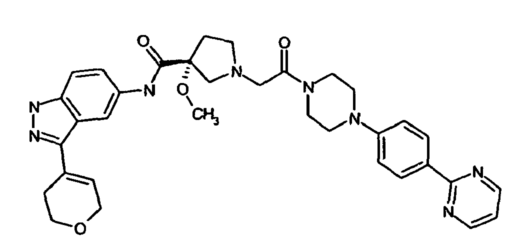 | 623.3 | 3.17 |
| 477 | 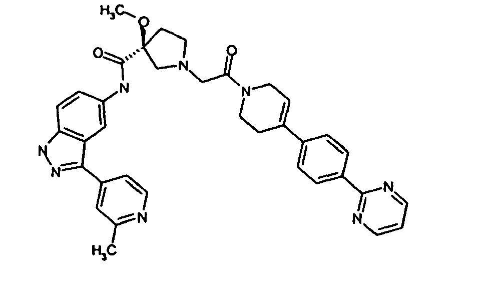 | -2.18 | 2.18 |
| 478 | 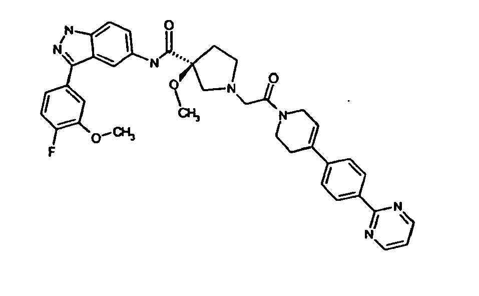 | 662.4 | 3.33 |
| 479 | 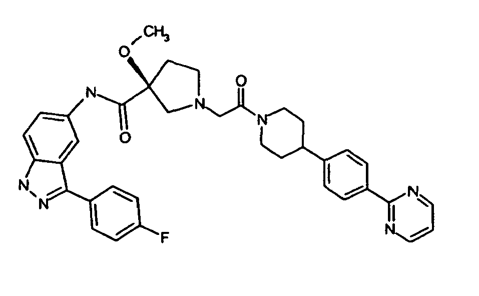 | 634 | 3.09 |
| 480 | 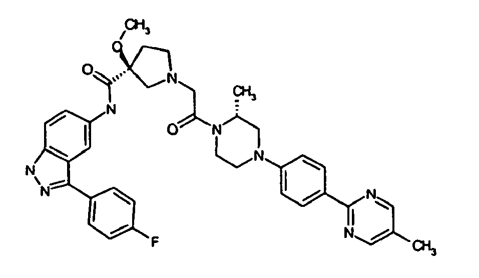 | 663 | 3.21 |
| 481 | 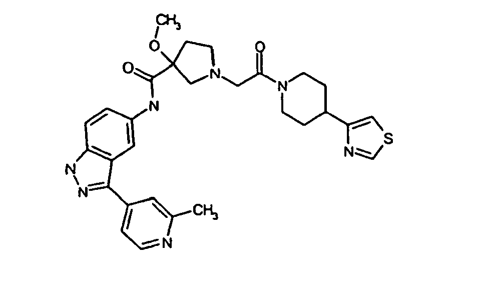 | 685.4 | 4.18 |
| 482 | 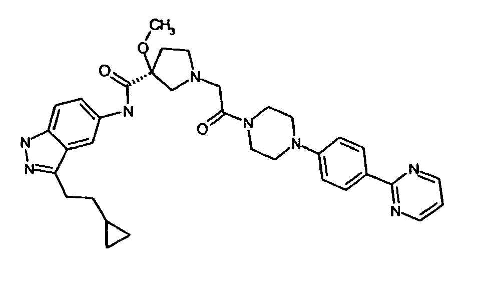 | | |
| 483 | 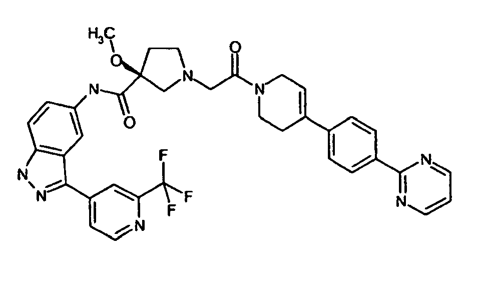 | 683 | 2.89 |
| 484 | 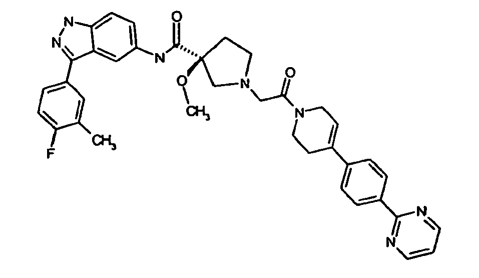 | 646.4 | 3.12 |
| 485 | 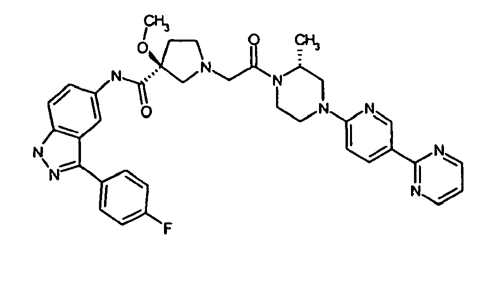 | 650 | 2.58 |
| 486 | 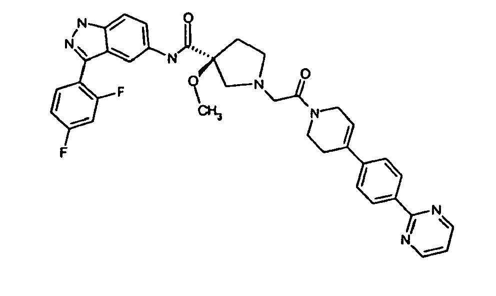 | 650.4 | 2.96 |
| 487 | 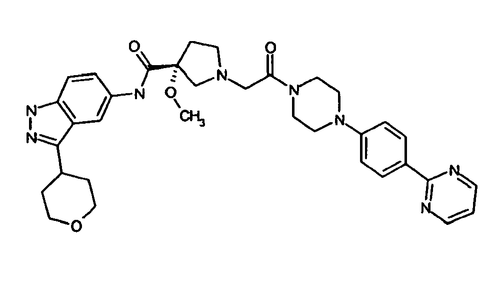 | | |
| 488 | 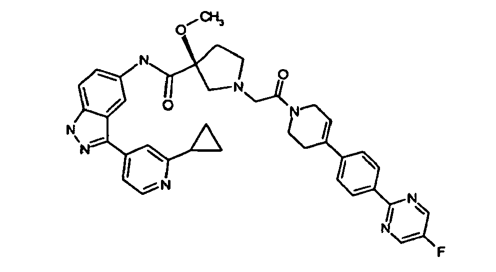 | 673 | 2.63 |
| 489 | 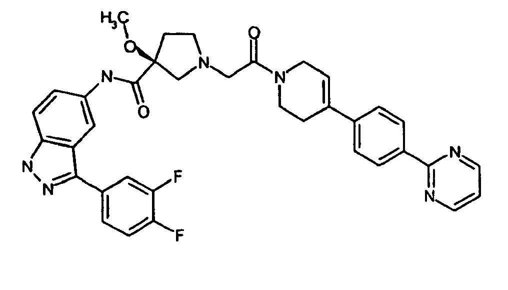 | 650.4 | 3.11 |
| 490 | 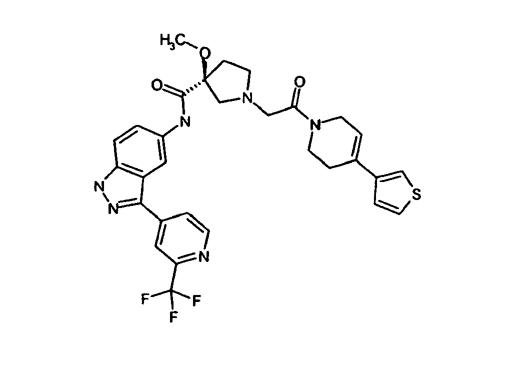 | 611.3 | 3.37 |
| 491 | 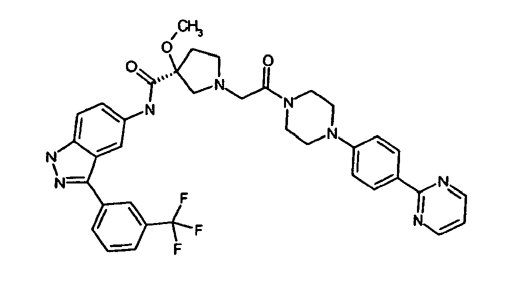 | | |
| 492 | 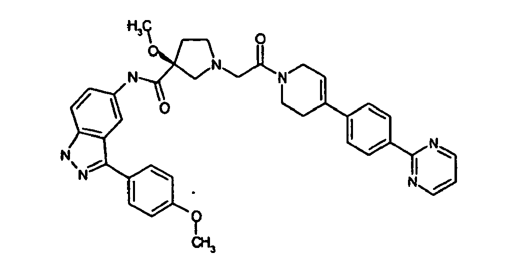 | 644 | 2.75 |
| 493 | 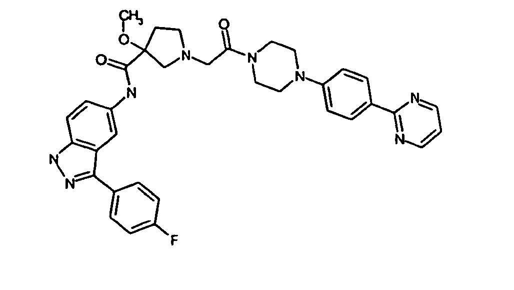 | | |
| 494 | 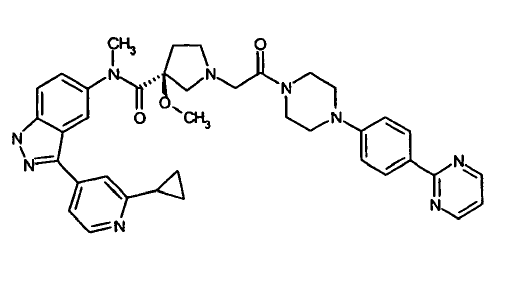 | 672.4 | 2.11 |
| 495 | 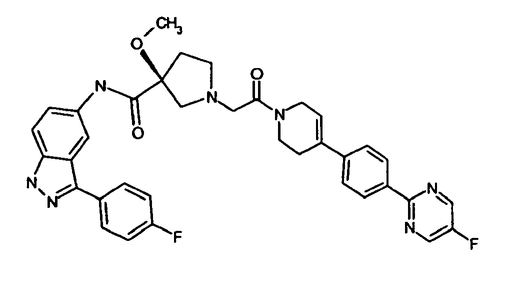 | 650 | 3.47 |
| 496 | 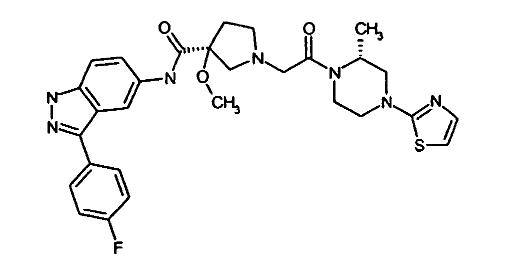 | 649.3 | 4.08 |
| 497 | 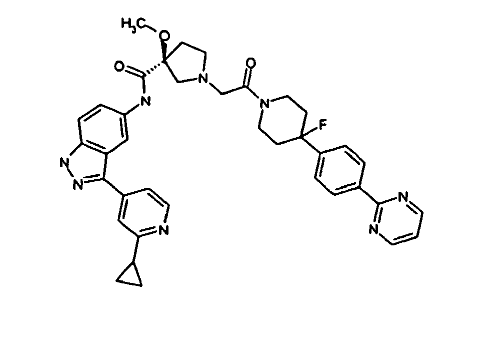 | 675.4 | 2.46 |
| 498 | 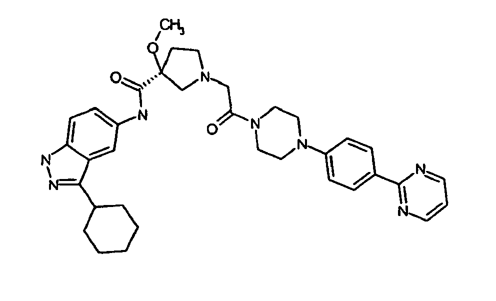 | | |
| 499 | 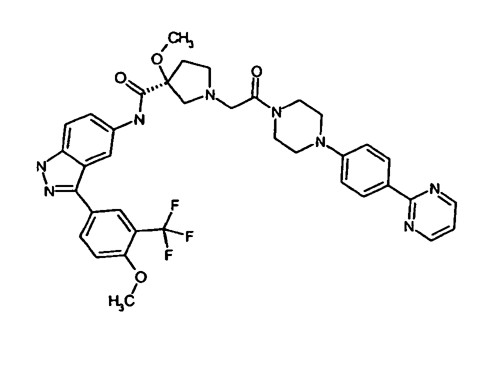 | | |
| 500 | 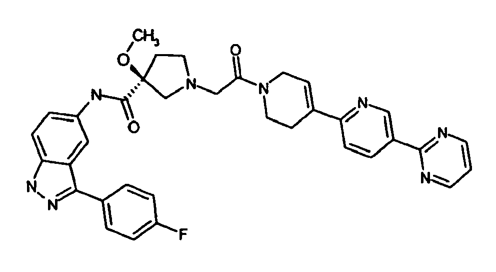 | 633 | 2.73 |
| 501 | 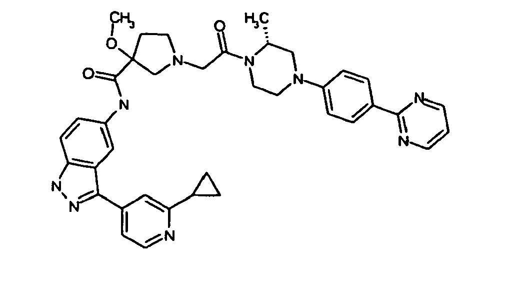 | | |
| 502 | 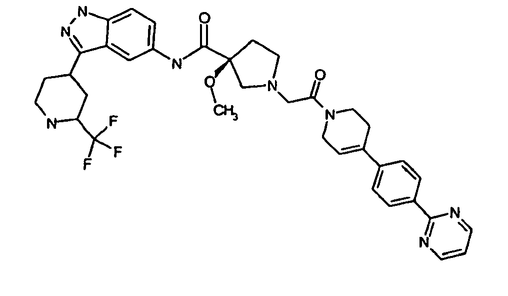 | 689 | 2.22 |
| 503 | 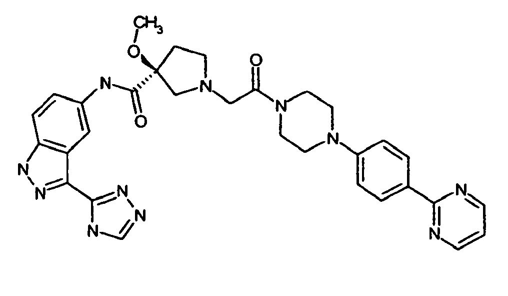 | 622.2 | 2.68 |
| 504 | 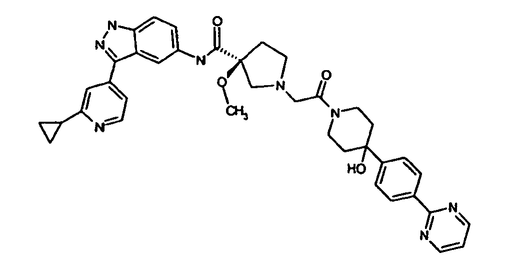 | 673.4 | 1.79 |
| 505 | 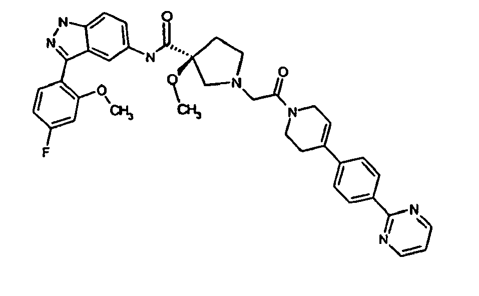 | | |
| 506 | 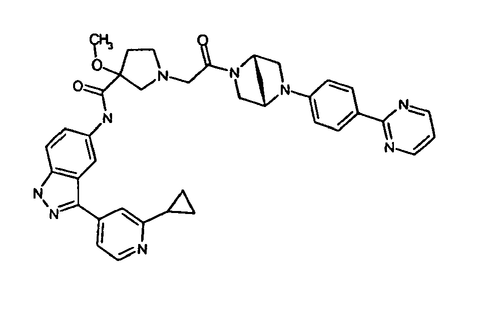 | | |
| 507 | 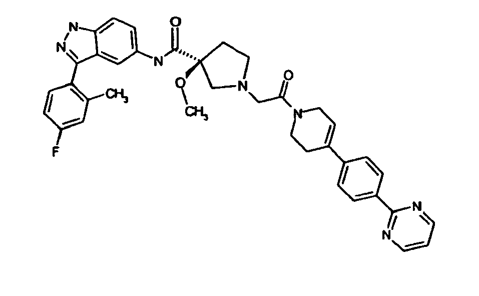 | 646.4 | 3.03 |
| 508 | 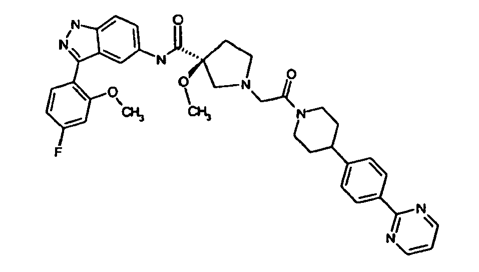 | 664.4 | 3.02 |
| 509 | 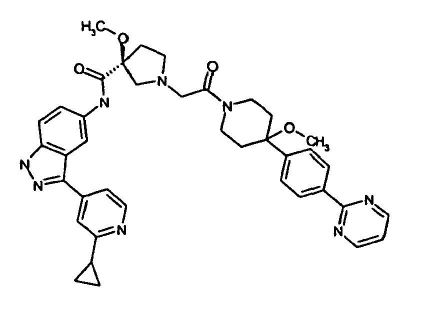 | 687.4 | 2.34 |

### EXAMPLES 510-602

Following procedures essentially similar to the examples described above, the compounds in Table 23 are prepared.

**Table 23**

| **Ex.** | **Compound** | **M+1** | **Retention time (min)** |
|---|---|---|---|
| 510 | 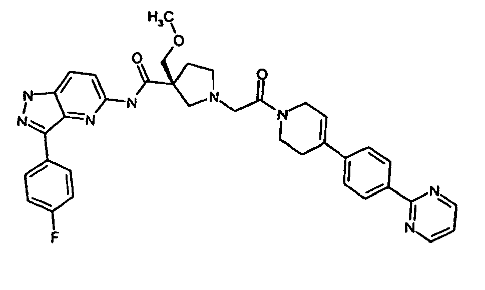 | 647.3 | 3.7 |
| 511 | 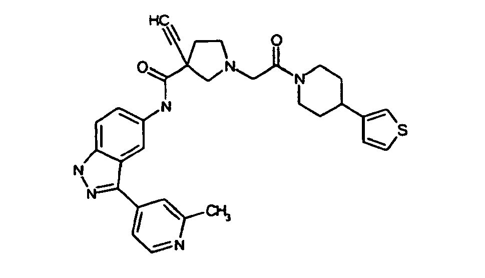 | 553 | 2.42 |
| 512 | 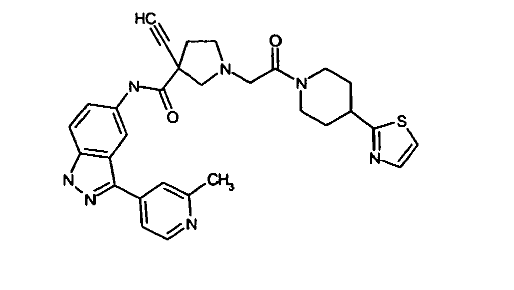 | 554 | 1.88 |
| 513 | 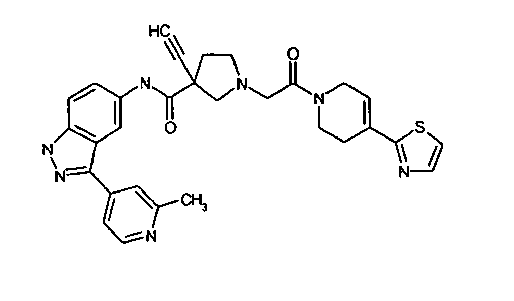 | 626 | 1.9 |
| 514 | 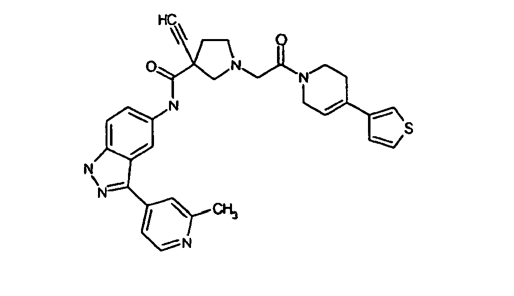 | 551 | 2.94 |
| 515 | 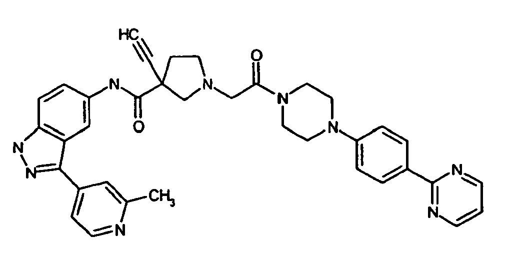 | 626 | 1.9 |
| 516 | 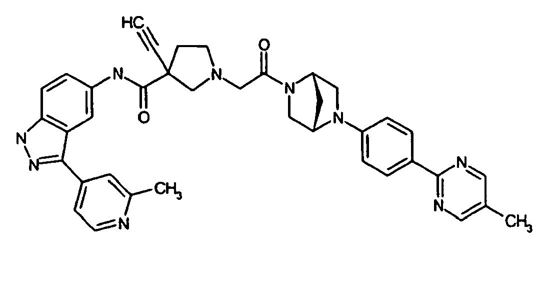 | 652 | 2.19 |
| 517 | 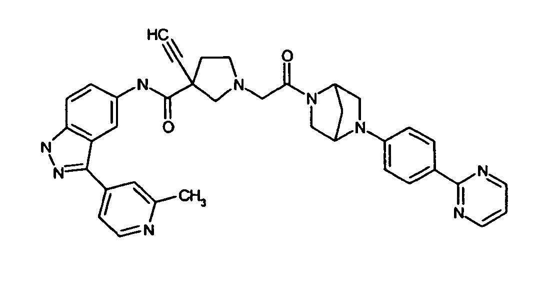 | 638 | 1.84 |
| 518 | 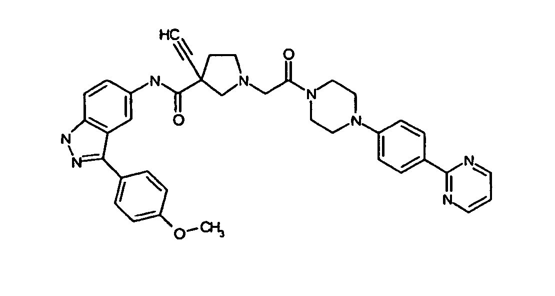 | 641 | 2.93 |
| 519 | 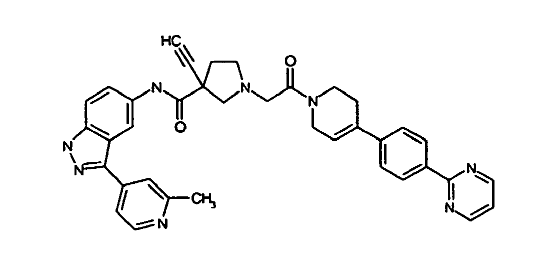 | 623 | 1.97 |
| 520 | 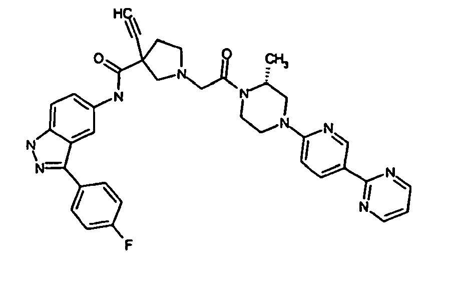 | 644 | 2.53 |
| 521 | 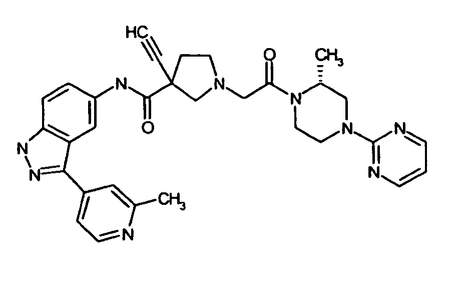 | 564 | 1.74 |
| 522 | 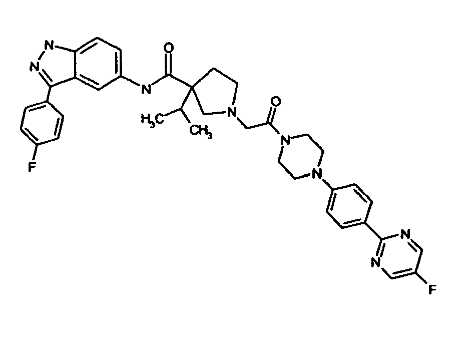 | 665 | 3.17 |
| 523 | 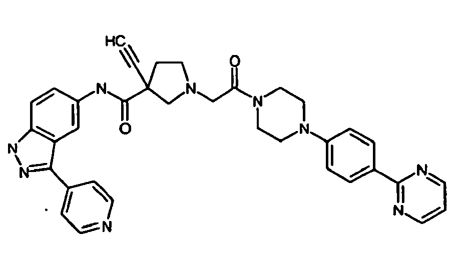 | 612 | 1.97 |
| 524 | 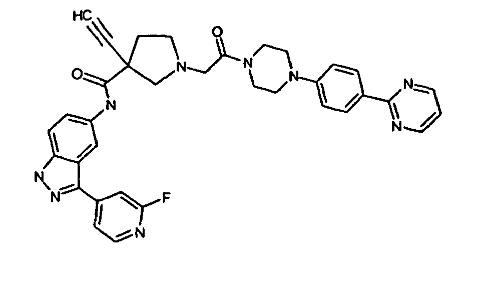 | 630 | 2.59 |
| 525 | 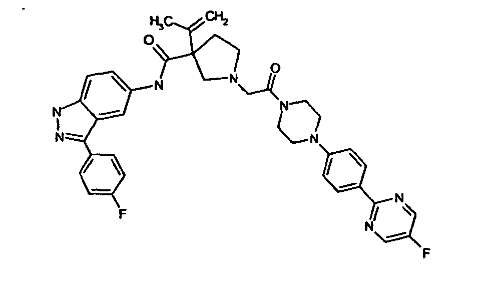 | 663 | 3.17 |
| 526 | 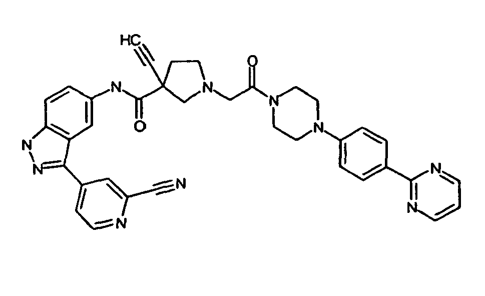 | 637 | 2.5 |
| 527 | 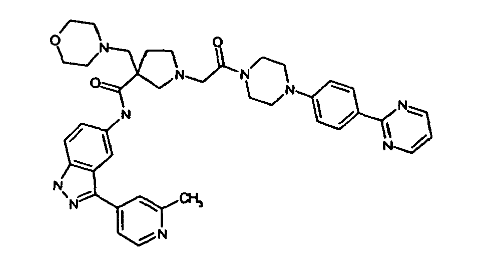 | 701.4 | 2.64 |
| 528 | 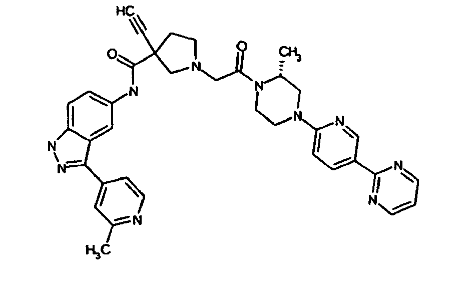 | | |
| 529 | 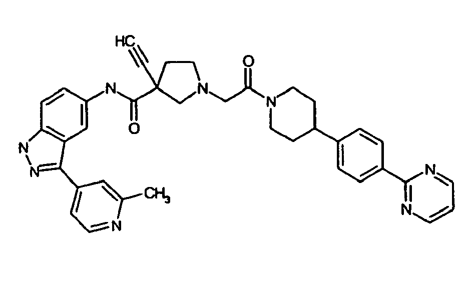 | 625 | 2.57 |
| 530 | 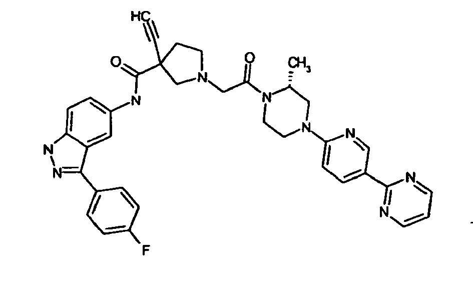 | 644 | 2.53 |
| 531 | 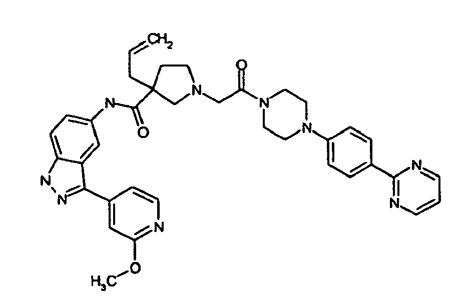 | 658.4 | 2.68 |
| 532 | 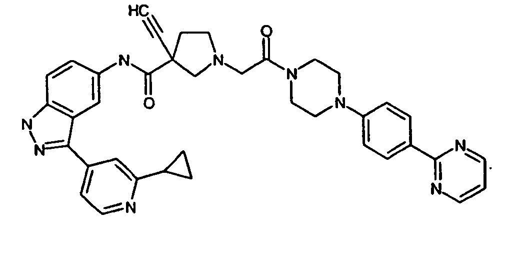 | 652 | 2.26 |
| 533 | 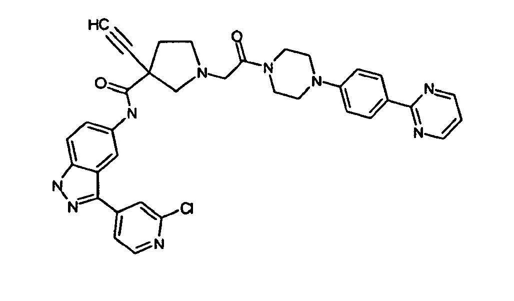 | 646 | 2.86 |
| 534 | 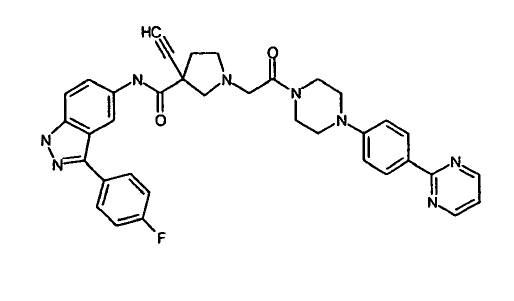 | 629 | 2.85 |
| 535 | 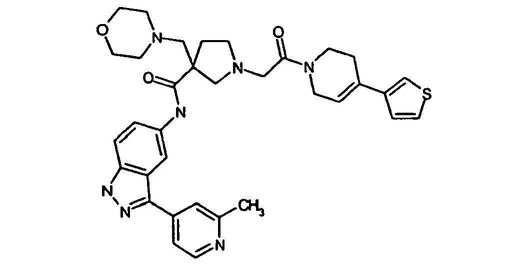 | 626.3 | 2.87 |
| 536 | 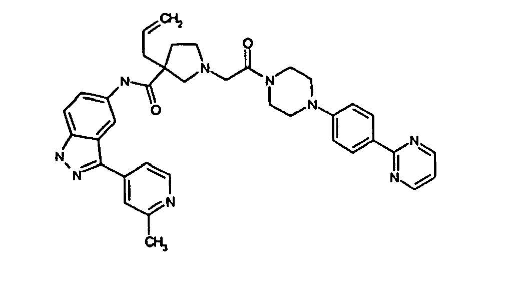 | 642.4 | 2.25 |
| 537 | 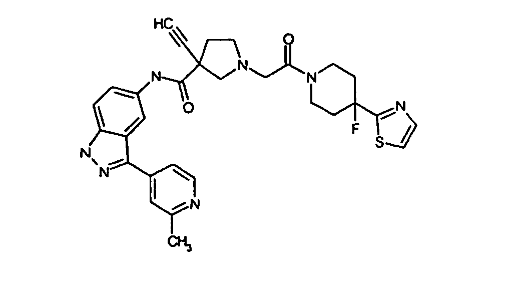 | 572.3 | 2.06 |
| 538 | 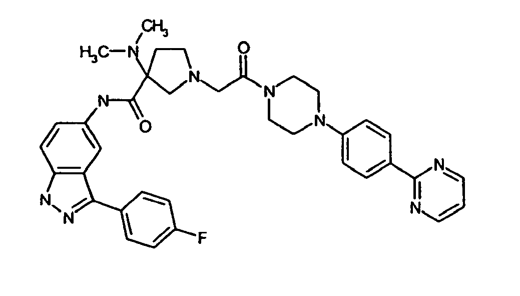 | | |
| 539 | 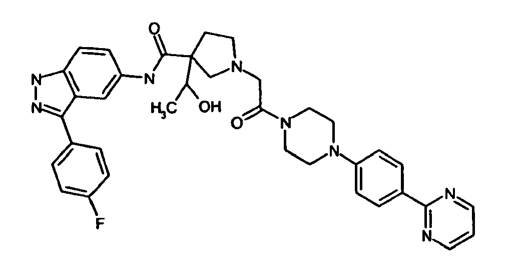 | 649 | 2.68 |
| 540 | 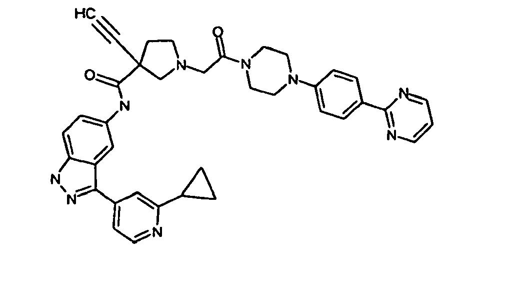 | 652 | 1.98 |
| 541 | 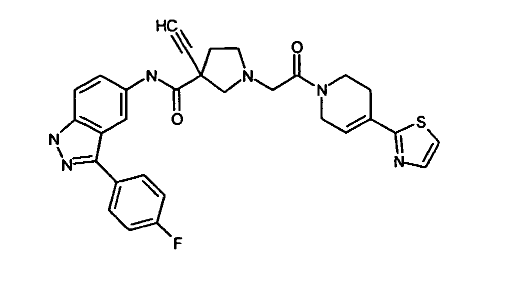 | 555 | 2.7 |
| 542 | 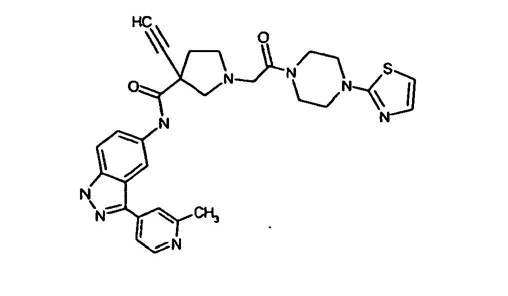 | | |
| 543 | 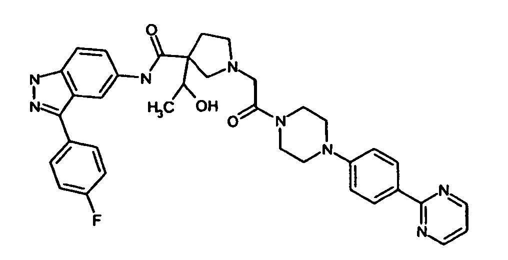 | 555 | 1.43 |
| 544 | 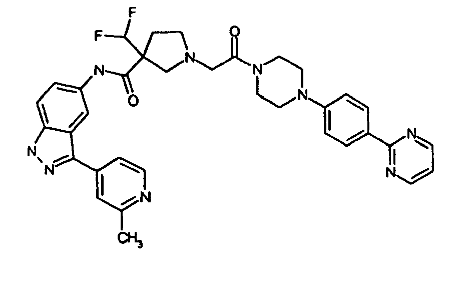 | 652.4 (2.21) | 2.21 |
| 545 | 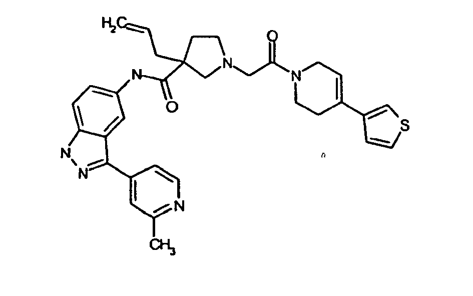 | 567.3 | |
| 546 | 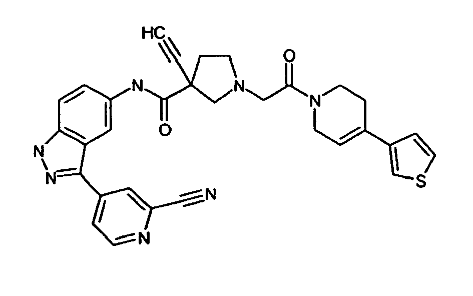 | 562 | 2.76 |
| 547 | 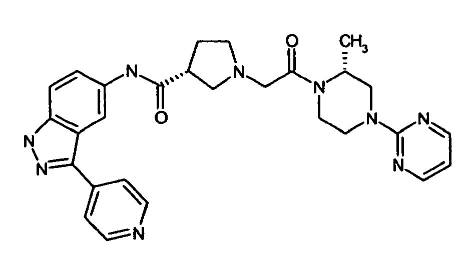 | 526 | 0.82 |
| 548 | 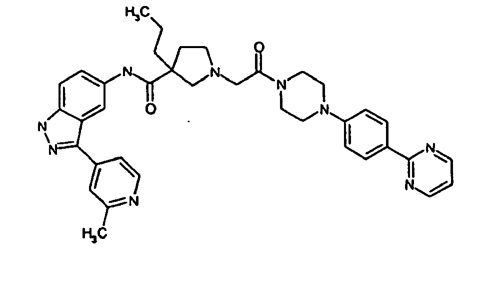 | 644.4 | 2.26 |
| 549 | 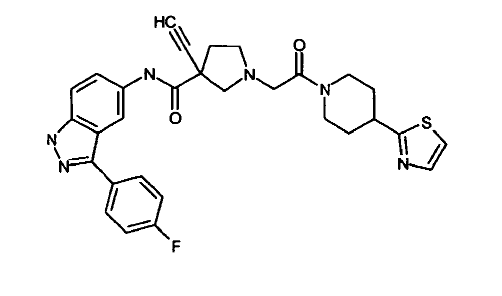 | 557 | 2.61 |
| 550 | 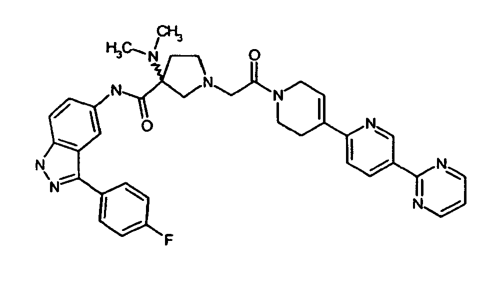 | 646 | 2.8 |
| 551 | 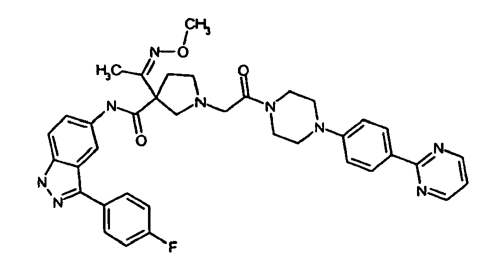 | 676 | 3.12 |
| 552 | 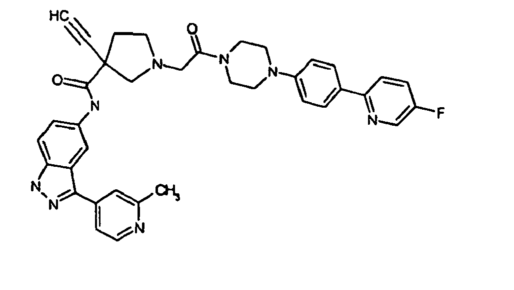 | 643 | 2.02 |
| 553 | 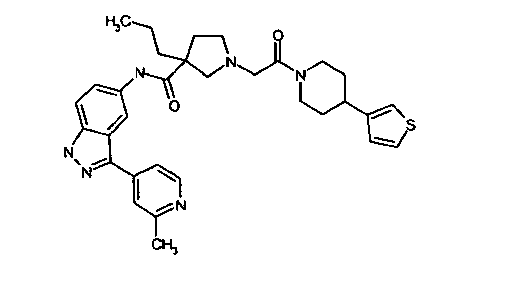 | 571.3 | 2.55 |
| 554 | 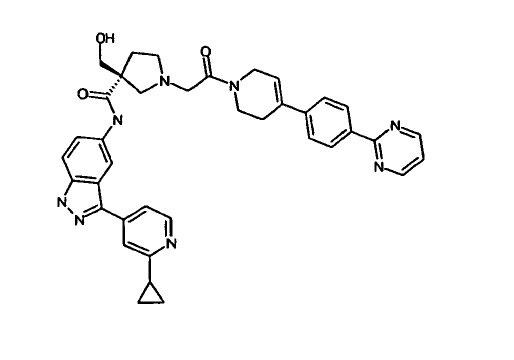 | 655.4 | 2.87 |
| 555 | 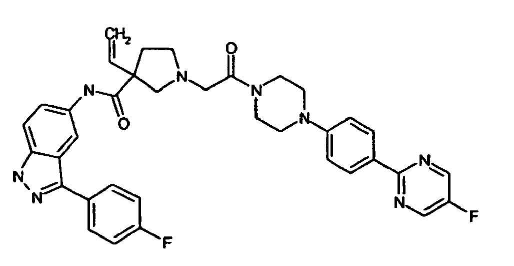 | 649 | 3.1 |
| 556 | 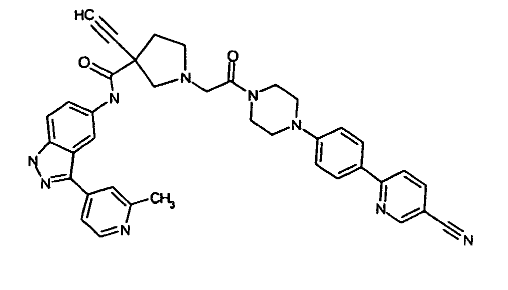 | 650 | 2.57 |
| 557 | 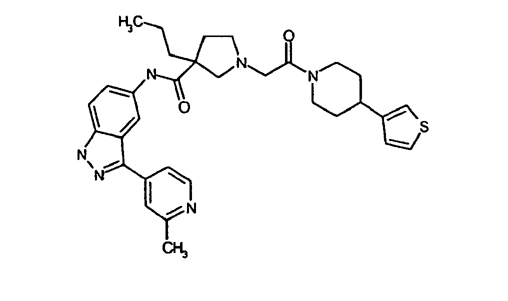 | 571.3 | 2.16 |
| 558 | 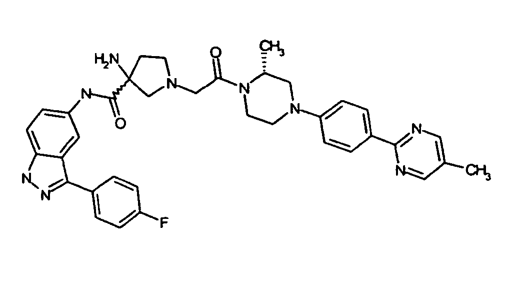 | 648 | 3.85 |
| 559 | 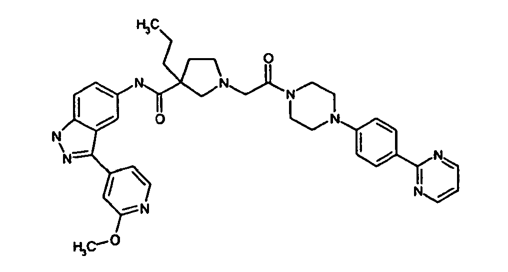 | 660.4 | 2.72 |
| 560 | 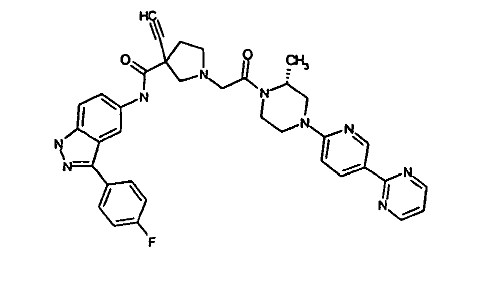 | 644 | 2.53 |
| 561 | 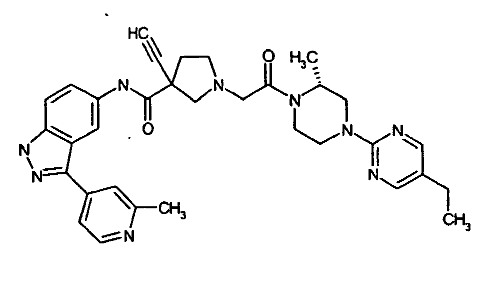 | 592 | 1.88 |
| 562 | 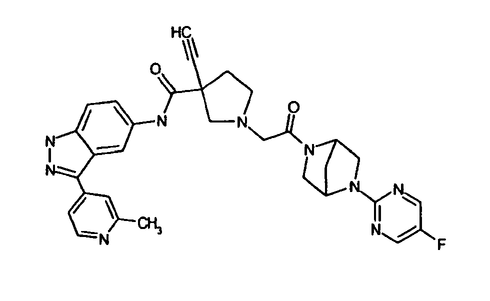 | 580 | 1.94 |
| 563 | 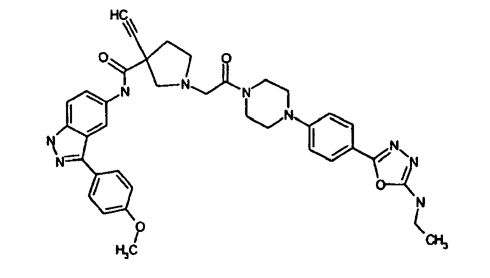 | 674 | 2.42 |
| 564 | 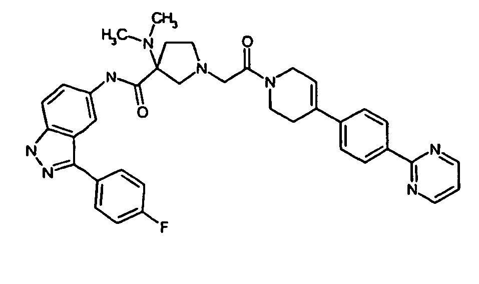 | 645 | 3.35 |
| 565 | 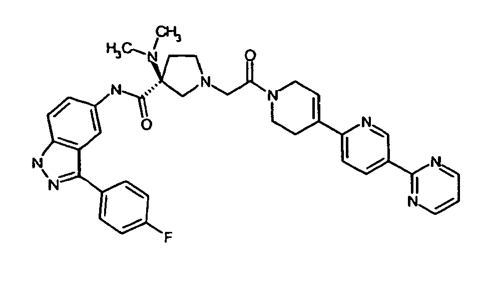 | 646 | 2.8 |
| 566 | 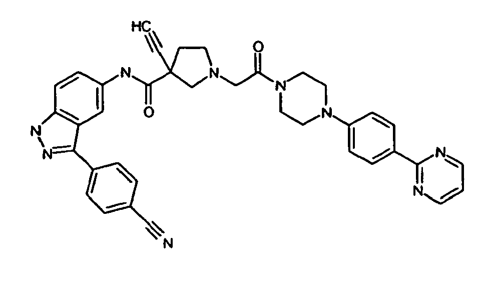 | | |
| 567 | 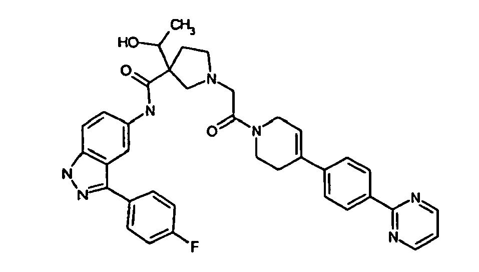 | 646 | 3.15 |
| 568 | 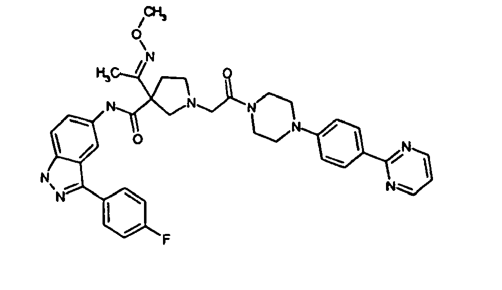 | 676 | 3.12 |
| 569 | 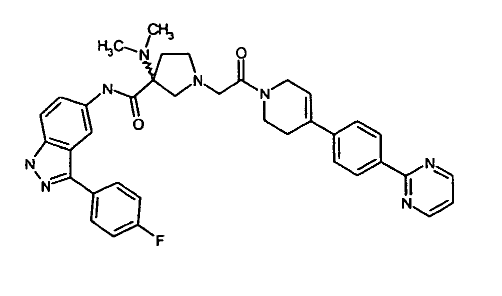 | 645 | 3.34 |
| 570 | 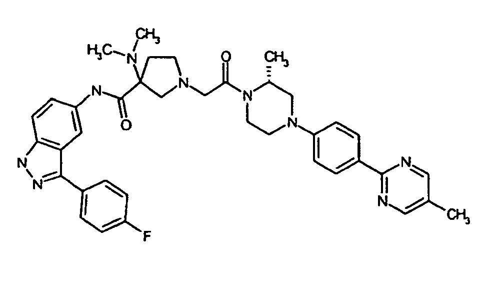 | 676 | 3.4 |
| 571 | 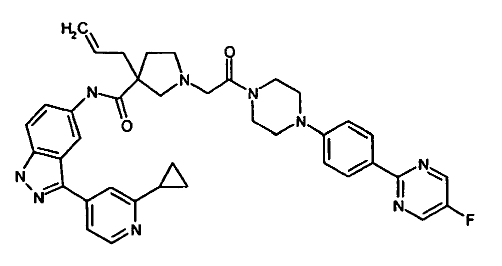 | 686.4 | 2.57 |
| 572 | 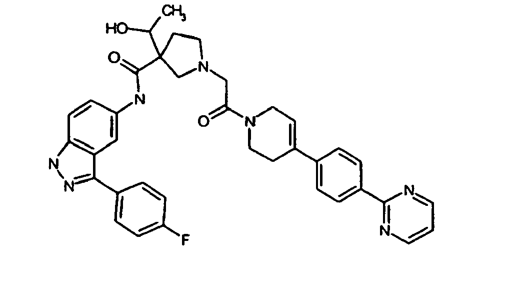 | 646 | 3.17 |
| 573 | 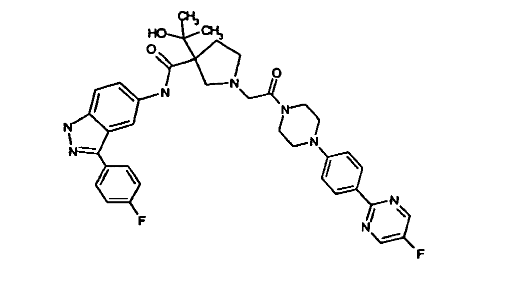 | 681 | 3.05 |
| 574 | 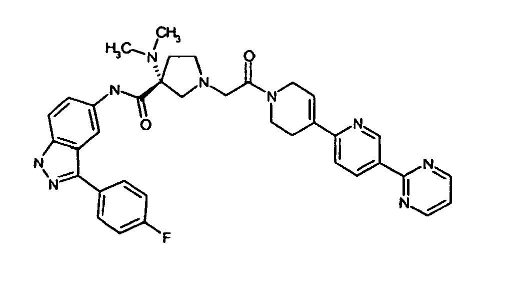 | 646 | 2.8 |
| 575 | 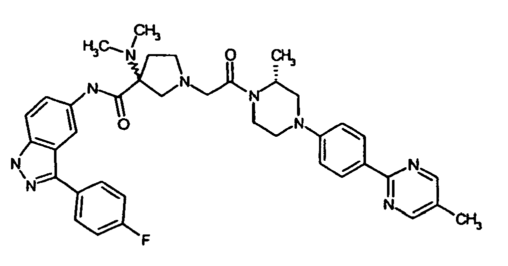 | 676 | 3.35 |
| 576 | 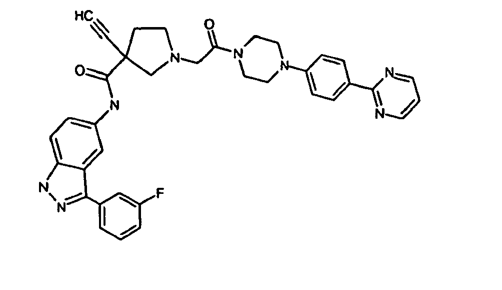 | 629 | 2.72 |
| 577 | 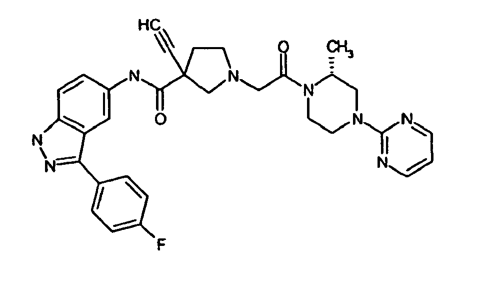 | 567 | 2.61 |
| 578 | 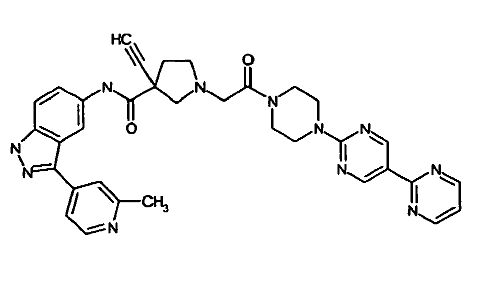 | 628 | 2.27 |
| 579 | 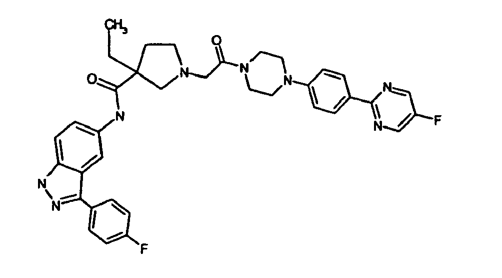 | 651 | 3.48 |
| 580 | 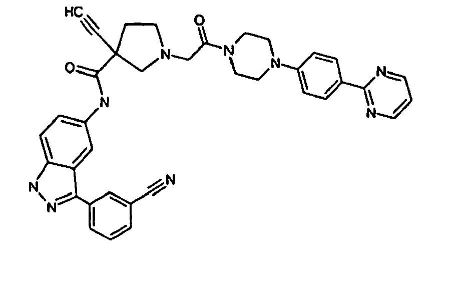 | 636 | 2.69 |
| 581 | 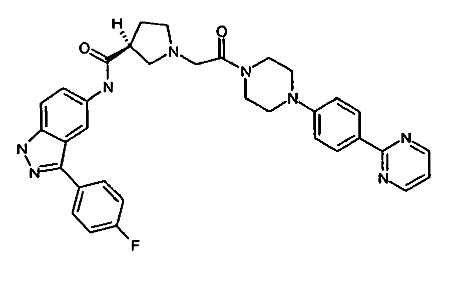 | 605.4 | 3.72 |
| 582 | 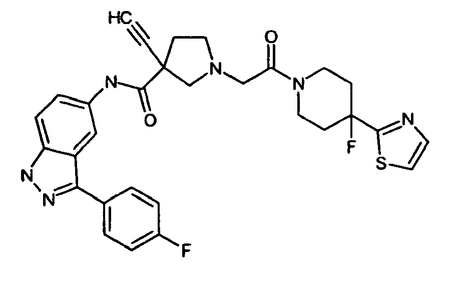 | 575.3 | 2.98 |
| 583 | 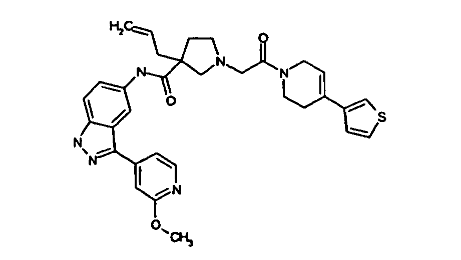 | 583.3 | 2.91 |
| 584 | 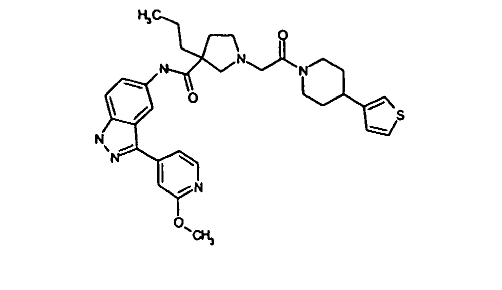 | 587.3 | 2.76 |
| 585 | 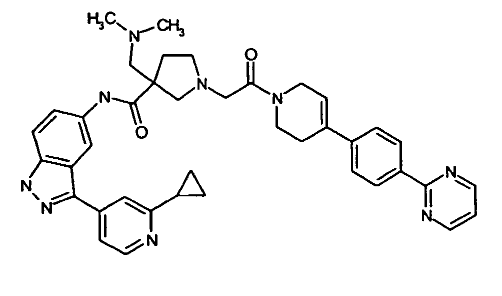 | | |
| 586 | 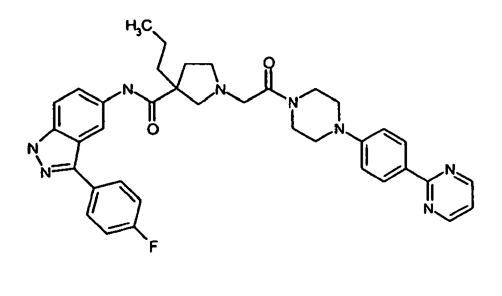 | 647.4 | 3.08 |
| 587 | 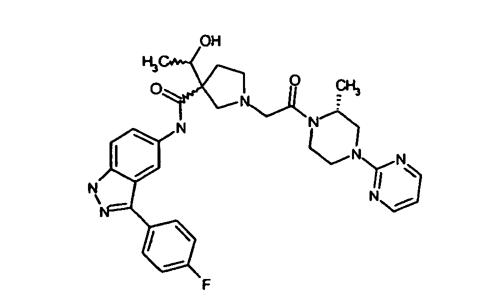 | | |
| 588 | 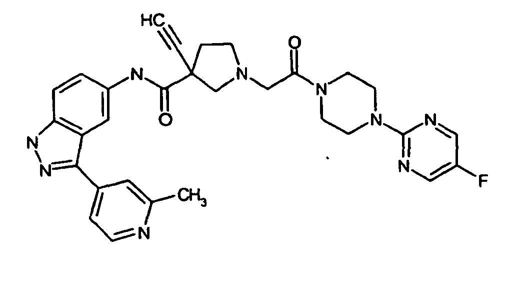 | 568 | 1.96 |
| 589 | 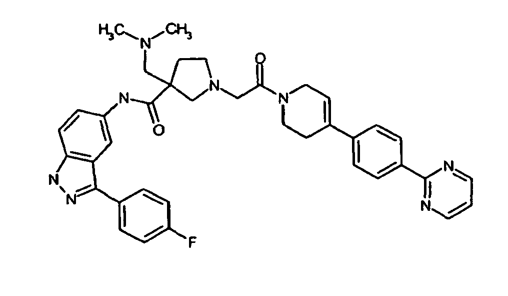 | | |
| 590 | 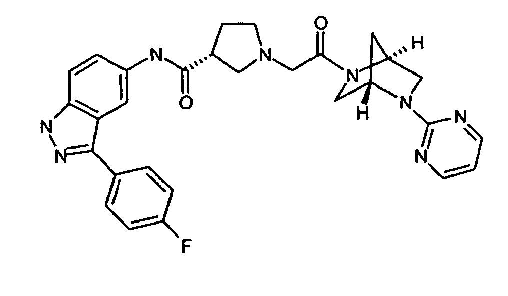 | 541 | 2.18 |
| 591 | 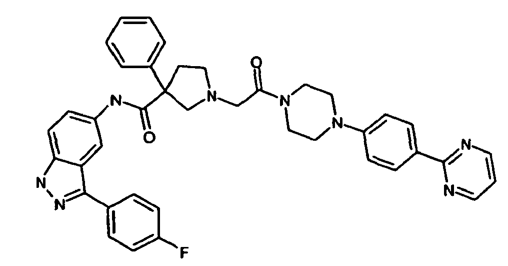 | 681.2 | 4.21 |
| 592 | 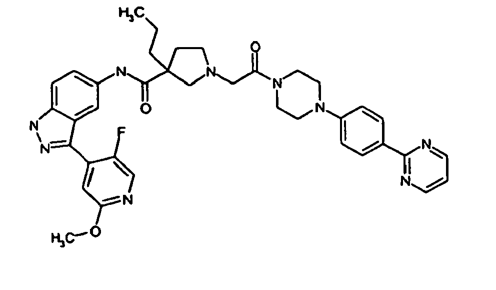 | 678.4 | 2.95 |
| 593 | 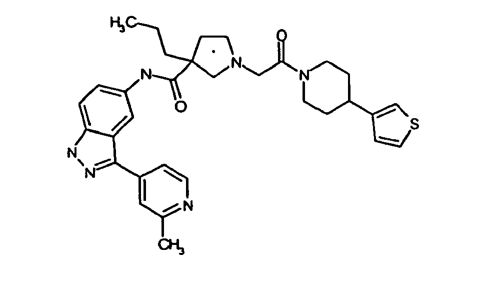 | 571.3 | 2.61 |
| 594 | 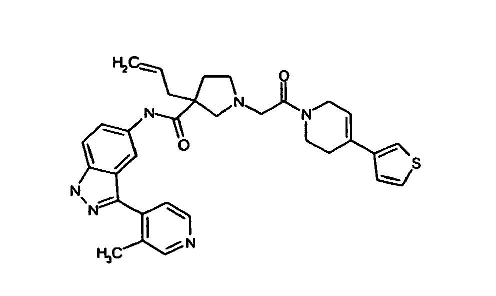 | 567.3 | 2.1 |
| 595 | 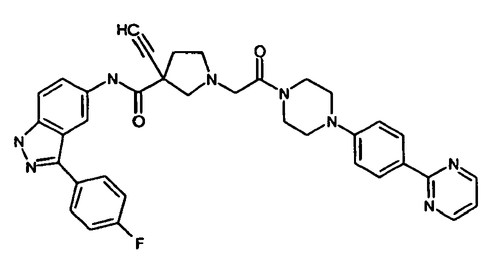 | 629 | 2.78 |
| 596 | 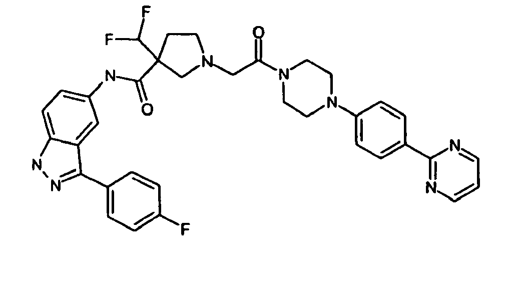 | 655.4 | |
| 597 | 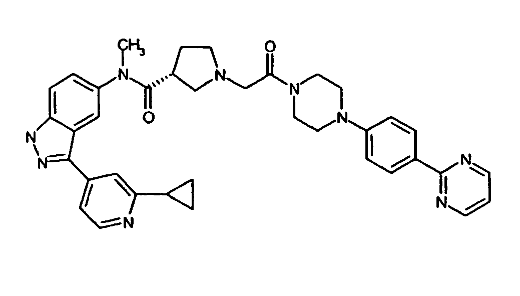 | 642.4 | 1.85 |
| 598 | 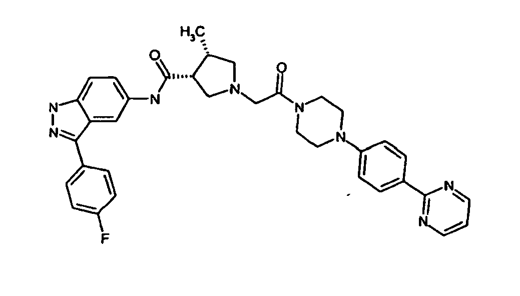 | 619.4 | 3.71 |
| 599 | 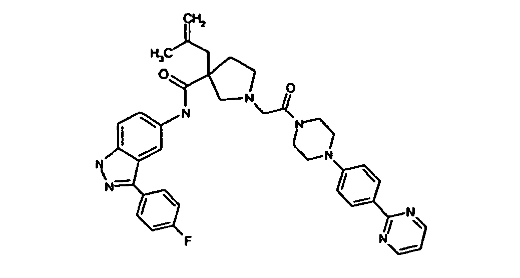 | 659 | 2.94 |
| 600 | 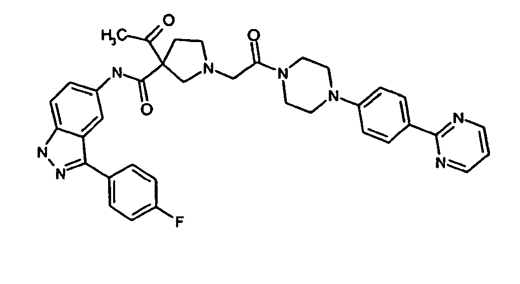 | 647 | 2.91 |
| 601 | 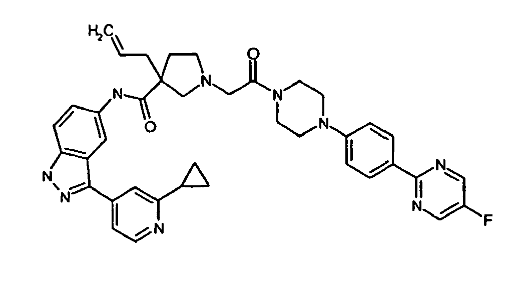 | | |
| 602 | 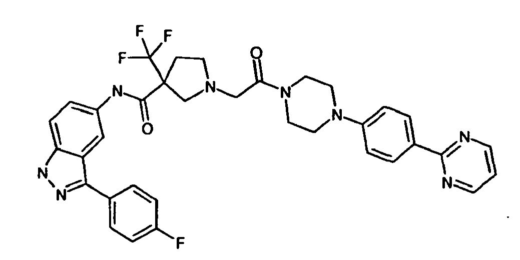 | | |

### Preparation of 37

### Step 1: Preparation of 3-cyano-3-trimethylsilanyloxy-pyrrolidine-1-carboxylic acid tert-methyl ester

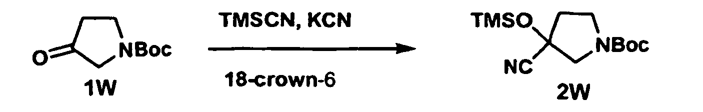

To a solution of 3-oxo-pyrrolidine-1-carboxylic acid *tert*-butyl ester **1W** (10 g, 52.3 mmol) in CH₂Cl₂ (150 ml) at 0 °C was added trimethylsilyl cyanide (8.5ml, 63.6 mmol), potassium cyanide (0.34 g, 5.23 mmol) and 18-crown-6 (1.38 g, 5.23 mmol). The reaction mixture was brought to room temperature and stirred overnight. The reaction mixture was cooled to 0 °C and quenched with saturated NaHCO₃ (200 ml). The organic layer was separated, dried over Na₂SO₄, filtered and concentrated. The residue was purified on silica gel eluting with 1/8 EtOAc/hexane to give the desired product **2W** (2 g, 81%).

### Step 2: Preparation of 3-hydroxy-pyrrotidine-1,3-dicarboxylic acid 1-tert-butyl ester 3-methyl ester

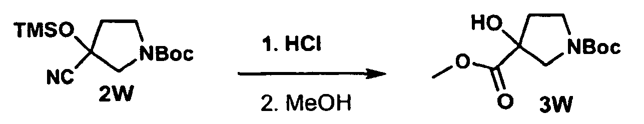

A mixture of compound **2W** (5.3 g, 25 mmol), MeOH (50 ml), 4N solution of HCl in dioxane (10 ml) was heated in a sealed tube at 70 °C for overnight. The reaction mixture was concentrated and THF (20 ml) was added followed by CH₂Cl₂ (50 ml), triethylamine (16 ml) and di-*tert*-butyl dicarbonate (11g). The reaction mixture was stirred at room temperature for overnight and then concentrated. Diluted the residue with ether (200 ml) and washed with water (100 ml). The organic layer was separated, dried over Na₂SO₄, filtered and concentrated. The residue was purified on silica gel eluting with 1/1 EtOAc/hexane then 2/1 EtOAC/hexane to give the desired product **3W** (4.35 g, 71%).

### Step 3: Preparation of 3-(benzo[1,3] dithiol-2-yloxy)-pyrrolidine-1,3-dicarboxylic acid 1-tert-butyl ester 3-methyl ester

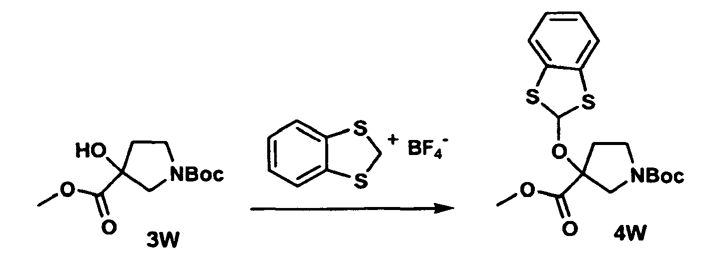

To a solution of compound **3W** (2.8 g, 11.4 mmol) in CH₂Cl₂ (100 ml) was added 1,3-benzodithiol-2-ylium tetrafluoroborate (5.4 g, 22.8 mmol) followed by pyridine (0.2 ml). The reaction mixture was stirred at room temperature for five days. Quenched the reaction mixture with triethylamine (9.6 ml) and stirred for 15 minutes. The reaction mixture was washed with water (100 ml), dried over Na₂SO₄, filtered and concentrated. The residue was purified on silica gel eluting with 1/4 EtOAc/hexane then 1/2 EtOAC/hexane to give the desired product **4W** (2.75 g, 61 %).

### Step 4: Preparation of 3-difluoromethoxy-pyrrolidine-1,3-dicarboxylic acid 1-tert-butyl ester 3-methyl ester

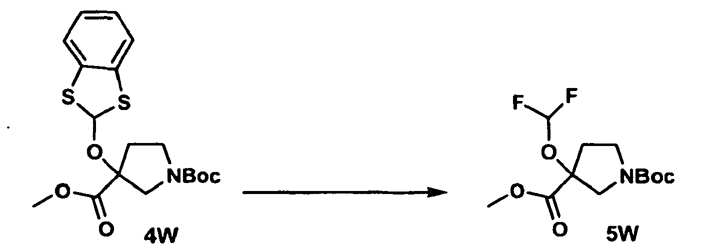

To a solution of compound **4W** (2.45 g, 6.16 mmol) in CH₂Cl₂ (100 ml) at 0 °C was added DAST (3.97 g, 24.64 mmol). After 5 minutes NIS (4.29 g, 18.5 mmol) was added. The reaction mixture was brought to room temperature and stirred for 2 hours. The reaction mixture was then cooled to 0 °C and treated carefully with saturated NaHCO₃ (60 ml) and stirred for 15 minutes. Diluted with CH₂Cl₂ (100 ml) and washed with saturated NaHCO₃ (2 x 100 ml). The organic was dried over Na₂SO₄, filtered and concentrated. The residue was purified on silica gel eluting with 1/4 EtOAc/hexane desired product **5W** (0.45 g, 25%).

### EXAMPLES 603-605

Following procedures similar to those described herein, for example, Examples 1, 3 to 60, 85, 98, 128, 183, 184, the compounds in Table 24 were prepared from compound **5W**. In Table 23 "Ex" represents "Example".

**Table 23**

| **Ex** | **Compound** | **Mass spec LCMS MH** | **Retention time (minutes)** |
|---|---|---|---|
| 603 | 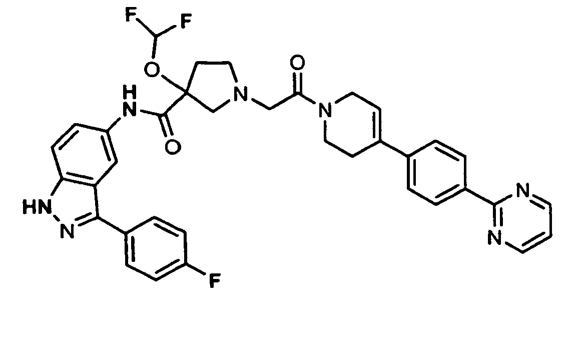 | 668 | 3.16 |
| 604 | 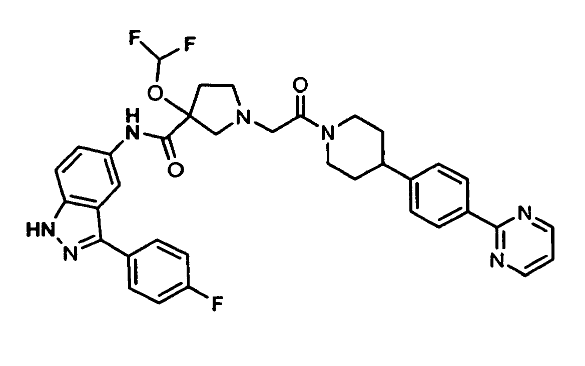 | 670 | 3.14 |
| 605 | 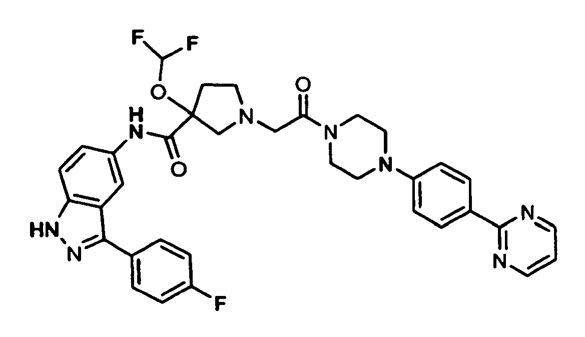 | 671 | 3.13 |

### Preparation of 38

### Step 1: Preparation of 4-(4-bromo-2-fluoro-phenyl)-3,6-dihydro-2H-pyridine-1-carboxylic acid tert-butyl ester

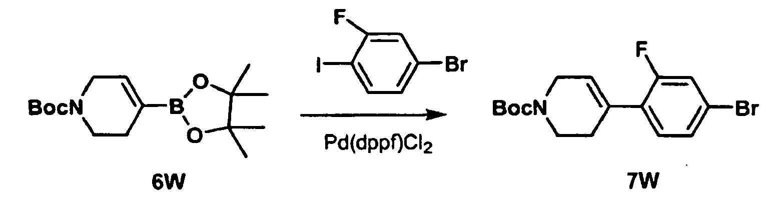

A mixture of compound **6W** (1 g, 3.23 mmol), 4-bromo-2-fluoro-1-iodo-benzene (1.46 g, 4.85 mmol), potassium carbonate (1.4 g, 9.69 mmol), Pd(dppf)Cl2 (0.264 g, 0.323 mmol) and 4/1 /dioxane/water (10 ml) was degassed for 15 minutes. Then it was heated at 80 °C for overnight. Cooled to room temperature and diluted with EtOAc (200 ml). The organic layer was washed with water (100 ml), dried over Na₂SO₄, filtered and concentrated. The residue was purified on silica gel eluting with 1/10 EtOAc/hexane to give the desired product **7W** (0.9 g, 78%).

### Step 2: Preparation of 4-(2-fluoro-4-pyrimid-2-yl-phenyl)-3,6-dihydro-2H pyridine-1-carboxylic acid 1-tert-butyl ester

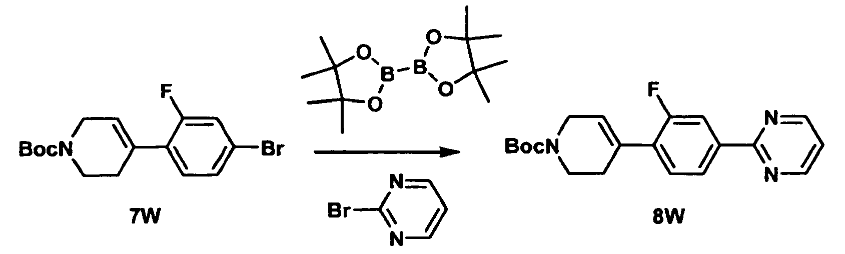

A mixture of compound **7W** (0.9 g, 2.53 mmol), bis(pinacolato)diboron (0.96 g, 3.79 mmol), potassium acetate (0.74 g, 7.6 mmol), Pd(dppf)Cl₂ (0.21 g, 0.25 mmol) and dimethyl sulfoxide (10 ml) was degassed for 10 minutes. Then it was heated at 100 °C for overnight. The reaction mixture was cooled to room temperature and potassium carbonate (1.75 g, 12.63 mmol), 2-bromopyrimidine (0.48g, 3.03 mmol) and water (10 ml) were added. The mixture was again purged with nitrogen for 20 min. Palladium tetrakistriphenylphosphine (0.29 g, 0.25 mmol) was added and the reaction mixture was stirred at 100 °C for a further 2 hr. Cooled to room temperature, filtered through a pad of celite and washed with ethyl acetate. Diluted with water (50 ml) and the organic layer was separated. The organic layer was dried over Na₂SO₄, filtered and concentrated. The residue was purified on silica gel eluting with 1/5 EtOAc/hexane to give the desired product **8W**.

### Preparation of 39

### Step 1: Preparation of 4-(4-bromo-3-fluoro-phenyl)-3,6-dihydro-2H-pyridine-1-carboxylic acid tert-butyl ester

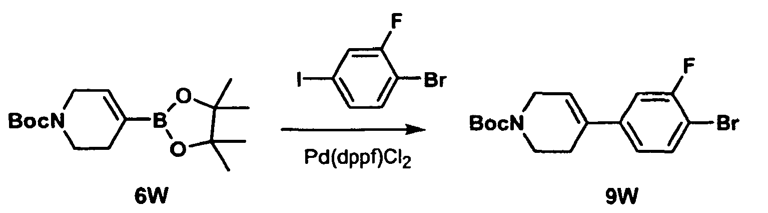

The compound **9W** was prepared from compound 6W using essentially the same procedure as described for the preparation of compound 7W from compound **6W**.

### Step 2: Preparation of 4-(3-fluoro-4-pyrimid-2-yl-phenyl)-3,6-dihydro-2H pyridine-1-carboxylic acid 1-tert-butyl ester

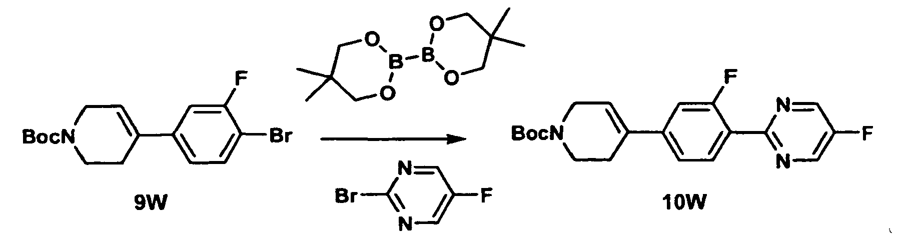

The compound **10W** was prepared from compound **9W** using essentially the same procedure as described for the preparation of compound **8W** from compound 7W but using bis(neopentylglycolato)diboron and 2-bromo-6-fluoro-pyrimidine in place of bis(pinacolato)diboron and 2-bromo-pyrimidine.

### Preparation 40

### Preparation of methyl α, α-dimethoxypropionate

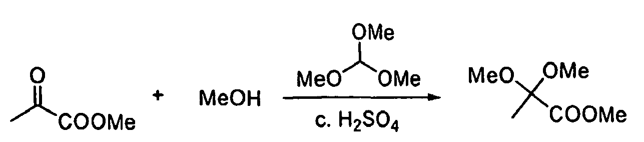

A procedure similar to Ernest Wenkert, et al. (JACS, 1983, 105, 2021-2029) was followed. A solution of methyl pyruvate (44g), trimethyl orthoformate (62 ml), concentrated H₂SO₄ (0.2 ml) in MeOH (120 ml) was refluxed for 4 hours. In the next one hour period, solvent (about 80 ml) was distilled out. The reaction mixture was cooled to 10 C, poured into a KOH solution (1.2 g KOH in 600 ml water), and extracted with ether (3x). Combined ether extracts were washed with brine and dried (MgSO₄). After concentration, the residue was distilled under vacuum to provide the acetal (40g, 62%, 40-43C/1 torr).

### Preparation 41

### Preparation of 2-methoxyacrylate

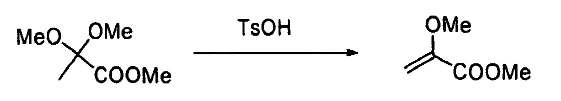

The procedure by Ernest Wenkert, et al. (JACS, 1983, 105, 2021-2029) was followed. To a one neck flask was charged α, α-dimethoxypropionate (150 g) and Tolunesulfonic acid monohydrate (3g) and a short path distillation head was attached. The mixture was heated at 140 C (oil bath temperature) and methanol began to come out first. The product (76 g) was then distilled out later after oil bath temperature was raised over 190C.

### Preparation 42

### Preparation of 1-benzyl-3-methoxy-pyrrolidine-3-carboxylic acid methyl ester

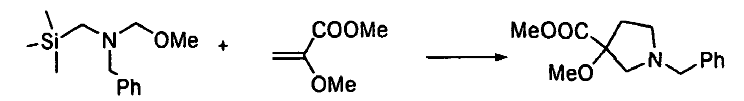

To a stirred solution of methyl 2-methoxyacrylate (20.8 g, 179 mmols) and *N-*(methoxymethyl)-*N*-(trimethylsilylmethyl)benzylamine (55 ml, 215 mmols) in dichloromethane (160 ml) was added at 0 C a solution of trifluoroacetic acid (2ml) in dichloromethane (10 ml). The resulting solution was warmed to room temperature and stirred overnight. After concentration, the crude product was purified by column chromatography on silica gel eluting with a solution of ethyl acetate/hexanes/Et₃N (1000:3000:4 to 1000:1000:3) to give the title compound (17.7 mg, 40 %).

### Preparation 43

### Preparation of 3-methoxy-pyrrolidine-3-carboxylic acid methyl ester tartaric acid salt

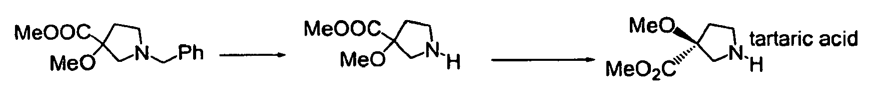

2.49 gm of 1-benzyl-3-methoxy-pyrrolidine-3-carboxylic acid methyl ester was hydrogenated in ethanol using 10% Pd/C at 55 psi hydrogen for 24 hrs. Filtration of the Pd/C followed by evaporation of the ethanol 1.6 gm of crude de-benzylated product. The crude product was dissolved in 95 ml of methanol and 1.35 gm of L-tartaric acid added. After 24 hrs, the crystals were filtered and re-crystallized from methanol to give 13. 4 grams of title product.

### EXAMPLES 606-608

Following procedures similar to those described herein, for example, Preparations 38 to 43, and Examples 1, 3 to 60, 85, 98, 128, 183, 184, the compounds in Table 25 were prepared. In Table 25 "Ex" represents "Example".

**Table 25**

| **Ex** | **Compound** | **Mass spec LCMS MH** | **Retention time (minutes)** |
|---|---|---|---|
| 606 | 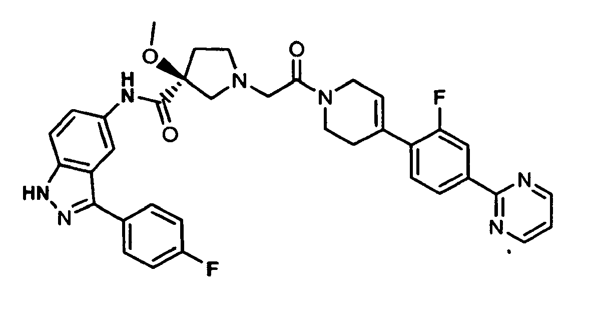 | 650 | 3.28 |
| 607 | 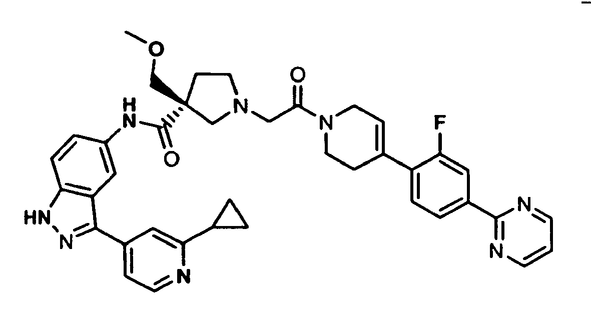 | 687 | 2.55 |
| 608 | 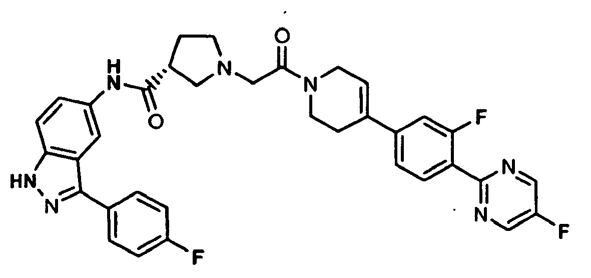 | 638 | 3.33 |

### Preparation 44

### Preparation of 3-(2-ethylamino-pyridin-4-yl)-1-trityl-1H-indazol-5-ylamine

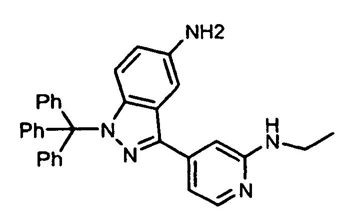

### Step 1: Preparation of 3-(2-fluoro-pyridin-4-yl)-5-nitro-1-trityl-1H-indazole

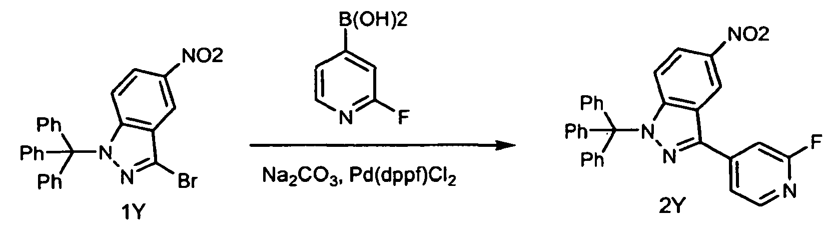

A mixture of 3-bromo-5-nitro-1-trytyl-1*H*-indazole (200 mg, 0.41 mmol), 2-fluoro-4-pyridine boronic acid (76 mg, 0.54 mmol), K₃PO₄ (174 mg, 0.82 mmol), Pd(dppf)Cl₂ (34 mg, 0.041 mmol) and 4/1 /dioxane/H₂O (10 mL) was stirred at 100 °C for 18 hours. Cooled to room temperature, poured into EtOAc (200 mL) and washed with water (100 mL). The organic layer was dried over Na₂SO₄, filtered and concentrated. The crude product was purified by column chromatography on silica get eluting with a solution of 15% ethyl acetate in hexane to give the title compound (90 mg, 44%).

### Step 2: Preparation of ethyl-[4-(5-nitro-1-trityl-1H-indazol-3-yl)-pyridin-2-yl]-amine

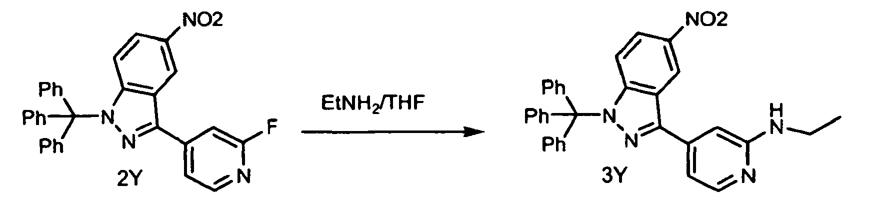

A mixture of 3-(2-fluoro-pyridin-4-yl)-5-nitro-1-trityl-1*H*-indazole (800 mg, 1.6 mmol) and 2M ethyl amine in THF (20 mL) was heated in a sealed tube at 80 °C for four days. Cooled to room temperature and concentrated. The crude product was purified by column chromatography on silica get eluting with a solution of ethyl acetate in hexane (1:1) to give the title compound (300 mg, 36%).

### Step 3: Preparation of 3-(2-ethylamino-pyridin-4-yl)-1-trityl-1H-indazol-5-ylamine

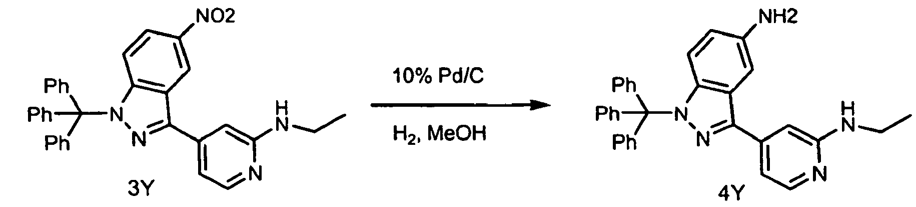

A mixture of ethyl-[4-(5-nitro-1-trityl-1H-indazol-3-yl)-pyridin-2-yl]-amine (30 mg), 10% Pd/C (25 mg) and MeOH (15 mL) was stirred at room temperature under hydrogen atmosphere for 4 hours. Filtered the catalyst, washed with MeOH and concentrated to give the title compound 4 (26 mg) which was used in the next reaction without further purification.

### Preparation 45

### Preparation of 3-(2-methylamino-pyridin-4-yl)-1-trityl-1H-indazol-5-ylamine

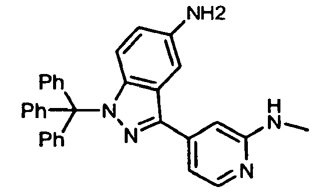

3-(2-methylamino-pyridin-4-yl)-1-trityl-1*H*-indazol-5-ylamine was prepared following essentially the same procedure as in Preparation 44, except that methylamine is used in Step 2 instead of ethylamine.

### EXAMPLES 609-613

Following procedures similar to those described herein, for example, Preparations 44 and 45, and Examples 1, 3 to 60, 85, 98, 128, 183, 184, the compounds in Table 26 were prepared.

In the preparation of the compound of Example 612, cyclopropylamine is used instead of ethylamine in Step 2 of Preparation 44.

In Table 26 "Ex" represents "Example".

**Table 26**

| **Ex** | **Compound** |
|---|---|
| 609 | 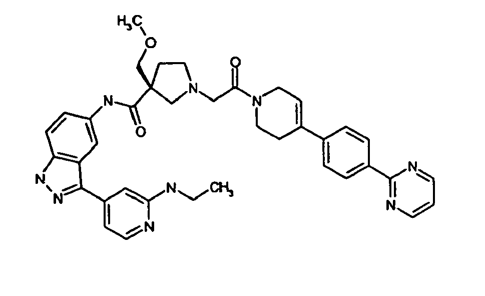 |
| 610 | 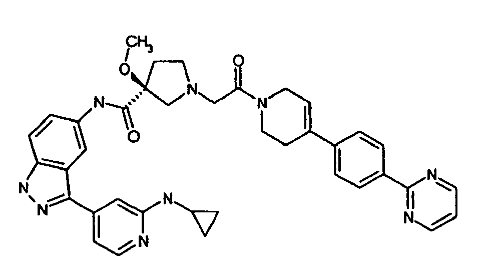 |

### EXAMPLE 611

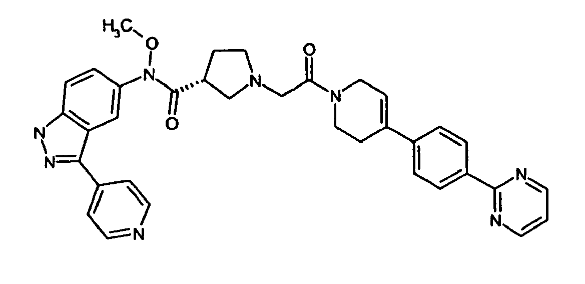

### Step 1:

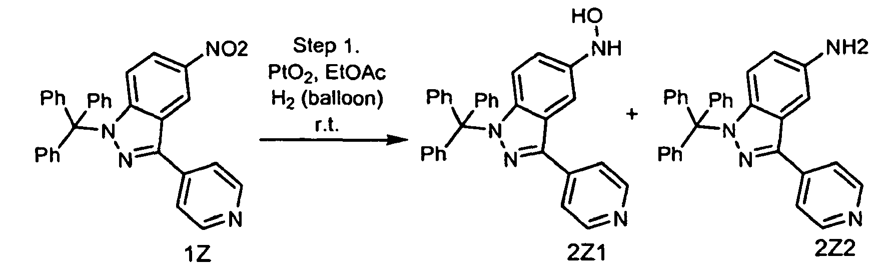

To a solution of compound 1 (510mg, 1.06mmol) in ethyl acetate (15ml), platinum oxide on charcoal (825mg, 0.011 mmol, 5%Pt, 50% wet) was added. The mixture was stirred under hydrogen (balloon) at r.t. until starting material disappeared. The mixture was filtered through Celite and solvents were removed in vacuum to give a mixture of compound **2Z1** and compound **2Z2**. The mixture was used in the next step without further purification.

### Step 2:

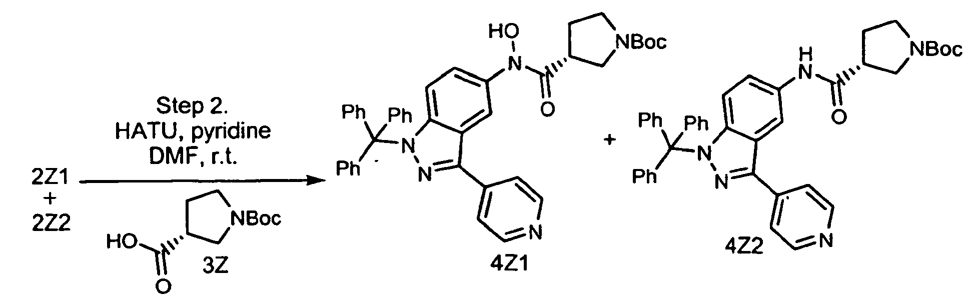

The mixture from step 1 was dissolved in N, N-dimethylformamide (10ml), HATU (484mg, 1.27mmol), compound **3Z** (274mg, 1.27mmol) followed by pyridine (0.11 ml, 1.27mmol) were added. The mixture was stirred at r.t. overnight. Water and ethyl acetate were added and layers were separated. The organic layer was washed with water (X2), dried (MgSO₄), filtered and solvents were removed in vacuum. Chromatographic purification [hexanes - acetone, 4:1 (v/v)] gave less polar compound **4Z2** (241 mg, 35%) as yellow oil. Continuous elution with the same solvent system gave more polar compound **4Z1** (161 mg, 23%) also as yellow oil.

### Step 3:

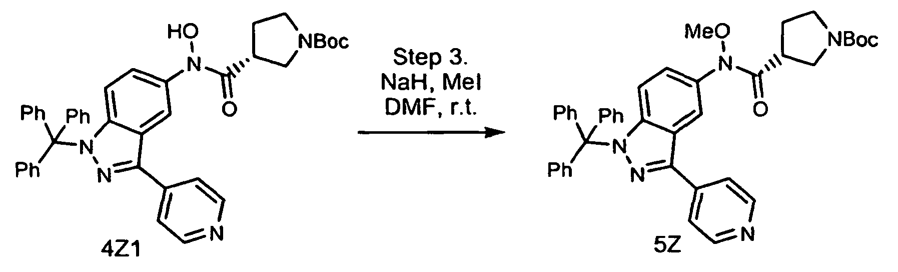

To a suspension of sodium hydride (14mg, 0.34mmol, 60% in oil) in N, N-dimethylforamide (5ml) at 0 °C, a solution of compound 4A (152mg, 0.23mmol) in N, N-dimethylforamide (2ml) was added. The mixture was stirred at 0 °C for 20 min and methyl iodide (21 µL, 0.34mmol) was added. The mixture was warmed to r.t. and was quenched with saturated ammonium chloride solution. Water and ethyl acetate were added and layers were separated. The organic layer was dried (MgSO₄), filtered and solvents were removed in vacuum. Chromatographic purification [hexanes - ethyl acetate, 1:1 (v/v)] gave compound **5Z** (23mg, 15%) as yellow oil.

### Step 4:

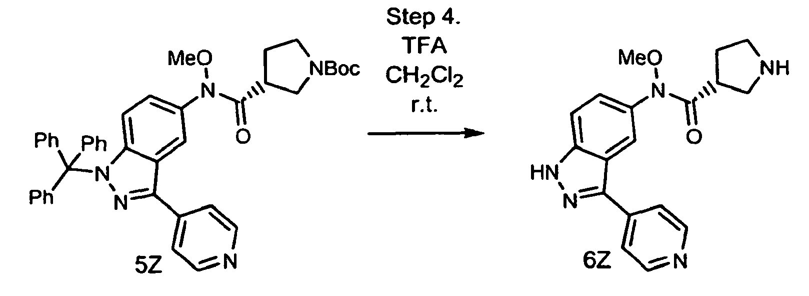

Compound **5Z** (15mg, 0.022mmol) was stirred in a mixture of dichloromethane (3 ml) and trifluoroacetic acid (1 ml) at r.t. for 5 hr. Solvents were removed in vacuum. Chromatographic purification [methanol (7N ammonia) - dichloromethane, 1:4 (v/v)] gave compound **6Z** as yellow oil (3.5mg, 30%).

### Step 5:

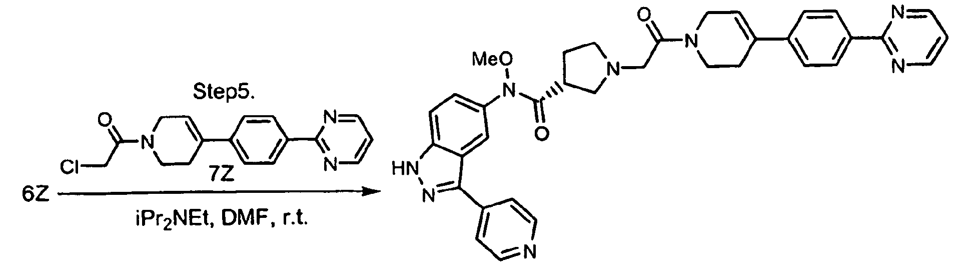

A mixture of compound **6Z** (3.5mg, 0.010mmol), compound **7Z** (3.9mg, 0.012mmol) and diisopropylethylamine (4µL, 0.021 mmol) in N, N-dimethylforamide (0.5ml) was stirred at r.t. overnight. Water and ethyl acetate were added and layers were separated. The organic layer was washed with water (X2), dried (MgSO₄), filtered and solvents were removed in vacuum. Chromatographic purification [methanol (7N ammonia) - dichloromethane, 1:9 (v/v)] gave title compound as yellow oil (2 mg, 31%). LCMS MH⁺ = 615 (RT = 2.27 min).

### ASSAYS

### Coupled ERK2 Assay:

Activity of compounds against inactive ERK2 was tested in a coupled MEK1/ERK2 IMAP assay as follows: Compounds were diluted to 25x final test concentration in 100% DMSO. 14µl of kinase buffer (10mM Tris.HCl pH 7.2, 10mM MgCl₂, 0.01 % Tween-20, 1 mM DTT) containing 0.4ng unphosphorylated Mouse ERK2 protein was added to each well of a black 384-well assay plate. 1µl of 25x compound was added to each well and incubated at room temperature for 30 minutes to allow an opportunity for the compound to bind to the inactive enzyme. DMSO concentration during initial incubation is 6.7%. ERK2 activity was determined to be insensitive to DMSO concentrations up to 20%. ERK2 was then activated and it's kinase activity measured by the addition of 10µl kinase buffer with the following components (final concentration per reaction): 2ng active (phosphorylated) human MEK1 protein and 4µM (total) ERK2 IMAP substrate peptides (3.9µM unlabeled IPTTPITTTYFFFK-CONH₂ and 100nM IPTTPITTTYFFFK(5-carboxyfluorescein)-CONH₂) and 30µM ATP. DMSO concentration during ERK activation was 4%. After one hour, reactions were terminated by addition of 60µl IMAP detections beads in binding buffer (Molecular Devices). Binding was allowed to equilibrate for 30 minutes before reading the plate on an LJL Analyst Fluorescence Polarization plate reader. Compound inhibition was calculated relative to DMSO and fully inhibited standards. Active compounds were reconfirmed in an independent assay.

### Active ERK2 Assay:

Activated ERK2 activity was also determined in the IMAP assay format using the procedure outlined above. 1µl of 25x compound was added to 14µl of kinase buffer containing 0.25ng fully phosphorylated, active Mouse ERK2 protein. Following a 30 minute incubation, the reactions were initiated by addition of 10µl of kinase buffer containing 1µM ERK2 IMAP substrate peptide (0.9µM unlabeled IPTTPITTTYFFFK-CONH₂ and 100nM IPTTPITTTYFFFK(5-carboxyfluorescein)-CONH₂) and 30µM ATP. Reactions proceeded for 30 minutes before termination by addition of 60µl IMAP detection beads in binding buffer. Plates were read as above after 30 minute binding equilibration. Active compounds were reconfirmed in an independent assay.

### Soft Agar Assay:

Anchorage-independent growth is a characteristic of tumorigenic cell lines. Human tumor cells can be suspended in growth medium containing 0.3% agarose and an indicated concentration of a farnesyl transferase inhibitor. The solution can be overlayed onto growth medium solidified with 0.6% agarose containing the same concentration of ERK1 and ERK2 inhibitor as the top layer. After the top layer is solidified, plates can be incubated for 10-16 days at 37°C under 5% CO₂ to allow colony outgrowth. After incubation, the colonies can be stained by overlaying the agar with a solution of MTT (3-[4,5-dimethyl-thiazol-2-yl]-2,5-diphenyltetrazolium bromide, Thiazolyl blue) (1 mg/mL in PBS). Colonies can be counted and the IC₅₀'s can be determined.

### The AUC (area under the concentration-time curve during the first 6 hours (AUC₆ₕᵣ) in Table 27 below was determined using the Protocol of Cassette Accelerating Rapid Rat screen (CARRS)

### Animal dosing and sample collection

Male Sprague-Dawley rats (Charles River, Co.) were pre-cannulated (femoral artery) in order to facilitate precise blood sampling times, and to reduce the stress on the animals caused by serial bleedings. Following an overnight fast, two rats were dosed orally with one compound at a dose of 10 mg/kg in a 5-mL/kg dose volume. Blood was collected into heparin-containing tubes serially from each animal at 0.5,1, 2, 3, 4 and 6 h post-dosing and centrifuged to generate plasma. Approximately 100 µL of plasma were collected at the individual time points. The plasma samples were stored at -20 °C until analysis.

### Plasma sample and standard curve preparation

A set of 12 rat plasma samples was generated for each NCE (i.e. 6 timepoints and n = 2 rats). These 12 samples were pooled across the two rats at each timepoint to provide 6 pooled samples (one sample per time point) for each NCE. The pooled samples were assayed as cassettes of six (36 samples total) to provide data on the six compounds. The50-µL aliquots of the 36 plasma samples were placed into individual wells of a 96-well plate. An additional compound (often a structural analog of the test compounds) was selected as the internal standard. A mini-calibration curve was prepared (three points plus a zero) for each compound assayed. Drug-free rat plasma was measured into 1-mL aliquots and each aliquot was spiked with known concentrations of the compounds to generate standards of the desired concentrations. The concentrations of the standards were chosen to bracket the expected concentration of the pooled samples based on historical data from previous studies on other compounds. For this work, the standards were set to contain concentrations of 25, 250 and 2500 ng NCE/mL plasma. The plasma standards were precipitated in duplicate along with the samples. Protein precipitation occurred after addition of 150 µL of acetonitrile containing the internal standard at a concentration of 1 ng/mL into each sample well using the Tomtec Quadra 96 system. The precipitated samples and standards were vortexed and centrifuged in the 96-well plate. Approximately 50-100 µL of the supernatant were removed and placed into a fresh 96-well plate using the Tomtec Quadra 96 system. A volume of 5-10 µL of the supernatant was used for analysis by HPLC-MS/MS. The mini-standard curve was run in duplicate, once before and once after the samples. Thus, a total of 14 study samples plus standards were analyzed per compound. In addition, solvent blanks were injected before and after each set of 14 and after the highest calibration standard for each compound; therefore, a total of 103 injections were made into each HPLC system for each set of six compounds. Multiple solvent blank injections could be made from a single well. Twelve solvent blank wells were designated in each 96-well plate. Thus, one batch (cassette) of six NCEs was prepared and assayed using one 96-well plate format.

### HPLC-MS/MS analysis

All the compounds were analyzed using selected reaction monitoring (SRM) methods with LC/MS/MS instruments. Once the method development had been completed, the assay was quickly set up using a standard injection sequence template for the CARRS assay.

The final compounds of Examples 1, 2, 4-61, 65-73, 77-84, 86, 88-98, 100, 102-114, 116-118, 120, 121, 124, 125, 151-155, 159-179, 183, 184, 186, 188-193, 196-199, 202-205, 250, 253-259, 260-299, 301-318, 320-323, 332-347, 356 (Isomer Z), 356 (Isomer E), 357-360, 362, 362 (compound 9P), 364, 368-436, 440-509, 511-602, 606, 607 and 609-611 had an AERK2 IC50 in the range of 0.16 to 20,000 nM.

The final compounds of Examples 1, 2, 4-28, 61, 86, 88, 89, 92, 95, 98, 100, 120, 125, 132, 142, 152, 154, 168, 176, 183, 184, 186, 188-193, 196-199, 202-205, 250, 251, 253-259, 261, 264, 269, 271, 274-276, 282, 286, 289, 290, 292, 294, 302, 303, 304, 306, 314-316, 332, 333, 335, 338, 343, 358, 362, 368-370, 384-390, 440-493, 510-558, 606, and 611 had an AERK2 IC₅₀ in the range of 0.16 to 18 nM.

The final compounds of Examples 6, 100, 183, 184, 186, 188-192, 261, 440-450, and 510-517 had an AERK2 IC₅₀ in the range of 0.16 to 1.5 nM.

The final compound of Example 183 had an AERK2 IC₅₀ of 0.16 nM. The final compound of Example 186 had an AERK2 IC₅₀ of 0.78 nM. The final compound of Example 335 had an AERK2 IC₅₀ of 4.9 nM.

Table 27 provides AERK2 IC₅₀ data and Rat Auc data for compounds of this invention.

**Table 27**

| **Ex.** | **Compound** | **AERK2 IC50 (nM)** | Rat AUC PO (nM.h) |
|---|---|---|---|
| 336 | 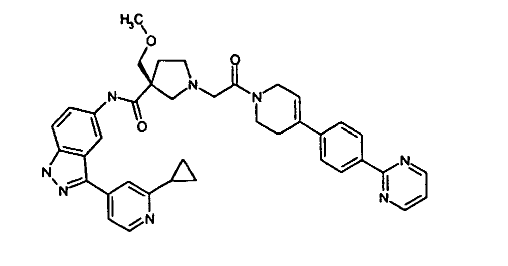 | 18.1 | 7219 |
| 469 | 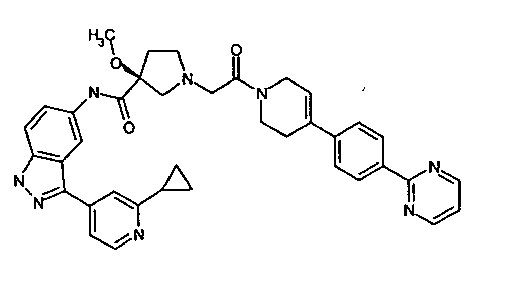 | 2.5 | 1200 |
| 480 | 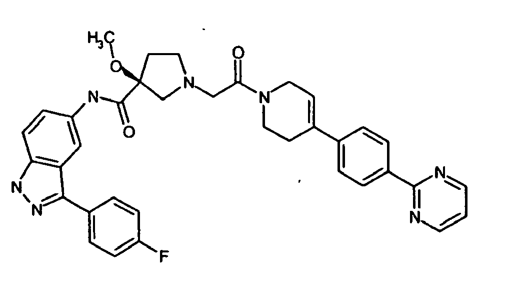 | 4.8 | 1880 |
| 489 | 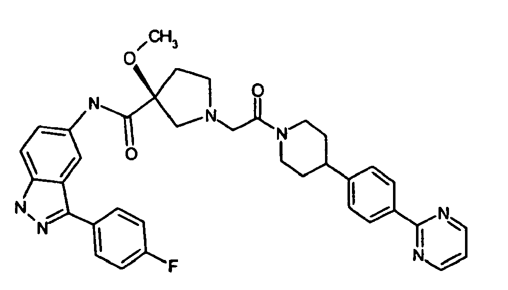 | 9.1 | 723 |
| 571 | 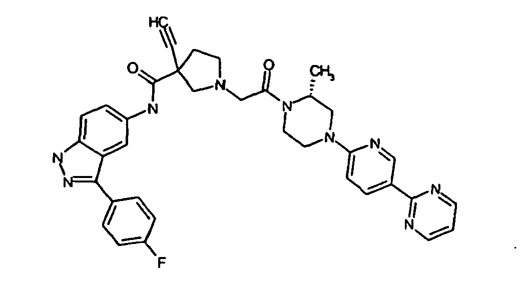 | 19.5 | 6466 |
| 413 | 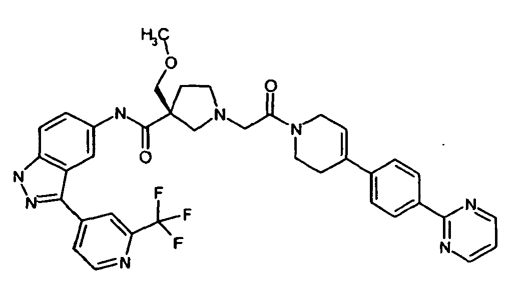 | 37.6 | 8946 |
| 412 | 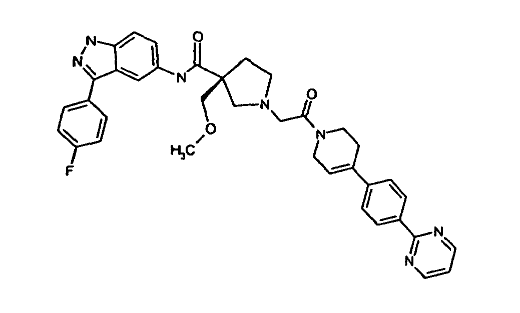 | 38.45 | 7836 |
| 462 | 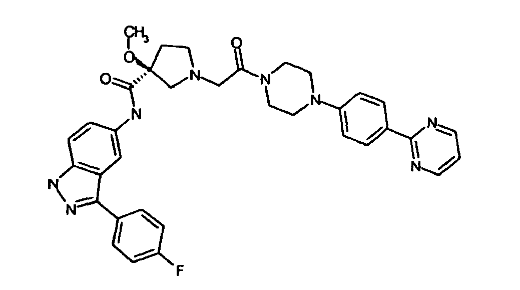 | 2.1 | 352 |
| 487 | 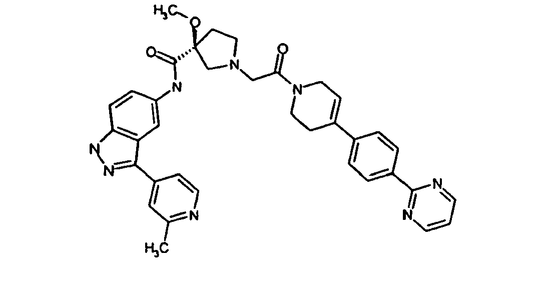 | 8.2 | 38 |
| 6 | 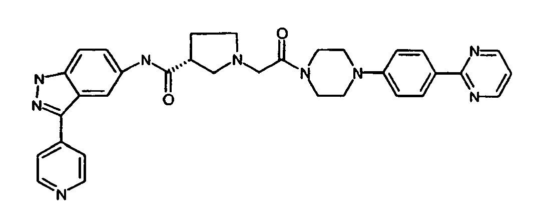 | 0.8 | 0 |

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington: The Science and Practice of Pharmacy, 20th Edition, (2000), Lippincott Williams & Wilkins, Baltimore, MD.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparations subdivided into suitably sized unit doses containing appropriate quantities of the active component, e.g., an effective amount to achieve the desired purpose.

The quantity of active compound in a unit dose of preparation may be varied or adjusted from about 0.01 mg to about 1000 mg, preferably from about 0.01 mg to about 750 mg, more preferably from about 0.01 mg to about 500 mg, and most preferably from about 0.01 mg to about 250 mg according to the particular application.

The actual dosage employed may be varied depending upon the requirements of the patient and the severity of the condition being treated. Determination of the proper dosage regimen for a particular situation is within the skill in the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 0.04 mg/day to about 4000 mg/day, in two to four divided doses.

## Claims

1. A compound of formula 1.0: 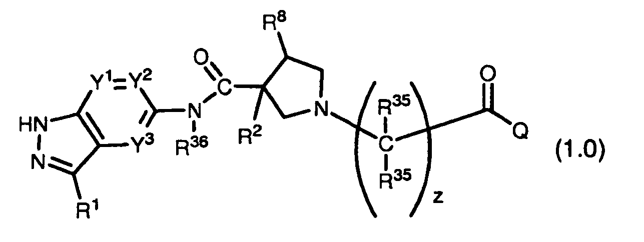 or the pharmaceutically acceptable salts thereof, wherein:
Y¹, Y², and Y³ are each independently selected from the group consisting of: -CH=, -N= and -CR⁹=;
z is 1 to 3;
Q is a substituent selected from the group consisting of: 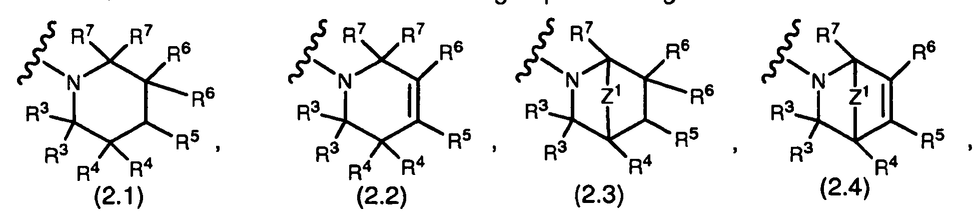 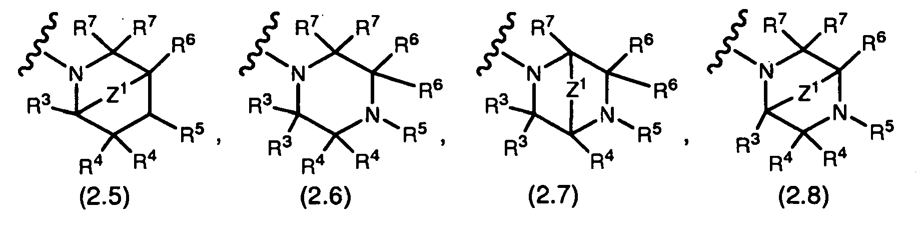 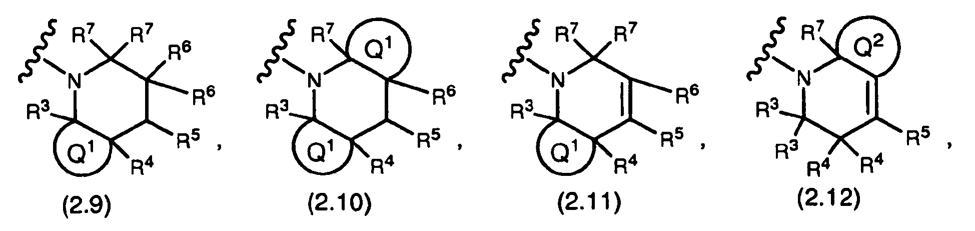 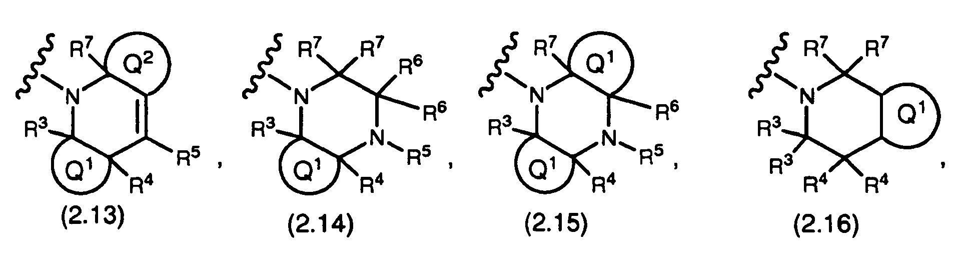 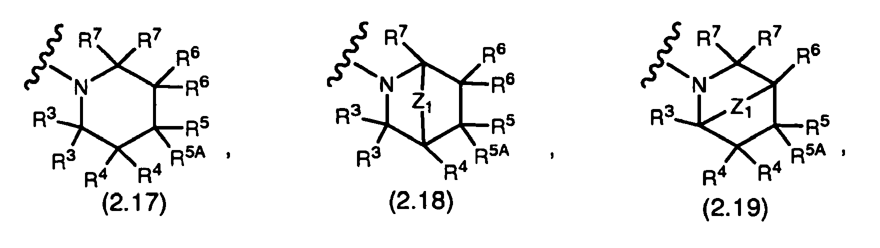 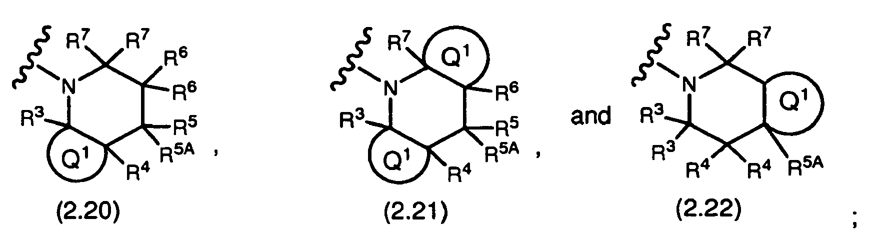
Each Q¹ represents a ring independently selected from the group consisting of: cycloalkyl, substituted cycloalkyl, heterocycloalkyl, substituted heterocycloalkyl, aryl, substituted aryl, heteroaryl, and substituted heteroaryl, wherein said substituted rings are substituted with 1 to 3 substituents independently selected from the group consisting of: the R¹⁰ moieties; provided that when Q¹ is aryl, heteroaryl, substituted aryl or substituted heteroaryl then the carbon atoms at the ring junction are not substituted;
Q² represents a ring selected from the group consisting of: cycloalkyl, substituted cycloalkyl, heterocycloalkyl, and substituted heterocycloalkyl, wherein said substituted rings are substituted with 1 to 3 substituents independently selected from the group consisting of: the R¹⁰ moieties;
Z¹ represents -(C(R²⁴)₂)_{w}- wherein each R²⁴ is independently selected from the group consisting of: H, alkyl and F, and wherein w is 1, 2 or 3;
Z² is selected from the group consisting of: -N(R⁴⁴)-, -O- and -C(R⁴⁶)₂-;
m is 1 to 6;
n is 1 to 6;
p is 0 to 6;
t is 0, 1, or 2;
R¹ is selected from the group consisting of:
(1) -CN,
(2) -NO₂,
(3) -OR¹⁰,
(4) -SR¹⁰,
(5) -N(R¹⁰)₂,
(6) R¹⁰,
(7) -C(O)R¹⁰,
(8) -(C(R³⁰)₂)ₙ-NR³²-C(O)-R¹⁰,
(9) -(C(R³⁰)₂)ₙ-NR³²-S(O)₁-R¹⁰,
(10) -(C(R³⁰)₂)ₙ-NR³²-C(O)-N(R³²)-R¹⁰,
(11) 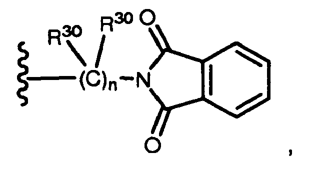
(12) -CF₃,
(13) -C(O)OR¹⁰,
(14) -(C(R³⁰)₂)ₙR¹³ (e.g., -(CH₂)ₙR¹³) wherein n is 1, each R³⁰ is H, and R¹³ is selected from the group consisting of: -OH and -N(R¹⁰)₂, wherein each R¹⁰ is independently selected,
(15) alkenyl,
(16) -NR³²-C(O)-R¹⁴,
(17) 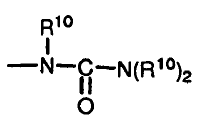 wherein each R¹⁰ is independently selected,
(18) 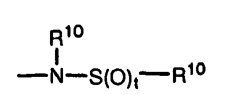 wherein each R¹⁰ is independently selected,
(19) 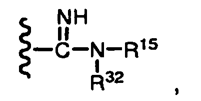
(20) -C(O)-NR³²-(C(R³⁰)₂)ₚ-OR¹⁰,
(21) -C(O)N(R¹⁰)₂ wherein each R¹⁰ is independently selected,
(22) -C(O)-NR³²-C(R¹⁸)₃ wherein each R¹⁸ is independently selected from the group consisting of: R¹⁰ and -C(O)OR¹⁹, and R¹⁹ is selected from the group consisting of:alkyl and substituted arylalkyl,
(23) -C(O)-NR³²-(C(R³⁰)₂)ₙ-C(O)-N(R¹⁰)₂,
(24) heterocycloalkenyl,
(25) 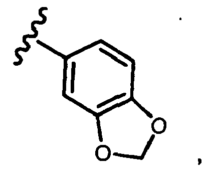 and
(26) arylalkenyl-;
R² is selected from the group consisting of:
(1) H,
(2) -CN,
(3) halo,
(4) alkyl,
(5) substituted alkyl wherein said substituted alkyl is substituted with 1 to 3 substitutents selected from the group consisting of: (a) -OH, (b) -O-alkyl (e.g., -O-(C₁-C₃alkyl), (c) -O-alkyl substituted with 1 to 3 F atoms, and (d) -N(R⁴⁰)₂ wherein each R⁴⁰ is independently selected from the group consisting of: (i) H, (ii) C₁-C₃ alkyl, (iii) - CF₃, and (e) halo,
(6) alkynyl,
(7) alkenyl,
(8) -(CH₂)ₘR¹¹,
(9) -N(R²⁶)₂,
(10) -OR²³,
(11) -N(R²⁶)C(O)R⁴²,
(12) cycloalkyl,
(13) cycloalkylalkyl,
(14) 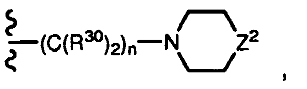
(15) -O-(substituted alkyl) wherein said substituted alkyl is substituted with 1 to 3 F atoms,
(16) -S(O)ₜ-alkyl,
(17) -C(O)-alkyl,
(18) 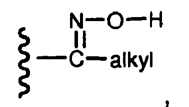
(19) 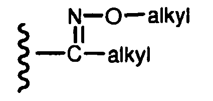 wherein each alkyl is independently selected,
(20) 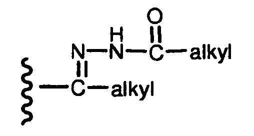 which each alkyl is independently selected,
(21) 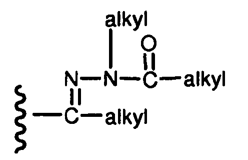 wherein each alkyl is independently selected,
(22) -N(R⁴⁸)-C(O)-R⁴⁸ wherein each R⁴⁸ is independently selected from the group consisting of: H and alkyl, and
(23) -C(O)-alkyl, such as, for example, -C(O)-(C₁-C₆ alkyl), such as, for example, -C(O)CH₃;
each R³, R⁴, R⁵, R⁶ and R⁷ is independently selected from the group consisting of:
(1) H,
(2) alkenyl,
(3) substituted alkenyl,
(4) alkyl,
(5) substituted alkyl,
(6) cycloalkyl,
(7) substituted cycloalkyl,
(8) cycloalkylalkyl-,
(9) substituted cycloalkylalkyl-,
(10) heterocycloalkyl,
(11) substituted heterocycloalkyl,
(12) heterocycloalkylalkyl-,
(13) substituted heterocycloalkylalkyl-,
(14) -C(O)R¹⁰,
(15) arylheteroaryl-,
(16) substituted arylheteroaryl-,
(17) heteroarylaryl-,
(18) substituted heteroarylaryl-,
(19) aryl,
(20) substituted aryl,
(21) heteroaryl,
(22) substituted heteroaryl,
(23) heteroarylheteroaryl-,
(24) substituted heteroarylheteroaryl-,
(25) arylaminoheteroaryl-,
(26) substituted arylaminoheteroaryl-,
(27) arylalkynyl-,
(28) substituted arylalkynyl-,
(29) heteroarylalkynyl-,
(30) substituted heteroarylalkynyl-,
wherein said R³, R⁴, R⁵, R⁶ and R⁷ substituted groups (7), (9), (11), (13), (16), (18), (20), (22), (24), (26), (28) and (30) are substituted with 1 to 3 substituents independently selected from the group consisting of: -NH₂, alkyl, alkenyl, halo, -C(O)-NH-R²⁸, -C(O)OR²⁸, and -C(O)R²⁸, and
wherein said R³, R⁴, R⁵, R⁶ and R⁷ substituted groups (3) and (5) are substituted with 1 to 3 substituents independently selected from the group consisting of: -NH₂, halo (e.g., F, Cl and Br, and in another example F), -C(O)-NH-R²⁸ (e.g., -C(O)-NH-CH₃), -C(O)OR²⁸ (e.g., -C(O)OC₂H₅), and -C(O)R²⁸ (e.g., -C(O)CH₃);
R^{5A} is selected from the group consisting of: halo, -OH, and -O-alkyl;
R⁸ is selected from the group consisting of: H, -OH, -N(R¹⁰)₂, -NR¹⁰C(O)R¹², and alkyl;
each R⁹ is independently selected from the group consisting of:halogen, -CN, -NO₂, -OR¹⁰, -SR¹⁰, -N(R¹⁰)₂, and R¹⁰;
each R¹⁰ is independently selected from the group consisting of: H, alkyl, aryl, arylalkyl, heteroaryl, heteroarylalkyl, cycloalkyl, cycloalkylalkyl, heterocycloalkyl, heterocycloalkylalkyl, alkylheteroaryl-, alkylaryl-, substituted alkyl, substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl-, substituted alkylaryl-, heterocycloalkenyl, and substituted heterocycloalkenyl, and wherein:
said R¹⁰ substituted alkyl is substituted with 1 to 3 substituents independently selected from the group consisting of: -NH₂, -NHR²⁰, -NO₂, -CN, -OR²⁶, halo, -C(O)-NH-R²⁶, -C(O)OR²⁶, and -C(O)R²⁶, and
said R¹⁰ substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl- are substituted with 1 to 3 substituents independently selected from the group consisting of: (1) -NH₂, (2) -NO₂, (3) -CN, (4) -OH, (5) -OR²⁰, (6) -OCF₃, (7) alkyl substituted with 1 to 3 independently selected halo atoms, (8) -C(O)R³⁸, (9) alkyl, (10) alkenyl, (11) halo, (12) -C(O)-NH-R²⁶, (13) -C(O)OR³⁸, (14) -C(O)-NR³²-(C(R³⁰)₂)ₙ-N(R³⁸)₂, (15) -S(O)ₜR³⁸, (16) -C(O)-NR³²-R³⁸, (17) -NR³²-C(O)-R³⁸, (18) 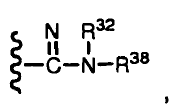 (19) -NHR²⁰, and (20) cycloalkyl;
R¹¹ is selected from the group consisting of: F, -OH, -CN, -OR¹⁰, -NHNR¹R¹⁰, -SR¹⁰ and heteroaryl;
R¹² is selected from the group consisting of: alkyl, aryl, heteroaryl, cycloalkyl, cycloalkylalkyl, heterocycloalkyl and heterocycloalkylalkyl;
R¹⁴ is selected from the group consisting of: alkyl, aryl, heteroaryl, cycloalkyl, cycloalkylalkyl-, heterocycloalkyl, alkylheterocycloalkyl, heterocycloalkylalkyl-, alkylheteroaryl- and alkylaryl-;
R¹⁵ is selected from the group consisting of: H, -OH, alkyl, aryl, heteroaryl, cycloalkyl, cycloalkylalkyl-, heterocycloalkyl and heterocycloalkylalkyl-, alkylheteroaryl- and alkylaryl-;
R²⁰ represents alkyl;
R²³ is selected from the group consisting of: H, alkyl, aryl, cycloalkyl, and cycloalkylalkyl-;
each R²⁶ is independently selected from the group consisting of: H and alkyl;
R²⁸ is alkyl;
each R³⁰ is independently selected from the group consisting of: H, alkyl, and F;
each R³² is independently selected from the group consisting of: H and alkyl, and wherein each R³² is generally H;
each R³⁵ is independently selected from the group consisting of: H and C₁ to C₆ alkyl;
R³⁶ is selected from the group consisting of: H, alkyl, and -O-alkyl;
each R³⁸ is independently selected from the group consisting of: H, alkyl, aryl, arylalkyl, heteroaryl, heteroarylalkyl, cycloalkyl, cycloalkylalkyl, heterocycloalkyl, heterocycloalkylalkyl, alkylheteroaryl-, alkylaryl-, substituted alkyl, substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl-, and wherein:
said R³⁸ substituted alkyl is substituted with 1 to 3 substituents independently selected from the group consisting of: -NH₂, -NO₂, -CN, -OR²⁶, halo, -C(O)-NH-R²⁸, -C(O)OR²⁸, and -C(O)R²⁸, and
said R³⁸ substituted aryl, substituted arylalkyl, substituted heteroaryl, substituted heteroarylalkyl, substituted cycloalkyl, substituted cycloalkylalkyl, substituted heterocycloalkyl, substituted heterocycloalkylalkyl, substituted alkylheteroaryl- and substituted alkylaryl- are substituted with 1 to 3 substituents independently selected from the group consisting of: (1) -NH₂, (2) -NO₂, (3) -CN, (4) -OH, (5) -OR²⁰, (6) -OCF₃, (7) -CF₃, (8) -C(O)R²⁶, (9) alkyl, (10) alkenyl, (11) halo, (12) -C(O)-NH-R²⁶, (13) -C(O)OR²⁶, (14) -C(O)-NR³²-(C(R³)₂)ₙ-N(R²⁶)₂, (15) -S(O)ₜR²⁶, (16) -C(O)N(R³²)(R²⁶), (17) -NR³²C(O)R²⁶, (18) 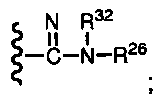 and (19) -NHR²⁰;
R⁴² is selected from the group consisting of: alkyl, aryl, heteroaryl, and cycloalkyl;
R⁴⁴ is selected from the group consisting of: H, alkyl, cycloalkyl, and cycloalkylalkyl; and
Each R⁴⁶ is independently selected from the group consisting of: H, alkyl, cycloalkyl, and cycloalkylalkyl.

2. The compound of Claim 1 having the formula selected from the group consisting of: 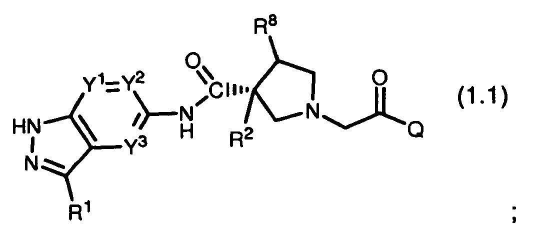 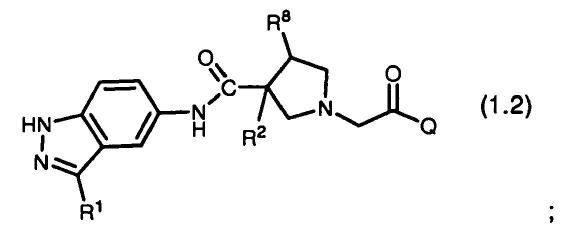 and 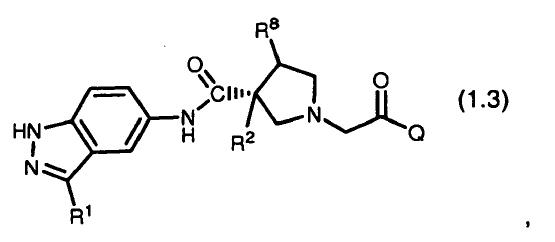 or a pharmaceutically acceptable salt thereof.

3. The compound of any of Claims 1 or 2, or a pharmaceutically acceptable salt thereof, wherein Q is selected from the group consisting of: 2.1, 2.2, 2.3, 2.4, 2.5, 2.6, 2.7, and 2.8.

4. The compound of any of Claims 1 or 2, or a pharmaceutically acceptable salt thereof, wherein Q is selected from the group consisting of: 2.17, 2.18, 2.19, 2.20, 2.21, and 2.22.

5. The compound of Claim 1, or a pharmaceutically acceptable salt thereof, wherein Z¹ is -CH₂-.

6. The compound of Claim 3, or a pharmaceutically acceptable salt thereof, wherein Q is selected from the group consisting of: moieties 2.1, 2.2, and 2.3.

7. The compound of Claim 6, or a pharmaceutically acceptable salt thereof, wherein Q is selected from the group consisting of: moieties 2.1, 2.2, and 2.3, and 2.3 is selected from the group consisting of:

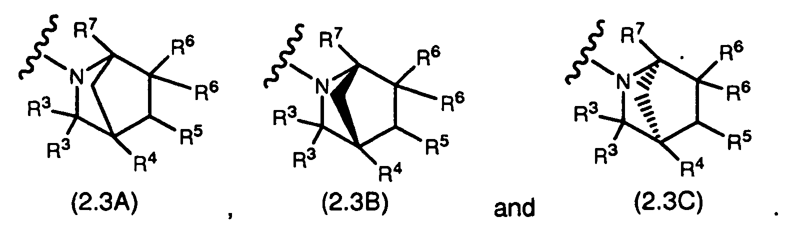

8. The compound of any of Claims 1 or 7, or a pharmaceutically acceptable salt thereof, wherein each R³, R⁴, R⁶, and R⁷ is independently selected from the group consisting of: H and alkyl.

9. The compound of Claim 8, or a pharmaceutically acceptable salt thereof, wherein each R³, R⁴, R⁶, and R⁷ is independently selected from the group consisting of: H and methyl.

10. The compound of Claim 9, or a pharmaceutically acceptable salt thereof, wherein each R³, R⁴, R⁶, and R⁷ is H.

11. The compound of Claim 3, or a pharmaceutically acceptable salt thereof, wherein Q is selected from the group consisting of: moieties 2.6 and 2.7.

12. The compound of Claim 11, or a pharmaceutically acceptable salt thereof, wherein Q is selected from the group consisting of: moieties 2.6 and 2.7, and 2.7 is selected from the group consisting of:

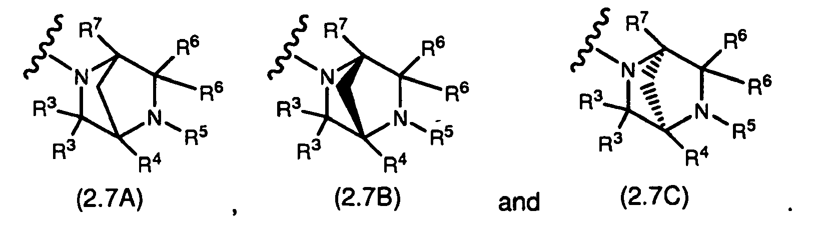

13. The compound of any of Claims 1, or 12, or a pharmaceutically acceptable salt thereof, wherein each R³, R⁴, R⁶, and R⁷ is independently selected from the group consisting of: H and alkyl.

14. The compound of Claim 13, or a pharmaceutically acceptable salt thereof, wherein each R³, R⁴, R⁶, and R⁷ is independently selected from the group consisting of: H and methyl.

15. The compound of Claim 14, or a pharmaceutically acceptable salt thereof, wherein each R³, R⁴, R⁶, and R⁷ is H.

16. The compound of Claim 10, or a pharmaceutically acceptable salt thereof, wherein Q is 2.1.

17. The compound of Claim 10, or a pharmaceutically acceptable salt thereof, wherein Q is 2.3B.

18. The compound of Claim 15, or a pharmaceutically acceptable salt thereof, wherein Q is 2.6.

19. The compound of Claim 15, or a pharmaceutically acceptable salt thereof, wherein Q is 2.7A.

20. The compound of Claim 15, or a pharmaceutically acceptable salt thereof, wherein Q is 2.7B.

21. The compound of Claim 1, or a pharmaceutically acceptable salt thereof, wherein Q is 2.17.

22. The compound of Claim 21, or a pharmaceutically acceptable salt thereof, wherein each R³, R⁴, R⁶, and R⁷ is independently selected from the group consisting of: H and methyl.

23. The compound of Claim 22, or a pharmaceutically acceptable salt thereof, wherein each R³, R⁴, R⁶, and R⁷ is H.

24. The compound of any of Claims 16, 17, 18, 19 or 20, or a pharmaceutically acceptable salt thereof, wherein the compound of formula 1.0 is:

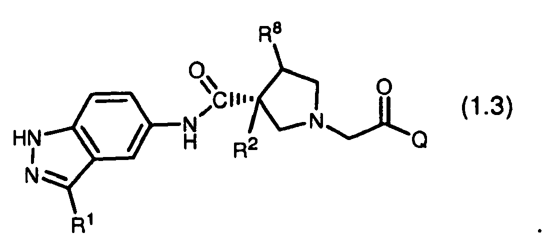

25. The compound of Claim 2, or a pharmaceutically acceptable salt thereof, wherein the compound of formula 1.0 is a compound of formula 1.3, and Q is selected from the group consisting of: 2.1, 2.2, and 2.3, and 2.3 is selected from the group consisting of: 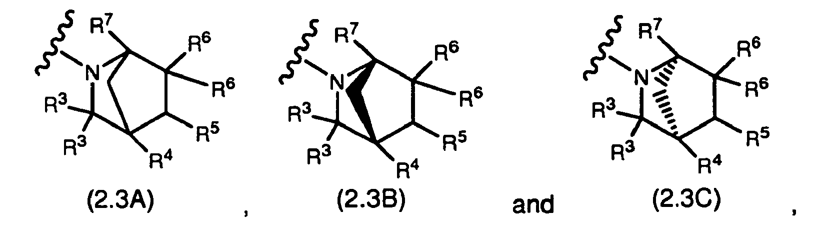 and R³, R⁴, R⁶, and R⁷ are each independently selected from the group consisting of: H and alkyl.

26. The compound of Claim 25, or a pharmaceutically acceptable salt thereof, wherein R³, R⁴, R⁶, and R⁷ are selected from the group consisting of: H and methyl.

27. The compound of Claim 26, or a pharmaceutically acceptable salt thereof, wherein Q is selected from the group consisting of: 2.1 and 2.3B.

28. The compound of Claim 27, or a pharmaceutically acceptable salt thereof, wherein Q is 2.1, and R³, R⁴, R⁶, and R⁷ are each H.

29. The compound of Claim 2, or a pharmaceutically acceptable salt thereof, wherein the compound of formula 1.0 is a compound of formula 1.3, and Q is selected from the group consisting of: 2.6 and 2.7, and 2.7 is selected from the group consisting of: 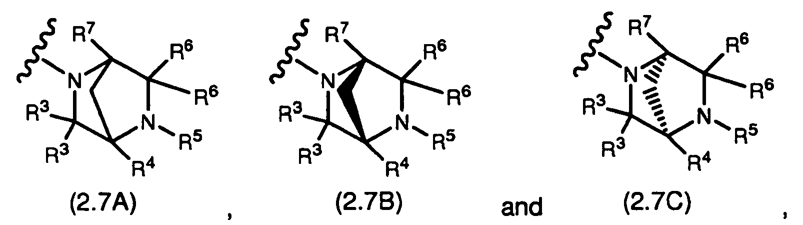 and R³, R⁴, R⁶, and R⁷ are each independently selected from the group consisting of: H and alkyl.

30. The compound of Claim 29, or a pharmaceutically acceptable salt thereof, wherein R³, R⁴, R⁶, and R⁷ are selected from the group consisting of: H and methyl.

31. The compound of Claim 30, or a pharmaceutically acceptable salt thereof, wherein Q is selected from the group consisting of: 2.6, 2.7A and 2.7B.

32. The compound of Claim 31, or a pharmaceutically acceptable salt thereof, wherein Q is 2.6, and R³, R⁴, R⁶, and R⁷ are each H.

33. The compound of Claim 31, or a pharmaceutically acceptable salt thereof, wherein Q is 2.7A, and R³, R⁴, R⁶, and R⁷ are each H.

34. The compound of Claim 31, or a pharmaceutically acceptable salt thereof, wherein Q is 2.7B, and R³, R⁴, R⁶, and R⁷ are each H.

35. The compound of Claim 1, or a pharmaceutically acceptable salt thereof, wherein Q is selected from the group consisting of:
(A) 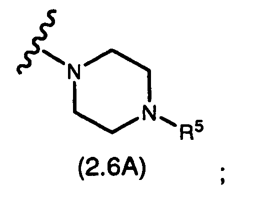
(B) 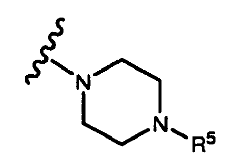 substituted with one or two substituents independently selected from the group consisting of R³ groups, provided that said one or two substitutents are not H;
(C) 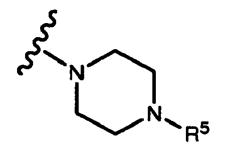 substituted with one or two alkyl groups;
(D) 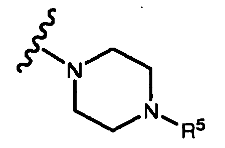 substituted with one or two methyl groups;
(E) 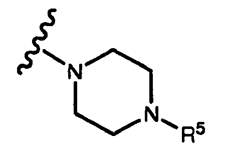 substituted with one methyl group; and
(F)

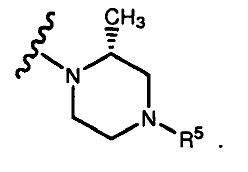

36. The compound of Claim 1, or a pharmaceutically acceptable salt thereof, wherein Q is selected from the group consisting of:
(A) 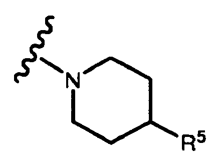 substituted with one or two substituents independently selected from the group consisting of R³ groups, provided that said one or two substitutents are not H;
(B) 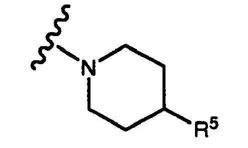 substituted with one or two alkyl groups;
(C) 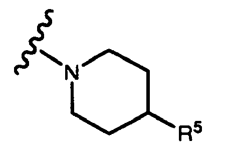 substituted with one or two methyl groups; and
(D) 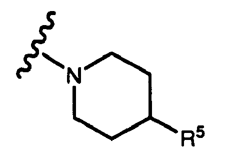 substituted with one methyl group.

37. The compound of any of Claims 1 or 2, or a pharmaceutically acceptable salt thereof, wherein R¹ is selected from the group consisting of:

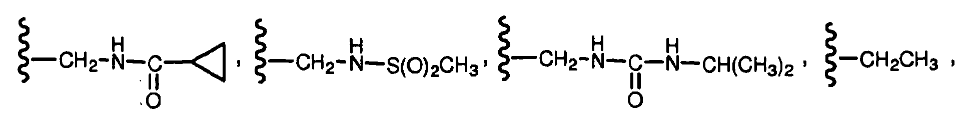

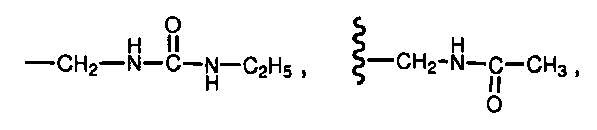

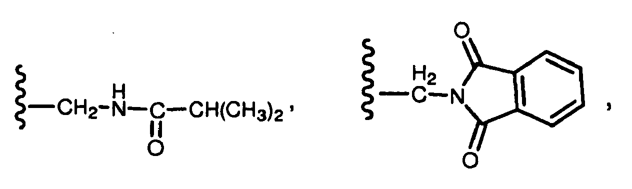

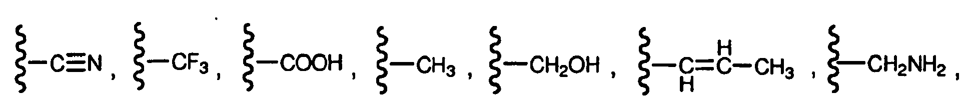

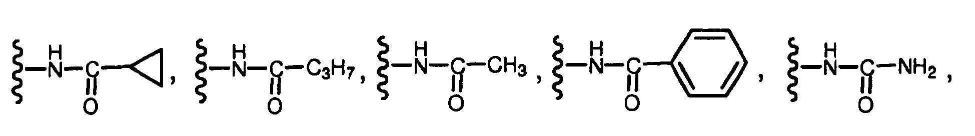

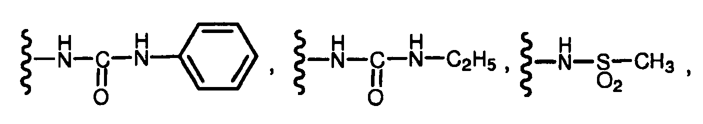

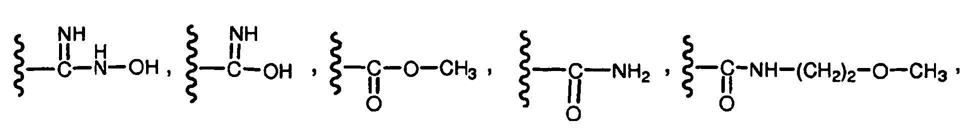

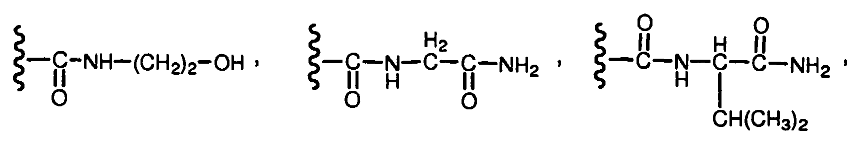

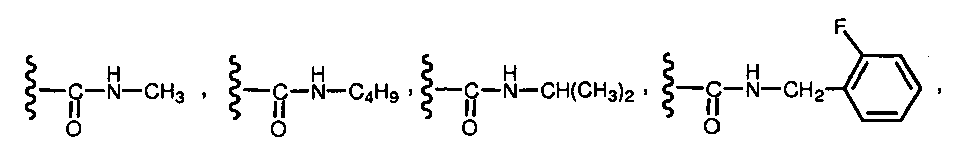

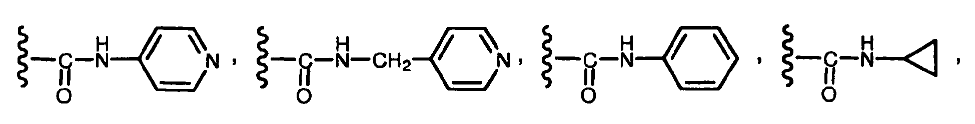

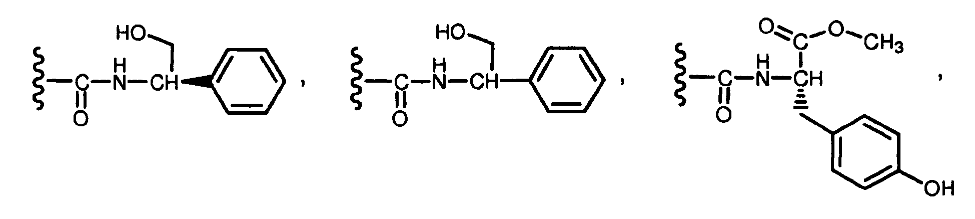

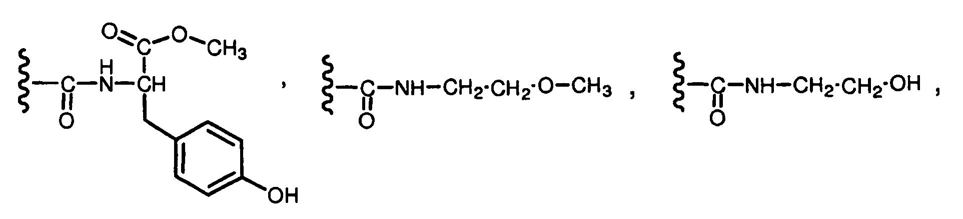

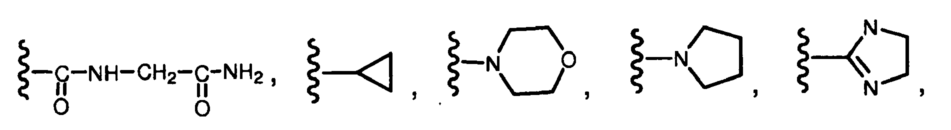

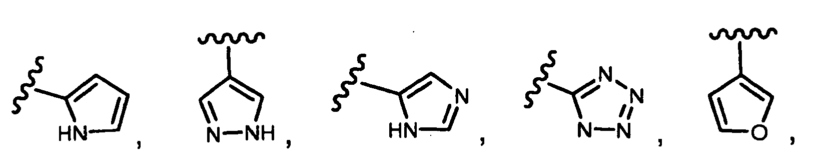

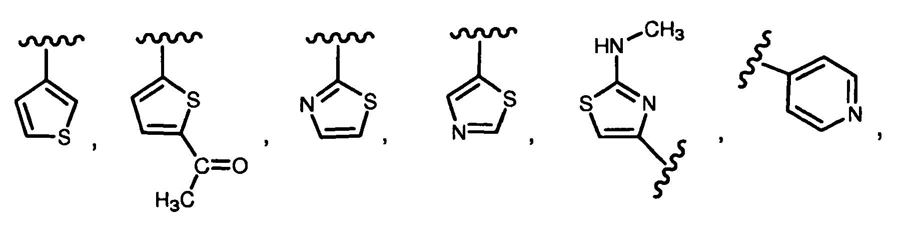

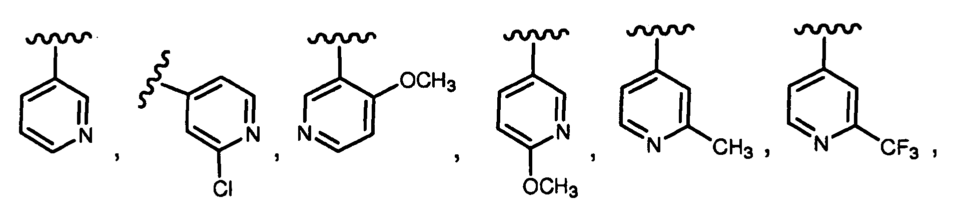

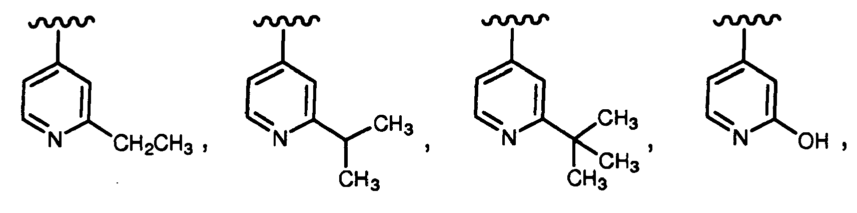

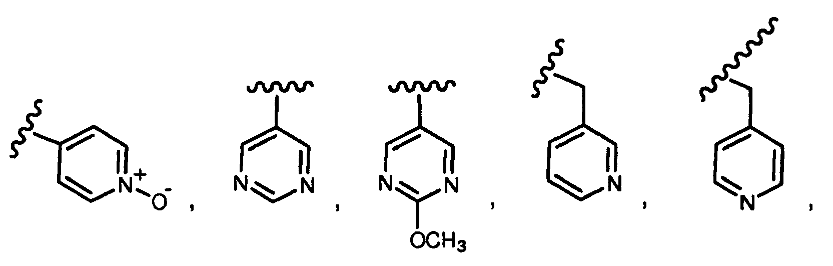

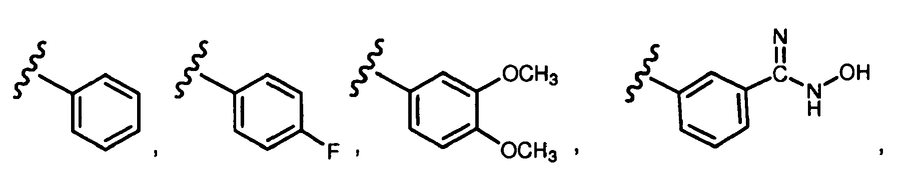

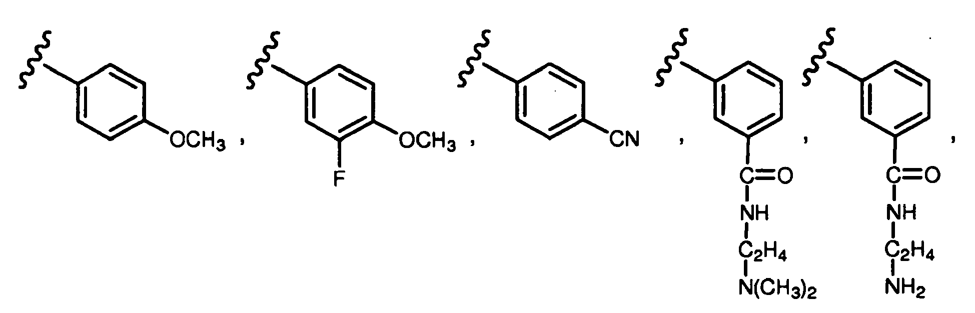

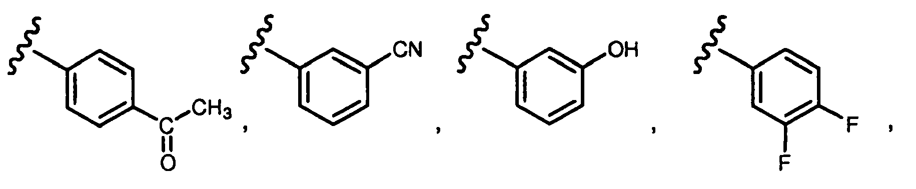

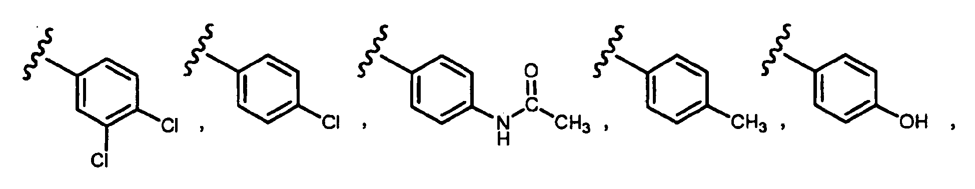

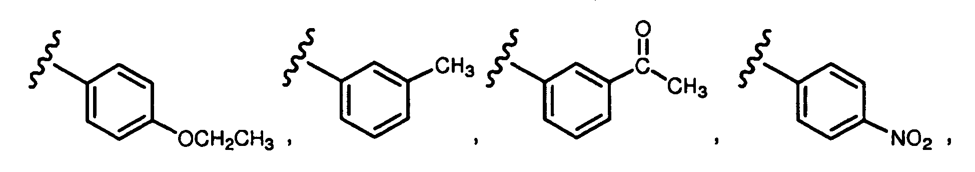

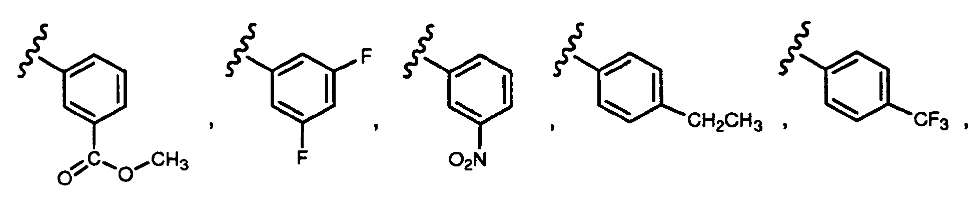

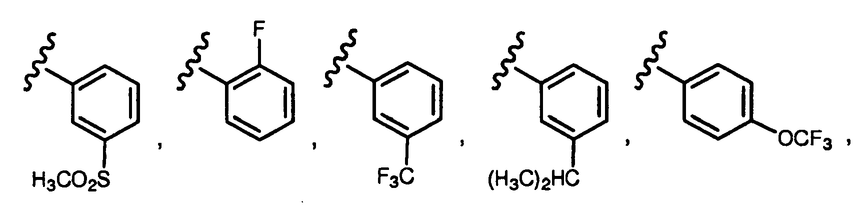

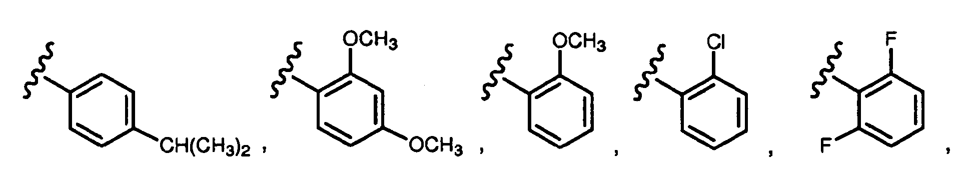

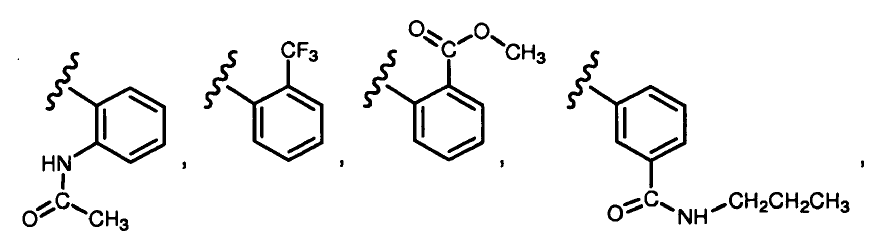

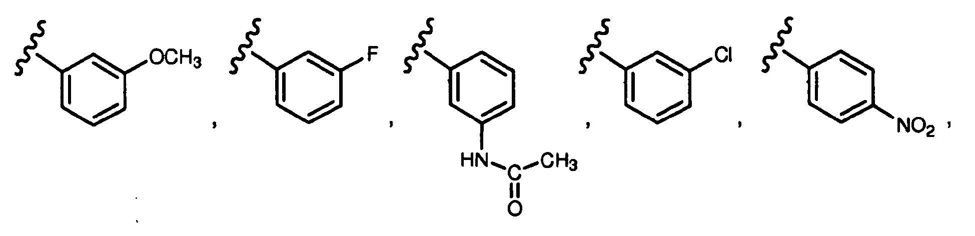

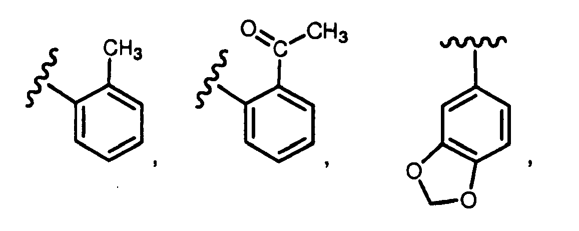

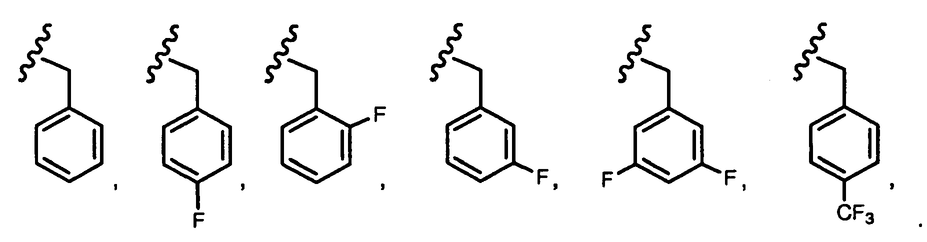

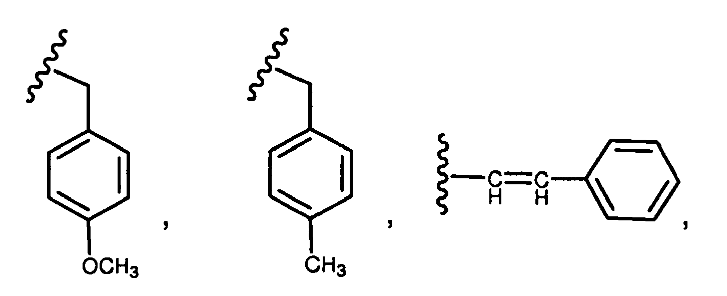

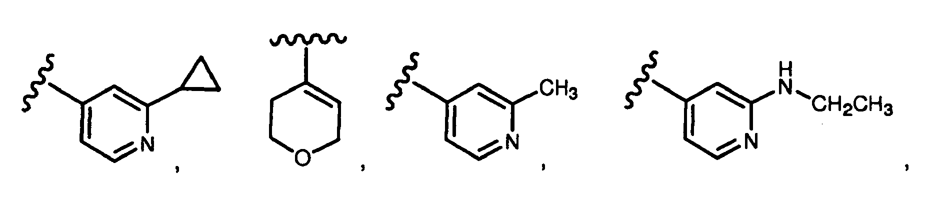

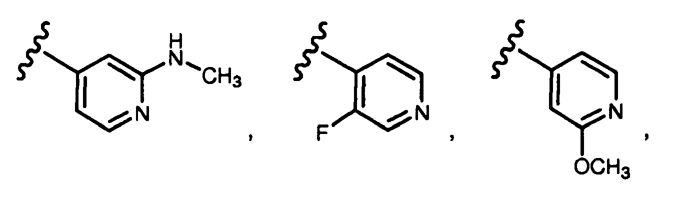

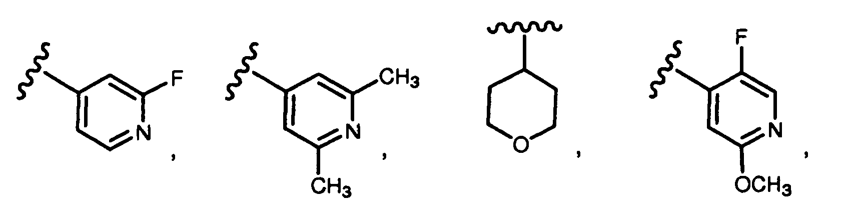

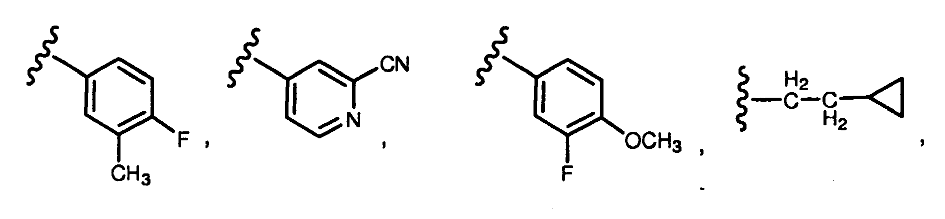

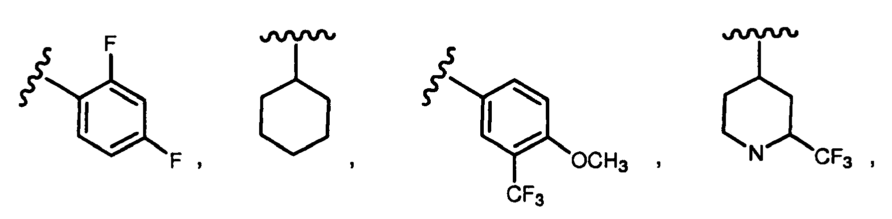

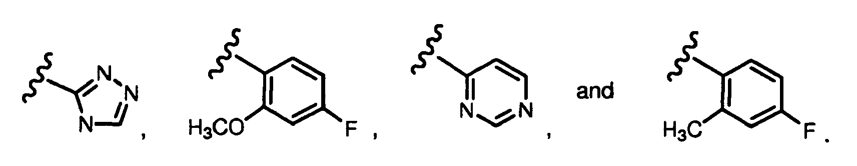

38. The compound of Claim 1, or a pharmaceutically acceptable salt thereof, wherein R¹ is aryl.

39. The compound of Claim 1, or a pharmaceutically acceptable salt thereof, wherein R¹ is heteroaryl or substituted heteroaryl wherein said substituted heteroaryl is substituted with 1 to 3 substituents independently selected from the group consisting of: (1) -NH₂, (2) -NO₂, (3) -CN, (4) -OH, (5) -OR²⁰, (6) -OCF₃, (7) alkyl substituted with 1 to 3 independently selected halo atoms, (8) -C(O)R³⁸, (9) alkyl, (10) alkenyl, (11) halo, (12) -C(O)-NH-R²⁶, (13) -C(O)OR³⁸, (14) -C(O)-NR³²-(C(R³⁰)₂)ₙ-N(R³⁸)₂, (15) -S(O)ₜR³⁸, (16) -C(O)-NR³²-R³⁸, (17) -NR³²-C(O)-R³⁸,
(18) 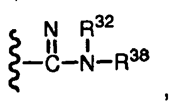 (19) -NHR²⁰, and (20) cycloalkyl.

40. The compound of any of Claims 1 or 2, or a pharmaceutically acceptable salt thereof, wherein R⁵ is selected from the group consisting of:

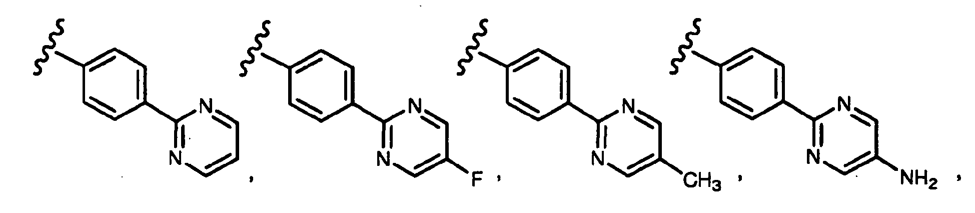

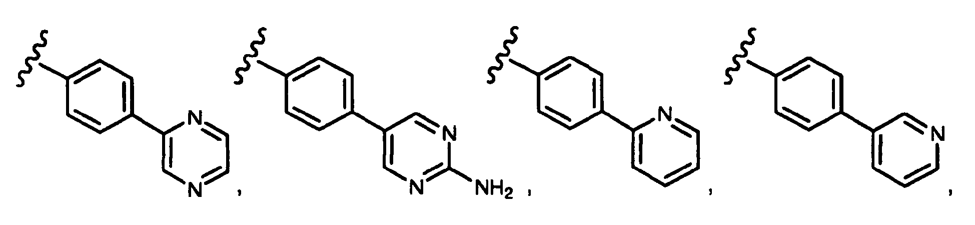

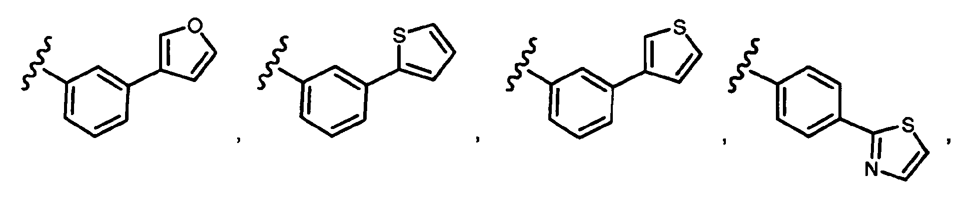

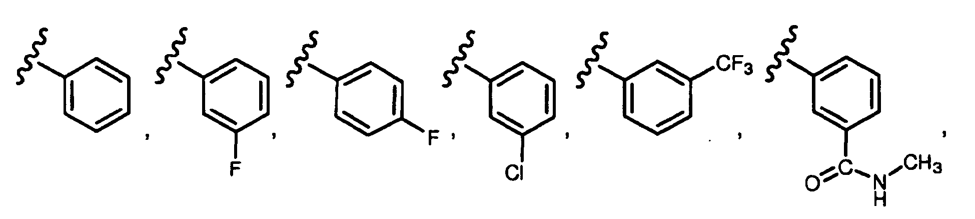

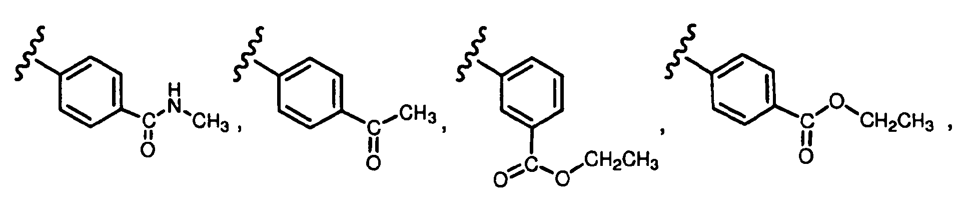

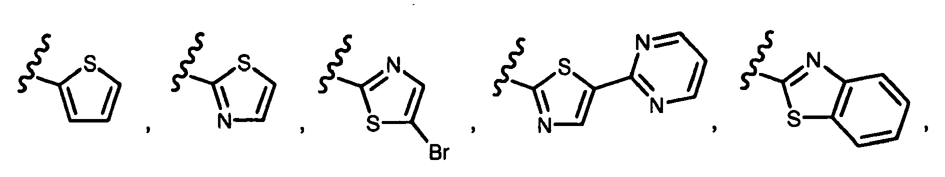

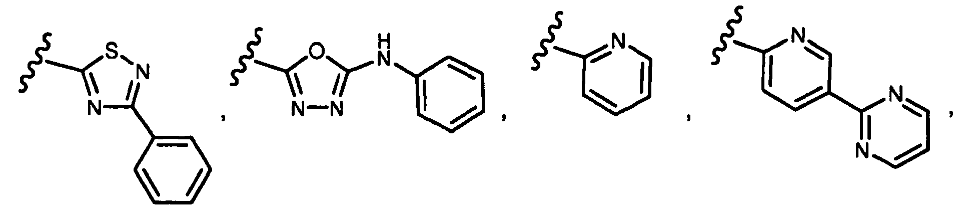

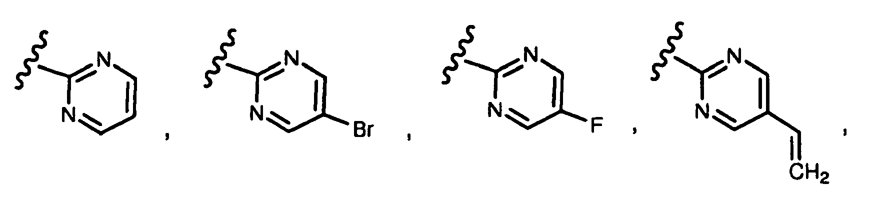

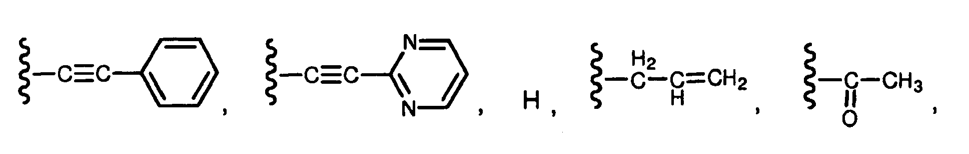

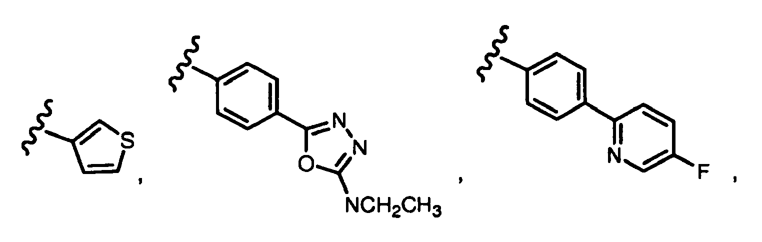

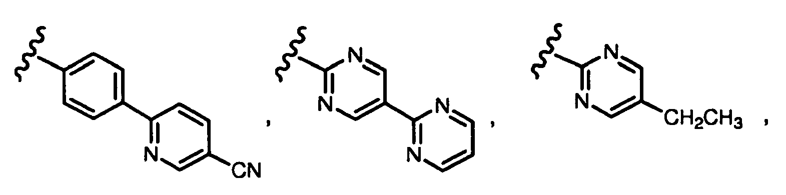

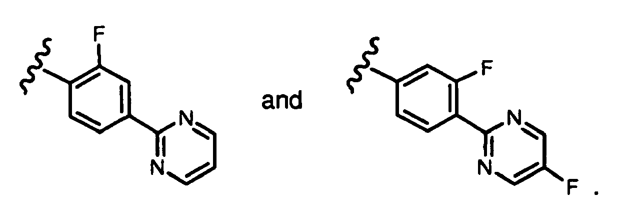

41. The compound of any of Claims 1 or 7, or a pharmaceutically acceptable salt thereof, wherein R¹ is selected from the group consisting of: 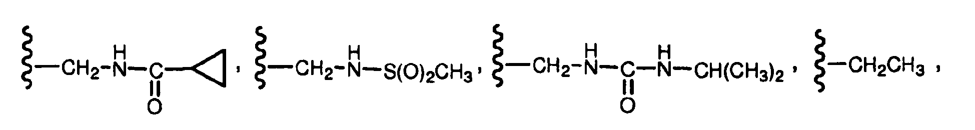 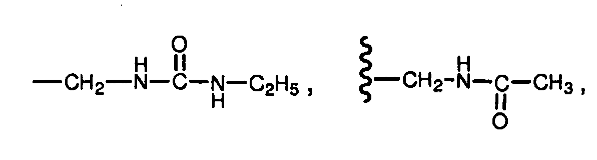 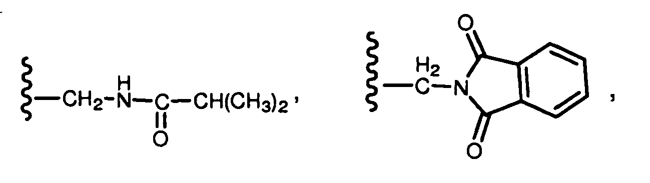 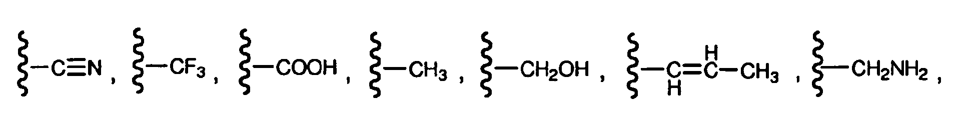 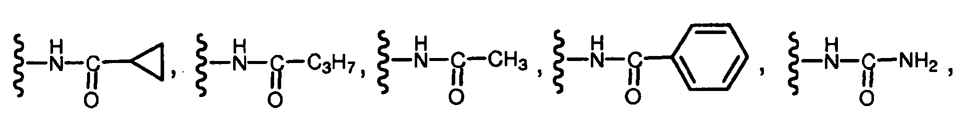 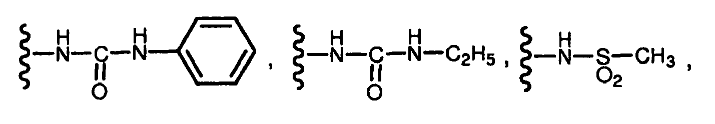 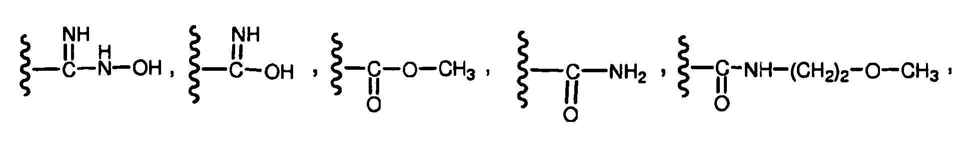 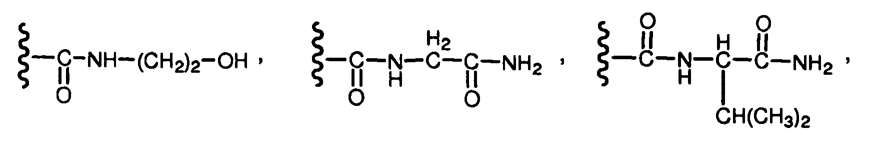 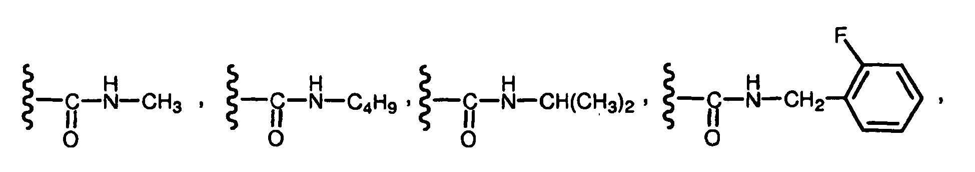 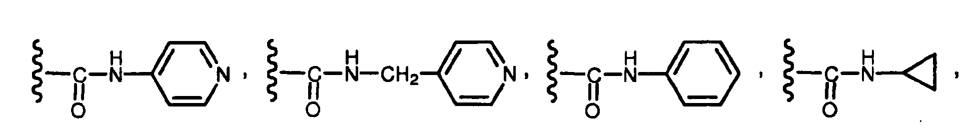 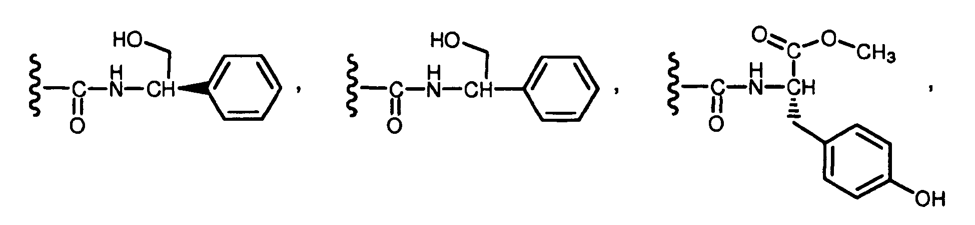 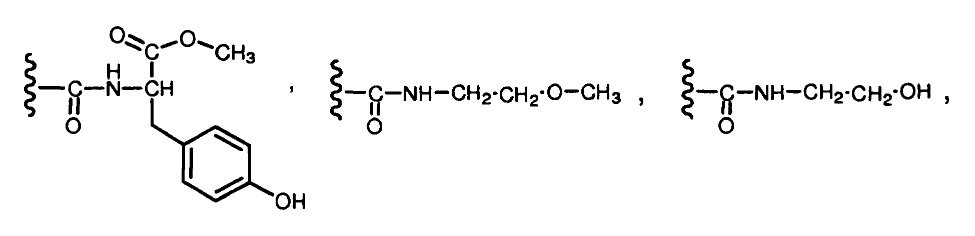 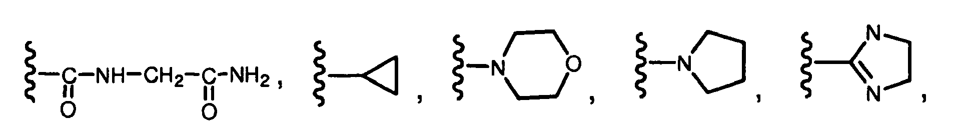 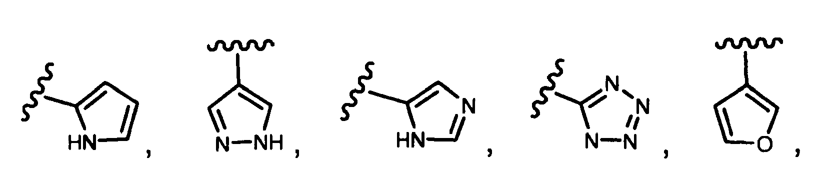 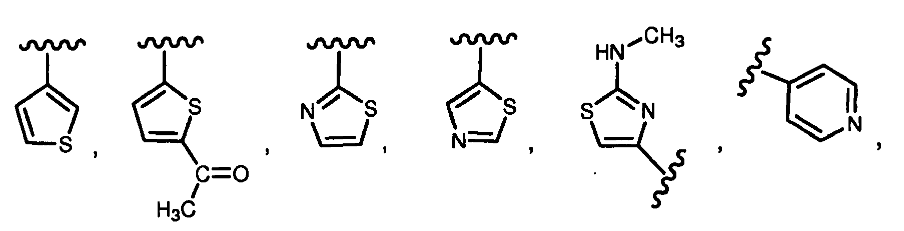 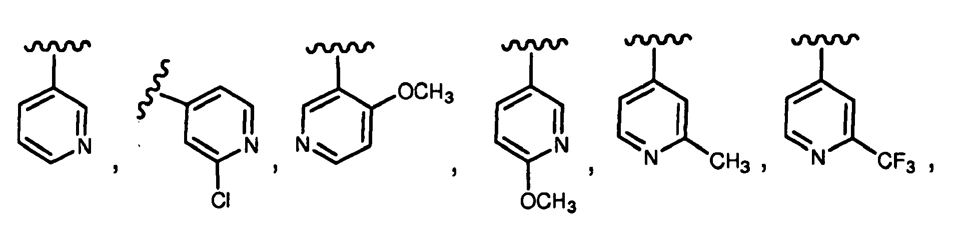 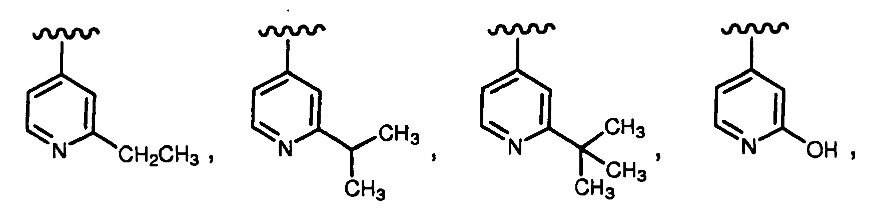 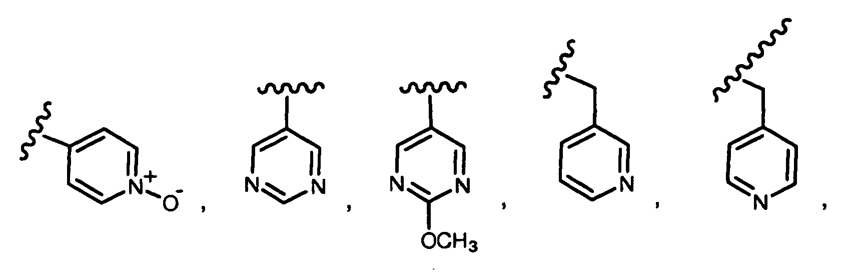 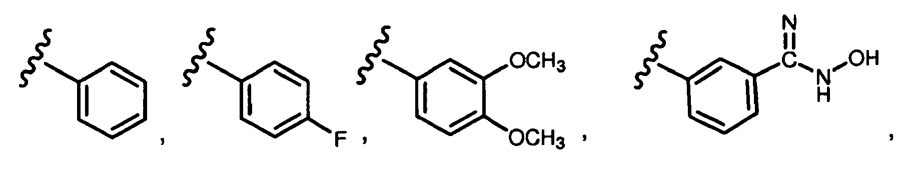 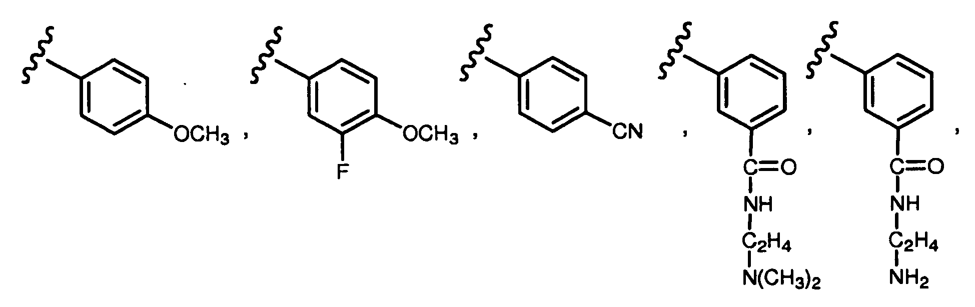 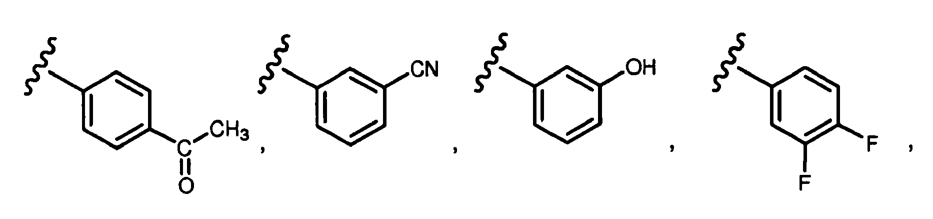 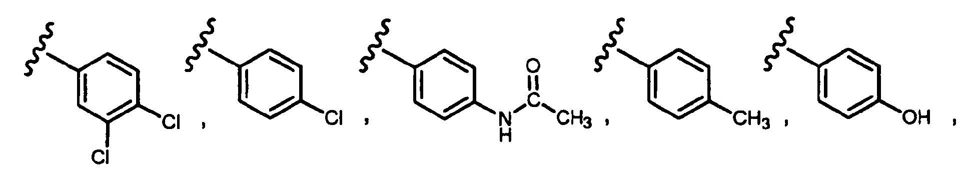 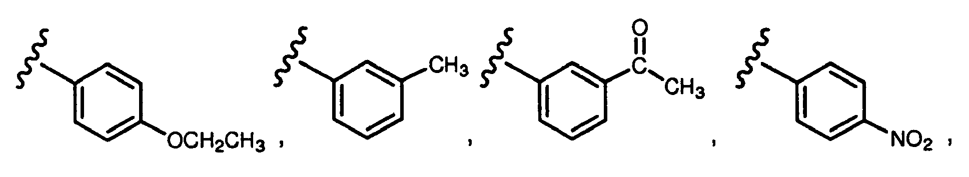 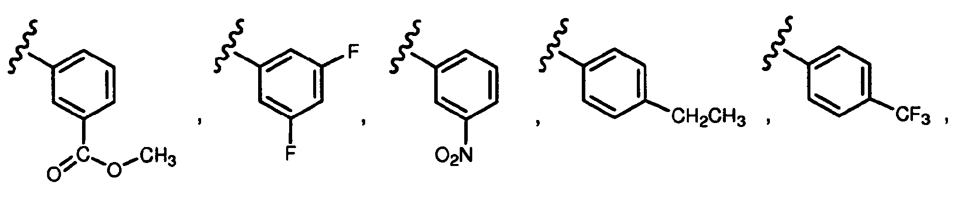 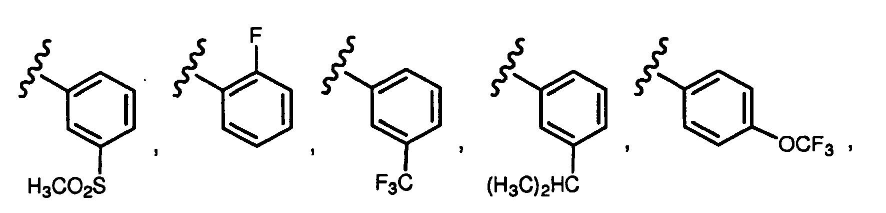 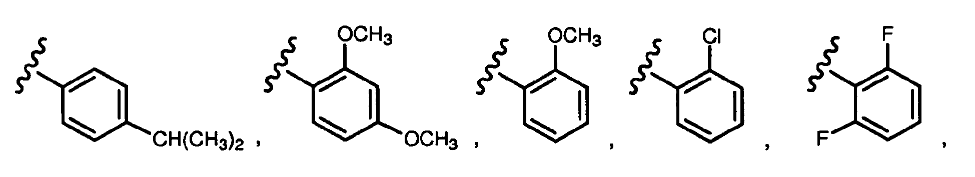 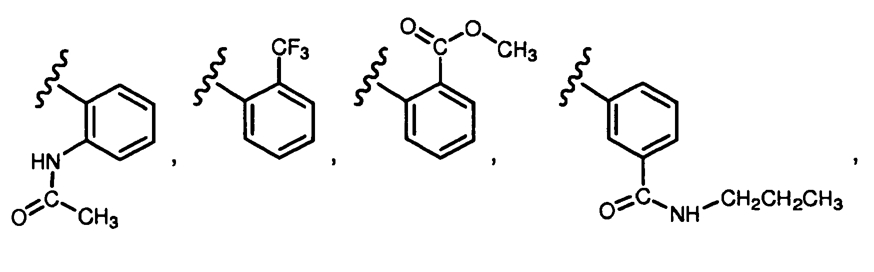 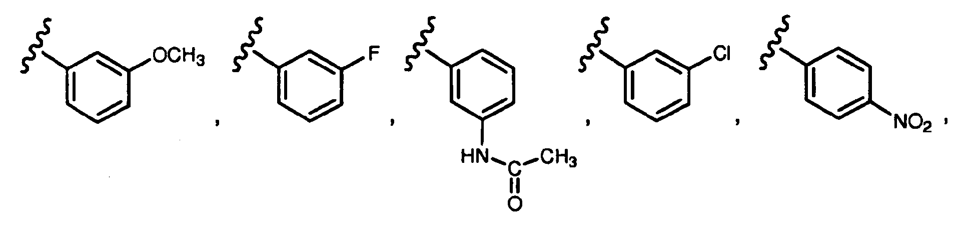 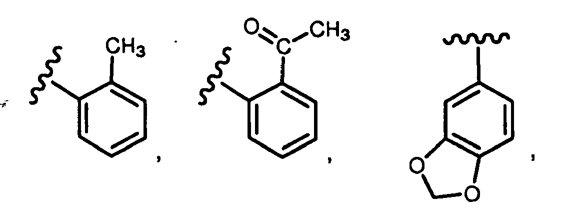 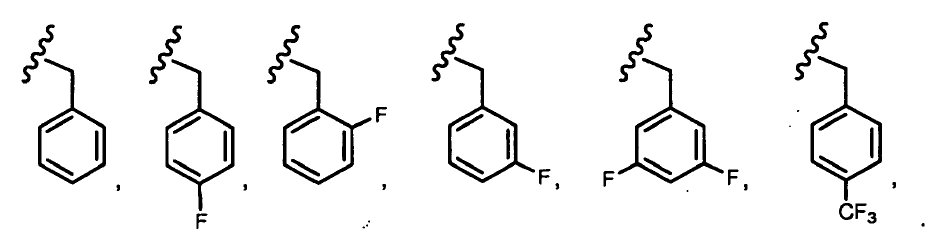 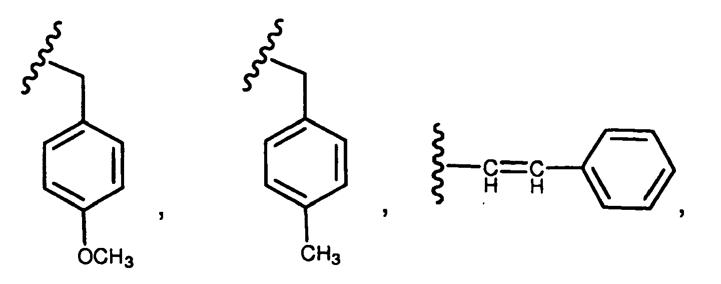 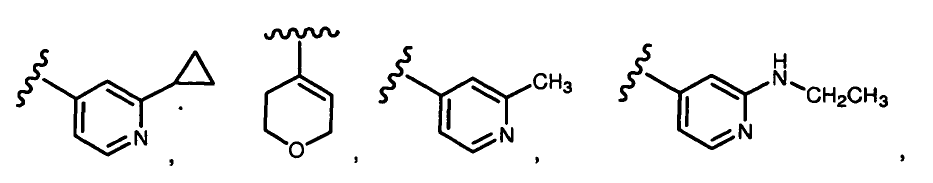 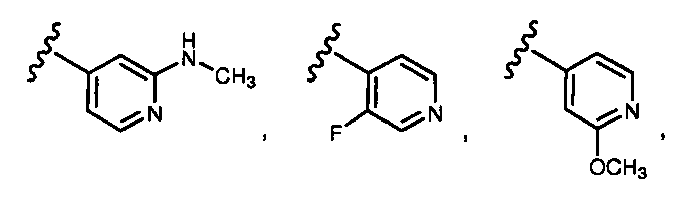 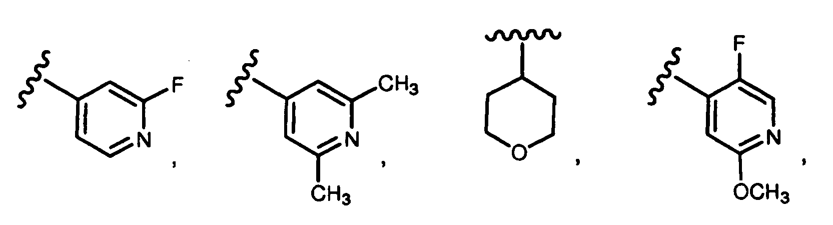 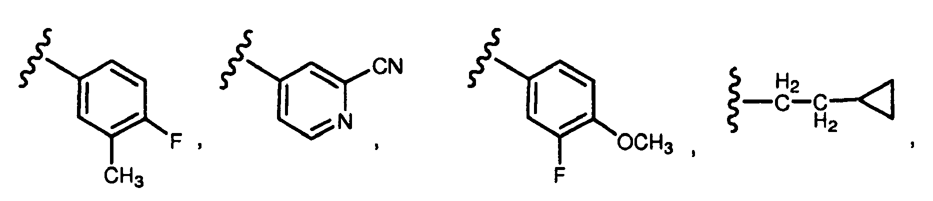 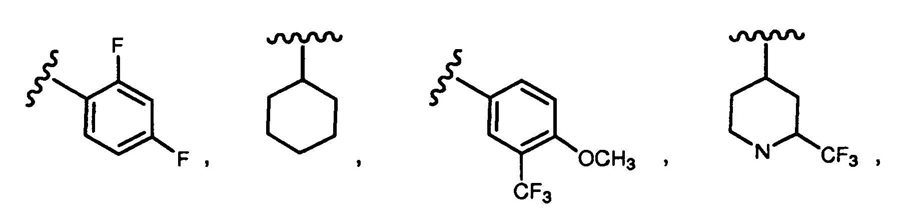 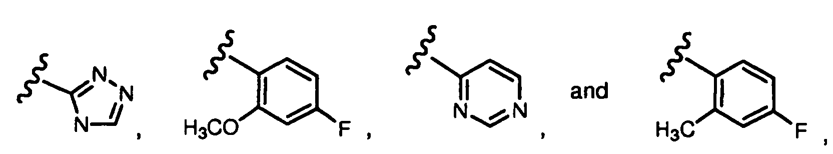 and
R⁵ is selected from the group consiting of:

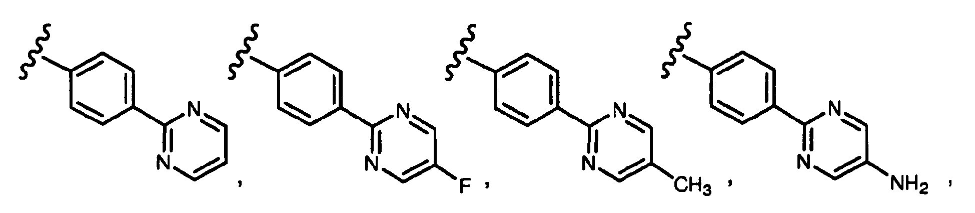

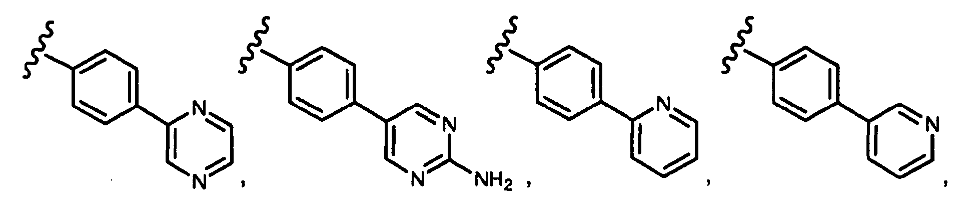

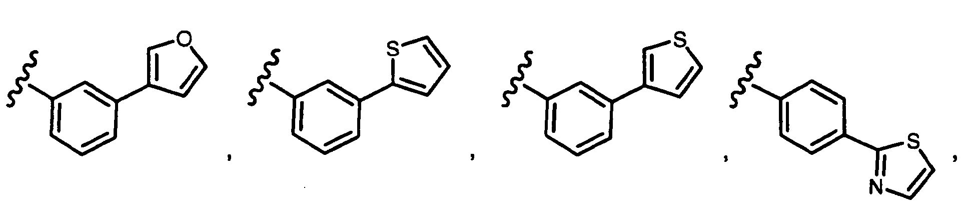

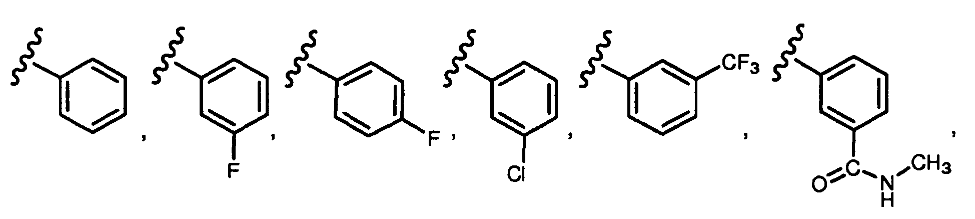

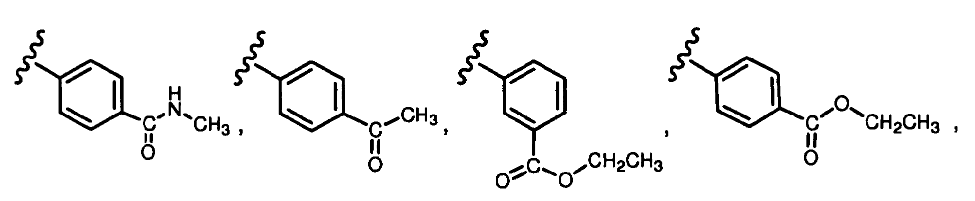

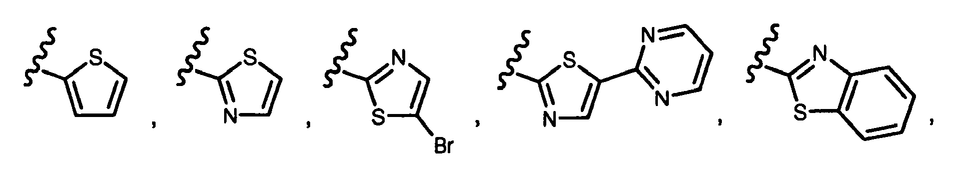

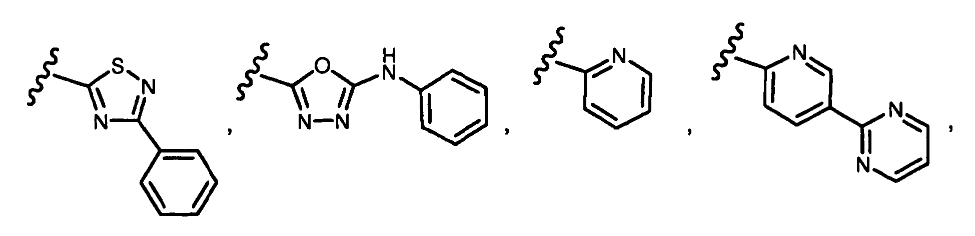

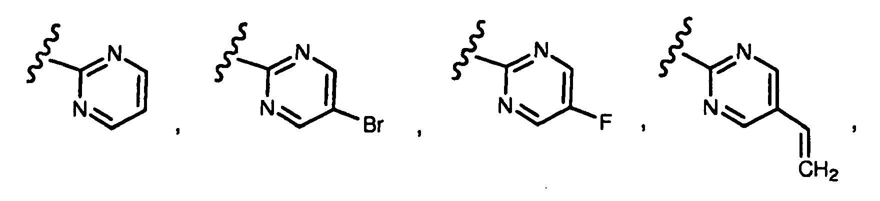

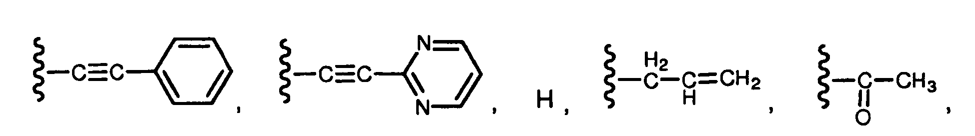

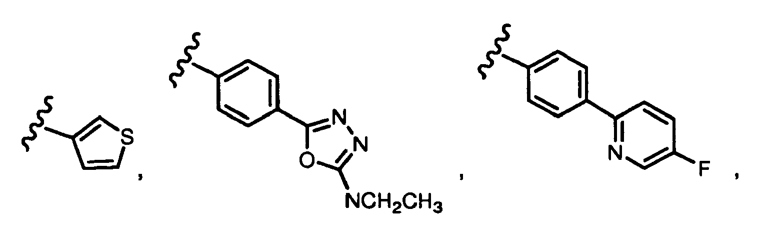

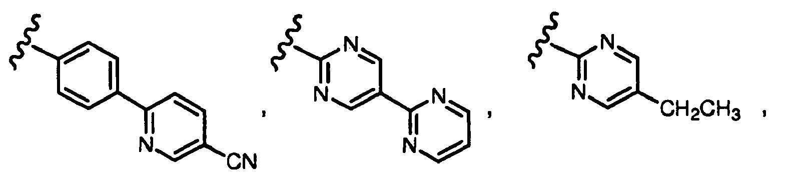

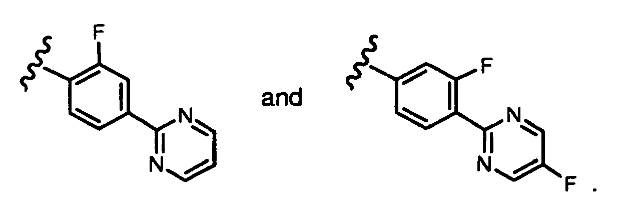

42. The compound of Claim 41, or a pharmaceutically acceptable salt thereof, wherein R¹ is selected from the group consisting of:

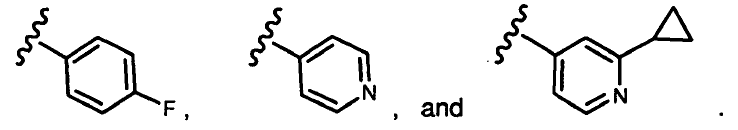

43. The compound of any of Claims 1 or 41, or a pharmaceutically acceptable salt thereof, wherein R² is selected from the group consisting of:

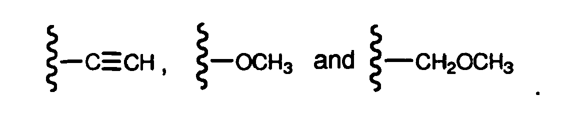

44. The compound of Claim 41, or a pharmaceutically acceptable salt thereof, wherein Q is selected from the group consisting of: 2.1, 2.2, and 2.3, and 2.3 is selected from the group consisting of: 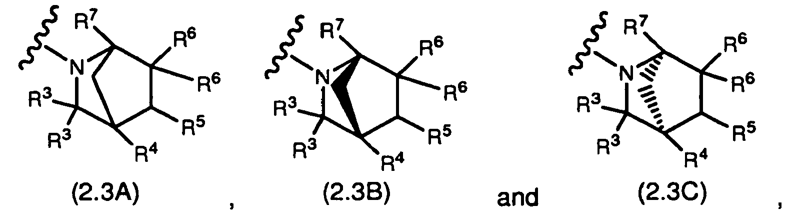 and R³, R⁴, R⁶, and R⁷ are each independently selected from the group consisting of: H and alkyl.

45. The compound of Claim 44, or a pharmaceutically acceptable salt thereof, wherein R³, R⁴, R⁶, and R⁷ are each H.

46. The compound of Claim 45, or a pharmaceutically acceptable salt thereof, wherein Q is 2.1.

47. The compound of Claim 41, or a pharmaceutically acceptable salt thereof, wherein Q is selected from the group consisting of: 2.6 and 2.7, and 2.7 is selected from the group consisting of: 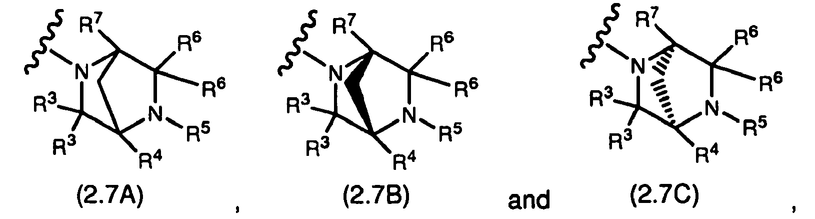 and R³, R⁴, R⁶, and R⁷ are each independently selected from the group consisting of: H and alkyl.

48. The compound of Claim 47, or a pharmaceutically acceptable salt thereof, wherein R³, R⁴, R⁶, and R⁷ are each H.

49. The compound of Claim 48, or a pharmaceutically acceptable salt thereof, wherein Q is 2.6.

50. The compound of Claim 48, or a pharmaceutically acceptable salt thereof, wherein Q is 2.7B.

51. The compound of Claim 41, or a pharmaceutically acceptable salt thereof, wherein Q is selected from the group consisting of: 2.17, 2.18, 2.19, 2.20, 2.21, and 2.22, and R³, R⁴, R⁶, and R⁷ are each independently selected from the group consisting of: H and alkyl.

52. The compound of Claim 1, or a pharmaceutically acceptable salt thereof, wherein Q is

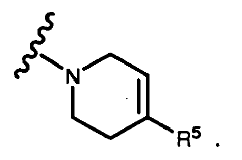

53. The compound of Claim 1 selected from the group consisting of:
(A) the final compounds of Examples 1-84, 86-125, 141-418, 420-429, 431, 432 and 434-611;
(B) the final compounds of Examples 1, 2, 4-61, 65-73, 77-84, 86, 88-98, 100, 102-114, 116-118, 120, 121, 124, 125, 151-155, 159-179, 183, 184, 186, 188-193, 196-199, 202-205, 250, 253-259, 260-299, 301-318, 320-323, 332-347, 356 (Isomer Z), 356 (Isomer E), 357-360, 362, 362 (compound 9P), 364, 368-418, 420-429, 431, 432, 434-436, 440-509, 511-602, 606, 607 and 609-611; and
(C) the final compounds of Examples 6, 100, 183, 184, 186, 188-192, 261, 440-450, and 510-517;
or a pharmaceutically acceptable salt thereof.

54. The compound of Claim 1 having the formula: 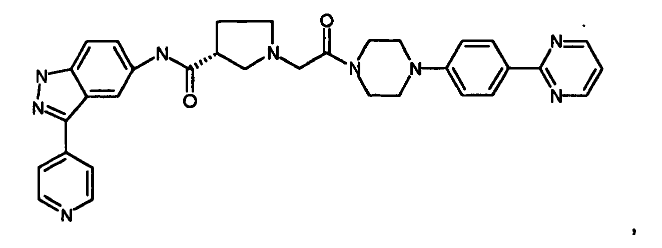 or a pharmaceutically acceptable salt thereof.

55. The compound of Claim 1 having the formula: 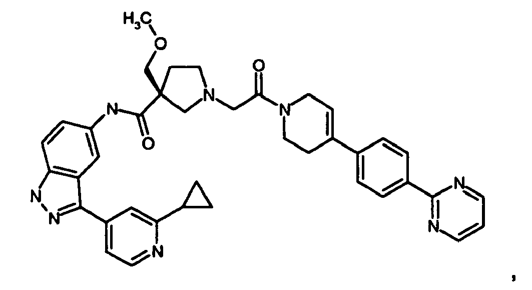 or a pharmaceutically acceptable salt thereof.

56. The compound of Claim 1 having the formula: 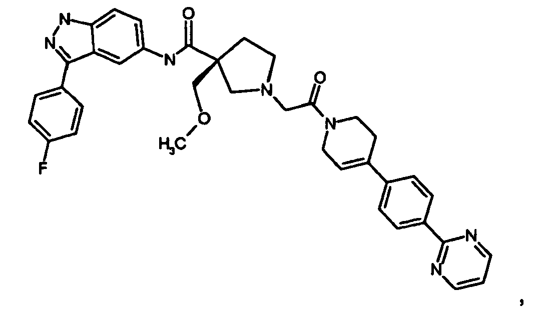 or a pharmaceutically acceptable salt thereof.

57. The compound of Claim 1 having the formula: 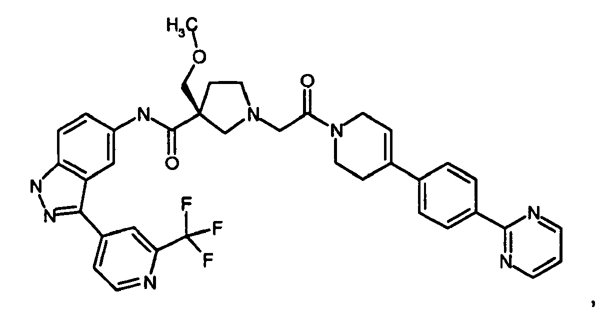 or a pharmaceutically acceptable salt thereof.

58. The compound of Claim 1 having the formula: 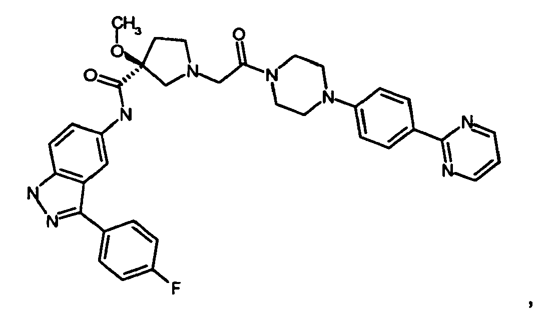 or a pharmaceutically acceptable salt thereof.

59. The compound of Claim 1 having the formula: 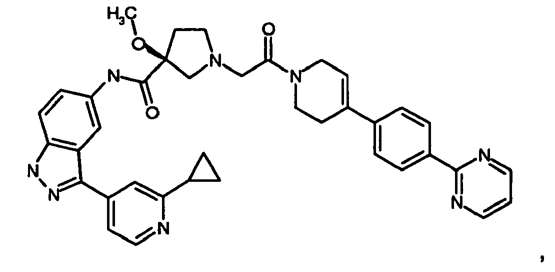 or a pharmaceutically acceptable salt thereof.

60. The compound of Claim 1 having the formula: 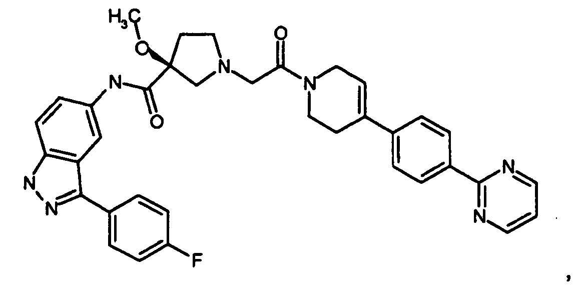 or a pharmaceutically acceptable salt thereof.

61. The compound of Claim 1 having the formula: 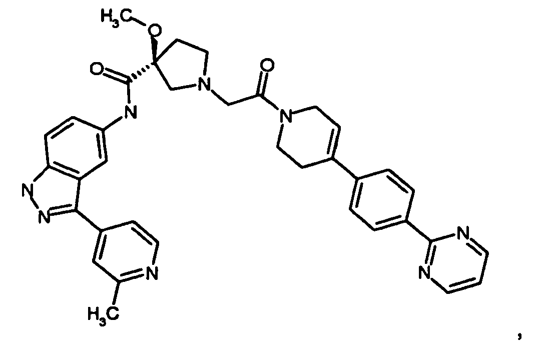 or a pharmaceutically acceptable salt thereof.

62. The compound of Claim 1 having the formula: 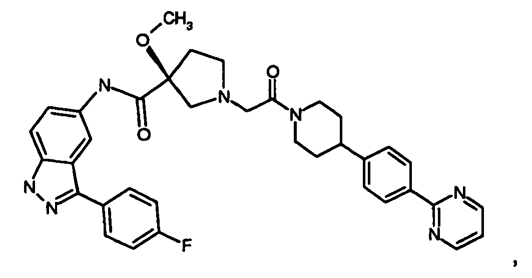 or a pharmaceutically acceptable salt thereof.

63. The compound of Claim 1 having the formula: 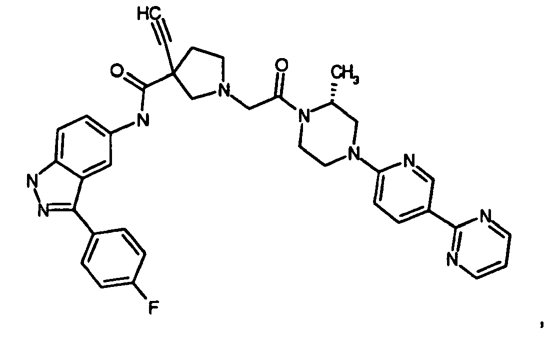 or a pharmaceutically acceptable salt thereof.

64. A pharmaceutical composition comprising at least one compound of Claim 1, or a pharmaceutically acceptable salt thereof, and a pharmaceutically acceptable carrier.

65. A compound of any one of claims 1 to 63, or a pharmaceutically acceptable salt thereof, for use as a medicament.

66. A use of at least one compound of any of Claims 1 to 63, or a pharmaceutically acceptable salt thereof:
(A) for the manufacture of a medicament for treating cancer;
(B) for the manufacture of a medicament for treating cancer, wherein said medicament is in a form for administration with at least one chemotherapeutic agent; or
(C) for the manufacture of a medicament for treating cancer, said medicament is in a form for administration with at least one chemotherapeutic agent, and radiation therapy.

67. A use of at least one compound of any of Claims 1 to 63, or a pharmaceutically acceptable salt thereof, for the manufacture of a medicament for treating cancer, wherein said medicament is in a form for administration with at least one chemotherapeutic agent selected from the group consisting of: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors.

68. A use of a compound of any of Claims 1 to 63, or a pharmaceutically acceptable salt thereof, for the manufacture of a medicament for treating cancer, wherein said medicament is in a form for administration with at least one signal transduction inhibitor.

69. A use of at least one compound of any of Claims 1 to 63, or a pharmaceutically acceptable salt thereof:
(A) for the manufacture of a medicament for treating cancer wherein said cancer is selected from the group consisting of: lung cancer, pancreatic cancer, colon cancer, myeloid leukemias, thyroid cancer, myelodysplastic syndrome, bladder carcinoma, epidermal carcinoma, melanoma, breast cancer, prostate cancer, head and neck cancers, ovarian cancer, brain cancers, cancers of mesenchymal origin, sarcomas, tetracarcinomas, nuroblastomas, kidney carcinomas, hepatomas, non-Hodgkin's lymphoma, multiple myeloma, and anaplastic thyroid carcinoma;
(B) for the manufacture of a medicament for treating cancer wherein said cancer is selected from the group consisting of: melanoma, pancreatic cancer, thryroid cancer, colorectal cancer, lung cancer, breast cancer, and ovarian cancer; or
(C) for the manufacture of a medicament for treating cancer wherein said medicament is in a form for administration in combination with at least one chemotherapeutic agent, and wherein said cancer is selected from the group consisting of: melanoma, pancreatic cancer, thryroid cancer, colorectal cancer, lung cancer, breast cancer, and ovarian cancer.

70. A use of at least one compound of any of Claims 1 to 63, or a pharmaceutically acceptable salt thereof:
(A) for the manufacture of a medicament for treating melanoma;
(B) for the manufacture of a medicament for treating melanoma, wherein said medicament is in a form for administration in combination with at least one chemotherapeutic agent;
(C) for the manufacture of a medicament for treating pancreatic cancer;
(D) for the manufacture of a medicament for treating pancreatic cancer, wherein said medicament is in a form for administration with at least one chemotherapeutic agent;
(E) for the manufacture of a medicament for treating thyroid cancer;
(F) for the manufacture of a medicament for treating thyroid cancer, wherein said medicamemnt is in a form for administration in combination with at least one chemotherapeutic agent;
(G) for the manufacture of a medicament for treating colorectal cancer;
(H) for the manufacture of a medicament for treating colorectal cancer, wherein said medicament is in a form for administration in combination with at least one chemotherapeutic agent;
(I) for the manufacture of a medicament for treating lung cancer;
(J) for the manufacture of a medicament for treating lung cancer, wherein said medicament is in a form for administration in combination with at least one chemotherapeutic agent;
(K) for the manufacture of a medicament for treating breast cancer;
(L) for the manufacture of a medicament for treating breast cancer, wherein said medicament is in a form for administration in combination with at least one chemotherapeutic agent;
(M) for the manufacture of a medicament for treating ovarian cancer;
(N) for the manufacture of a medicament for treating ovarian cancer, wherein said medicament is in a form for administration in combination with at least one chemotherapeutic agent;
(O) for the manufacture of a medicament for treating hormone-dependent breast cancer, wherein said medicament is in a form for administration in combination with antihormonal agents;
(P) for the manufacture of a medicament for treating hormone-dependent breast cancer, wherein said medicament is in a form for administration in combination with antihormonal agents, and in combination with at least one chemotherapeutic agent;
(Q) for the manufacture of a medicament for preventing hormone-dependent breast cancer, wherein said medicament is in a form for administration in combination with antihormonal agents;
(R) for the manufacture of a medicament for preventing hormone-dependent breast cancer, wherein said medicament is in a form for administration in combination with antihormonal agents, and in combination with at least one chemotherapeutic agent;
(S) for the manufacture of a medicament for treating brain cancer;
(T) for the manufacture of a medicament for treating brain cancer, wherein said medicament is in a form for administration in combination with at least one chemotherapeutic agent;
(U) for the manufacture of a medicament for treating brain cancer, wherein said medicament is in a form for administration in combination with a chemotherapeutic agent wherein said chemotherapeutic agent is temozolomide;
(V) for the manufacture of a medicament for treating prostate cancer;
(W) for the manufacture of a medicament for treating prostate cancer, wherein said medicament is in a form for administration in combination with at least one chemotherapeutic agent;
(X) for the manufacture of a medicament for treating myelodysplastic syndrome ;
(Y) for the manufacture of a medicament for treating myelodysplastic syndrome, wherein said medicament is in a form for administration in combination with at least one chemotherapeutic agent;
(Z) for the manufacture of a medicament for treating myeloid leukemias;
(AA) for the manufacture of a medicament for treating myeloid leukemias, wherein said medicament is in a form for administration in combination with at least one chemotherapeutic agent;
(AB) for the manufacture of a medicament for treating acute myelogenous leukemia;
(AC) for the manufacture of a medicament for treating acute myelogenous leukemia, wherein said medicament is in a form for administration in combination with at least one chemotherapeutic agent;
(AD) for the manufacture of a medicament for treating chronic myelomonocytic leukemia;
(AE) for the manufacture of a medicament for treating chronic myelomonocytic leukemia, wherein said medicament is in a form for administration in combination with at least one chemotherapeutic agent;
(AF) for the manufacture of a medicament for treating chronic myelogenous leukemia;
(AG) for the manufacture of a medicament for treating chronic myelogenous leukemia, wherein said medicament is in a form for administration in combination with at least one chemotherapeutic agent;
(AH) for the manufacture of a medicament for treating bladder cancer; (Al) for the manufacture of a medicament for treating bladder cancer, wherein said medicament is in a form for administration in combination with at least one chemotherapeutic agent;
(AJ) for the manufacture of a medicament for treating non-Hodgkin's lymphoma;
(AK) for the manufacture of a medicament for treating non-Hodgkin's lymphoma, wherein said medicament is in a form for administration in combination with at least one chemotherapeutic agent;
(AL) for the manufacture of a medicament for treating multiple myeloma; or
(AM) for the manufacture of a medicament for treating multiple myeloma, wherein said medicament is in a form for administration in combination at least one chemotherapeutic agent.

## Patentansprüche

1. Eine Verbindung der Formel 1.0: 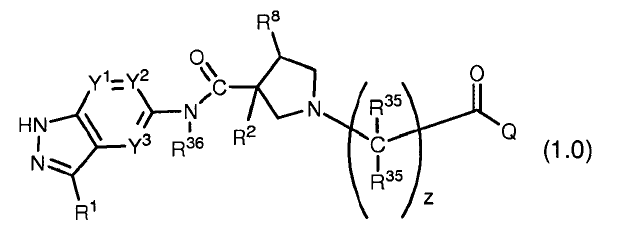 oder die pharmazeutisch annehmbaren Salze davon, wobei:
Y¹, Y² und Y³ jeweils unabhängig ausgewählt sind aus der Gruppe, bestehend aus: -CH=, -N= und -CR⁹=,
z 1 bis 3 ist,
Q ein Substituent ist, ausgewählt aus der Gruppe, bestehend aus: 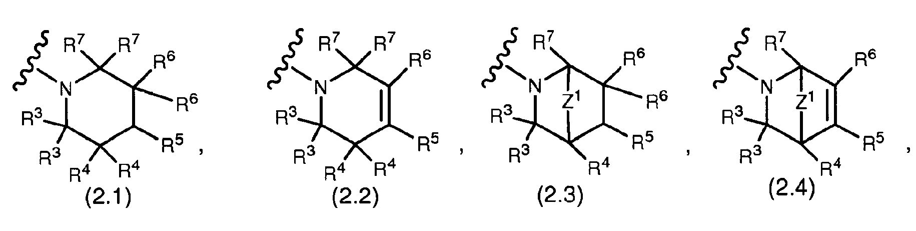 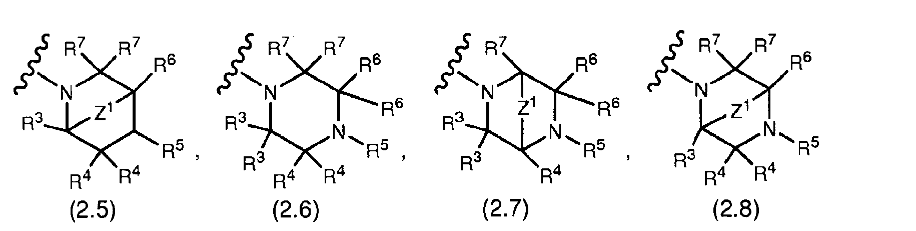 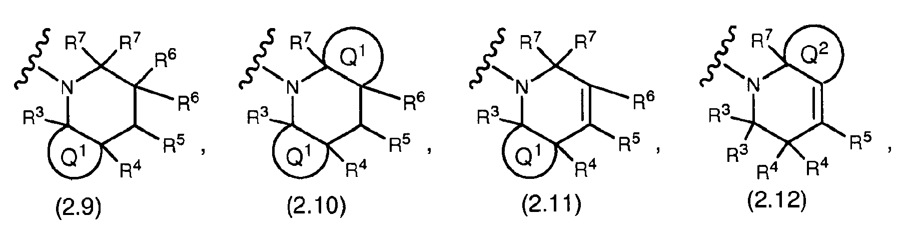 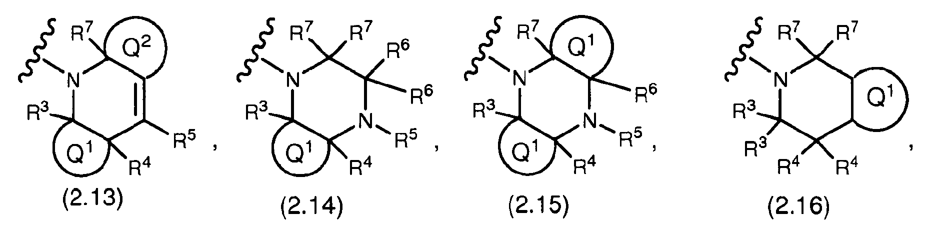 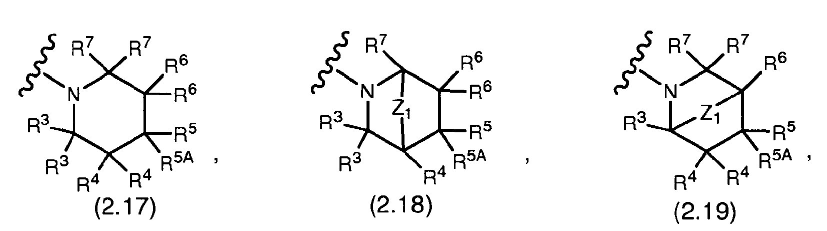 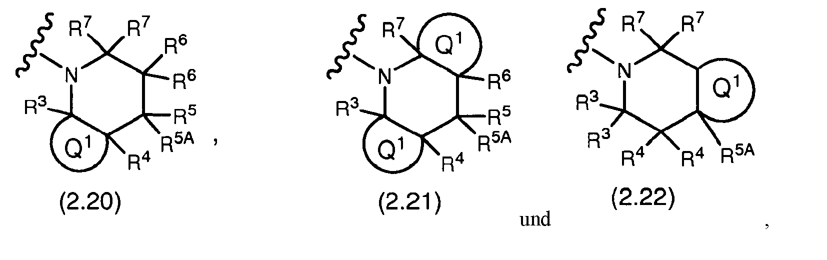
jedes Q¹ einen Ring bedeutet, unabhängig ausgewählt aus der Gruppe, bestehend aus: Cycloalkyl, substituiertem Cycloalkyl, Heterocycloalkyl, substituiertem Heterocycloalkyl, Aryl, substituiertem Aryl, Heteroaryl und substituiertem Heteroaryl, wobei die substituierten Ringe substituiert sind mit 1 bis 3 Substituenten, unabhängig ausgewählt aus der Gruppe, bestehend aus: den R¹⁰-Resten, mit der Maßgabe, dass, wenn Q¹ Aryl, Heteroaryl, substituiertes Aryl oder substituiertes Heteroaryl ist, die Kohlenstoffatome am Ring-Knotenpunkt dann nicht substituiert sind,
Q² einen Ring bedeutet, ausgewählt aus der Gruppe, bestehend aus: Cycloalkyl, substituiertem Cycloalkyl, Heterocycloalkyl und substituiertem Heterocycloalkyl, wobei die substituierten Ringe substituiert sind mit 1 bis 3 Substituenten, unabhängig ausgewählt aus der Gruppe, bestehend aus: den R¹⁰-Resten,
Z¹-(C(R²⁴)₂)_{w}- bedeutet, wobei jedes R²⁴ unabhängig ausgewählt ist aus der Gruppe, bestehend aus: H, Alkyl und F, und wobei w 1, 2 oder 3 ist,
Z² ausgewählt ist aus der Gruppe, bestehend aus: -N(R⁴⁴)-, -O- und -C(R⁴⁶)₂-,
m 1 bis 6 ist,
n 1 bis 6 ist,
p 0 bis 6 ist,
t 0, 1 oder 2 ist,
R¹ ausgewählt ist aus der Gruppe, bestehend aus:
(1) -CN,
(2) -NO₂,
(3) -OR¹⁰,
(4) -SR¹⁰,
(5) -N(R¹⁰)₂,
(6) R¹⁰,
(7) -C(O)R¹⁰,
(8) -(C(R³⁰)₂)ₙ-NR³²-C(O)-R¹⁰,
(9) -(C(R³⁰)₂)ₙ-NR³²-S(O)ₜR¹⁰,
(10) -(C(R³⁰)₂)ₙ-NR³²-C(O)-N(R³²)-R¹⁰,
(11) 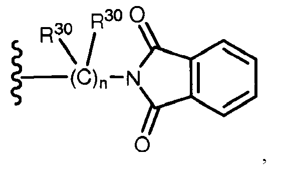
(12) -CF₃,
(13) -C(O)OR¹⁰,
(14) -(C(R³⁰)₂)ₙR¹³ (z.B. -(CH₂)ₙR¹³), wobei n 1 ist, jedes R³⁰ H ist und R¹³ ausgewählt ist aus der Gruppe, bestehend aus: -OH und -N(R¹⁰)₂, wobei jedes R¹⁰ unabhängig ausgewählt ist,
(15) Alkenyl,
(16) -NR³²-C(O)-R¹⁴,
(17) 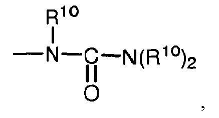 wobei jedes R¹⁰ unabhängig ausgewählt ist,
(18) 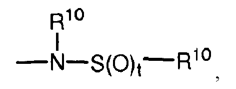 wobei jedes R¹⁰ unabhängig ausgewählt ist,
(19) 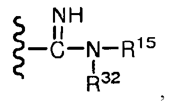
(20) -C(O)-NR³²-(C(R³⁰)₂)ₚ-OR¹⁰,
(21) -C(O)N(R¹⁰)₂, wobei jedes R¹⁰ unabhängig ausgewählt ist,
(22) -C(O)-NR³²-C(R¹⁸)₃, wobei jedes R¹⁸ unabhängig ausgewählt ist aus der Gruppe, bestehend aus: R¹⁰ und -C(O)OR¹⁹, und R¹⁹ ausgewählt ist aus der Gruppe, bestehend aus: Alkyl und substituiertem Arylalkyl,
(23) -C(O)-NR³²-(C(R³⁰)₂)ₙ-C(O)-N(R¹⁰)₂,
(24) Heterocycloalkenyl,
(25) 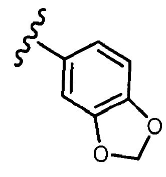 und
(26) Arylalkenyl-,
R² ausgewählt ist aus der Gruppe, bestehend aus:
(1) H,
(2) -CN,
(3) Halogen,
(4) Alkyl,
(5) substituiertem Alkyl, wobei das substituierte Alkyl substituiert ist mit 1 bis 3 Substituenten, ausgewählt aus der Gruppe, bestehend aus: (a) -OH, (b) -O-Alkyl (z.B. -O-(C₁-C₃-Alkyl), (c) -O-Alkyl, substituiert mit 1 bis 3 F-Atomen, und (d) -N(R⁴⁰)₂, wobei jedes R⁴⁰ unabhängig ausgewählt ist aus der Gruppe, bestehend aus: (i) H, (ii) C₁-C₃-Alkyl, (iii) -CF₃ und (e) Halogen,
(6) Alkinyl,
(7) Alkenyl,
(8) -(CH₂)ₘR¹¹,
(9) -N(R²⁶)₂,
(10) -OR²³,
(11) -N(R²⁶)C(O)R⁴²,
(12) Cycloalkyl,
(13) Cycloalkylalkyl,
(14) 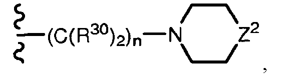
(15) -O-(substituiertes Alkyl), wobei das substituierte Alkyl substituiert ist mit 1 bis 3 F-Atomen,
(16) -S(O)ₜ-Alkyl,
(17) -C(O)-Alkyl,
(18) 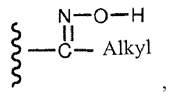
(19) 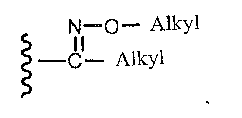 wobei jedes Alkyl unabhängig ausgewählt ist,
(20) 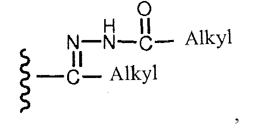 wobei jedes Alkyl unabhängig ausgewählt ist,
(21) 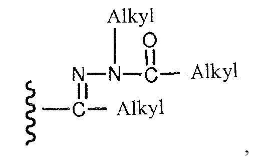 wobei jedes Alkyl unabhängig ausgewählt ist,
(22) -N(R⁴⁸)-C(O)-R⁴⁸, wobei jedes R⁴⁸ unabhängig ausgewählt ist aus der Gruppe, bestehend aus: H und Alkyl, und
(23) -C(O)-Alkyl, wie zum Beispiel -C(O)-(C₁-C₆-Alkyl), wie zum Beispiel -C(O)CH₃,
jedes R³, R⁴, R⁵, R⁶ und R⁷ unabhängig ausgewählt ist aus der Gruppe, bestehend aus:
(1) H,
(2) Alkenyl,
(3) substituiertem Alkenyl,
(4) Alkyl,
(5) substituiertem Alkyl,
(6) Cycloalkyl,
(7) substituiertem Cycloalkyl,
(8) Cycloalkylalkyl-,
(9) substituiertem Cycloalkylalkyl-,
(10) Heterocycloalkyl,
(11) substituiertem Heterocycloalkyl,
(12) Heterocycloalkylalkyl-,
(13) substituiertem Heterocycloalkylalkyl-,
(14) -C(O)R¹⁰,
(15) Arylheteroaryl-,
(16) substituiertem Arylheteroaryl-,
(17) Heteroarylaryl-,
(18) substituiertem Heteroarylaryl-,
(19) Aryl,
(20) substituiertem Aryl,
(21) Heteroaryl,
(22) substituiertem Heteroaryl,
(23) Heteroarylheteroaryl-,
(24) substituiertem Heteroarylheteroaryl-,
(25) Arylaminoheteroaryl-,
(26) substituiertem Arylaminoheteroaryl-,
(27) Arylalkinyl-,
(28) substituiertem Arylalkinyl-,
(29) Heteroarylalkinyl-,
(30) substituiertem Heteroarylalkinyl-,
wobei die R³-, R⁴-, R⁵-, R⁶- und R⁷-substituierten Gruppen (7), (9), (11), (13), (16), (18), (20), (22), (24), (26), (28) und (30) mit 1 bis 3 Substituenten substituiert sind, unabhängig ausgewählt aus der Gruppe, bestehend aus: -NH₂, Alkyl, Alkenyl, Halogen, -C(O)-NH-R²⁸, -C(O)OR²⁸ und -C(O)R²⁸, und
wobei die R³-, R⁴-, R⁵-, R⁶- und R⁷-substituierten Gruppen (3) und (5) substituiert sind mit 1 bis 3 Substituenten, unabhängig ausgewählt aus der Gruppe, bestehend aus: -NH₂, Halogen (z.B. F, Cl und Br, und in einem weiteren Beispiel F), -C(O)-NH-R²⁸ (z.B. -C(O)-NH-CH₃), -C(O)OR²⁸ (z.B. -C(O)OC₂H₅) und -C(O)R²⁸ (z.B. -C(O)CH₃),
R^{5A} ausgewählt ist aus der Gruppe, bestehend aus: Halogen, -OH und -O-Alkyl,
R⁸ ausgewählt ist aus der Gruppe, bestehend aus: H, -OH, -N(R¹⁰)₂, -NR¹⁰C(O)R¹² und Alkyl,
jedes R⁹ unabhängig ausgewählt ist aus der Gruppe, bestehend aus: Halogen, -CN, -NO₂, -OR¹⁰, -SR¹⁰, -N(R¹⁰)₂ und R¹⁰,
jedes R¹⁰ unabhängig ausgewählt ist aus der Gruppe, bestehend aus: H, Alkyl, Aryl, Arylalkyl, Heteroaryl, Heteroarylalkyl, Cycloalkyl, Cycloalkylalkyl, Heterocycloalkyl, Heterocycloalkylalkyl, Alkylheteroaryl,-, Alkylaryl-, substituiertem Alkyl, substituiertem Aryl, substituiertem Arylalkyl, substituiertem Heteroaryl, substituiertem Heteroarylalkyl, substituiertem Cycloalkyl, substituiertem Cycloalkylalkyl, substituiertem Heterocycloalkyl, substituiertem Heterocycloalkylalkyl, substituiertem Alkylheteroaryl-, substituiertem Alkylaryl-, Heterocycloalkenyl und substituiertem Heterocycloalkenyl, und wobei:
das mit R¹⁰ substituierte Alkyl substituiert ist mit 1 bis 3 Substituenten, unabhängig ausgewählt aus der Gruppe, bestehend aus: -NH₂, -NHR²⁰, -NO₂, -CN, -OR²⁶, Halogen, -C(O)-NH-R²⁶, -C(O)OR²⁶ und -C(O)R²⁶, und
das mit R¹⁰ substituierte Aryl, substituierte Arylalkyl, substituierte Heteroaryl, substituierte Heteroarylalkyl, substituierte Cycloalkyl, substituierte Cycloalkylalkyl, substituierte Heterocycloalkyl, substituierte Heterocycloalkylalkyl, substituierte Alkylheteroaryl- und substituierte Alkylaryl- substituiert sind mit 1 bis 3 Substituenten, unabhängig ausgewählt aus der Gruppe, bestehend aus: (1) -NH₂, (2) -NO₂, (3) -CN, (4) -OH, (5) -OR²⁰, (6) -OCF₃, (7) Alkyl, substituiert mit 1 bis 3 unabhängig ausgewählten Halogenatomen, (8) -C(O)R³⁸, (9) Alkyl, (10) Alkenyl, (11) Halogen, (12) -C(O)-NH-R²⁶, (13) -C(O)OR²⁸, (14) -C(O)-NR³²-(C(R³⁰)₂)ₙ-N(R³⁸)₂, (15) -S(O)ₜR³⁸, (16) -C(O)-NR³²-R³⁸, (17) -NR³²-C(O)-R³⁸,
(18) 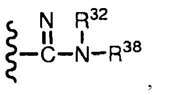 (19) -NHR²⁰ und (20) Cycloalkyl,
R¹¹ ausgewählt ist aus der Gruppe, bestehend aus: F, -OH, -CN, -OR¹⁰, -NHNR¹R¹⁰, -SR¹⁰ und Heteroaryl,
R¹² ausgewählt ist aus der Gruppe, bestehend aus: Alkyl, Aryl, Heteroaryl, Cycloalkyl, Cycloalkylalkyl, Heterocycloalkyl und Heterocycloalkylalkyl,
R¹⁴ ausgewählt ist aus der Gruppe, bestehend aus: Alkyl, Aryl, Heteroaryl, Cycloalkyl, Cycloalkylalkyl-, Heterocycloalkyl, Alkylheterocycloalkyl, Heterocycloalkylalkyl-, Alkylheteroaryl- und Alkylaryl-,
R¹⁵ ausgewählt ist aus der Gruppe, bestehend aus: H, -OH, Alkyl, Aryl, Heteroaryl, Cycloalkyl, Cycloalkylalkyl-, Heterocycloalkyl und Heterocycloalkylalkyl-, Alkylheteroaryl- und Alkylaryl-,
R²⁰ Alkyl bedeutet,
R²³ ausgewählt ist aus der Gruppe, bestehend aus: H, Alkyl, Aryl, Cycloalkyl und Cycloalkylalkyl-,
jedes R²⁶ unabhängig ausgewählt ist aus der Gruppe, bestehend aus: H und Alkyl,
R²⁸ Alkyl ist,
jedes R³⁰ unabhängig ausgewählt ist aus der Gruppe, bestehend aus: H, Alkyl und F,
jedes R³² unabhängig ausgewählt ist aus der Gruppe, bestehend aus: H und Alkyl, und wobei jedes R³² im Allgemeinen H ist,
jedes R³⁵ unabhängig ausgewählt ist aus der Gruppe, bestehend aus: H und C₁- bis C₆-Alkyl,
R³⁶ ausgewählt ist aus der Gruppe, bestehend aus: H, Alkyl und -O-Alkyl,
jedes R³⁸ unabhängig ausgewählt ist aus der Gruppe, bestehend aus: H, Alkyl, Aryl, Arylalkyl, Heteroaryl, Heteroarylalkyl, Cycloalkyl, Cycloalkylalkyl, Heterocycloalkyl, Heterocycloalkylalkyl, Alkylheteroaryl,-, Alkylaryl-, substituiertem Alkyl, substituiertem Aryl, substituiertem Arylalkyl, substituiertem Heteroaryl, substituiertem Heteroarylalkyl, substituiertem Cycloalkyl, substituiertem Cycloalkylalkyl, substituiertem Heterocycloalkyl, substituiertem Heterocycloalkylalkyl, substituiertem Alkylheteroaryl- und substituiertem Alkylaryl-, und wobei:
das mit R³⁸ substituierte Alkyl substituiert ist mit 1 bis 3 Substituenten, unabhängig ausgewählt aus der Gruppe, bestehend aus: -NH₂, -NO₂, -CN, -OR²⁶, Halogen, -C(O)-NH-R²⁸, -C(O)OR²⁸ und -C(O)R²⁸, und
das mit R³⁸ substituierte Aryl, substituierte Arylalkyl, substituierte Heteroaryl, substituierte Heteroarylalkyl, substituierte Cycloalkyl, substituierte Cycloalkylalkyl, substituierte Heterocycloalkyl, substituierte Heterocycloalkylalkyl, substituierte Alkylheteroaryl- und substituierte Alkylaryl- substituiert sind mit 1 bis 3 Substituenten, unabhängig ausgewählt aus der Gruppe, bestehend aus: (1) -NH₂, (2) -NO₂, (3) -CN, (4) -OH, (5) -OR²⁰, (6) -OCF₃, (7) -CF₃, (8) -C(O)R²⁶, (9) Alkyl, (10) Alkenyl, (11) Halogen, (12) -C(O)-NH-R²⁶, (13) -C(O)OR²⁶, (14) -C(O)-NR³²-(C(R³⁰)₂)ₙ-N(R₂₆)₂, (15) -S(O)ₜR²⁶, (16) -C(O)-N(R³²)(R²⁶), (17) -NR³²C(O)R²⁶,
(18) 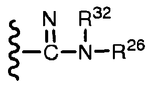 und
(19) -NHR²⁰,
R⁴² ausgewählt ist aus der Gruppe, bestehend aus: Alkyl, Aryl, Heteroaryl und Cycloalkyl,
R⁴⁴ ausgewählt ist aus der Gruppe, bestehend aus: H, Alkyl, Cycloalkyl und Cycloalkylalkyl, und
jedes R⁴⁶ unabhängig ausgewählt ist aus der Gruppe, bestehend aus: H, Alkyl, Cycloalkyl und Cycloalkylalkyl.

2. Die Verbindung nach Anspruch 1 mit der Formel, ausgewählt aus der Gruppe, bestehend aus: 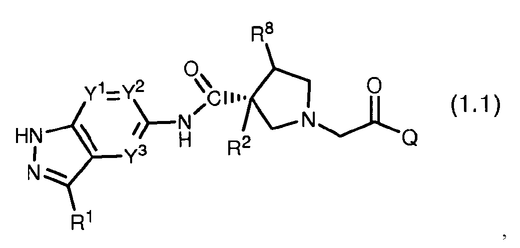 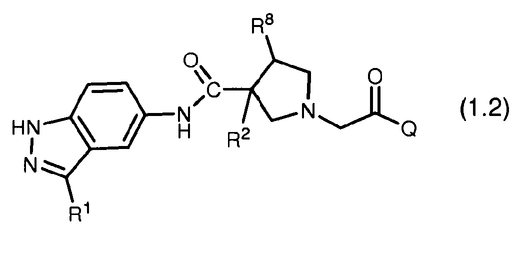 und 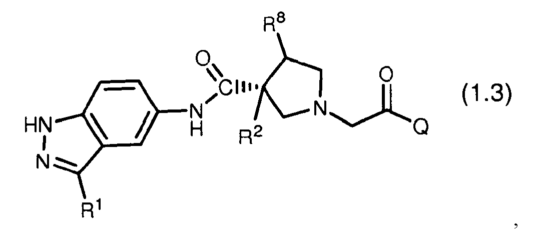 oder ein pharmazeutisch annehmbares Salz davon.

3. Die Verbindung nach einem der Ansprüche 1 oder 2 oder ein pharmazeutisch annehmbares Salz davon, wobei Q ausgewählt ist aus der Gruppe, bestehend aus: 2.1, 2.2, 2.3, 2.4, 2.5, 2.6, 2.7 und 2.8.

4. Die Verbindung nach einem der Ansprüche 1 oder 2 oder ein pharmazeutisch annehmbares Salz davon, wobei Q ausgewählt ist aus der Gruppe, bestehend aus: 2.17, 2.18, 2.19, 2.20, 2.21 und 2.22.

5. Die Verbindung nach Anspruch 1 oder ein pharmazeutisch annehmbares Salz davon, wobei Z¹ -CH₂- ist.

6. Die Verbindung nach Anspruch 3 oder ein pharmazeutisch annehmbares Salz davon, wobei Q ausgewählt ist aus der Gruppe, bestehend aus: Gruppen 2.1, 2.2 und 2.3.

7. Die Verbindung nach Anspruch 6 oder ein pharmazeutisch annehmbares Salz davon, wobei Q ausgewählt ist aus der Gruppe, bestehend aus: Gruppen 2.1, 2.2 und 2.3, und wobei 2.3 ausgewählt ist aus der Gruppe, bestehend aus:

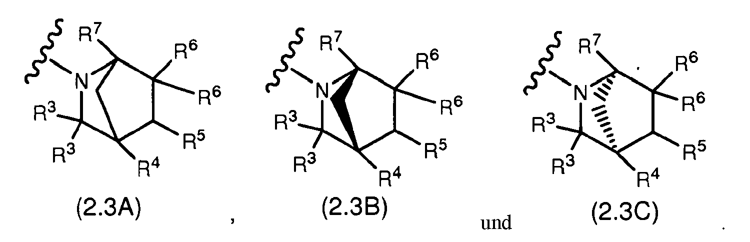

8. Die Verbindung nach einem der Ansprüche 1 oder 7 oder ein pharmazeutisch annehmbares Salz davon, wobei jedes R³, R⁴, R⁶ und R⁷ unabhängig ausgewählt ist aus der Gruppe, bestehend aus: H und Alkyl.

9. Die Verbindung nach Anspruch 8 oder ein pharmazeutisch annehmbares Salz davon, wobei jedes R³, R⁴, R⁶ und R⁷ unabhängig ausgewählt ist aus der Gruppe, bestehend aus: H und Methyl.

10. Die Verbindung nach Anspruch 9 oder ein pharmazeutisch annehmbares Salz davon, wobei jedes R³, R⁴, R⁶ und R⁷ H ist.

11. Die Verbindung nach Anspruch 3 oder ein pharmazeutisch annehmbares Salz davon, wobei Q ausgewählt ist aus der Gruppe, bestehend aus: Gruppen 2.6 und 2.7.

12. Die Verbindung nach Anspruch 11 oder ein pharmazeutisch annehmbares Salz davon, wobei Q ausgewählt ist aus der Gruppe, bestehend aus: Gruppen 2.6 und 2.7, und wobei 2.7 ausgewählt ist aus der Gruppe, bestehend aus:

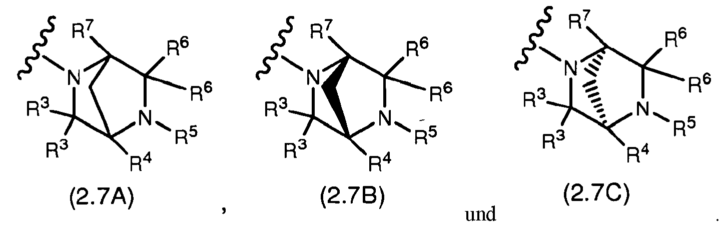

13. Die Verbindung nach einem der Ansprüche 1 oder 12 oder ein pharmazeutisch annehmbares Salz davon, wobei jedes R³, R⁴, R⁶ und R⁷ unabhängig ausgewählt ist aus der Gruppe, bestehend aus: H und Alkyl.

14. Die Verbindung nach Anspruch 13 oder ein pharmazeutisch annehmbares Salz davon, wobei jedes R³, R⁴, R⁶ und R⁷ unabhängig ausgewählt ist aus der Gruppe, bestehend aus: H und Methyl.

15. Die Verbindung nach Anspruch 14 oder ein pharmazeutisch annehmbares Salz davon, wobei jedes R³, R⁴, R⁶ und R⁷ H ist.

16. Die Verbindung nach Anspruch 10 oder ein pharmazeutisch annehmbares Salz davon, wobei Q 2.1 ist.

17. Die Verbindung nach Anspruch 10 oder ein pharmazeutisch annehmbares Salz davon, wobei Q 2.3B ist.

18. Die Verbindung nach Anspruch 15 oder ein pharmazeutisch annehmbares Salz davon, wobei Q 2.6 ist.

19. Die Verbindung nach Anspruch 15 oder ein pharmazeutisch annehmbares Salz davon, wobei Q 2.7A ist.

20. Die Verbindung nach Anspruch 15 oder ein pharmazeutisch annehmbares Salz davon, wobei Q 2.7B ist.

21. Die Verbindung nach Anspruch 1 oder ein pharmazeutisch annehmbares Salz davon, wobei Q 2.17 ist.

22. Die Verbindung nach Anspruch 21 oder ein pharmazeutisch annehmbares Salz davon, wobei jedes R³, R⁴, R⁶ und R⁷ unabhängig ausgewählt ist aus der Gruppe, bestehend aus: H und Methyl.

23. Die Verbindung nach Anspruch 22 oder ein pharmazeutisch annehmbares Salz davon, wobei jedes R³, R⁴, R⁶ und R⁷ H ist.

24. Die Verbindung nach einem der Ansprüche 16, 17, 18, 19 oder 20 oder ein pharmazeutisch annehmbares Salz davon, wobei die Verbindung der Formel 1.0 ist:

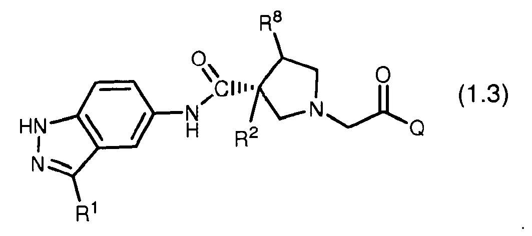

25. Die Verbindung nach Anspruch 2 oder ein pharmazeutisch annehmbares Salz davon, wobei die Verbindung der Formel 1.0 eine Verbindung der Formel 1.3 ist und Q ausgewählt ist aus der Gruppe, bestehend aus: 2.1, 2.2 und 2.3, und wobei 2.3 ausgewählt ist aus der Gruppe, bestehend aus: 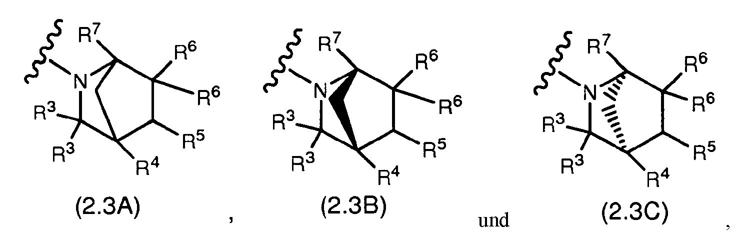 und R³, R⁴, R⁶ und R⁷ jeweils unabhängig ausgewählt sind aus der Gruppe, bestehend aus: H und Alkyl.

26. Die Verbindung nach Anspruch 25 oder ein pharmazeutisch annehmbares Salz davon, wobei R³, R⁴, R⁶ und R⁷ ausgewählt sind aus der Gruppe, bestehend aus: H und Methyl.

27. Die Verbindung nach Anspruch 26 oder ein pharmazeutisch annehmbares Salz davon, wobei Q ausgewählt ist aus der Gruppe, bestehend aus: 2.1 und 2.3B.

28. Die Verbindung nach Anspruch 27 oder ein pharmazeutisch annehmbares Salz davon, wobei Q 2.1 ist und R³, R⁴, R⁶ und R⁷ jeweils H sind.

29. Die Verbindung nach Anspruch 2 oder ein pharmazeutisch annehmbares Salz davon, wobei die Verbindung der Formel 1.0 eine Verbindung der Formel 1.3 ist und Q ausgewählt ist aus der Gruppe, bestehend aus: 2.6 und 2.7, und wobei 2.7 ausgewählt ist aus der Gruppe, bestehend aus: 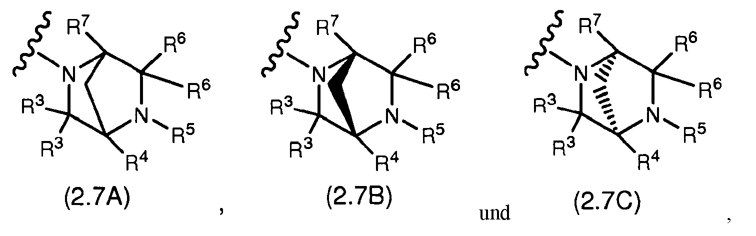 und R³, R⁴, R⁶ und R⁷ jeweils unabhängig ausgewählt sind aus der Gruppe, bestehend aus: H und Alkyl.

30. Die Verbindung nach Anspruch 29 oder ein pharmazeutisch annehmbares Salz davon, wobei R³, R⁴, R⁶ und R⁷ ausgewählt sind aus der Gruppe, bestehend aus: H und Methyl.

31. Die Verbindung nach Anspruch 30 oder ein pharmazeutisch annehmbares Salz davon, wobei Q ausgewählt ist aus der Gruppe, bestehend aus: 2.6, 2.7A und 2.7B.

32. Die Verbindung nach Anspruch 31 oder ein pharmazeutisch annehmbares Salz davon, wobei Q 2.6 ist und R³, R⁴, R⁶ und R⁷ jeweils H sind.

33. Die Verbindung nach Anspruch 31 oder ein pharmazeutisch annehmbares Salz davon, wobei Q 2.7A ist und R³, R⁴, R⁶ und R⁷ jeweils H sind.

34. Die Verbindung nach Anspruch 31 oder ein pharmazeutisch annehmbares Salz davon, wobei Q 2.7B ist und R³, R⁴, R⁶ und R⁷ jeweils H sind.

35. Die Verbindung nach Anspruch 1 oder ein pharmazeutisch annehmbares Salz davon, wobei Q ausgewählt ist aus der Gruppe, bestehend aus:
(A) 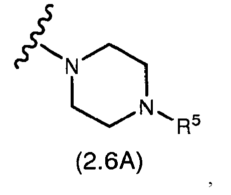
(B) 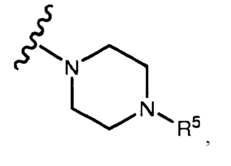 substituiert mit einem oder zwei Substituenten, unabhängig ausgewählt aus der Gruppe, bestehend aus R³-Gruppen, mit der Maßgabe, dass der eine oder die zwei Substituenten nicht H ist/sind,
(C) 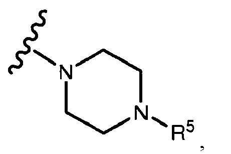 substituiert mit einer oder zwei Alkylgruppen,
(D) 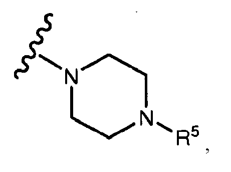 substituiert mit einer oder zwei Methylgruppen,
(E) 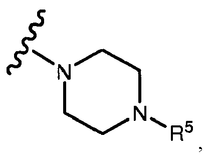 substituiert mit einer Methylgruppe, und
(F)

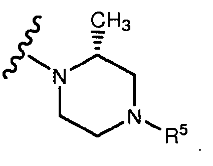

36. Die Verbindung nach Anspruch 1 oder ein pharmazeutisch annehmbares Salz davon, wobei Q ausgewählt ist aus der Gruppe, bestehend aus:
(A) 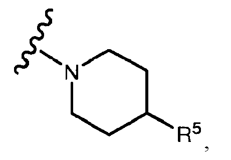 substituiert mit einem oder zwei Substituenten, unabhängig ausgewählt aus der Gruppe, bestehend aus R³-Gruppen, mit der Maßgabe, dass der eine oder die zwei Substituenten nicht H ist/sind,
(B) 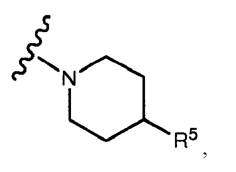 substituiert mit einer oder zwei Alkylgruppen,
(C) 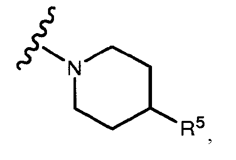 substituiert mit einer oder zwei Methylgruppen, und
(D) 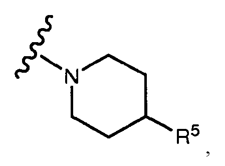 substituiert mit einer Methylgruppe.

37. Die Verbindung nach einem der Ansprüche 1 oder 2 oder ein pharmazeutisch annehmbares Salz davon, wobei R¹ ausgewählt ist aus der Gruppe, bestehend aus:

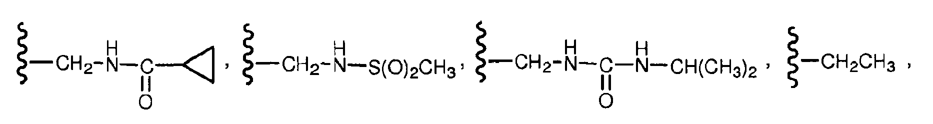

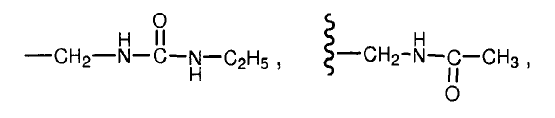

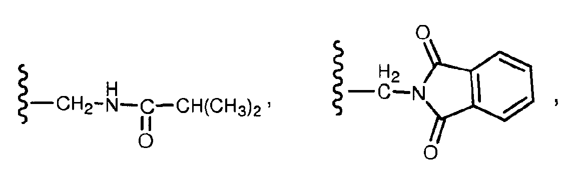

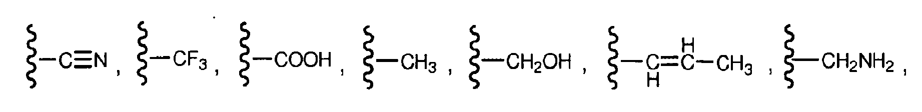

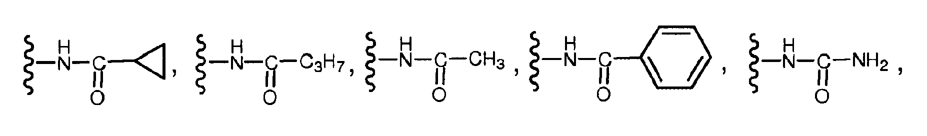

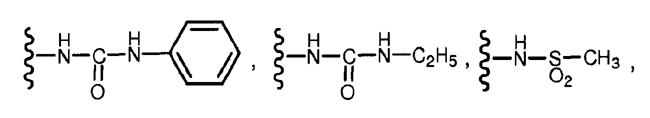

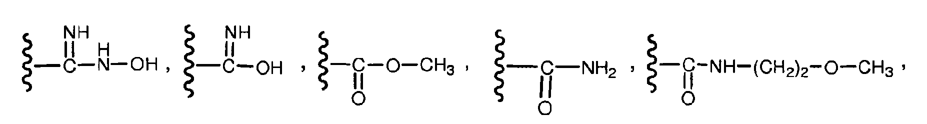

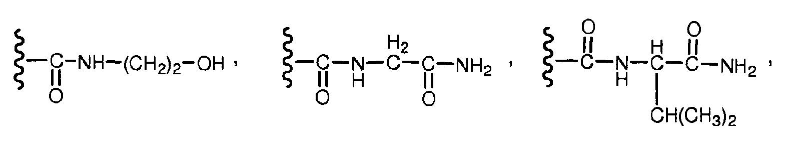

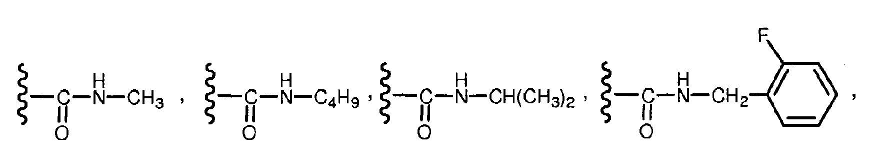

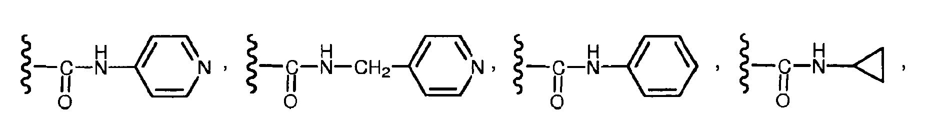

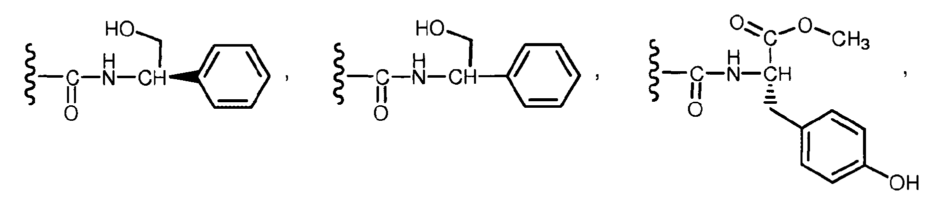

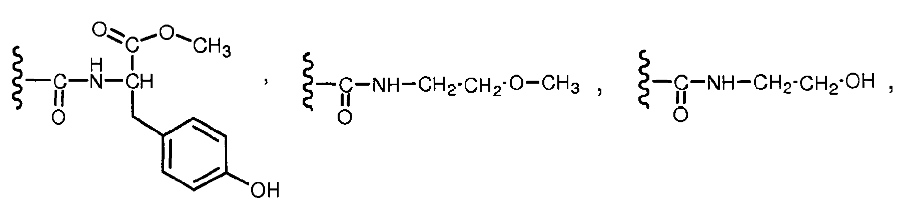

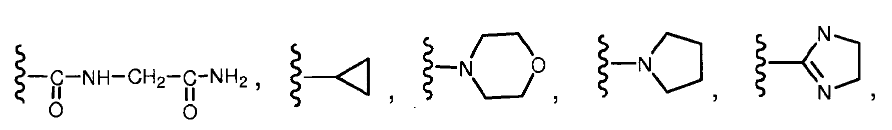

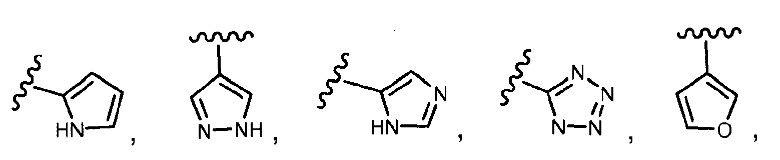

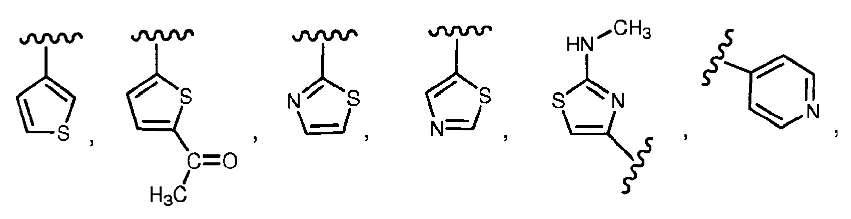

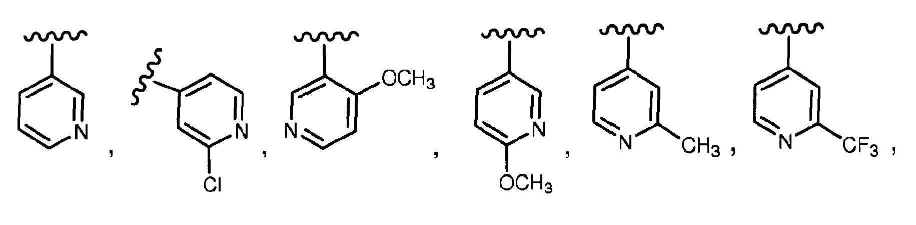

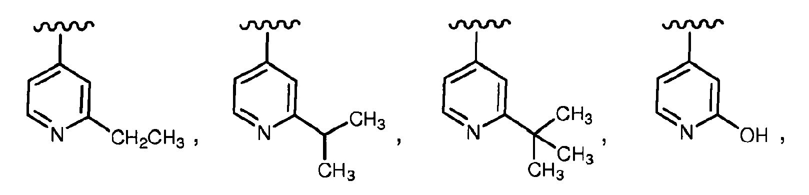

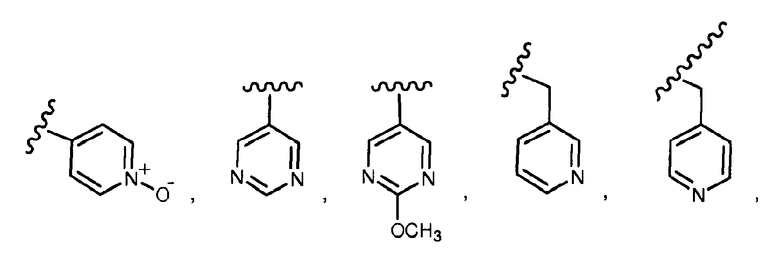

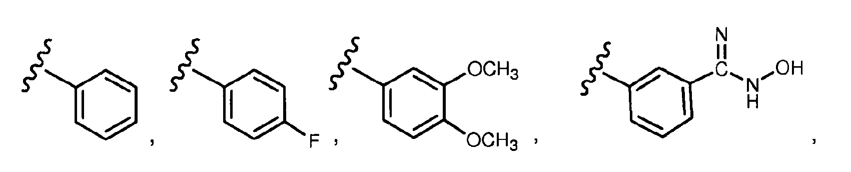

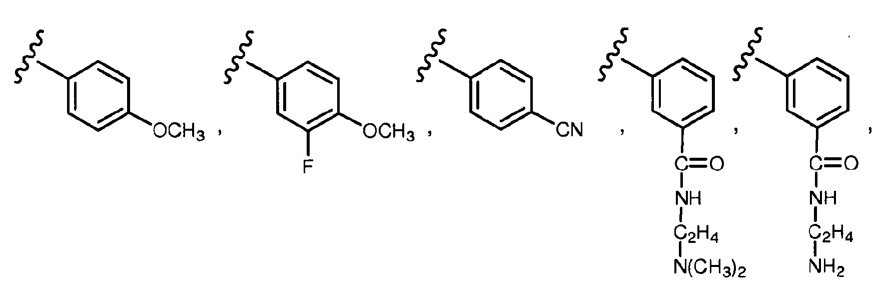

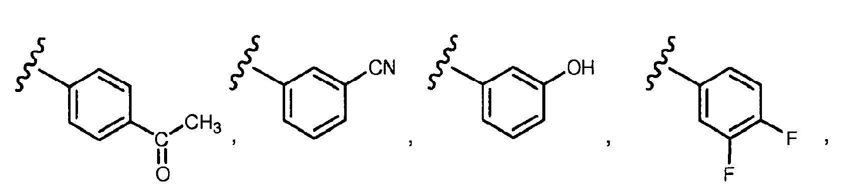

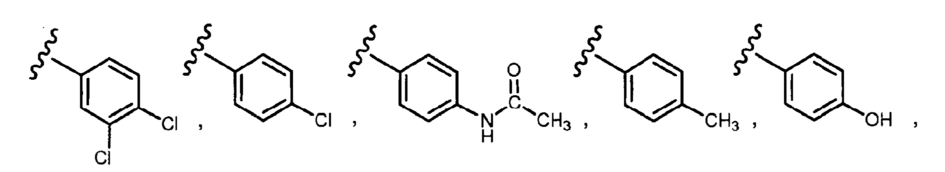

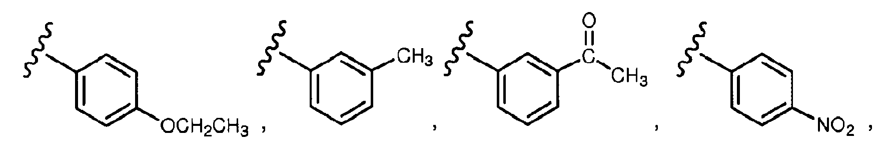

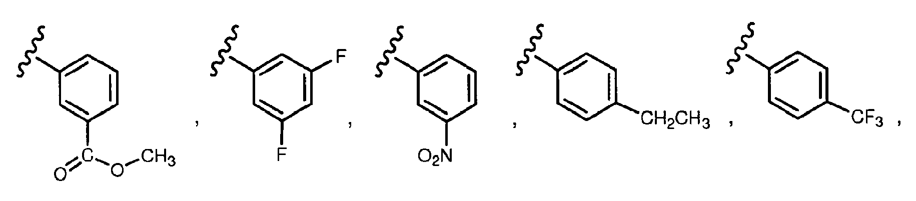

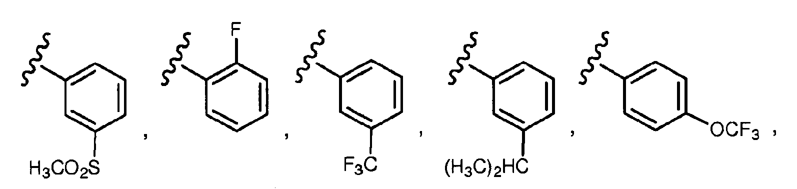

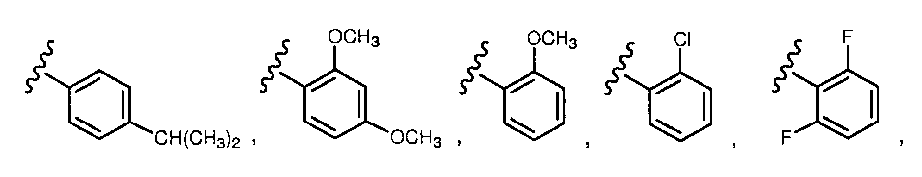

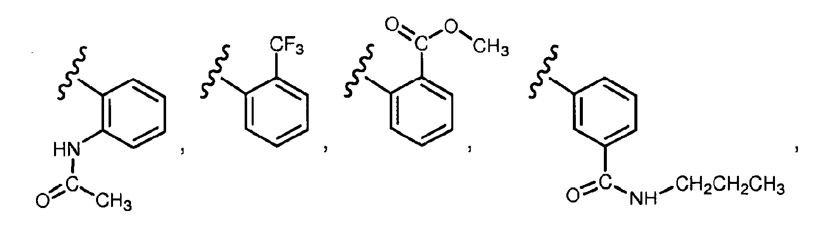

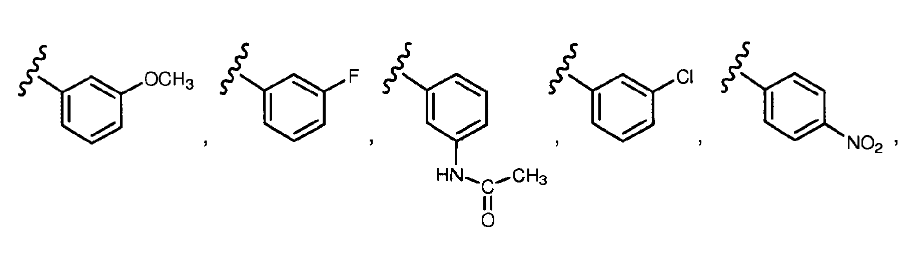

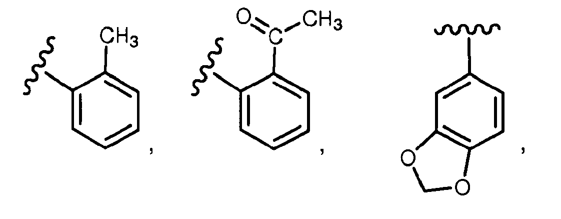

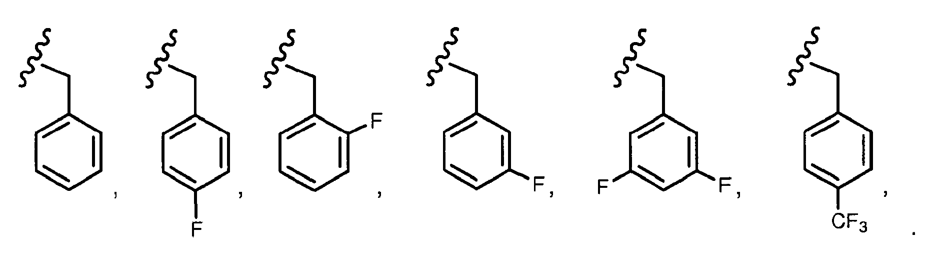

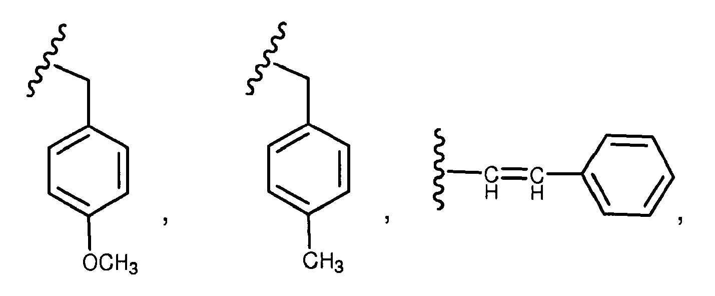

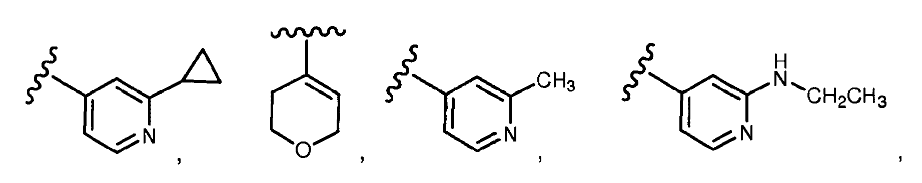

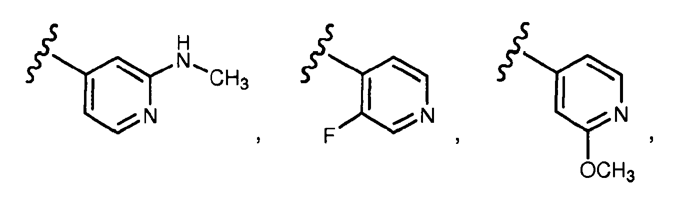

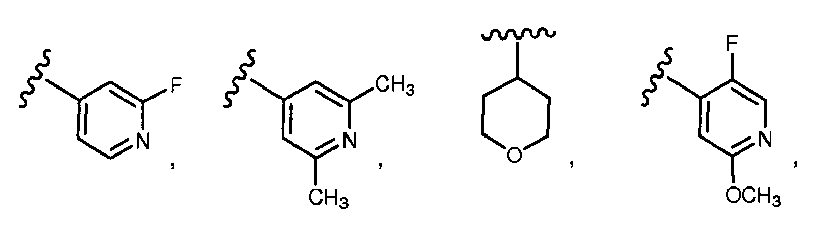

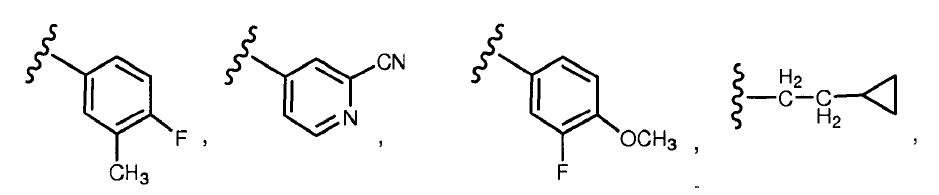

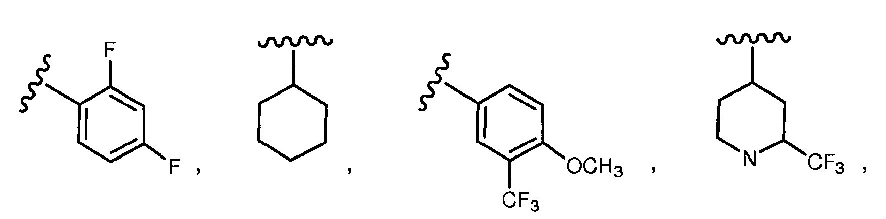

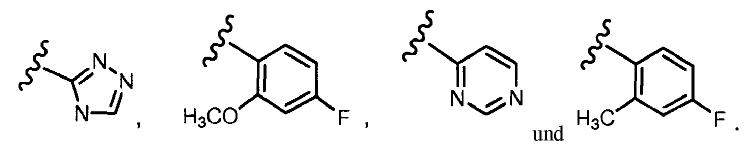

38. Die Verbindung nach Anspruch 1 oder ein pharmazeutisch annehmbares Salz davon, wobei R¹ Aryl ist.

39. Die Verbindung nach Anspruch 1 oder ein pharmazeutisch annehmbares Salz davon, wobei R¹ Heteroaryl oder substituiertes Heteroaryl ist, wobei das substituierte Heteroaryl substituiert ist mit 1 bis 3 Substituenten, unabhängig ausgewählt aus der Gruppe, bestehend aus: (1) -NH₂, (2) -NO₂, (3) -CN, (4) -OH, (5) -OR²⁰, (6) -OCF₃, (7) Alkyl, substituiert mit 1 bis 3 unabhängig ausgewählten Halogenatomen, (8) -C(O)R³⁸, (9) Alkyl, (10) Alkenyl, (11) Halogen, (12) -C(O)-NH-R²⁶, (13) -C(O)OR³⁸, (14) -C(O)-NR³²-(C(R³⁰)₂)ₙ-N(R³⁸)₂, (15) -S(O)ₜR³⁸, (16) -C(O)-NR³²-R³⁸, (17) -NR³²-C(O)-R³⁸,
(18) 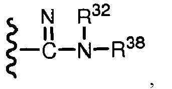 (19) -NHR²⁰ und (20) Cycloalkyl.

40. Die Verbindung nach einem der Ansprüche 1 oder 2 oder ein pharmazeutisch annehmbares Salz davon, wobei R⁵ ausgewählt ist aus der Gruppe, bestehend aus:

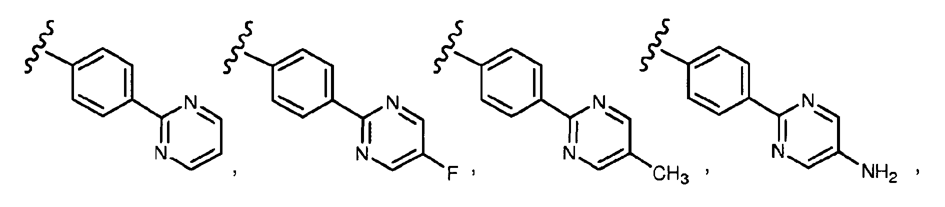

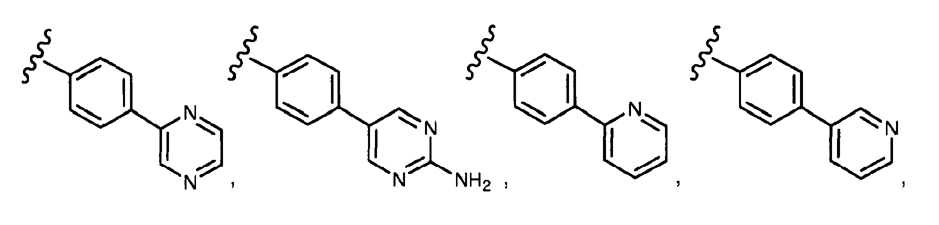

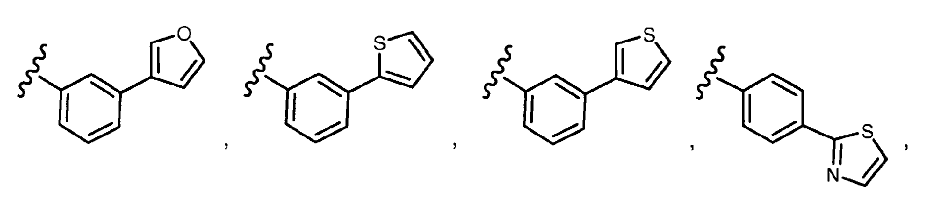

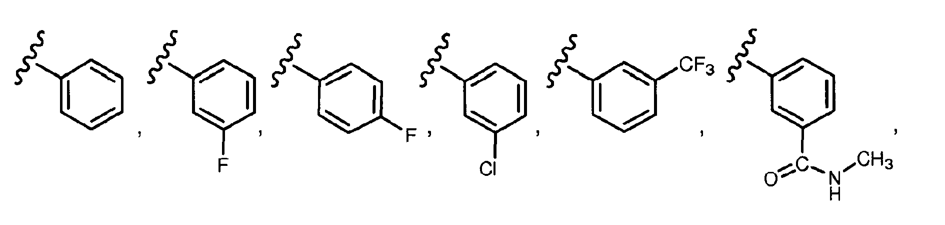

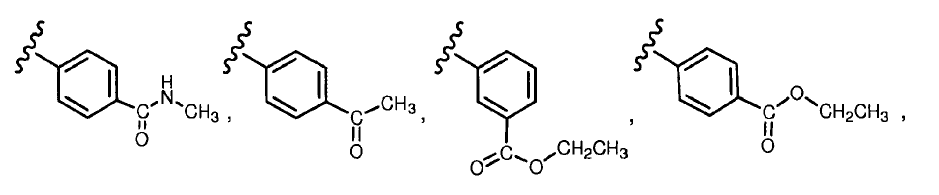

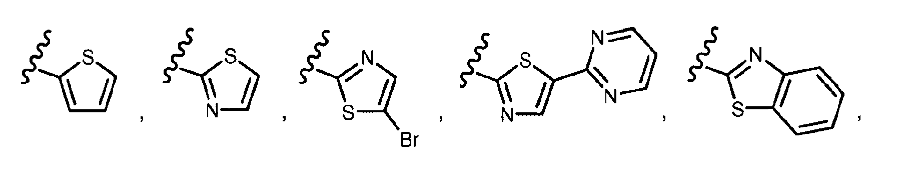

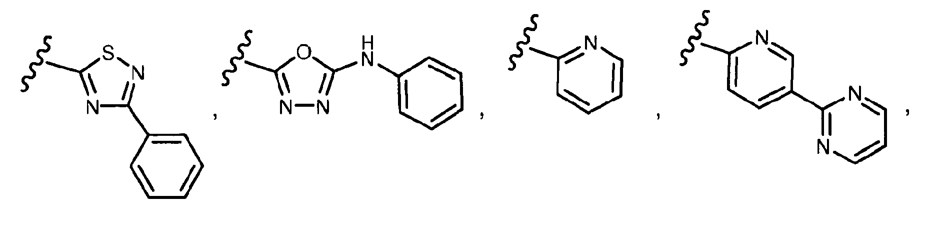

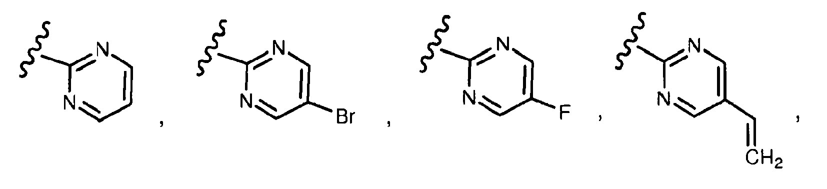

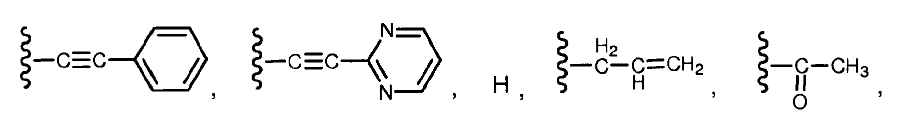

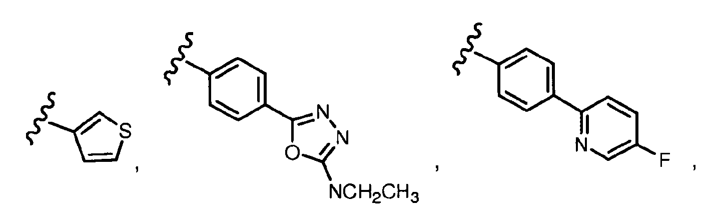

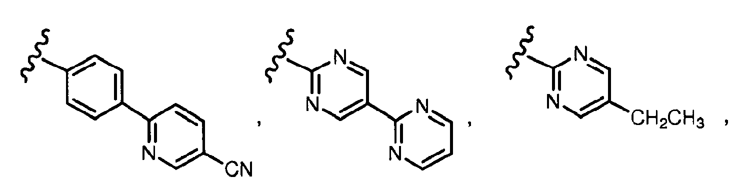

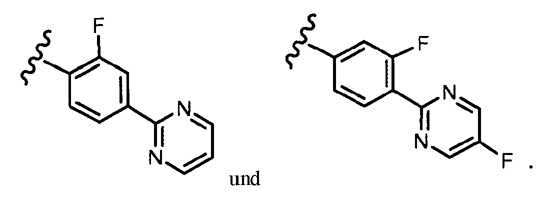

41. Die Verbindung nach einem der Ansprüche 1 oder 7 oder ein pharmazeutisch annehmbares Salz davon, wobei R¹ ausgewählt ist aus der Gruppe, bestehend aus: 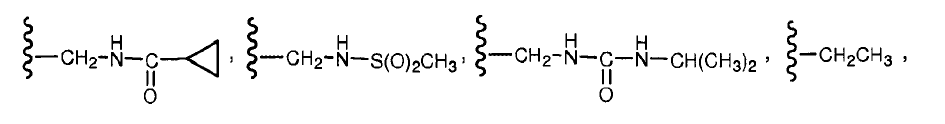 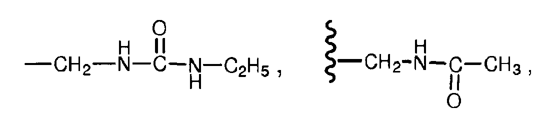 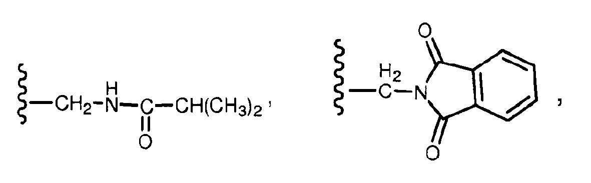 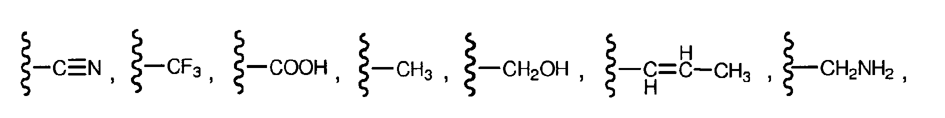 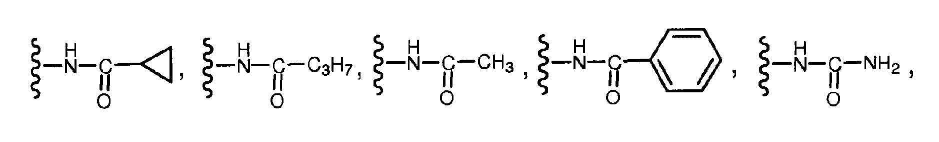 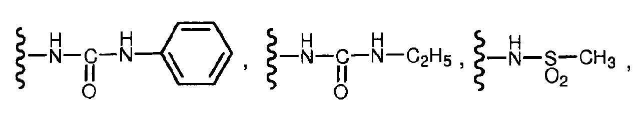 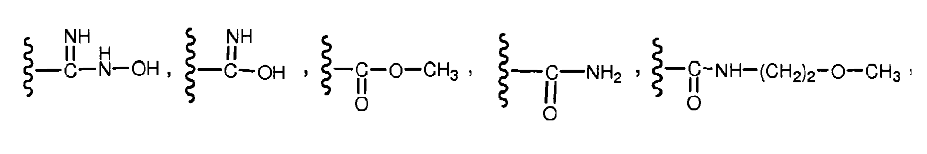 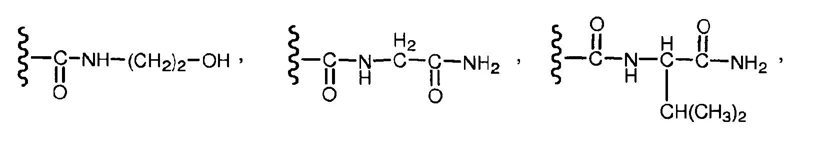 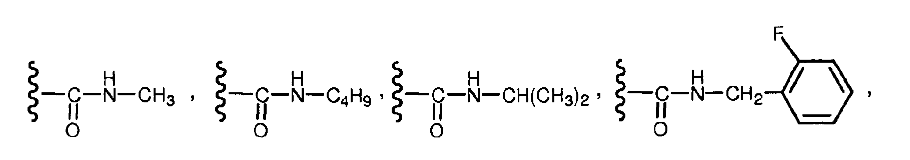 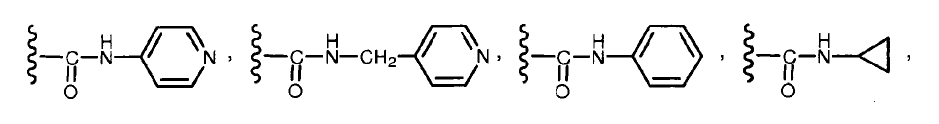 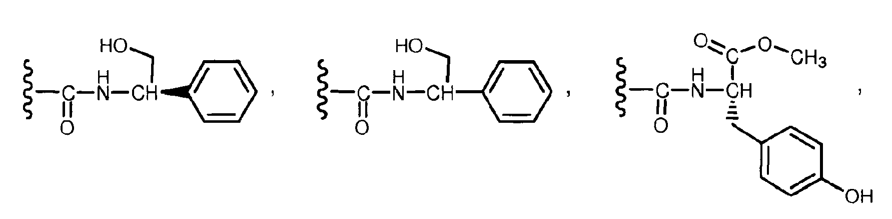 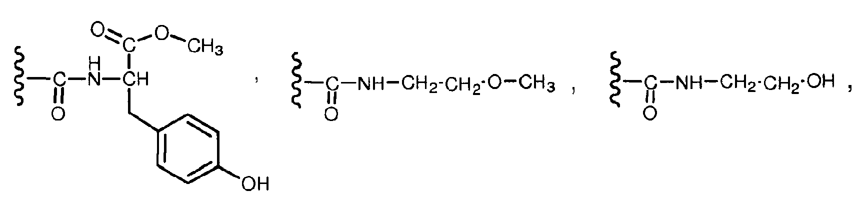 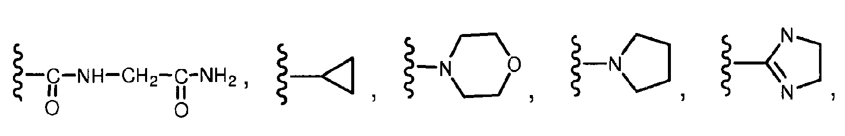 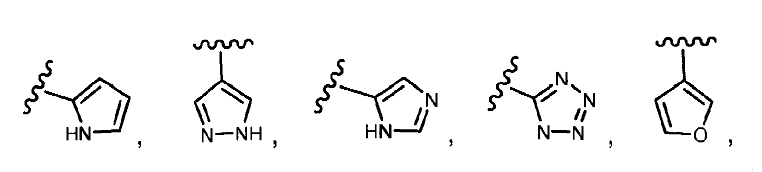 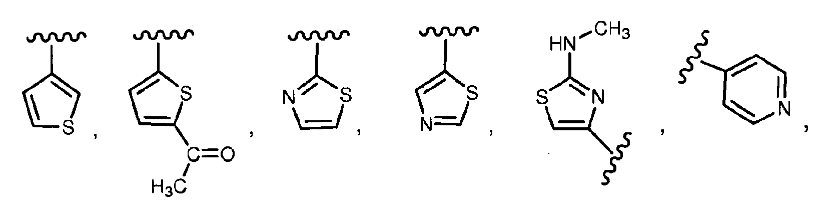 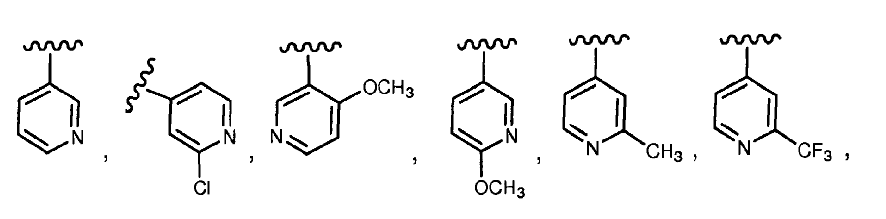 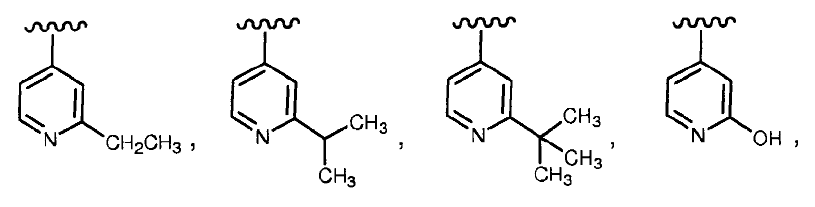 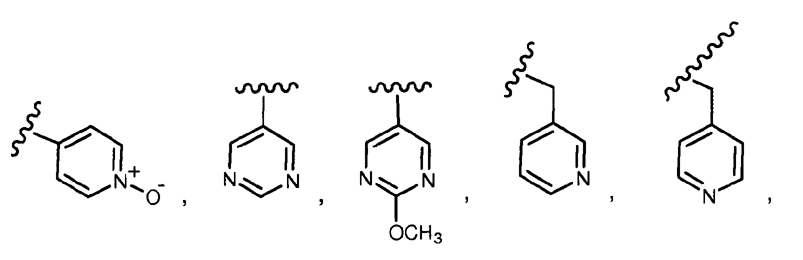 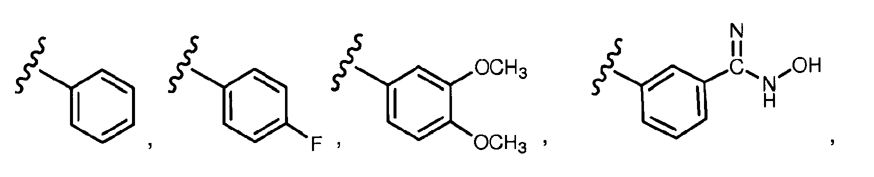 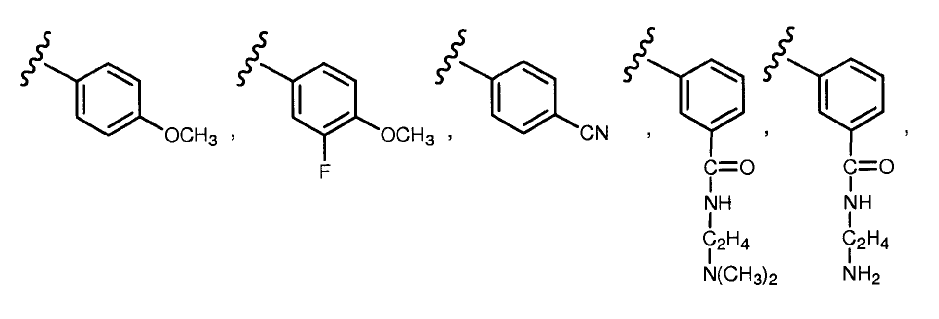 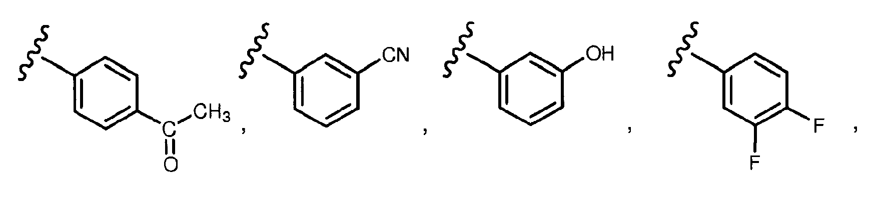 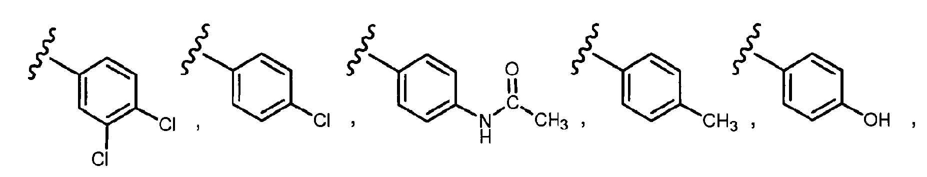 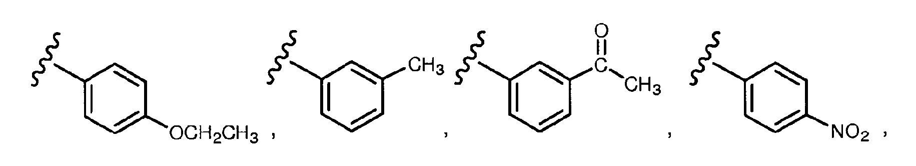 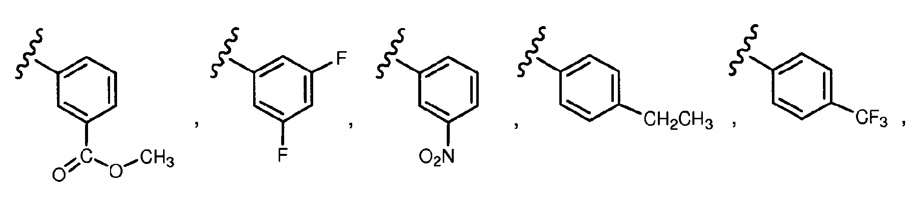 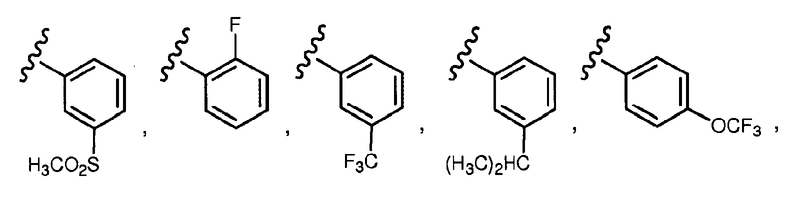 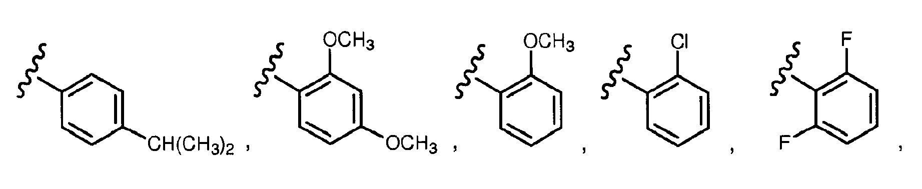 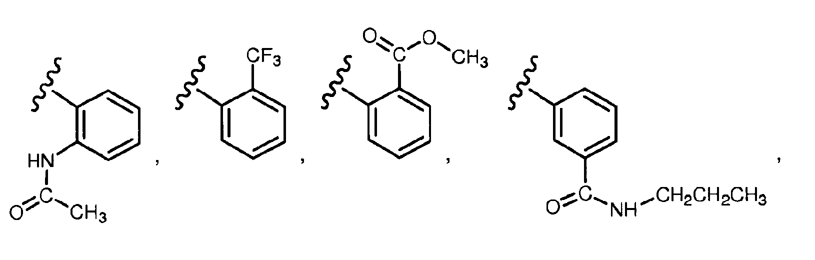 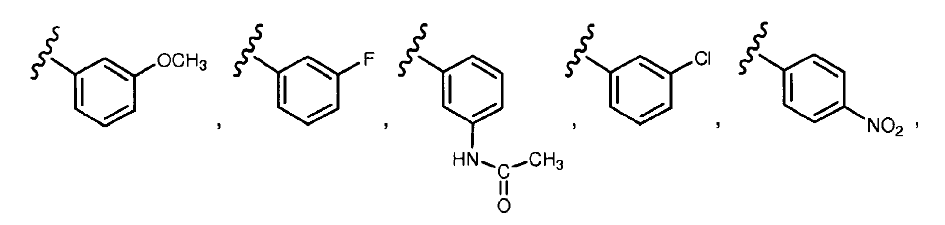 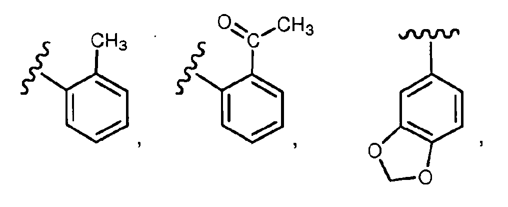 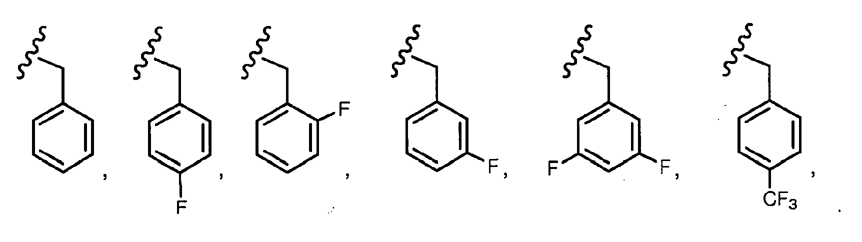 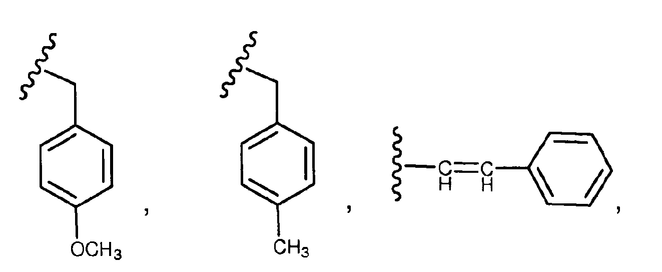 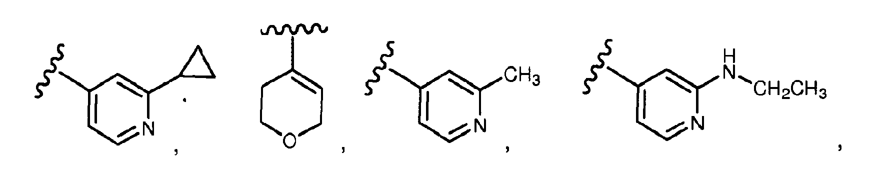 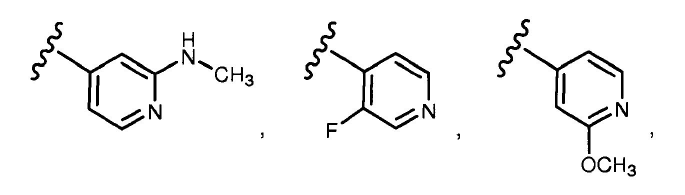 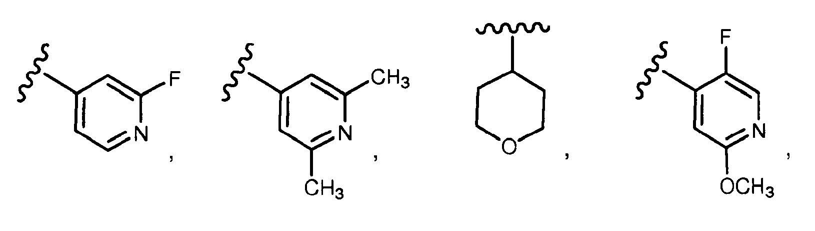 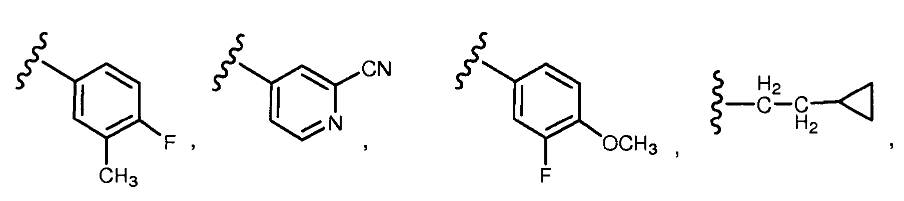 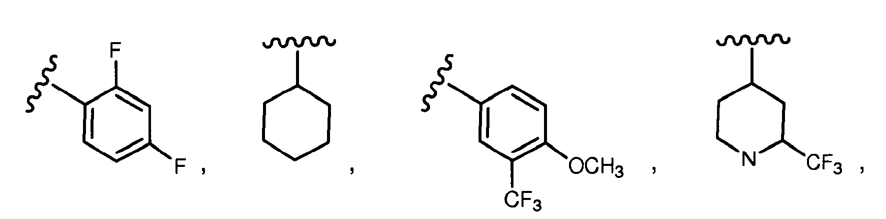 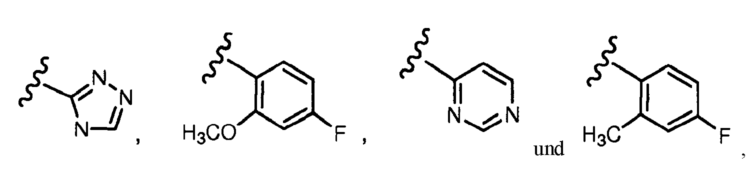 und
R⁵ ausgewählt ist aus der Gruppe, bestehend aus:

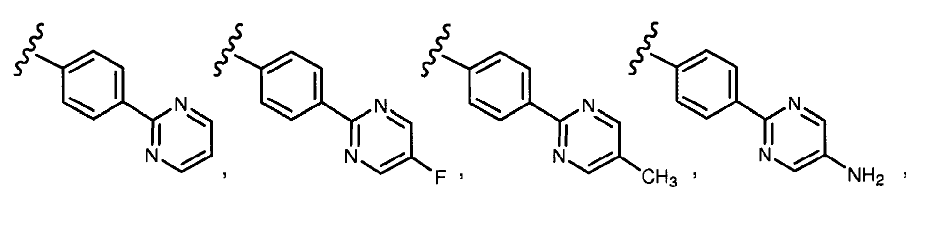

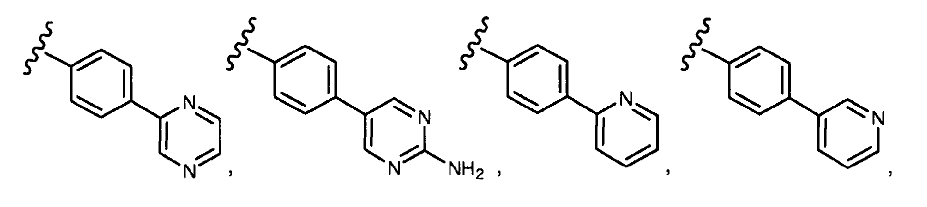

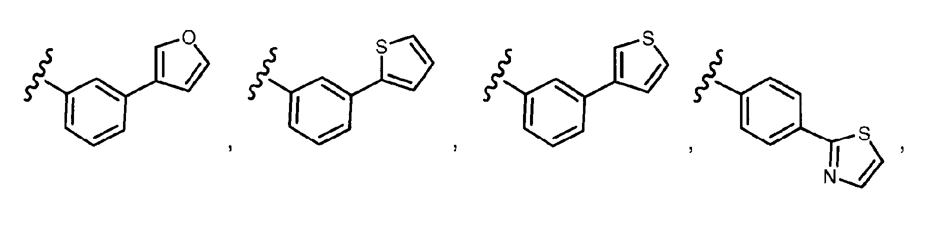

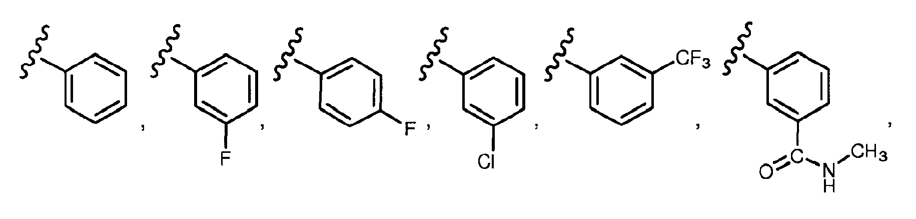

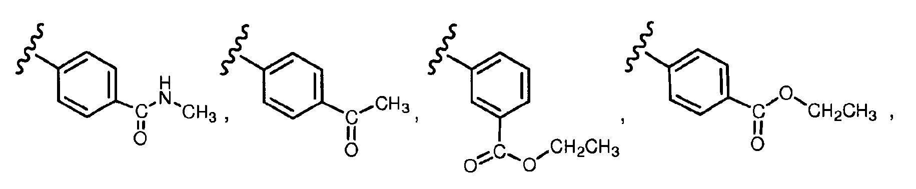

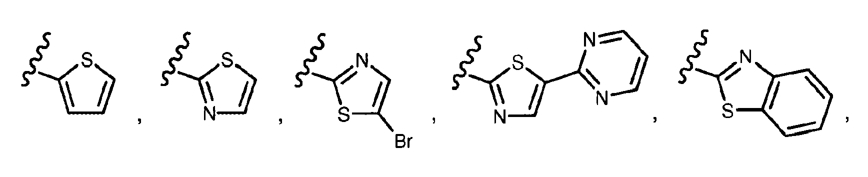

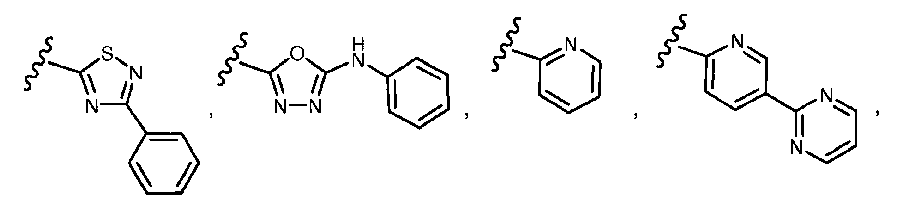

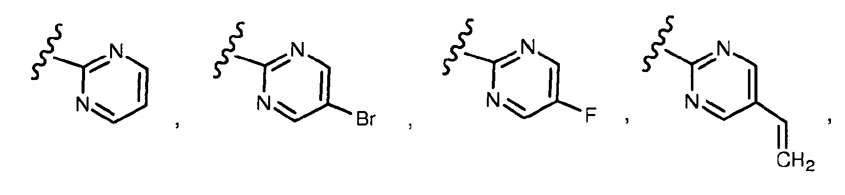

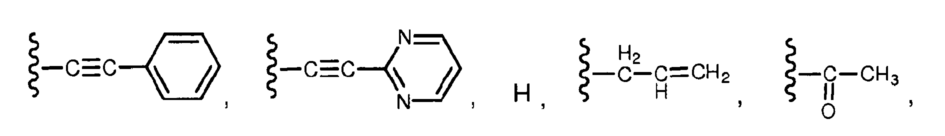

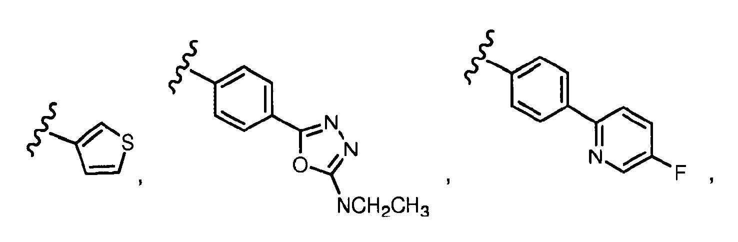

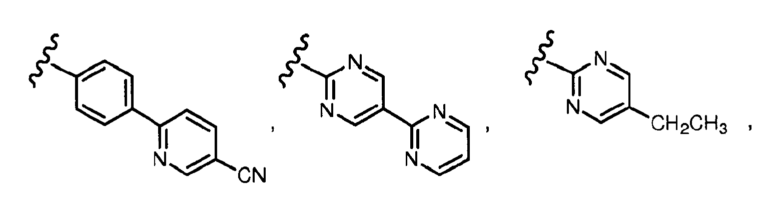

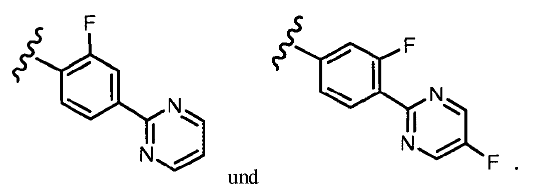

42. Die Verbindung nach Anspruch 41 oder ein pharmazeutisch annehmbares Salz davon, wobei R¹ ausgewählt ist aus der Gruppe, bestehend aus:

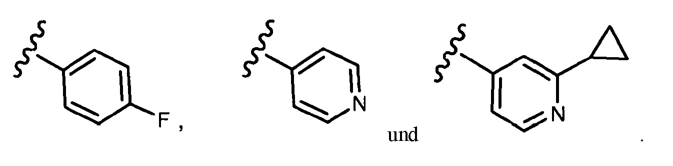

43. Die Verbindung nach einem der Ansprüche 1 oder 41 oder ein pharmazeutisch annehmbares Salz davon, wobei R² ausgewählt ist aus der Gruppe, bestehend aus:

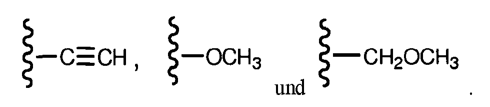

44. Die Verbindung nach Anspruch 41 oder ein pharmazeutisch annehmbares Salz davon, wobei Q ausgewählt ist aus der Gruppe, bestehend aus: 2.1, 2.2 und 2.3, und wobei 2.3 ausgewählt ist aus der Gruppe, bestehend aus: 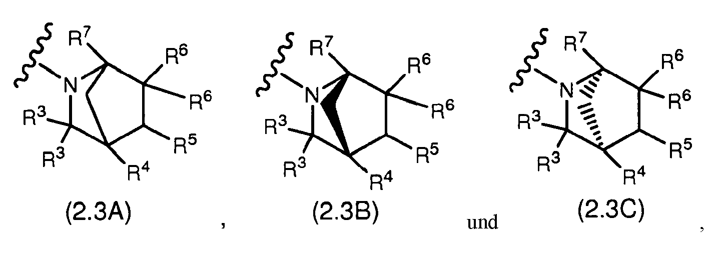 und R³, R⁴, R⁶ und R⁷ jeweils unabhängig ausgewählt sind aus der Gruppe, bestehend aus: H und Alkyl.

45. Die Verbindung nach Anspruch 44 oder ein pharmazeutisch annehmbares Salz davon, wobei R³, R⁴, R⁶ und R⁷ jeweils H sind.

46. Die Verbindung nach Anspruch 45 oder ein pharmazeutisch annehmbares Salz davon, wobei Q 2.1 ist.

47. Die Verbindung nach Anspruch 41 oder ein pharmazeutisch annehmbares Salz davon, wobei Q ausgewählt ist aus der Gruppe, bestehend aus: 2.6 und 2.7, und wobei 2.7 ausgewählt ist aus der Gruppe, bestehend aus: 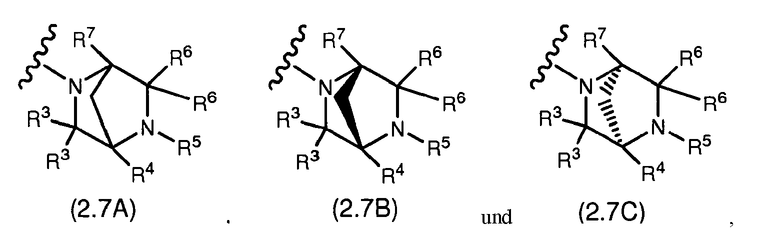 und R³, R⁴, R⁶ und R⁷ jeweils unabhängig ausgewählt sind aus der Gruppe, bestehend aus: H und Alkyl.

48. Die Verbindung nach Anspruch 47 oder ein pharmazeutisch annehmbares Salz davon, wobei R³, R⁴, R⁶ und R⁷ jeweils H sind.

49. Die Verbindung nach Anspruch 48 oder ein pharmazeutisch annehmbares Salz davon, wobei Q 2.6 ist.

50. Die Verbindung nach Anspruch 48 oder ein pharmazeutisch annehmbares Salz davon, wobei Q 2.7B ist.

51. Die Verbindung nach Anspruch 41 oder ein pharmazeutisch annehmbares Salz davon, wobei Q ausgewählt ist aus der Gruppe, bestehend aus: 2.17, 2.18, 2.19, 2.20, 2.21 und 2.22, und wobei R³, R⁴, R⁶ und R⁷ jeweils unabhängig ausgewählt sind aus der Gruppe, bestehend aus: H und Alkyl.

52. Die Verbindung nach Anspruch 1 oder ein pharmazeutisch annehmbares Salz davon, wobei Q 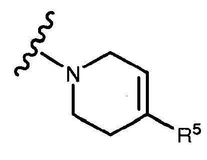 ist.

53. Die Verbindung nach Anspruch 1, ausgewählt aus der Gruppe, bestehend aus:
(A) den Endverbindungen der Beispiele 1-84, 86-125, 141-418, 420-429, 431, 432 und 434-611,
(B) den Endverbindungen der Beispiele 1, 2, 4-61, 65-73, 77-84, 86, 88-98, 100, 102-114, 116-118, 120, 121, 124, 125, 151-155, 159-179, 183, 184, 186, 188-193, 196-199, 202-205, 250, 253-259, 260-299, 301-318, 320-323, 332-347, 356 (Isomer Z), 356 (Isomer E), 357-360, 362, 362 (Verbindung 9P), 364, 368-418, 420-429, 431, 432, 434-436, 440-509, 511-602, 606, 607 und 609-611, und
(C) den Endverbindungen der Beispiele 6, 100, 183, 184, 186, 188-192, 261, 440-450 und 510-517,
oder ein pharmazeutisch annehmbares Salz davon.

54. Die Verbindung nach Anspruch 1 mit der Formel: 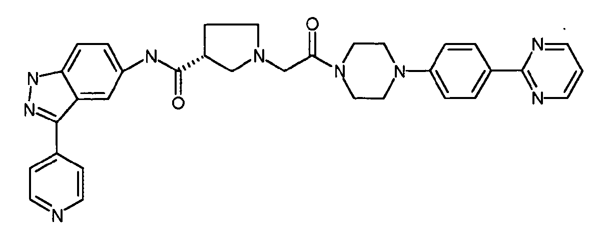 oder ein pharmazeutisch annehmbares Salz davon.

55. Die Verbindung nach Anspruch 1 mit der Formel: 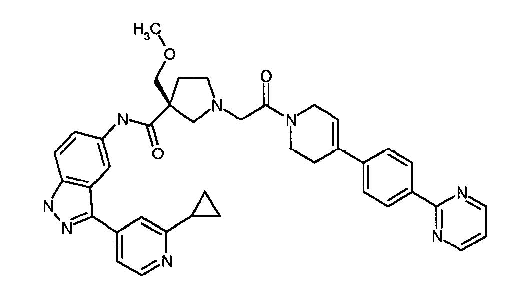 oder ein pharmazeutisch annehmbares Salz davon.

56. Die Verbindung nach Anspruch 1 mit der Formel: 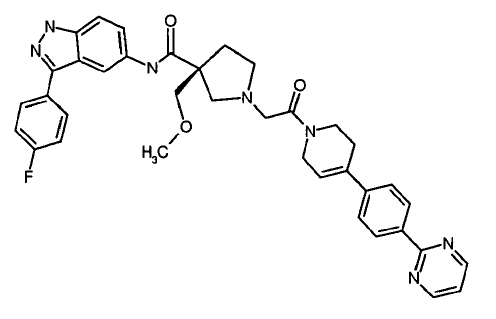 oder ein pharmazeutisch annehmbares Salz davon.

57. Die Verbindung nach Anspruch 1 mit der Formel: 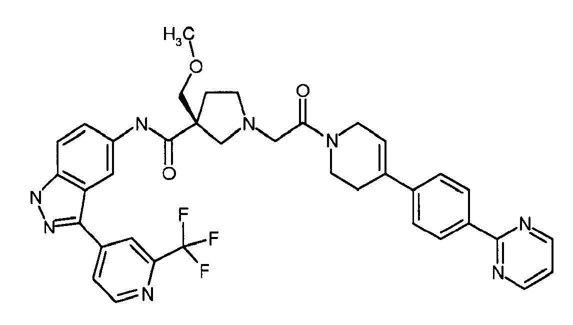 oder ein pharmazeutisch annehmbares Salz davon.

58. Die Verbindung nach Anspruch 1 mit der Formel: 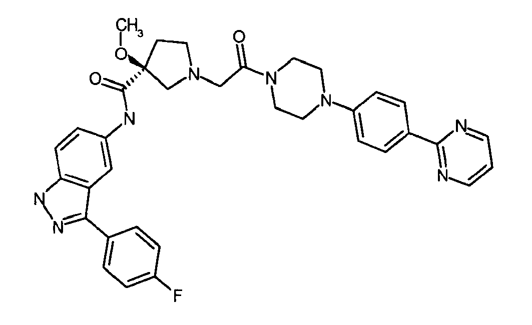 oder ein pharmazeutisch annehmbares Salz davon.

59. Die Verbindung nach Anspruch 1 mit der Formel: 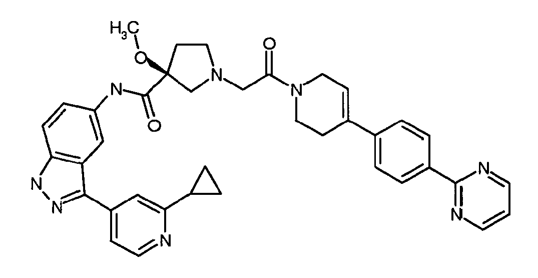 oder ein pharmazeutisch annehmbares Salz davon.

60. Die Verbindung nach Anspruch 1 mit der Formel: 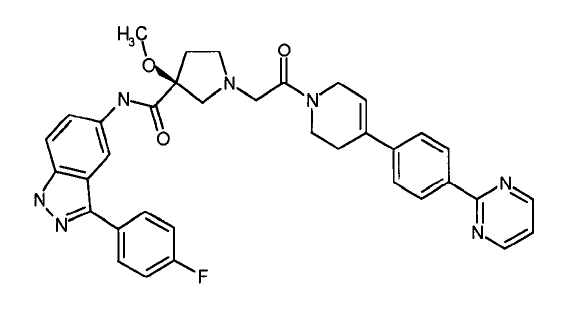 oder ein pharmazeutisch annehmbares Salz davon.

61. Die Verbindung nach Anspruch 1 mit der Formel: 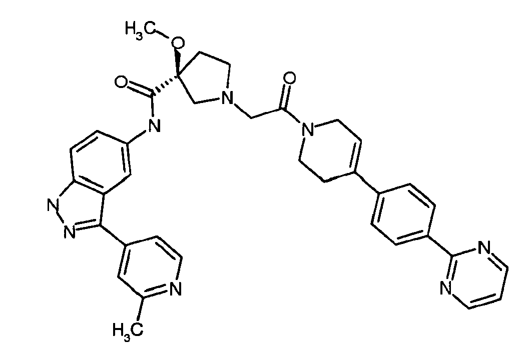 oder ein pharmazeutisch annehmbares Salz davon.

62. Die Verbindung nach Anspruch 1 mit der Formel: 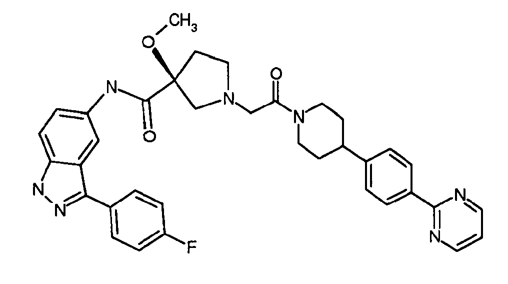 oder ein pharmazeutisch annehmbares Salz davon.

63. Die Verbindung nach Anspruch 1 mit der Formel: 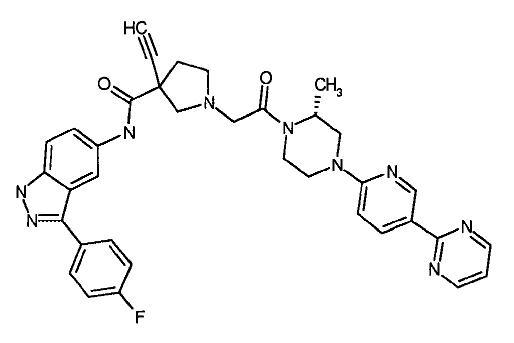 oder ein pharmazeutisch annehmbares Salz davon.

64. Eine pharmazeutische Zusammensetzung, umfassend wenigstens eine Verbindung nach Anspruch 1 oder ein pharmazeutisch annehmbares Salz davon und einen pharmazeutisch annehmbaren Träger.

65. Eine Verbindung nach einem der Ansprüche 1 bis 63 oder ein pharmazeutisch annehmbares Salz davon zur Verwendung als ein Medikament.

66. Eine Verwendung wenigstens einer Verbindung nach einem der Ansprüche 1 bis 63 oder eines pharmazeutisch annehmbaren Salzes davon:
(A) zur Herstellung eines Medikaments zur Behandlung von Krebs,
(B) zur Herstellung eines Medikaments zur Behandlung von Krebs, wobei das Medikament in einer Form zur Verabreichung mit wenigstens einem chemotherapeutischen Mittel vorliegt, oder
(C) zur Herstellung eines Medikaments zur Behandlung von Krebs, wobei das Medikament in einer Form zur Verabreichung mit wenigstens einem chemotherapeutischen Mittel und Strahlentherapie vorliegt.

67. Eine Verwendung wenigstens einer Verbindung nach einem der Ansprüche 1 bis 63 oder eines pharmazeutisch annehmbaren Salzes davon zur Herstellung eines Medikaments zur Behandlung von Krebs, wobei das Medikament in einer Form zur Verabreichung mit wenigstens einem chemotherapeutischen Mittel vorliegt, ausgewählt aus der Gruppe, bestehend aus: (1) Taxanen, (2) Platin-Koordinationsverbindungen, (3) Inhibitoren des epidermalen Wachstumsfaktors (EGF), welche Antikörper sind, (4) EGF-Inhibitoren, welche kleine Moleküle sind, (5) Inhibitoren des vaskulo-endothelialen Wachstumsfaktors (VEGF), welche Antikörper sind, (6) VEGF-Kinase-Inhibitoren, welche kleine Moleküle sind, (7) Östrogenrezeptorantagonisten oder selektiven Östrogenrezeptormodulatoren (SERMs), (8) Antitumor-Nukleosid-Derivate, (9) Epothilone, (10) Topoisomerase-Inhibitoren (11) Vincaalkaloide, (12) Antikörper, die Inhibitoren der αVβ3-Integrine sind, (13) Folat-Antagonisten, (14) Ribonukleotidreduktase-Inhibitoren, (15) Anthracycline, (16) biologische Präparate, (17) Inhibitoren der Angiogenese und/oder Suppressoren von Tumornekrosefaktor alpha (TNF-alpha), wie z.B. Thalidomid (oder verwandtes Imid), (18) Bcr/abl-Kinase-Inhibitoren, (19) MEK1- und/oder MEK2-Inhibitoren, die kleine Moleküle sind, (20) IGF-1- und IGF-2-Inhibitoren, die kleine Moleküle sind, (21) RAF- und BRAF-Kinasen-Inhibitoren, die kleine Moleküle sind, (22) Inhibitoren von zellzyklusabhängigen Kinasen, wie z.B. CDK1, CDK2, CDK4 und CDK6, die kleine Moleküle sind, (23) Alkylierungsmittel und (24) Farnesylproteintransferase-Inhibitoren.

68. Eine Verwendung einer Verbindung nach einem der Ansprüche 1 bis 63 oder eines pharmazeutisch annehmbaren Salzes davon zur Herstellung eines Medikaments zur Behandlung von Krebs, wobei das Medikament in einer Form zur Verabreichung mit wenigstens einem Signaltransduktionsinhibitor vorliegt.

69. Eine Verwendung wenigstens einer Verbindung nach einem der Ansprüche 1 bis 63 oder eines pharmazeutisch annehmbaren Salzes davon:
(A) zur Herstellung eines Medikaments zur Behandlung von Krebs, wobei der Krebs ausgewählt ist aus der Gruppe, bestehend aus: Lungenkrebs, Bauchspeicheldrüsenkrebs, Kolonkrebs, myeloischen Leukämien, Schilddrüsenkrebs, myelodysplastischem Syndrom, Blasenkrebs, Hautkrebs, Melanom, Brustkrebs, Prostatakrebs, Kopf- und Halskarzinomen, Ovarialkarzinom, Hirntumoren, Karzinomen mesenchymalen Ursprungs, Sarkomen, Tetrakarzinomen, Nuroblastomen, Nierenkarzinomen, Hepatomen, Nicht-Hodgkins-Lmyphom, multiplem Myelom und anaplastischem Schilddrüsenkarzinom,
(B) zur Herstellung eines Medikaments zur Behandlung von Krebs, wobei der Krebs ausgewählt ist aus der Gruppe, bestehend aus: Melanom, Bauchspeicheldrüsenkrebs, Schilddrüsenkrebs, Kolorektalkarzinom, Lungenkrebs, Brustkrebs und Ovarialkarzinom, oder
(C) zur Herstellung eines Medikaments zur Behandlung von Krebs, wobei das Medikament in einer Form zur Verabreichung in Kombination mit wenigstens einem chemotherapeutischen Mittel vorliegt, und wobei der Krebs ausgewählt ist aus der Gruppe, bestehend aus: Melanom, Bauchspeicheldrüsenkrebs, Schilddrüsenkrebs, Kolorektalkarzinom, Lungenkrebs, Brustkrebs und Ovarialkarzinom

70. Eine Verwendung wenigstens einer Verbindung nach einem der Ansprüche 1 bis 63 oder eines pharmazeutisch annehmbaren Salzes davon:
(A) zur Herstellung eines Medikaments zur Behandlung von Melanom,
(B) zur Herstellung eines Medikaments zur Behandlung von Melanom, wobei das Medikament in einer Form zur Verabreichung in Kombination mit wenigstens einem chemotherapeutischen Mittel vorliegt,
(C) zur Herstellung eines Medikaments zur Behandlung von Bauchspeicheldrüsenkrebs,
(D) zur Herstellung eines Medikaments zur Behandlung von Bauchspeicheldrüsenkrebs, wobei das Medikament in einer Form zur Verabreichung mit wenigstens einem chemotherapeutischen Mittel vorliegt,
(E) zur Herstellung eines Medikaments zur Behandlung von Schilddrüsenkrebs,
(F) zur Herstellung eines Medikaments zur Behandlung von Schilddrüsenkrebs, wobei das Medikament in einer Form zur Verabreichung in Kombination mit wenigstens einem chemotherapeutischen Mittel vorliegt,
(G) zur Herstellung eines Medikaments zur Behandlung von Kolorektalkarzinom,
(H) zur Herstellung eines Medikaments zur Behandlung von Kolorektalkarzinom, wobei das Medikament in einer Form zur Verabreichung in Kombination mit wenigstens einem chemotherapeutischen Mittel vorliegt,
(I) zur Herstellung eines Medikaments zur Behandlung von Lungenkrebs,
(J) zur Herstellung eines Medikaments zur Behandlung von Lungenkrebs, wobei das Medikament in einer Form zur Verabreichung in Kombination mit wenigstens einem chemotherapeutischen Mittel vorliegt,
(K) zur Herstellung eines Medikaments zur Behandlung von Brustkrebs,
(L) zur Herstellung eines Medikaments zur Behandlung von Brustkrebs, wobei das Medikament in einer Form zur Verabreichung in Kombination mit wenigstens einem chemotherapeutischen Mittel vorliegt.
(M) zur Herstellung eines Medikaments zur Behandlung von Ovarialkarzinom,
(N) zur Herstellung eines Medikaments zur Behandlung von Ovarialkarzinom, wobei das Medikament in einer Form zur Verabreichung in Kombination mit wenigstens einem chemotherapeutischen Mittel vorliegt,
(O) zur Herstellung eines Medikaments zur Behandlung von hormonabhängigem Brustkrebs, wobei das Medikament in einer Form zur Verabreichung in Kombination mit antihormonellen Mitteln vorliegt,
(P) zur Herstellung eines Medikaments zur Behandlung von hormonabhängigem Brustkrebs, wobei das Medikament in einer Form zur Verabreichung in Kombination mit antihormonellen Mitteln und in Kombination mit wenigstens einem chemotherapeutischen Mittel vorliegt,
(Q) zur Herstellung eines Medikaments zur Prävention von hormonabhängigem Brustkrebs, wobei das Medikament in einer Form zur Verabreichung in Kombination mit antihormonellen Mitteln vorliegt,
(R) zur Herstellung eines Medikaments zur Prävention von hormonabhängigem Brustkrebs, wobei das Medikament in einer Form zur Verabreichung in Kombination mit antihormonellen Mitteln und in Kombination mit wenigstens einem chemotherapeutischen Mittel vorliegt,
(S) zur Herstellung eines Medikaments zur Behandlung von Gehirntumor,
(T) zur Herstellung eines Medikaments zur Behandlung von Gehirntumor, wobei das Medikament in einer Form zur Verabreichung in Kombination mit wenigstens einem chemotherapeutischen Mittel vorliegt,
(U) zur Herstellung eines Medikaments zur Behandlung von Gehirntumor, wobei das Medikament in einer Form zur Verabreichung in Kombination mit wenigstens einem chemotherapeutischen Mittel vorliegt, wobei das chemotherapeutische Mittel Temozolomid ist,
(V) zur Herstellung eines Medikaments zur Behandlung von Prostatakrebs,
(W) zur Herstellung eines Medikaments zur Behandlung von Prostatakrebs, wobei das Medikament in einer Form zur Verabreichung in Kombination mit wenigstens einem chemotherapeutischen Mittel vorliegt,
(X) zur Herstellung eines Medikaments zur Behandlung von myelodysplastischem Syndrom,
(Y) zur Herstellung eines Medikaments zur Behandlung von myelodysplastischem Syndrom, wobei das Medikament in einer Form zur Verabreichung in Kombination mit wenigstens einem chemotherapeutischen Mittel vorliegt,
(Z) zur Herstellung eines Medikaments zur Behandlung von myeloischen Leukämien,
(AA) zur Herstellung eines Medikaments zur Behandlung von myeloischen Leukämien, wobei das Medikament in einer Form zur Verabreichung in Kombination mit wenigstens einem chemotherapeutischen Mittel vorliegt,
(AB) zur Herstellung eines Medikaments zur Behandlung von akuter myeloischer Leukämie, (AC) zur Herstellung eines Medikaments zur Behandlung von akuter myeloischer Leukämie, wobei das Medikament in einer Form zur Verabreichung in Kombination mit wenigstens einem chemotherapeutischen Mittel vorliegt,
(AD) zur Herstellung eines Medikaments zur Behandlung von chronischer myelomonozytischer Leukämie,
(AE) zur Herstellung eines Medikaments zur Behandlung von chronischer myelomonozytischer Leukämie, wobei das Medikament in einer Form zur Verabreichung in Kombination mit wenigstens einem chemotherapeutischen Mittel vorliegt,
(AF) zur Herstellung eines Medikaments zur Behandlung von chronischer myeloischer Leukämie, (AG) zur Herstellung eines Medikaments zur Behandlung von chronischer myeloischer Leukämie, wobei das Medikament in einer Form zur Verabreichung in Kombination mit wenigstens einem chemotherapeutischen Mittel vorliegt,
(AH) zur Herstellung eines Medikaments zur Behandlung von Blasenkrebs,
(AI) zur Herstellung eines Medikaments zur Behandlung von Blasenkrebs, wobei das Medikament in einer Form zur Verabreichung in Kombination mit wenigstens einem chemotherapeutischen Mittel vorliegt,
(AJ) zur Herstellung eines Medikaments zur Behandlung von Nicht-Hodgkins-Lymphom,
(AK) zur Herstellung eines Medikaments zur Behandlung von Nicht-Hodgkins-Lymphom, wobei das Medikament in einer Form zur Verabreichung in Kombination mit wenigstens einem chemotherapeutischen Mittel vorliegt,
(AL) zur Herstellung eines Medikaments zur Behandlung von multiplem Myelom oder
(AM) zur Herstellung eines Medikaments zur Behandlung von multiplem Myelom, wobei das Medikament in einer Form zur Verabreichung in Kombination mit wenigstens einem chemotherapeutischen Mittel vorliegt.

## Revendications

1. Composé de la formule 1.0: 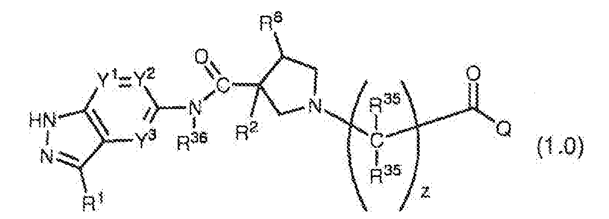 ou les sels pharmaceutiquement acceptables de celui-ci, où:
Y¹, Y² et Y³ sont sélectionnés indépendamment chacun parmi le groupe consistant en:
-CH=, -N= et -CR⁹=;
z est 1 à 3;
Q est un substituant sélectionné parmi le groupe consistant en: 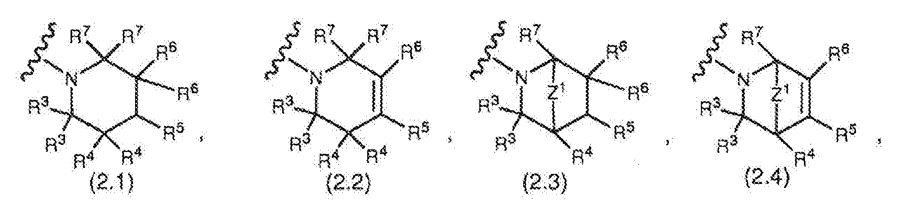 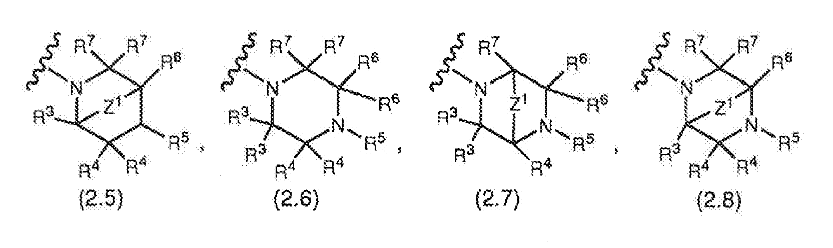 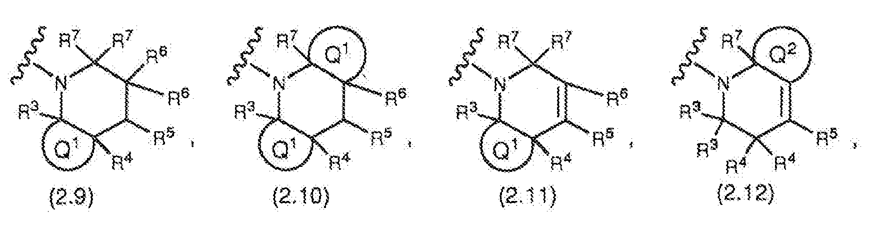 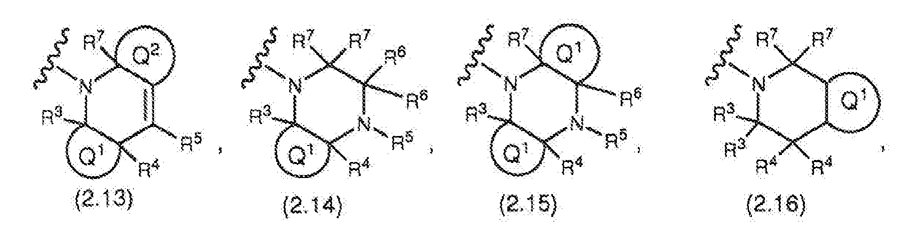 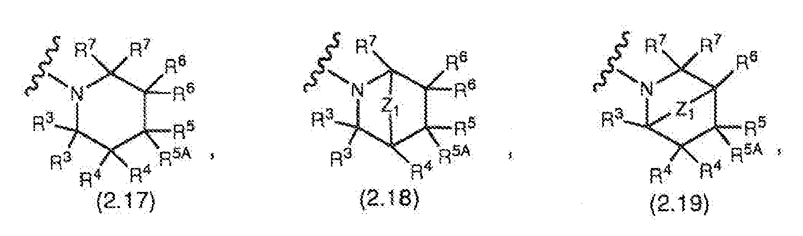 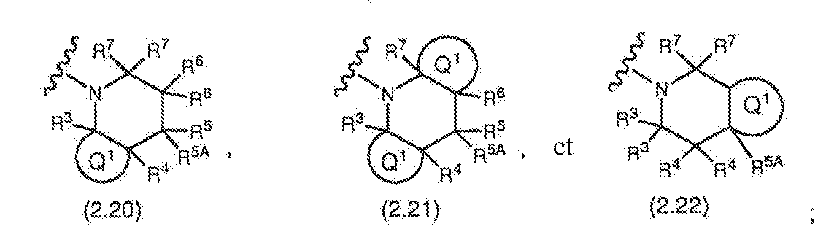
Chaque Q¹ représente un cycle sélectionné indépendamment parmi le groupe consistant en: cycloalkyle, cycloalkyle substitué, hétérocycloalkyle, hétérocycloalkyle substitué, aryle, aryle substitué, hétéroaryle et hétéroaryle substitué, où lesdits cycles substitués sont substitués par 1 à 3 substituants sélectionnés indépendamment parmi le groupe consistant en: les fractions R¹⁰; à condition que lorsque Q¹ est aryle, hétéroaryle, aryle substitué ou hétéroaryle substitué, les atomes de carbone à la jonction du cycle ne sont alors pas substitués;
Q² représente un cycle sélectionné parmi le groupe consistant en: cycloalkyle, cycloalkyle substitué, hétérocycloalkyle et hétérocycloalkyle substitué, où lesdits cycles substitués sont substitués par 1 à 3 substituants sélectionnés indépendamment parmi le groupe consistant en: les fractions R¹⁰;
Z¹ représente -(C(R²⁴)₂)_{w} où chaque R²⁴ est sélectionné indépendamment parmi le groupe consistant en: H, alkyle et F et où w est 1, 2 ou 3;
Z² est sélectionné parmi le groupe consistant en: -N(R⁴⁴)-, -O- et -C(R⁴⁶)₂-;
m est 1 à 6;
n est 1 à 6;
p est 0 à 6;
t est 0, 1 ou 2;
R¹ est sélectionné parmi le groupe consistant en:
(1) -CN,
(2) -NO₂;
(3) -OR¹⁰,
(4) -SR¹⁰,
(5) -N(R¹⁰)₂;
(6) R¹⁰,
(7) -C(O)R¹⁰,
(8) -(C(R³⁰)₂)ₙ-NR³²-C(O)-R¹⁰,
(9) -(C(R³⁰)₂)ₙ-NR³²-S(O)ₜR¹⁰,
(10) -(C(R³⁰)₂)ₙ-NR³²-C(O)-N(R³²)-R¹⁰,
(11) 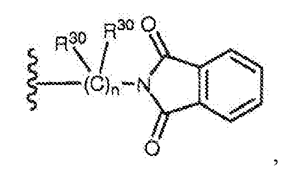
(12) -CF₃,
(13) -C(O)OR¹⁰,
(14) -(C(R³⁰)₂)ₙR¹³ (par ex. -(CH₂)ₙR¹³) où n est 1, chaque R³⁰ est H et R¹³ est sélectionné parmi le groupe consistant en: -OH et -N(R¹⁰)₂ où chaque R¹⁰ est sélectionné indépendamment,
(15) alcényle,
(16) -NR¹²-C(O)-R¹⁴,
(17) 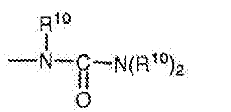 où chaque R¹⁰ est sélectionné indépendamment,
(18) 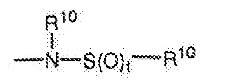 où chaque R¹⁰ est sélectionné indépendamment
(19) 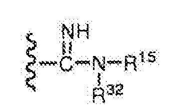
(20) -C(O)-NR³²-(C(R³⁰)₂)ₚ-OR¹⁰,
(21) -C(O)N(R¹⁰)₂ où chaque R¹⁰ est sélectionné indépendamment,
(22) -C(O)-NR³²-C(R¹⁸)₃ où chaque R¹⁸ est sélectionné indépendamment parmi le groupe consistant en: R¹⁰ et -C(O)OR¹⁹ et R¹⁹ est sélectionné parmi le groupe consistant en: alkyle et arylalkyle substitué,
(23) -C(O)-NR³²-(C(R³⁰)₂)ₙ-C(O)-N(R¹⁰)₂,
(24) hétérocycloalcényle,
(25) 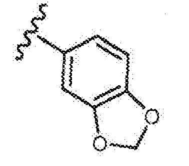 et
(26) arylalcényle-,
R² est sélectionné parmi le groupe consistant en:
(1) H,
(2) -CN,
(3) halo,
(4) alkyle,
(5) alkyle substitué où ledit alkyle substitué est substitué par 1 à 3 substituants sélectionnés parmi le groupe consistant en: (a) -OH, (b), -O-alkyle (par ex. -O-(alkyle C₁-C₃), (c) -O-alkyle substitué par 1 à 3 atomes F et (d) -N(R⁴⁰)₂ où chaque R⁴⁰ est sélectionné indépendamment parmi le groupe consistant en: (i) H, (ii) alkyle C₁-C₃, (iii)-CF₃ et (e) halo,
(6) alcynyle,
(7) alcényle,
(8) -(CH₂)ₘR¹¹,
(9) -N(R²⁶)₂,
(10) -OR²³,
(11) -N(R²⁶)C(O)R⁴²,
(12) cycloalkyle,
(13) cycloalkylalkyle,
(14) 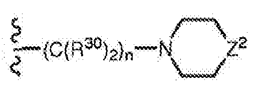
(15) -O-(alkyle substitué) où ledit alkyle substitué est substitué par 1 à 3 atomes F,
(16) -S(O)ₜ-alkyle,
(17) -C(O)-alkyle,
(18) 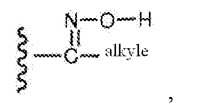
(19) 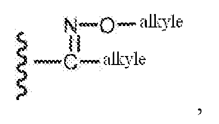 où chaque alkyle est sélectionné indépendamment,
(20) 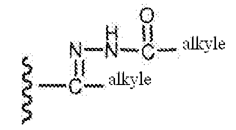 dont chaque alkyle est sélectionné indépendamment,
(21) 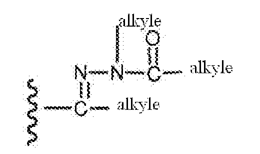 où chaque alkyle est sélectionné indépendamment,
(22) -N(R⁴⁸)-C(O)-R⁴⁸ où chaque R⁴⁸ est sélectionné indépendamment parmi le groupe consistant en: H et alkyle, et
(23) -C(O)-alkyle tel que, par exemple, -C(O)-(alkyle C₁-C₆) tel que, par exemple, -C(O)CH₃;
chaque R³, R⁴, R⁵, R⁶ et R⁷ est sélectionné indépendamment parmi le groupe consistant en:
(1) H,
(2) alcényle,
(3) alcényle substitué,
(4) alkyle,
(5) alkyle substitué,
(6) cycloalkyle,
(7) cycloalkyle substitué,
(8) cycloalkylalkyle-,
(9) cycloalkylalkyle- substitué,
(10) hétérocycloalkyle,
(11) hétérocycloalkyle substitué,
(12) hétérocycloalkylalkyle-,
(13) hétérocycloalkylalkyle- substitué,
(14) -C(O)R¹⁰,
(15) arylhétéroaryle-,
(16) arylhétéroaryle- substitué,
(17) hétéroarylaryle-,
(18) hétéroarylaryle- substitué,
(19) aryle,
(20) aryle substitué,
(21) hétéroaryle,
(22) hétéroaryle substitué,
(23) hétéroarylhétéroaryle-,
(24) hétéroarylhétéroaryle- substitué,
(25) arylaminohétéroaryle-,
(26) arylaminohétéroaryle- substitué,
(27) arylalcynyle-,
(28) arylalcynyle- substitué,
(29) hétéroarylalcynyle-,
(30) hétéroarylalcynyle- substitué,
où lesdits groupes R³, R⁴, R⁵, R⁶ et R⁷ substitués (7), (9), (11), (13), (16), (18), (20), (22), (24), (26), (28) et (30), sont substitués par 1 à 3 substituants sélectionnés indépendamment parmi le groupe consistant en: -NH₂, alkyle, alcényle, halo, -C(O)-NH-R²⁸, -C(O)OR²¹ et -C(O)R²¹, et
où lesdits groupes R³, R⁴, R⁵, R⁶ et R⁷ substitués (3) et (5), sont substitués par 1 à 3 substituants sélectionnés indépendamment parmi le groupe consistant en: -NH₂, halo (par ex. F, Cl et Br et dans un autre exemple F), -C(O)-NH-R²⁸ (par ex. -C(O)-NH-CH₃), -C(O)OR²⁸ (par ex. -C(O)OC₂H₅) et -C(O)R²⁸ (par ex. -C(O)CH₃);
R^{5A} est sélectionné parmi le groupe consistant en: halo, -OH et -O-alkyle;
R⁸ est sélectionné parmi le groupe consistant en: H, -OH, -N(R¹⁰)₂, -NR¹⁰C(O)R¹² et alkyle;
chaque R⁹ est sélectionné indépendamment parmi le groupe consistant en: halogène, -CN, -NO₂, -OR¹⁰, -SR¹⁰, -N(R¹⁰)₂ et R¹⁰;
chaque R¹⁰ est sélectionné indépendamment parmi le groupe consistant en: H, alkyle, aryle, arylalkyle, hétéroaryle, hétéroarylalkyle, cycloalkyle, cycloalkylalkyle, hétérocycloalkyle, hétérocycloalkylalkyle, alkylhétéroaryle-, alkylaryle-, alkyle substitué, aryle substitué, arylalkyle substitué, hétéroaryle substitué, hétéroarylalkyle substitué, cycloalkyle substitué, cycloalkylalkyle substitué, hétérocycloalkyle substitué, hétérocycloalkylalkyle substitué, alkylhétéroalkyle- substitué, alkylaryle substitué, hétérocycloalcényle et hétérocycloalcényle substitué, et où:
ledit alkyle substitué de R¹⁰ est substitué par 1 à 3 substituants sélectionnés indépendamment parmi le groupe consistant en: -NH₂, -NHR²⁰, -NPO₂, -CN, -OR²⁶, halo, - C(O)-NH-R²⁶, -C(O)OR²⁶ et -C(O)R²⁶, et
lesdits aryle substitué, arylalkyle substitué, hétéroaryle substitué, hétéroarylalkyle substitué, cycloalkyle substitué, cycloalkylalkyle substitué, hétérocycloalkyle substitué, hétérocycloalkylalkyle substitué, alkylhétéroaryle- substitué et alkylaryle- substitué de R¹⁰ sont substitués par 1 à 3 substituants sélectionnés indépendamment parmi le groupe consistant en: (1) -NH₂, (2) -NO₂, (3) -CN, (4) -OH, (5) -OR²⁰, (6) -OCF₃, (7) alkyle substitué par 1 à 3 atomes halo sélectionnés indépendamment, (8) -C(O)R³⁸, (9) alkyle, (10) alcényle, (11) halo, (12) -(O)-NH-R²⁶, (13) -C(O)OR³⁸, (14) -C(O)-NR³²-(C(R³²)₂)ₙ-N(R³⁸)₂, (15) -S(O)₁R³⁸, (16) -C(O)-NR³²-R³⁸, (17) -NR³²-C(O)-R³⁸,
(18) 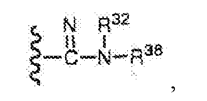 (19) -NHR²⁰ et (20) cycloalkyle;
R¹¹ est sélectionné parmi le groupe consistant en: F, -OH, -CN, -OR¹⁰, - NHNR¹R¹⁰, -SR¹⁰ et hétéroaryle;
R¹² est sélectionné parmi le groupe consistant en: alkyle, aryle, hétéroaryle, cycloalkyle, cycloalkylalkyle, hétérocycloalkyle et hétérocycloalkylalkyle;
R¹⁴ est sélectionné parmi le groupe consistant en: alkyle, aryle, hétéroaryle, cycloalkyle, cycloalkylalkyle-, hétérocycloalkyle, alkylhétérocycloalkyle, hétérocycloalkylalkyle-, alkylhétéroaryle- et alkylaryle-;
R¹⁵ est sélectionné parmi le groupe consistant en: H, -OH, alkyle, aryle, hétéroaryle, cycloalkyle, cycloalkylalkyle-, hétérocycloalkyle et hétérocycloalkylalkyle-, alkylhétéroaryle- et alkylaryle-;
R²⁰ représente alkyle;
R²³ est sélectionné parmi le groupe consistant en: H, alkyle, aryle, cycloalkyle et cycloalkylalkyle-;
chaque R²⁶ est sélectionné indépendamment parmi le groupe consistant en: H et alkyle; R²⁸ est alkyle;
chaque R³⁰ est sélectionné indépendamment parmi le groupe consistant en: H, alkyle et F;
chaque R³² est sélectionné indépendamment parmi le groupe consistant en: H et alkyle et où chaque R³² est généralement H;
chaque R³⁵ est sélectionné indépendamment parmi le groupe consistant en: H et alkyle en C₁ à C₆;
R³⁶ est sélectionné parmi le groupe consistant en: H, alkyle et -O-alkyle;
chaque R³⁸ est sélectionné indépendamment parmi le groupe consistant en: H, alkyle, aryle, arylalkyle, hétéroaryle, hétéroarylalkyle, cycloalkyle, cycloalkylalkyle, hétérocycloalkyle, hétérocycloalkylalkyle, alkylhétéroaryle-, alkylaryle-, alkyle substitué, aryle substitué, arylalkyle substitué, hétéroaryle substitué, hétéroarylalkyle substitué, cycloalkyle substitué, cycloalkylalkyle substitué, hétérocycloalkyle substitué, hétérocycloalkylalkyle substitué, alkylhétéroaryle- substitué et alkylaryle- substitué et où:
ledit alkyle substitué de R³⁸ est substitué par 1 à 3 substituants sélectionnés indépendamment parmi le groupe consistant en: -NH₂, -NO₂, -CN, -OR²⁶, halo, -C(O)-NH-R²⁸, -C(O)OR²⁸ et -C(O)R²⁸, et
lesdits aryle substitué, arylalkyle substitué, hétéroaryle substitué, hétéroarylalkyle substitué, cycloalkyle substitué, cycloalkylalkyle substitué, hétérocycloalkyle substitué, hétérocycloalkylalkyle substitué, alkylhétéroaryle- substitué et alkylaryle- substitué de R³⁸ sont substitués par 1 à 3 substituants sélectionnés indépendamment parmi le groupe consistant en: (1) -NH₂, (2) -NO₂, (3) -CN, (4) -OH, (5) -OR²⁰, (6) -OCF₃, (7) -CF₃, (8) -C(O)R²⁶, (9) alkyle, (10) alcényle, (11) halo, (12) -C(O)-NH-R²⁶, (13) -C(O)OR²⁶, (14) -C(O)-NR³²-(C(R³⁰)₂)ₙ-N(R²⁶)₂, (15) -S(O)₁R²⁶, (16) -(O)N(R³²)(R²⁶), (17)-NR³²C(O)R²⁶, (18) 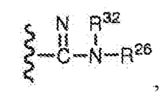 et
(19)-NHR²⁰;
R⁴² est sélectionné parmi le groupe consistant en: alkyle, aryle, hétéroaryle et cycloalkyle;
R⁴⁴ est sélectionné parmi le groupe consistant en: H, alkyle, cycloalkyle et cycloalkylalkyle; et
chaque R⁴⁶ est sélectionné indépendamment parmi le groupe consistant en: H, alkyle, cycloalkyle et cycloalkylalkyle.

2. Composé selon la revendication 1 ayant la formule sélectionnée parmi le groupe consistant en: 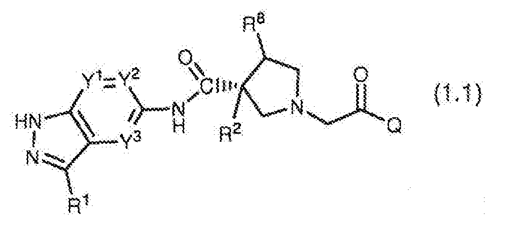 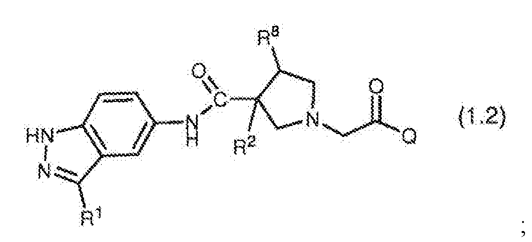 et 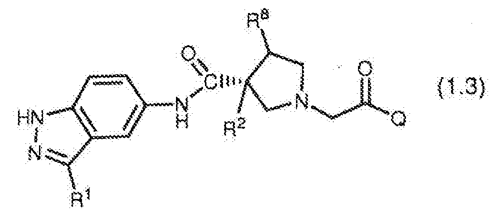 ou un sel pharmaceutiquement acceptable de celui-ci.

3. Composé selon l'une quelconque des revendications 1 ou 2 ou un sel pharmaceutiquement acceptable de celui-ci, dans lequel Q est sélectionné parmi le groupe consistant en: 2.1, 2.2, 2.3, 2.4, 2.5, 2.6, 2.7 et 2.8.

4. Composé selon l'une quelconque des revendications 1 ou 2 ou un sel pharmaceutiquement acceptable de celui-ci, dans lequel Q est sélectionné parmi le groupe consistant en: 2.17, 2.18, 2.19. 2.20. 2.21 et 2.22.

5. Composé selon la revendication 1 ou un sel pharmaceutiquement acceptable de celui-ci, dans lequel Z¹ est -CH₂-.

6. Composé selon la revendication 3 ou un sel pharmaceutiquement acceptable de celui-ci, dans lequel Q est sélectionné parmi le groupe consistant en: fractions 2.1, 2.2 et 2.3.

7. Composé selon la revendication 6 ou un sel pharmaceutiquement acceptable de celui-ci, dans lequel Q est sélectionné parmi le groupe consistant en: fractions 2.1, 2.2 et 2.3 et 2.3 est sélectionné parmi le groupe consistant en:

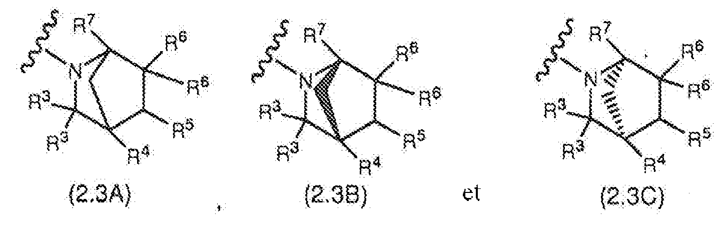

8. Composé selon l'une quelconque des revendications 1 ou 7 ou un sel pharmaceutiquement acceptable de celui-ci, dans lequel chaque R³, R⁴, R⁶ et R⁷ est sélectionné indépendamment parmi le groupe consistant en: H et alkyle.

9. Composé selon la revendication 8 ou un sel pharmaceutiquement acceptable de celui-ci, dans lequel chaque R³, R⁴, R⁶ et R⁷ est sélectionné indépendamment parmi le groupe consistant en: H et méthyle.

10. Composé selon la revendication 9 ou un sel pharmaceutiquement acceptable de celui-ci, dans lequel chaque R³, R⁴, R⁶ et R⁷ est H.

11. Composé selon la revendication 3 ou un sel pharmaceutiquement acceptable de celui-ci, dans lequel Q est sélectionné parmi le groupe consistant en: fractions 2.6 et 2.7.

12. Composé selon la revendication 11 ou un sel pharmaceutiquement acceptable de celui-ci, dans lequel Q est sélectionné parmi le groupe consistant en: fractions 2.6 et 2.7 et 2.7 est sélectionné parmi le groupe consistant en:

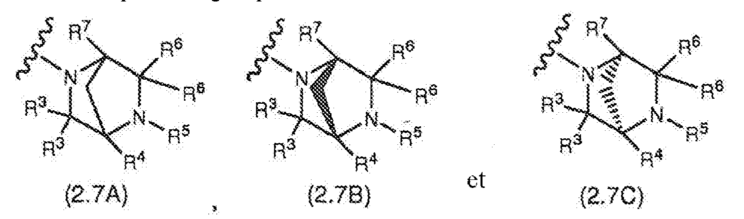

13. Composé selon l'une quelconque des revendications 1 ou 12 ou un sel pharmaceutiquement acceptable de celui-ci, dans lequel chaque R³, R⁴, R⁶ et R⁷ est sélectionné indépendamment parmi le groupe consistant en: H et alkyle.

14. Composé selon la revendication 13 ou un sel pharmaceutiquement acceptable de celui-ci, dans lequel chaque R³, R⁴, R⁶ et R⁷ est sélectionné indépendamment parmi le groupe consistant en: H et méthyle.

15. Composé selon la revendication 14 ou un sel pharmaceutiquement acceptable de celui-ci, dans lequel chaque R³, R⁴, R⁶ et R⁷ est H.

16. Composé selon la revendication 10 ou un sel pharmaceutiquement acceptable de celui-ci, dans lequel Q est 2.1.

17. Composé selon la revendication 10 ou un sel pharmaceutiquement acceptable de celui-ci, dans lequel Q est 2.3B.

18. Composé selon la revendication 15 ou un sel pharmaceutiquement acceptable de celui-ci, dans lequel Q est 2.6.

19. Composé selon la revendication 15 ou un sel pharmaceutiquement acceptable de celui-ci, dans lequel Q est 2.7A.

20. Composé selon la revendication 15 ou un sel pharmaceutiquement acceptable de celui-ci, dans lequel Q est 2.7B.

21. Composé selon la revendication 1 ou un sel pharmaceutiquement acceptable de celui-ci, dans lequel Q est 2.17.

22. Composé selon la revendication 21 ou un sel pharmaceutiquement acceptable de celui-ci, dans lequel chaque R³, R⁴, R⁶ et R⁷ est sélectionné indépendamment parmi le groupe consistant en: H et méthyle.

23. Composé selon la revendication 22 ou un sel pharmaceutiquement acceptable de celui-ci, dans lequel chaque R³, R⁴, R⁶ et R⁷ est H.

24. Composé selon l'une quelconque des revendications 16, 17, 18, 19 ou 20 ou un sel pharmaceutiquement acceptable de celui-ci, dans lequel le composé de la formule 1.0 est:

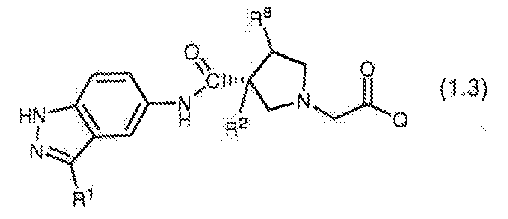

25. Composé selon la revendication 2 ou un sel pharmaceutiquement acceptable de celui-ci, dans lequel le composé de la formule 1.0 est un composé de la formule 1.3 et Q est sélectionné parmi le groupe consistant en: 2.1, 2.2 et 2.3 et 2.3 est sélectionné parmi le groupe consistant en: 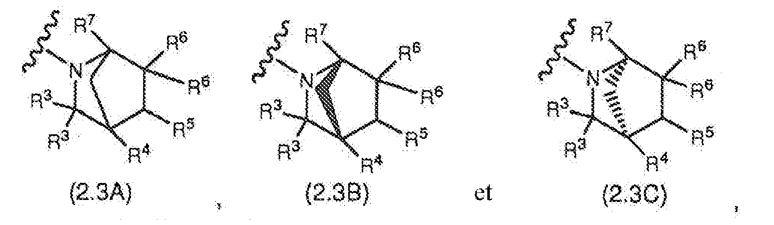 et R³, R⁴, R⁶ et R⁷ sont sélectionnés chacun indépendamment parmi le groupe consistant en H et alkyle.

26. Composé selon la revendication 25 ou un sel pharmaceutiquement acceptable de celui-ci, dans lequel R³, R⁴, R⁶ et R⁷ sont sélectionnés parmi le groupe consistant en: H et méthyle.

27. Composé selon la revendication 26 ou un sel pharmaceutiquement acceptable de celui-ci, dans lequel Q est sélectionné parmi le groupe consistant en: 2.1 et 2.3B.

28. Composé selon la revendication 27 ou un sel pharmaceutiquement acceptable de celui-ci, dans lequel Q est 2.1 et R³, R⁴, R⁶ et R⁷ sont chacun H.

29. Composé selon la revendication 2 ou un sel pharmaceutiquement acceptable de celui-ci, dans lequel le composé de la formule 1.0 est un composé de la formule 1.3 et Q est sélectionné parmi le groupe consistant en: 2.6 et 2.7 et 2.7 est sélectionné parmi le groupe consistant en: 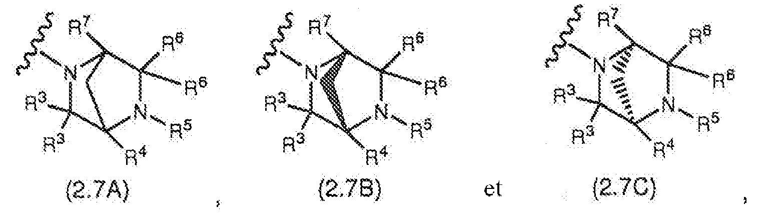 et R³, R⁴, R⁶ et R⁷ sont sélectionnés chacun indépendamment parmi le groupe consistant en: H et alkyle.

30. Composé selon la revendication 29 ou un sel pharmaceutiquement acceptable de celui-ci, dans lequel R³, R⁴, R⁶ et R⁷ sont sélectionnés parmi le groupe consistant en: H et méthyle.

31. Composé selon la revendication 30 ou un sel pharmaceutiquement acceptable de celui-ci, dans lequel Q est sélectionné parmi le groupe consistant en: 2.6, 2.7A et 2.7B.

32. Composé selon la revendication 31 ou un sel pharmaceutiquement acceptable de celui-ci, dans lequel Q est 2.6 et R³, R⁴, R⁶ et R⁷ sont chacun H.

33. Composé selon la revendication 31 ou un sel pharmaceutiquement acceptable de celui-ci, dans lequel Q est 2.7A et R³, R⁴, R⁶ et R⁷ sont chacun H.

34. Composé selon la revendication 31 ou un sel pharmaceutiquement acceptable de celui-ci, dans lequel Q est 2.7B et R³, R⁴, R⁶ et R⁷ sont chacun H.

35. Composé selon la revendication 1 ou un sel pharmaceutiquement acceptable de celui-ci, dans lequel Q est sélectionné parmi le groupe consistant en:
(A) 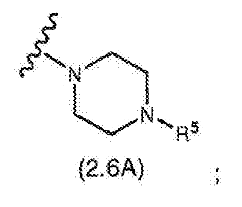
(B) 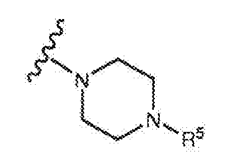 substitué par un ou deux substituants sélectionnés indépendamment parmi le groupe consistant en groupes R³, à condition que lesdits un ou deux substituants ne soient pas H;
(C) 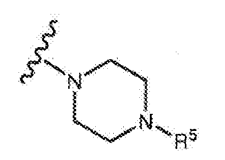 substitué par un ou deux groupes alkyle;
(D) 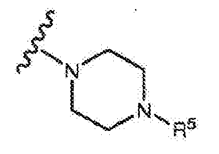 substitué par un ou deux groupes méthyle;
(E) 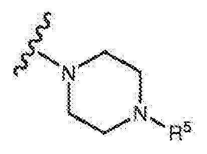 substitué par un groupe méthyle; et (F)

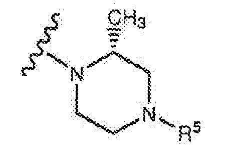

36. Composé selon la revendication 1 ou un sel pharmaceutiquement acceptable de celui-ci, dans lequel Q est sélectionné parmi le groupe consistant en:
(A) 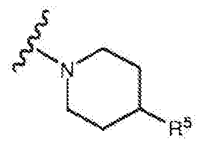 substitué par un ou deux substituants sélectionnés indépendamment parmi le groupe consistant en groupes R³, à condition que lesdits un ou deux substituants ne soient pas H;
(B) 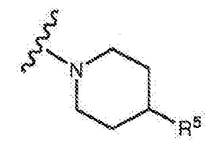 substitué par un ou deux groupes alkyle;
(C) 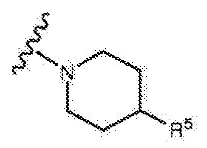 substitué par un ou deux groupes méthyle; et
(D) 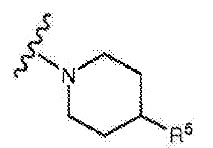 substitué par un groupe méthyle.

37. Composé selon l'une quelconque des revendications 1 ou 2 ou un sel pharmaceutiquement acceptable de celui-ci, dans lequel R¹ est sélectionné parmi le groupe consistant en:

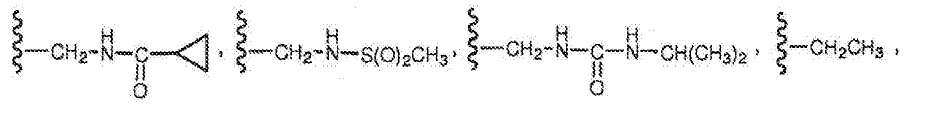

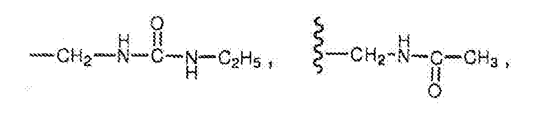

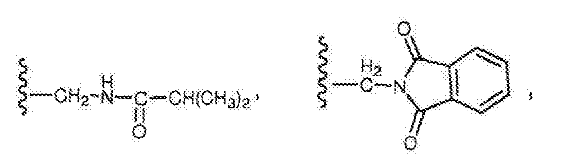

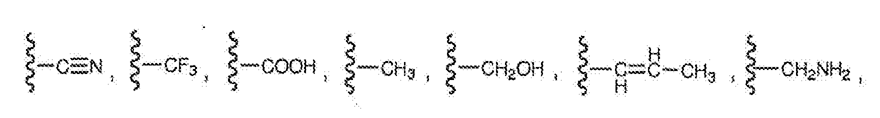

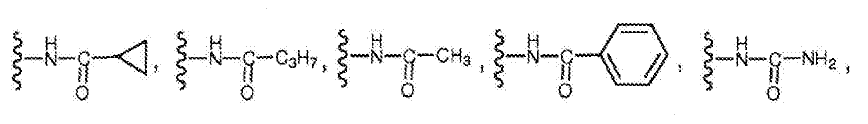

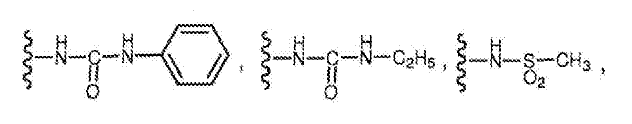

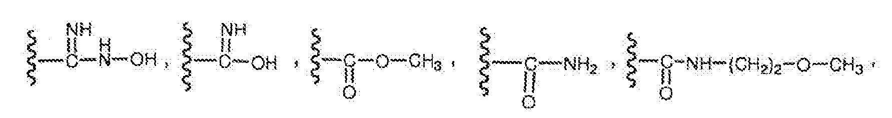

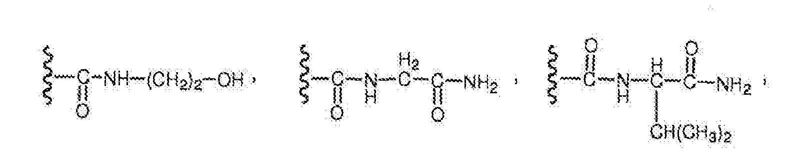

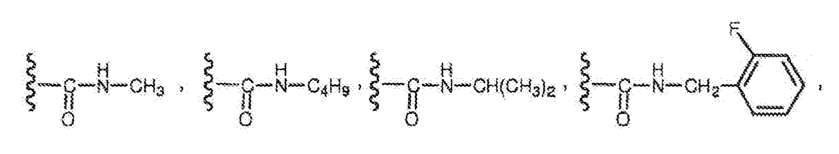

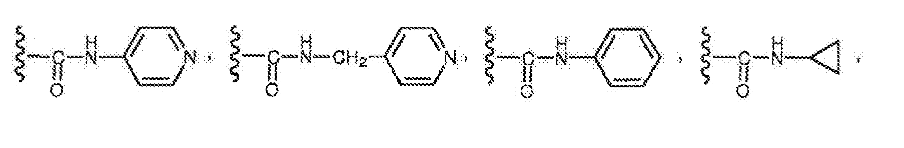

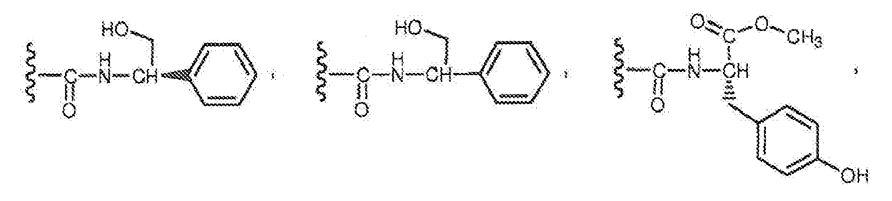

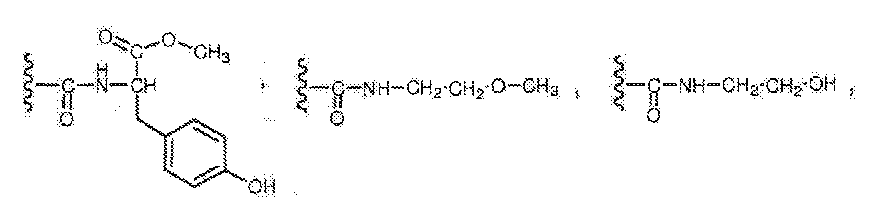

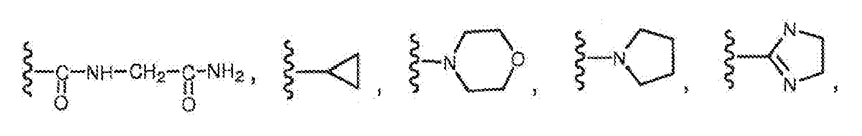

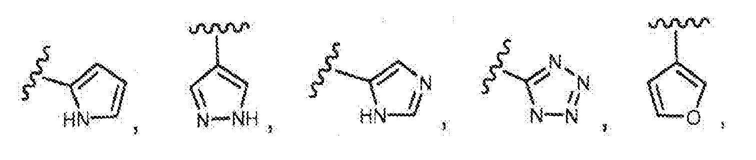

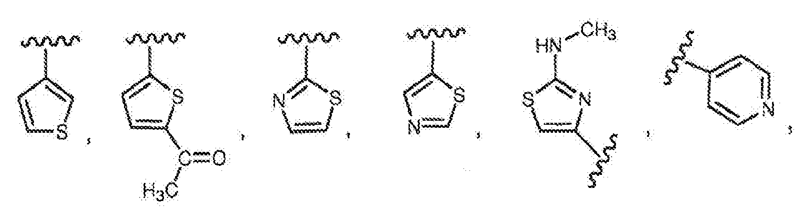

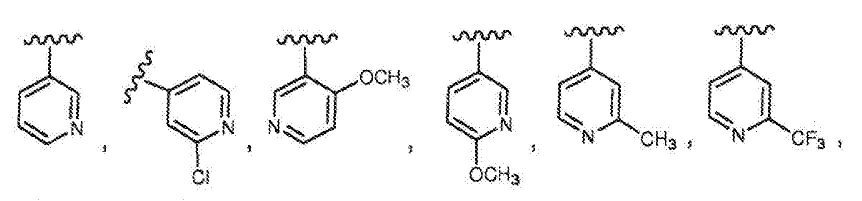

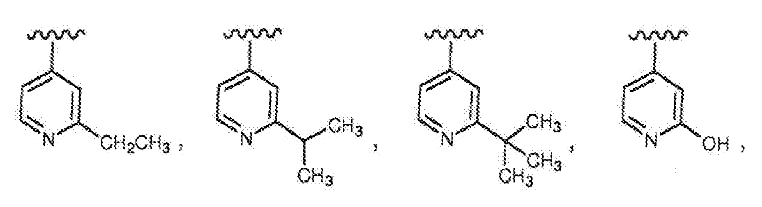

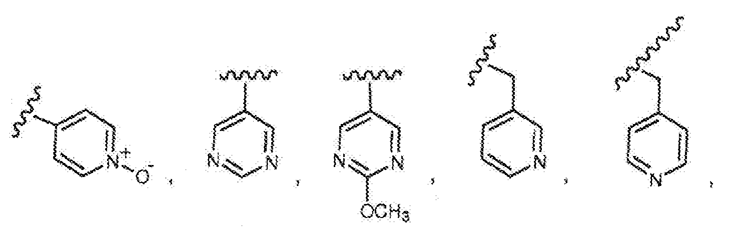

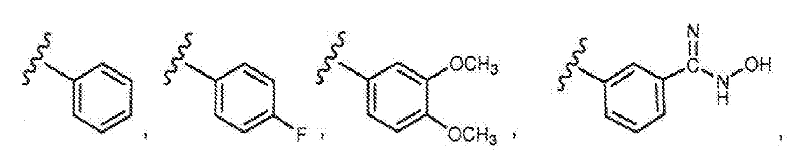

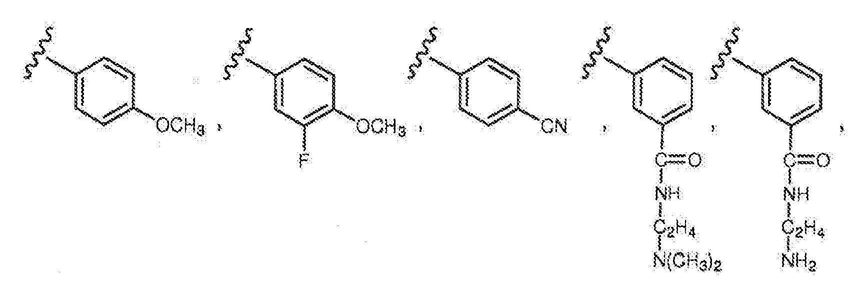

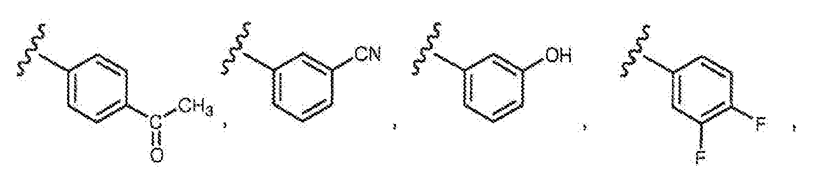

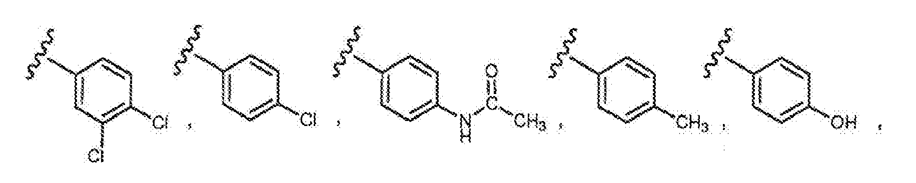

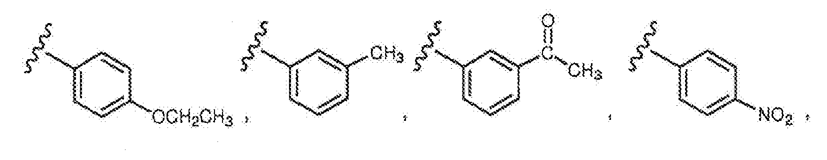

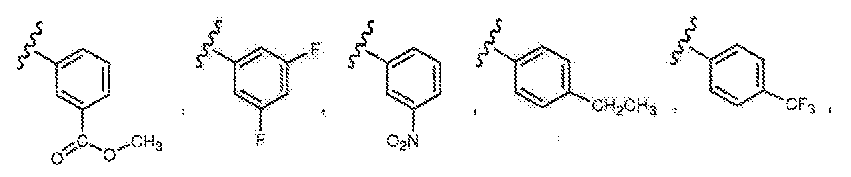

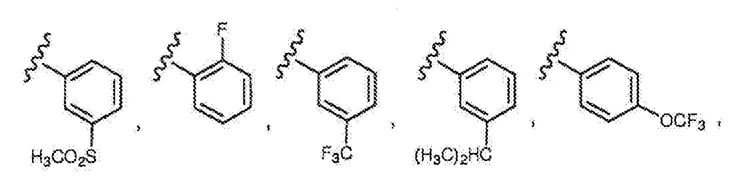

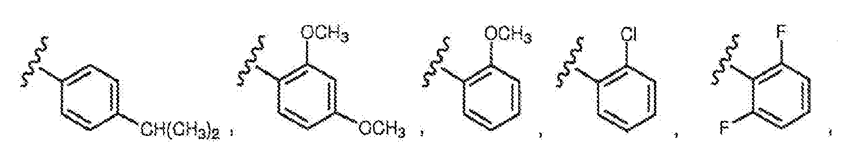

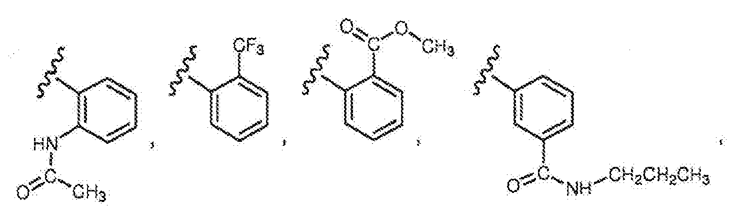

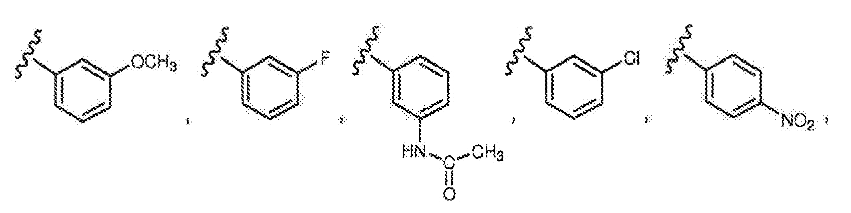

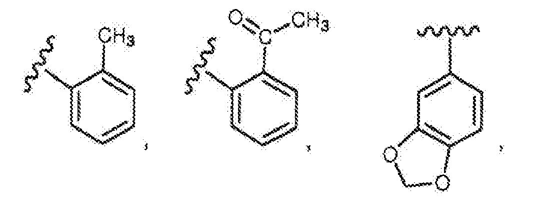

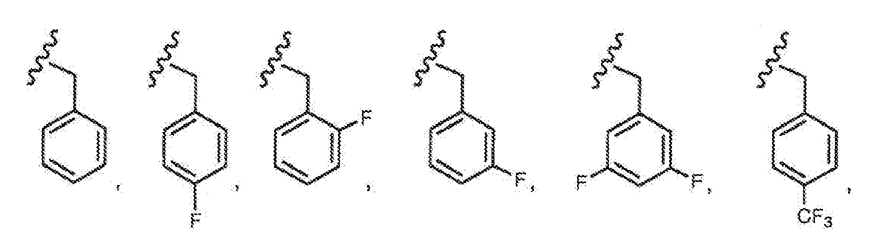

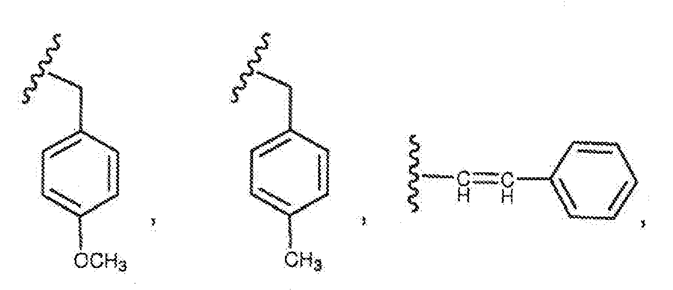

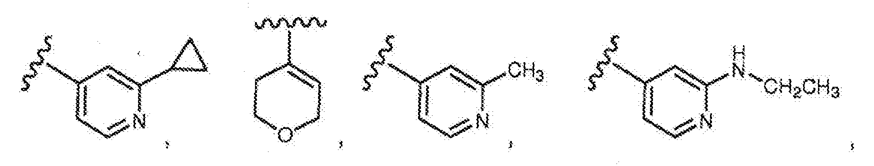

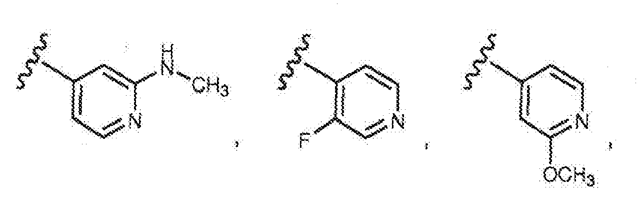

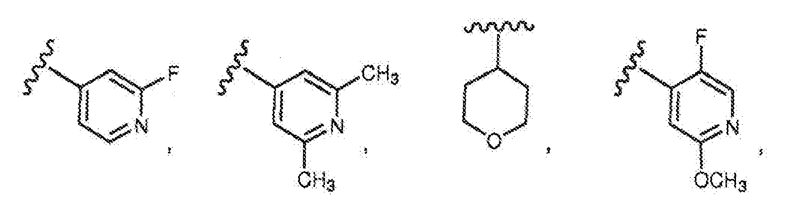

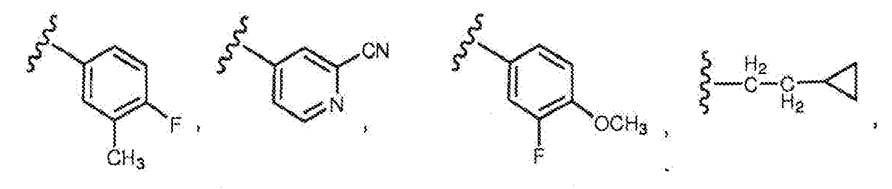

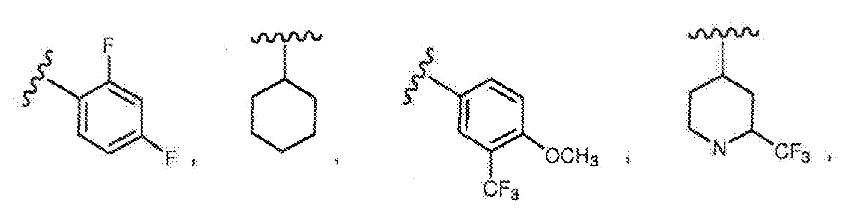

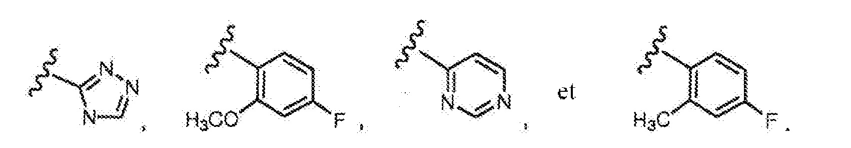

38. Composé selon la revendication 1 ou un sel pharmaceutiquement acceptable de celui-ci, dans lequel R¹ est aryle.

39. Composé selon la revendication 1 ou un sel pharmaceutiquement acceptable de celui-ci, dans lequel R¹ est hétéroaryle ou hétéroaryle substitué où ledit hétéroaryle substitué est substitué par 1 à 3 substituants sélectionnés indépendamment parmi le groupe consistant en: (1) -NH₂, (2) -NO₂, (3) -CN, (4) -OH, (5) -OR²⁰, (6) -OCF₃, (7) alkyle substitué par 1 à 3 atomes halo sélectionnés indépendamment, (8) -C(O)R³⁸, (9) alkyle, (10) alcényle, (11) halo, (12) -C(O)-NH-R²⁶, (13) -C(O)OR³⁸, (14) -C(O)-NR³²-(C(R³⁰)₂)ₙ-N(R³⁸)₂, (15) -S(O)₁R³⁸, (16) -C(O)-NR³²-R³⁸, (17) -NR³²-CO)-R³⁸, (18) 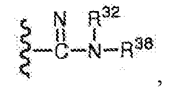 (19) -NHR²⁰ et (20) cycloalkyle.

40. Composé selon l'une quelconque des revendications 1 ou 2 ou un sel pharmaceutiquement acceptable de celui-ci, dans lequel R⁵ est sélectionné parmi le groupe consistant en:

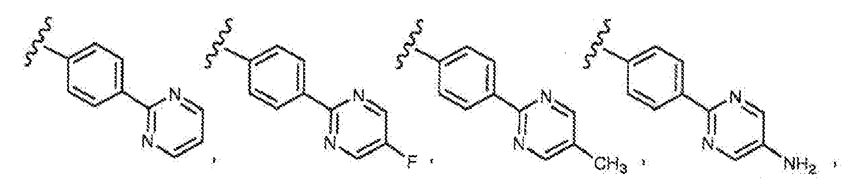

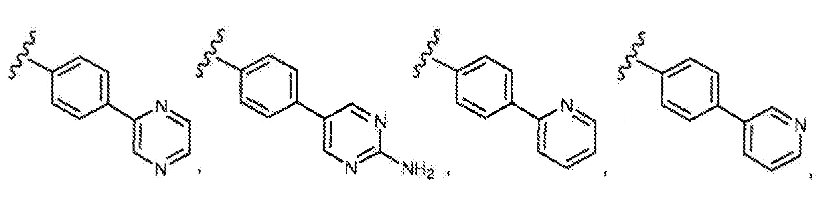

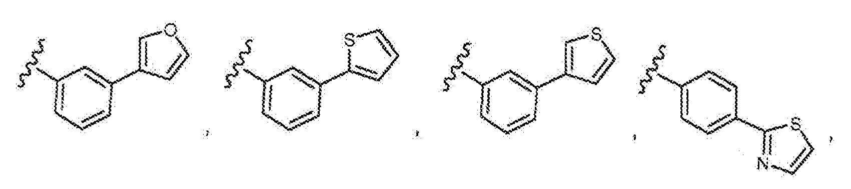

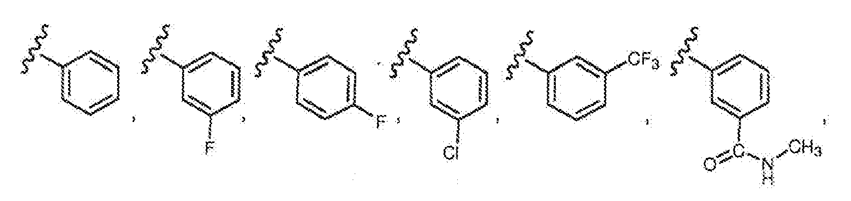

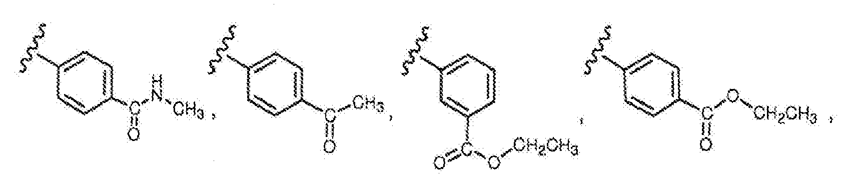

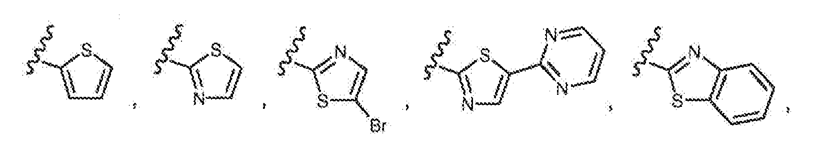

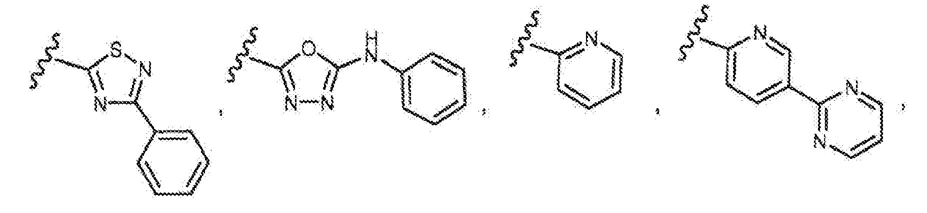

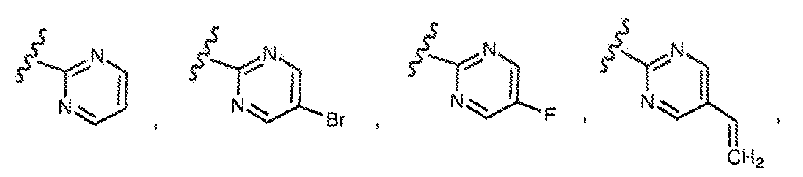

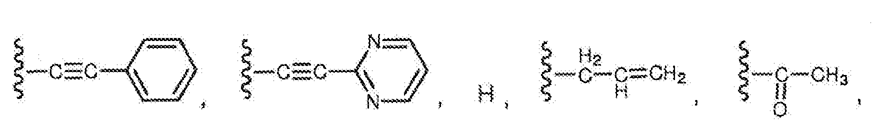

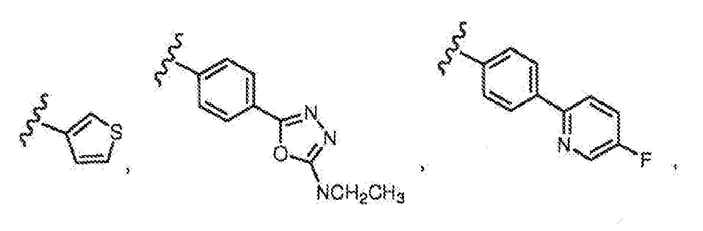

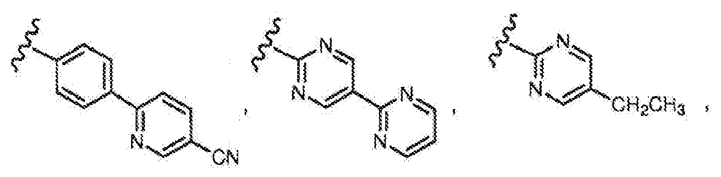

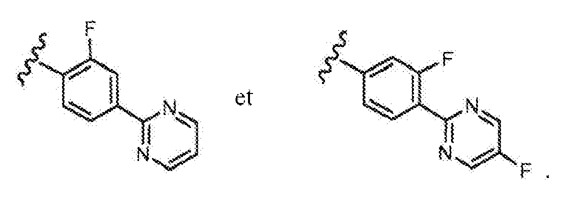

41. Composé selon l'une quelconque des revendications 1 ou 7 ou un sel pharmaceutiquement acceptable de celui-ci, dans lequel R¹ est sélectionné parmi le groupe consistant en: 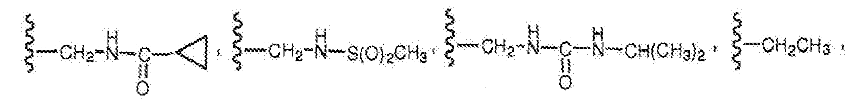 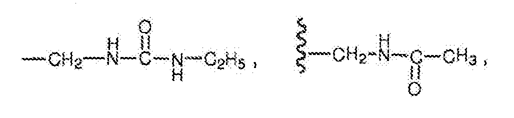 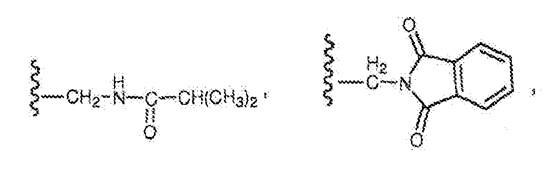 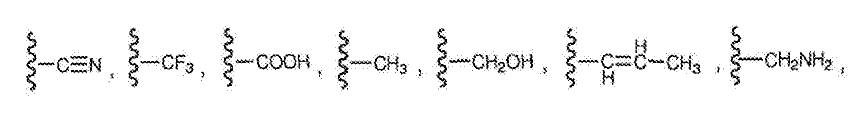 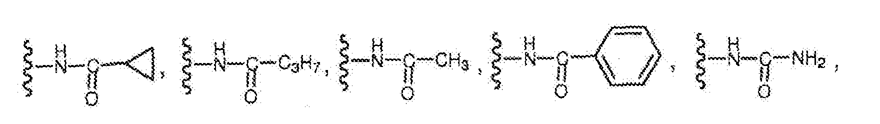 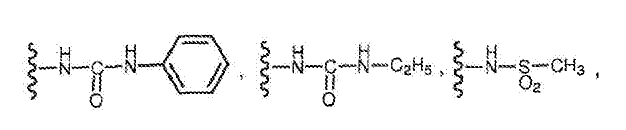 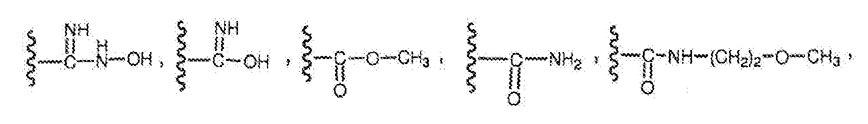 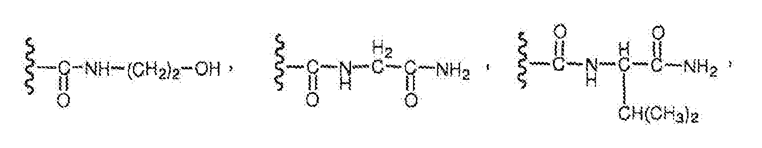 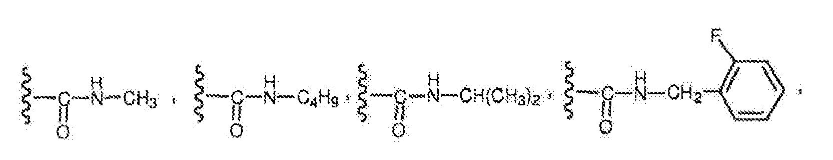 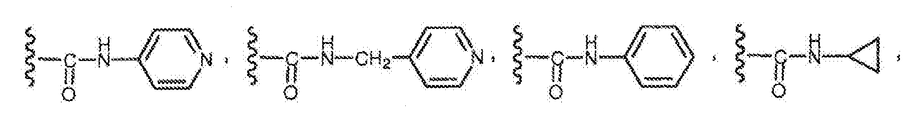 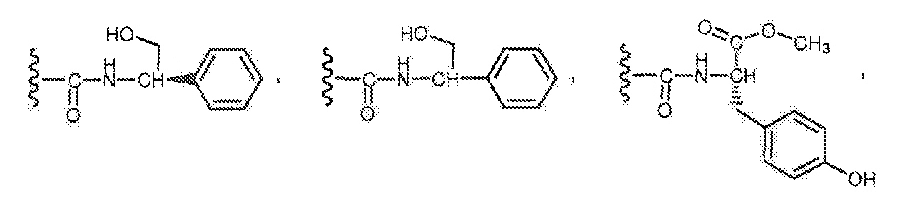 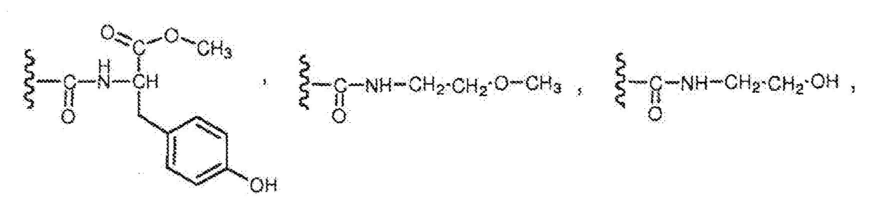 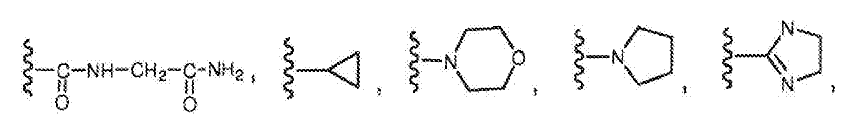 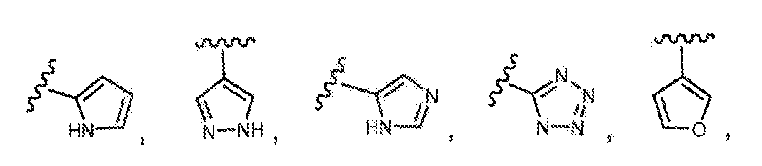 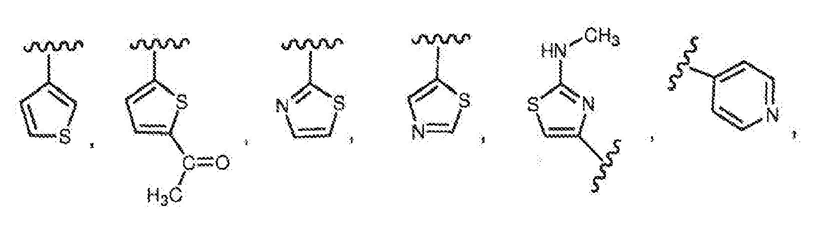 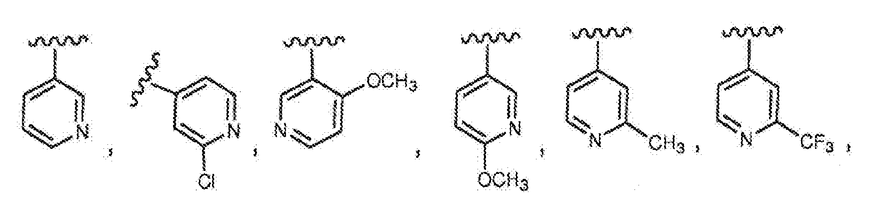 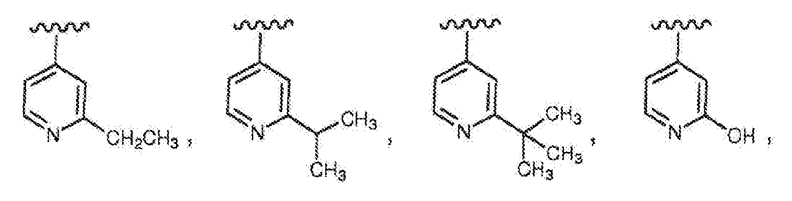 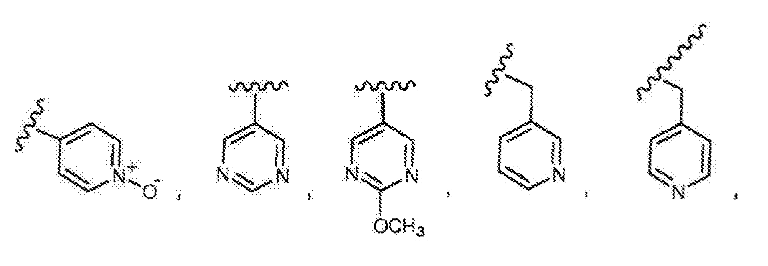 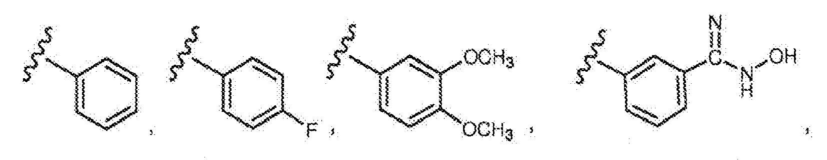 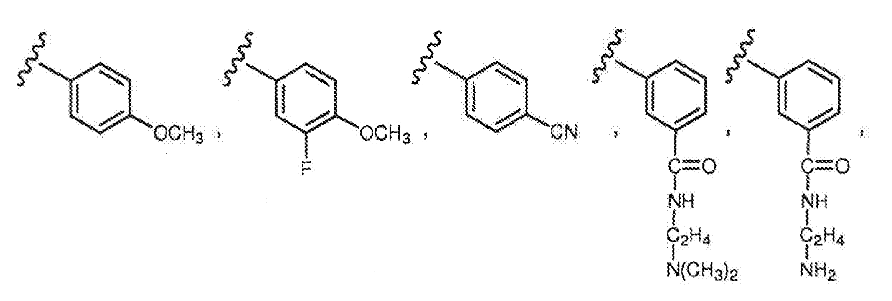 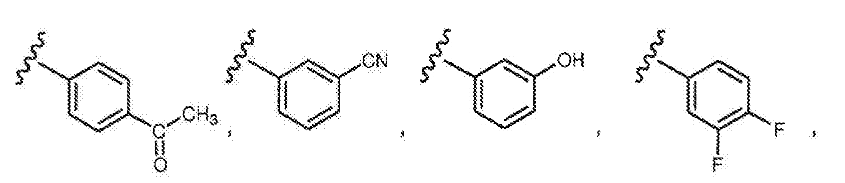 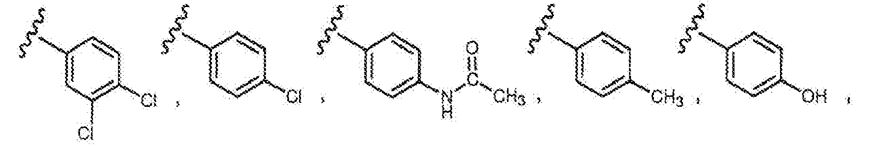 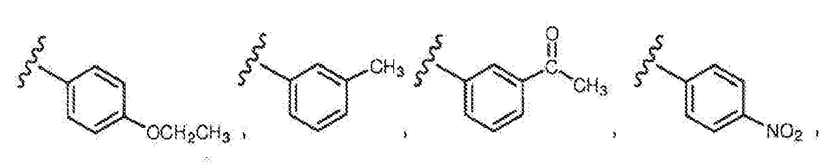 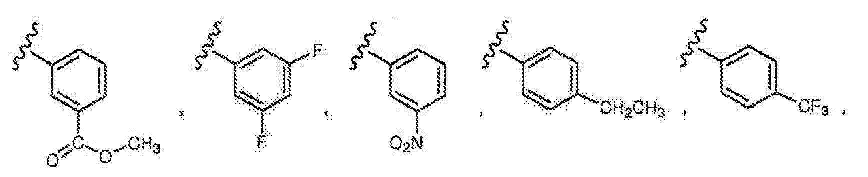 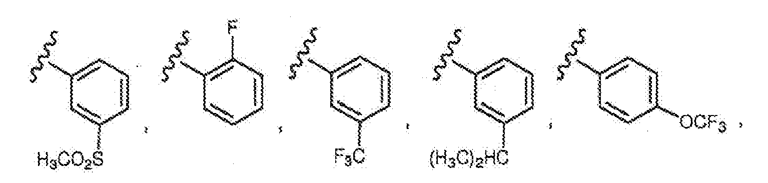 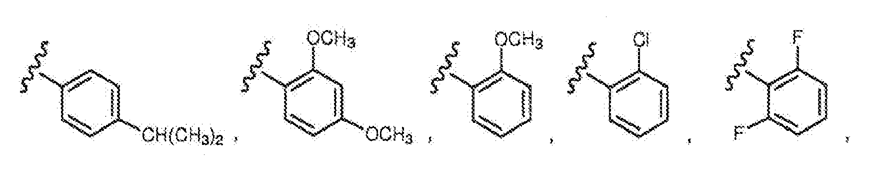 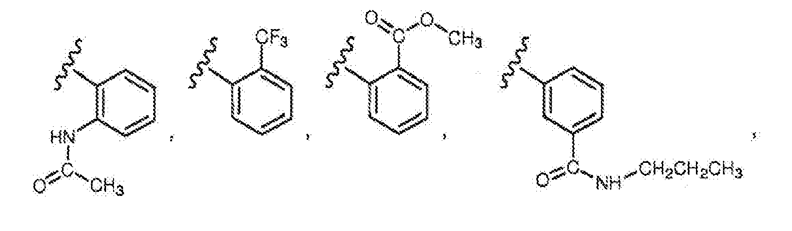 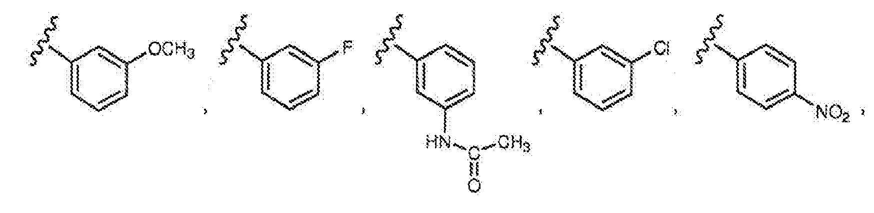 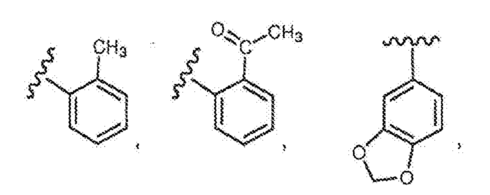 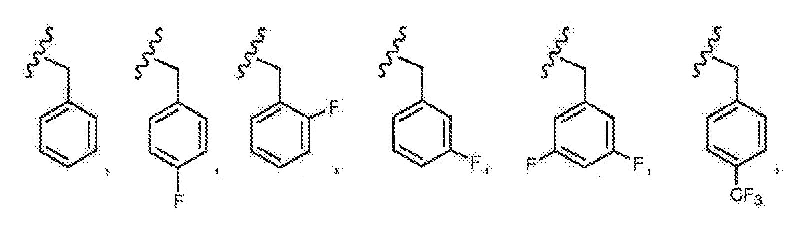 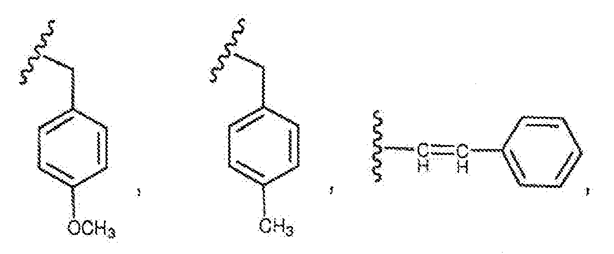 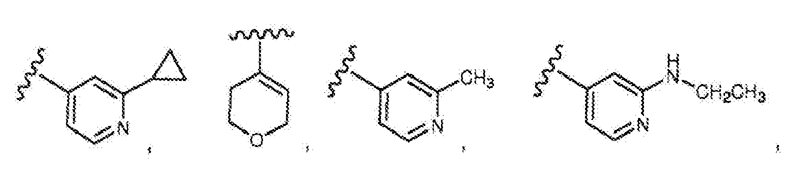 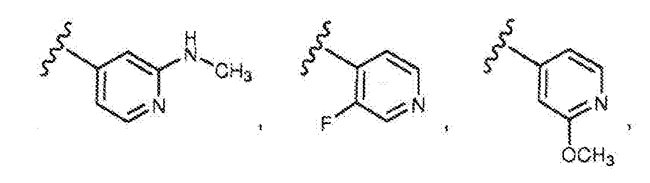 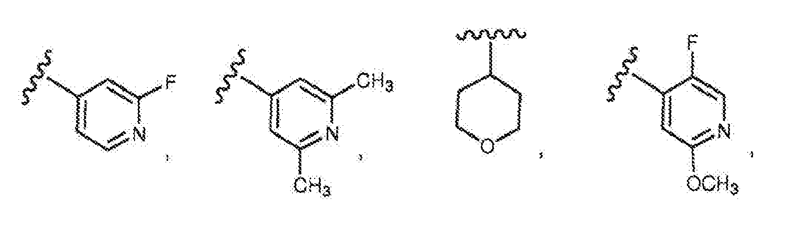 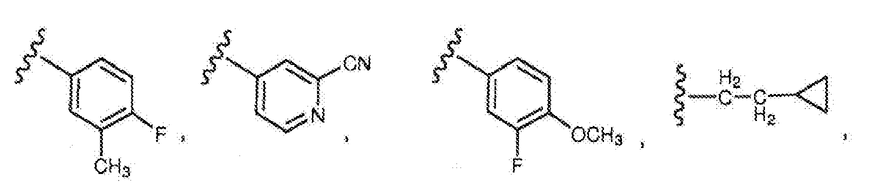 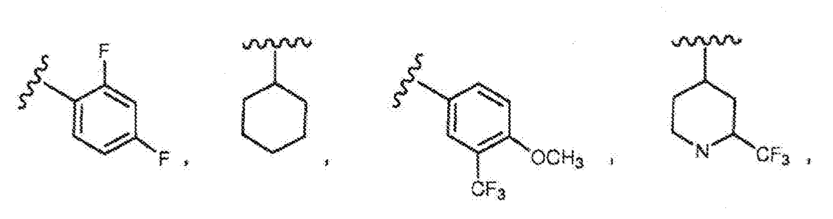 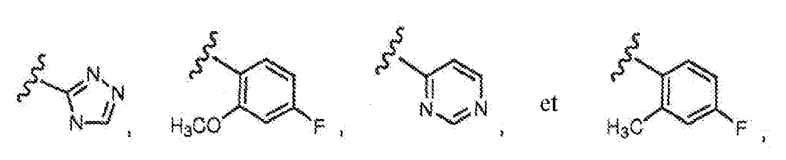 et R⁵ est sélectionné parmi le groupe consistant en:

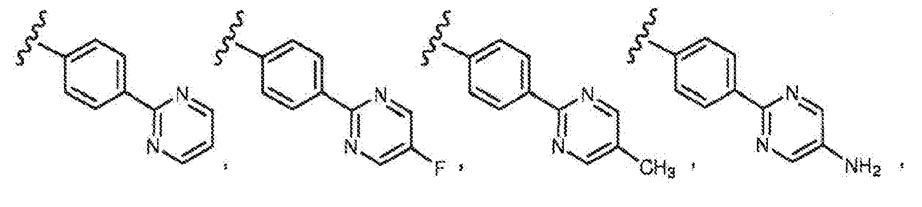

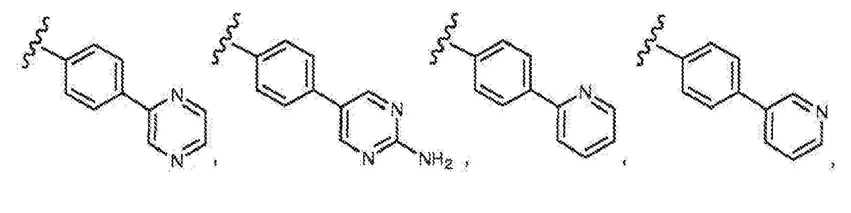

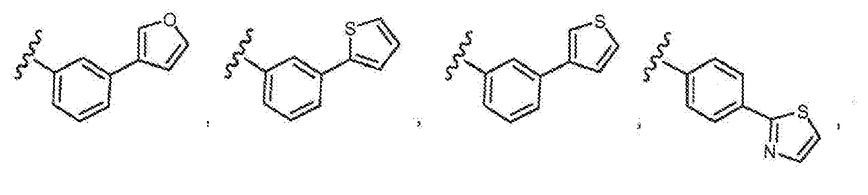

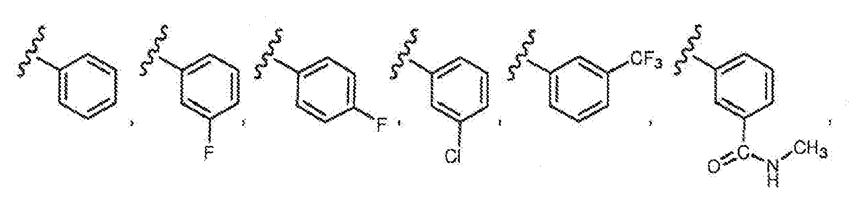

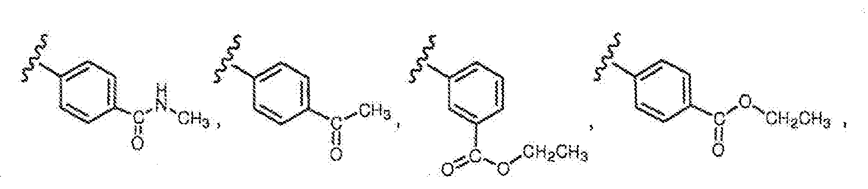

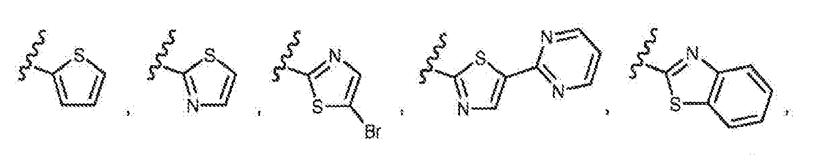

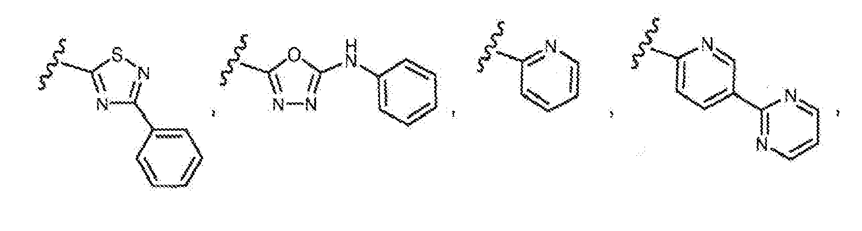

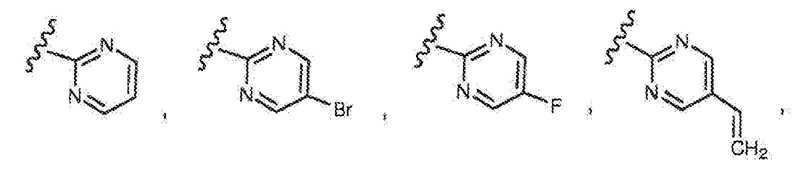

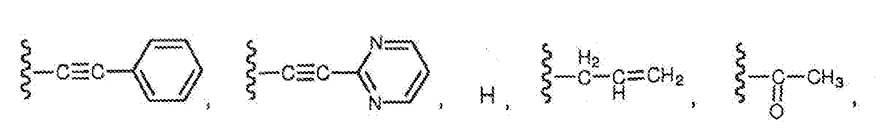

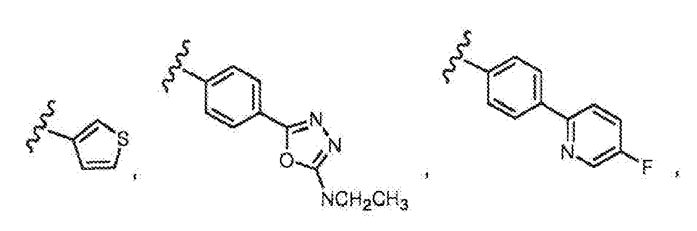

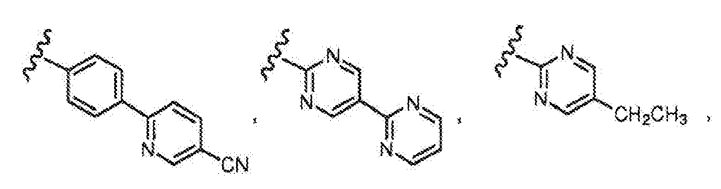

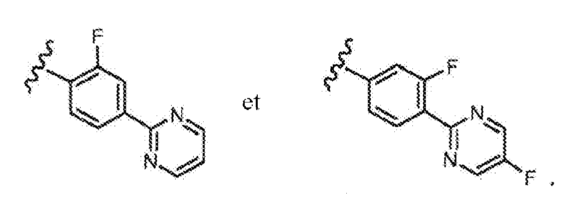

42. Composé selon la revendication 41 ou un sel pharmaceutiquement acceptable de celui-ci, dans lequel R¹ est sélectionné parmi le groupe consistant en:

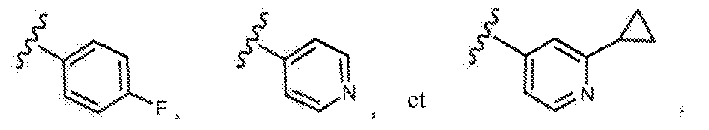

43. Composé selon l'une quelconque des revendications 1 ou 41 ou un sel pharmaceutiquement acceptable de celui-ci, dans lequel R² est sélectionné parmi le groupe consistant en:

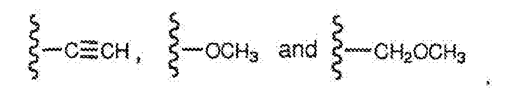

44. Composé selon la revendication 41 ou un sel pharmaceutiquement acceptable de celui-ci, dans lequel Q est sélectionné parmi le groupe consistant en: 2.1, 2.2 et 2.3 et 2.3 est sélectionné parmi le groupe consistant en: 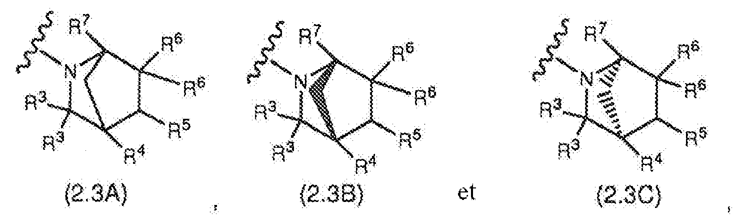 R³, R⁴, R⁶ et R⁷ sont sélectionnés chacun indépendamment parmi le groupe consistant en: H et alkyle.

45. Composé selon la revendication 44 ou un sel pharmaceutiquement acceptable de celui-ci, dans lequel R³, R⁴, R⁶ et R⁷ sont chacun H.

46. Composé selon la revendication 45 ou un sel pharmaceutiquement acceptable de celui-ci, dans lequel Q est 2.1.

47. Composé selon la revendication 41 ou un sel pharmaceutiquement acceptable de celui-ci, dans lequel Q est sélectionné parmi le groupe consistant en: 2.6 et 2.7 et 2.7 est sélectionné parmi le groupe consistant en: 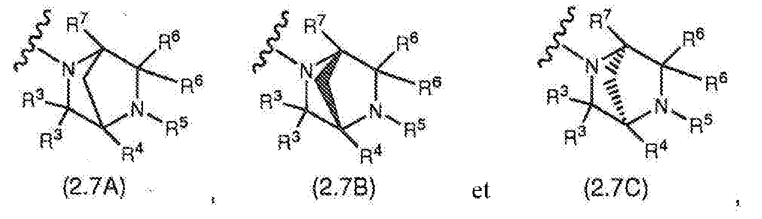 et R³, R⁴, R⁶ et R⁷ sont sélectionnés chacun indépendamment parmi le groupe consistant en: H et alkyle.

48. Composé selon la revendication 47 ou un sel pharmaceutiquement acceptable de celui-ci, dans lequel R³, R⁴, R⁶ et R⁷ sont chacun H.

49. Composé selon la revendication 48 ou un sel pharmaceutiquement acceptable de celui-ci, dans lequel Q est 2.6.

50. Composé selon la revendication 48 ou un sel pharmaceutiquement acceptable de celui-ci, dans lequel Q est 2.7B.

51. Composé selon la revendication 41 ou un sel pharmaceutiquement acceptable de celui-ci, dans lequel Q est sélectionné parmi le groupe consistant en: 2.17, 2.18, 2.19, 2.20, 2.21 et 2.22 et R³, R⁴, R⁶ et R⁷ sont sélectionnés chacun indépendamment parmi le groupe consistant en: H et alkyle.

52. Composé selon la revendication 1 ou un sel pharmaceutiquement acceptable de celui-ci, dans lequel Q est

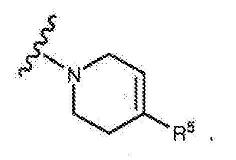

53. Composé selon la revendication 1 sélectionné parmi le groupe consistant en:
(A) les composés finaux des Exemples 1-84, 86-125, 141-418, 420-429, 431, 432 et 434-611.
(B) les composés finaux des Exemples 1, 2, 4-61, 65-73, 77-84, 86, 88-98, 100, 102-114, 116-118, 120, 121, 124, 125, 151-155, 159-179, 183, 184, 186, 188-193, 196-199, 202-205, 250, 253-259, 260-299, 301-318, 320-323, 332-347, 356 (isomère Z), 356 (isomère E), 357-360, 362, 362 (composé 9P), 364, 368-418, 420-429, 431, 432, 434-436, 440-509, 511-602, 606, 607 et 609-611; et
(C) les composés finaux des Exemples 6, 100, 183, 184, 186, 188-192, 261, 440-450 et 510-517;
ou un sel pharmaceutiquement acceptable de ceux-ci.

54. Composé selon la revendication 1 ayant la formule: 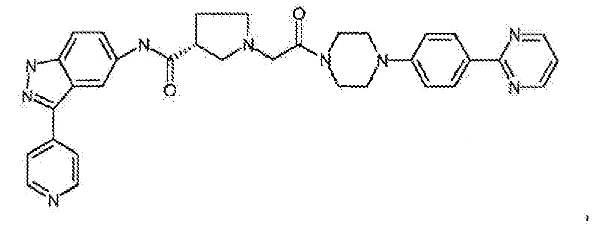 ou un sel pharmaceutiquement acceptable de celui-ci.

55. Composé selon la revendication 1 ayant la formule: 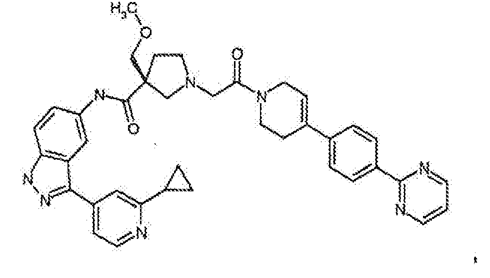 ou un sel pharmaceutiquement acceptable de celui-ci.

56. Composé selon la revendication 1 ayant la formule: 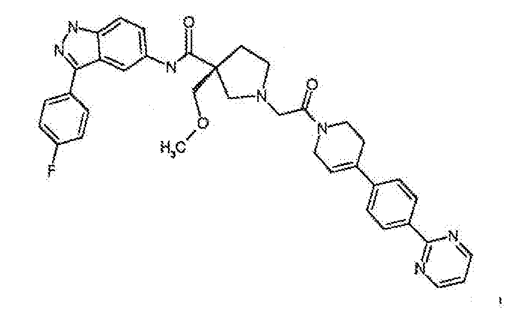 ou un sel pharmaceutiquement acceptable de celui-ci.

57. Composé selon la revendication 1 ayant la formule: 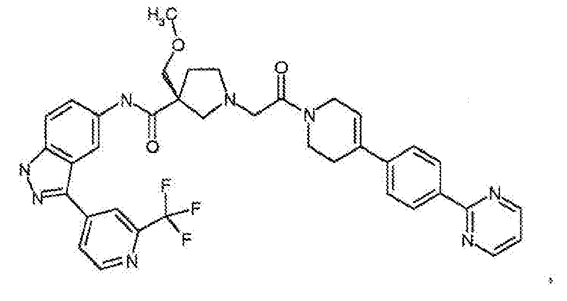 ou un sel pharmaceutiquement acceptable de celui-ci.

58. Composé selon la revendication 1 ayant la formule: 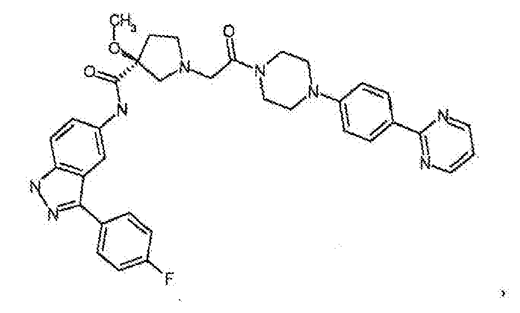 ou un sel pharmaceutiquement acceptable de celui-ci.

59. Composé selon la revendication 1 ayant la formule: 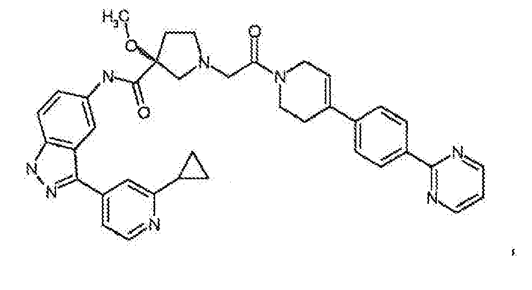 ou un sel pharmaceutiquement acceptable de celui-ci.

60. Composé selon la revendication 1 ayant la formule: 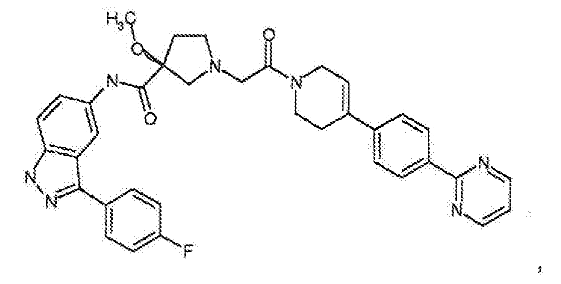 ou un sel pharmaceutiquement acceptable de celui-ci.

61. Composé selon la revendication 1 ayant la formule: 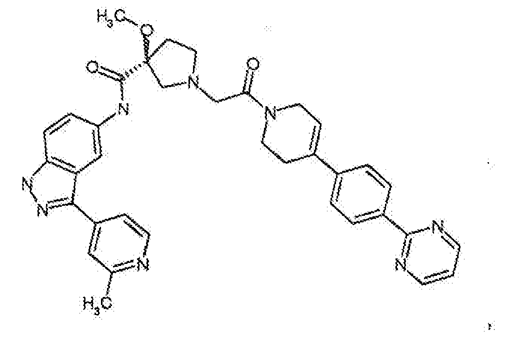 ou un sel pharmaceutiquement acceptable de celui-ci.

62. Composé selon la revendication 1 ayant la formule: 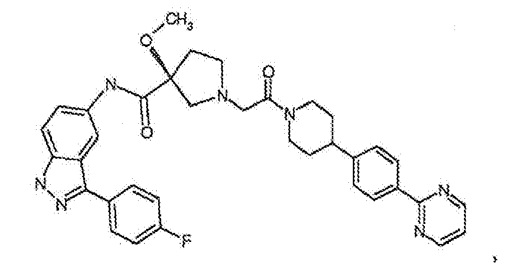 ou un sel pharmaceutiquement acceptable de celui-ci.

63. Composé selon la revendication 1 ayant la formule: 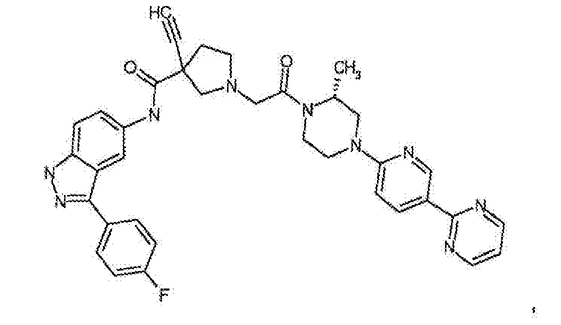 ou un sel pharmaceutiquement acceptable de celui-ci.

64. Composition pharmaceutique comprenant au moins un composé selon la revendication 1 ou un sel pharmaceutiquement acceptable de celui-ci et un véhicule pharmaceutiquement acceptable.

65. Composé selon l'une quelconque des revendications 1 à 63 ou un sel pharmaceutiquement acceptable de celui-ci, à utiliser en tant que médicament.

66. Utilisation d'au moins un composé selon l'une quelconque des revendications 1 à 63 ou d'un sel pharmaceutiquement acceptable de celui-ci:
(A) dans la fabrication d'un médicament pour le traitement du cancer;
(B) dans la fabrication d'un médicament pour le traitement du cancer, où ledit médicament est sous une forme destinée à être administrée avec au moins un agent chimiothérapeutique; ou
(C) dans la fabrication d'un médicament pour le traitement du cancer, ledit médicament est sous une forme destinée à être administrée avec au moins un agent chimiothérapeutique et une radiothérapie.

67. Utilisation d'un composé selon l'une quelconque des revendications 1 à 63 ou d'un sel pharmaceutiquement acceptable de celui-ci, dans la fabrication d'un médicament pour le traitement du cancer, où ledit médicament est sous une forme destinée à être administrée avec au moins un agent chimiothérapeutique sélectionné parmi le groupe consistant en: (1) taxanes, (2) composés de coordination du platine, (3) inhibiteurs du facteur de croissance épidermique (EGF) qui sont des anticorps, (4) inhibiteurs d'EGF qui sont de petites molécules, (5) inhibiteurs du facteur de croissance de l'endothélium vasculaire (VEGF) qui sont des anticorps, (6) inhibiteurs de VEGF-kinase qui sont de petites molécules, (7) antagonistes des récepteurs des oestrogènes ou modulateurs sélectifs des récepteurs des oestrogènes (SERM), (8) dérivés nucléosidiques antitumoraux, (9) épothilones, (10) inhibiteurs de la topoisomérase, (11) vinca-alcaloïdes, (12) anticorps qui sont des inhibiteurs des intégrines αVβ3, (13) antagonistes des folates, (14) inhibiteurs de la ribonucléotide réductase, (15) anthracyclines, (16) substances biologiques, (17) inhibiteurs de l'angiogenèse et/ou suppresseurs du facteur alpha de nécrose tumorale (TNF-alpha) tels que thalidomide (ou imide apparenté), (18) inhibiteurs de Bcr/abl kinase, (19) inhibiteurs de MEK1 et/ou de MEK2 qui sont de petites molécules, (20) inhibiteurs d'IGF-1 et d'IGF-2 qui sont de petites molécules, (21) inhibiteurs à petites molécules de RAF et de BRAF kinases, (22) inhibiteurs à petites molécules des kinases dépendantes du cycle cellulaire telles que CDK1, CDK2, CDK4 et CDK6, (23) agents alkylants et (24) inhibiteurs de la farnésyl protéine transférase.

68. Utilisation d'un composé selon l'une quelconque des revendications 1 à 63 ou d'un sel pharmaceutiquement acceptable de celui-ci, dans la fabrication d'un médicament pour le traitement du cancer, où ledit médicament est sous une forme destinée à être administrée avec au moins un inhibiteur de transduction du signal.

69. Utilisation d'au moins un composé selon l'une quelconque des revendications 1 à 63 ou d'un sel pharmaceutiquement acceptable de celui-ci:
(A) dans la fabrication d'un médicament pour le traitement du cancer, où ledit cancer est sélectionné parmi le groupe consistant en: cancer du poumon, cancer du pancréas, cancer du côlon, leucémies myéloïdes, cancer de la thyroïde, syndrome myélodysplasique, carcinome de la vessie, carcinome épidermique, mélanome, cancer du sein, cancer de la prostate, cancers de la tête et du cou, cancer de l'ovaire, cancers du cerveau, cancers d'origine mésenchymateuse, sarcomes, tétracarcinomes, neuroblastomes, carcinomes du rein, hépatomes, lymphome non hodgkinien, myélome multiple et carcinome anaplasique de la thyroïde;
(B) dans la fabrication d'un médicament pour le traitement du cancer, où ledit cancer est sélectionné parmi le groupe consistant en: mélanome, cancer du pancréas, cancer de la thyroïde, cancer colorectal, cancer du poumon, cancer du sein et cancer de l'ovaire; ou bien
(C) dans la fabrication d'un médicament pour le traitement du cancer, où ledit médicament est sous une forme destinée à être administrée en combinaison avec au moins un agent chimiothérapeutique et où ledit cancer est sélectionné parmi le groupe consistant en: mélanome, cancer du pancréas, cancer de la thyroïde, cancer colorectal, cancer du poumon, cancer du sein et cancer de l'ovaire.

70. Utilisation d'un moins un composé selon l'une quelconque des revendications 1 à 63 ou d'un sel pharmaceutiquement acceptable de celui-ci:
(A) dans la fabrication d'un médicament pour le traitement d'un mélanome;
(B) dans la fabrication d'un médicament pour le traitement d'un mélanome, où ledit médicament est sous une forme destinée à être administrée en combinaison avec au moins un agent chimiothérapeutique;
(C) dans la fabrication d'un médicament pour le traitement du cancer du pancréas;
(D) dans la fabrication d'un médicament pour le traitement du cancer du pancréas, où ledit médicament est sous une forme destinée à être administrée avec au moins un agent chimiothérapeutique;
(E) dans la fabrication d'un médicament pour le traitement du cancer de la thyroïde;
(F) dans la fabrication d'un médicament pour le traitement du cancer de la thyroïde, où ledit médicament est sous une forme destinée à être administrée en combinaison avec au moins un agent chimiothérapeutique;
(G) dans la fabrication d'un médicament pour le traitement du cancer colorectal;
(H) dans la fabrication d'un médicament pour le traitement du cancer colorectal, où ledit médicament est sous une forme destinée à être administrée en combinaison avec au moins un agent chimiothérapeutique;
(I) dans la fabrication d'un médicament pour le traitement du cancer du poumon;
(J) dans la fabrication d'un médicament pour le traitement du cancer du poumon, où ledit médicament est sous une forme destinée à être administrée en combinaison avec au moins un agent chimiothérapeutique;
(K) dans la fabrication d'un médicament pour le traitement du cancer du sein;
(L) dans la fabrication d'un médicament pour le traitement du cancer du sein, où ledit médicament est sous une forme destinée à être administrée en combinaison avec au moins un agent chimiothérapeutique;
(M) dans la fabrication d'un médicament pour le traitement du cancer de l'ovaire;
(N) dans la fabrication d'un médicament pour le traitement du cancer de l'ovaire, où ledit médicament est sous une forme destinée à être administrée en combinaison avec au moins un agent chimiothérapeutique;
(O) dans la fabrication d'un médicament pour le traitement du cancer du sein hormono-dépendant, où ledit médicament est sous une forme destinée à être administrée en combinaison avec des agents anti-hormonaux;
(P) dans la fabrication d'un médicament pour le traitement du cancer du sein hormono-dépendant, où ledit médicament est sous une forme destinée à être administrée en combinaison avec des agents anti-hormonaux et en combinaison avec au moins un agent chimiothérapeutique;
(Q) dans la fabrication d'un médicament pour la prévention du cancer du sein hormono-dépendant, où ledit médicament est sous une forme destinée à être administrée en combinaison avec des agents anti-hormonaux;
(R) dans la fabrication d'un médicament pour la prévention du cancer du sein hormono-dépendant, où ledit médicament est sous une forme destinée à être administrée en combinaison avec des agents anti-hormonaux et en combinaison avec au moins un agent chimiothérapeutique;
(S) dans la fabrication d'un médicament pour le traitement du cancer du cerveau;
(T) dans la fabrication d'un médicament pour le traitement du cancer du cerveau, où ledit médicament est sous une forme destinée à être administrée en combinaison avec au moins un agent chimiothérapeutique;
(U) dans la fabrication d'un médicament pour le traitement du cancer du cerveau, où ledit médicament est sous une forme destinée à être administrée en combinaison avec un agent chimiothérapeutique et où ledit agent chimiothérapeutique est le témozolomide;
(V) dans la fabrication d'un médicament pour le traitement du cancer de la prostate;
(W) dans la fabrication d'un médicament pour le traitement du cancer de la prostate, où ledit médicament est sous une forme destinée à être administrée en combinaison avec au moins un agent chimiothérapeutique;
(X) dans la fabrication d'un médicament pour le traitement du syndrome myélodysplasique;
(Y) dans la fabrication d'un médicament pour le traitement du syndrome myélodysplasique, où ledit médicament est sous une forme destinée à être administrée en combinaison avec au moins un agent chimiothérapeutique;
(Z) dans la fabrication d'un médicament pour le traitement de leucémies myéloïdes;
(AA) dans la fabrication d'un médicament pour le traitement de leucémies myéloïdes, où ledit médicament est sous une forme destinée à être administrée en combinaison avec au moins un agent chimiothérapeutique;
(AB) dans la fabrication d'un médicament pour le traitement de la leucémie myéloïde aiguë;
(AC) dans la fabrication d'un médicament pour le traitement de la leucémie myéloïde aiguë, où ledit médicament est sous une forme destinée à être administrée en combinaison avec au moins un agent chimiothérapeutique;
(AD) dans la fabrication d'un médicament pour le traitement de la leucémie myélomonocytaire chronique;
(AE) dans la fabrication d'un médicament pour le traitement de la leucémie myélomonocytaire chronique, où ledit médicament est sous une forme destinée à être administrée en combinaison avec au moins un agent chimiothérapeutique;
(AF) dans la fabrication d'un médicament pour le traitement de la leucémie myéloïde chronique;
(AG) dans la fabrication d'un médicament pour le traitement de la leucémie myéloïde chronique, où ledit médicament est sous une forme destinée à être administrée en combinaison avec au moins un agent chimiothérapeutique;
(AH) dans la fabrication d'un médicament pour le traitement du cancer de la vessie;
(AI) dans la fabrication d'un médicament pour le traitement du cancer de la vessie, où ledit médicament est sous une forme destinée à être administrée en combinaison avec au moins un agent chimiothérapeutique;
(AJ) dans la fabrication d'un médicament pour le traitement du lymphome non hodgkinien;
(AK) dans la fabrication d'un médicament pour le traitement du lymphome non hodgkinien, où ledit médicament est sous une forme destinée à être administrée en combinaison avec au moins un agent chimiothérapeutique;
(AL) dans la fabrication d'un médicament pour le traitement du myélome multiple;
(AM) dans la fabrication d'un médicament pour le traitement du myélome multiple, où ledit médicament est sous une forme destinée à être administrée en combinaison avec au moins un agent chimiothérapeutique.
